# EUROPEAN PATENT APPLICATION

(11) **EP 1 946 777 A1**
(43) Date of publication of application: **23.07.2008**
(21) Application number: 07384007.6
(22) Date of filing: 16.01.2007
(51) Int. Cl.: A61K 45/06, A61P 3/04

(54) **Substituted pyrazoline for preventing weight gain**

(71) Applicant: Laboratorios del Dr. Esteve S.A., 08041 Barcelona (ES)
(72) Inventor: Fisas-Escasany, María Ángeles, 08025 Barcelona (ES); Buschmann, Helmut H. Dr., 08960 Sant Just Desverns (ES)
(74) Representative: Peters, Hajo

(57) **Abstract**

The present invention relates to an active substance combination comprising at least one substituted pyrazoline compound and at least one drug, known to produce weight-gain as a side effect, a medicament comprising said active substance combination, a pharmaceutical formulation comprising said active substance combination and the use of said active substance combination for the manufacture of a medicament.

## Description

The present invention relates to an active substance combination comprising at least one substituted pyrazoline compound and at least one drug, known to produce weight-gain as a side effect, a medicament comprising said active substance combination, a pharmaceutical formulation comprising said active substance combination and the use of said active substance combination for the manufacture of a medicament.

It is well known by those skilled in the art that many drugs cause weight-gain in subjects taking them (eg see review by Pijl & Meinders, 1996) and that such weight-gain can be deleterious to health and a significant cause of non-compliance to drug therapy.

It was therefore an object of the present invention to provide a way of using these well-known drugs while at the same time preventing the weight-gain in subjects taking them.

It has now surprisingly been found that an active substance combination comprising at least one substituted pyrazoline compound as described herein and the respective drug known to produce weight-gain mostly as a side effect, do prevent this effect.

Thus, in one of its aspects the present invention relates to an active substance combination comprising
(A) at least one substituted pyrazoline compound of general formula I 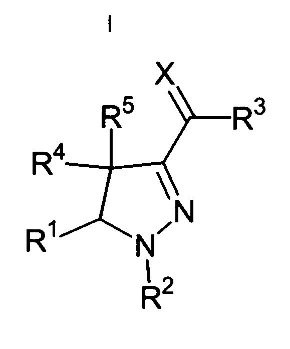 wherein
   - X: is O or S;
   - R¹ and R²,: independently of one another, in each case represent an unsubstituted or at least mono-substituted aryl or heteroaryl radical, which may be condensed with an unsubstituted or at least mono-substituted mono- or polycyclic ring system;
   or a saturated or unsaturated, unsubstituted or at least mono-substituted, optionally at least one heteroatom as a ring member containing cycloaliphatic radical, which may be condensed with an unsubstituted or at least mono- substituted mono- or polycyclic ring system and/or may be bridged by at least one unsubstituted or at least mono-substituted alkylene group;
   - R³: an unsubstituted or at least mono-substituted aryl or heteroaryl radical, which may be condensed with an unsubstituted or at least mono-substituted mono- or polycyclic ring system;
   a saturated or unsaturated, unsubstituted or at least mono-substituted, optionally at least one heteroatom as a ring member containing cycloaliphatic radical, which may be condensed with an unsubstituted or at least mono- substituted mono- or polycyclic ring system and/or may be bridged by at least one unsubstituted or at least mono-substituted alkylene group;
   a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
   a -O-R⁶ moiety; a -NR⁷R⁸ moiety or a -NR⁹-O-R¹⁰ moiety;
   - R⁴: represents H; F; Cl; Br; l; -CN; -NO₂; -NC; -OH; -NH₂; -SH; -C(=O)-H; -C(=O)-OH; -C(=O)-OR¹⁷;
   a saturated or unsaturated, unsubstituted or at least mono-substituted aliphatic radical;
   a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
   an unsubstituted or at least mono-substituted aryl or heteroaryl radical, which may be condensed with an unsubstituted or at least mono-substituted mono- or polycyclic ring system and/or may be bonded via an optionally at least mono-substituted alkylene group, alkenylene group or alkinylene group;
   a -O-R¹¹ moiety; a -S-R¹² moiety; a -NH-R¹³ moiety or a -NR¹⁴R¹⁵ moiety;
   - R⁵: represents H; F; Cl; Br; I; -CN; -NO₂; -NC; -OH; -NH₂; -SH; -C(=O)-H; -C(=O)-OH; -C(=O)-OR¹⁷;
   a saturated or unsaturated, unsubstituted or at least mono-substituted aliphatic radical;
   a saturated or unsaturated, unsubstituted or at least mono-substituted, optionally at least one heteroatom as a ring member containing cycloaliphatic radical, which may be condensed with an unsubstituted or at least mono- substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
   an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
   a -O-R¹¹ moiety; a -S-R¹² moiety; a -NH-R¹³ moiety or a -NR¹⁴R¹⁵ moiety;
   or R⁴ and R⁵ together with the bridging carbon atom form a saturated or unsaturated, unsubstituted or at least mono-substituted, optionally at least one heteroatom as a ring member containing cycloaliphatic radical;
   - R⁶: represents a hydrogen atom;
   a saturated or unsaturated, unsubstituted or at least mono-substituted aliphatic radical;
   a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
   an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
   a -P(=O)(OR¹⁶)₂ moiety; a -C(=O)-OR¹⁷ moiety; a -C(=O)-NH-R¹⁸ moiety or a-C(=O)-R¹⁹ moiety;
   - R⁷ and R⁸,: independently of one another, in each case represent a hydrogen atom;
   a saturated or unsaturated, unsubstituted or at least mono-substituted aliphatic radical;
   a saturated or unsaturated, unsubstituted or at least mono-substituted, optionally at least one heteroatom as a ring member containing cycloaliphatic radical, which may be condensed with an unsubstituted or at least mono- substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
   a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
   an unsubstituted or at least mono-substituted aryl or heteroaryl radical, which may be condensed with an unsubstituted or at least mono-substituted mono-or polycyclic ring system and/or may be bonded via an unsubstituted or at least mono-substituted alkylene group, alkenylene group or alkinylene group;
   a -P(=O)(OR¹⁶)₂ moiety; a -C(=O)-OR¹⁷ moiety; a -C(=O)-NH-R¹⁸ moiety; a -C(=O)-R¹⁹ moiety; a -S(=O)₂-R²⁰ moiety; or a -NR²¹R²² moiety;
   - R⁹: represents hydrogen or a saturated or unsaturated, unsubstituted or at least mono-substituted aliphatic radical;
   - R¹⁰, R¹¹, R¹², R¹³, R¹⁴, R¹⁵ and R²⁰,: independently of one another, in each case represent a saturated or unsaturated, unsubstituted or at least mono-substituted aliphatic radical;
   a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
   or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
   - R¹⁶ , R¹⁷, R¹⁸ and R¹⁹,: independently of one another, in each case represent a saturated or unsaturated, unsubstituted or at least mono-substituted aliphatic radical;
   or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
   and
   - R²¹ and R²²,: independently of one another, in each case represent a hydrogen atom;
   a saturated or unsaturated, unsubstituted or at least mono-substituted aliphatic radical;
   a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group;
   a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
   or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
   optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.
   and
(B) a drug producing weight-gain or obesity.

The effect of the combination was tested using the atypical antipsychotic, olanzapine, as a representative for any drug that has the potential to cause weight-gain and obesity. As stated in the reviews by Newcomer (2005) and Melkersson & Dahl (2004), the "second generation" or "atypical antipsychotic" drugs are known to cause serious weight-gain in approximately 50% of patients on therapy, and in some cases, the increased adiposity has a deleterious effect on their cardio-metabolic status by causing dyslipidaemia, insulin resistance, impaired glucose tolerance, Type 2 diabetes and the Metabolic Syndrome as defined for example by the World Health Organisation (1999), National Cholesterol Education Program Adult Treatment Panel III (2002) and International Diabetes Federation (2005). The two atypical antipsychotic drugs with the greatest propensity to cause weight-gain and obesity are clozapine and olanzapine (see Newcomer, 2005; Melkersson & Dahl, 2004). Olanzapine treatment causes a mean increase in body weight of -5 kg, but the effect can be much greater in some individuals, especially children and adolescents (Haapasalo-Pesu & Saarijarvi, 2001; Ratzoni et al, 2002), and in addition, treatment of psychiatric disorders with this antipsychotic has also been reported to cause dyslipidaemia and Type 2 diabetes (see Newcomer, 2005; Melkersson & Dahl, 2004). For these reasons, olanzapine was chosen to exemplify the beneficial effects of an active substance combination comprising at least one substituted pyrazoline compound according and the respective drug known to produce weight-gain or obesity, especially as a fixed dose, using olanzapine as the weight-gain-inducing drug. As can be seen in the experimental part the effect was surprisingly good. In addition it has also to be considered that the substituted pyrazolines as described ans exemplified herein have also a very beneficial effect on cognitive functions and also mood disorders, which will naturally be also helpful especially if used in combination with an antipsychotic or antidepressant.

Thus - as will be exemplified here - it has now been surprisingly demonstrated that the substituted pyrazolines as described herein given in a fixed-dose combination with olanzapine, an example of a drug known to cause weight-gain and obesity, prevented the hyperphagia and weight-gain induced by this atypical antipsychotic drug, and in addition, this drug combination also caused significcant wight-loss below control values. Such prevention of drug-induced weight-gain provides clinical benefit to the patient by avoiding a deleterious changes in obesity-related cardio-metabolic risk factors, ie increased visceral adiposity and/or increases in plasma triglycerides, cholesterol and/or LDL-cholesterol, decreases in HDL-cholesterol, insulin resistance and/or impaired glucose tolerance and/or hypertension that predisposes him/her to a greatly increased risk of developing dyslipidaemia and/or Type 2 diabetes and/or Metabolic Syndrome, or of suffering a cardio- or cerebrovascular accident.

The weight-loss provided by an active substance combination comprising at least one substituted pyrazoline compound and the respective drug known to produce weight-gain or obesity together in a fixed dose ratio will be of especial therapeutic benefit to a patient, who is in need of therapy with a medication that causes weight-gain, and who is overweight or obese and/or metabolically compromised, and as a consequence, is at increased cardio-metabolic risk. The weight-loss will improve various obesity-related cardio-metabolic risk factors, ie increased visceral adiposity and/or increases in plasma triglycerides, cholesterol and/or LDL-cholesterol, decreases in HDL-cholesterol, insulin resistance and/or impaired glucose tolerance and/or hypertension that predispose him/her to a greatly increased risk of developing dyslipidaemia and/or Type 2 diabetes and/or Metabolic Syndrome, or of suffering a cardio- or cerebrovascular accident.

Examples of therapeutic classes and drugs causing weight-gain include, but are not limited to, the following:

Antipsychotics, comprising the atypical antipsychotics including, but not limited to, olanzapine clozapine, quetiepine, risperidone, amisulpiride, zotepine, sertindole, aripiprazole and ziprasidone, and the older typical neuroleptics including, but not limited to, chlorpromazine, haloperidol, fluphenazine, mesoridazine and thioridazine.

Antidepressants, comprising the non-selective, irreversible monoamine oxidase A and B inhibitors (MAOIs) including, but not limited to, tranylcypromine, phenelzine and isocarboxacid, the tricyclic antidepressants (TCAs) including, but not limited to, amitriptyline, amoxapine, clomipramine, desipramine, doxepin, imiprimine, nortriptyline, protriptyline and trimiprimine, the serotonin-selective reuptake inhibitors including, but not limited to, fluoxetine, sertraline, paroxetine and citalopram, the so-called "third generation" and atypical antidepressants including, but not limited to, mirtazepine, venlafaxine, mianserin and trazodone.

Drugs to treat mania in bipolar disorder including, but not limited to, lithium, olanzapine, quetiepine and sodium valproate.

Corticosteroids to treat inflammation including, but not limited to, prednisone, prednisolone, budesonide, dexamethasone, methylprednisolone and betamethasone

Drugs for the treatment of Type 1 diabetes, insulin resistance, impaired glucose tolerance and Type 2 diabetes including, but not limited to the sulphonylureas (chlorpropamide, tolbutamide, acetoheximide, tolazamide, glibenclamide, glipizide, gliclazide, glimepiride, gliquidone, glyburide), the meglitinides (netaglinide, repaglinide, mitiglitinide), peroxisome proliferator activated receptors type gamma (PPARy) agonists (troglitazone, rosiglitazone, pioglitazone, ciglitazone, darglitazone), inhibitors of hepatic glucose production (glucagon antagonists, glucose-6-phosphatase inhibitors, glycogen phosphorylase inhibitors), insulin (human insulin lispro, human insulin aspart, neutral insulin, human neutral insulin [pyr], human neutral insulin [prb], bovine neutral insulin, porcine neutral insulin, human isophane insulin, isophase insulin [prb], human isophane insulin [pyr], porcine isophane insulin, bovine isophane insulin, human insulin zinc suspension [pyr[, humulin [prb], porcine insulin zinc suspension, human insulin zinc suspension [prb], human insulin glargine, protamine zinc insulin injection [prb], bovine insulin zinc suspension, bovine protamine zinc insulin, biphasic human insulin [pyr], biphasic human insulin [prb], biphasic neutral and isophane human insulin [prb], human insulin [emp], biphasic porcine neutral and isophane insulin, biphasic insulin aspart, insulin determir), dual acting insulin secretagogues/insulin sensitisers (pituitary adenylate cyclase-activating peptide/ vasoactive intestinal peptide receptor [VPAC] 2 receptor agonists), modulators of glucose uptake (GLUT 4 translocators, retinoid X receptor agonists, Iκ β kinase [IKK] β inhibitors, p38 MAP kinase inhibitors, protein tyrosine phosphatase 1B [PTP1B] inhibitors, insulin receptor tyrosine kinase activators, *α*-glucosidase inhibitors, sodium/glucose co-transport inhibitors, glycogen synthase kinase 3 [GSK3] inhibitors) and modulators of mitochondrial metabolism. Medications may also comprise combinations of the above listed compounds, including, but not limited to rosiglitazone + metformin, rosiglitazone + simvastatin, rosiglitazone + sulphonurea, metformin + glipizide, sitagliptin + metformin, PPARγ/α combination medications (sipoglitazar, tesaglitazar, muraglitazar, reglitazar, peliglitazar, cevoglitazar, ragaglitazar, naveglitazar, imiglitazar, aloglitazar, sodelglitazar, peliglitazar, pemoglitazar, netoglitazone), PPARγ/α/δ combination medications.

Antihypertensives including, but not limited to, β-adrenoceptor blockers, eg propranolol, metoprolol and atenolol, and α₁-adrenoceptor blockers, eg doxasin and prazosin.

Drugs for the treatment of gastro-oesophogeal reflux, dyspepsia and ulcers including, but not limited to, esomeprazole, omeprazole and lansoprazole.

Drugs for the treatment and prevention of epilepsy and seizure disorders including, but not limited to, vigabatrin, piracetam, carbamazepine, pregabalin, gabapentin, lamotrigine, and sodium valproate.

Drugs for the treatment or prevention of neuropathic pain including, but not limited to, carbamazepine, pregabalin, gabapentin, sodium valproate, lamotrigine and the tricyclic antidepressants, eg amitriptyline, amoxapine, clomipramine, desipramine, doxepin, imiprimine, nortriptyline, protriptyline and trimiprimine, or atypical and "third generation" antidepressant drugs, eg mirtazepine, venlafaxine, mianserin and trazodone.

Drugs for the treatment and prevention of migraine including, but not limited to, methysergide, pizotifen, memantine, antiepileptic drugs, eg vigabatrin, piracetam, carbamazepine, pregabalin, gabapentin, lamotrigine, and sodium valproate, tricyclic antidepressants, eg amitriptyline, amoxapine, clomipramine, desipramine, doxepin, imiprimine, nortriptyline, protriptyline and trimiprimine, the serotonin-selective reuptake inhibitors including, but not limited to, fluoxetine, sertraline, paroxetine and citalopram, and atypical and "third generation" antidepressant drugs, eg mirtazepine, venlafaxine, mianserin and trazodone.

Antihistamines including, but not limited to, diphenhydramine and chlorpheniramine.

Benzodiazepines including, but not limited to, clobazam.

Contraceptive medication, particularly injectable depot contraceptives including, but not limited to, depot medroxyprogesterone acetate (DMPA)

Hormone replacement therapy including, but not limited to, synthetic or natural oestrogen and progesterone and their analogues administered either alone or in combination.

In a highly preferred embodiment the active substance combination according to the present invention comprises as (B) the drug producing weight-gain or obesity a drug selected from the following therapeutic classes antipsychotics, comprising the atypical antipsychotics and typical neuroleptics, antidepressants including MAOls, TCAs, SSRls, "third generation" and atypical antidepressants, anti-manic agents, corticosteroids, medicaments for treating insulin resistance, impaired glucose tolerance and/or Type 2 diabetes, antihypertensives, anti-ulceratives, medicaments for treating neuropathic pain, antihistamines, medicaments for treating migraine, benzodiazepines, contraceptives and hormone replacement therapy. More specific examples in the therapeutic classes have been exemplified above.

Preferably the active substance combination according to the present invention comprises as compound (A) one or more substituted pyrazoline compounds of general formula I given above, wherein
- X: is O or S;
- R¹ and R²,: independently of one another, in each case represent an unsubstituted or at least mono-substituted 6- or 10-membered aryl radical, which may be condensed with an unsubstituted or at least mono-substituted mono- or polycyclic ring system;
an unsubstituted or at least mono-substituted 5- to 14-membered heteroaryl radical, which may be condensed with an unsubstituted or at least mono-substituted mono- or polycyclic ring system;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅ alkylene group;
or an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅alkylene group;
- R³: represents an unsubstituted or at least mono-substituted 6- or 10-membered aryl radical, which may be condensed with an unsubstituted or at least mono-substituted mono- or polycyclic ring system;
an unsubstituted or at least mono-substituted 5- to 14-membered heteroaryl radical, which may be condensed with an unsubstituted or at least mono-substituted mono- or polycyclic ring system;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅ alkylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅ alkylene group;
an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical which together with an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical forms an unsubstituted or at least mono-substituted spirocyclic residue via a common ring atom;
a -O-R⁶ moiety; a -NR⁷R⁸ moiety or a -NR⁹-O-R¹⁰ moiety;
- R⁴: represents H; F; Cl; Br; I; -CN; -NO₂; -NC; -OH; -NH₂; -SH; -C(=O)-H; -C(=O)-OH; -C(=O)-OR¹⁷;
an unsubstituted or at least mono-substituted C₁₋₁₆ alkyl radical, C₂₋₁₆ alkenyl radical or C₂₋₁₆alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bonded via an unsubstituted or at least mono-substituted C₁₋₅ alkylene group, C₂₋₅alkenylene group or C₂₋₅ alkinylene group and/or may be bridged by at least one unsubstituted or at least mono-substituted C₁₋₅ alkylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bonded via an unsubstituted or at least mono-substituted C₁₋₅ alkylene group, C₂₋₅ alkenylene group or C₂₋₅ alkinylene group and/or may be bridged by at least one unsubstituted or at least mono-substituted C₁₋₅ alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅ alkinylene group;
a -O-R¹¹ moiety; a -S-R¹² moiety; a -NH-R¹³ moiety or a -NR¹⁴R¹⁵ moiety;
- R⁵: represents H; F; Cl; Br; I; -CN; -NO₂; -NC; -OH; -NH₂; -SH; -C(=O)-H; -C(=O)-OH; -C(=O)-OR¹⁷;
an unsubstituted or at least mono-substituted C₁₋₁₆ alkyl radical, C₂₋₁₆alkenyl radical or C₂₋₁₆alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bonded via an unsubstituted or at least mono-substituted C₁₋₅ alkylene group, C₂₋₅alkenylene group or C₂₋₅alkinylene group and/or may be bridged by at least one unsubstituted or at least mono-substituted C₁₋₅ alkylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bonded via an unsubstituted or at least mono-substituted C₁₋₅ alkylene group, C₂₋₅ alkenylene group or C₂₋₅ alkinylene group and/or may be bridged by at least one unsubstituted or at least mono-substituted C₁₋₅alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅ alkinylene group;
-O-R¹¹ moiety; a -S-R¹² moiety; a -NH-R¹³ moiety or a -NR¹⁴R¹⁵ moiety;
or R⁴ and R⁵ together with the bridging carbon atom form an unsubstituted or at least mono-substituted C₃₋₁₆ cycloalkyl radical or C₄₋₁₆ cycloalkenyl radical;
- R⁶: represents a hydrogen atom;
an unsubstituted or at least mono-substituted C₁₋₁₆ alkyl radical, C₂₋₁₆ alkenyl radical or C₂₋₁₆ alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅ alkylene group and/or may be bonded via an unsubstituted or at least mono-substituted C₁₋₅alkylene group, C₂₋₅alkenylene group or C₂₋₅ alkinylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅ alkylene group and/or may be bonded via an unsubstituted or at least mono-substituted C₁₋₅alkylene group, C₂₋₅alkenylene group or C₂₋₅ alkinylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅ alkinylene group;
an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₁₅ alkylene group, C₂₋₅ alkenylene group or C₂₋₅alkinylene group;
a -P(=O)(OR¹⁶)₂ moiety; a -C(=O)-OR¹⁷ moiety; a -C(=O)-NH-R¹⁸ moiety or a-C(=O)-R¹⁹ moiety;
- R⁷ and R⁸,: independently of one another, in each case represent a hydrogen atom;
an unsubstituted or at least mono-substituted C₁-₁₆ alkyl radical, C₂₋₁₆-alkenyl radical or C₂₋₁₆-alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅alkylene group and/or may be bonded via an unsubstituted or at least mono-substituted C₁₋₅alkylene group, C₂₋₅alkenylene group or C₂₋₅ alkinylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅ alkylene group and/or may be bonded via an unsubstituted or at least mono-substituted C₁₋₅alkylene group, C₂₋₅alkenylene group or C₂₋₅ alkinylene group;
an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical which together with an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical forms an unsubstituted or at least mono-substituted spirocyclic residue via a common ring atom;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅ alkinylene group;
an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅ alkinylene group;
a -P(=O)(OR¹⁶)₂ moiety; a -C(=O)-OR¹⁷ moiety; a -C(=O)-NH-R¹⁸ moiety; a-C(=O)-R¹⁹ moiety; a -S(=O)₂-R²⁰ moiety; or a -NR²¹R²² moiety;
- R⁹: represents a hydrogen atom or an unsubstituted or at least mono-substituted C₁₋₁₆alkyl radical, C₂₋₁₆alkenyl radical or C₂₋₁₆alkinyl radical;
- R¹⁰, R¹¹, R¹², R¹³, R¹⁴, R¹⁵ and R²⁰,: independently of one another, in each case represent an unsubstituted or at least mono-substituted C₁₋₁₆ alkyl radical, C₂₋₁₆ alkenyl radical or C₂₋₁₆alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅alkylene group and/or may be bonded via an unsubstituted or at least mono-substituted C₁₋₅alkylene group, C₂₋₅ alkenylene group or C₂₋₅ alkinylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅ alkylene group and/or may be bonded via an unsubstituted or at least mono-substituted C₁₋₅alkylene group, C₁₋₅alkenylene group or C₂₋₅ alkinylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅ alkylene group, C₂₋₅ alkenylene group or C₂₋₅alkinylene group;
- R¹⁶, R¹⁷, R¹⁸ and R¹⁹,: independently of one another, in each case represent an unsubstituted or at least mono-substituted C₁₋₁₆ alkyl radical, C₂₋₁₆alkenyl radical or C₂₋₁₆alkinyl radical;
or an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅alkinylene group;
and
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅ alkinylene group;
- R²¹ and R²²,: independently of one another, in each case represent a hydrogen atom;
an unsubstituted or at least mono-substituted C₁₋₁₆ alkyl radical, C₂₋₁₆ alkenyl radical or C₂₋₁₆ alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bonded via an unsubstituted or at least mono-substituted C₁₋₅ alkylene group, C₂₋₅alkenylene group or C₂₋₅alkinylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bonded via an unsubstituted or at least mono-substituted C₁₋₅ alkylene group, C₂₋₅alkenylene group or C₂₋₅alkinylene group;
an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical which together with an unsubstituted or at least mono-substituted C₃₋₁₆ cycloalkyl radical, C₄₋₁₆ cycloalkenyl radical, C₄₋₁₆ heterocycloalkyl radical or C₅-₁₆ heterocycloalkenyl radical forms an unsubstituted or at least mono-substituted spirocyclic residue via a common ring atom;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅alkinylene group;
whereby
the rings of the aforementioned ring system are in each case independently of one another 5- 6- or 7-membered and may in each case independently of one another optionally contain 1, 2 or 3 heteroatom(s) independently selected from the group consisting of nitrogen, oxygen and sulfur;
the aforementioned heteroaryl radicals in each case optionally contain 1, 2, 3 or 4 heteroatom(s) independently selected from the group consisting of nitrogen, oxygen and sulfur as ring member(s);
the aforementioned heterocycloalkyl radicals and heterocycloalkenyl radicals in each case optionally contain 1, 2, 3 or 4 heteroatom(s) independently selected from the group consisting of nitrogen, oxygen and sulfur as ring member(s);
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

Preferably the active substance combination according to the present invention comprises as compound (A) one or more substituted pyrazoline compounds of general formula I given above, wherein
- X: is O or S;
- R¹: represents a radical selected from the group consisting of phenyl and thienyl (thiophenyl), which may optionally be substituted with 1, 2, 3, 4 or 5 substituent(s) independently selected from the group consisting of -OH, -O-CH₃, -O-C₂H₅, F, Cl, Br and I;
- R²: represents a phenyl radical, which may be substituted with 1, 2, 3, 4 or 5 substituent(s) independently selected from the group consisting of -OH, -O-CH₃, -O-C₂H₅, F, Cl, Br and I;
- R³: represents a radical selected from the group consisting of 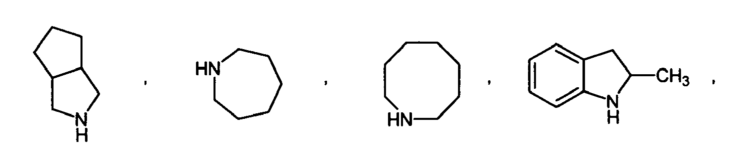 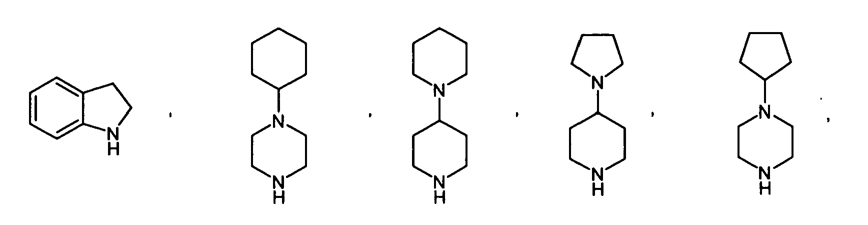 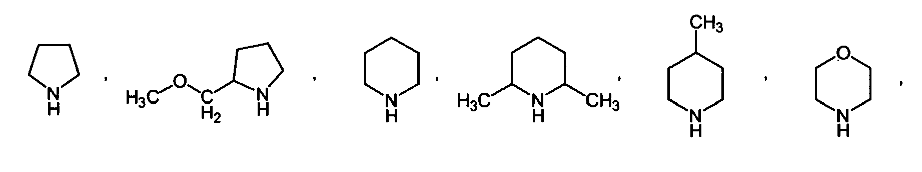 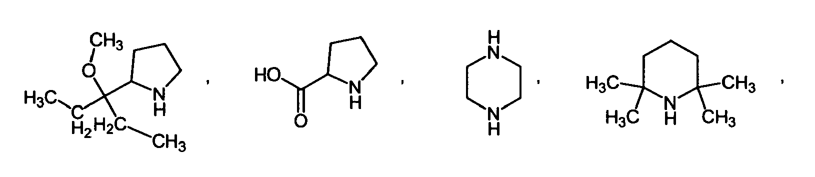 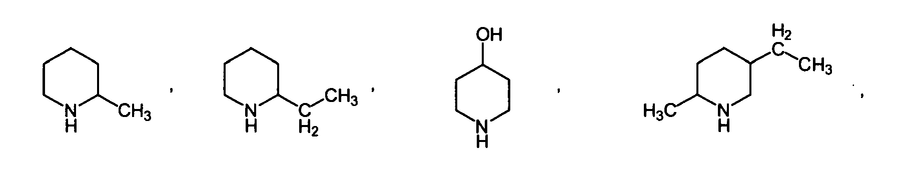 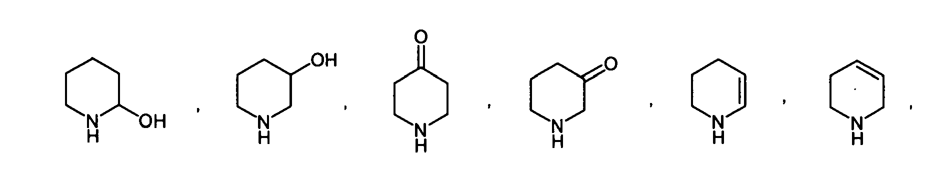 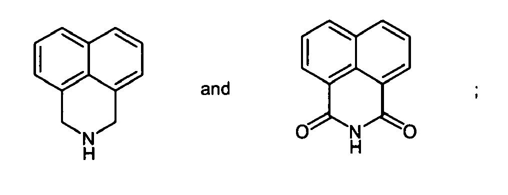
which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a -OR⁶-moiety or a -NR⁷R⁸ moiety;
- R⁴: represents H; F; Cl; Br; I; -OH, -CN; -C(=O)-H; -C(=O)-OH; -C(=O)-OR¹⁷;
a radical selected from the group consisting of methyl, -CF₃, -CH₂F, -CF₂H,-C₂F₅, ethyl, -CH₂-CN, -CH₂-OH, n-propyl, isopropyl, -CH₂-CH₂-CN, -CH₂-CH₂-OH, n-butyl, -CH₂-CH₂-CH₂-CN, -CH₂-CH₂-CH₂-OH, isobutyl, sec-butyl, tert-butyl, n-pentyl, 2-pentyl, 3-pentyl, neo-pentyl, n-hexyl, 2-hexyl and 3-hexyl;
a benzyl radical or a -O-R¹¹ moiety;
- R⁵: represents H; F; Cl; Br;-C(=O)-OH; -C(=O)-OR¹⁷; or a radical selected from the group consisting of methyl, ethyl, n-propyl, iso-propyl and n-butyl;
or R⁴ and R⁵ together with the bridging carbon atom form a radical selected from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl;
- R⁶: represents a hydrogen atom; a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;
- R⁷: represents a hydrogen atom or a radical selected from the group consisting of methyl, ethyl, n-propyl, isopropyl, n-butyl, sec-butyl and tert-butyl;
- R⁸: represents a radical selected from the group consisting of [1,2,3,4]-tetrahydronaphthyl, bicyclo[2.2.1]heptyl, cyclopropyl, cyclobutyl, cyclopentyl, cyclohexyl, cycloheptyl, cyclooctyl, cyclononyl, cyclodecyl, cycloundecyl and cyclododecyl, which may be bonded via a -(CH₂)-, -(CH₂)-(CH₂)- or -(CH₂)-(CH₂)-(CH₂)-group and/or subsituted with 1, 2, 3 or 4 substituents selected from the group consisting of methyl, ethyl, n-propyl, isopropyl, n-butyl, sec-butyl, tert-butyl, -OH, -O-CH₃ and -O-C₂H₅;
or a radical selected from the group consisting of 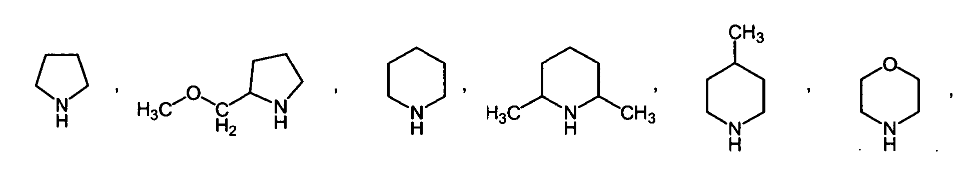 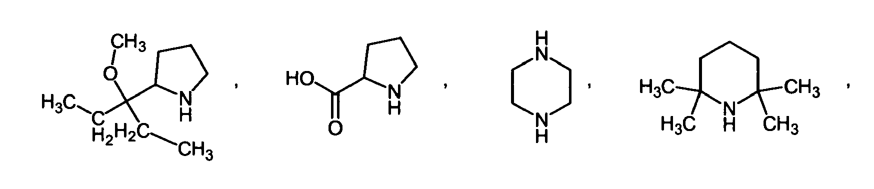 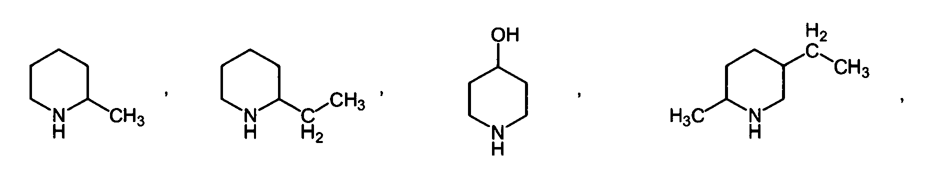 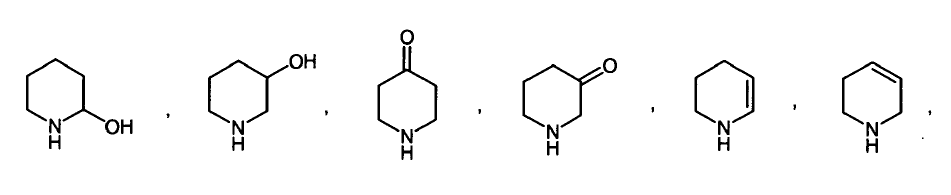 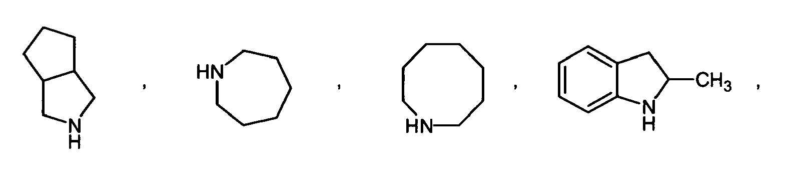 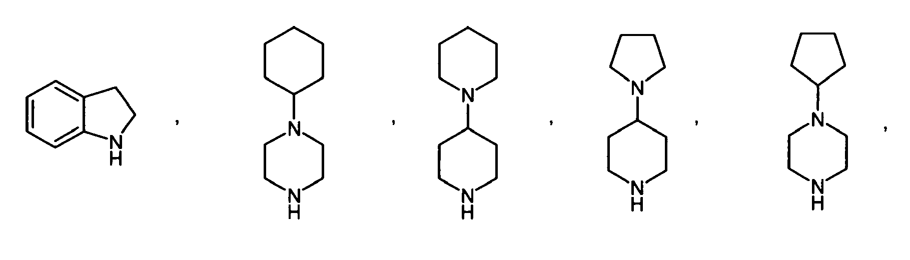 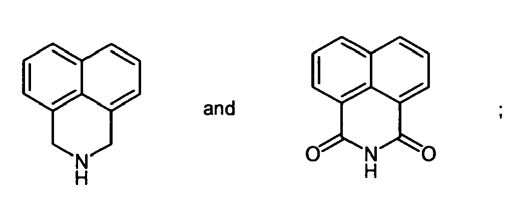 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
and
- R¹¹ and R¹⁷,: independently of one another, each represent a radical selected from the group consisting of methyl, ethyl, n-propyl, isopropyl, n-butyl, isobutyl, sec-butyl, tert-butyl, n-pentyl, 2-pentyl, 3-pentyl and neo-pentyl;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the active substance combination according to the present invention comprises as compound (A) of subgroup A1 of general formula I given above, wherein
X represents O
R¹ represents a phenyl ring, which is mono-substituted with a halogen atom, preferably a chlorine atom, in its 4-position,
R² represents a phenyl ring, which is di-substituted with two halogen atoms, preferably chlorine atoms, in its 2- and 4-position,
R³ represents a pyrrolidinyl group; a piperidinyl group; a piperazinyl group; a homo-piperazinyl group; a morpholinyl group; or an -NR⁷R⁸-moiety,
R⁴ represents a hydrogen atom,
R⁵ represents a hydrogen atom
R⁷ represents a hydrogen atom or a linear or branched C₁₋₆-alkyl group,
R⁸ represents a linear or branched C₁₋₆ alkyl group; an SO₂-R²⁰-moiety; a pyrrolidinyl group; a piperidinyl group; a piperazinyl group; a homo-piperazinyl group; a morpholinyl group; or a triazolyl group, whereby each of the heterocyclic rings may be substituted with one or more, identical or different, C₁₋₆-alkyl groups, and
R²⁰ represents a phenyl group, which is optionally substituted with one or more C₁₋₆ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

As a preferred embodiment of subgroup A1 the compound (A) of general formula I given above, is selected from the group consisting of:
N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1 H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsulfonamide,
optionally in the form of a corresponding N-oxide, a corresponding salt or a corresponding solvate.

As a preferred embodiment the active substance combination according to the present invention comprises as compound (A) of subgroup A2 compounds of general formula Ia or Ib 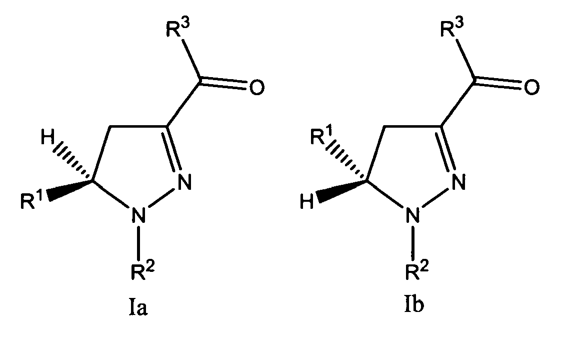 wherein
X represents O
R¹ represents a phenyl ring, which is mono-substituted with a halogen atom, preferably a chlorine atom, in its 4-position,
R² represents a phenyl ring, which is di-substituted with two halogen atoms, preferably chlorine atoms, in its 2- and 4-position,
R³ represents a pyrrolidinyl group, a piperidinyl group, a piperazinyl group, a homo-piperazinyl group, a morpholinyl group, or an -NR⁷R⁸-moiety,
R⁴ represents a hydrogen atom,
R⁵ represents a hydrogen atom,
R⁷ represents a hydrogen atom or a linear or branched C₁₋₆-alkyl group,
R⁸ represents a linear or branched C₁₋₆ alkyl group; an -SO₂-R²⁰-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, C₁₋₆-alkyl groups, and
R²⁰ represents a phenyl group, which is optionally substituted with one or more C₁₋₆ alkyl groups, which may be identical or different,
optionally in form of a corresponding N-oxide thereof, or a corresponding salt thereof, or a corresponding solvate thereof.

As a preferred embodiment of compound (A) of subgroup A2 the compound is selected from:
- (R)-N-piperidinyl-5-(4-chlorophenyl)-1-(2,4-dichlorophenyl)-4,5-dihydro-1H-pyrazole-3-carboxamide and
- (S)-N-piperidinyl-5-(4-chlorophenyl)-1-(2,4-dichlorophenyl)-4,5-dihydro-1H-pyrazole-3-carboxamide;
   optionally in form of a corresponding N-oxide thereof, or a corresponding salt thereof, or a corresponding solvate thereof.

As a preferred embodiment the active substance combination according to the present invention comprises as compound (A) of subgroup A3 of general formula I given above, wherein
- X: is O;
- R¹: represents a radical selected from the group consisting of 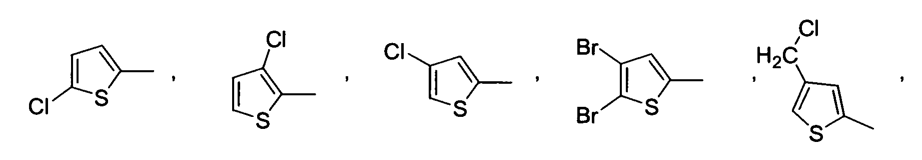 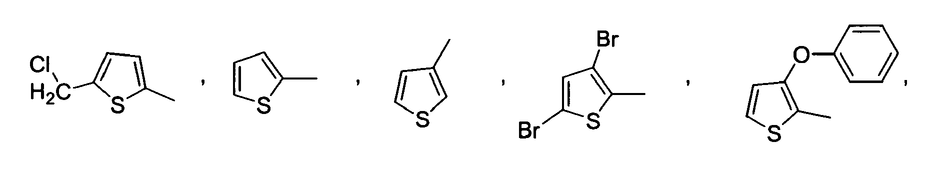 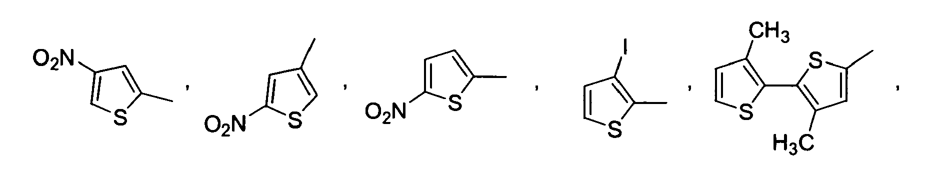 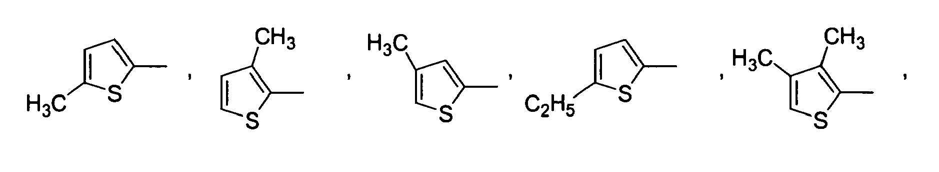 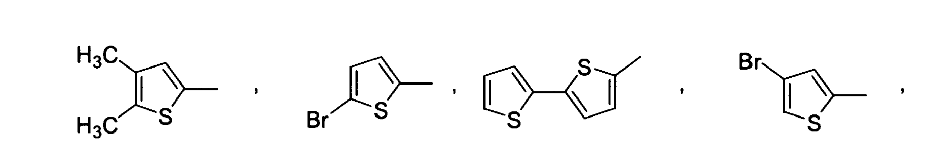 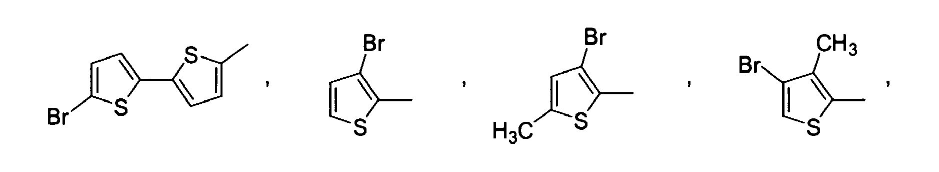 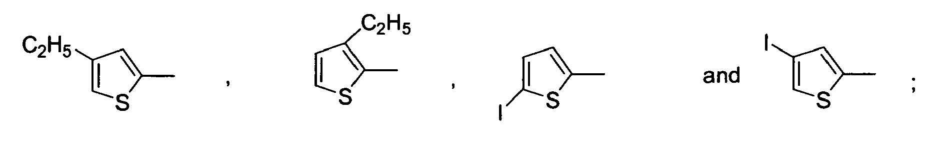 wherein the single line in each case represents the bond to the pyrazoline compound of general formula I;
- R²: represents a phenyl radical, which may be substituted with 1, 2, 3, 4 or 5 substituent(s) independently selected from the group consisting of F, Cl, Br and I;
- R³: represents a radical selected from the group consisting 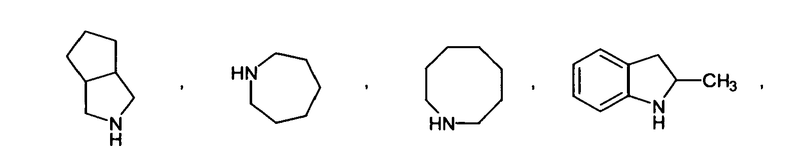 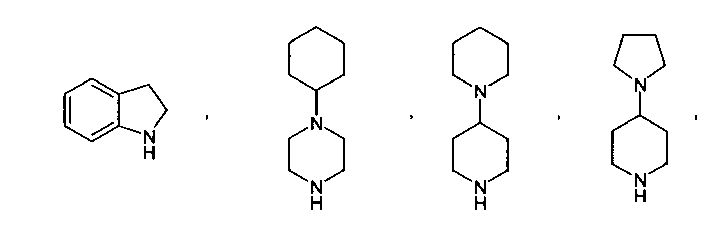 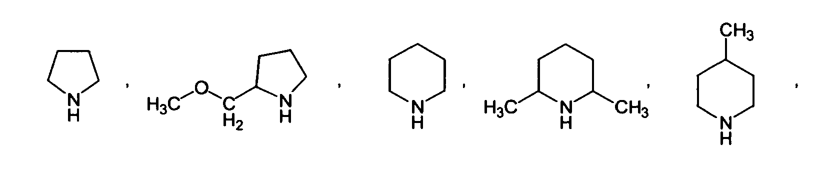 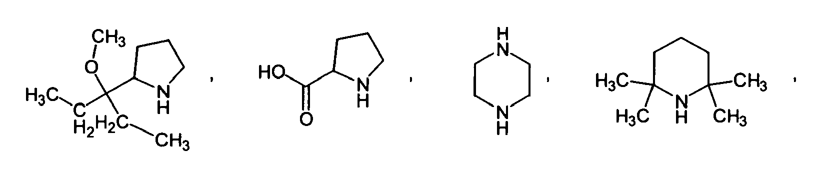 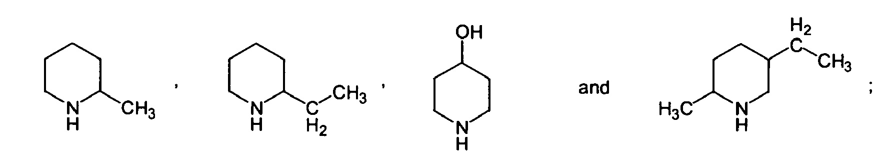 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a -O-R⁶ moiety or a -NR⁷R⁸-moiety ;
- R⁴: represents a hydrogen atom;
- R⁵: represents a hydrogen atom;
- R⁶: represents a hydrogen atom, a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;
- R⁷: represents a hydrogen atom;
- R⁸: a radical selected from the group consisting of cyclopropyl, cyclobutyl, cyclopentyl, cyclohexyl and cycloheptyl, which may be bonded via a -(CH₂)-,-(CH₂)-(CH₂)- or -(CH₂)-(CH₂)-(CH₂)-group;
or a radical selected from the group consisting of 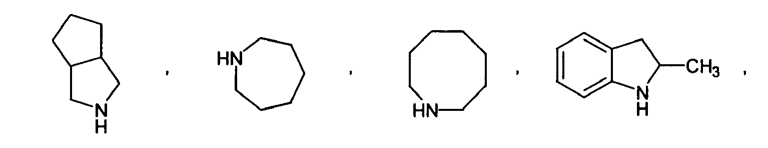 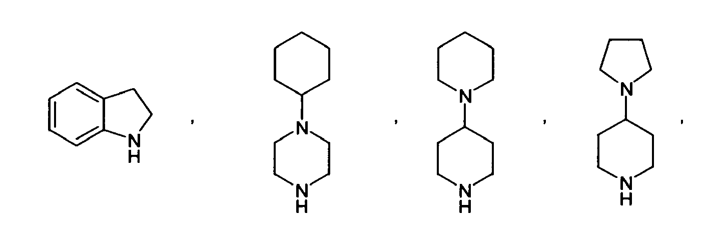 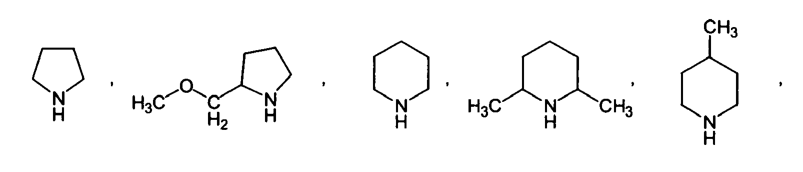 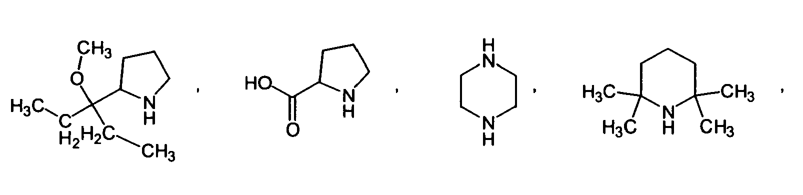 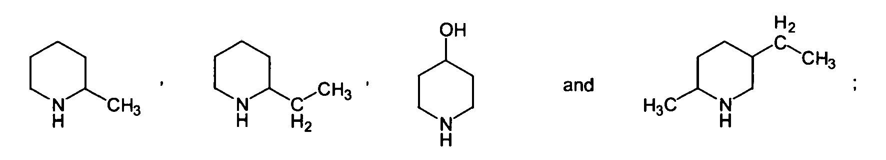 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment of the compound (A) of subgroup A3 the compound is selected from
- [1]: 5-(5-Chloro-thiophen-2-yl)-1-(2,4-dichloro-phenyl)-4,5-dihydro-1H-pyrazole-3-carboxylic acid piperidin-1-ylamide
- [2]: 5-(5-Chloro-thiophen-2-yl)-1-(2,4-dichloro-phenyl)-4,5-dihydro-1H-pyrazole-3-carboxylic acid azepan-1-ylamide
- [3]: 5-(5-Chloro-thiophen-2-yl)-1-(2,4-dichloro-phenyl)-4,5-dihydro-1H-pyrazole-3-carboxylic acid pyrrolidin-1-ylamide
- [4]: 5-(5-Chloro-thiophen-2-yl)-1-(2 ,4-dichloro-phenyl)-4,5-dihydro-1H-pyrazole-3-carboxylic acid cyclohexyl-amide
- [5]: [5-(5-Chloro-thiophen-2-yl)-1-(2,4-dichloro-phenyl)-4,5-dihydro-1H-pyrazol-3-yl]-pyrrolid in-1-yl-methanone
- [6]: [5-(5-Chloro-thiophen-2-yl)-1-(2,4-dichloro-phenyl)-4,5-dihydro-1H-pyrazol-3-yl]-(4-cyclohexyl-piperazin-1-yl)-methanone
- [7]: 5-(5-Chloro-thiophen-2-yl)-1-(2,4-dichloro-phenyl)-4,5-dihydro-1H-pyrazole-3-carboxylic acid cyclohexylamide
- [8]: 5-(5-Chloro-thiophen-2-yl)-1-(2,4-dichloro-phenyl)-4,5-dihydro-1H-pyra zole-3-carboxylic acid
- [9]: 5-(5-Chloro-thiophen-2-yl)-1-(2,4-dichloro-phenyl)-4,5-dihydro-1H-pyrazole-3-carboxylic acid methyl ester
- [10]: 5-(5-Chloro-thiophen-2-yl)-1-(2,4-dichloro-phenyl)-4,5-dihydro-1H-pyrazole-3-carboxylic acid ethyl ester
- [11]: 5-(5-Chloro-thiophen-2-yl)-1-(2,4-dichloro-phenyl)-4,5-dihydro-1H-pyrazole-3-carboxylic acid cyclohexyl ester
- [12]: 5-(3-Chloro-thiophen-2-yl)-1-(2,4-dichloro-phenyl)-4,5-dihydro-1H-pyrazole-3-carboxylic acid piperidin-1-ylamide
- [13]: 5-(5-Bromo-thiophen-2-yl)-1-(2,4-dichloro-phenyl)-4,5-dihydro-1H-pyrazole-3-carboxylic acid piperidin-1-ylamide
- [14]: 5-(5-Bromo-thiophen-2-yl)-1-(2,4-dichloro-phenyl)-4,5-dihydro-1H-pyrazole-3-carboxylic acid (4-methyl-piperidin-1-yl)-amide
- [15]: 5-(5-Bromo-thiophen-2-yl)-1-(2,4-dichloro-phenyl)-4,5-dihydro-1H-pyrazole-3-carboxylic acid (hexahydro-cyclopenta[c]pyrrol-2-yl)-amide
- [16]: 5-(5-Bromo-thiophen-2-yl)-1-(2,4-dichloro-phenyl)-4,5-dihydro-1H-pyrazole-3-carboxylic acid (2,3-dihydroindol-1-yl)-amide
- [17]: 5-(5-Bromo-thiophen-2-yl)-1-(2,4-dichloro-phenyl)-4,5-dihydro-1H-pyrazole-3-carboxylic acid cyclopropylamide
- [18]: 5-(5-Bromo-thiophen-2-yl)-1-(2,4-dichloro-phenyl)-4,5-dihydro-1H-pyrazole-3-carboxylic acid cyclohexylmethyl-amide
- [19]: 5-(5-Bromo-thiophen-2-yl)-1-(2,4-dichloro-phenyl)-4,5-dihydro-1H-pyrazole-3-carboxylic acid
- [20]: 5-(5-Bromo-thiophen-2-yl)-1-(2,4-dichloro-phenyl)-4,5-dihydro-1H-pyrazole-3-carboxylic acid cyclohexyl ester
- [21]: 5-(5-Bromo-thiophen-2-yl)-1-(2,4-dichloro-phenyl)-4,5-dihydro-1H-pyrazole-3-carboxylic acid methyl ester
- [22]: 5-(5-Bromo-thiophen-2-yl)-1-(2,4-dichloro-phenyl)-4,5-dihydro-1H-pyrazole-3-carboxylic acid ethyl ester
- [23]: 5-(4-Bromo-thiophen-2-yl)-1-(2,4-dichloro-phenyl)-4,5-dihydro-1H-pyrazole-3-carboxylic acid piperidin-1-ylamide hydrochloride
- [24]: 5-(4-Bromo-thiophen-2-yl)-1-(2,4-dichloro-phenyl)-4,5-dihydro-1H-pyrazole-3-carboxylic acid azepan-1-ylamide
- [25]: 5-(4-Bromo-thiophen-2-yl)-1-(2,4-dichloro-phenyl)-4,5-dihydro-1H-pyrazole-3-carboxylic acid (4-methyl-piperidin-1-yl)-amide
- [26]: [5-(4-Bromo-thiophen-2-yl)-1-(2,4-dichloro-phenyl)-4,5-dihydro-1H-pyrazol-3-yl]-piperidin-1-yl-methanone
- [27]: 5-(4-Bromo-thiophen-2-yl)-1-(2,4-dichloro-phenyl)-4,5-dihydro-1H-pyrazole-3-carboxylic acid
- [28]: 5-(4-Bromo-thiophen-2-yl)-1-(2,4-dichloro-phenyl)-4,5-dihydro-1H-pyrazole-3-carboxylic acid methyl ester
- [29]: 5-(4-Bromo-thiophen-2-yl)-1-(2,4-dichloro-phenyl)-4,5-dihydro-1H-pyrazole-3-carboxylic acid ethyl ester
- [30]: 5-(3-Bromo-thiophen-2-yl)-1-(2,4-dichloro-phenyl)-4,5-dihydro-1H-pyrazole-3-carboxylic acid piperidin-1-ylamide
- [31]: 5-(3-Bromo-5-methyl-thiophen-2-yl)-1-(2,4-dichloro-phenyl)-4,5-dihydro-1H-pyrazole-3-carboxylic acid piperidin-1-ylamide
- [32]: 5-(4-Bromo-3-methyl-thiophen-2-yl)-1-(2,4-dichloro-phenyl)-4,5-dihydro-1H-pyrazole-3-carboxylic acid piperidin-1-ylamide
- [33]: 5-(4,5-Dibromo-thiophen-2-yl)-1-(2,4-dichloro-phenyl)-4,5-dihydro-1H-pyrazole-3-carboxylic acid piperidin-1-ylamide
- [34]: 5-(3,5-Dibromo-thiophen-2-yl)-1-(2,4-dichloro-phenyl)-4,5-dihydro-1H-pyrazole-3-carboxylic acid piperidin-1-ylamide
- [35]: 1-(2,4-Dichloro-phenyl)-5-(3-iodo-thiophen-2-yl)-4,5-dihydro-1H-pyrazole-3-carboxylic acid piperidin-1-ylamide
- [36]: 5-(4-Chloromethyl-thiophen-2-yl)-1-(2,4-dichloro-phenyl)-4,5-dihydro-1H-pyrazole-3-carboxylic acid piperidin-1-ylamide
- [37]: 1-(2,4-Dichloro-phenyl)-5-(3,4-dimethyl-thiophen-2-yl)-4,5-dihydro-1H-pyrazole-3-carboxylic acid piperidin-1-ylamide
- [38]: 1-(2,4-Dichloro-phenyl)-5-(4,5-dimethyl-thiophen-2-yl)-4,5-dihydro-1H-pyrazole-3-carboxylic acid piperidin-1-ylamide
- [39]: 5-[2,2']Bithiophenyl-5-yl-1-(2,4-dichloro-phenyl)-4,5-dihydro-1H-pyrazole-3-carboxylic acid piperidin-1-ylamide
- [40]: 1-(2,4-Dichloro-phenyl)-5-(5-methyl-thiophen-2-yl)-4,5-dihydro-1H-pyrazole-3-carboxylic acid piperidin-1-ylamide
- [41]: 1-(2,4-Dichloro-phenyl)-5-(3-methyl-thiophen-2-yl)-4,5-dihydro-1H-pyrazole-3-carboxylic acid piperidin-1-ylamide
- [42]: 1-(2,4-Dichloro-phenyl)-5-(5-ethyl-thiophen-2-yl)-4,5-dihydro-1H-pyrazole-3-carboxylic acid piperidin-1-ylamide
- [43]: 1-(2,4-Dichloro-phenyl)-5-(3,3'-dimethyl-[2,2']bithiophenyl-5-yl)-4,5-dihydro-1H-pyrazole-3-carboxylic acid piperidin-1-ylamide
- [44]: 1-(2,4-Dichloro-phenyl)-5-(5-nitro-thiophen-2-yl)-4,5-dihydro-1H-pyrazole-3-carboxylic acid piperidin-1-ylamide
- [45]: 1-(2,4-Dichloro-phenyl)-5-(4-nitro-thiophen-2-yl)-4,5-dihydro-1H-pyrazole-3-carboxylic acid piperidin-1-ylamide
- [46]: 1-(2,4-Dichloro-phenyl)-5-thiophen-2-yl-4,5-dihydro-1H-pyrazole-3-carboxylic acid piperidin-1-ylamide
- [47]: 1-(2,4-Dichloro-phenyl)-5-thiophen-3-yl-4,5-dihydro-1H-pyrazole-3-carboxylic acid piperidin-1-ylamide
and
- [48]: 1-(2,4-Dichloro-phenyl)-5-(5-nitro-thiophen-3-yl)-4,5-dihydro-1H-pyrazole-3-carboxylic acid piperidin-1-ylamide;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the active substance combination according to the present invention comprises as compound (A) of subgroup A4 of general formula I given above, wherein
- X: represents S;
- R¹: represents a phenyl radical which may be substituted with 1, 2, 3, 4 or 5 substituent(s) independently selected from the group consisting of F, Cl, Br, I, -O-CH₃ and -O-C₂H₅;
- R²: represents a phenyl radical which may be substituted with 1, 2, 3, 4 or 5 substituent(s) independently selected from the group consisting of F, Cl, Br and I;
- R³: represents a radical selected from the group consisting of 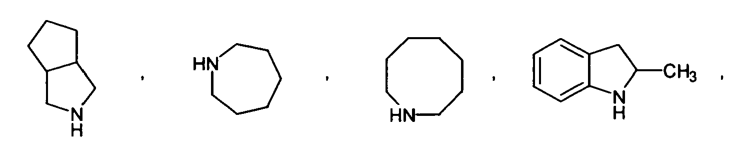 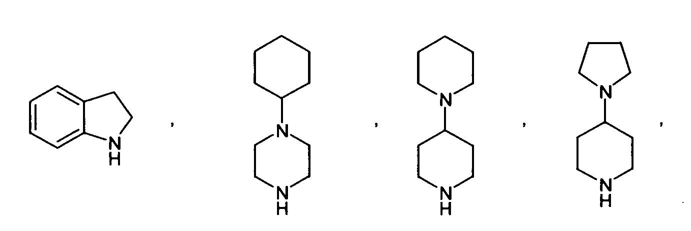 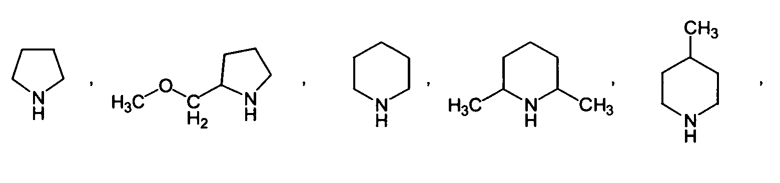 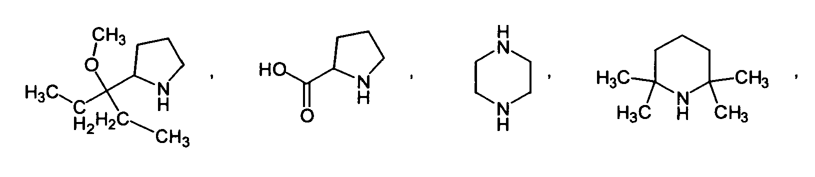 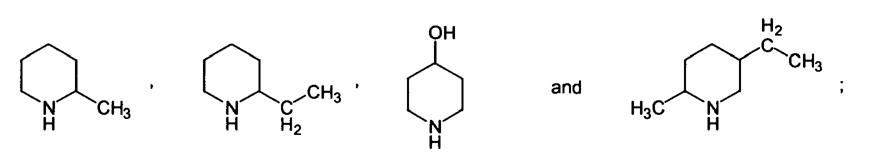
which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a -O-R⁶ moiety or a -NR⁷R⁸-moiety;
- R⁴: represents a hydrogen atom;
- R⁵: represents a hydrogen atom;
- R⁶: represents a hydrogen atom or a radical selected from the group consisting of cyclopropyl, cyclobutyl, cyclopentyl, cyclohexyl and cycloheptyl;
- R⁷: represents a hydrogen atom;
- R⁸: a radical selected from the group consisting of cyclopropyl, cyclobutyl, cyclopentyl, cyclohexyl and cycloheptyl, which may be bonded via a -(CH₂)-,-(CH₂)-(CH₂)- or -(CH₂)-(CH₂)-(CH₂)-group;
or a radical selected from the group consisting of 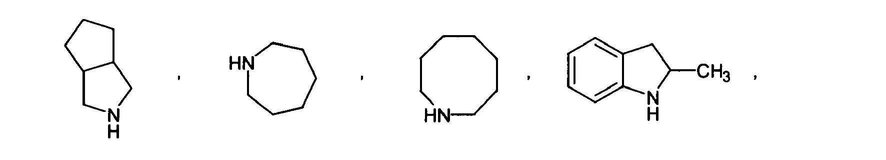 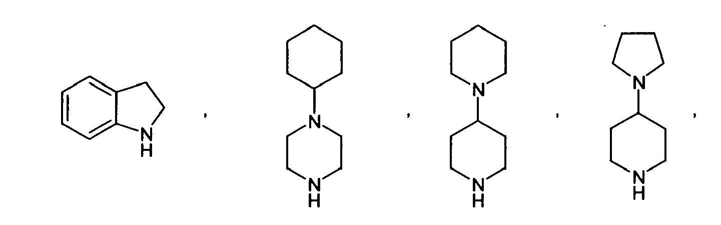 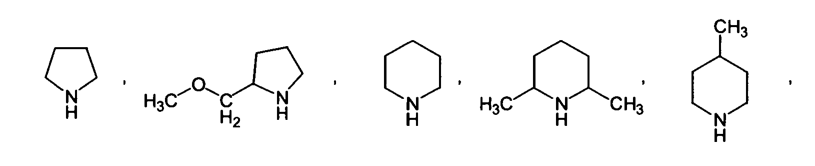 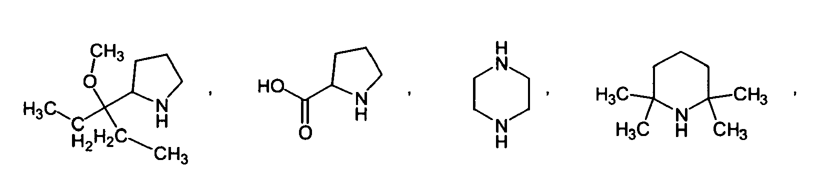 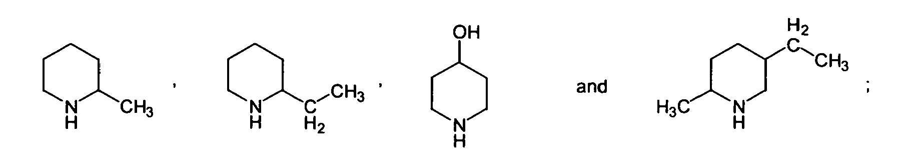 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the compound (A) of subgroup A4 is selected from:
- [1]: 5-(4-Chloro-phenyl)-1-(2,4-dichloro-phenyl)-4,5-dihydro-1H-pyrazole-3-carbothioic acid piperidin-1-ylamide
- [2]: 5-(4-Bromo-phenyl)-1-(2,4-dichloro-phenyl)-4,5-dihydro-1H-pyrazole-3-carbothioic acid piperidin-1-ylamide
- [3]: 1-(2,4-Dichloro-phenyl)-5-(4-iodo-phenyl)-4,5-dihydro-1H-pyrazole-3-carbothioic acid piperidin-1-ylamide
- [4]: 1-(2,4-Dichloro-phenyl)-5-(4-fluoro-phenyl)-4,5-dihydro-1H-pyrazole-3-carbothioic acid piperidin-1-ylamide
- [5]: 1-(2 ,4-Dichloro-phenyl)-5-(4-methoxy)-4,5-dihydro-1H-pyrazole-3-carbothioic acid piperidin-1-ylamide
- [6]: 5-(4-Chloro-phenyl)-1-(2,4-dichloro-phenyl)-4,5-dihydro-1H-pyrazole-3-carbothioic acid morpholin-4-ylamide
- [7]: 5-(4-Bromo-phenyl)-1-(2,4-dichloro-phenyl)-4,5-dihydro-1H-pyrazole-3-carbothioic acid morpholin-4-ylamide
- [8]: 1-(2,4-Dichloro-phenyl)-5-(4-iodo-phenyl)-4,5-dihydro-1H-pyrazole-3-carbothioic acid morpholin-4-ylamide
- [9]: 1-(2,4-Dichloro-phenyl)-5-(4-fluoro-phenyl)-4,5-dihydro-1H-pyrazole-3-carbothioic acid morpholin-4-ylamide
- [10]: 1-(2,4-Dichloro-phenyl)-5-(4-methoxy-phenyl)-4,5-dihydro-1H-pyrazole-3-carbothioic acid morpholin-4-ylamide
- [11]: 5-(4-Chloro-phenyl)-1-(2,4-dichloro-phenyl)-4,5-dihydro-1H-pyrazole-3-carbothioic acid (hexahydro-cyclopenta[c]pyrrol-2-yl)-amide
- [12]: 5-(4-Bromo-phenyl)-1-(2,4-dichloro-phenyl)-4,5-dihydro-1 H-pyrazole-3-carbothioic acid (hexahydro-cyclopenta[c]pyrrol-2-yl)-amide
- [13]: 1-(2,4-Dichloro-phenyl)-5-(4-fluoro-phenyl)-4,5-dihydro-1 H-pyrazole-3-carbothioic acid (hexahydro-cyclopenta[c]pyrrol-2-yl)-amide
- [14]: 1-(2,4-Dichloro-phenyl)-5-(4-iodo-phenyl)-4,5-dihydro-1H-pyrazole-3-carbothioic acid (hexahydro-cyclopenta[c]pyrrol-2-yl)-amide
- [15]: 1-(2,4-Dichloro-phenyl)-5-(4-methoxy-phenyl)-4,5-dihydro-1H-pyrazole-3-carbothioic acid (hexahydro-cyclopenta[c]pyrrol-2-yl)-amide
- [16]: 1-(2,4-Dichloro-phenyl)-5-(4-iodo-phenyl)-4,5-dihydro-1H-pyrazole-3-carbothioic acid azepan-1-ylamide
- [17]: 5-(4-Chloro-phenyl)-1-(2,4-dichloro-phenyl)-4,5-dihydro-1H-pyrazole-3-carbothioic acid azepan-1-ylamide
- [18]: 5-(4-Bromo-phenyl)-1-(2,4-dichloro-phenyl)-4,5-dihydro-1H-pyrazole-3-carbothioic acid azepan-1-ylamide
- [19]: 1-(2,4-Dichloro-phenyil)-5-(4-fluoro-phenyl)-4,5-dihydro-1H-pyrazole-3-carbothioic acid azepan-1-ylamide
- [20]: 1-(2,4-Dichloro-phenyl)-5-(4-methoxy-phenyl)-4,5-dihydro-1H-pyrazole-3-carbothioic acid azepan-1-ylamide
- [21]: 1-(2,4-Dichloro-phenyl)-5-(4-methoxy-phenyl)-4,5-dihydro-1H-pyrazole-3-carbothioic acid pyrrolidin-1-ylamide
- [22]: 5-(4-Bromo-phenyl)-1-(2,4-dichloro-phenyl)-4,5-dihydro-1H-pyrazole-3-carbothioic acid pyrrolidin-1-ylamide
- [23]: 5-(4-Chloro-phenyl)-1-(2,4-dichloro-phenyl)-4,5-dihydro-1H-pyrazole-3-carbothioic acid pyrrolidin-1-ylamide
- [24]: 1-(2,4-Dichloro-phenyl)-5-(4-fluoro-phenyl)-4,5-dihydro-1H-pyrazole-3-carbothioic acid pyrrolidin-1-ylamide
- [25]: 1-(2,4-Dichloro-phenyl)-5-(4-iodo-phenyl)-4,5-dihydro-1H-pyrazole-3-carbothioic acid pyrrolidin-1-ylamide
- [26]: 1-(2,4-Dichloro-phenyl)-5-(4-methoxy-phenyl)-4,5-dihydro-1H-pyrazole-3-carbothioic acid (4-cyclopentyl-piperazin-1-yl)-amide
- [27]: 5-(4-Chloro-phenyl)-1-(2,4-dichloro-phenyl)-4,5-dihydro-1H-pyrazole-3-carbothioic acid (4-cyclopentyl-piperazin-1-yl)-amide
- [28]: 5-(4-Bromo-phenyl)-1-(2,4-dichloro-phenyl)-4,5-dihydro-1H-pyrazole-3-carbothioic acid (1 H,3H-benzo[de]isoquinolin-2-yl)-amide
- [29]: 5-(4-Chloro-phenyl)-1-(2,4-dichloro-phenyl)-4,5-dihydro-1 H-pyrazole-3-carbothioic acid (1 H,3H-benzo[de]isoquinolin-2-yl)-amide
- [30]: 5-(4-Chloro-phenyl)-1-(2,4-dichloro-phenyl)-4,5-dihydro-1H-pyrazole-3-carbothioic acid cyclopentylamide
- [31]: Azocan-1-yl-[5-(4-chloro-phenyl)-1-(2,4-dichloro-phenyl)-4,5-dihydro-1H-pyrazole-3-yl]-methanethione
and
- [32]: [5-(4-Chloro-phenylyl-(2,4-dichloro-phenyl)-4,5-dihydro-1H-pyrazole-3-yl]-piperidin-1-yl-methanethione;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the active substance combination according to the present invention comprises as compound (A) of subgroup A5.1 of general formula I given above, wherein
- X: represents O;
- R¹: represents a phenyl radical that is substituted with a hydroxy, fluorine, chlorine, bromine or iodine atom or a -O-CH₃-group in the para-(4-)-position of the phenyl radical;
- R²: represents a (2,4)-dichloro-phenyl radical;
- R³: represents a radical selected from the group consisting of 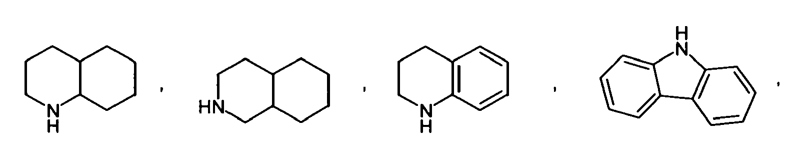 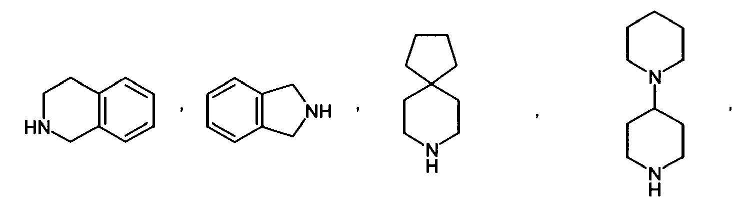 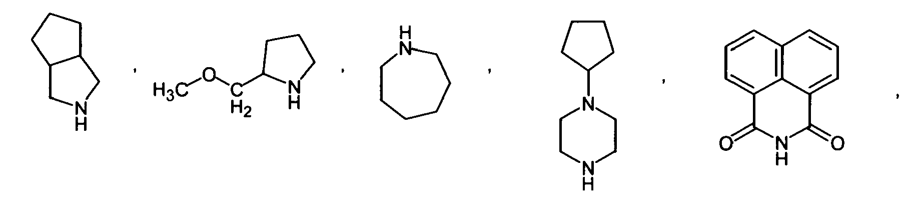 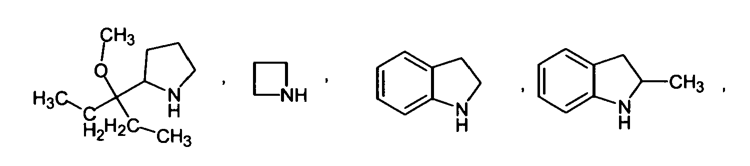 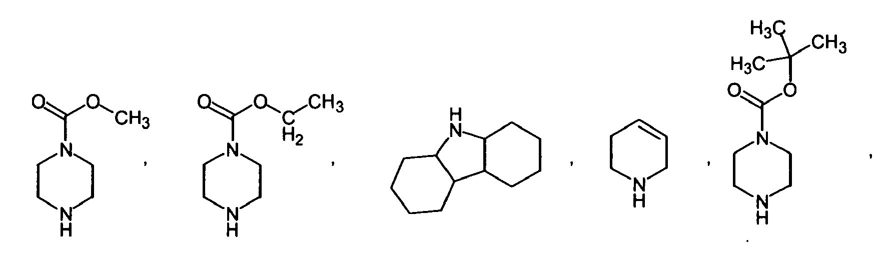 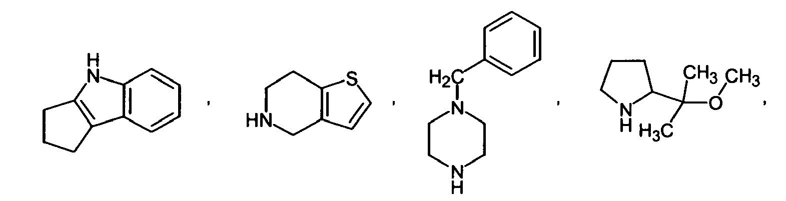 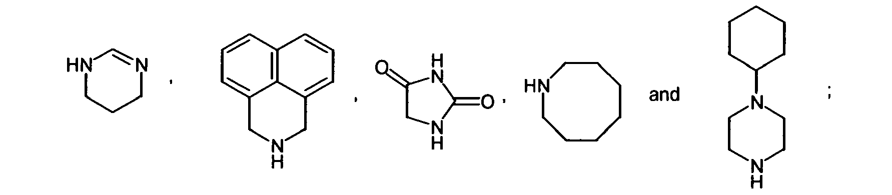
which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
or a -NR⁷R⁸ moiety;
- R⁴: represents a hydrogen atom;
- R⁵: represents a hydrogen atom;
- R⁷: represents a hydrogen atom;
- R⁸: represents a radical selected from the group consisting of 2-heptyl, 3-heptyl, 4-heptyl, 2-octyl, 3-octyl, 4-octyl, -CH₂-N(CH₃)₂, -CH₂-N(C₂H₅)₂, -CH₂-CH₂-N(CH₃)₂,-CH₂-CH₂-N(C₂H₅)₂, -CH₂-CH₂-CH₂-N(CH₃)₂ and -CH₂-CH₂-CH₂-N(C₂H₅)₂;
a radical selected from the group consisting of adamantyl, bicyclo[2.2.1]heptyl, bicyclo[3.1.1]heptyl, (1,7,7)-trimethyl-bicyclo[2.2.1]heptyl, [1,2,3,4]-tetrahydronaphthyl, cyclononyl, cyclodecyl, cycloundecyl and cyclododecyl;
a radical selected from the group consisting of cyclopropyl, cyclobutyl, cyclopentyl, cyclohexyl, cycloheptyl, cyclooctyl, pyrrolidinyl, piperidinyl, piperazinyl, homopiperazinyl, morpholinyl, azepanyl, diazepanyl and azocanyl, which is bonded via a -(CH₂)-, -(CH₂)-(CH₂)- or -(CH₂)-(CH₂)-(CH₂)-group;
a substituted radical selected from the group consisting of cyclopentyl, cyclohexyl and cyloheptyl which is substituted with a -O-Benzyl radical;
or a radical selected from the group consisting of 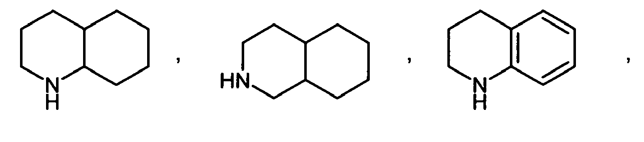 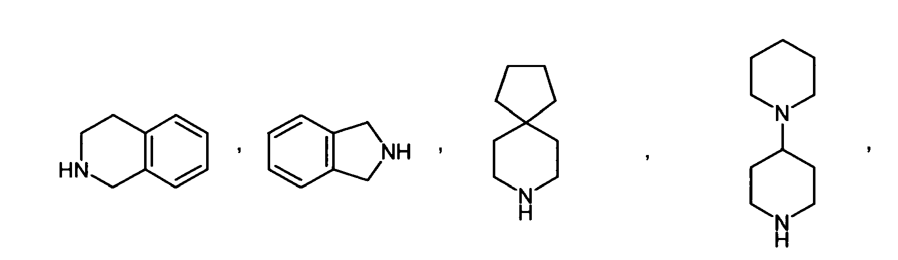 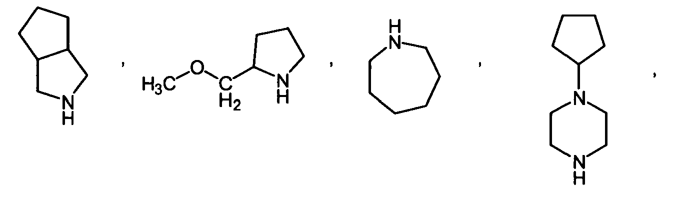 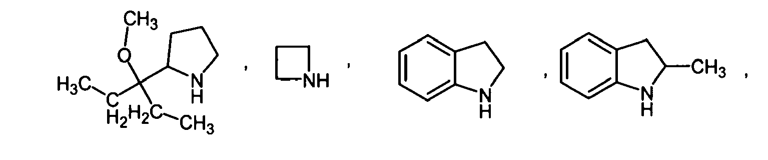 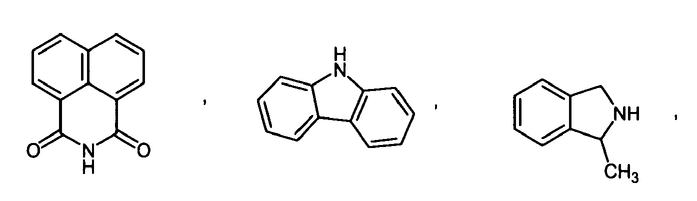 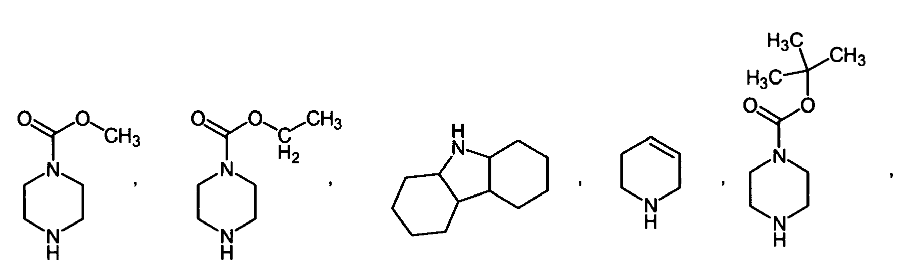 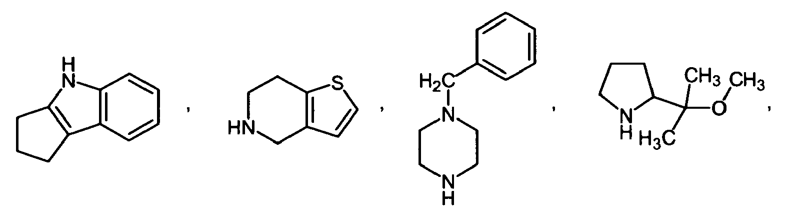 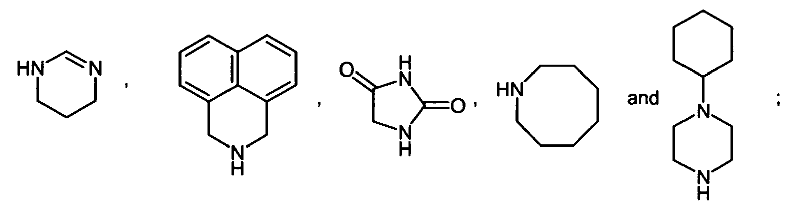 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.
acceptable salt thereof, or a corresponding solvate thereof.

As a preferred embodiment the compound (A) of subgroup A5.1 is selected from:
- [10]: 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide
- [13]: 5-(4-Chloro-phenyl)-1-(2,4-dichloro-phenyl)-4,5-dihydro-1H-pyrazole-3-carboxylic acid [2-(S)-(1-methoxy-1-methyl-ethyl)-pyrrolidin-1-yl]-amide
- [14]: 5-(4-Chloro-phenyl)-1-(2,4-dichloro-phenyl)-4,5-dihydro-1H-pyrazole-3-carboxylic acid [2-(R)-(1-methoxy-1-methyl-ethyl)-pyrrolidin-1-yl]-amide
- [15]: 5-(4-Chloro-phenyl)-1-(2,4-dichloro-phenyl)-4,5-dihydro-1H-pyrazole-3-carboxylic acid [2-(1-methoxy-1-methyl-ethyl)-pyrrolidin-1-yl]-amide
- [16]: 5-(4-Chloro-phenyl)-1-(2,4-dichloro-phenyl)-4,5-dihydro-1H-pyrazole-3-carboxylic acid azepan-1-ylamide
- [21]: [5-(4-Chloro-phenyl)-1-(2,4-dichloro-phenyl)-4,5-dihydro-1H-pyrazole-3-yl]-(4-cyclohexyl-piperazin-1-yl)-methanone
- [22]: [5-(4-Chloro-phenyl)-1-(2,4-dichloro-phenyl)-4,5-dihydro-1H-pyrazole-3-yl]-(octahydro-isoquinolin-2-yl)-methanone
- [24]: 5-(4-Chloro-phenyl)-1-(2,4-dichloro-phenyl)-4,5-dihydro-1H-pyrazole-3-carboxylic acid (1,3-dioxo-1H,3H-benzo[de]isoquinolin-2-yl)-amide
- [25]: 5-(4-Chloro-phenyl)-1-(2,4-dichloro-phenyl)-4,5-dihydro-1H-pyrazole-3-carboxylic acid (1 H,3H-benzo[de]isoquinolin-2-yl)-amide
- [26]: 5-(4-Chloro-phenyl)-1-(2,4-dichloro-phenyl)-4,5-dihydro-1H-pyrazole-3-carboxylic acid (4-cyclopentyl-piperazin-1-yl)-amide
- [27]: 5-(4-Chloro-phenyl)-1-(2,4-dichloro-phenyl)-4,5-dihydro-1H-pyrazole-3-carboxylic acid (2-methyl-2,3-dihydro-indol-1-yl)-amide
- [28]: 5-(4-Chloro-phenyl)-1-(2,4-dichloro-phenyl)-4,5-dihydro-1H-pyrazole-3-carboxylic acid (2,3-dihydro-indol-1-yl)-amide
- [32]: 5-(4-Chloro-phenyl)-1-(2,4-dichloro-phenyl)-4,5-dihydro-1H-pyrazole-3-carboxylic acid cyclohexylmethyl-amide
- [35]: 5-(4-Chloro-phenyl)-1-(2,4-dichloro-phenyl)-4,5-dihydro-1H-pyrazole-3-carboxylic acid (1-methyl-hexyl)-amide
- [38]: Azocan-1-yl-[5-(4-chloro-phenyl)-1-(2,4-dichloro-phenyl)-4,5-dihydro-1H-pyrazol-3-yl]-methanone
- [39]: [5-(4-Chloro-phenyl)-1-(2,4-dichloro-phenyl)-4,5-dihydro-1H-pyrazole-3-yl]-(1,3-dihydro-isoindol-2-yl)-methanone
- [40]: Azetidin-1-yl-[5-(4-chloro-phenyl)-1-(2,4-dichloro-phenyl)-4,5-dihydro-1H-pyrazol-3-yl]-methanone
- [42]: [1,4']Bipiperidin-1'-yl-[5-(4-chloro-phenyl)-1-(2,4-dichloro-phenyl)-4,5-dihydro-1H-pyrazol-3-yl]-methanone
- [44]: 4-[5-(4-Chloro-phenyl)-1-(2,4-dichloro-phenyl)-4,5-dihydro-1H-pyrazole-3-carbonyl]-piperazine-1-carboxylic acid ethyl ester
- [45]: [5-(4-Chloro-phenyl)-1-(2,4-dichloro-phenyl)-4,5-dihydro-1H-pyrazole-3-yl]-(3,4-dihydro-1H-isoquinolin-2-yl)-methanone
- [48]: 5-(4-Chloro-phenyl)-1-(2,4-dichloro-phenyl)-4,5-dihydro-1H-pyrazole-3-carboxylic acid (2,4-dioxo-imidazolidin-1-yl)-amide
- [49]: 5-(4-Chloro-phenyl)-1-(2,4-dichloro-phenyl)-4,5-dihydro-1H-pyrazole-3-carboxylic acid cyclododecylamide
- [52]: (4-Benzyl-piperazin-1-yl)-[5-(4-chloro-phenyl)-1-(2,4-dichloro-phenyl)-4,5-dihydro-1H-pyrazol-3-yl]-methanone
- [56]: [5-(4-Chloro-phenyl)-1-(2,4-dichloro-phenyl)-4,5-dihydro-1 H-pyrazole-3-yl]-(6,7-dihydro-4H-thieno[3,2-c]pyridin-5-yl)-methanone
- [58]: 5-(4-Chloro-phenyl)-1-(2,4-dichloro-phenyl)-4,5-dihydro-1H-pyrazole-3-carboxylic acid (1,7,7-trimethyl-bicyclo[2.2.1]hept-2-yl)-amide
- [63]: 5-(4-Bromo-phenyl)-1-(2,4-dichloro-phenyl)-4,5-dihydro-1H-pyrazole-3-carboxylic acid (hexahydro-cyclopenta[c]pyrrol-2-yl)-amide
- [64]: 5-(4-Bromo-phenyl)-1-(2,4-dichloro-phenyl)-4,5-dihydro-1H-pyrazole-3-carboxylic acid (4-cyclopentyl-piperazin-1-yl)-amide
- [65]: 5-(4-Bromo-phenyl)-1-(2,4-dichloro-phenyl)-4,5-dihydro-1 H-pyrazole-3-carboxylic acid (1H,3H-benzo[de]isoquinolin-2-yl)-amide
- [66]: 5-(4-Bromo-phenyl)-1-(2,4-dichloro-phenyl)-4,5-dihydro-1H-pyrazole-3-carboxylic acid (2,3-dihydro-indol-1-yl)-amide hydrochloride
- [68]: 5-(4-Bromo-phenyl)-1-(2,4-dichloro-phenyl)-4,5-dihydro-1H-pyrazole-3-carboxylic acid azepan-1-yl-amide
- [71]: 5-(4-Chloro-phenyl)-1-(2,4-dichloro-phenyl)-4,5-dihydro-1H-pyrazole-3-carboxylic acid (2-morpholin-4-yl-ethyl)-amide
- [73]: 5-(4-Chloro-phenyl)-1-(2,4-dichloro-phenyl)-4,5-dihydro-1H-pyrazole-3-carboxylic acid (3-dimethylamino-propyl)-amide
- [74]: [5-(4-Chloro-phenyl)-1-(2,4-dichloro-phenyl)-4,5-dihydro-1H-pyrazole-3-yl]-(3,6-dihydro-2H-pyridin-1-yl)-methanone
- [75]: [5-(4-Chloro-phenyl)-1-(2,4-dichloro-phenyl)-4,5-dihydro-1H-pyrazole-3-yl]-(5,6-dihydro-4H-pyrimidin-1-yl)-methanone
- [76]: 5-(4-Chloro-phenyl)-1-(2,4-dichloro-phenyl)-4,5-dihydro-1H-pyrazole-3-carboxylic acid (1,2,3,4-tetrahydro-naphthalen-1-yl)-amide
- [78]: [5-(4-Chloro-phenyl)-1-(2,4-dichloro-phenyl)-4,5-dihydro-1H-pyrazole-3-yl]-(2,3-dihydro-1H-cyclopenta[b]indol-4-yl)-methanone
- [81]: [5-(4-Chloro-phenyl)-1-(2,4-dichloro-phenyl)-4,5-dihydro-1H-pyrazole-3-yl]-(3,4-dihydro-2H-quinolin-1-yl)-methanone
- [84]: 5-(4-Chloro-phenyl)-1-(2,4-dichloro-phenyl)-4,5-dihydro-1H-pyrazole-3-carboxylic acid (2-cyclohexyl-ethyl)-amide
- [85]: [5-(4-Chloro-phenyl)-1-(2,4-dichloro-phenyl)-4,5-dihydro-1H-pyrazole-3-yl]-(dodecahydro-carbazol-9-yl)-methanone
- [87]: 5-(4-Chloro-phenyl)-1-(2,4-dichloro-phenyl)-4,5-dihydro-1H-pyrazole-3-carboxylic acid (2-cyclopentyl-ethyl)-amide
- [88]: 5-(4-Chloro-phenyl)-1-(2,4-dichloro-phenyl)-4,5-dihydro-1H-pyrazole-3-carboxylic acid bicyclo[2.2.1]hept-2-ylamide
- [91]: 1-(2,4-Dichloro-phenyl)-5-(4-fluoro-phenyl)-4,5-dihydro-1H-pyrazole-3-carboxylic acid (hexahydro-cyclopenta[c]pyrrol-2-yl)-amide hydrochloride
- [92]: 1-(2,4-Dichloro-phenyl)-5-(4-fluoro-phenyl)-4,5-dihydro-1H-pyrazole-3-carboxylic acid (4-cyclopentyl-piperazin-1-yl)-amide
- [93]: 1-(2,4-Dichloro-phenyl)-5-(4-fluoro-phenyl)-4,5-dihydro-1H-pyrazole-3-carboxylic acid (1 H,3H-benzo[de]isoquinolin-2-yl)-amide
- [94]: 1-(2,4-Dichloro-phenyl)-5-(4-fluoro-phenyl)-4,5-dihydro-1H-pyrazole-3-carboxylic acid (2,3-dihydro-indol-1-yl)-amide
- [96]: 1-(2,4-Dichloro-phenyl)-5-(4-fluoro-phenyl)-4,5-dihydro-1H-pyrazole-3-carboxylic acid azepan-1-yl-amide
- [100]: 1-(2,4-Dichloro-phenyl)-5-(4-iodo-phenyl)-4,5-dihydro-1H-pyrazole-3-carboxylic acid (hexahydro-cyclopenta[c]pyrrol-2-yl)-amide
- [101]: 1-(2,4-Dichloro-phenyl)-5-(4-iodo-phenyl)-4,5-dihydro-1H-pyrazole-3-carboxylic acid (4-cyclopentyl-piperazin-1-yl)-amide
- [102]: 1-(2,4-Dichloro-phenyl)-5-(4-iodo-phenyl)-4,5-dihydro-1H-pyrazole-3-carboxylic acid (1H,3H-benzo[de]isoquinolin-2-yl)-amide
- [103]: 1-(2,4-Dichloro-phenyl)-5-(4-iodo-phenyl)-4,5-dihydro-1H-pyrazole-3-carboxylic acid (2,3-dihydro-indol-1-yl)-amide
- [105]: 1-(2,4-Dichloro-phenyl)-5-(4-iodo-phenyl)-4,5-dihydro-1H-pyrazole-3-carboxylic acid azepan-1-ylamide;
- [109]: 1-(2,4-Dichloro-phenyl)-5-(4-methoxy-phenyl)-4,5-dihydro-1H-pyrazole-3-carboxylic acid (hexahydro-cyclopenta[c]pyrrol-2-yl)-amide hydrochloride
- [110]: 1-(2,4-Dichloro-phenyl)-5-(4-methoxy-phenyl)-4,5-dihydro-1H-pyrazole-3-carboxylic acid (4-cyclopentyl-piperazin-1-yl)-amide
- [111]: 1-(2,4-Dichloro-phenyl)-5-(4-methoxy-phenyl)-4,5-dihydro-1H-pyrazole-3-carboxylic acid (1H,3H-benzo[de]isoquinolin-2-yl)-amide
- [112]: 1-(2,4-Dichloro-phenyl)-5-(4-methoxy-phenyl)-4,5-dihydro-1H-pyrazole-3-carboxylic acid (2,3-dihydro-indol-1-yl)-amide hydrochloride
- [114]: 1-(2,4-Dichloro-phenyl)-5-(4-methoxy-phenyl)-4,5-dihydro-1H-pyrazole-3-carboxylic acid azepan-1-ylamide hydrochloride
- [145]: 5-(4-Chloro-phenyl)-1-(2,4-dichloro-phenyl)-4,5-dihydro-1H-pyrazole-3-carboxylic acid azocan-1-ylamide hydrochloride
- [146]: 5-(4-bromo-phenyl)-1-(2,4-dichloro-phenyl)-4,5-dihydro-1H-pyrazole-3-carboxylic acid azocan-1-ylamide
- [147]: 5-(4-fluoro-phenyl)-1-(2,4-dichloro-phenyl)-4,5-dihydro-1H-pyrazole-3-carboxylic acid azocan-1-ylamide
- [148]: 5-(4-methoxy-phenyl)-1-(2,4-dichloro-phenyl)-4,5-dihydro-1 H-pyrazole-3-carboxylic acid azocan-1-ylamide
- [149]: 5-(4-hydroxy-phenyl)-1-(2,4-dichloro-phenyl)-4,5-dihydro-1 H-pyrazole-3-carboxylic acid azocan-1-ylamide hydrochloride
- [150]: 5-(4-iodo-phenyl)-1-(2,4-dichloro-phenyl)-4,5-dihydro-1H-pyrazole-3-carboxylic acid azocan-1-ylamide
- [151]: N-((1S,2S)-2-(benzyloxy)cyclohexyl)-5-(4-chlorophenyl)-1-(2,4-dichlorophenyl)-4,5-dihydro-1H-pyrazole-3-carboxamide
- [158]: 5-(4-Chloro-phenyl)-1-(2,4-dichloro-phenyl)-4,5-dihydro-1H-pyrazole-3-carboxylic acid adamantan-1-ylamide
- [159]: (R)-5-(4-Chloro-phenyl)-1-(2,4-dichloro-phenyl)-4,5-dihydro-1H-pyrazole-3-carboxylic acid adamantan-1-ylamide
- [160]: (S)-5-(4-Chloro-phenyl)-1-(2,4-dichloro-phenyl)-4,5-dihydro-1H-pyrazole-3-carboxylic acid adamantan-1-ylamide
- [161]: 5-(4-bromo-phenyl)-1-(2,4-dichloro-phenyl)-4,5-dihydro-1H-pyrazole-3-carboxylic acid adamantan-1-ylamide
- [162]: 5-(4-fluoro-phenyl)-1-(2,4-dichloro-phenyl)-4,5-dihydro-1H-pyrazole-3-carboxylic acid adamantan-1-ylamide
- [163]: 5-(4-methoxy-phenyl)-1-(2,4-dichloro-phenyl)-4,5-dihydro-1H-pyrazole-3-carboxylic acid adamantan-1-ylamide
- [164]: 5-(4-hydroxy-phenyl)-1-(2,4-dichloro-phenyl)-4,5-dihydro-1H-pyrazole-3-carboxylic acid adamantan-1-ylamide
- [165]: 5-(4-iodo-phenyl)-1-(2,4-dichloro-phenyl)-4,5-dihydro-1H-pyrazole-3-carboxylic acid adamantan-1-ylamide
- [166]: 5-(4-Chloro-phenyl)-1-(2,4-dichloro-phenyl)-4,5-dihydro-1H-pyrazole-3-carboxylic acid adamantan-2-ylamide
- [167]: 5-(4-bromo-phenyl)-1-(2,4-dichloro-phenyl)-4,5-dihydro-1H-pyrazole-3-carboxylic acid adamantan-2-ylamide
- [168]: 5-(4-fluoro-phenyl)-1-(2,4-dichbro-phenyl)-4,5-dihydro-1H-pyrazole-3-carboxylic acid adamantan-2-ylamide
- [169]: 5-(4-methoxy-phenyl)-1-(2,4-dichloro-phenyl)-4,5-dihydro-1H-pyrazole-3-carboxylic acid adamantan-2-ylamide
- [170]: 5-(4-hydroxy-phenyl)-1-(2,4-dichloro-phenyl)-4,5-dihydro-1H-pyrazole-3-carboxylic acid adamantan-2-ylamide
- [171]: 5-(4-iodo-phenyl)-1-(2,4-dichloro-phenyl)-4,5-dihydro-1H-pyrazole-3-carboxylic acid adamantan-2-ylamide
- [173]: 1-(2,4-Dichloro-phenyl)-5-(4-hydroxy-phenyl)-4,5-dihydro-1H-pyrazole-3 -carboxylic acid azepan-1-ylamide hydrochloride
- [174]: 1-(2,4-Dichloro-phenyl)-5-(4-hydroxy-phenyl)-4,5-dihydro-1 H-pyrazole-3 -carboxylic acid (hexahydro-cyclopenta[c]pyrrol-2-yl)-amide hydrochloride
- [175]: 1-(2,4-Dichloro-phenyl)-5-(4-hydroxy-phenyl)-4,5-dihydro-1H-pyrazole-3-carboxylic acid (2,3-dihydro-indol-1-yl)-amide hydrochloride
- [176]: (R)-5-(4-Chloro-phenyl)-1-(2,4-dichloro-phenyl)-4,5-dihydro-1 H-pyrazole-3-carboxylic acid azocan-1-ylamide hydrochloride
- [177]: (S)-5-(4-Chloro-phenyl)-1-(2,4-dichloro-phenyl)-4,5-dihydro-1 H-pyrazole-3-carboxylic acid azocan-1-ylamide hydrochloride,
- [178]: N-((1R,2R)-2-(benzyloxy)cyclohexyl)-5-(4-chlorophenyl)-1-(2,4-dichlorophenyl)-4,5-dihydro-1 H-pyrazole-3-carboxamide
- [179]: 1-(2,4-Dichloro-phenyl)-5-(4-fluoro-phenyl)-4,5-dihydro-1H-pyrazole-3-carboxylic acid (2,3-dihydro-indol-1-yl)-amide hydrochloride
- [181]: (R)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride
- [182]: (S)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride
- [183]: (S)-5-(4-Chloro-phenyl)-1-(2,4-dichloro-phenyl)-4,5-dihydro-1 H-pyrazole-3-carboxylic acid azepan-1-ylamide hydrochloride
- [184]: (S)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride
- [185]: (S)-5-(4-Chloro-phenyl)-1-(2,4-dichloro-phenyl)-4,5-dihydro-1H-pyrazole-3-carboxylic acid azepan-1-ylamide
- [187]: (R)-5-(4-Chloro-phenyl)-1-(2,4-dichloro-phenyl)-4.5-dihydro-1H-pyrazole-3-carboxylic acid azepan-1-ylamide hydrochloride
- [188]: (R)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide
- [190]: (R)-5-(4-Chloro-phenyl)-1-(2,4-dichloro-phenyl)-4,5-dihydro-1H-pyrazole-3-carboxylic acid (2,3-dihydro-indol-1-yl)-amide
- [191]: 5-(4-Chloro-phenyl)-1-(2,4-dichloro-phenyl)-4,5-dihydro-1H-pyrazole-3-carboxylic acid cycloheptylmethyl-amide
- [193]: 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the active substance combination according to the present invention comprises as compound (A) of subgroup A6.1 of general formula I given above, wherein
- X: is O or S;
- R¹: represents a radical selected from the group consisting of phenyl and thienyl (thiophenyl), which may optionally be substituted with 1, 2, 3, 4 or 5 substituent(s) independently selected from the group consisting of -OH, -O-CH₃, -O-C₂H₅, F, Cl, Br and I;
- R²: represents a phenyl radical, which may be substituted with 1, 2, 3, 4 or 5 substituent(s) independently selected from the group consisting of -OH, -O-CH₃, -O-C₂H₅, F, Cl, Br and I;
- R³: represents a radical selected from the group consisting of 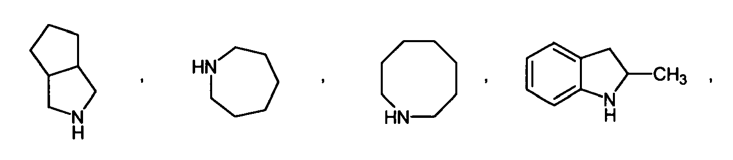 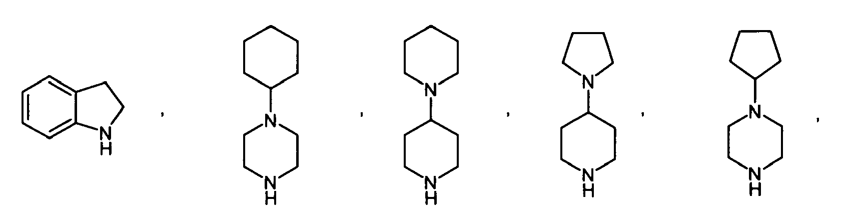 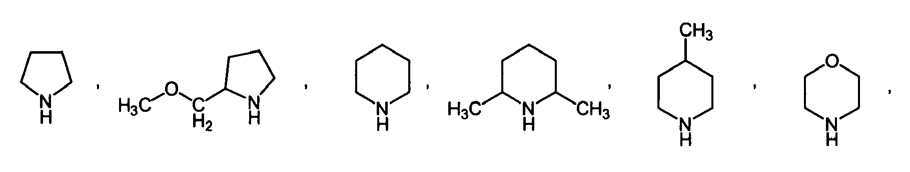 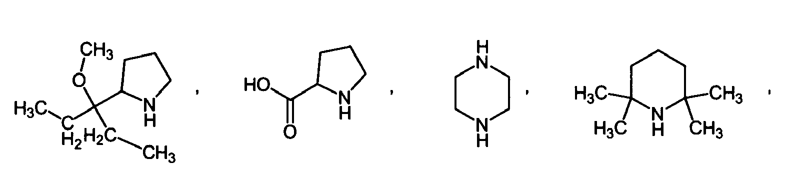 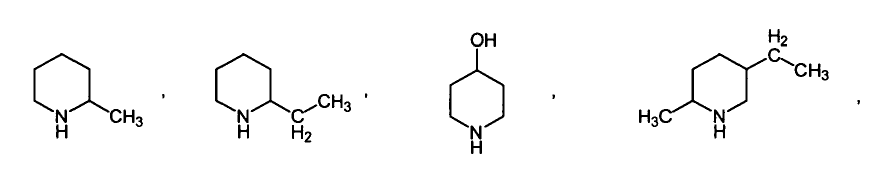 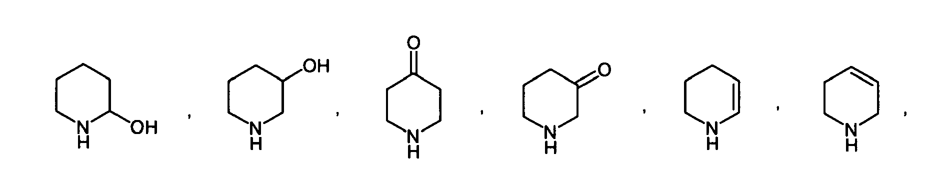 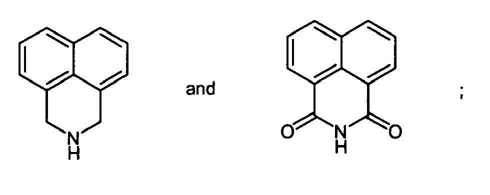
which is in each case bonded to the pyrazoline compound of general formula I in any position of the cyclic part of the aforementioned radicals including the NH-groups,
preferably said radicals are bonded to the pyrazoline compound of general formula I at the nitrogen atom of the cyclic part of the aforementioned radicals;
a -OR⁶-moiety or a -NR⁷R⁸ moiety;
- R⁴: represents F; Cl; Br; I; -OH, -CN; -C(=O)-H; -C(=O)-OH; -C(=O)-OR¹⁷;
a radical selected from the group consisting of methyl, -CF₃, -CH₂F, -CF₂H,-C₂F₅, ethyl, -CH₂-CN, -CH₂-OH, n-propyl, isopropyl, -CH₂-CH₂-CN, -CH₂-CH₂-OH, n-butyl, -CH₂-CH₂-CH₂-CN, -CH₂-CH₂-CH₂-OH, isobutyl, sec-butyl, tert-butyl, n-pentyl, 2-pentyl, 3-pentyl, neo-pentyl, n-hexyl, 2-hexyl and 3-hexyl;
a benzyl radical or a -O-R¹¹ moiety;
- R⁵: represents H; F; Cl; Br;-C(=O)-OH; -C(=O)-OR¹⁷; or a radical selected from the group consisting of methyl, ethyl, n-propyl, iso-propyl and n-butyl;
or R⁴ and R⁵ together with the bridging carbon atom form a radical selected from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl;
- R⁶: represents a hydrogen atom; a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;
- R⁷: represents a hydrogen atom or a radical selected from the group consisting of methyl, ethyl, n-propyl, isopropyl, n-butyl, sec-butyl and tert-butyl;
- R⁸: represents a radical selected from the group consisting of [1,2,3,4]-tetrahydronaphthyl, bicyclo[2.2.1]heptyl, cyclopropyl, cyclobutyl, cyclopentyl, cyclohexyl, cycloheptyl, cyclooctyl, cyclononyl, cyclodecyl, cycloundecyl and cyclododecyl, which may be bonded via a -(CH₂)-, -(CH₂)-(CH₂)- or -(CH₂)-(CH₂)-(CH₂)-group and/or subsituted with 1, 2, 3 or 4 substituents selected from the group consisting of methyl, ethyl, n-propyl, isopropyl, n-butyl, sec-butyl, tert-butyl, -OH, -O-CH₃ and -O-C₂H₅;
or a radical selected from the group consisting of 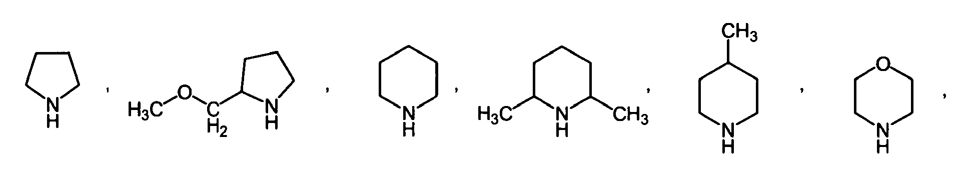 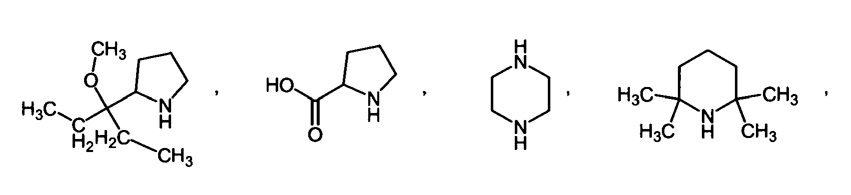 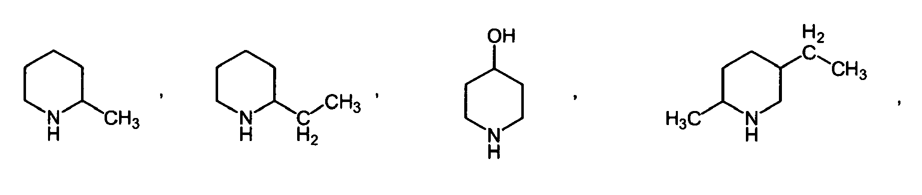 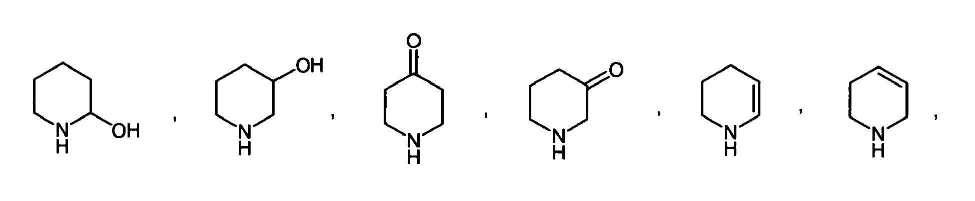 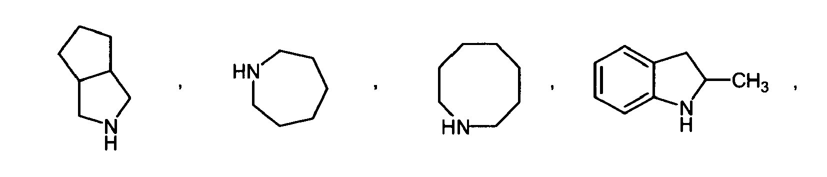 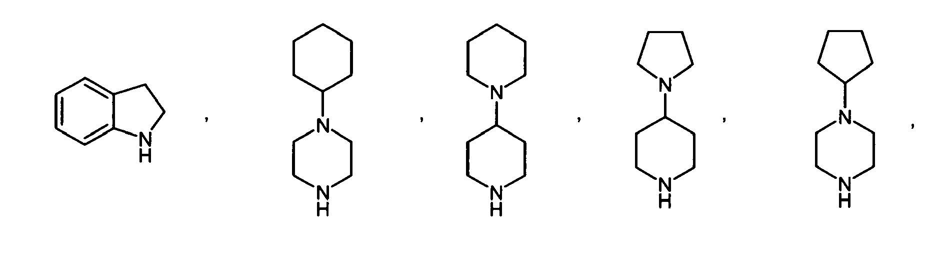 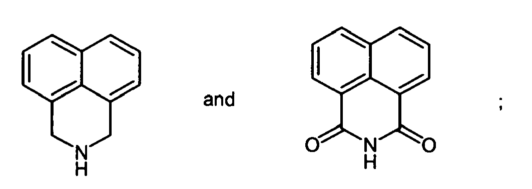 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
and
R¹¹ and R¹⁷, independently of one another, each represent a radical selected from the group consisting of methyl, ethyl, n-propyl, isopropyl, n-butyl, isobutyl, sec-butyl, tert-butyl, n-pentyl, 2-pentyl, 3-pentyl and neo-pentyl;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

As a preferred embodiment the compound (A) of subgroup A6.1 is selected from:
- [1]: *cis*-5-(4-Chloro-phenyl)-1-(2,4-dichloro-phenyl)-4-methyl-4,5-dihydro-1H-pyrazole-3-carboxylic acid piperidin-1-ylamide hydro-chloride
- [2]: *trans*-5-(4-Chloro-phenyl)-1-(2,4-dichloro-phenyl)-4-methyl-4,5-dihydro-1H-pyrazole-3-carboxylic acid piperidin-1-ylamide hydro-chloride
- [3]: *cis-*5-(4-Chloro-phenylyl-(2,4-dichloro-phenyl)-4-methyl-4,5-dihydro-1H-pyrazole-3-carboxylic acid azepan-1-ylamide
- [4]: *trans*-5-(4-Chloro-phenyl)-1-(2,4-dichloro-phenyl)-4-methyl-4,5-dihydro-1H-pyrazole-3-carboxylic acid azepan-1-ylamide
- [5]: *cis*-[5-(4-Chloro-phenyly)-1-(2,4-dichloro-phenyl)-4-methyl-4,5-dihydro-1H-pyrazol-3-yl]-(4-cyclohexyl-piperazin-1-yl)-methanone
- [6]: *trans*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone
- [7]: *cis*-5-(4-Chloro-phenylyl-(2,4-dichloro-phenyl)4-methyl-4,5-dihydro-1H-pyrazole-3-carboxylic acid pyrrolidin-1-ylamide
- [8]: *trans*-5-(4-Chloro-phenyl)-1-(2,4-dichloro-phenyl)-4-methyl-4,5-dihydro-1H-pyrazole-3-carboxylic acid pyrrolidin-1-ylamide
- [9]: *cis*-5-(4-Chloro-phenyl)-1-(2,4-dichloro-phenyl)-4-methyl-4,5-dihydro-1H-pyrazole-3-carboxylic acid (4-methyl-piperidin-1-yl)-amide
- [10]: *trans*-5-(4-Chloro-phenyl)-1-(2,4-dichloro-phenyl)-4-methyl-4,5-dihydro-1H-pyrazole-3-carboxylic acid (4-methyl-piperidin-1-yl)-amide
- [11]: *cis*-[5-(4-Chloro-phenyl)-1-(2,4-dichloro-phenyl)-4-methyl-4,5-dihydro-1H-pyrazol-3-yl]-piperidin-1-yl-methanone
- [12]: *trans*-[5-(4-Chloro-phenyl)-1-(2,4-dichloro-phenyl)-4-methyl-4,5-dihydro-1H-pyrazol-3-yl]-piperidin-1-yl-methanone
- [13]: *cis*-5-(4-Chlro-phenyl)-1-(2,4-dichloro-phenyl)-4-methyl-4,5-dihydro-1H-pyrazole-3-carboxylic acid (hexahydro-cyclopenta[c]pyrrol-2-yl)-amide
- [14]: *trans*-5-(4-Chloro-phenyl)-1-(2,4-dichloro-phenyl)-4-methyl-4,5-dihydro-1H-pyrazole-3-carboxylic acid (hexahydro-cyclopenta-[c]py-rrol-2-yl)-amide
- [15]: *cis*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide
- [16]: *trans*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide
- [17]: *cis*-5-(4-Chloro-phenyl)-1-(2,4-dichloro-phenyl)-4-methyl-4,5-dihydro-1H-pyrazole-3-carboxylic acid cyclobutylamide
- [18]: 5-(4-Chloro-phenyl)-1-(2,4-dichloro-phenyl)-4-methyl-4,5-dihydro-1H-pyrazole-3-carboxylic acid cyclohexyl methyl-amide
- [19]: *cis-*5-(4-Chloro-phenyl)-1-(2,4-dichloro-phenyl)-4-methyl-4,5-dihydro-1H-pyrazole-3-carboxylic acid
- [20]: *trans*-5-(4-Chloro-phenyl)-1-(2,4-dichloro-phenyl)-4-methyl-4,5-dihydro-1H-pyrazole-3-carboxylic acid
- [21]: *cis*-5-(4-Chloro-phenyl)-1-(2,4-dichloro-phenyl)-4-ethyl-4,5-dihydro-1H-pyrazole-3-carboxylic acid
- [22]: *trans*-5-(4-Chloro-phenyl)-1-(2,4-dichloro-phenyl)-4-ethyl-4,5-dihydro-1H-pyrazole-3-carboxylic acid piperidin-1-ylamide
- [23]: *cis*-5-(4-Chloro-phenyl)-1-(2,4-dichloro-phenyl)-4-methyl-4,5-dihydro-1H-pyrazole-3-carboxylic acid methyl ester
- [24]: *trans*-5-(4-Chloro-phenyl)-1-(2,4-dichloro-phenyl)-4-methyl-4,5-dihydro-1H-pyrazole-3-carboxylic acid methyl ester
- [25]: *cis*-5-(4-Chloro-phenyl)-1-(2,4-dichloro-phenyl)-4-methyl-4,5-dihydro-1H-pyrazole-3-carboxylic acid ethyl ester
- [26]: *trans-*5-(4-Chloro-phenyl)-1-(2,4-dichloro-phenyl)-4-methyl-4,5-dihydro-1H-pyrazole-3-carboxylic acid ethyl ester
- [27]: 5-(4-Chloro-phenyl)-1-(2,4-dichloro-phenyl)-4-methyl-4,5-dihydro-1H-pyrazole-3-carboxylic acid cyclohe-xyl ester
- [28]: 5-(4-Chloro-phenyl)-1-(2,4-dichloro-phenyl)-4,4-dimethyl-4,5-dihydro-1-H-pyrazole-3-carboxylic acid
- [29]: 5-(4-Chloro-phenyl)-1-(2,4-dichloro-phenyl)-4,4-dimethyl-4,5-dihydro-1-H-pyrazole-3-carboxylic acid piperidin-1-ylamide
- [30]: 5-(4-Chloro-phenyl)-1-(2,4-dichloro-phenyl)-4-trifluoromethyl-4,5-dihydro-1H-pyrazole-3-carboxylic acid piperidin-1-ylamide
- [31]: 5-(4-Chloro-phenyl)-1-(2,4-dichloro-phenyl)-4-hydroxy-4,5-dihydro-1H-pyrazole-3-carboxylic acid
- [32]: 5-(4-Chloro-phenyl)-1-(2,4-dichloro-phenyl)-4-hydroxy-4,5-dihydro-1H-pyrazole-3-carboxylic acid piperidin-1-ylamide
- [33]: 5-(4-Chloro-phenyl)-1-(2,4-dichloro-phenyl)-4-(2-hydroxy-ethyl)-4,5-dihydro-1H-pyrazole-3-carboxylic acid piperidin-1-ylamide
- [34]: 5-(4-Chloro-phenyl)-1-(2,4-dichloro-phenyl)-4-(2-hydroxy-ethyl)-4,5-dihydro-1H-pyrazole-3-carboxylic acid
- [35]: 5-(4-Chloro-phenyl)-1-(2,4-dichloro-phenyl)-4-fluoromethyl-4,5-dihydro-1H-pyrazole-3-carboxylic acid piperidin-1-ylamide
- [36]: 5-(4-Chloro-phenyl)-1-(2,4-dichloro-phenyl)-4-fluoromethyl-4,5-dihydro-1H-pyrazole-3-carboxylic acid
- [37]: 5-(4-Chloro-phenyl)-1-(2,4-dichloro-phenyl)-4-formyl-4,5-dihydro-1H-pyrazole-3-carboxylic acid piperidin-1-ylamide
- [38]: 5-(4-Chloro-phenyl)-1-(2,4-dichloro-phenyl)-4-formyl-4,5-dihydro-1H-pyrazole-3-carboxylic acid
- [39]: 5-(4-chlorophenyl)-4-cyano-1-(2,4-dichlorophenyl)-N-(piperidin-1-yl)-4,5-dihydro-1 H-pyrazole-3-carboxamide
- [40]: 5-(4-Chloro-phenyl)-4-cyano-1-(2,4-dichloro-phenyl)-4,5-dihydro-1 H-pyrazole-3-carboxylic acid
- [41]: 1-(2,4-Dichloro-phenyl)-5-(4-fluoro-phenyl)-4-methyl-4,5-dihydro-1H-pyrazole-3-carboxylic acid piperidin-1-ylamide
- [42]: 1-(2,4-Dichloro-phenyl)-4-ethyl-5-(4-fluoro-phenyl)-4,5-dihydro-1H-pyrazole-3-carboxylic acid piperidin-1-ylamide
- [43]: 1-(2,4-Dichloro-phenyl)-5-(4-fluoro-phenyl)-4,4-dimethyl-4,5-dihydro-1-H-pyrazole-3-carboxylic acid piperidin-1-ylamide
- [44]: 1-(2,4-Dichloro-phenyl)-5-(4-fluoro-phenyl)-4-trifluoromethyl-4,5-dihydro-1H-pyrazole-3-carboxylic acid piperidin-1-ylamide
- [45]: 1-(2,4-Dichloro-phenyl)-5-(4-fluoro-phenyl)-4-hydroxy-4,5-dihydro-1H-pyrazole-3-carboxylic acid piperidin-1-ylamide
- [46]: 1-(2,4-Dichloro-phenyl)-5-(4-fluoro-phenyl)-4-(2-hydroxy-ethyl)-4,5-dihydro-1H-pyrazole-3-carboxylic acid piperidin-1-ylamide
- [47]: 1-(2,4-Dichtloro-phenyl)-4-fluoromethyl-5-(4-fluoro-phenyl)-4,5-dihydro-1H-pyrazole-3-carboxylic acid piperidin-1-ylamide
- [48]: 1-(2,4-Dichloro-phenyl)-5-(4-fluoro-phenyl)-4-formyl-4,5-dihydro-1H-pyrazole-3-carboxylic acid piperidin-1-ylamide
- [49]: 4-Cyano-1-(2,4-dichloro-phenyl)-5-(4-fluoro-phenyl)-4,5-dihydro-1H-pyrazole-3-carboxylic acid piperidin-1-ylamide
- [50]: 5-(4-Bromo-phenyl)-1-(2,4-dichloro-phenyl)-4-methyl-4,5-dihydro-1H-pyrazole-3-carboxylic acid piperidin-1-ylamide
- [51]: 5-(4-Bromo-phenyl)-1-(2,4-dichloro-phenyl)-4-ethyl-4,5-dihydro-1H-pyrazole-3-carboxylic acid piperidin-1-ylamide
- [52]: 5-(4-Bromo-phenyl)-1-(2,4-dichloro-phenyl)-4,4-dimethyl-4,5-dihydro-1H-pyrazole-3-carboxylic acid piperidin-1-ylamide
- [53]: 5-(4-Bromo-phenyl)-1-(2,4-dichloro-phenyl)-4-trifluoromethyl-4,5-dihydro-1H-pyrazole-3-carboxylic acid piperidin-1-ylamide
- [54]: 5-(4-Bromo-phenyl)-1-(2,4-dichloro-phenyl)-4-hydroxy-4,5-dihydro-1H-pyrazole-3-carboxylic acid piperidin-1-ylamide
- [55]: 5-(4-Bromo-phenyl)-1-(2,4-dichloro-phenyl)-4-(2-hydroxy-ethyl)-4,5-dihydro-1 H-pyrazole-3-carboxylic acid piperidin-1-ylamide
- [56]: 5-(4-Bromo-phenyl)-1-(2,4-dichloro-phenyl)-4-fluoromethyl-4,5-dihydro-1H-pyrazole-3-carboxylic acid piperidin-1-ylamide
- [57]: 5-(4-Bromo-phenyl)-1-(2,4-dichloro-phenyl)-4-formyl-4,5-dihydro-1H-pyrazole-3-carboxylic acid piperidin-1-ylamide
- [58]: 5-(4-Bromo-phenyl)-4-cyano-1-(2,4-dichloro-phenyl)-4,5-dihydro-1H-pyrazole-3-carboxylic acid piperidin-1-ylamide
- [59]: *cis*-5-(5-Chloro-thiophen-2-yl)-1-(2,4-dichloro-phenyl)-4-methyl-4,5-dihydro-1H-pyrazole-3-carboxylic acid piperidin-1-ylamide
- [60]: *trans*-5-(5-Chloro-thiophen-2-yl)-1-(2,4-dichloro-phenyl)-4-methyl-4,5-dihydro-1H-pyrazole-3-carboxylic acid piperidin-1-ylamide
- [61]: *cis*-5-(5-Chloro-thiophen-2-yl)-1-(2,4-dichloro-phenyl)-4-ethyl-4,5-dihydro-1H-pyrazole-3-carboxylic acid piperidin-1-ylamide
- [62]: *trans-5-*(5-Chloro-thiophen-2-yl)-1-(2,4-dichloro-phenyl)-4-ethyl-4,5-dihydro-1H-pyrazole-3-carboxylic acid piperidin-1-ylamide
- [63]: 5-(5-Chloro-thiophen-2-yl)-1-(2,4-dichloro-phenyl)-4,4-dimethyl-4,5-dihydro-1H-pyrazole-3-carboxylic acid piperidin-1-ylamide
- [64]: 5-(5-Chloro-thiophen-2-yl)-1-(2,4-dichloro-phenyl)-4-trifluoromethyl-4,5-dihydro-1H-pyrazole-3-carboxylic acid piperidin-1-ylamide
- [65]: 5-(5-Chloro-thiophen-2-yl)-1-(2,4-dichloro-phenyl)-4-hydroxy-4,5-dihydro-1H-pyrazole-3-carboxylic acid piperidin-1-ylamide
- [66]: 5-(5-Chloro-thiophen-2-yl)-1-(2,4-dichloro-phenyl)-4-(2-hydroxy-ethyl)-4,5-dihydro-1H-pyrazole-3-carboxylic acid piperidin-1-ylamide
- [67]: 5-(5-Chloro-thiophen-2-yl)-1-(2,4-dichloro-phenyl)-4-fluoromethyl-4,5-dihydro-1H-pyrazole-3-carboxylic acid piperidin-1-ylamide
- [68]: 5-(5-Chloro-thiophen-2-yl)-1-(2,4-dichbro-phenyl)-4-formyl-4,5-dihydro-1H-pyrazole-3-carboxylic acid piperidin-1-ylamide
- [69]: 5-(5-Chloro-thiophen-2-yl)-4-cyano-1-(2,4-dichloro-phenyl)-4,5-dihydro-1H-pyrazole-3-carboxylic acid piperidin-1-ylamide
- [70]: *cis-*5-(5-Bromo-thiophen-2-yl)-1-(2,4-dichloro-phenyl)-4-methyl-4, 5-dihydro-1H-pyrazole-3-carboxylic acid piperidin-1-ylamide
- [71]: *trans*-5-(5-Bromo-thiophen-2-yl)-1-(2,4-dichloro-phenyl)-4-methyl-4,5-dihydro-1H-pyrazole-3-carboxylic acid piperidin-1-ylamide
- [72]: *cis*-5-(5-Bromo-thiophen-2-yl)-1-(2,4-dichloro-phenyl)-4-ethyl-4,5-dihydro-1H-pyrazole-3-carboxylic acid piperidin-1-ylamide
- [73]: *trans*-5-(5-Bromo-thiophen-2-yl)-1-(2,4-dichloro-phenyl)-4-ethyl-4,5-dihydro-1H-pyrazole-3-carboxylic acid piperidin-1-ylamide
- [74]: 5-(5-Bromo-thiophen-2-yl)-1-(2,4-di-chloro-phenyl)-4,4-dimethyl-4,5-dihydro-1H-pyrazole-3-carboxylic acid piperidin-1-ylamide
- [75]: *cis*-5-(4-Bromo-thiophen-2-yl)-1-(2,4-dichloro-phenyl)-4-methyl-4,5-dihydro-1H-pyrazole-3-carboxylic acid piperidin-1-ylamide
- [76]: *trans*-5-(4-Bromo-thiophen-2-yl)-1-(2,4-dichloro-phenyl)-4-methyl-4,5-dihydro-1H-pyrazole-3-carboxylic acid piperidin-1-ylamide
- [77]: *cis*-5-(4-Bromo-thiophen-2-yl)-1-(2,4-dichloro-phenyl)-4-ethyl-4,5-dihydro-1H-pyrazole-3-carboxylic acid piperidin-1-ylamide
- [78]: *trans*-5-(4-Bromo-thiophen-2-yl)-1-(2,4-dichloro-phenyl)-4-ethyl-4,5-dihydro-1H-pyrazole-3-carboxylic acid piperidin-1-ylamide
- [79]: 5-(4-Bromo-thiophen-2-yl)-1-(2,4-dichloro-phenyl)-4,4-dimethyl-4,5-dihydro-1H-pyrazole-3-carboxylic acid piperidin-1-ylamide
- [80]: 5-(5-Bromo-thiophen-2-yl)-1-(2,4-dichloro-phenyl)-4-trifluoromethyl-4,5-dihydro-1H-pyrazole-3-carboxylic acid piperidin-1-ylamide
- [81]: 5-(5-Bromo-thiophen-2-yl)-1-(2,4-di-chloro-phenyl)-4-hydroxy-4,5-dihydro-1H-pyrazole-3-carboxylic acid piperidin-1-ylamide
- [82]: 5-(5-Bromo-thiophen-2-yl)-1-(2,4-di-chloro-phenyl)-4-(2-hydroxy-ethyl)-4,5-dihydro-1H-pyrazole-3-carboxylic acid piperidin-1-ylamide
- [83]: 5-(5-Bromo-thiophen-2-yl)-1-(2,4-di-chloro-phenyl)-4-fluoromethyl-4,5-dihydro-1H-pyrazole-3-carboxylic acid piperidin-1-ylamide
- [84]: 5-(5-Bromo-thiophen-2-yl)-1-(2,4-di-chloro-phenyl)-4-formyl-4,5-dihydro-1H-pyrazole-3-carboxylic acid piperidin-1-ylamide
- [85]: 5-(5-Bromo-thiophen-2-yl)-4-cyano-1-(2,4-dichloro-phenyl)-4,5-dihydro-1 H-pyrazole-3-carboxylic acid piperidin-1-ylamide
- [86]: *cis*-5-(5-Chloro-thiophen-2-yl)-1-(2,4-dichloro-phenyl)-4-methyl-4,5-dihydro-1 H-pyrazole-3-carboxylic acid
- [87]: *trans*-[5-(5-Chloro-thiophen-2-yl)-1-(2,4-dichloro-phenyl)-4-methyl-4,5-dihydro-1 H-pyrazol-3-yl]-piperidin-1-yl-methanone
- [88]: *cis*-5-(5-Bromo-thiophen-2-yl)-1-(2,4-dichloro-phenyl)-4-methyl-4,5-dihydro-1 H-pyrazole-3-carboxylic acid
- [89]: *trans*-5-(5-Bromo-thiophen-2-yl)-1-(2,4-dichloro-phenyl)-4-methyl-4,5-dihydro-1 H-pyrazole-3-carboxylic acid
- [90]: *cis-*5-(4-Bromo-thiophen-2-yl)-1-(2,4-dichloro-phenyl)-4-methyl-4,5-dihydro-1H-pyrazole-3-carboxylic acid
- [91]: *trans-*5-(4-Bromo-thiophen-2-yl)-1-(2,4-dichloro-phenyl)-4-methyl-4,5-dihydro-1 H-pyrazole-3-carboxylic acid,
- 92: (+)-cis-5-(4-chlorophenyl)-1-(2,4-dichlorophenyl)-4-methyl-N-(p)peridin-1-yl)-4,5-dihydro-1H-pyrazole-3-carboxamide hydrochloride
- 93: (-)-cis-5-(4-chlorophenyl)-1-(2,4-dichlorophenyl)-4-methyl-N-(piperidin-1-yl)-4,5-dihydro-1H-pyrazole-3-carboxamide hydrochloride
- 94: *cis*-1-(2-chlorophenyl)-5-(4-chlorophenyl)-4-methyl-N-(piperidin-1-yl)-4,5-dihydro-1H-pyrazole-3-carboxamide
- 95: *trans*-1-(2-chlorophenyl)-5-(4-chlorophenyl)-4-methyl-N-(piperidin-1-yl)-4,5-dihydro-1H-pyrazole-3-carboxamide
- 96: *cis-*5-(4-Chlorophenyl)-1-(2,4-dichlorophenyl)-4-methyl-4,5-dihydro-1H-pyrazole-3-carboxylic acid azepan-1-ylamide hydrochloride
- 97: *trans*-5-(4-Chlorophenyl)-1-(2,4-dichloro-phenyl)-4-methyl-4,5-dihydro-1H-pyrazole-3-carboxylic acid azepan-1-ylamide hydrochloride
- 98: *cis*-N-(azepan-1-yl)-1-(2-chlorophenyl)-5-(4-chlorophenyl)-4-methyl-4,5-dihydro-1H-pyrazole-3-carboxamide
- 99: *trans*-N-(azepan-1-yl)-1-(2-chlorophenyl)-5-(4-chlorophenyl)-4-methyl-4,5-dihydro-1H-pyrazole-3-carboxamide
- 100: *cis-*5-(4-chlorophenyl)-N-(4-cyclopentylpiperazin-1-yl)-1-(2,4-dichlorophenyl)-4-methyl-4,5-dihydro-1 H-pyrazole-3-carboxamide hydrochloride
- 101: *cis-*5-(4-chlorophenyl)-1-(2,4-dichlorophenyl)-4-methyl-N-morpholino-4,5-dihydro-1H-pyrazole-3-carboxamide
- 102: *cis-*1-(2-chlorophenyl)-5-(4-chlorophenyl)-4-methyl-N-(4-methylpiperidin-1-yl)-4,5-dihydro-1H-pyrazole-3-carboxamide
- 103: *cis-5-(*4-chlorophenyl)-1-(2,4-dichlorophenyl)-N-(2,6-dimethylpiperidin-1-yl)-4-methyl-4,5-dihydro-1H-pyrazole-3-carboxamide
- *104*: *cis*-5-(4-Chlorophenylyl-(2,4-dichlorophenyl)-4-methyl-4,5-dihydro-1H-pyrazole-3-carboxylic acid (1,3-dioxo-1H,3H-benzo[de]isoquinolin-2-yl)-*amide*
- 105: *cis*-5-(4-Chlorophenyl)-1-(2,4-dichlorophenyl)-4-methyl-4,5-dihydro-1H-pyrazole-3-carboxylic acid (1H,3H-benzo[de]isoquinolin-2-yl)-amide
- 106: *cis*-5-(4-chlorophenyl)-1-(2,4-dichlorophenyl)-N-((R)-2-(methoxymethyl)pyrrolid in-1-yl)-4-methyl-4,5-dihydro-1H-pyrazole-3-carboxamide hydrochloride
- 107: *cis*-5-(4-chlorophenyl)-1-(2,4-dichlorophenyl)-N-((S)-2-(methoxymethyl)pyrroridin-1-yl)-4-methyl-4,5-dihydro-1H-pyrazole-3-carboxamide hydrochloride
- 108: *cis*-5-(4-Chlorophenyl)-1-(2,4-dichlorophenyl)-4-methyl-4,5-dihydro-1H-pyrazole-3-carboxylic acid (hexahydro-cyclopenta[c]pyrrol-2-yl)-amide hydrochloride
- 109: cis-1-(2-chlorophenyl)-5-(4-chlorophenyl)-N-(hexahydrocyclopenta[c]pyrrol-2(1H)-yl)-4-methyl-4,5-dihydro-1H-pyrazole-3-carboxamide
- 110: *trans*-1-(2-chlorophenyl)-5-(4-chlorophenyl)-N-(hexahydrocyclopenta[c]pyrrol-2(1 H)-yl)-4-methyl-4,5-dihydro-1 H-pyrazole-3-carboxamide
- 111: *cis*-1-(2-chlorophenyl)-5-(4-chlorophenyl)-N-(indolin-1-yl)-4-methyl-4,5-dihydro-1 H-pyrazole-3-carboxamide
- 112: *trans*-1-(2-chlorophenyl)-5-(4-chlorophenyl)-N-(indolin-1-yl)-4-methyl-4,5-dihydro-1 H-pyrazole-3-carboxamide
- 113: *cis*-5-(4-chlorophenyl)-1-(2,4-dichlorophenyl)-4-methyl-N-(2-methylindolin-1-yl)-4,5-dihydro-1 H-pyrazole-3-carboxamide
- 114: *cis*-5-(4-chlorophenyl)-N-(cyclohexylmethyl)-1-(2,4-dichlorophenyl)-4-methyl-4,5-dihydro-1 H-pyrazole-3-carboxamide
- 115: *cis*-5-(4-chlorophenyl)-N-(cycloheptylmethyl)-1-(2,4-dichlorophenyl)-4-methyl-4,5-dihydro-1 H-pyrazole-3-carboxamide
- 116: *cis*-5-(4-chlorophenyl)-N-cycloclodecyl-1-(2,4-dichlorophenyl)-4-methyl-4,5-dihydro-1H-pyrazole-3-carboxamide
- 117: *cis*-5-(4-chlorophenyl)-1-(2,4-dichlorophenyl)-4-methyl-N-(1,2,3,4-tetrahydronaphthalen-1-yl)-4,5-dihydro-1H-pyrazole-3-carboxamide
- 118: *cis*-N-(4-tert-butylcyclohexyl)-5-(4-chlorophenyl)-1-(2,4-dichlorophenyl)-4-methyl-4,5-dihydro-1H-pyrazole-3-carboxamide
- 119: *cis*-5-(4-chlorophenyl)-N-cyclooctyl-1-(2,4-dichlorophenyl)-4-methyl-4,5-dihydro-1H-pyrazole-3-carboxamide
- 120: cis-N-(azocan-1-yl)-5-(4-chlorophenyl)-1-(2,4-dichlorophenyl)-4-methyl-4,5-dihydro-1H-pyrazole-3-carboxamide
- 121: *cis*-N-(azocan-1-yl)-1-(2-chlorophenyl)-5-(4-chlorophenyl)-4-methyl-4,5-dihydro-1H-pyrazole-3-carboxamide
- 122: *cis-*azocan-1-yl(cis-5-(4-chlorophenyl)-1-(2,4-dichlorophenyl)-4-methyl-4,5-dihydro-1H-pyrazol-3-yl)methanone
- 123: *cis-*5-(4-chlorophenyl)-N-cycloheptyl-1-(2,4-dichlorophenyl)-4-methyl-4,5-dihydro-1H-pyrazole-3-carboxamide
- 124: *cis*-1-(2-chlorophenyl)-5-(4-chlorophenyl)-N-cycloheptyl-4-methyl-4,5-dihydro-1H-pyrazole-3-carboxamide
- 125: *trans*-5-(4-chlorophenyl)-N-cycloheptyl-1-(2,4-dichlorophenyl)-4-methyl-4,5-dihydro-1H-pyrazole-3-carboxamide
- 126: *trans*-1-(2-chlorophenyl)-5-(4-chlorophenyl)-N-cycloheptyl-4-methyl-4,5-dihydro-1H-pyrazole-3-carboxamide
- 127: *cis*-5-(4-chlorophenyl)-N-(cyclohexylmethyl)-1-(2,4-dichlorophenyl)-4-methyl-4,5-dihydro-1H-pyrazole-3-carboxamide
- 128: (+)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide
- 129: (-)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide
- 130: (-)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide
- 131: (+)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide
- 132: (+)-cis-1-(2-chlorophenyl)-5-(4-chlorophenyl)-N-((1S,2S)-2-hydroxycyclohexyl)-4-methyl-4,5-dihydro-1H-pyrazole-3-carboxamide
- 133: (-)-cis-1-(2-chlorophenyl)-5-(4-chlorophenyl)-N-((1S,2S)-2-hydroxycyclohexyl)-4-methyl-4,5-dihydro-1H-pyrazole-3-carboxamide
- 134: *cis*-5-(4-chlorophenyl)-1-(2,4-dichlorophenyl)-N-(4-hydroxypiperidin-1-yl)-4-methyl-4,5-dihydro-1 H-pyrazole-3-carboxamide
- 135: *cis*-1-(2-chlorophenyl)-5-(4-chlorophenyl)-N-(4-hydroxypiperidin-1-yl)-4-methyl-4,5-dihydro-1H-pyrazole-3-carboxamide
- 136: *cis*-5-(4-chlorophenyl)-1-(2,4-dichlorophenyl)-N-(3-hydroxypiperidin-1-yl)-4-methyl-4,5-dihydro-1H-pyrazole-3-carboxamide
- 137: *cis*-1-(2-chlorophenyl)-5-(4-chlorophenyl)-N-(3-hydroxypiperidin-1-yl)-4-methyl-4,5-dihydro-1H-pyrazole-3-carboxamide
- 138: *cis*-5-(4-chlorophenyl)-1-(2,4-dichlorophenyl)-N-(2-hydroxypiperidin-1-yl)-4-methyl-4,5-dihydro-1H-pyrazole-3-carboxamide
- 139: cis-1-(2-chlorophenyl)-5-(4-chlorophenyl)-N-(2-hydroxypiperidin-1-yl)-4-methyl-4,5-dihydro-1H-pyrazole-3-carboxamide
- 140: *cis*-5-(4-chlorophenyl)-1-(2,4-dichlorophenyl)-4-methyl-N-(4-oxopiperidin-1-yl)-4,5-dihydro-1H-pyrazole-3-carboxamide
- 141: *cis*-1-(2-chlorophenyl)-5-(4-chlorophenyl)-4-methyl-N-(4-oxopiperidin-1-yl)-4,5-dihydro-1 H-pyrazole-3-carboxamide
- 142: *cis-*5-(4-chlorophenyl)-1-(2,4-dichlorophenyl)-4-methyl-N-(3-oxopiperidin-1-yl)-4,5-dihydro-1 H-pyrazole-3-carboxamide
- 143: cis-1-(2-chlorophenyl)-5-( 4-chlorophenyl)-4-methyl-N-(3-oxopiperidin-1-yl)-4,5-dihydro-1 H-pyrazole-3-carboxamide
- 144: *cis*-5-(4-chlorophenyl)-1-(2,4-dichlorophenyl)-N-(3,4-dihydropyridin-1(2H)-yl)-4-methyl-4,5-dihydro-1H-pyrazole-3-carboxamide
- 145: *cis*-1-(2-chlorophenyl)-5-(4-chlorophenyl)-N-(3,4-dihydropyridin-1(2H)-yl)-4-methyl-4,5-dihydro-1H-pyrazole-3-carboxamide
- 146: *cis*-5-(4-chlorophenyl)-1-(2,4-dichlorophenyl)-N-(5,6-dihydropyridin-1(2H)-yl)-4-methyl-4,5-dihydro-1H-pyrazole-3-carboxamide
- 147: *cis*-1-(2-chlorophenyl)-5-(4-chlorophenyl)-N-(5,6-dihydropyridin-1(2H)-yl)-4-methyl-4,5-dihydro-1H-pyrazole-3-carboxamide
- 148: *cis*-1-(2-chlorophenyl)-5-(4-chlorophenyl)-4-methyl-4,5-dihydro-1H-pyrazole-3-carboxylic acid
- 149: *trans*-1-(2-chlorophenyl)-5-(4-chlorophenyl)-4-methyl-4,5-dihydro-1H-pyrazole-3-carboxylic acid
- 150: *cis*-5-(4-Chlorophenyl)-1-(2,4-dichlorophenyl)-4-methyl-4,5-dihydro-1H-pyrazole-3-carboxylic acid cyclohexyl ester
- 151: N-(azepan-1-yl)-5-(4-chlorophenyl)-1-(2,4-dichlorophenyl)-4,4-dimethyl-4,5-dihydro-1H-pyrazole-3-carboxamide
- 152: 1-(2-chlorophenyl)-5-(4-chlorophenyl)-4,4-dimethyl-N-(piperidin-1-yl)-4,5-dihydro-1H-pyrazole-3-carboxamide
- 153: N-(azepan-1-yl)-1-(2-chlorophenyl)-5-(4-chlorophenyl)-4,4-dimethyl-4,5-dihydro-1H-pyrazole-3-carboxamide
- 154: *cis*-5-(4-Chlorophenyl)-1-(2,4-dichlorophenyl)-4-trifluoromethyl-4,5-dihydro-1H-pyrazole-3-carboxylic acid piperidin-1-ylamide
- 155: *trans*-5-(4-Chloro-phenyl)-1-(2,4-dichlorophenyl)-4-trifluoromethyl-4,5-dihydro-1H-pyrazole-3-carboxylic acid piperidin-1-ylamide
- 156: *cis*-N-(azepan-1-yl)-5-(4-chlorophenyl)-1-(2,4-dichlorophenyl)-4-(trifluoromethyl)-4,5-dihydro-1H-pyrazole-3-carboxamide
- 157: *trans*-N-(azepan-1-yl)-5-(4-chlorophenyl)-1-(2,4-dichlorophenyl)-4-(trifluoromethyl)-4,5-dihydro-1 H-pyrazole-3-carboxamide
- 158: *cis*-1-(2-chlorophenyl)-5-(4-chlorophenyl)-N-(piperidin-1-yl)-4-(trifluoromethyl)-4,5-dihydro-1 H-pyrazole-3-carboxamide
- 159: *trans* 1-(2-chlorophenyl)-5-(4-chlorophenyl)-N-(piperidin-1-yl)-4-(trifluoromethyl)-4,5-dihydro-1H-pyrazole-3-carboxamide
- 160: 5-(4-chlorophenyl)-1-(2 ,4-dichlorophenyl)-3-(piperidin-1-ylcarbamoyl)-4,5-dihydro-1 H-pyrazole-4-carboxylic acid
- 161: 5-(4-chlorophenyl)-1-(2,4-dichlorophenyl)-4,4-difluoro-N-(piperidin-1-yl)-4,5-dihydro-1 H-pyrazole-3-carboxamide
- 162: *cis*-5-(4-chlorophenyl)-4-cyano-1-(2,4-dichlorophenyl)-N-(piperidin-1-yl)-4,5-dihydro-1H-pyrazole-3-carboxamide
- 163: *cis*-N-(azepan-1-yl)-5-(4-chlorophenyl)-4-cyano-1-(2,4-dichlorophenyl)-4,5-dihydro-1H-pyrazole-3-carboxamide
- 164: *cis*-1-(2-chlorophenyl)-5-(4-chlorophenyl)-4-cyano-N-(piperidin-1-yl)-4,5-dihydro-1H-pyrazole-3-carboxamide
- 165: *cis*-N-(azepan-1-yl)-1-(2-chlorophenyl)-5-(4-chlorophenyl)-4-cyano-4,5-dihydro-1H-pyrazole-3-carboxamide
- 166: *trans*-5-(4-chlorophenyl)-4-cyano-1-(2,4-dichlorophenyl)-N-(piperidin-1-yl)-4,5-dihydro-1H-pyrazole-3-carboxamide
- 167: *trans*-N-(azepan-1-yl)-5-(4-chlorophenyl)-4-cyano-1-(2,4-dichlorophenyl)-4,5-dihydro-1H-pyrazole-3-carboxamide
- 168: *trans*-1-(2-chlorophenyl)-5-(4-chlorophenyl)-4-cyano-N-(piperidin-1-yl)-4,5-dihydro-1H-pyrazole-3-carboxamide
- 169: *cis*-1-(2-chlorophenyl)-5-(4-chlorophenyl)-4-ethyl-4,5-dihydro-1H-pyrazole-3-carboxylic acid
- 170: *trans*-1-(2-chlorophenyl)-5-(4-chlorophenyl)-4-ethyl-4,5-dihydro-1H-pyrazole-3-carboxylic acid
- 171: *cis*-5-(4-chlorophenyl)-1-(2,4-dichlorophenyl)-4-ethyl-N-(piperidin-1-yl)-4,5-dihydro-1H-pyrazole-3-carboxamide
- 172: *trans-*5-(4-chlorophenyl)-1-(2,4-dichlorophenyl)-4-ethyl-N-(piperidin-1-yl)-4,5-dihydro-1H-pyrazole-3-carboxamide
- 173: *cis*-1-(2-chlorophenyl)-5-(4-chlorophenyl)-4-ethyl-N-(piperidin-1-yl)-4,5-dihydro-1H-pyrazole-3-carboxamide
- 174: *trans*-1-(2-chlorophenyl)-5-(4-chlorophenyl)-4-ethyl-N-(piperidin-1-yl)-4,5-dihydro-1H-pyrazole-3-carboxamide
- 175: *cis*-N-(azepan-1-yl)-5-(4-chlorophenyl)-1-(2,4-dichlorophenyl)-4-ethyl-4,5-dihydro-1H-pyrazole-3-carboxamide
- 176: *trans-*N-(azepan-1-yl)-5-(4-chlorophenyl)-1-(2,4-dichlorophenyl)-4-ethyl-4,5-dihydro-1H-pyrazole-3-carboxamide
- 177: *cis*-N*-*(azepan-1-yl)-1-(2-chlorophenyl)-5-(4-chlorophenyl)-4-ethyl-4,5-dihydro-1H-pyrazole-3-carboxamide
- 178: *trans*-N-(azepan-1-yl)-1-(2-chlorophenyl)-5-(4-chlorophenyl)-4-ethyl-4,5-dihydro-1H-pyrazole-3-carboxamide
- 179: *cis-*5-(4-chlorophenyl)-N-cycloheptyl-1-(2,4-dichlorophenyl)-4-ethyl-4,5-dihydro-1H-pyrazole-3-carboxamide
- 180: *cis*-1-(2-chlorophenyl)-5-(4-chlorophenyl)-N-cycloheptyl-4-ethyl-4,5-dihydro-1H-pyrazole-3-carboxamide
- 181: *cis*-5-(4-chlorophenyl)-1-(2,4-dichlorophenyl)-4-ethyl-N-(hexahydrocyclopenta[c]pyrrol-2(1H)-yl)-4,5-dihydro-1H-pyrazole-3-carboxamide
- 182: *cis*-1-(2-chlorophenyl)-5-(4-chlorophenyl)-4-ethyl-N-(hexahydrocyclopenta[c]pyrrol-2(1H)-yl)-4,5-dihydro-1 H-pyrazole-3-carboxamide
- 183: *cis*-5-(4-chlorophenyl)-1-(2,4-dichlorophenyl)-4-ethyl-N-(indolin-1-yl)-4,5-dihydro-1H-pyrazole-3-carboxamide
- 184: cis-1-(2-chlorophenyl)-5-(4-chlorophenyl)-4-ethyl-N-(indolin-1-yl)-4,5-dihydro-1H-pyrazole-3-carboxamide
- 185: *cis*-5-(4-bromophenyl)-1-(2,4-dichlorophenyl)-4-ethyl-4,5-dihydro-1H-pyrazole-3-carboxylic acid
- 186: *trans*-5-(4-bromophenyl)-1-(2,4-dichlorophenyl)-4-ethyl-4,5-dihydro-1H-pyrazole-3-carboxylic acid
- 187: *cis*-5-(4-bromophenyl)-1-(2,4-dichlorophenyl)-4-ethyl-N-(piperidin-1-yl)-4,5-dihydro-1H-pyrazole-3-carboxamide
- 188: *trans*-5-(4-bromophenyl)-1-(2,4-dichlorophenyl)-4-ethyl-N-(piperidin-1-yl)-4,5-dihydro-1H-pyrazole-3-carboxamide
- 189: cis-5-(4-bromophenyl)-1-(2-chlorophenyl)-4-ethyl-N-(piperidin-1-yl)-4,5-dihydro-1H-pyrazole-3-carboxamide
- 190: *trans*-5-(4-bromophenyl)-1-(2-chlorophenyl)-4-ethyl-N-(piperidin-1-yl)-4,5-dihydro-1H-pyrazole-3-carboxamide
- 191: *cis*-N-(azepan-1-yl)-5-(4-bromophenyl)-1-(2,4-dichlorophenyl)-4-ethyl-4,5-dihydro-1H-pyrazole-3-carboxamide
- 192: *cis*-N-(azepan-1-yl)-5-(4-bromophenyl)-1-(2-chlorophenyl)-4-ethyl-4,5-dihydro-1 H-pyrazole-3-carboxamide
- 193: *cis*-5-(4-bromophenyl)-N-cycloheptyl-1-(2,4-dichlorophenyl)-4-ethyl-4,5-dihydro-1H-pyrazole-3-carboxamide
- 194: *cis-*5-(4-bromophenyl)-1-(2-chlorophenyl)-N-cycloheptyl-4-ethyl-4,5-dihydro-1 H-pyrazole-3-carboxamide
- 195: *cis*-5-(4-bromophenyl)-1-(2,4-dichlorophenyl)-4-ethyl-N-(hexahydrocyclopenta[c]pyrrol-2(1H)-yl)-4,5-dihydro-1H-pyrazole-3-carboxamide
- 196: *cis-*5-(4-bromophenyl)-1-(2-chlorophenyl)-4-ethyl-N-(hexahydrocyclopenta[c]pyrrol-2(1H)-yl)-4,5-dihydro-1H-pyrazole-3-carboxamide
- 197: *cis*-5-(4-bromophenyl)-1-(2,4-dichlorophenyl)-4-ethyt-N-(indo)in-1-yl)-4,5-dihydro-1 H-pyrazole-3-carboxamide
- 198: *cis-*5-(4-bromophenyl)-1-(2-chlorophenyl)-4-ethyl-N-(indolin-1-yl)-4,5-dihydro-1 H-pyrazole-3-carboxamide
- 199: *cis-*5-(4-bromophenyl)-1-(2,4-dichlorophenyl)-4-methyl-4,5-dihydro-1H-pyrazole-3-carboxylic acid
- 200: *trans*-5-(4-bromophenyl)-1-(2,4-dichlorophenyl)-4-methyl-4,5-dihydro-1H-pyrazole-3-carboxylic acid
- 201: *cis*-5-(4-bromophenyl)-1-(2-chlorophenyl)-4-methyl-4,5-dihydro-1H-pyrazole-3-carboxylic acid
- 202: *trans* -5-(4-bromophenyl)-1-(2-chlorophenyl)-4-methyl-4,5-dihydro-1H-pyrazole-3-carboxylic acid
- 203: *cis*-5-(4-bromophenyl)-1-(2,4-dichlorophenyl)-4-methyl-N-(piperidin-1-yl)-4,5-dihydro-1H-pyrazole-3-carboxamide
- 204: *trans-*5-(4-bromophenyl)-1-(2,4-dichlorophenyl)-4-methyl-N-(piperidin-1-yl)-4,5-dihydro-1H-pyrazole-3-carboxamide
- 205: *cis* 5-(4-bromophenyl)-1-(2-chlorophenyl)-4-methyl-N-(piperidin-1-yl)-4,5-dihydro-1H-pyrazole-3-carboxamide
- 206: *trans*-5-(4-bromophenyl)-1-(2-chlorophenyl)-4-methyl-N-(piperidin-1-yl)-4,5-dihydro-1H-pyrazole-3-carboxamide
- 207: *cis*-N-(azepan-1-yl)-5-(4-bromophenyl)-1-(2,4-dichlorophenyl)-4-methyl-4,5-dihydro-1H-pyrazole-3-carboxamide hydrochloride
- 208: *trans*-N-(azepan-1-yl)-5-(4-bromophenyl)-1-(2,4-dichlorophenyl)-4-methyl-4,5-dihydro-1H-pyrazole-3-carboxamide hydrochloride
- 209: *cis*-N-(azepan-1-yl)-5-(4-bromophenyl)-1-(2-chlorophenyl)-4-methyl-4,5-dihydro-1 H-pyrazole-3-carboxamide
- 210: *cis*-N-(azepan-1-yl)-5-(4-bromophenyl)-1 -(2-chlorophenyl)-4-methyl-4,5-dihydro-1 H-pyrazole-3-carboxamide
- 211: *cis*-5-(4-bromophenyl)-N-cycloheptyl-1-(2,4-dichlorophenyl)-4-methyl-4,5-dihydro-1 H-pyrazole-3-carboxamide
- 212: *cis*-5*-*(4-bromophenyl)-1-(2-chlorophenyl)-N-cycloheptyl-4-methyl-4,5-dihydro-1 H-pyrazole-3-carboxamide
- 213: *cis*-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
- 214: *ci*s*-*5-(4-bromophenyl)-1-(2-chlorophenyl)-N-(hexahydrocyclopenta[c]pyrrol-2(1H)-yl)-4-methyl-4,5-dihydro-1H-pyrazole-3-carboxamide
- 215: *cis-*5-(4-bromophenyl)-1-(2,4-dichlorophenyl)-N-(indolin-1-yl)-4-methyl-4,5-dihydro-1H-pyrazole-3-carboxamide
- 216: *cis*-5-(4-bromophenyl)-(2-chlorophenyl)-N-(indolin-1-yl)-4-methyl-4,5-dihydro-1H-pyrazole-3-carboxamide
- 217: (+)-*cis*-5-(4-bromophenyl)-1-(2,4-dichlorophenyl)-N-((1S,2S)-2-hydroxycyclohexyl)-4-methyl-4,5-dihydro-1H-pyrazole-3-carboxamide
- 218: (-)-*cis*-5-(4-bromophenyl)-1-(2,4-dichlorophenyl)-N-((1S,2S)-2-hydroxycyclohexyl)-4-methyl-4,5-dihydro-1H-pyrazole-3-carboxamide
- 219: (+)-*cis*-5-(4-bromophenyl)-1-(2-chlorophenyl)-N-((1S,2S)-2-hydroxycyclohexyl)-4-methyl-4,5-dihydro-1H-pyrazole-3-carboxamide
- 220: (-)-*cis*-5-(4-bromophenyl)-1-(2-chlorophenyl)-N-((1S,2S)-2-hydroxycyclohexyl)-4-methyl-4,5-dihydro-1H-pyrazole-3-carboxamide
- 221: *cis*-1-(2,4-dichlorophenyl)-5-(4-fluorophenyl)-4-methyl-4,5-dihydro-1H-pyrazole-3-carboxylic acid
- 222: *trans*-1-(2,4-dichlorophenyl)-5-(4-fluorophenyl)-4-methyl-4,5-dihydro-1H-pyrazole-3-carboxylic acid
- 223: *cis*-1-(2,4-dichlorophenyl)-5-(4-fluorophenyl)-4-methyl-N-(piperidin-1-yl)-4,5-dihydro-1H-pyrazole-3-carboxamide
- 224: *trans*-1-(2,4-dichlorophenyl)-5-(4-fluorophenyl)-4-methyl-N-(piperidin-1-yl)-4,5-dihydro-1H-pyrazole-3-carboxamide
- 225: *cis*-1-(2-chlorophenyl)-5-(4-fluorophenyl)-4-methyl-N-(piperidin-1-yl)-4,5-dihydro-1H-pyrazole-3-carboxamide
- 226: *trans*-1-(2-chlorophenyl)-5-(4-fluorophenyl)-4-methyl-N-(piperidin-1-yl)-4,5-dihydro-1H-pyrazole-3-carboxamide
- 227: *cis*-N-(azepan-1-yl)-1-(2,4-dichlorophenyl)-5-(4-fluorophenyl)-4-methyl-4,5-dihydro-1H-pyrazole-3-carboxamide hydrochloride
- 228: *trans*-N-(azepan-1-yl)-1-(2,4-dichlorophenyl)-5-(4-fluorophenyl)-4-methyl-4,5-dihydro-1H-pyrazole-3-carboxamide hydrochloride
- 229: *cis*-N-(azepan-1-yl)-1-(2-chlorophenyl)-5-(4-fluorophenyl)-4-methyl-4,5-dihydro-1H-pyrazole-3-carboxamide
- 230: *trans*-N-(azepan-1-yl)-1-(2-chlorophenyl)-5-(4-fluorophenyl)-4-methyl-4,5-dihydro-1H-pyrazole-3-carboxamide
- 231: *cis*-N-cycloheptyl-1-(2,4-dichlorophenyl)-5-(4-fluorophenyl)-4-methyl-4,5-dihydro-1H-pyrazole-3-carboxamide
- 232: *cis*-1-(2-chlorophenyl)-N-cycloheptyl-5-(4-fluorophenyl)-4-methyl-4,5-dihydro-1 H-pyrazole-3-carboxamide
- 233: *cis*-1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride
- 234: *cis*-1-(2-chlorophenyl)-5-(4-fluorophenyl)-N-(hexahydrocyclopenta[c]pyrrol-2(1H)-yl)-4-methyl-4,5-dihydro-1 H-pyrazole-3-carboxamide
- 235: *cis*-1-(2,4-dichlorophenyl)-5-(4-fluorophenyl)-N-(indolin-1-yl)-4-methyl-4,5-dihydro-1 H-pyrazole-3-carboxamide
- 236: *cis*-1-(2-chlorophenyl)-5-(4-fluorophenyl)-N-(indolin-1-yl)-4-methyl-4,5-dihydro-1 H-pyrazole-3-carboxamide
- 237: (+)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide
- 238: (-)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide
- 239: (+)-cis-1-(2-chlorophenyl)-5-(4-fluorophenyl)-N-((1S,2S)-2-hydroxycyclohexyl)-4-methyl-4,5-dihydro-1H-pyrazole-3-carboxamide
- 240: (-)-cis-1-(2-chlorophenyl)-5-(4-fluorophenyl)-N-((1S,2S)-2-hydroxycyclohexyl)-4-methyl-4,5-dihydro-1 H-pyrazole-3-carboxamide
- 241: *cis*-1-(2,4-dichlorophenyl)-5-(4-iodophenyl)-4-methyl-4,5-dihydro-1H-pyrazole-3-carboxylic acid
- 242: *trans*-1-(2,4-dichlorophenyl)-5-(4-iodophenyl)-4-methyl-4,5-dihydro-1H-pyrazole-3-carboxylic acid
- 243: *cis*-1-(2,4-dichlorophenyl)-5-(4-iodophenyl)-4-methyl-N-(piperidin-1-yl)-4,5-dihydro-1 H-pyrazole-3-carboxamide
- 244: *trans*-1-(2,4-dichlorophenyl)-5-( 4-iodophenyl)-4-methyl-N-(piperidin-1-yl)-4,5-dihydro-1 H-pyrazole-3-carboxamide
- 245: *cis*-1-(2-chlorophenyl)-5-(4-iodophenyl)-4-methyl-N-(piperidin-1-yl)-4,5-dihydro-1H-pyrazole-3-carboxamide
- 246: *trans*-1-(2-chlorophenyl)-5-(4-iodophenyl)-4-methyl-N-(piperidin-1-yl)-4,5-dihydro-1H-pyrazole-3-carboxamide
- 247: *cis*-N-(azepan-1-yl)-1-(2,4-dichlorophenyl)-5-(4-iodophenyl)-4-methyl-4,5-dihydro-1H-pyrazole-3-carboxamide
- 248: *trans*-N-(azepan-1-yl)-1-(2,4-dichlorophenyl)-5-(4-iodophenyl)-4-methyl-4,5-dihydro-1H-pyrazole-3-carboxamide
- 249: *cis*-N-(azepan-1-yl)-1-(2-chlorophenyl)-5-(4-iodophenyl)-4-methyl-4,5-dihydro-1H-pyrazole-3-carboxamide
- 250: *trans*-N-(azepan-1-yl)-1-(2-chlorophenyl)-5-(4-iodophenyl)-4-methyl-4,5-dihydro-1H-pyrazole-3-carboxamide
- 251: *cis*-N-cycloheptyl-1-(2,4-dichlorophenyl)-5-(4-iodophenyl)-4-methyl-4,5-dihydro-1H-pyrazole-3-carboxamide
- 252: *cis*-1-(2-chlorophenyl)-N-cycloheptyl-5-(4-iodophenyl)-4-methyl-4,5-dihydro-1H-pyrazole-3-carboxamide
- 253: *cis-*1-(2,4-dichlorophenyl)-N-(hexahydrocyclopenta[c]pyrrol-2(1H)-yl)-5-(4-iodophenyl)-4-methyl-4,5-dihydro-1H-pyrazole-3-carboxamide
- 254: *cis*-1-(2-chlorophenyl)-N-(hexahydrocyclopenta[c]pyrrol-2(1H)-yl)-5-(4-iodophenyl)-4-methyl-4,5-dihydro-1H-pyrazole-3-carboxamide
- 255: *cis-1-(2*,4-dichlorophenyl)-N-(indolin-1-yl)-5-(4-iodophenyl)-4-methyl-4,5-dihydro-1 H-pyrazole-3-carboxamide
- 256: *cis-*1-(2-chlorophenyl)-N-(indolin-1-yl)-5-(4-iodophenyl)-4-methyl-4,5-dihydro-1 H-pyrazole-3-carboxamide
- 257: (+)-*cis*-1-(2,4-dichlorophenyl)-N-((1S,2S)-2-hydroxycyclohexyl)-5-(4-iodophenyl)-4-methyl-4,5-dihydro-1H-pyrazole-3-carboxamide
- 258: (-)-cis-1-(2,4-dichlorophenyl)-N-((1S,2S)-2-hydroxycyclohexyl)-5-(4-iodophenyl)-4-methyl-4,5-dihydro-1H-pyrazole-3-carboxamide
- 259: (+)-cis-1-(2-chlorophenyl)-N-((1S,2S)-2-hydroxycyclohexyl)-5-(4-iodophenyl)-4-methyl-4,5-dihydro-1H-pyrazole-3-carboxamide
- 260: (-)-cis-1-(2-chlorophenyl)-N-((1S,2S)-2-hydroxycyclohexyl)-5-(4-iodophenyl)-4-methyl-4,5-dihydro-1H-pyrazole-3-carboxamide
- 261: *cis*-1-(2,4-dichlorophenyl)-5-(4-methoxyphenyl)-4-methyl-4,5-dihydro-1H-pyrazole-3-carboxylic acid
- 262: *trans*-1-(2,4-dichlorophenyl)-5-(4-methoxyphenyl)-4-methyl-4,5-dihydro-1H-pyrazole-3-carboxylic acid
- 263: *cis*-1-(2-chlorophenyl)-5-(4-methoxyphenyl)-4-methyl-4,5-dihydro-1H-pyrazole-3-carboxylic acid
- 264: *trans*-1-(2-chlorophenyl)-5-(4-methoxyphenyl)-4-methyl-4,5-dihydro-1H-pyrazole-3-carboxylic acid
- 265: *cis*-1-(2,4-dichlorophenyl)-5-(4-methoxyphenyl)-4-methyl-N-(piperidin-1-yl)-4,5-dihydro-1H-pyrazole-3-carboxamide hydrochloride
- 266: (+)-cis-1-(2,4-dichlorophenyl)-5-(4-methoxyphenyl)-4-methyl-N-(piperidin-1-yl)-4,5-dihydro-1H-pyrazole-3-carboxamide hydrochloride
- 267: (-)-cis-1-(2,4-dichlorophenyl)-5-(4-methoxyphenyl)-4-methyl-N-(piperidin-1-yl)-4,5-dihydro-1H-pyrazole-3-carboxamide hydrochloride
- 268: *trans*-1-(2,4-dichlorophenyl)-5-(4-methoxyphenyl)-4-methyl-N-(piperidin-1-yl)-4,5-dihydro-1H-pyrazole-3-carboxamide
- 269: *cis*-1-(2-chlorophenyl)-5-(4-methoxyphenyl)-4-methyl-N-(piperidin-1-yl)-4,5-dihydro-1H-pyrazole-3-carboxamide
- 270: *trans*-1-(2-chlorophenyl)-5-(4-methoxyphenyl)-4-methyl-N-(piperidin-1-yl)-4,5-dihydro-1H-pyrazole-3-carboxamide
- 271: *cis*-N-(azepan-1-yl)-1-(2,4-dichlorophenyl)-5-(4-methoxyphenyl)-4-methyl-4,5-dihydro-1H-pyrazole-3-carboxamide hydrochloride
- 272: *trans*-N-(azepan-1-yl)-1-(2,4-dichlorophenyl)-5-(4-methoxyphenyl)-4-methyl-4,5-dihydro-1H-pyrazole-3-carboxamide hydrochloride
- 273: *cis*-N-(azepan-1-yl)-1-(2-chlorophenyl)-5-(4-methoxyphenyl)-4-methyl-4,5-dihydro-1H-pyrazole-3-carboxamide
- 274: *trans*-N-(azepan-1-yl)-1-(2-chlorophenyl)-5-(4-methoxyphenyl)-4-methyl-4,5-dihydro-1H-pyrazole-3-carboxamide
- 275: *cis*-N-cycloheptyl-1-(2,4-dichlorophenyl)-5-(4-methoxyphenyl)-4-methyl-4,5-dihydro-1H-pyrazole-3-carboxamide
- 276: *cis*-1-(2-chlorophenyl)-N-cycloheptyl-5-(4-methoxyphenyl)-4-methyl-4,5-dihydro-1H-pyrazole-3-carboxamide
- 277: *cis*-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride
- 278: *cis*-1-(2-chlorophenyl)-N-(hexahydrocyclopenta[c]pyrrol-2(1H)-yl)-5-(4-methoxyphenyl)-4-methyl-4,5-dihydro-1H-pyrazole-3-carboxamide
- 279: *cis*-1-(2,4-dichlorophenyl)-N-(indolin-1-yl)-5-(4-methoxyphenyl)-4-methyl-4,5-dihydro-1H-pyrazole-3-carboxamide
- 280: *cis*-1-(2-chlorophenyl)-N-(indolin-1-yl)-5-(4-methoxyphenyl)-4-methyl-4,5-dihydro-1 H-pyrazole-3-carboxamide
- 281: (+)-cis-1-(2,4-dichlorophenyl)-N-((1S,2S)-2-hydroxycyclohexyl)-5-(4-methoxyphenyl)-4-methyl-4,5-dihydro-1 H-pyrazole-3-carboxamide
- 282: (-)-cis-1-(2,4-dichlorophenyl)-N-((1S,2S)-2-hydroxycyclohexyl)-5-(4-methoxyphenyl)-4-methyl-4,5-dihydro-1H-pyrazole-3-carboxamide
- 283: (+)-cis-1-(2-chlorophenyl)-N-((1S,2S)-2-hydroxycyclohexyl)-5-(4-methoxyphenyl)-4-methyl-4,5-dihydro-1 H-pyrazole-3-carboxamide
- 284: (-)-cis-1-(2-chlorophenyl)-N-((1S,2S)-2-hydroxycyclohexyl)-5-(4-methoxyphenyl)-4-methyl-4,5-dihydro-1H-pyrazole-3-carboxamide
- 285: *cis*-1-(2,4-dichlorophenyl)-N-(4-hydroxypiperidin-1-yl)-5-(4-methoxyphenyl)-4-methyl-4,5-dihydro-1H-pyrazole-3-carboxamide
- 286: *cis*-1-(2-chlorophenyl)-N-(4-hydroxypiperidin-1-yl)-5-(4-methoxyphenyl)-4-methyl-4,5-dihydro-1H-pyrazole-3-carboxamide
- 287: *cis*-1-(2,4-dichlorophenyl)-N-(3-hydroxypiperidin-1-yl)-5-(4-methoxyphenyl)-4-methyl-4,5-dihydro-1H-pyrazole-3-carboxamide
- 288: *cis*-1-(2-chlorophenyl)-N-(3-hydroxypiperidin-1-yl)-5-(4-methoxyphenyl)-4-methyl-4,5-dihydro-1H-pyrazole-3-carboxamide
- 289: *cis-*1-(2,4-dichlorophenyl)-N-(2-hydroxypiperidin-1-yl)-5-(4-methoxyphenyl)-4-methyl-4,5-dihydro-1H-pyrazole-3-carboxamide
- 290: *cis*-1-(2-chlorophenyl)-N-(2-hydroxypiperidin-1-yl)-5-(4-methoxyphenyl)-4-methyl-4,5-dihydro-1H-pyrazole-3-carboxamide
- 291: *cis*-1-(2,4-dichlorophenyl)-5-(4-methoxyphenyl)-4-methyl-N-(4-oxopiperidin-1-yl)-4,5-dihydro-1H-pyrazole-3-carboxamide
- 292: *cis*-1-(2-chlorophenyl)-5-(4-methoxyphenyl)-4-methyl-N-(4-oxopiperidin-1-yl)-4,5-dihydro-1H-pyrazole-3-carboxamide
- 293: *cis*-1-(2,4-dichlorophenyl)-5-(4-methoxyphenyl)-4-methyl-N-(3-oxopiperidin-1-yl)-4,5-dihydro-1H-pyrazole-3-carboxamide
- 294: *cis*-1-(2-chlorophenyl)-5-(4-methoxyphenyl)-4-methyl-N-(3-oxopiperidin-1-yl)-4,5-dihydro-1H-pyrazole-3-carboxamide
- 295: *cis*-1-(2,4-dichlorophenyl)-N-(3,4-dihydropyridin-1(2H)-yl)-5-(4-methoxyphenyl)-4-methyl-4,5-dihydro-1H-pyrazole-3-carboxamide
- 296: *cis*-1-(2-chlorophenyl)-N-(3,4-dihydropyridin-1(2H)-yl)-5-(4-methoxyphenyl)-4-methyl-4,5-dihydro-1H-pyrazole-3-carboxamide
- 297: *cis*-1-(2,4-dichlorophenyl)-N-(5,6-dihydropyridin-1(2H)-yl)-5-(4-methoxyphenyl)-4-methyl-4,5-dihydro-1H-pyrazole-3-carboxamide
- 298: *cis*-1-(2-chlorophenyl)-N-(5,6-dihydropyridin-1(2H)-yl)-5-(4-methoxyphenyl)-4-methyl-4,5-dihydro-1H-pyrazole-3-carboxamide
- 299: *cis-*1-(2,4-dichlorophenyl)-5-(4-methoxyphenyl)-N-(pipendin-1-yl)-4-(trifluoromethyl)-4,5-dihydro-1H-pyrazole-3-carboxamide
- 300: *trans*-1-(2,4-dichlorophenyl)-5-(4-methoxyphenyl)-N-(piperidin-1-yl)-4-(trifluoromethyl)-4,5-dihydro-1H-pyrazole-3-carboxamide
- 301: *cis*-N-(azepan-1-yl)-1-(2,4-dichlorophenyl)-5-(4-methoxyphenyl)-4-(trifluoromethyl)-4,5-dihydro-1H-pyrazole-3-carboxamide
- 302: *trans*-N-(azepan-1-yl)-1-(2,4-dichlorophenyl)-5-(4-methoxyphenyl)-4-(trifluoromethyl)-4,5-dihydro-1H-pyrazole-3-carboxamide
- 303: *cis*-1-(2-chlorophenyl)-5-(4-methoxyphenyl)-N-(piperidin-1-yl)-4-(trifluoromethyl)-4,5-dihydro-1H-pyrazole-3-carboxamide
- 304: trans-1-(2-chlorophenyl)-5-(4-methoxyphenyl)-N-(piperidin-1-yl)-4-(trifluoromethyl)-4,5-dihydro-1H-pyrazole-3-carboxamide
- 305: 1-(2,4-dichlorophenyl)-5-(4-methoxyphenyl)-4,4-dimethyl-N-(piperidin-1-yl)-4,5-dihydro-1 H-pyrazole-3-carboxamide
- 306: N-(azepan-1-yl)-1-(2,4-dichlorophenyl)-5-(4-methoxyphenyl)-4,4-dimethyl-4,5-dihydro-1 H-pyrazole-3-carboxamide
- 307: 1-(2-chlorophenyl)-5-(4-methoxyphenyl)-4,4-dimethyl-N-(piperidin-1-yl)-4,5-dihydro-1H-pyrazole-3-carboxamide
- 308: 1-(2,4-dichlorophenyl)-4-formyl-5-(4-methoxyphenyl)-N-(piperidin-1-yl)-4,5-dihydro-1 H-pyrazole-3-carboxamide
- 309: 1-(2,4-dichlorophenyl)-5-(4-methoxyphenyl)-3-(piperidin-1-ylcarbamoyl)-4,5-dihydro-1H-pyrazole-4-carboxylic acid
- 310: 1-(2,4-dichlorophenyl)-4-(2-hydroxyethyl)-5-(4-methoxyphenyl)-N-(piperidin-1-yl)-4,5-dihydro-1H-pyrazole-3-carboxamide
- 311: 1-(2,4-dichlorophenyl)-4-(fluoromethyl)-5-(4-methoxyphenyl)-N-(piperidin-1-yl)-4,5-dihydro-1H-pyrazole-3-carboxamide
- 312: *cis*-4-cyano-1-(2,4-dichlorophenyl)-5-(4-methoxyphenyl)-N-(piperidin-1-yl)-4,5-dihydro-1H-pyrazole-3-carboxamide
- 313: *trans*-4-cyano-1-(2,4-dichlorophenyl)-5-(4-methoxyphenyl)-N-(piperidin-1-yl)-4,5-dihydro-1H-pyrazole-3-carboxamide
- 314: *cis*-N-(azepan-1-yl)-4-cyano-1-(2,4-dichlorophenyl)-5-(4-methoxyphenyl)-4,5-dihydro-1H-pyrazole-3-carboxamide
- 315: *trans-*N-(azepan-1-yl)-4-cyano-1-(2,4-dichlorophenyl)-5-(4-methoxyphenyl)-4,5-dihydro-1H-pyrazole-3-carboxamide
- 316: *cis* -1-(2-chlorophenyl)-4-cyano-5-(4-methoxyphenyl)-N-(piperidin-1-yl)-4,5-dihydro-1H-pyrazole-3-carboxamide
- 317: *trans*-1-(2-chlorophenyl)-4-cyano-5-(4-methoxyphenyl)-N-(piperidin-1-yl)-4,5-dihydro-1 H-pyrazole-3-carboxamide
- 318: 1-(2,4-dichlorophenyl)-4-hydroxy-5-(4-methoxyphenyl)-N-(piperidin-1-yl)-4,5-dihydro-1 H-pyrazole-3-carboxamide
- 319: 1-(2-chlorophenyl)-4,4-difluoro-5-(4-methoxyphenyl)-N-(piperidin-1-yl)-4,5-dihydro-1 H-pyrazole-3-carboxamide
- 320: 1-(2-chlorophenyl)-4-formyl-5-(4-methoxyphenyl)-N-(piperidin-1-yl)-4,5-dihydro-1 H-pyrazole-3-carboxamide
- 321: 1-(2-chlorophenyl)-5-(4-methoxyphenyl)-3-(piperidin-1-ylcarbamoyl)-4,5-dihydro-1 H-pyrazole-4-carboxylic acid
- 322: 1-(2-chlorophenyl)-4-(2-hydroxyethyl)-5-(4-methoxyphenyl)-N-(piperidin-1-yl)-4,5-dihydro-1H-pyrazole-3-carboxamide
- 323: 1-(2-chlorophenyl)-4-(fluoromethyl)-5-(4-methoxyphenyl)-N-(piperidin-1-yl)-4,5-dihydro-1H-pyrazole-3-carboxamide
- 324: 1-(2-chlorophenyl)-4-hydroxy-5-(4-methoxyphenyl)-N-(piperidin-1-yl)-4,5-dihydro-1H-pyrazole-3-carboxamide
- 325: *cis*-1-(2,4-dichlorophenyl)-5-(4-ethoxyphenyl)-4-methyl-N-(piperidin-1-yl)-4,5-dihydro-1H-pyrazole-3-carboxamide
- 326: *trans*-1-(2,4-dichlorophenyl)-5-(4-ethoxyphenyl)-4-methyl-N-(piperidin-1-yl)-4,5-dihydro-1H-pyrazole-3-carboxamide
- 327: *cis*-1-(2-chlorophenyl)-5-(4-ethoxyphenyl)-4-methyl-N-(piperidin-1-yl)-4,5-dihydro-1 H-pyrazole-3-carboxamide
- 328: *trans*-1-(2-chlorophenyl)-5-(4-ethoxyphenyl)-4-methyl-N-(piperidin-1-yl)-4,5-dihydro-1H-pyrazole-3-carboxamide
- 329: *cis-*N-(azepan-1-yl)-1-(2,4-dichlorophenyl)-5-(4-ethoxyphenyl)-4-methyl-4,5-dihydro-1H-pyrazole-3-carboxamide
- 330: *trans*-N-(azepan-1-yl)-1-(2,4-dichlorophenyl)-5-(4-ethoxyphenyl)-4-methyl-4,5-dihydro-1H-pyrazole-3-carboxamide
- 331: *cis*-N-(azepan-1-yl)-1-(2-chlorophenyl)-5-(4-ethoxyphenyl)-4-methyl-4,5-dihydro-1H-pyrazole-3-carboxamide
- 332: *trans-*N-(azepan-1-yl)-1-(2-chlorophenyl)-5-(4-ethoxyphenyl)-4-methyl-4,5-dihydro-1H-pyrazole-3-carboxamide
- 333: *cis-*N-cycloheptyl-1-(2,4-dichlorophenyl)-5-(4-ethoxyphenyl)-4-methyl-4,5-dihydro-1H-pyrazole-3-carboxamide
- 334: *cis-*1-(2-chlorophenyl)-N-cycloheptyl-5-(4-ethoxyphenyl)-4-methyl-4,5-dihydro-1H-pyrazole-3-carboxamide
- 335: *cis*-1-(2,4-dichlorophenyl)-5-(4-ethoxyphenyl)-N-(hexahydrocyclopenta[c]pyrrol-2(1H)-yl)-4-methyl-4,5-dihydro-1H-pyrazole-3-carboxamide
- 336: *cis*-1-(2-chlorophenyl)-5-(4-ethoxyphenyl)-N-(hexahydrocyclopenta[c]pyrrol-2(1 H)-yl)-4-methyl-4,5-dihydro-1 H-pyrazole-3-carboxamide
- 337: *cis*-1-(2,4-dichlorophenyl)-5-(4-ethoxyphenyl)-N-(indolin-1-yl)-4-methyl-4,5-dihydro-1H-pyrazole-3-carboxamide
- 338: *cis-*1-(2-chlorophenyl)-5-(4-ethoxyphenyl)-N-(indolin-1-yl)-4-methyl-4,5-dihydro-1H-pyrazole-3-carboxamide
- 339: (+)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide
- 340: (-)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide
- 341: (+)-cis-1-(2-chlorophenyl)-5-(4-ethoxyphenyl)-N-((1S,2S)-2-hydroxycyclohexyl)-4-methyl-4,5-dihydro-1 H-pyrazole-3-carboxamide
- 342: (-)-cis-1-(2-chlorophenyl)-5-(4-ethoxyphenyl)-N-((1S,2S)-2-hydroxycyclohexyl)-4-methyl-4,5-dihydro-1 H-pyrazole-3-carboxamide
- 343: *cis*-1-(2,4-dichlorophenyl)-5-(4-hydroxyphenyl)-4-methyl-N-(piperidin-1-yl)-4,5-dihydro-1 H-pyrazole-3-carboxamide
- 344: *trans*-1-(2,4-dichlorophenyl)-5-(4-hydroxyphenyl)-4-methyl-N-(piperidin-1-yl)-4,5-dihydro-1H-pyrazole-3-carboxamide
- 345: *cis*-1-(2-chlorophenyl)-5-(4-hydroxyphenyl)-4-methyl-N-(piperidin-1-yl)-4,5-dihydro-1H-pyrazole-3-carboxamide
- 346: *trans*-1-(2-chlorophenyl)-5-(4-hydroxyphenyl)-4-methyl-N-(piperidin-1-yl)-4,5-dihydro-1H-pyrazole-3-carboxamide
- 347: *cis*-N-(azepan-1-yl)-1-(2,4-dichlorophenyl)-5-(4-hydroxyphenyl)-4-methyl-4,5-dihydro-1H-pyrazole-3-carboxamide
- 348: *trans*-N-(azepan-1-yl)-1-(2,4-dichlorophenyl)-5-(4-hydroxyphenyl)-4-methyl-4,5-dihydro-1 H-pyrazole-3-carboxamide
- 349: *cis-*N-(azepan-1-yl)-1-(2-chlorophenyl)-5-(4-hydroxyphenyl)-4-methyl-4,5-dihydro-1 H-pyrazole-3-carboxamide
- 350: *trans*-N-(azepan-1-yl)-1-(2-chlorophenyl)-5-(4-hydroxyphenyl)-4-methyl-4,5-dihydro-1 H-pyrazole-3-carboxamide
- 351: *cis*-N-cycloheptyl-1-(2,4-dichlorophenyl)-5-(4-hydroxyphenyl)-4-methyl-4,5-dihydro-1 H-pyrazole-3-carboxamide
- 352: *cis*-1-(2-chlorophenyl)-N-cycloheptyl-5-(4-hydroxyphenyl)-4-methyl-4,5-dihydro-1 H-pyrazole-3-carboxamide
- 353: *cis*-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride
- 354: *cis*-1-(2-chlorophenyl)-N-(hexahydrocyclopenta[c]pyrrol-2(1H)-yl)-5-(4-hydroxyphenyl)-4-methyl-4,5-dihydro-1H-pyrazole-3-carboxamide
- 355: *cis*-1-(2,4-dichlorophenyl)-5-(4-hydroxyphenyl)-N-(indolin-1-yl)-4-methyl-4,5-dihydro-1H-pyrazole-3-carboxamide
- 356: *cis*-1-(2-chlorophenyl)-5-(4-hydroxyphenyl)-N-(indolin-1-yl)-4-methyl-4,5-dihydro-1H-pyrazole-3-carboxamide
- 357: (+)-cis-1-(2,4-dichlorophenyl)-N-((1S,2S)-2-hydroxycyclohexyl)-5-(4-hydroxyphenyl)-4-methyl-4,5-dihydro-1H-pyrazole-3-carboxamide
- 358: (-)-cis-1-(2,4-dichlorophenyl)-N-((1S,2S)-2-hydroxycyclohexyl)-5-(4-hydroxyphenyl)-4-methyl-4,5-dihydro-1H-pyrazole-3-carboxamide
- 359: (+)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide
- 360: (-)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide
- 361: *cis*-5-(5-chlorothiophen-2-yl)-1-(2,4-dichlorophenyl)-4-methyl-4,5-dihydro-1H-pyrazole-3-carboxylic acid
- 362: *trans*-5-(5-chlorothiophen-2-yl)-1-(2,4-dichlorophenyl)-4-methyl-4,5-dihydro-1 H-pyrazole-3-carboxylic acid
- 363: *cis*-1-(2-chlorophenyl)-5-(5-chlorothiophen-2-yl)-4-methyl-4,5-dihydro-1H-pyrazole-3-carboxylic acid
- 364: *trans*-1-(2-chlorophenyl)-5-(5-chlorothiophen-2-yl)-4-methyl-4,5-dihydro-1H-pyrazole-3-carboxylic acid
- 365: *cis*-5-(5-bromothiophen-2-yl)-1-(2,4-dichlorophenyl)-4-ethyl-4,5-dihydro-1H-pyrazole-3-carboxylic acid
- 366: *trans-*5-(5-bromothiophen-2-yl)-1-(2,4-dichlorophenyl)-4-ethyl-4,5-dihydro-1H-pyrazole-3-carboxylic acid
- 367: *cis*-1-(2,4-dichlorophenyl)-4-methyl-5-(thiophen-2-yl)-4,5-dihydro-1H-pyrazole-3-carboxylic acid
- 368: *trans*-1-(2,4-dichlorophenyl)-4-methyl-5-(thiophen-2-yl)-4,5-dihydro-1H-pyrazole-3-carboxylic acid
- 369: *cis-1*-(2,4-dichlorophenyl)-4-methyl-N-(piperidin-1-yl)-5-(thiophen-2-yl)-4,5-dihydro-1H-pyrazole-3-carboxamide
- 370: *trans*-1-(2,4-dichlorophenyl)-4-methyl-N-(piperidin-1-yl)-5-(thiophen-2-yl)-4,5-dihydro-1H-pyrazole-3-carboxamide
- 371: *cis*-1-(2-chlorophenyl)-4-methyl-N-(piperidin-1-yl)-5-(thiophen-2-yl)-4,5-dihydro-1H-pyrazole-3-carboxamide
- 372: *trans-*1-(2-chlorophenyl)-4-methyl-N-(piperidin-1-yl)-5-(thiophen-2-yl)-4,5-dihydro-1H-pyrazole-3-carboxamide
- 373: *cis-*1-(2,4-dichlorophenyl)-4-methyl-5-phenyl-4,5-dihydro-1H-pyrazole-3-carboxylic acid
- 374: *trans*-1-(2,4-dichlorophenyl)-4-methyl-5-phenyl-4,5-dihydro-1H-pyrazole-3-carboxylic acid
- 375: *cis*-1-(2,4-dichlorophenyl)-4-methyl-5-phenyl-N-(piperidin-1-yl)-4,5-dihydro-1H-pyrazole-3-carboxamide
- 376: *trans* -1-(2,4-dichlorophenyl)-4-methyl-5-phenyl-N-(piperidin-1-yl)-4,5-dihydro-1H-pyrazole-3-carboxamide
- 377: *cis*-1-(2-chlorophenyl)-4-methyl-5-phenyl-N-(piperidin-1-yl)-4,5-dihydro-1H-pyrazole-3-carboxamide
- 378: *trans*-1-(2-chlorophenyl)-4-methyl-5-phenyl-N-(piperidin-1-yl)-4,5-dihydro-1H-pyrazole-3-carboxamide
- 379: *cis*-5-(4-chlorophenyl)-1-(2,4-dichlorophenyl)-4-methyl-N-(piperidin-1-yl)-4,5-dihydro-1H-pyrazole-3-carbothioamide hydrochloride
- 380: *trans*-5-(4-chlorophenyl)-1-(2,4-dichlorophenyl)-4-methyl-N-(piperidin-1-yl)-4,5-dihydro-1H-pyrazole-3-carbothioamide
- 381: *cis*-1-(2-chlorophenyl)-5-(4-chlorophenyl)-4-methyl-N-(piperidin-1-yl)-4,5-dihydro-1H-pyrazole-3-carbothioamide
- 382: *trans*-1-(2-chlorophenyl)-5-(4-chlorophenyl)-4-methyl-N-(piperidin-1-yl)-4,5-dihydro-1 H-pyrazole-3-carbothioamide
- 383: *cis*-N-(azepan-1-yl)-5-(4-chlorophenyl)-1-(2,4-dichlorophenyl)-4-methyl-4,5-dihydro-1H-pyrazole-3-carbothioamide
- 384: *cis*-N-(azepan-1-yl)-1-(2-chlorophenyl)-5-(4-chlorophenyl)-4-methyl-4,5-dihydro-1H-pyrazole-3-carbothioamide
- 385: *cis-*5-(4-bromophenyl)-1-(2,4-dichlorophenyl)-4-methyl-N-(piperidin-1-yl)-4,5-dihydro-1H-pyrazole-3-carbothioamide
- 386: *trans-*5-(4-bromophenyl)-1-(2,4-dichlorophenyl)-4-methyl-N-(piperidin-1-yl)-4,5-dihydro-1H-pyrazole-3-carbothioamide
- 387: *cis*-5-(4-bromophenyl)-1-(2-chlorophenyl)-4-methyl-N-(piperidin-1-yl)-4,5-dihydro-1H-pyrazole-3-carbothioamide
- 388: *trans*-5-(4-bromophenyl)-1-(2-chlorophenyl)-4-methyl-N-(piperidin-1-yl)-4,5-dihydro-1 H-pyrazole-3-carbothioamide
- 389: *cis*-1-(2,4-dichlorophenyl)-5-(4-fluorophenyl)-4-methyl-N-(piperidin-1-yl)-4,5-dihydro-1H-pyrazole-3-carbothioamide
- 390: *trans*-1-(2,4-dichlorophenyl)-5-(4-fluorophenyl)-4-methyl-N-(piperidin-1-yl)-4,5-dihydro-1H-pyrazole-3-carbothioamide
- 391: *cis*-1-(2-chlorophenyl)-5-(4-fluorophenyl)-4-methyl-N-(piperidin-1-yl)-4,5-dihydro-1H-pyrazole-3-carbothioamide
- 392: *trans*-1-(2-chlorophenyl)-5-(4-fluorophenyl)-4-methyl-N-(piperidin-1-yl)-4,5-dihydro-1H-pyrazole-3-carbothioamide
- 393: *cis-*1-(2,4-dichlorophenyl)-5-(4-methoxyphenyl)-4-methyl-N-(piperidin-1-yl)-4,5-dihydro-1H-pyrazole-3-carbothioamide
- 394: *cis*-N-(azepan-1-yl)-1-(2,4-dichlorophenyl)-5-(4-methoxyphenyl)-4-methyl-4,5-dihydro-1H-pyrazole-3-carbothioamide
- 395: *trans*-1-(2,4-dichlorophenyl)-5-(4-methoxyphenyl)-4-methyl-N-(piperidin-1-yl)-4,5-dihydro-1H-pyrazole-3-carbothioamide
- 396: *cis*-1-(2-chlorophenyl)-5-(4-methoxyphenyl)-4-methyl-N-(piperidin-1-yl)-4,5-dihydro-1H-pyrazole-3-carbothioamide
- 397: cis-N-(azepan-1-yl)-1-(2-chlorophenyl)-5-(4-methoxyphenyl)-4-methyl-4,5-dihydro-1H-pyrazole-3-carbothioamide
- 398: *trans*-1-(2-chlorophenyl)-5-(4-methoxyphenyl)-4-methyl-N-(piperidin-1-yl)-4,5-dihydro-1H-pyrazole-3-carbothioamide
- 399: *cis-*1-(2,4-dichlorophenyl)-5-(4-ethoxyphenyl)-4-methyl-N-(piperidin-1-yl)-4,5-dihydro-1H-pyrazole-3-ca rbothioa mide
- 400: *trans*-1-(2,4-dichlorophenyl)-5-(4-ethoxyphenyl)-4-methyl-N-(piperidin-1-yl)-4,5-dihydro-1 H-pyrazole-3-carbothioamide
- 401: *cis-*1-(2-chlorophenyl)-5-(4-ethoxyphenyl)-4-methyl-N-(piperidin-1-yl)-4,5-dihydro-1H-pyrazole-3-carbothioamide
- 402: *trans*-1-(2-chlorophenyl)-5-(4-ethoxyphenyl)-4-methyl-N-(piperidin-1-yl)-4,5-dihydro-1H-pyrazole-3-carbothioamide
- 403: *cis*-1-(2,4-dichlorophenyl)-5-(4-hydroxyphenyl)-4-methyl-N-(piperidin-1-yl)-4,5-dihydro-1H-pyrazole-3-carbothioamide
- 404: *trans*-1-(2,4-dichlorophenyl)-5-(4-hydroxyphenyl)-4-methyl-N-(piperidin-1-yl)-4,5-dihydro-1H-pyrazole-3-carbothioamide
- 405: *cis*-1-(2-chorophenyl)-5-(4-hydroxyphenyl)-4-methyl-N-(piperidin-1-yl)-4,5-dihydro-1H-pyrazole-3-carbothioamide
- 406: *trans*-1-(2-chlorophenyl)-5-(4-hydroxyphenyl)-4-methyl-N-(piperidin-1-yl)-4,5-dihydro-1H-pyrazole-3-carbothioamide
- 407: 7-(4-Chloro-phenyl)-6-(2,4-dichlorophenyl)-5,6-diaza-spiro[2.4]hept-4-ene-4-carboxylic acid piperidin-1-ylamide
- 408: 6-(2,4-Dichloro-phenyl)-7-(4-methoxy-phenyl)-5,6-diaza-spiro[2.4]hept-4-ene-4-carboxylic acid piperidin-1-ylamide
- 409: (+)-*cis*-N-((1S,2S)-2-(benzyloxy)cyclohexyl)-5-(4-chlorophenyl)-1-(2,4-dichlorophenyl)-4-methyl-4,5-dihydro-1 H-pyrazole-3-carboxamide
- [410]: *cis*-1-(2,4-dichlorophenyl)-5-(4-methoxyphenyl)-4-methyl-N-(piperidin-1-yl)-4,5-dihydro-1 H-pyrazole-3-carboxamide
- [411]: *cis*-1-(2,4-dichlorophenyl)-5-(4-methoxyphenyl)-4-methyl-N-(piperidin-1-yl)-4,5-dihydro-1 H-pyrazole-3-carboxamide
- [412]: (4R,5S)-1-(2,4-dichlorophenyl)-5-(4-methoxyphenyl)-4-methyl-4,5-dihydro-1H-pyrazole-3-carboxylic acid
- [413]: *cis*-5-(4-bromophenyl)-1-(2,4-dichlorophenyl)-4-methyl-4,5-dihydro-1H-pyrazole-3-carboxylic acid
- [414]: cis-N-(azepan-1-yl)-1-(2,4-dichlorophenyl)-5-(4-fluorophenyl)-4-methyl-4,5-dihydro-1H-pyrazole-3-carboxamide
- 415: *cis-*5-(4-bromophenyl)-1-(2,4-dichlorophenyl)-N-(indolin-1-yl)-4-methyl-4,5-dihydro-1H-pyrazole-3-carboxamide
- 416: *trans*-ethyl 5-(4-chlorophenyl)-1-(2,4-dichlorophenyl)-4-(trifluoromethyl)-4,5-dihydro-1H-pyrazole-3-carboxylate
- 417: *cis*-1-(2,4-dichlorophenyl)-5-(4-fluorophenyl)-4-methyl-N-(piperidin-1-yl)-4,5-dihydro-1H-pyrazole-3-carboxamide hydrochloride
- 418: *cis*-5-(4-bromophenyl)-1-(2,4-dichlorophenyl)-4-methyl-N-(piperidin-1-yl)-4,5-dihydro-1H-pyrazole-3-carboxamide hydrochloride
- 419: (-)-*cis*-5-(4-chlorophenyl)-1-(2,4-dichlorophenyl)-4-methyl-N-(piperidin-1-yl)-4,5-dihydro-1H-pyrazole-3-carboxamide
- 420: (+)-*cis*-5-(4-chlorophenyl)-1-(2,4-dichlorophenyl)-4-methyl-N-(piperidin-1-yl)-4,5-dihydro-1H-pyrazole-3-carboxamide
- 421: *trans-*ethyl 1-(2,4-dichlorophenyl)-4-methyl-5-phenyl-4,5-dihydro-1H-pyrazole-3-carboxylate
- 422: *cis*-5-(4-chlorophenyl)-1-(2,4-dichlorophenyl)-4-methyl-N-((1R,2S,4R)-1,7,7-trimethylbicyclo[2.2.1]heptan-2-yl)-4,5-dihydro-1H-pyrazole-3-carboxamide
- 423: *cis*-5-(4-chlorophenyl)-1-(2,4-dichlorophenyl)-4-methyl-N-(3-methylcyclohexyl)-4,5-dihydro-1H-pyrazole-3-carboxamide
- 424: *cis*-5-(4-chlorophenyl)-1-(2,4-dichlorophenyl)-4-methyl-N-(2-methylcyclohexyl)-4,5-dihydro-1H-pyrazole-3-carboxamide
- 426: *trans-5*-(5-chlorothiophen-2-yl)-1-(2,4-dichlorophenyl)-4-methyl-N-(piperidin-1-yl)-4,5-dihydro-1H-pyrazole-3-carboxamide hydrochloride
- 427: *cis*-5-(5-chlorothiophen-2-yl)-1-(2,4-dichlorophenyl)-4-methyl-N-(piperidin-1-yl)-4,5-dihydro-1H-pyrazole-3-carboxamide hydrochloride
- 428: *cis*-5-(4-bromophenyl)-1-(2,4-dichlorophenyl)-4-ethyl-N-(piperidin-1-yl)-4,5-dihydro-1H-pyrazole-3-carboxamide hydrochloride
- [429]: 1-(2,4-dichlorophenyl)-4-methyl-5-phenyl-4,5-dihydro-1H-pyrazole-3-carboxylic acid
- [430]: cis-1-(2,4-dichlorophenyl)-4-methyl-N-(piperidin-1-yl)-5-(thiophen-2-yl)-4,5-dihydro-1H-pyrazole-3-carboxamide hydrochloride
- [431]: trans-5-(4-ch)orophenyl)-1-(2,4-dichiorophenyl)-4-methyl-N-(piperidin-1-yl)-4,5-dihydro-1H-pyrazole-3-carboxamide
- 432: *cis-*5-(4-chlorophenyl)-1-(2,4-dichlorophenyl)-4-ethyl-N-(piperidin-1-yl)-4,5-dihydro-1H-pyrazole-3-carboxamide
- 434: *cis-*5-(4-chlorophenyl)-1-(2,4-dichlorophenyl)-4-methyl-N-(piperidin-1-yl)-4,5-dihydro-1H-pyrazole-3-carboxamide
- 436: (+)*-cis-N-*((1S,2S)-2-(benzyloxy)cyclohexyl)-5-(4-chlorophenyl)-1-(2,4-dichlorophenyl)-4-methyl-4,5-dihydro-1H-pyrazole-3-carboxamide
- 437: (-)-*cis*-N-((1R,2R)-2-(benzyloxy)cyclohexyl)-5-(4-chlorophenyl)-1-(2,4-dichlorophenyl)-4-methyl-4,5-dihydro-1H-pyrazole-3-carboxamide
- 438: *cis*-N-(azocan-1-yl)-1-(2,4-dichlorophenyl)-5-(4-methoxyphenyl)-4-methyl-4,5-dihydro-1H-pyrazole-3-carboxamide
- 439: *cis*-N-(azocan-1-yl)-1-(2-chlorophenyl)-5-(4-methoxyphenyl)-4-methyl-4,5-dihydro-1H-pyrazole-3-carboxamide
- 440: *cis*-N-(azocan-1-yl)-5-(4-bromophenyl)-1-(2,4-dichlorophenyl)-4-methyl-4,5-dihydro-1 H-pyrazole-3-carboxamide
- 441: *cis*-N-(azocan-1-yl)-5-(4-bromophenyl)-1-(2-chlorophenyl)-4-methyl-4,5-dihydro-1H-pyrazole-3-carboxamide
- 442: *cis*-N-(azocan-1-yl)-1-(2,4-dichlorophenyl)-5-(4-fluorophenyl)-4-methyl-4,5-dihydro-1H-pyrazole-3-carboxamide
- 443: *cis*-N-(azocan-1-yl)-1-(2-chlorophenyl)-5-(4-fluorophenyl)-4-methyl-4,5-dihydro-1H-pyrazole-3-carboxamide
- 444: (+)-*cis*-5-(4-Chlorophenyl)-1-(2,4-dichlorophenyl)-4-methyl-4,5-dihydro-1H-pyrazole-3-carboxylic acid azepan-1-ylamide hydrochloride
- 445: (-)*-cis-*(4-Chlorophenyl)-1-(2,4-dichlorophenyl)-4-methyl-4,5-dihydro-1H-pyrazole-3-carboxylic acid azepan-1-ylamide hydrochloride
- [446]: *cis*-5-(4-chlorophenyl)-1-(2,4-dichlorophenyl)-4-methyl-N-(piperidin-1-yl)-4,5-dihydro-1H-pyrazole-3-carboxamide N-oxide
- [447]: *cis*-5-(4-chlorophenyl)-1-(2-chlorophenyl)-4-methyl-N-(piperidin-1-yl)-4,5-dihydro-1H-pyrazole-3-carboxamide N-oxide
- [448]: *cis*-N-(azepan-1-yl)-5-(4-chlorophenyl)-1-(2,4-dichlorophenyl)-4-methyl-4,5-dihydro-1H-pyrazole-3-carboxamide N-oxide
- [449]: *cis-*N-(azepan-1-yl)-5-(4-chlorophenyl)-1-(2-chlorophenyl)-4-methyl-4,5-dihydro-1H-pyrazole-3-carboxamide N-oxide
- [450]: *cis*-1-(2,4-dichlorophenyl)-5-(4-methoxyphenyl)-4-methyl-N-(piperidin-1-yl)-4,5-dihydro-1H-pyrazole-3-carboxamide N-oxide and
- [451]: *cis*-1-(2-chlorophenyl)-5-(4-methoxyphenyl)-4-methyl-N-(piperidin-1-yl)-4,5-dihydro-1H-pyrazole-3-carboxamide N-oxide;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

Also preferred are Indoline-substituted pyrazoline compounds (A) of subgroup A5.1 of general formulas IV, IVa or IVb, 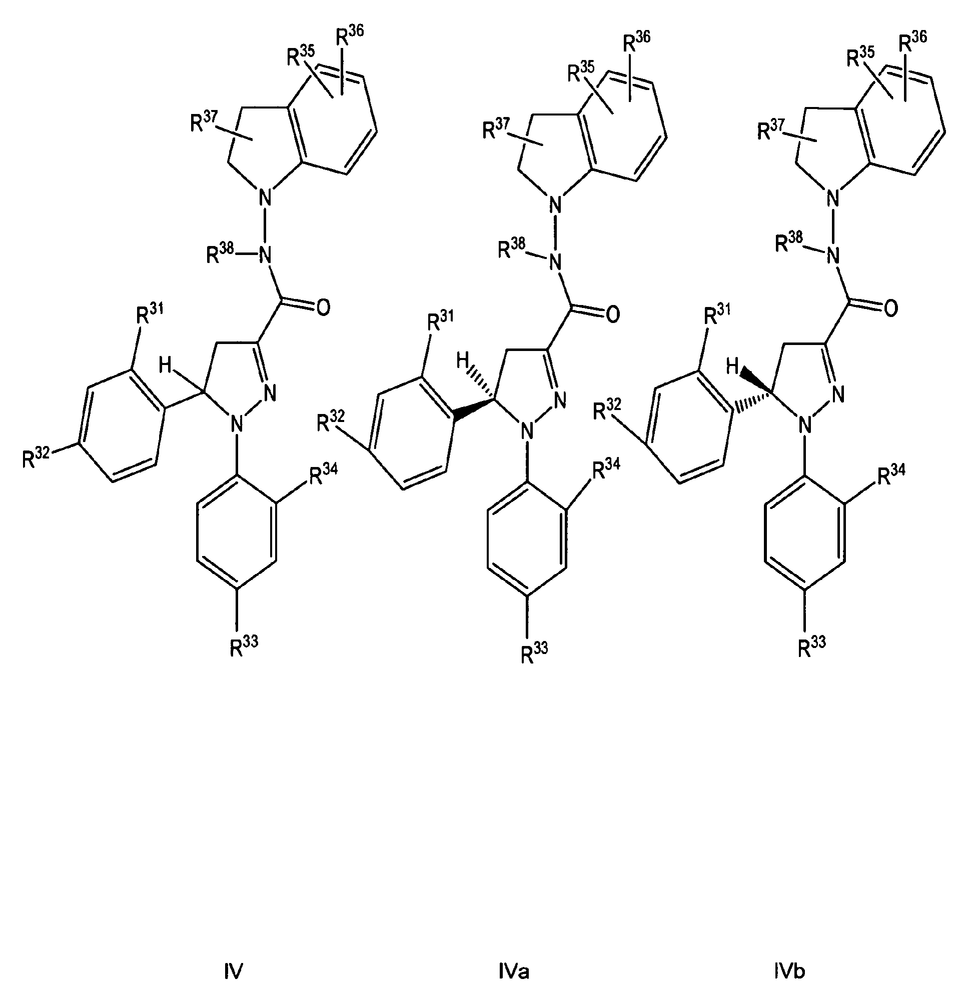 wherein
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
- R³⁸: representing a hydrogen atom or a branched or linear C₁₋₃-alkyl group;
optionally in the form shown or in form of the acid or base or in form of a salt, especially a physiologically acceptable salt, or in form of a solvate, especially a hydrate or in form of a corresponding N-oxide thereof;
or Indoline-substituted pyrazoline compounds (A) of subgroup A5.1 of general formulas IVc, IVd or IVe 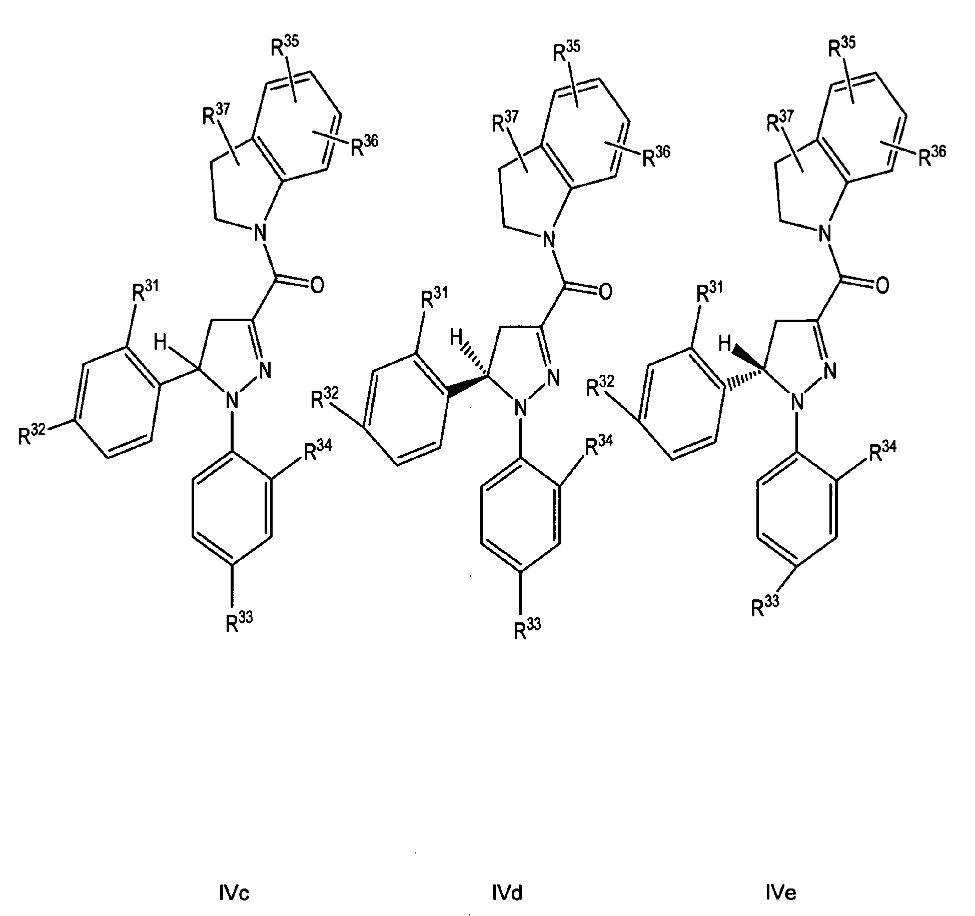 wherein
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- p: is 1 or 2;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form shown or in form of the acid or base or in form of a salt, especially a physiologically acceptable salt, or in form of a solvate, especially a hydrate or in form of a corresponding N-oxide thereof.

Especially preferred compounds of subgroup A5.1 are selected from
● 5-(4-chlorophenyl)-1-(2,4-dichlorophenyl)-N-(indolin-1-yl)-4,5-dihydro-1H-pyrazole-3-ca rboxa m ide,
● (R)-5-(4-chlorophenyl)-1-(2,4-dichlorophenyl)-N-(indolin-1-y1)-4,5-dihydro-1H-pyrazole-3-carboxamide.
● (S)-5-(4-chlorophenylyl-(2,4-dichlorophenyl)-N-(indolin-1-yl)-4,5-dihydro-1H-pyrazole-3-carboxamide,
● 5-(4-bromophenyl)-1-(2,4-dichlorophenyl)-N-(indolin-1-yl)-4,5-dihydro-1 H-pyrazole-3-carboxamide,
● 1-(2,4-dichlorophenyl)-N-(indolin-1-yl)-5-(4-methoxyphenyl)-4,5-dihydro-1H-pyrazole-3-carboxamide.

Also preferred are cykloalkane-substituted pyrazoline compounds (A) of subgroup A5.2 of general formulas IV, IVa or IVb 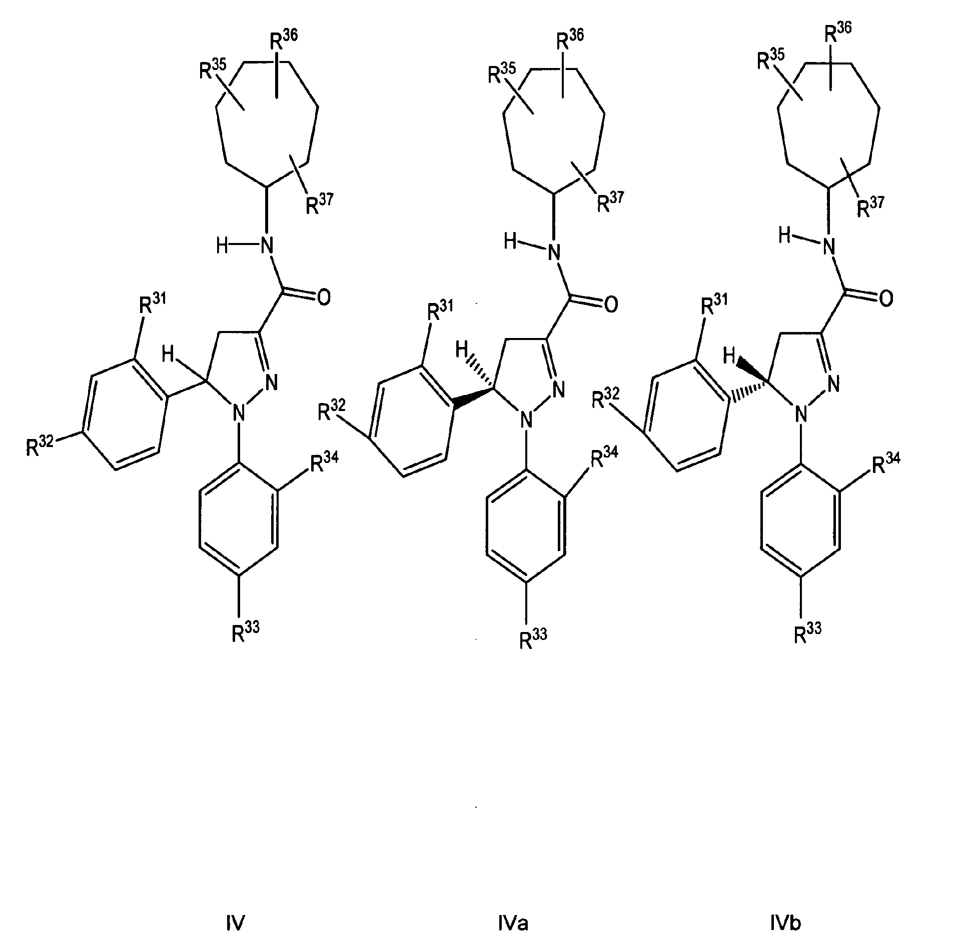 wherein
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of substituted or unsubstituted, branched or linear, saturated or unsaturated C₈₋₂₀-alkyl or C(O)-C₁₋₂₀-alkyl; substituted or unsubstituted aryl, alkyl-aryl, heterocyclyl or alkyl-heterocyclyl; substituted or unsubstituted C(O)-aryl, C(O)-alkyl-aryl, C(O)-heterocyclyl or C(O)-alkyl-heterocyclyl;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form shown or in form of the acid or base or in form of a salt, especially a physiologically acceptable salt, or in form of a solvate, especially a hydrate or in form of a corresponding N-oxide thereof.

Especially preferred compounds of subgroup A5.2 are selected from
- 5-(4-chlorophenyl)-N-cycloheptyl-1-(2,4-dichlorophenyl)-4,5-dihydro-1H-pyrazole-3-carboxamide,
- (R)- 5-(4-chlorophenyl)-N-cycloheptyl-1-(2,4-dichlorophenyl)-4,5-dihydro-1H-pyrazole-3-carboxamide,
- (S)- 5-(4-chlorophenyl)-N-cycloheptyl-1-(2,4-dichlorophenyl)-4,5-dihydro-1H-pyrazole-3-carboxamide,
- 5-(4-chlorophenyl)-N-(cycloheptylmethyl)-1-(2,4-dichlorophenyl)-4,5-dihydro-1H-pyrazole-3-carboxamide,
- 5-(4-bromophenyl)-N-cycloheptyl-1-(2,4-dichlorophenyl)-4,5-dihydro-1H-pyrazole-3-carboxamide,
- 5-(4-fluorophenyl)-N-cycloheptyl-1-(2,4-dichlorophenyl)-4,5-dihydro-1H-pyrazole-3-carboxamide,
- 5-(4-methoxyphenyl)-N-cycloheptyl-1-(2,4-dichlorophenyl)-4,5-dihydro-1H-pyrazole-3-carboxamide.

Also preferred are Octahydropentalene -substituted pyrazoline compounds (A) of subgroup A5.3 of general formulas IV, IVa or IVb 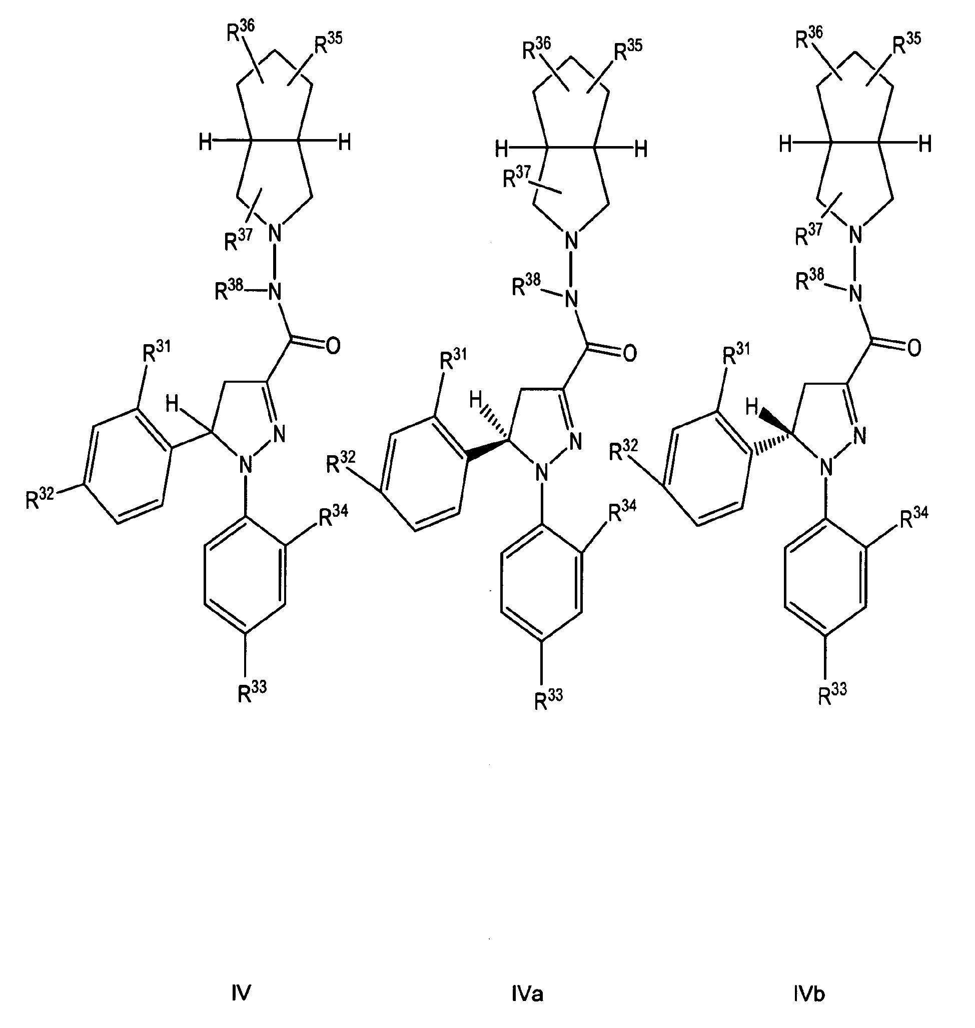 wherein
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
- R³⁸: representing a hydrogen atom or a branched or linear C₁₋₃-alkyl group;
optionally in the form shown or in form of the acid or base or in form of a salt, especially a physiologically acceptable salt, or in form of a solvate, especially a hydrate or in form of a corresponding N-oxide thereof;
or
octahydropentalene-substituted pyrazoline compounds of general formulas IVc, IVd or IVe 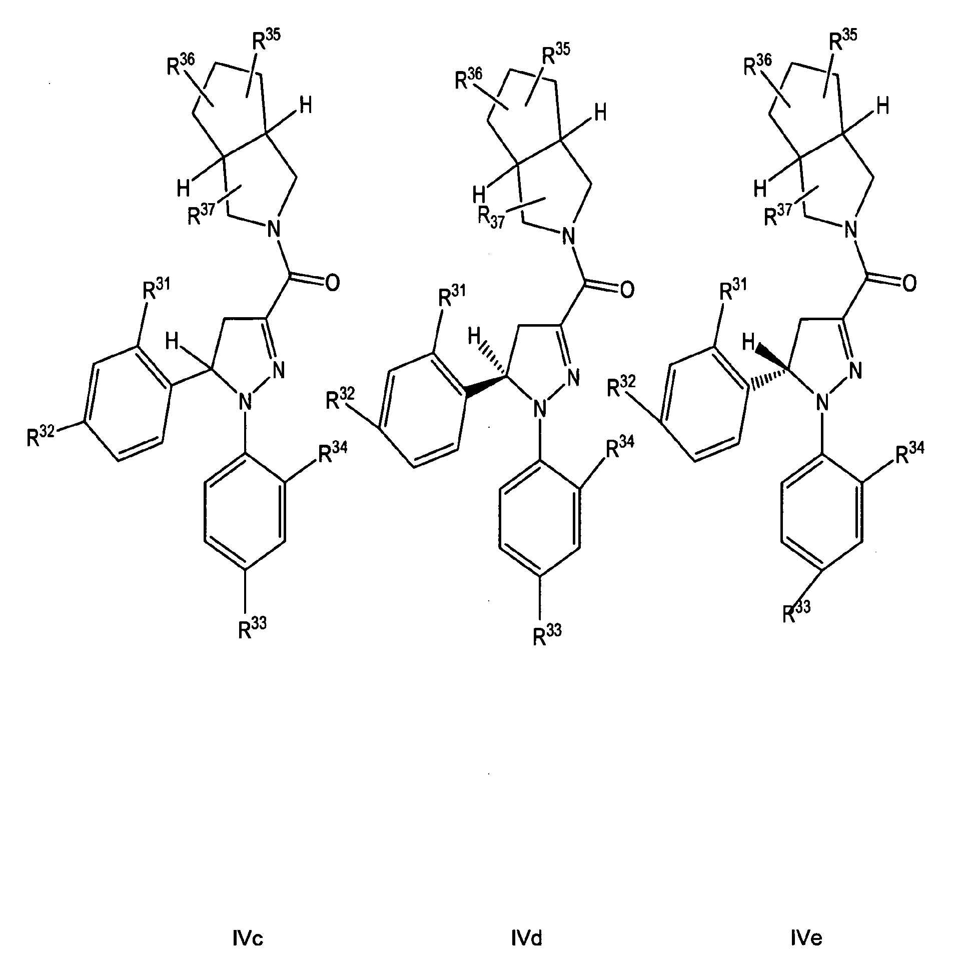 wherein
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- R³⁵, R³⁶ and R³⁷: are independently from one another selected from, H, F, CI, Br, I, OH, SH, C₁₋₄alkyl, C₁₋₄alkoxy, CF₃, CHF₂, CH₂F, OCF₃, a keto-group, NO₂ or NH₂;
optionally in the form shown or in form of the acid or base or in form of a salt, especially a physiologically acceptable salt, or in form of a solvate, especially a hydrate or in form of a corresponding N-oxide thereof.

Especially preferred compounds of subgroup A5.3 are selected from
- 5-(4-chlorophenyl)-1-(2,4-dichlorophenyl)-N-(hexahydrocyclopenta[c]pyrrol-2(1H)-yl)-4,5-dihydro-1H-pyrazole-3-carboxamide,
- (R)-5-(4-chlorophenyl)-1-(2,4-dichlorophenyl)-N-(hexahydrocyclopenta[c]pyrrol-2(1H)-yl)-4,5-dihydro-1H-pyrazole-3-carboxamide,
- (S)-5-(4-chorophenyl)-1-(2,4-dichlorophenyl)-N-(hexahydrocyclopenta[c]pyrrol-2(1H)-yl)-4,5-dihydro-1H-pyrazole-3-carboxamide,
- 5-(4-bromophenyl)-1-(2,4-dichlorophenyl)-N-(hexahydrocyclopenta[c]pyrrol-2(1H)-yl)-4,5-dihydro-1H-pyrazole-3-carboxamide,
- 1-(2,4-d ichlorophenyl)-5-(4-fluorophenyl)-N-(hexahydrocyclopenta[c]pyrrol-2(1H)-yl)-4,5-dihydro-1H-pyrazole-3-carboxamide,
1-(2,4-dichlorophenyl)-N-(hexahydrocyclopenta[c]pyrrol-2(1H)-yl)-5-(4-methoxyphenyl)-4,5-dihydro-1H-pyrazole-3-carboxamide.

Also preferred are Azepane-substituted pyrazoline compounds (A) of subgroup A5.4 of general formulas IV, IVa or IVb 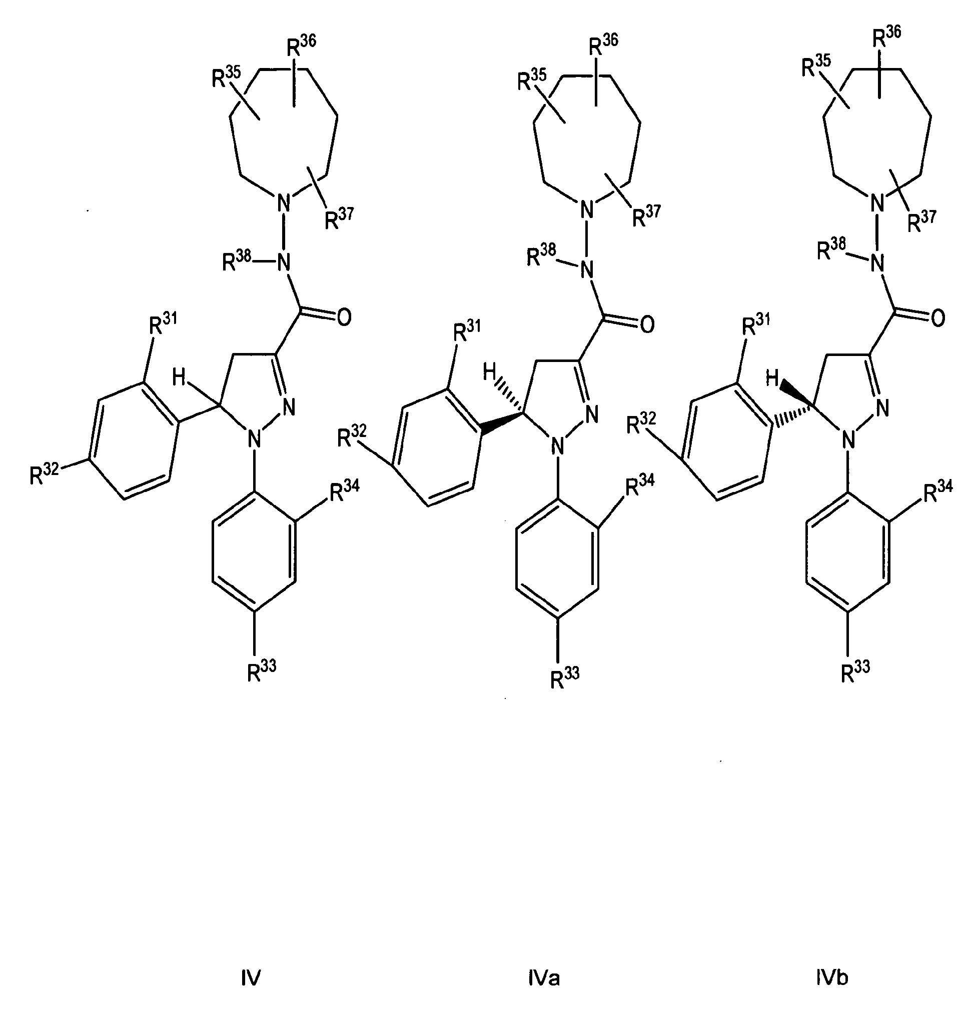 wherein
- R³¹, R³², R³³ and R³⁴: independently of one another represent: H; branched or linear C₁-₃-alkyl or branched or linear C₁-₃-alkoxy, phenyl, hydroxy, chloro, bromo, fluoro, iodo, SH, trifluoromethyl, trifluoromethylthio, trifluoromethoxy, methylsuffonyl, carboxyl, trifluoromethylsulfonyl, cyano, carbamoyl, sulfamoyl and acetyl; O-P, with P denominating a prodrug group consisting of aryl, C₈-₂₀-alkyl, heteroaryl, C(O)-aryl, C(O)-heteroaryl, C(O)-C₁₋₂₀-alkyl;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
- R³⁸: representing a hydrogen atom or a branched or linear C₁₋₃-alkyl group;
optionally in the form shown or in form of the acid or base or in form of a salt, especially a physiologically acceptable salt, or in form of a solvate, especially a hydrate or in form of a corresponding N-oxide thereof;
or
azepane substituted pyrazoline compounds of general formulas IVc, IVd or IVe 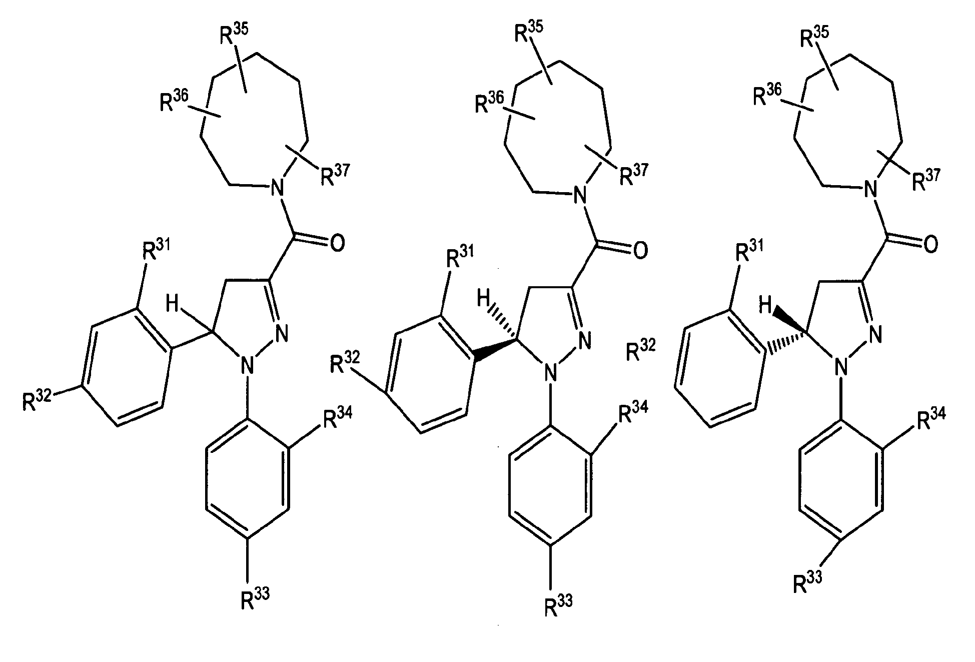 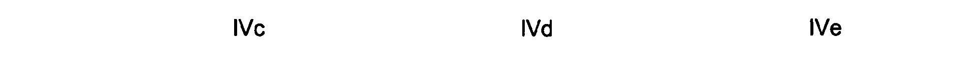 wherein
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form shown or in form of the acid or base or in form of a salt, especially a physiologically acceptable salt, or in form of a solvate, especially a hydrate or in form of a corresponding N-oxide thereof.

Especially preferred compounds of subgroup A5.4 are selected from
- N-(azepan-1-yl)-5-(4-chlorophenyl)-1-(2,4-dichlorophenyl)-4,5-dihydro-1H-pyrazole-3-carboxamide;
- (R)-N-(azepan-1-yl)-5-(4-chlorophenyl)-1-(2,4-dichlorophenyl)-4,5-dihydro-1H-pyrazole-3-carboxamide;
- (S)-N-(azepan-1-yl)-5-(4-chlorophenyl)-1-(2,4-dichlorophenyl)-4,5-dihydro-1H-pyrazole-3-carboxamide;
- N-(azepan-1-yl)-5-(4-bromophenyl)-1-(2,4-dichlorophenyl)-4,5-dihydro-1H-pyrazole-3-carboxamide,
- N-(azepan-1-yl)-5-(4-fluorophenyl)-1-(2,4-dichlorophenyl)-4,5-dihydro-1H-pyrazole-3-carboxamide,
- N-(azepan-1-yl)-5-(4-methoxyphenyl)-1-(2,4-dichlorophenyl)-4,5-dihydro-1H-pyrazole-3-carboxamide,
- (azepan-1-yl)(1-(2,4-dichlorophenyl)-5-(4-chlorophenyl)-4,5-dihydro-1H-pyrazol-3-yl)methanone,

Also preferred are Indoline-substituted pyrazoline compounds (A) of subgroup A6.1 of general formulas IV, IVa or IVb 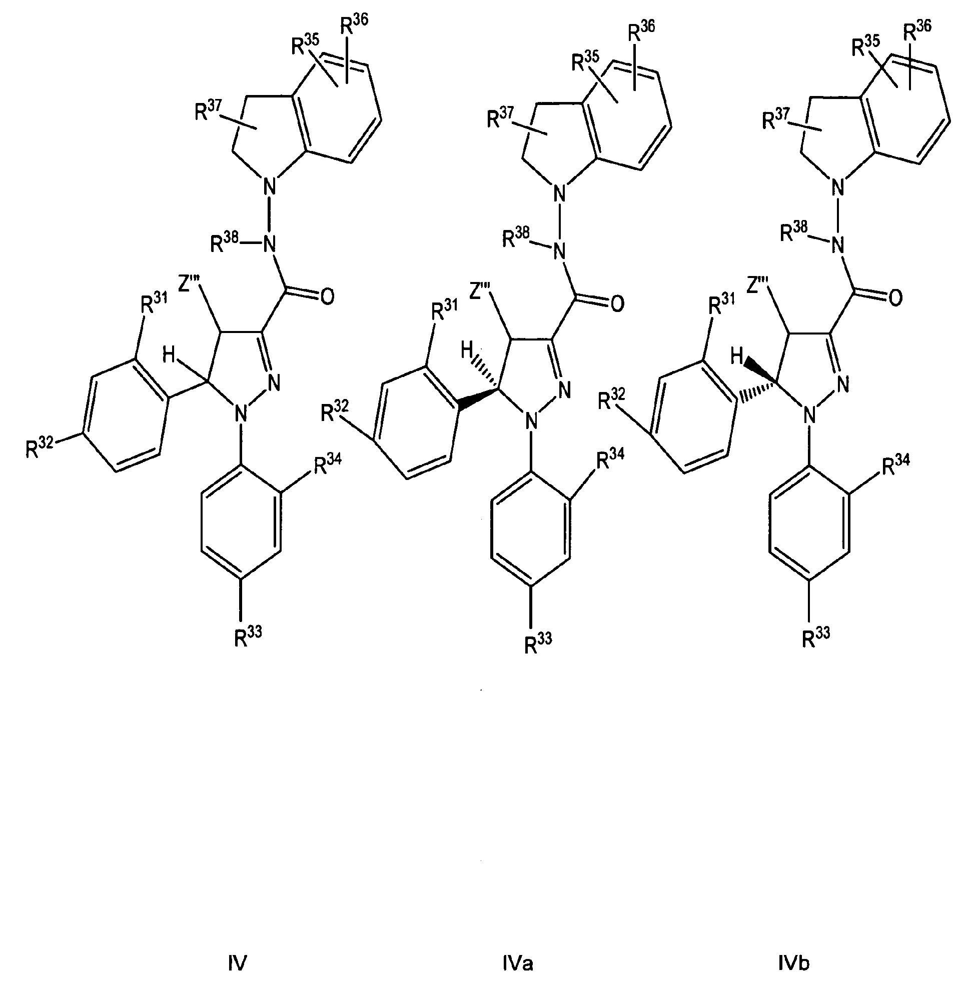 wherein
- Z"': is selected from CH₃ or C₂H₅;
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
- R³⁸: representing a hydrogen atom or a branched or linear C₁₋₃-alkyl group;
optionally in the form shown or in form of the acid or base or in form of a salt, especially a physiologically acceptable salt, or in form of a solvate, especially a hydrate or in form of a corresponding N-oxide thereof;
or
Indoline-substituted pyrazoline compounds of general formulas IVc, IVd or IVe 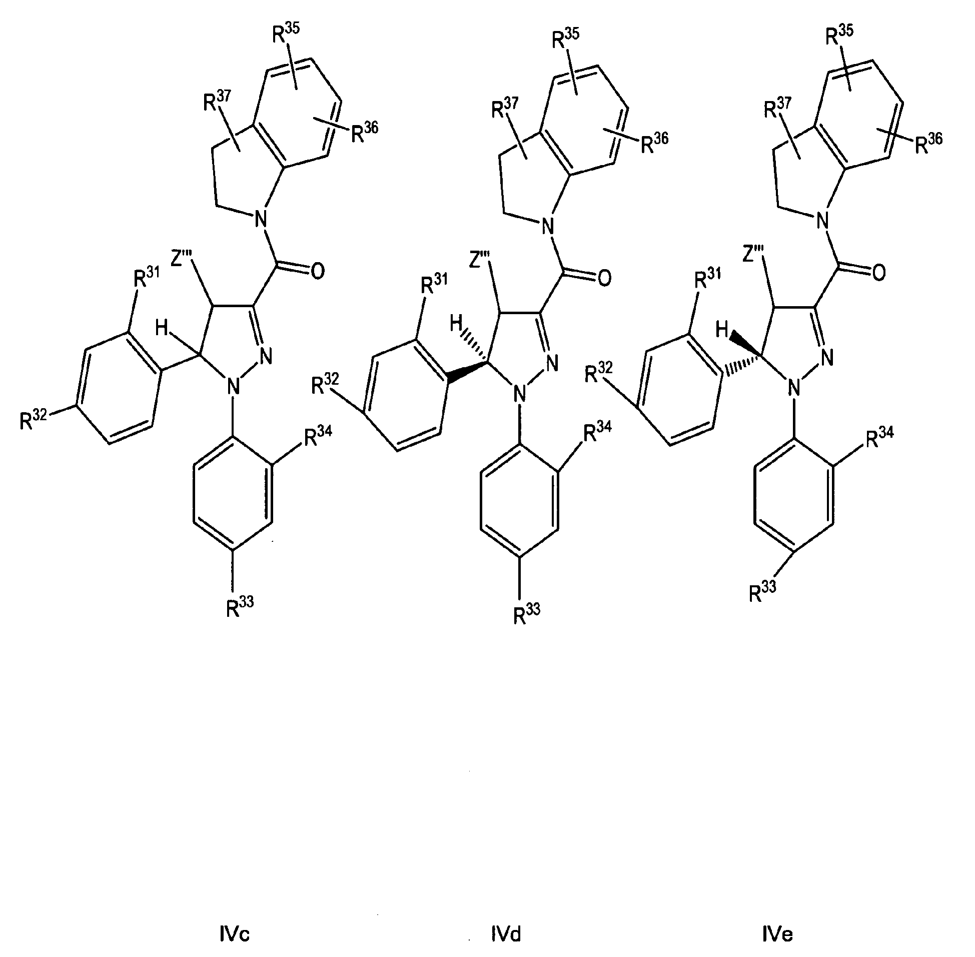 wherein
- Z'": is selected from CH₃ or C₂H₅;
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- p: is 1 or 2;
- R³⁵ R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form shown or in form of the acid or base or in form of a salt, especially a physiologically acceptable salt, or in form of a solvate, especially a hydrate or in form of a corresponding N-oxide thereof.

Especially preferred compounds of subgroup A6.1 are selected from
- cis-5-(4-chlorophenyl)-1-(2,4-dichlorophenyl)-N-(indolin-1-yl)-methyl-4,5-dihydro-1 H-pyrazole-3-carboxamide
- trans-5-(4-chlorophenyl)-1-(2,4-dichlorophenyl)-N-(indolin-1-yl)-4-methyl-4,5-dihydro-1 H-pyrazole-3-carboxamide
- (4R,5R)-5-(4-chlorophenyl)-1-(2,4-dichlorophenyl)-N-(indolin-1-yl)-4-methyl-4,5-dihydro-1 H-pyrazole-3-carboxamide
- (4S,5S)-5-(4-chlorophenylyl)-(2,4-dichlorophenyl)-N-(indolin-1-yl)-4-methyl-4,5-dihydro-1 H-pyrazole-3-carboxamide
- cis-5-(4-chlorophenyl)-1-(2,4-dichlorophenyl)-4-methyl-N-(2-methylindolin-1-yl)-4,5-dihydro-1H-pyrazole-3-carboxamide
- cis-5-(4-bromophenyl)-1-(2,4-dichlorophenyl)-N-(indolin-1-yl)-4-methyl-4,5-dihydro-1H-pyrazole-3-carboxamide
- trans-5-(4-bromophenyl)-1-(2,4-dichlorophenyl)-N-(indolin-1-yl)-4-methyl-4,5-dihydro-1H-pyrazole-3-carboxamide
- cis-1-(2,4-dichlorophenyl)-5-(4-fluorophenyl)-N-(indolin-1-yl)-4-methyl-4,5-dihydro-1H-pyrazole-3-carboxamide
- trans-1-(2,4-dichlorophenyl)-5-(4-fluorophenyl)-N-(indolin-1-yl)-4-methyl-4,5-dihydro-1H-pyrazole-3-carboxamide
- *cis*-1-(2,4-dichlorophenyl)-N-(indolin-1-yl)-5-(4-iodophenyl)-4-methyl-4,5-dihydro-1 H-pyrazole-3-carboxamide
- trans-1-(2,4-dichlorophenyl)-N-(indolin-1-yl)-5-(4-iodophenyl)-4-methyl-4,5-dihydro-1 H-pyrazole-3-carboxamide
- cis-1-(2,4-dichlorophenyl)-N-(indolin-1-yl)-5-(4-methoxyphenyl)-4-methyl-4,5-dihydro-1 H-pyrazole-3-carboxamide
- trans-1-(2,4-dichlorophenyl)-N-(indolin-1-yl)-5-(4-methoxyphenyl)-4-methyl-4,5-dihydro-1 H-pyrazole-3-carboxamide
- cis-1-(2,4-dichlorophenyl)-5-(4-hydroxyphenyl)-N-(indolin-1-yl)-4-methyl-4,5-dihydro-1 H-pyrazole-3-carboxamide
- trans-1-(2,4-dichlorophenyl)-5-(4-hydroxyphenyl)-N-(indolin-1-yl)-4-methyl-4,5-dihydro-1H-pyrazole-3-carboxamide
- cis-5-(5-chlorothiophen-2-yl)-1-(2,4-dichlorophenyl)-N-(indolin-1-yl)-4-methyl-4,5-dihydro-1H-pyrazole-3-carboxamide
- trans-5-(5-chlorothiophen-2-yl)-1-(2,4-dichlorophenyl)-N-(indolin-1-yl)-4-methyl-4,5-dihydro-1H-pyrazole-3-carboxamide
- cis-5-(5-bromothiophen-2-yl)-1-(2,4-dichlorophenyl)-N-(indolin-1-yl)-4-methyl-4,5-dihydro-1H-pyrazole-3-carboxamide
- trans-5-(5-bromothiophen-2-yl)-1-(2,4-dichlorophenyl)-N-(indolin-1-yl)-4-methyl-4,5-dihydro-1H-pyrazole-3-carboxamide
- cis-5-(4-chlorophenyl)-1-(2,4-dichlorophenyl)-4-ethyl-N-(indolin-1-yl)-4,5-dihydro-1H-pyrazole-3-carboxamide
- trans-5-(4-chlorophenyl)-1-(2,4-dichlorophenyl)-4-ethyl-N-(indolin-1-yl)-4,5-dihydro-1H-pyrazole-3-carboxamide
- cis-5-(4-bromophenyl)-1-(2,4-dichlorophenyl)-4-ethyl-N-(indolin-1-yl)-4,5-dihydro-1 H-pyrazole-3-carboxamide
trans-5-(4-bromophenyl)-1-(2,4-dichlorophenyl)-4-ethyl-N-(indolin-1-yl)-4,5-dihydro-1 H-pyrazole-3-carboxamide.

Also preferred are Cycloalkane-substituted pyrazoline compounds (A) of subgroup A6.2 of general formulas IV, IVa or IVb 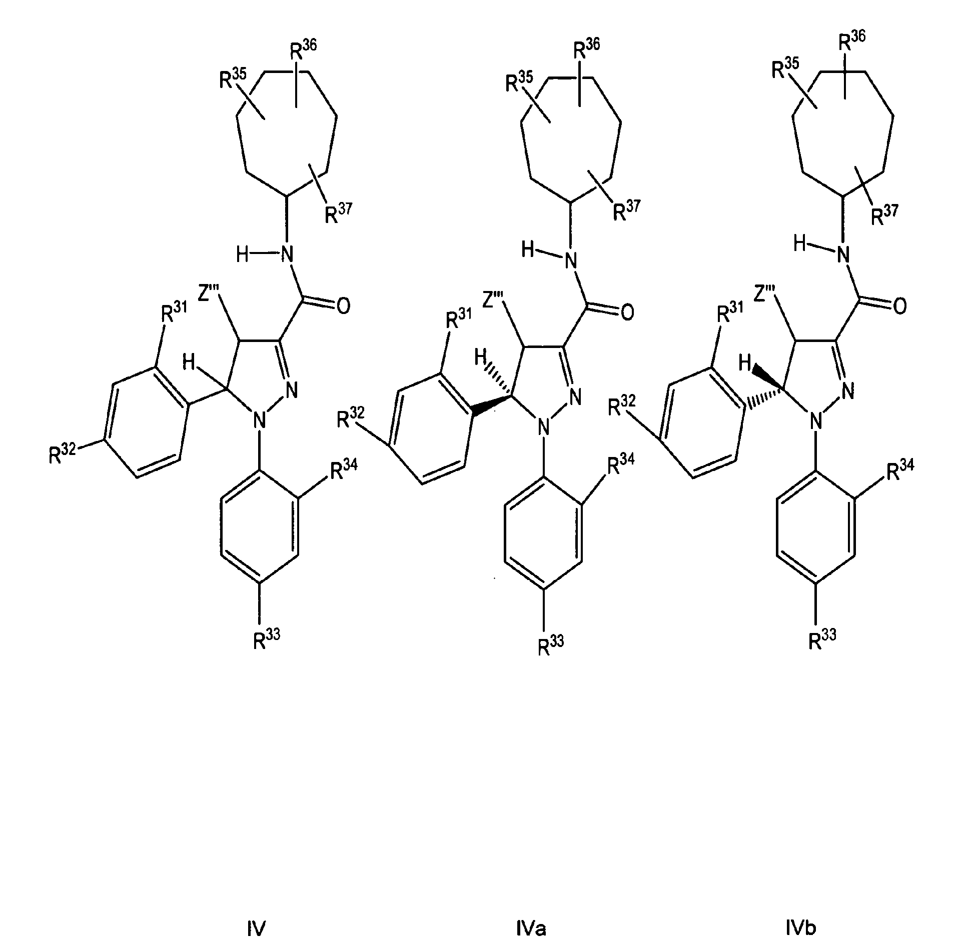 wherein
- Z"': is selected from CH₃ or C₂H₅;
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of substituted or unsubstituted, branched or linear, saturated or unsaturated C₈₋₂₀-alkyl or C(O)-C₁₋₂₀-alkyl; substituted or unsubstituted aryl, alkyl-aryl, heterocyclyl or alkyl-heterocyclyl; substituted or unsubstituted C(O)-aryl, C(O)-alkyl-aryl, C(O)-heterocyclyl or C(O)-alkyl-heterocyclyl;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form shown or in form of the acid or base or in form of a salt, especially a physiologically acceptable salt, or in form of a solvate, especially a hydrate or in form of a corresponding N-oxide thereof;

Especially preferred compounds of subgroup A6.2 are selected from
- cis-5-(4-chlorophenyl)-N-cycloheptyl-1-(2,4-dichlorophenyl)-4-methyl-4,5-dihydro-1 H-pyrazole-3-carboxamide
- trans-5-(4-chlorophenyl)-N-cycloheptyl-1-(2,4-dichlorophenyl)-4-methyl-4,5-dihydro-1H-pyrazole-3-carboxamide
- (4R,5R)-5-(4-chlorophenyl)-N-cycloheptyl-1-(2,4-dichlorophenyl)-4-methyl-4,5-dihydro-1H-pyrazole-3-carboxamide
- (4S,5S)-5-(4-chlorophenyl)-N-cycloheptyl-1-(2,4-dichlorophenyl)-4-methyl-4,5-dihydro-1 H-pyrazole-3-carboxamide
- cis-5-(4-bromophenyl)-N-cycloheptyl-1-(2,4-dichlorophenyl)-4-methyl-4,5-dihydro-1H-pyrazole-3-carboxamide
- trans-5-(4-bromophenyl)-N-cycloheptyl-1-(2,4-dichlorophenyl)-4-methyl-4,5-dihydro-1H-pyrazole-3-carboxamide
- cis-N-cycloheptyl-1-(2,4-dichlorophenyl)-5-(4-fluorophenyl)-4-methyl-4,5-dihydro-1H-pyrazole-3-carboxamide
- trans-N-cycloheptyl-1-(2,4-dichlorophenyl)-5-(4-fluorophenyl)-4-methyl-4,5-dihydro-1H-pyrazole-3-carboxamide
- cis-N-cycloheptyl-1-(2,4-dichlorophenyl)-5-(4-iodophenyl)-4-methyl-4,5-dihydro-1H-pyrazole-3-carboxamide
- trans-N-cycloheptyl-1-(2,4-dichlorophenyl)-5-(4-iodophenyl)-4-methyl-4,5-dihydro-1H-pyrazole-3-carboxamide
- *cis*-N-cycloheptyl-1-(2,4-dichlorophenyl)-5-(4-methoxyphenyl)-4-methyl-4,5-dihydro-1 H-pyrazole-3-carboxamide
- trans-N-cycloheptyl-1-(2,4-dichlorophenyl)-5-(4-methoxyphenyl)-4-methyl-4,5-dihydro-1 H-pyrazole-3-carboxamide
- cis-N-cycloheptyl-1-(2,4-dichlorophenyl)-5-(4-hydroxyphenyl)-4-methyl-4,5-dihydro-1 H-pyrazole-3-carboxamide
- trans-N-cycloheptyl-1-(2,4-dichlorophenyl)-5-(4-hydroxyphenyl)-4-methyl-4,5-dihydro-1H-pyrazole-3-carboxamide
- cis-5-(4-chlorophenyl)-N-cydoheptyl-1 -(2,4-dichlorophenyl)-4-ethyl-4,5-dihydro-1H-pyrazole-3-carboxamide
- trans-5-(4-chlorophenyl)-N-cycloheptyl-1-(2,4-dichlorophenyl)-4-ethyl-4,5-dihydro-1H-pyrazole-3-carboxamide
- cis-5-(4-bromophenyl)-N-cycloheptyl-1-(2,4-dichlorophenyl)-4-ethyl-4,5-dihydro-1H-pyrazole-3-carboxamide
- trans-5-(4-bromophenyl)-N-cycloheptyl-1-(2,4-dichlorophenyl)-4-ethyl-4,5-dihydro-1H-pyrazole-3-carboxamide.

Also preferred are Octahydropentalene-substituted pyrazoline compounds (A) of subgroup A6.3 of general formulas IV, IVa or IVb 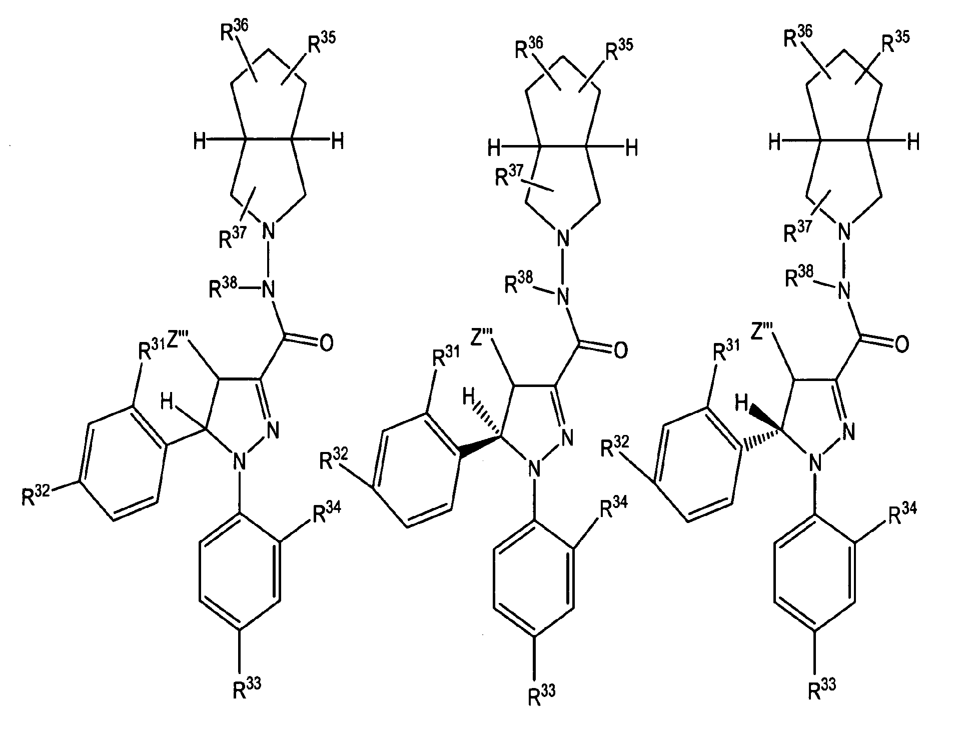 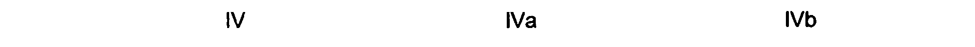 wherein
- Z'": is selected from CH₃ or C₂H₅;
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- R³⁵, R³⁶ and R³⁷: are independently from one another selected from, H, F, Cl, Br, I, OH, SH, C₁-₄alkyl, C₁-₄alkoxy, CF₃, CHF₂, CH₂F, OCF₃, a keto-group, NO₂ or NH₂;
- R³⁸: representing a hydrogen atom or a branched or linear C₁-₃-alkyl group;
optionally in the form shown or in form of the acid or base or in form of a salt, especially a physiologically acceptable salt, or in form of a solvate, especially a hydrate or in form of a corresponding N-oxide thereof;
or
Octahydropentalene-substituted pyrazoline compounds of general formulas IVc, lVd or IVe 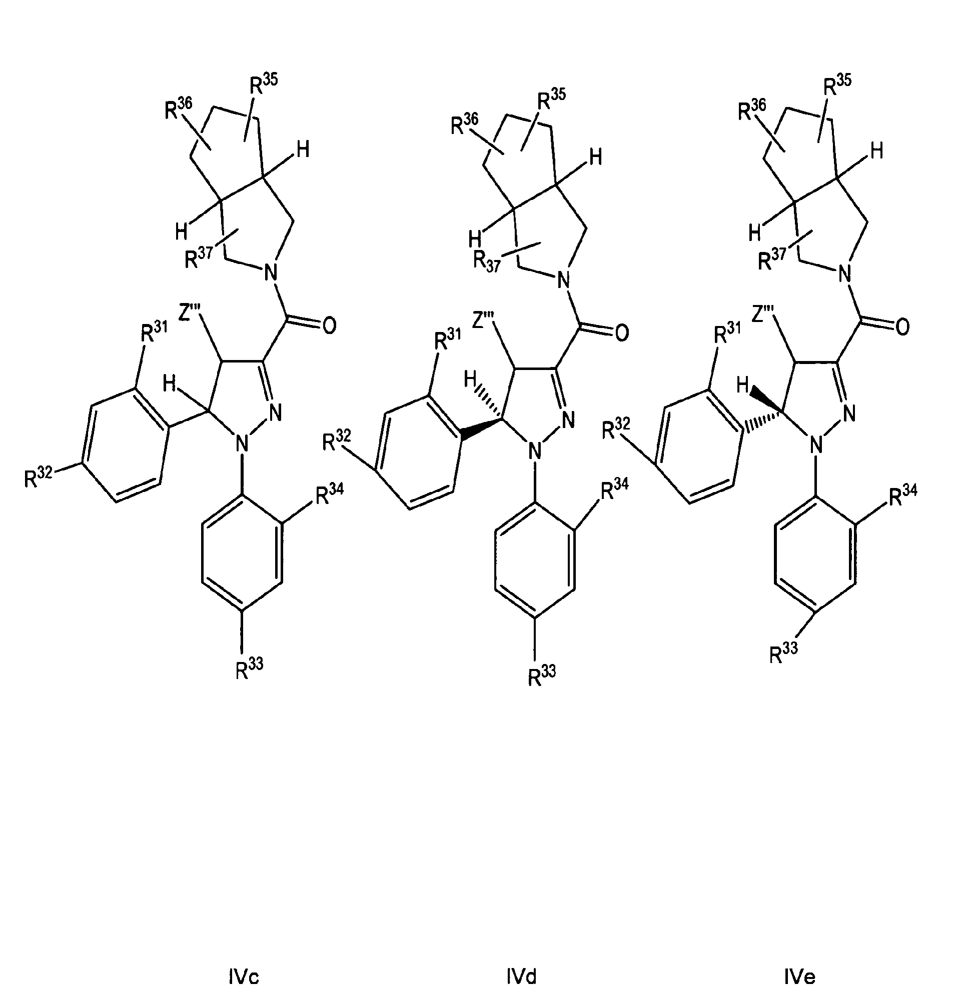 wherein
- Z'": is selected from CH₃ or C₂H₅;
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- R³⁵ , R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form shown or in form of the acid or base or in form of a salt, especially a physiologically acceptable salt, or in form of a solvate, especially a hydrate or in form of a corresponding N-oxide thereof.

Especially preferred compounds of subgroup A6.3 are selected from
- cis-5-(4-chlorophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
- trans-5-(4-chlorophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
- cis-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
- trans-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
- cis-1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride
- trans-1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride
- cis-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride
- trans-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride
- cis-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride
- trans-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride
- cis-5-(4-chlorophenyl)-1-(2,4-dichlorophenyl)-N-(hexahydrocyclopenta[c]pyrrol-2(1H)-yl)-4-ethyl-4,5-dihydro-1H-pyrazole-3-carboxamide hydrochloride
- trans-5-(4-chlorophenyl)-1-(2,4-dichlorophenyl)-N-(hexahydrocydopenta[c]pyrrol-2(1H)-yl)-4-ethyl-4,5-dihydro-1H-pyrazole-3-carboxamide hydrochloride
- cis-5-(4-bromophenyl)-1-(2,4-dichlorophenyl)-N-(hexahydrocyclopenta[c]pyrrol-2(1H)-yl)-4-ethyl-4,5-dihydro-1H-pyrazole-3-carboxamide hydrochloride
- trans-5-(4-bromophenyl)-1-(2,4-dichlorophenyl)-N-(hexahydrocyclopenta[c]pyrrol-2(H)-yl)-4-ethy)-4,5-dihydro-1H-pyrazole-3-carboxamide hydrochloride.

Also preferred are Azepane-substituted pyrazoline compounds (A) of subgroup A6.4 of general formulas IV, IVa or IVb 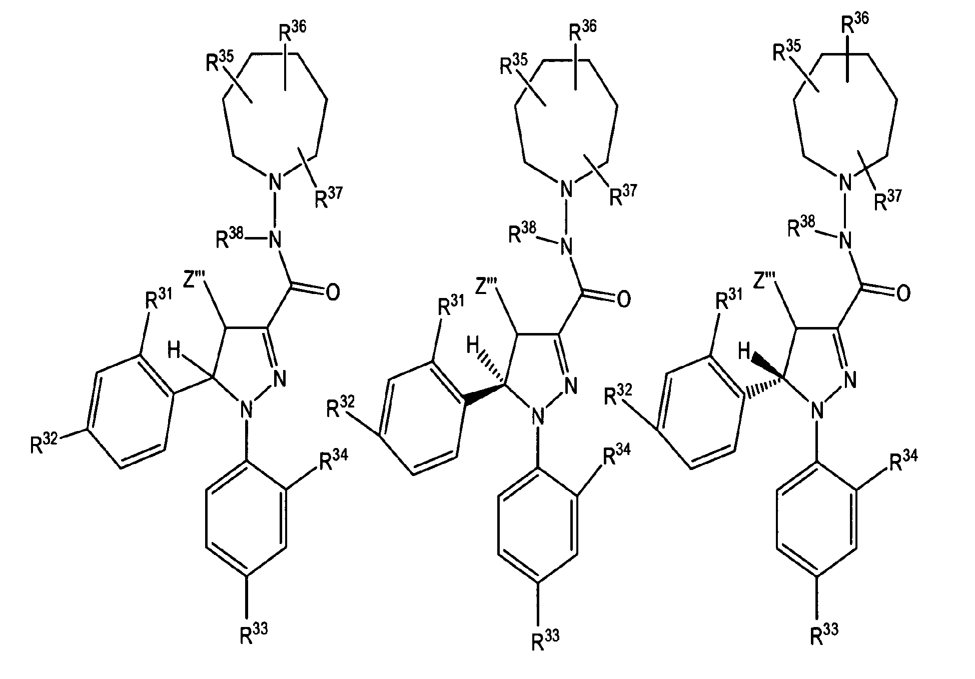 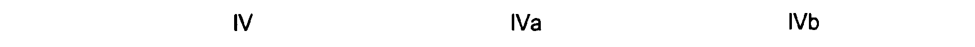 wherein
- Z'": is selected from CH₃ or C₂H₅;
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trffluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
- R³⁸: representing a hydrogen atom or a branched or linear C₁₋₃-alkyl group;
optionally in the form shown or in form of the acid or base or in form of a salt, especially a physiologically acceptable salt, or in form of a solvate, especially a hydrate or in form of a corresponding N-oxide thereof.
or
Azepane substituted pyrazoline compounds of general formulas IVc, IVd or IVe 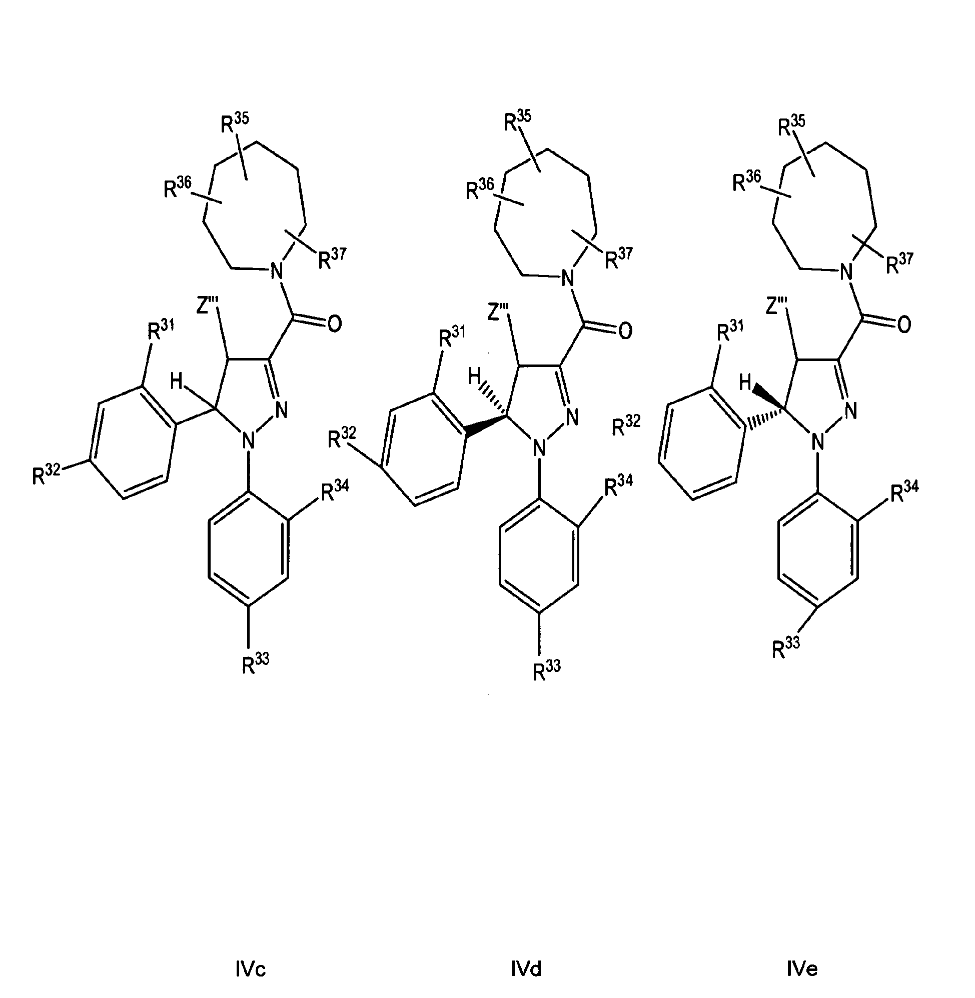 wherein
- Z'": is selected from CH₃ or C₂H₅;
- R³¹, R³², R³³ and R³⁴: independently of one another represent: H; branched or linear C₁₋₃-alkyl or branched or linear C₁₋₃-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, C₈₋₂₀-alkyl, heteroaryl, C(O)-aryl, C(O)-heteroaryl, C(O)-C₁₋₂₀-alkyl;
- R³⁵, R³⁶ and R³⁷: are independently from one another selected from, H, F, Cl, Br, I, OH, SH, C₁₋₄alkyl, C₁₋₄alkoxy, CF₃, CHF₂, CH₂F, OCF₃, a keto-group, NO₂ or NH₂;
optionally in the form shown or in form of the acid or base or in form of a salt, especially a physiologically acceptable salt, or in form of a solvate, especially a hydrate or in form of a corresponding N-oxide thereof.

Especially preferred compounds of subgroup A6.4 are selected from
- cis-N-(azepan-1-yl)-5-(4-chlorophenyl)-1-(2,4-dichlorophenyl)-4-methyl-4,5-dihydro-1H-pyrazole-3-carboxamide hydrochloride
- trans-N-(azepan-1-yl)-5-(4-chlorophenyl)-1-(2,4-dichlorophenyl)-4-methyl-4,5-dihydro-1H-pyrazole-3-carboxamide hydrochloride
- (4R,5R)-N-(azepan-1-yl)-5-(4-chlorophenyl)-1-(2,4-dichlorophenyl)-4-methyl-4,5-dihydro-1H-pyrazole-3-carboxamide hydrochloride
- (4S,5S)-N-(azepan-1-yl)-5-(4-chlorophenyl)-1-(2,4-dichlorophenyl)-4-methyl-4,5-dihydro-1H-pyrazole-3-carboxamide hydrochloride
- cis-N-(azepan-1-yl)-5-(4-bromophenyl)-1-(2,4-dichlorophenyl)-4-methyl-4,5-dihydro-1H-pyrazole-3-carboxamide hydrochloride
- trans-N-(azepan-1-yl)-5-(4-bromophenyl)-1-(2,4-dichlorophenyl)-4-methyl-4,5-dihydro-1H-pyrazole-3-carboxamide hydrochloride
- cis-N-(azepan-1-yl)-5-(4-fluorophenyl)-1-(2,4-dichlorophenyl)-4-methyl-4,5-dihydro-1H-pyrazole-3-carboxamide hydrochloride
- trans-N-(azepan-1-yl)-5-(4-fluorophenyl)-1-(2,4-dichlorophenyl)-4-methyl-4,5-dihydro-1H-pyrazole-3-carboxamide hydrochloride
- cis-N-(azepan-1-yl)-1-(2,4-dichlorophenyl)-5-(4-methoxyphenyl)-4-methyl-4,5-dihydro-1H-pyrazole-3-carboxamide hydrochloride
- trans-N-(azepan-1-yl)-1-(2,4-dichlorophenyl)-5-(4-methoxyphenyl)-4-methyl-4,5-dihydro-1H-pyrazole-3-carboxamide hydrochloride
- cis-N-(azepan-1-yl)-1-(2,4-dichlorophenyl)-5-(4-hydroxyphenyl)-4-methyl-4,5-dihydro-1H-pyrazole-3-carboxamide hydrochloride
- trans-N-(azepan-1-yl)-1-(2,4-dichlorophenyl)-5-(4-hydroxyphenyl)-4-methyl-4,5-dihydro-1H-pyrazole-3-carboxamide hydrochloride
- cis-N-(azepan-1-yl)-5-(4-chlorophenyl)-1-(2,4-dichlorophenyl)-4-ethyl-4,5-dihydro-1H-pyrazole-3-carboxamide hydrochloride
- trans-N-(azepan-1-yl)-5-(4-chlorophenyl)-1-(2,4-dichlorophenyl)-4-ethyl-4,5-dihydro-1H-pyrazole-3-carboxamide hydrochloride
- cis-N-(azepan-1-yl)-5-(4-bromophenyl)-1-(2,4-dichlorophenyl)-4-ethyl-4,5-dihydro-1H-pyrazole-3-carboxamide hydrochloride
- trans-N-(azepan-1-yl)-5-(4-bromophenyl)-1-(2,4-dichlorophenyl)-4-ethyl-4,5-dihydro-1H-pyrazole-3-carboxamide hydrochloride
- azepan-1-yl-cis-5-(4-chlorophenyl)-1-(2,4-dichlorophenyl)-4-methyl-4,5-dihydro-1H-pyrazol-3-yl)methanone
- azepan-1-yl-trans-5-(4-chlorophenyl)-1-(2,4-dichlorophenyl)-4-methyl-4,5-dihydro-1H-pyrazol-3-yl)methanone.

If one or more of the residues R¹ to R²² represents or comprises an aryl or heteroaryl radical, which may be substituted, unless defined otherwise, preferably said aryl or heteroaryl radical may optionally be substituted with 1, 2, 3, 4 or 5 substituent(s) independently selected from the group consisting of -C₁₋₆-perfluoralkyl, -C₁₋₆-alkyl substituted with one or more methoxy and/or ethoxy groups, -C₁₋₆-alkyl, -C₁₋₆-alkyl substituted with one or more hydroxy groups,-C₁₋₆-alkyl substituted with one or more chlorine atoms, -O-C₁₋₆-alkyl, -O-C₁₋₆-alkyl substituted with one or more methoxy and/or ethoxy groups, -S-C₁₋₆-alkyl, -C(=O)-OH, -C(=O)-O-C₁₋₆-alkyl, -O-C(=O)-C₁₋₆-alkyl, F, Cl, Br, I, -CN, -OCF₃, -O-C₂F₅, -O-C₃F₇, -O-C₄F₉, -SCF₃, -SCF₃, -SCF₂H, -SCFH₂, -OH, -SH, -SO₃H, -NH-C(=O)-C₁₋₆-alkyl, -N(C₁₋₆-alkyl)-C(=O)-C₁₋₆-alkyl,-NO₂, -CHO, -C(=O)-C₁₋₆-alkyl, -C(=O)-C₁₋₆-perfluoroalkyl, -C(=S)-NH-C₁₋₆-alkyl, -CF₂H,-CFH₂, -C(=O)-NR^{A}R^{B}, -C(=O)-NH-NR^{C}R^{D}, -S(=O)-C₁₋₆-alkyl, -S(=O)₂-C₁₋₆-alkyl, -S(=O)₂-phenyl, -(C₁₋₅-alkylene)-S-C₁₋₆-alkyl, -(C₁₋₅-alkylene)-S(=O)-C₁₋₆-alkyl, -(C₁₋₅-alkylene)-S(=O)₂-C₁₋₆-alkyl, -NR^{E}R^{F}, -(C₁₋₅-alkylene)-NR^{E}R^{F}, -S(=O)-NH₂, -S(=O)₂-NH-C₁₋₆-alkyl, -S(=O)₂-NH-phenyl, -NH-S(=O)₂-C₁₋₆-alkyl, cyclopropyl, cyclobutyl, cyclopentyl, cyclohexyl, pyrrolidinyl, piperidinyl, phenyl, thiophenyl, phenoxy and benzyl;
whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, -CF₃, -CN, -NO₂, -C₁₋₆-alkyl, -O-C₁₋₆-alkyl, -O-CF₃ and -S-CF₃ and
whereby R^{A}, R^{B}, R^{E} and R^{F}, independently of one another, represent hydrogen or -C₁₋₆-alkyl or R^{A} and R^{B} in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different C₁₋₆ alkyl radicals
and whereby R^{C} and R^{D}, independently of one another, represent hydrogen, -C₁₋₆-alkyl,-C(=O )-O-C₁₋₆-alkyl, C₃₋₈-cycloalkyl, -(C₁₋₅-alkylene)-C₃₋₈-cycloalkyl, -(C₁₋₆-alkylene)-O-C₁₋₆-alkyl or -C₁₋₆-alkyl substituted with one or more hydroxy groups or R^{C} and R^{D} in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting -C₁₋₆-alkyl, -C(=O)-C₁₋₆-alkyl, -C(=O)-O-C₁-₆-alkyl, -C(=O)-NH-C₁₋₆-alkyl, -C(=S)-NH-C₁₋₆-alkyl, oxo (=O), -C₁₋₆-alkyl substituted with one or more hydroxy groups, -(C₁₋₆-alkylene)-O-C₁₋₆-alkyl and -C(=O)-NH₂.

More preferably said aryl and heteroaryl radicals may optionally be substituted with 1, 2, 3, 4 or 5 substituent(s) independently selected from the group consisting of -CF₃, -C₂F₅, -C₃F₇,-C₄F₉, -CH₂Cl, -CHCl₂, -C₂H₄Cl, methyl, ethyl, n-propyl, isopropyl, n-butyl, isobutyl, 2-butyl, tert-butyl, n-pentyl, 2-pentyl, n-hexyl, -CH₂-OH, -CH₂-CH₂-OH, -CH₂-CH₂-CH₂-OH, -O-CH₂-O-CH₃, -O-CH₂-CH₂-O-CH₃, -O-CH₂-O-C₂H₅, -C(OCH₃)(C₂H₅)₂, -C(OCH₃)(CH₃)₂, -O-CH₃, -O-C₂H₅, -O-CH₂-CH₂-CH₃, -O-CH(CH₃)₂, -O-CH₂-CH₂-CH₂-CH₃, -O-C(CH₃)₃, -S-CH₃, -S-C₂H₅,-S-CH₂-CH₂-CH₃, -S-CH(CH₃)₂, -S-CH₂-CH₂-CH₂-CH₃, -S-C(CH₃)₃, -C(=O)-OH, -C(=O)-O-CH₃, -C(=O)O-C₂H₅, -C(=O)-O-C₃H₇, -C(=O)-O-C(CH₃)₃, -O-C(=O)-CH₃, -O-C(=O)-C₂H₅, -O-C(=O)-CH(CH₃)₂, -O-C(=O)-CH₂-CH₂-CH₃, -O-C(=O)-C(CH₃)₃, F, Cl, Br, I, -CN, -OCF₃, -O-C₂F₅, -O-C₃F₇, -O-C₄F₉, -SCF₃, -SCF₂H, -SCFH₂, -OH, -SH, -SO₃H, -NH-C(=O)-CH₃, -NH-C(=O)-C₂H₅, -NH-C(=OYC(CH₃)₃, -NO₂, -CHO, -C(=O)-CH₃, -C(=O)-C₂H₅, -C(=O)-C(CH₃)₃,-C(=O)-CF₃, -C(=O)-C₂F₅, -C(=O)-C₃F₇, -C(=S)-NH-CH₃, -C(=S)-NH-C₂H₅, -CF₂H, -CFH₂,-C(=O)-NH₂, -C(=O)-NH-CH₃, -C(=O)-NH-C₂H₅, -C(=O)-NH-C₃H₇, -C(=O)-N(CH₃)₂, -C(=O)-N(C₂H₅)₂, -C(=O)-NH-NH-CH₃, -C(=O)-NH-NH-C₂H₅, -C(=O)-NH-NH₂, -C(=O)-NH-N(CH₃)₂,-S(=O)-CH₃, -S(=O)-C₂H₅, -S(=O)-C₃H₇, -S(=O)₂-CH₃, -S(=O)₂-C₂H₅, -S(=O)₂-C₃H₇, -S(=O)₂-phenyl, -NH₂, -NH-CH₃, -NH-C₂H₅, -N(CH₃)₂, -N(C₂H₅)₂, -CH₂-N(CH₃)₂, -(CH₂)-morpholinyl,-(CH₂)-piperidinyl, -(CH₂)-piperazinyl, -(CH₂)-N(C₂H₅)₂, -CH₂-N(C₃H₇)₂, -CH₂-N(C₄H₉)₂, -CH₂-N(CH₃)(C₂H₅), -S(=O)-NH₂, -S(=O)₂-NH-CH₃, -S(=O)₂-NH-phenyl, -NH-S(=O)₂-CH₃, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituent(s) independently selected from the group consisting of F, Cl, Br, methyl, ethyl and n-propyl.

Preferred aryl radicals which are optionally at least mono-substituted are phenyl and naphthyl (1- and 2-naphthyl).

Preferably the heteroatoms which are present as ring members in the heteroaryl radical may, unless defined otherwise, independently be selected from the group consisting of nitrogen, oxygen and sulfur. More preferably a heteroaryl radical is 5- to 14-membered and may comprise 1, 2, 3 or 4 heteroatoms independently selected from the group consisting of nitrogen, oxygen and sulfur.

Preferred heteroaryl radicals which are unsubstituted or at least mono-substituted are pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl, imidazo[2,1-b]thiazolyl, 2H-chromenyl, pyranyl, indazolyl and quinazolinyl.

Preferred aryl and heteroaryl radicals which are condensed with a mono- or polycyclic ring system are [1,3]-benzodioxolyl, [1,4]-benzodioxanyl, [1,2,3,4]-tetrahydronaphthyl, (2,3)-dihydro-1H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl.

If one or more of the residues R¹ to R²² represents or comprises a saturated or unsaturated, optionally at least one heteroatom as a ring member containing cycloaliphatic radical, preferably a C₃₋₁₈ cycloaliphatic radical, a heterocyclic ring, preferably a 4- to 10-membered heterocyclic ring, a C₃-₁₆ cycloalkyl radical, a C₄₋₁₆cycloalkenyl radical, a C₄₋₁₆heterocycloalkyl radical, or a C₅-₁₆ heterocycloalkenyl radical" which may be substituted, unless defined otherwise, preferably said cycloaliphatic radical, heterocyclic ring, C₃₋₁₆ cycloalkyl radical, C₄-₁₆ cycloalkenyl radical, C₄₋₁₆ heterocycloalkyl radical, or C₅₋₁₆ heterocycloalkenyl radical, may in each case optionally be substituted with 1, 2, 3, 4 or 5 substituent(s) independently selected from the group consisting of oxo (=O), thioxo (=S), -C₁₋₆-perfluoralkyl, -C₁₋₆-alkyl,-C₁₋₆-alkyl substituted with one or more hydroxy groups, -C₁₋₆-alkyl substituted with one or more chlorine atoms, -C₁₋₆-alkyl substituted with one or more methoxy and/or ethoxy groups, -O-C₁₋₆-alkyl, -O-C₁₋₆-alkyl substituted with one or more methoxy and/or ethoxy groups, -S-C₁₋₆-alkyl, -C(=O)-OH, -C(=O)-O-C₁₋₆-alkyl, -O-C(=O)-C₁₋₆-alkyl, F, Cl, Br, I, -CN, -OCF₃, -O-C₂F₅, -O-C₃F₇, -O-C₄F₉, -SCF₃, -SCF₂H, -SCFH₂, -OH, -SH, -SO₃H, -NH-C(=O)-C₁₋₆-alkyl,-N(C₁₋₆-alkyl)-C(=O)-C₁₋₆-alkyl, -NO₂, -CHO, -C(=O)-C₁₋₆-alkyl, -C(=O)-C₁₋₆-perfluoroalkyl,-C(=S)-NH-C₁₋₆-alkyl, -CF₂H, -CFH₂, -C(=O)-NR^{A}R^{B}, -C(=O)-NH-NR^{C}R^{D}, -S(=O)-C₁₋₆-alkyl,-S(=O)₂-C₁₋₆-alkyl, -S(=O)₂-phenyl, -(C₁₋₅-alkylene)-S-C₁₋₆-alkyl, -(C₁₋₅-alkylene)-S(=O)-C₁₋₆-alkyl, -(C₁₋₅-alkylene)-S(=O)₂-C₁₋₆-alkyl, -NR^{E}R^{F}, -(C₁₋₅-alkylene)-NR^{E}R^{F}, -S(=O)-NH₂,-S(=O)₂-NH-C₁₋₆-alkyl, -S(=O)₂-NH-phenyl, -NH-S(=O)₂-C₁₋₆-alkyl, -O-Benzyl, -O-Phenyl, cyclopropyl, cyclobutyl, cyclopentyl, cyclohexyl, pyrrolidinyl, piperidinyl, phenyl, thiophenyl, phenoxy and benzyl;
whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, -CF₃, -CN, -NO₂, -C₁₋₆-alkyl, -O-C₁₋₆-alkyl, -O-CF₃ and -S-CF₃ and
whereby R^{A}, R^{B}, R^{E} and R^{F}, independently of one another, represent hydrogen or -C₁₋₆-alkyl or R^{A} and R^{B} in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different C₁₋₆ alkyl radicals
and whereby R^{C} and R^{D}, independently of one another, represent hydrogen, -C₁₋₆-alkyl,-C(=O)-O-C₁₋₆-alkyl, C₃₋₈-cycloalkyl, -(C₁₋₅-alkylene)-C₃₋₈-cycloalkyl, -(C₁₋₆-alkylene)-O-C₁₋₆-alkyl or -C₁₋₆-alkyl substituted with one or more hydroxy groups or R^{C} and R^{D} in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting -C₁₋₆-alkyl, -C(=O)-C₁₋₆-alkyl, -C(=O)-O-C₁₋₆-alkyl, -C(=O)-NH-C₁₋₆-alkyl, -C(=S)-NH-C₁₋₆-alkyl, oxo (=O), -C₁₋₆-alkyl substituted with one or more hydroxy groups, -(C₁₋₆-alkylene)-O-C₁₋₆-alkyl and -C(=O)-NH₂.

More preferably said cycloaliphatic radicals, heterocyclic rings, C₃₋₁₆cycloalkyl radicals, C₄₋₁₆ cycloalkenyl radicals, C₄₋₁₆ heterocycloalkyl radicals, or C₅₋₁₆ heterocycloalkenyl radicals, may optionally be substituted with 1, 2, 3, 4 or 5 substituent(s) independently selected from the group consisting of oxo (=O), thioxo (=S), -CF₃, -C₂F₅, -C₃F₇, -C₄F₉, -CH₂Cl, -CHCl₂, -C₂H₄Cl, methyl, ethyl, n-propyl, isopropyl, n-butyl, isobutyl, 2-butyl, tert-butyl, n-pentyl, 2-pentyl, n-hexyl, -CH₂-OH, -CH₂-CH₂-OH, -CH₂-CH₂-CH₂-OH, -O-CH₂-O-CH₃, -O-CH₂-CH₂-O-CH₃, -O-CH₂-O-C₂H₅, -C(OCH₃)(C₂H₅)₂, -C(OCH₃)(CH₃)₂, -O-CH₃, -O-C₂H₅, -O-CH₂-CH₂-CH₃, -O-CH(CH₃)₂, -O-CH₂-CH₂-CH₂-CH₃, -O-C(CH₃)₃, -S-CH₃, -S-C₂H₅, -S-CH₂-CH₂-CH₃, -S-CH(CH₃)₂, -S-CH₂-CH₂-CH₂-CH₃, -S-C(CH₃)₃, -C(=O)-OH, -C(=O)-O-CH₃, -C(=O)-O-C₂H₅,-C(=O)-O-C₃H₇, -C(=O)-O-C(CH₃)₃, -O-C(=O)-CH₃, -O-C(=O)-C₂H₅, -O-C(=O)-CH(CH₃)₂, -O-C(=O)-CH₂-CH₂-CH₃, -O-C(=O)-C(CH₃)₃, F, Cl, Br, I, -CN, -OCF₃, -O-C₂F₅, -O-C₃F₇, -O-C₄F₉, -SCF₃, -SCF₂H, -SCFH₂, -OH, -SH, -SO₃H, -NH-C(=O)-CH₃, -NH-C(=O)-C₂H₅, -NH-C(=O)-C(CH₃)₃, -NO₂, -CHO, -C(=O)-CH₃, -C(=O)-C₂H₅, -C(=O)-C(CH₃)₃, -C(=O)-CF₃, -C(=O)-C₂F₅, -C(=O)-C₃F₇, -C(=S)-NH-CH₃, -C(=S)-NH-C₂H₅, -CF₂H, -CFH₂, -C(=O)-NH₂, -C(=O)-NH-CH₃, -C(=O)-NH-C₂H₅, -C(=O)-NH-C₃H₇, -C(=O)-N(CH₃)₂, -C(=O)-N(C₂H₅)₂, -C(=O)-NH-NH-CH₃,-C(=O)-NH-NH-C₂H₅, -C(=O)-NH-NH₂, -C(=O)-NH-N(CH₃)₂, -S(=O)-CH₃, -S(=O)-C₂H₅,-S(=O)-C₃H₇, -S(=O)₂-CH₃, -S(=O)₂-C₂H₅, -S(=O)₂-C₃H₇, -S(=O)₂-phenyl, -NH₂, -NH-CH₃, -NH-C₂H₅, -N(CH₃)₂, -N(C₂H₅)₂, -CH₂-N(CH₃)₂, -(CH₂)-morpholinyl, -(CH₂)-piperidinyl, -(CH₂)-piperazinyl, -(CH₂)-N(C₂H₅)₂, -CH₂-N(C₃H₇)₂, -CH₂-N(C₄H₉)₂, -CH₂-N(CH₃)(C₂H₅), -*S(=O)-NH₂, -S(=O)₂-NH-CH₃, -S(=O)₂-NH-phenyl, -NH-S(=O)₂-CH₃, -O-Benzyl, -O-Phenyl, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituents independently selected from the group consisting of F, Cl, Br, methyl, ethyl and n-propyl.

If one or more of the residues R¹ to R²² represents or comprises a cycloaliphatic radical, preferably a C₃₋₁₆ cycloaliphatic radical, which contains one or more heteroatoms as ring members, unless defined otherwise, each of these heteroatoms may preferably be selected from the group consisting of nitrogen, oxygen and sulfur. More preferably a cycloaliphatic group may optionally contain 1, 2, 3 or 4 heteroatom(s) independently selected from the group consisting of N, O and S as ring members.

Suitable saturated or unsaturated, optionally at least one heteroatom as ring member containing cycloaliphatic radicals may preferably be selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl. (2,3)-dihydrofuranyl, (2,5)-dihydro-1 H-pyrrolyl, (2,3)-dihydro-1 H-pyrrolyl, tetrahydrothiopyranyl, tetrahydropyranyl, (3,4)-dihydro-2H-pyranyl, (3,4)-dihydro-2H-thiopyranyl, (1,2,3,6ytetrahydropyhdinyl, (1,2,3,4)-tetrahydropyridinyl, (1,2,5,6)-tetrahydropyridiny), [1,3]-oxazinanyl, hexahydropyrimidinyl, (5,6)-dihydro-4H-pyrimidinyl, oxazolidinyl, (1,3)-dioxanyl, (1,4)-dioxanyl and (1,3)-dioxolanyl.

Suitable saturated or unsaturated, optionally at least one heteroatom as ring member containing cycloaliphatic radicals which are condensed with an unsubstituted or at least mono-substituted mono- or polycyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl and (1,2,3,4)-tetrahydroquinoxazlinyl.

Preferably a cycloaliphatic radical, a C₃₋₁₆ cycloalkyl radical, a C₄₋₁₆ cycloalkenyl radical, a C₄₋₁₆ heterocycloalkyl radical or a C₅₋₁₆ heterocycloalkenyl radical may be bridged by 1, 2 or 3 unsubstituted or at least mono-substituted alkylene group(s).

Suitable saturated or unsaturated, optionally at least one heteroatom as ring member containing cycloaliphatic radicals which are bridged by at least one unsubstituted or at least mono-substituted alkylene group may preferably be selected from the group consisting of adamantyl, bicyclo[2.2.1]heptyl, bicyclo[3.1.1]heptyl, norbomenyl and 8-aza-bicyclo[3.2.1]octyl.

A suitable saturated or unsaturated, optionally at least one heteroatom as ring member containing cycloaliphatic radical which together with a saturated or unsaturated, unsubstituted or at least mono-substituted cycloaliphatic radical forms a spirocyclic residue via a common ring atom is 8-aza-spiro[4.5]decanyl.

A mono- or polycyclic ring system according to the present invention - if not defined otherwise - means a mono- or polycyclic hydrocarbon ring system, preferably a mono- or bicyclic ring system, that may be saturated, unsaturated or aromatic. Each of its different rings may show a different degree of saturation, i.e. they may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of nitrogen, oxygen and sulfur. The rings of the mono- or bicyclic ring system are preferably 5-, 6- or 7-membered.

The term "condensed" according to the present invention means that a ring or ring system is attached to another ring or ring system, whereby the terms "annulated" or "annelated" are also used by those skilled in the art to designate this kind of attachment.

If one or more of the residues R¹ to R²² comprises a mono- or polycyclic ring system, which may be substituted, unless defined otherwise, preferably said mono- or polycyclic ring system may optionally be substituted with 1, 2, 3, 4 or 5 substituent(s) independently selected from the group consisting of oxo (=O), thioxo (=S), -C₁₋₆-perfluoralkyl, -C₁₋₆-alkyl, - C₁₋₆-alkyl substituted with one or more hydroxy groups, -C₁₋₆-alkyl substituted with one or more chlorine atoms, -C₁₋₆-alkyl substituted with one or more methoxy and/or ethoxy groups, -O-C₁₋₆-alkyl, -O-C₁₋₆-alkyl substituted with one or more methoxy and/or ethoxy groups, -S-C₁₋₆-alkyl, -C(=O)-OH, -C(=O)-O-C₁₋₆-alkyl, -O-C(=O)-C₁₋₆-alkyl, F, Cl, Br, I, -CN, -OCF₃, -O-C₂F₅, -O-C₃F₇, -O-C₄F₉, -SCF₃, -SCF₂H, -SCFH₂, -OH, -SH, -SO₃H, -NH-C(=O)-C₁₋₆-alkyl,-N(C₁₋₆-alkyl)-C(=O)-C₁₋₆-alkyl, -NO₂, -CHO, -C(=O)-C₁₋₆-alkyl, -C(=O)-C₁₋₆-perfluoroalkyl,-C(=S)-NH-C₁₋₆-alkyl, -CF₂H, -CFH₂, -C(=O)-NR^{A}R^{B}, -C(=O)-NH-NR^{C}R^{D}, -S(=O)-C₁₋₆-alkyl,-S(=O)₂-C₁₋₆-alkyl, -S(=O)₂-phenyl, -(C₁₋₅-alkylene)-S-C₁₋₆-alkyl, -(C₁₋₅-alkylene)-S(=O)-C₁₋₆-alkyl, -(C₁₋₅-alkylene)-S(=O)₂-C₁₋₆-alkyl, -NR^{E}R^{F}, -(C₁₋₅-alkyleneyNR^{E}R^{F}, -S(=O)-NH₂, - S(=O)₂-NH-C₁₋₆-alkyl, -S(=O)₂-NH-phenyl, -NH-S(=O)₂-C₁₋₆-alkyl, cyclopropyl, cyclobutyl, cyclopentyl, cyclohexyl, pyrrolidinyl, piperidinyl, phenyl, thiophenyl, phenoxy and benzyl;
whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, -CF₃, -CN, -NO₂, -C₁₋₆-alkyl, -O-C₁₋₆-alkyl, -O-CF₃ and -S-CF₃ and
whereby R^{A}, R^{B}, R^{E} and R^{F}, independently of one another, represent hydrogen or -C₁₋₆-alkyl or R^{A} and R^{B} in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different C₁₋₆ alkyl radicals
and whereby R^{C} and R^{D}, independently of one another, represent hydrogen, -C₁₋₆-alkyl,-C(=O)-O-C₁₋₆-alkyl, C₃₋₈-cycloalkyl, -(C₁₋₅-alkylene)-C₃₋₈-cycloalkyl, -(C₁₋₆-alkylene)-O-C₁₋₆-alkyl or -C₁₋₆-alkyl substituted with one or more hydroxy groups or R^{C} and R^{D} in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting -C₁₋₆-alkyl, -C(=O)-C₁₋₆-alkyl, -C(=O)-O-C₁₋₆-alkyl, -C(=O)-NH-C₁₋₆-alkyl, -C(=S)-NH-C₁₋₆-alkyl, oxo (=O), -C₁₋₆-alkyl substituted with one or more hydroxy groups, -(C₁₋₆-alkylene)-O-C₁₋₆-alkyl and -C(=O)-NH₂.

More preferably said mono- or polycyclic ring system may optionally be substituted with 1, 2, 3, 4 or 5 substituent(s) independently selected from the group consisting of oxo (=O), thioxo (=S), -CF₃, -C₂F₅, -C₃F₇, -C₄F₉, -CH₂Cl, -CHCl₂, -C₂H₄Cl, methyl, ethyl, n-propyl, isopropyl, n-butyl, isobutyl, 2-butyl, tert-butyl, n-pentyl, 2-pentyl, n-hexyl, -CH₂-OH, -CH₂-CH₂-OH, -CH₂-CH₂-CH₂-OH, -O-CH₂-O-CH₃, -O-CH₂-CH₂-O-CH₃, -O-CH₂-O-C₂H₅, -C(OCH₃)(C₂H₅)₂,-C(OCH₃)(CH₃)₂, -O-CH₃, -O-C₂H₅, -O-CH₂-CH₂-CH₃, -O-CH(CH₃)₂, -O-CH₂-CH₂-CH₂-CH₃,-O-C(CH₃)₃, -S-CH₃, -S-C₂H₅, -S-CH₂-CH₂-CH₃, -S-CH(CH₃)₂, -S-CH₂-CH₂-CH₂-CH₃, -S-C(CH₃)₃, -C(=O)-OH, -C(=O)-O-CH₃, -C(=O)-O-C₂H₅, -C(=O)-O-C₃H₇, -C(=O)-O-C(CH₃)₃, -O-C(=O)-CH₃, -O-C(=O)-C₂H₅, -O-C(=O)-CH(CH₃)₂, -O-C(=O)-CH₂-CH₂-CH₃, -O-C(=O)-C(CH₃)₃, F, Cl, Br, I, -CN, -OCF₃, -O-C₂F₅, -O-C₃F₇, -O-C₄F₉, -SCF₃, -SCF₂H, -SCFH₂, -OH, - SH, -SO₃H, -NH-C(=O)-CH₃, -NH-C(=O)C₂H₅, -NH-C(=O)-C(CH₃)₃, -NO₂, -CHO, -C(=O)-CH₃, -C(=O)C₂H₅, -C(=O)-C(CH₃)₃, -C(=O)-CF₃, -C(=O)-C₂F₅, -C(=O)C₃F₇, -C(=S)-NH-CH₃, -C(=S)-NH-C₂H₅, -CF₂H, -CFH₂, -C(=O)-NH₂, -C(=O)-NH-CH₃, -C(=O)-NH-C₂H₅, -C(=O)-NH-C₃H₇, -C(=O)-N(CH₃)₂, -C(=O)-N(C₂H₅)₂, -C(=O)-NH-NH-CH₃, -C(=O)-NH-NH-C₂H₅, -C(=O)-NH-NH₂, -C(=O)-NH-N(CH₃)₂, -S(=O)-CH₃, -S(=O)-C₂H₅, -S(=O)-C₃H₇, -S(=O)₂-CH₃, -S(=O)₂-C₂H₅, -S(=O)-C₃H₇, -S(=O)₂-phenyl, -NH₂, -NH-CH₃, -NH-C₂H₅, -N(CH₃)₂, -N(C₂H₅)₂, -CH₂-N(CH₃)₂, -(CH₂)-morpholinyl, -(CH₂)-piperidinyl, -(CH₂)-piperazinyl, -(CH₂)-N(C₂H₅)₂, -CH₂-N(C₃H₇)₂, -CH₂-N(C₄H₉)₂, -CH₂-N(CH₃)(C₂H₅), -S(=O)-NH₂, -S(=O)₂-NH-CH₃, -S(=O)₂-NH-phenyl, -NH-S(=O)₂-CH₃, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituents independently selected from the group consisting of F, Cl, Br, methyl, ethyl and n-propyl.

If one or more of the residues R⁴ to R²² represent or comprise a saturated or unsaturated, unsubstituted or at least mono-substituted aliphatic radical, preferably a C₁₋₁₆aliphatic radical, said aliphatic radical may be linear or branched.

Preferably aliphatic radicals, C₁₋₁₆ alkyl radicals, C₂₋₁₆ alkenyl radical and C₂₋₁₆ alkinyl radicals, unless defined otherwise, may optionally be substituted with 1, 2, 3, 4, 5, 6, 7, 8 or 9 substituents independently selected from the group consisting of -OH, F, Cl, Br, I, -O-C₁₋₆-alkyl, -OCF₃, -O-C₂F₅, -O-C₃F₇, -O-C₄F₉, -CF₃, -C₂F₅, -C₃F₇, -C₄F₉, -NH₂, -NH-C₁₋₆-alkyl,-N(C₁₋₆-alkyl)₂, -C(=O)-OH, -C(=O)-O-C₁₋₆-alkyl, -C(=O)-NH₂, -C(=O)-NH-C₁₋₆-alkyl, -C(=O)-N(C₁₋₆-alkyl)₂, -CN, -NO₂, -S(=O)-NH₂, -CHO, -C(=O)-C₁₋₆-alkyl, -S(=O)-C₁₋₆-alkyl, -S(=O)₂-C₁₋₆-alkyl, -NH-S(=O)-C₁₋₆-alkyl, -NH-C(=O)-O-C₁₋₆-alkyl and -NH-C(=O)-C₁₋₆-alkyl.

More preferably aliphatic radicals, C₁₋₁₆ alkyl radicals, C₂₋₁₆ alkenyl radical and C₂₋₁₆ alkinyl radicals may optionally be substituted with 1, 2, 3, 4, 5, 6, 7, 8 or 9 substituents independently selected from the group consisting of -OH, F, Cl, Br, I, -O-CH₃, -O-C₂H₅, -O-CH₂-CH₂-CH₃, -O-CH(CH₃)₂, -O-CH₂-CH₂-CH₂-CH₃, -O-C(CH₃)₃, -NH₂, -NH-CH₃, -NH-C₂H₅,-N(CH₃)₂, -N(C₂H₅)₂, -CN, -NO₂, -NH-C(=O)-CH₃, -NH-C(=O)-C₂H₅, -NH-C(=O)-C(CH₃)₃, -NH-C(=O)-O-CH₃, -NH-C(=O)-O-C₂H₅, -NH-C(=O)-O-C(CH₃)₃, -C(=O)-NH-CH₃, -C(=O)NH-C₂H₅, -C(=O)-NH-C(CH₃)₃, -C(=O)-N(CH₃)₂, -C(O)-N(C₂H₅)₂, -C(=O)-OH, -C(=O)-O-CH₃, -C(=O)-O-C₂H₅, -C(=O)-O-C(CH₃)₃, -C(=O)-CH₃, -C(=O)-C₂H₅ and -C(=OYC(CH₃)₃.

Suitable alkyl radicals, preferably C₁₋₁₆ alkyl radicals, are selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl, 2-(6-methyl)-octyl, n-nonyl, n-decyl, n-undecyl, n-dodecyl, n-tridecyl, n-tetradecyl, n-pentadecycl and n-hexadecyl.

Suitable at least mono-substituted alkyl radicals are selected from the group consisting of-CF₃, -CH₂F, -CF₂H, -CH₂-O-CH₃, -C₂F₅, -CH₂-CH₂-F, -CH₂-CN, -CH₂-OH, -CH₂-CH₂-CN,-CH₂-CH₂-OH, -CH₂-CH₂-OCH₃, -CH₂-CH₂-CH₂-CN, -CH₂-CH₂-CH₂-OH, -CH₂-CH₂-CH₂-O-CH₃, -CH₂-CH₂-CH₂-CH₂-O-CH₃, -CH₂-NH₂, -CH₂-N(CH₃)₂, -CH₂-N(C₂H₅)₂, -CH₂-CH-NH₂,-CH₂-CH₂-N(CH₃)₂, -CH₂-CH₂-N(C₂H₅)₂, -CH₂-CH₂-CH₂-NH₂, -CH₂-CH₂-CH₂-N(CH₃)₂ and-CH₂-CH₂-CH₂-N(C₂H₅)₂.

An alkenyl radical according to the present invention comprises at least one carbon-carbon double bond. Suitable alkenyl radicals, preferably C₂₋₁₆alkenyl radicals, are selected from the group consisting of vinyl, n-propenyl, n-butenyl, n-pentenyl, n-hexenyl, n-heptenyl, n-octenyl, n-nonenyl, n-decenyl, n-undecenyl, n-dodecenyl, n-tridecenyl, n-tetradecenyl, n-pentadecenyl and n-hexadecenyl.

An alkinyl radical comprises at least one carbon-carbon triple bond. Suitable alkinyl radicals, preferably C₂₋₁₆alkinyl radicals, are selected from the group consisting of ethinyl, propinyl, n-butinyl, n-pentinyl, n-hexinyl, n-octinyl, n-noninyl, n-decinyl, n-undecinyl, n-dodecinyl, n-tridecinyl, n-tetradecinyl, n-pentadecinyl and n-hexadecinyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituent(s). Said substituent(s) may preferably be selected independently from the group consisting of -O-C₁₋₆-alkyl, -S-C₁₋₆-alkyl, -F, Cl, Br, I, -CN, -CF₃, -OCF₃, -SCF₃, -OH, -SH, -SO₃H, -NH₂, -NH(C₁₋₆-alkyl), -N(C₁₋₆-alkyl)₂ and phenyl. More preferably said substituent(s) may be selected from the group consisting of -F, Cl, Br, I, -CN, -CF₃, -OCF₃, -SCF₃, -OH, -SH, -SO₃H, -NH₂, -NH-CH₃, -N(CH₃)₂, -O-CH₃ and -O-C₂H₅. An alkenylene group comprises at least one carbon-carbon double bond, an alkinylene group comprises at least one carbon-carbon triple bond.

Suitable alkylene groups, preferably C₁₋₅-alkylene groups, include -(CH₂)-, -CH(CH₃)-,-CH(phenyl), -(CH₂)₂-, -(CH₂)₃-,-(CH₂)₄-,-(CH₂)₅ and -(CH₂)₆-, suitable alkenylene groups, preferably C₂₋₅-alkenylene groups, include -CH=CH-, -CH₂-CH=CH- and -CH=CH-CH₂- and suitable alkinylene groups, preferably C₂₋₅-alkinylene groups, include -C≡C-, -CH₂-C≡C- and -C≡C-CH₂-.

The substituted pyrazoline compounds of general formula I given above, wherein R⁵ represents hydrogen, may be prepared by a process wherein at least one compound of general formula II, 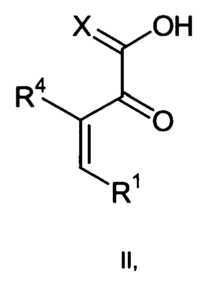 wherein R¹, X and R⁴ have the meaning given above, is reacted with at least one compound of general formula III, 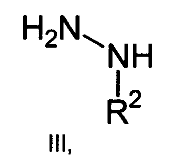 or a corresponding salt thereof, wherein R² has the meaning given above, in a reaction medium, optionally in an inert atmosphere, optionally in the presence of at least one acid, to yield at least one compound of general formula IV, 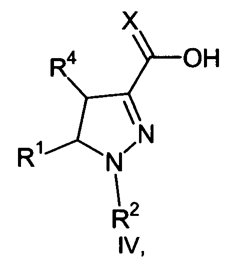 wherein R¹, X, R² and R⁴ have the meaning given above, which is optionally isolated and/or purified,
and at least one compound of general formula IV is reacted with an activating agent in a reaction medium, optionally in an inert atmosphere, to yield at least one compound of general formula V, 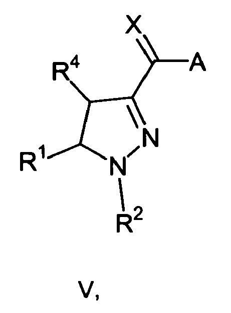 wherein R¹, X, R² and R⁴ have the meaning according given above and A represents a leaving group, which is optionally purified and/or isolated,
and at least one compound of general formula V is reacted with at least one compound of general formula R³-H, wherein R³ has the meaning given above, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula I, wherein R¹, R², X, R³ and R⁴ have the meaning given above and R⁵ represents hydrogen, which is optionally purified and/or isolated;
or at least one compound of general formula IV is reacted with at least one compound of general formula R³-H, wherein R³ represents a -NR⁷R⁸ moiety, whereby R⁷ and R⁸ have the meaning given above, in a reaction medium, in the presence of at least one coupling agent, optionally in the presence of at least one base, to yield at least one compound of general formula I, wherein R¹, R², X and R⁴ have the meaning given above, R³ represents a -NR⁷R⁸ moiety and R⁵ represents hydrogen, which is optionally purified and/or isolated.

Also described is the process for the preparation of a compound of general formula I given above, wherein R⁵ represents a hydrogen atom, according to which
at least one compound of general formula R¹-C(=O)-H (general formula VII), wherein R¹ has the meaning given above, is reacted with at least one compound of general formula VI, 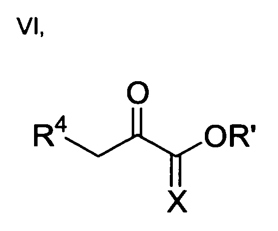 wherein R⁴ and X have the meaning given above and R' represents a linear or branched C₁₋₆-alkyl radical, preferably an ethyl radical, a potassium cation or a sodium cation, in a reaction medium, optionally in an inert atmosphere, optionally in the presence of at least one base, to yield at least one compound of general formula II, 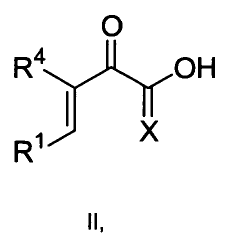 wherein R¹, X and R⁴ have the meaning given above, which is optionally purified and/or isolated,
and at least one compound of general formula II is reacted with an activating agent in a reaction medium, optionally in an inert atmosphere, to yield at least one compound of general formula VIII, 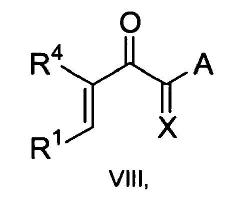 wherein R¹, X and R⁴ have the meaning given above and A represents a leaving group, which is optionally purified and/or isolated,
and at least one compound of general formula VIII is reacted with at least one compound of general formula R³-H, wherein R³ has the meaning given above, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula IX, 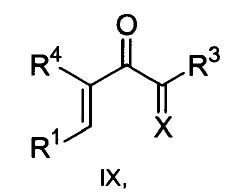 wherein R¹, X, R³ and R⁴ have the meaning given above, which is optionally purified and/or isolated;
or at least one compound of general formula II is reacted with at least one compound of general formula R³-H, wherein R³ represents a -NR⁷R⁸ moiety, whereby R⁷ and R⁸ have the meaning given above, in a reaction medium, in the presence of at least one coupling agent, optionally in the presence of at least one base, to yield at least one compound of general formula IX, wherein R³ represents a -NR⁷R⁸ moiety, which is optionally purified and/or isolated,
and at least one compound of general formula IX is reacted with at least one compound of general formula III, 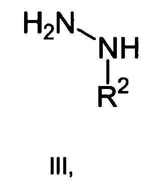 wherein R² has the meaning given above, in a reaction medium, optionally in an inert atmosphere, optionally in the presence of at least one acid, to yield a compound of general formula I, wherein R¹, X, R², R³ and R⁴ have the meaning given above and R⁵ represents hydrogen, which is optionally purified and/or isolated.

The process is also illustrated in scheme I given below:

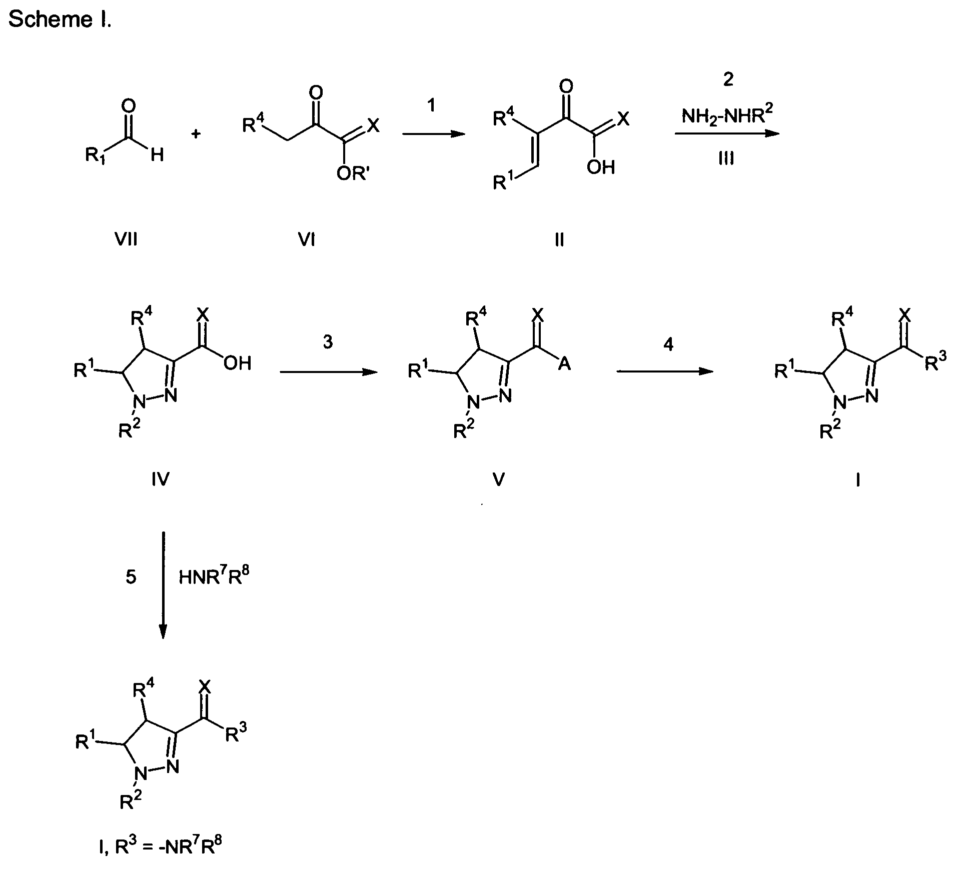

In step 1 a compound of general formula VI is reacted with a compound of general formula VII in a protic reaction medium, preferably in a reaction medium selected from the group consisting of methanol, ethanol, isopropanol, n-butanol, water and mixtures thereof, in the presence of at least one base, preferably in the presence of an alkali metal hydroxide such as sodium hydroxide or potassium hydroxide or an alkali metal methoxide such as sodium methoxide, as described, for example, in Synthetic Communications, 26(11), 2229-33, (1996). The respective description is hereby incorporated by reference and forms part of the disclosure. Reaction temperature as well as the duration of the reaction may vary over a broad range. Preferred reaction temperatures range from -10 °C to the boiling point of the reaction medium. Suitable reaction times may vary for example from several minutes to several hours.

Preferably the reaction between a compound of general formula VI and general formula VII can also be carried out under acid catalysed conditions, more preferably by refluxing the above mentioned compounds in dichloromethane in the presence of copper(II)trifluoromethanesulfonate as described, for example, in Synlett, (1), 147-149, 2001. The respective description is hereby incorporated by reference and forms part of the disclosure.

In step 2 a compound of general formula II is reacted with a compound of general formula III in a reaction medium, preferably in a reaction medium selected from the group consisting of methanol, ethanol, isopropanol, n-butanol, diethylether, tert-butyl-methylether, dioxane, Tetrahydrofuran or mixtures of at least two of these afore mentioned reaction media. Also preferably, said reaction may be carried out in the presence of an acid, whereby the acid may be organic such as acetic acid and/or inorganic such as hydrochloric acid. Alternatively the reaction may also be carried out in the presence of a base such as piperidine, piperazine, sodium hydroxide, potassium hydroxide, sodium methoxide or sodium ethoxide or mixtures of at least two of these bases.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours. This reaction preferably leads to racemates of compounds of general formula IV, wherein the substituents R¹ and R⁴ are located cis to one another on the pyrazoline ring. The respective enantiomers can be separated by conventional methods including chiral HPLC or resolution via the formation of diastereomeric salts and thus leading to compounds of general formula IV with an ee > 99 %. The configuration of compounds of general formula IV is maintained during the subsequent reaction steps, thus leading to enantiomerically pure compounds of general formula I (ee > 99 %). In step 2 racemates of compounds of general formula IV, wherein the substituents R¹ and R⁴ are located trans to one another on the pyrazoline ring, are also formed, albeit in a low yield.

In step 3 the carboxylic group of the compound of general formula IV may be activated for further reactions by the introduction of a suitable leaving group according to conventional methods well known to those skilled in the art. Preferably the compounds of general formula IV are transferred into an acid chloride, an acid anhydride, a mixed anhydride, a C₁₋₄ alkyl ester or an activated ester such as p-nitrophenylester. Suitable activating agent therefore are selected from the group consisting of thionyl chloride, oxalyl chloride and ethylchloroformate.

If said activated compound of general formula V is an acid chloride, wherein A represents a chlorine atom, that compound is preferably prepared by the reaction of the corresponding acid of general formula IV with thionyl chloride or oxalyl chloride, whereby said chlorinating agent is also used as the reaction medium, in the presence of at least one base, preferably in the presence of a base selected from the group consisting of triethylamine, N-methylmorpholine, pyridine, dimethylaminopyridine and diisopropylethylamine. Also preferably an additional reaction medium may be used. Suitable reaction media include hydrocarbons such as benzene, toluene or xylene, halogenated hydrocarbons such as dichloromethane, chloroform or carbon tetrachloride, ethers such as diethyl ether, dioxane, Tetrahydrofuran or dimethoxyethane or dimethylformamide and mixtures thereof. More preferably toluene in the presence of a catalytic amount of dimethylformamide is used as reaction medium. Preferred reaction temperature range from 0° C to the boiling point of the solvent and reaction times vary from several minutes to several hours.

If said activated compound of general formula V is a mixed anhydride, wherein A represents -O-C(=O)-O-C₂H₅, said anhydride may preferably be prepared, for example, by reaction of the corresponding acid of general formula IV with ethylchloroformate in the presence of a base such as triethylamine, pyridine or diisopropylethylamine, in a suitable solvent such as dichloromethane, optionally in an inert atmosphere, at a temperature between -50 °C and 50 °C.

In step 4 the reaction between a compound of general formula V with a compound of general formula H-R³ to yield a compound of general formula I, wherein R³ represents a -NR⁷R⁸ moiety, is preferably carried out in the presence of a base such as triethylamine in a reaction medium such as methylenchloride. The temperature is preferably in the range from 0 °C to the boiling point of the reaction medium. The reaction time may vary over a broad range, e.g. from several hours to several days.

Alternatively the reaction of a compound of general formula V with a compound of general formula H-R³ to yield compounds of general formula I may be carried out according to conventional methods well known to those skilled in the art, e.g. from Pascual, A., J. Prakt Chem., 1999, 341(7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of FeCl₃, ZnCl₂ and AlCl₃, in a suitable reaction medium such as toluene, benzene, Tetrahydrofuran or similar reaction media. The temperature is preferably in the range from 0° C to the boiling point of the reaction medium, more preferably from 15 to 25 °C. The reaction time may vary over a broad range, e.g. from several minutes to several hours.

In step 5 a compound of general formula IV is reacted with a compound of general formula H-R³, wherein R³ represents a -NR⁷R⁸ moiety, in a reaction medium, preferably in a reaction medium selected from the group consisting of diethylether, Tetrahydrofuran, acetonitrile, methanol, ethanol, (1,2)-dichlorethane, dimethylformamide, dichlormethane and mixtures thereof, in the presence of at least one coupling agent, preferably in the presence of a coupling agent selected from the group consisting of 1-benzotriazolyloxy-tris-(dimethylamino)-phosphonium hexafluorophosphate (BOP), dicyclohexylcarbodiimide (DCC), N'-(3-dimethylaminopropyl)-N-ethylcarbodiimide (EDCI), diisoproylcarbodiimide, 1,1'-carbonyl-diimidazole (CDI), N-[(dimethyamino)-1H-1, 2, 3-triazolo[4, 5-b]pyridino-1-ylmethylen]-N-methylmethanaminium hexafluorophosphate N-oxid (HATU), O-(benzotriazol-1-yl)-N,N,N',N'-tetramethyluroniom hexafluorophosphate (HBTU), O-(benzotriazol-1-yl)-N,N,N',N'-tetramethyluronium-tetrafluoroborate (TBTU), 1-hydroxy-benzotriazole (HOBt) and 1-hydroxy-7-azabenzotriazol (HOAt), optionally in the presence of a base, preferably in the presence of a base selected from the group consisting of pyridine, dimethylaminopyridine, N-methylmorpholine, triethylamine and diisopropylethylamine to yield a compound of general formula I, wherein R³ represents a -NR⁷R⁸ moiety.

Preferably said reaction is carried out in the presence of EDCI and HOBt, optionally in the presence of N-methylmorpholine or triethylamine, in an aprotic reaction medium such as dimethylformamide or Tetrahydrofuran, at a temperature between 20 °C and 30 °C for 15 to 24 hours as described in Tetrahedron Lett. 2004, 45, 4977. The respective description is hereby incorporated by reference and forms part of the disclosure. Polymer-supported EDCI (P-EDCI) can also suitably be used for this process instead of EDCI as described in Tetrahedron Lett. 1998, 39, 1487 and Tetrahedron Lett. 2002, 43, 7685. The respective descriptions are hereby incorporated by reference and form part of the disclosure.

Alternatively said reaction can be carried out by using HBTU in the presence of a base such as diisopropylethylamine in an aprotic solvent, such as acetonitrile, preferably at a temperature between 20 and 30 °C for 15 to 24 hours.

A further process to obtain compounds of general formula IV is illustrated in scheme II given below.

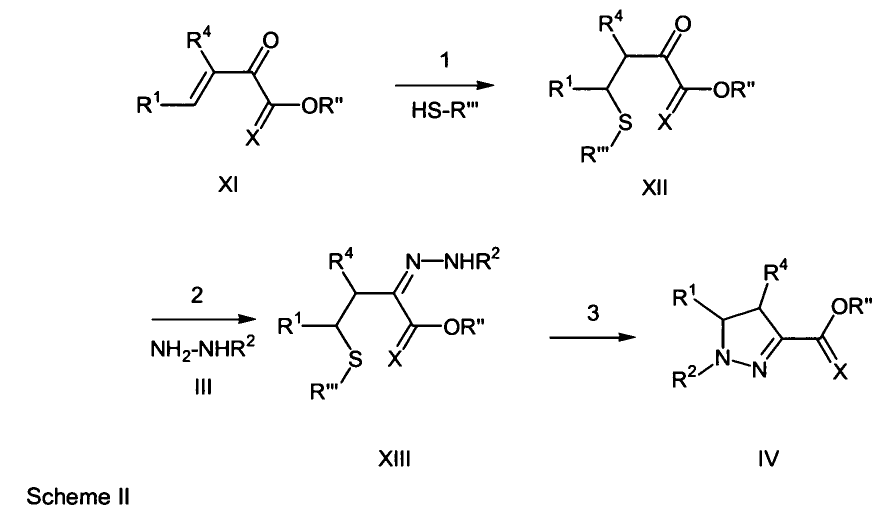

In step 1 a compound of general formula XI, wherein R¹, R⁴ and X have the meaning given above and R" represents a hydrogen atom or a C₁₋₆-alkyl radical, is reacted with a compound of general formula HS-R"', wherein R'" represents an unsubstituted or at least mono-substituted phenyl radical, in a reaction medium, preferably in an dry aprotic reaction medium, more preferably in toluene, optionally in the presence of an organic base, preferably in the presence of an organic base selected from the group consisting of triethylamine, pyridine, diisopropylethylamine, dimethylaminopyridine and N-methylmorpholine, preferably at a temperature between - 50 °C and 50 °C, preferably for 4 to 24 hours, to yield a compound of general formula XII, wherein R¹, R⁴, R", R"' and X have the meaning given above.

In step 2 a compound of general formula XII is reacted with a compound of general formula III, wherein R² has the meaning given above, in a reaction medium, preferably in a protic reaction medium, more preferably in methanol, optionally in the presence of an inorganic base, preferably in the presence of KHSO₄, preferably at a temperature between 0 °C and 100 °C, preferably for 4 to 15 hours, to yield a compound of general formula XIII, wherein R¹, R², R⁴, R", R'" and X have the meaning given above.

In step 3 the compound of general formula XIII is cyclized intramolecularly in a reaction medium, preferably in a dry aprotic reaction medium, more preferably in dimethylformamide, preferably under an inert atmosphere, in the presence of a base, preferably in the presence of a metal hydride salt, more preferably in the presence of sodium hydride and/or potassium hydride to yield a compound of general formula IV. If R" represents a C₁₋₆-alkyl radical, the compound of general formula IV, wherein R" represents a hydrogen atom, is obtained after saponification of the cyclized compound according to methods known to those skilled in the art.

The sequence illustrated in scheme 1 is also described in, for example, Tetrahedron 2005, 81, 5235 - 5240 and Tetrahedron Asymmetry 2001, 12, 1923 - 1928. The respective descriptions are hereby incorporated by reference and form part of the disclosure.

A compound of general formula IV can also be obtained as described in scheme III given below.

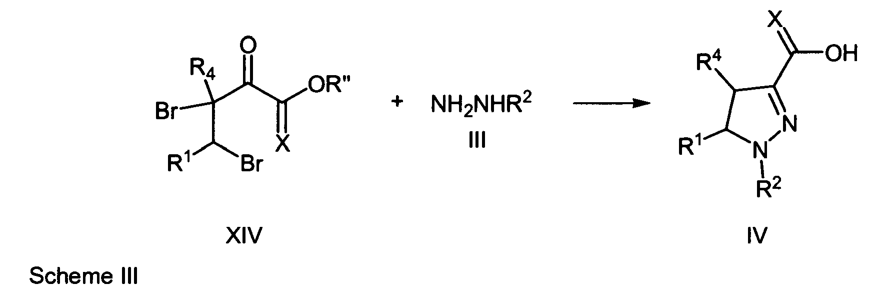

The compound of general formula XIV, wherein R¹, R⁴ and X have the meaning given above and R" represents a hydrogen atom or a C₁₋₆-alkyl radical, is obtained by the bromination of a compound of general formula XI in a reaction medium, preferably in an aprotic reaction medium, more preferably in dichloromethane, with bromine at a temperature between 0 °C and 30 °C for several hours as described in Tetrahedron Lett. 1998, 39 (44), 8163 - 8166; J. Chem. Soc. Perkin Trans 1, 1999, 21, 3069 - 3070; Tetrahedron 1999, 55 (36), 11127 - 11142 and J. Heterocyclic Chem. 1986, 23, 1199. Preferably a compound of general formula XIV is reacted with bromine in the presence of an aprotic solvent, preferably in the presence of dichloromethane, at ambient temperature for 1 to 2 hours. The respective descriptions are hereby incorporated by reference and form part of the disclosure.

The compound of general formula XIV is reacted with a compound of general formula III, wherein R² has the meaning given above, and cyclized intramolecularly in a reaction medium, preferably in a dry aprotic reaction medium, more preferably in dimethylformamide or in a mixture of dioxane, water and acetic acid, at a temperature between 0 °C and 250 °C to yield a compound of general formula IV as described in

Chemistry of Heterocyclic Compounds 1997, 33(6); Indian J. Chem. 20B, 1981, 1090; Indian J. Chem. 29B, 1990, 887 and J. Indian Chem. Soc. 1997, 74(3), 202 - 205. The respective descriptions are hereby incorporated by reference and form part of the disclosure. If R" represents a C₁₋₆-alkyl radical, the compound of general formula IV, wherein R" represents a hydrogen atom, is obtained after saponification of the cyclized compound according to methods known to those skilled in the art.

A compound of general formula IV can also be obtained by the process described in scheme IV.

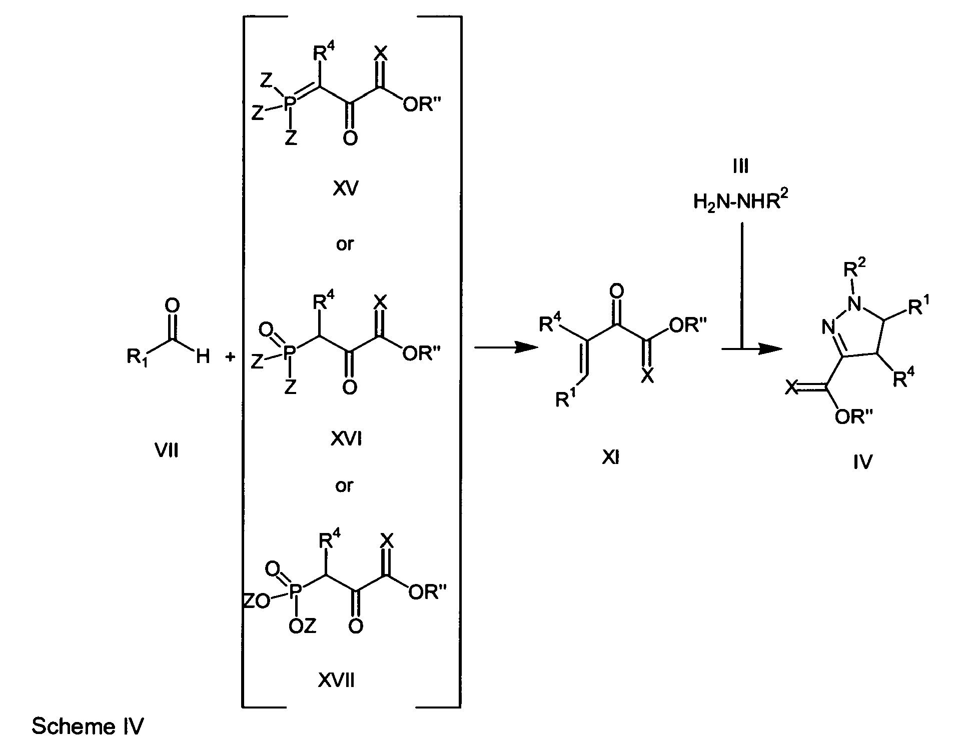

An aldehyde of general formula VII, wherein R¹ has the meaning given above, is reacted with either a phosphonium ylide of general formula XV, a phosphine oxide of general formula XVI or a phosphonate of general formula XVII, wherein in each case R⁴ and X have the meaning given above, R" represents a hydrogen atom or a C₁₋₆-alkyl radical and Z represents an unsubstituted or at least mono-substituted phenyl radical, in a reaction medium, preferably in an aprotic reaction medium, more preferably in Tetrahydrofuran, optionally in the presence of at least one base, preferably in the presence of a base selected from the group consisting of potassium tert-butylat, n-butyllithium, sodium hydride and lithium diisopropylamide, to yield a compound of general formula XI which is reacted with a compound of general formula III, wherein R² has the meaning given above, and cyclized intramolecularly to yield a compound of general formula IV as described above. The process is also described in Tetrahedron 1994, 50 (44), 12727 - 12742 and Zhumal Obshchei Khimii 1986, 56 (2), 347 - 353. The respective descriptions are hereby incorporated by reference and form part of the disclosure. If R" represents a C₁₋₆-alkyl radical, the compound of general formula IV, wherein R" represents a hydrogen atom, is obtained after saponification of the cyclized compound according to methods known to those skilled in the art.

Another method for the preparation of compounds of general formula XI is illustrated in scheme V below.

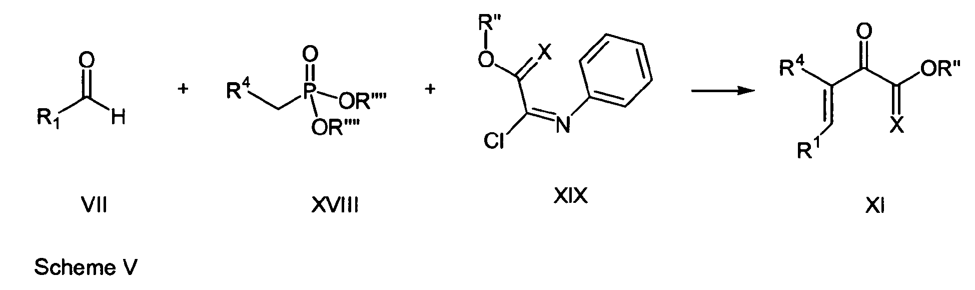

A compound of general formula VII, wherein R¹ has the meaning given above, is reacted with a phosphonate of general formula XVIII, wherein R⁴ has the meaning given above and R"" represents a C₁₋₆-alkyl radical, preferably an ethyl radical, and a compound of general formula XIX, wherein X has the meaning given above and R" represents a hydrogen atom or a C₁₋₆-alkyl radical to yield a compound of general formula XI, wherein R¹, R⁴, X and R" have the meaning given above. The process is described in J. Chem. Soc. Perkin Trans 1, 1995, 741-742. Preferably the reaction is carried out by the addition of phosphonate of general formula XVIII to a solution of n-butyllithium in a dry reaction medium, preferably in Tetrahydrofuran, at a temperature between -100 °C and - 50 °C, followed by the addition of N-phenylalcoxycarbonylacetimidoyl chloride of general formula XIX and aldehyde of general formula VII and stirring at a temperature between 0 °C and 30 °C for several hours. The respective description is hereby incorporated by reference and forms part of the disclosure.

A compound of general formula IV can also be obtained according to the process described in scheme VI.

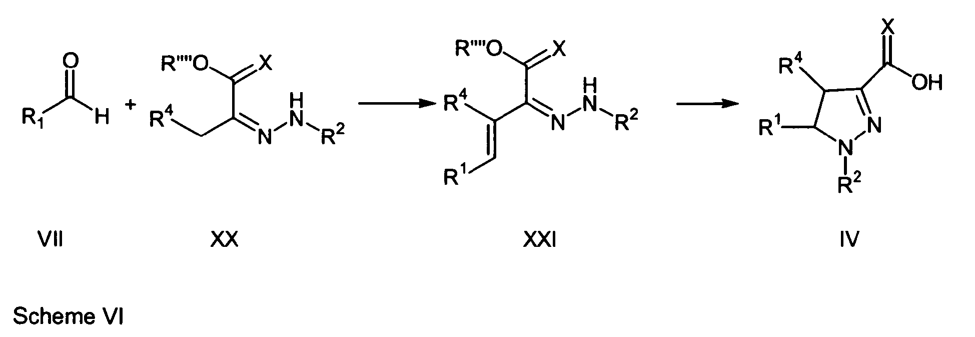

A compound of general formula VII, wherein R¹ has the meaning given above, is reacted with a compound of general formula XX, wherein R², R⁴ and X have the meaning given above and R"" represents a C₁₋₆-alkyl radical, is reacted in a reaction medium, preferably in ethanol, in the presence of a base, preferably sodium acetate, at a temperature between 30 °C and 90 °C, or in a reaction medium, preferably in ethanol, in the presence of glacial acetic acid at a temperature between 0 °C and 50 °C to yield a compound of general formula XXI, wherein R¹, R², R⁴ and X have the meaning given above and R"" represents a C₁₋₆-alkyl radical. The compound of general formula XXI is converted into the compound of general formula IV in a reaction medium, preferably in ethanol and/or water, in the presence of an acid, preferably in the presence of hydrochloric acid, at a temperature between 50 °C and 120 °C to yield a compound of general formula IV. The process is described in J. Chem. Engineering Data 1984, 29(2), 225 - 229 and Indian J. Chem. 27B, 1988, 3, 245-249. The respective descriptions are hereby incorporated by reference and form part of the disclosure.

A compound of general formula IV can also be obtained according to the process described in scheme VII. Said process is also described in WO88/005046. The respective description is hereby incorporated by reference and forms part of the disclosure.

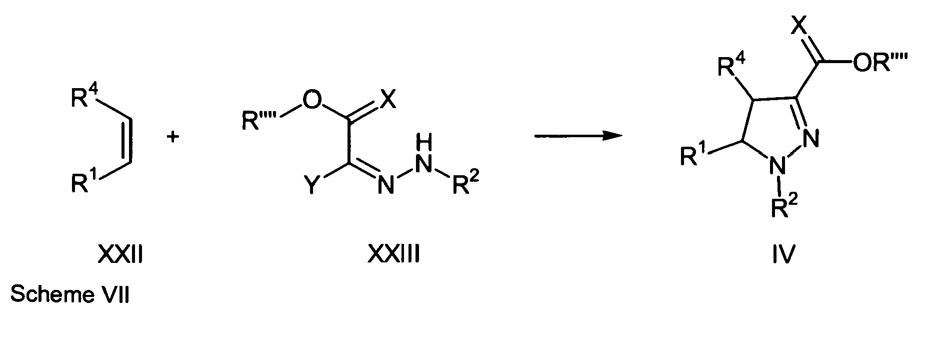

A compound of general formula XXII, wherein R¹ and R⁴ have the meaning given above, is reacted with a compound of general formula XXIII, wherein R² and X have the meaning given above, R"" represents a C₁₋₆-alkyl radical and Y represents a chlorine or bromine atom, in a reaction medium, preferably in an aprotic or protic reaction medium, more preferably in toluene and/or chloroform and/or ethanol, in the presence of a base, preferably an organic base, more preferably an organic base selected from the group consisting of triethylamine, pyridine, diisopropylethylamine, dimethylaminopyridine, 1,4-diazabicyclo[2.2.2]octane and N-methylmorpholine, at a temperature between 0 °C and 150 °C to yield a compound of general formula IV. If regioisomers are obtained during the reaction, these regioisomers can be separated by conventional chromatographic techniques. The compound of general formula IV, wherein R"" represents a C₁₋₆-alkyl radical is converted into the corresponding acid by using standard methods which are known to those skilled in the art. The process is disclosed in Bull. Chem. Soc. Japan 1984, 57 (3), 787 - 790 and Chem. Lett. 1982, 543 - 546. The respective descriptions are hereby incorporated by reference and form part of the disclosure. The method as depicted in scheme VII is stereospecific. Thus, using (E)-olefins of general formula XII compounds of general formula IV are obtained, wherein the radicals R¹ and R⁴ on the pyrazoline ring are orientated trans to one another. Starting from (Z)-olefins the respective pyrazolines with cis-configuration are obtained. The racemates of pyrazoline compounds with either trans- or cis-configuration can be separated by conventional methods including chiral HPLC separation and separation via the formation of diastereomeric salts. Thus, enantiomerically pure compounds are obtained (ee > 99%). The configuration of compounds of general formula IV is maintained during the subsequent reaction steps, thus leading to enantiomerically pure compounds of general formula I (ee > 99 %).

The compound of general formula XXIII can be prepared according to the processes described in scheme VIII.

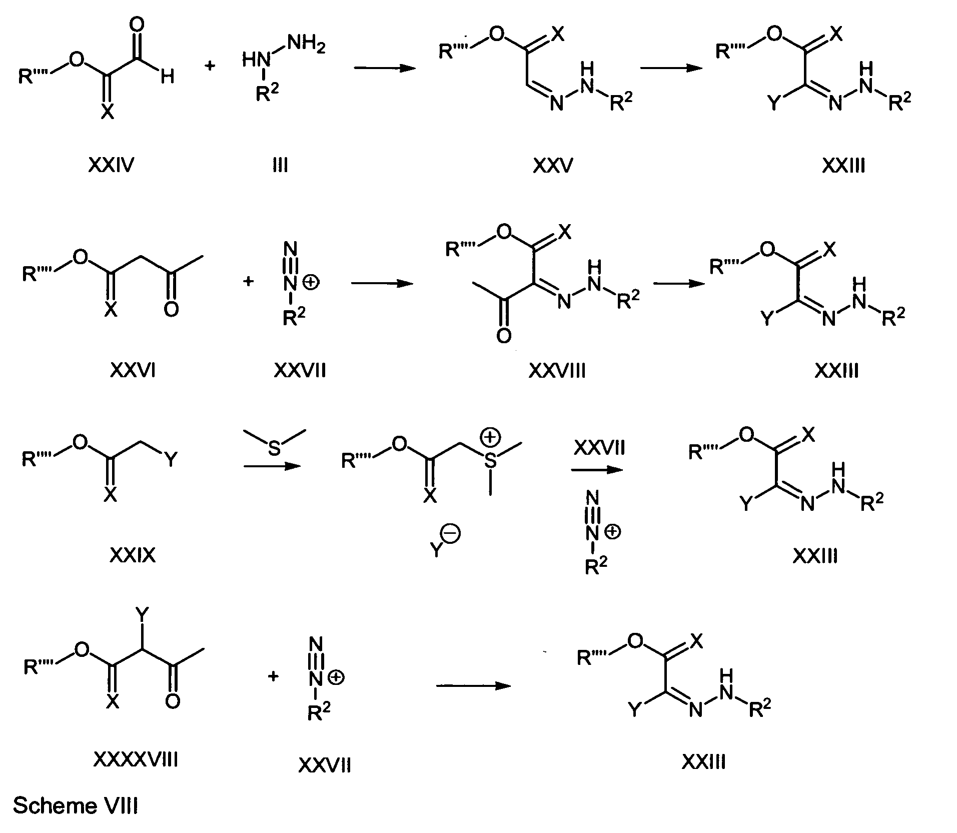

The compound of general formula XXIV, wherein X has the meaning given above and R"" represents a C₁₋₆-alkyl radical, is reacted in a reaction medium, preferably in a mixture of water and ethanol, in the presence of at least one acid, preferably in the presence of acetic acid, at a temperature between 70 °C and 120 °C with a compound of general formula III, wherein R² has the meaning given above, to yield a compound of general formula XXV, wherein R² and X have the meaning given above and R"" represents a C₁₋₆-alkyl radical, which is reacted with N-chloro-succinimide or N-bromo-succinimide in a reaction medium, preferably in an aprotic reaction medium, more preferably in dimethylformamide, at a temperature between 0 °C and 30 °C, to yield a compound of general formula XXIII. The process is described in Synth. Commun. 2001, 31(1), 111 - 115 and Tetrahedron 1994, 50 (25), 7543 - 7556. The respective descriptions are hereby incorporated by reference and form part of the disclosure.

The compound of general formula XXIII can also be prepared by the reaction of a compound of general formula XXVI, wherein X has the meaning given above and R"" represents a C₁₋₆-alkyl radical, with a compound of general formula XXVII, wherein R² has the meaning given above and subsequent bromination of the resulting compound of general formula XXVIII, wherein R² and X have the meaning given above and R"" represents a C₁₋₆-alkyl radical, by using bromine in the presence of acetic acid as described in Synthesis 1975, 333 and J. Chem. Soc. Perkin Trans. 1, 1977, 2092. The diazonium salt of general formula XXVII can preferably be obtained by the addition of an aqueous solution of sodium nitrite to a compound of general formula R²-NH₂ in aqueous hydrochloride acid, wherein R² has the meaning given above. Alternatively this transformation can also be achieved in the presence of a compound of general formula XXVI by adjusting the pH of the reaction medium to 4 by the addition of sodium acetate at a temperature between 0 °C and 30 °C. The respective descriptions are hereby incorporated by reference and form part of the disclosure.

The compound of general formula XXIII can also be prepared by the reaction of a compound of general formula XXIX, wherein X has the meaning given above, R"" represents a C₁₋₆-alkyl radical and Y represents a chlorine or bromine atom, with dimethylsulfide, in a reaction medium, preferably in ethanol, at a temperature between 70 °C and 120 °C. Optionally the dimethylsulfonium salt is isolated and further reacted with a compound of general formula XXVII, wherein R² has the meaning given above, in the presence of sodium acetate and acetic acid at a temperature between 0 °C and 30 °C as described in Heterocycles 1991, 32(6), 1101 - 1107. The respective description is hereby incorporated by reference and forms part of the disclosure.
The compound of general formula XXIII can also be prepared by the reaction of a compound of general formula XXXXVIII, wherein X has the meaning given above, R"" represents a C₁₋₆-alkyl radical and Y represents a leaving group, preferably a leaving group selected from the group consisting of chlorine and bromine, with a compound of general formula XXVII, wherein R² has the meaning given above, in the presence of a protic solvent, preferably in the presence of a protic solvent selected from the group consisting of methanol and ethanol, or in the presence of an aprotic solvent, preferably in the presence of tetrahydrofuran, in the presence of a base, preferably in the presence of sodium acetate, or in the presence of an acid, preferably in the presence of acetic acid. The method is described in J. Chem. Soc. Perkin Transaction 1 1998, 24, 4103-4106; Tetrahedron Asymmetry 2000, 11(9), 1975-1983; Tetrahedron 1998, 54(49), 14859-14868; Synthesis 1996, 9, 1076-1078 and Synthesis 1995, 12, 1483-1484. The respective descriptions are hereby incorporated by reference and form part of the disclosure.

Another method for the preparation of a compound of general formula IV is described in scheme IX.

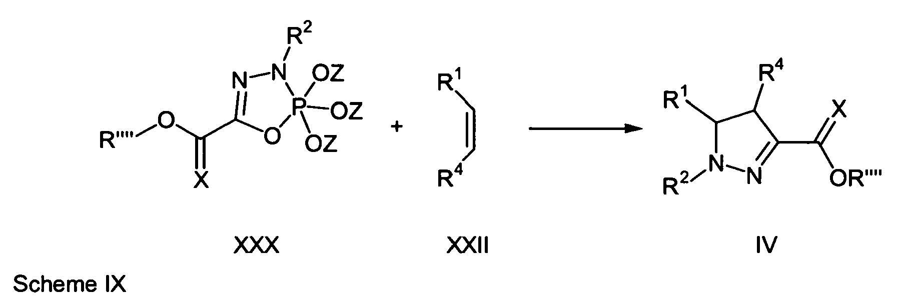

A compound of general formula XXX, wherein R² and X have the meaning given above, Z represents an unsubstituted or at least mono-substituted phenyl radical, preferably an unsubstituted phenyl radical, and R"" represents a C₁₋₆-alkyl radical, preferably an ethyl radical, is reacted with a compound of general formula XXII, wherein R¹ and R⁴ have the meaning given above, in a reaction medium, preferably in xylene, at a temperature between 50 °C and 200 °C for 2 to 30 hours to yield a compound of general formula IV. The process is described in Chem. Lett. 1983, 507 - 510 and Bull. Chem. Soc. Japan 1984, 57(9), 2689 - 2690. The respective descriptions are hereby incorporated by reference and form part of the disclosure. The compound of general formula IV, wherein R"" represents a C₁₋₆-alkyl radical, is converted into the corresponding acid by using standard methods which are known to those skilled in the art.

A process for the preparation of a compound of general formula XXX is described in scheme X.

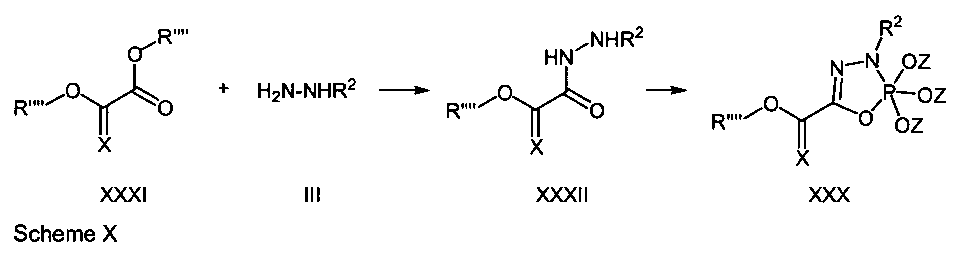

A compound of general formula XXXI, wherein X has the meaning given above and R"" is a C₁₋₆-alkyl radical, is reacted with a compound of general formula III, wherein R² has the meaning given above, to yield a compound of general formula XXXII, wherein R² and X have the meaning given above and R"" represents a C₁₋₆-alkyl radical. Subsequently, the compound of general formula XXXIII is reacted with phosphorous pentachloride or POCl₃ in a reaction medium, preferably in toluene, at a temperature between 0 °C and 50 °C, followed by the addition of a phenolic compound, preferably O-trimethylsilyl-p-cresol, in refluxing toluene to yield a compound of general formula XXX.

Another method for the preparation of a compound of general formula IV is described in scheme XI.

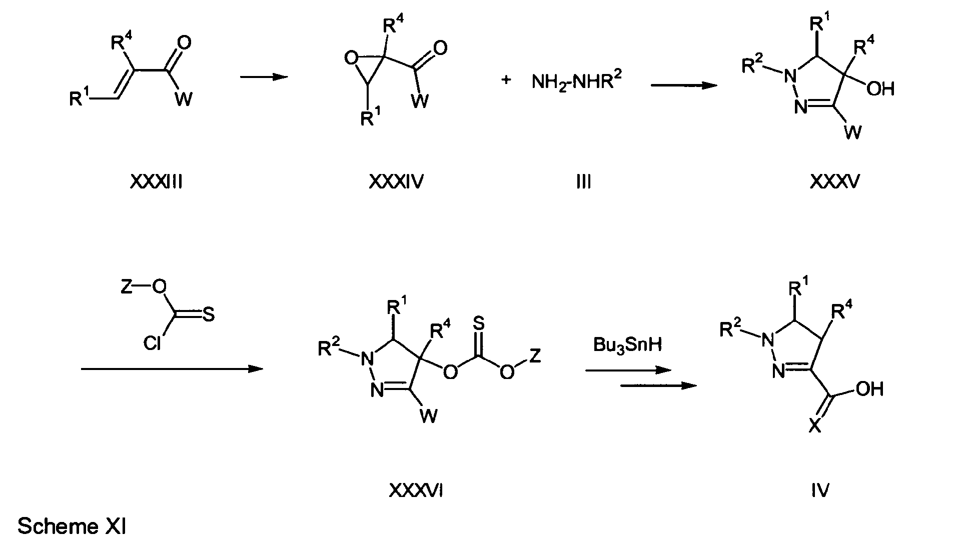

A compound of general formula XXXIII, wherein R¹ and R⁴ have the meaning given above and W represents -C(=X)-OH, -C(=X)-OR"" or -CN, whereby X has the meaning given above and R"" represents a C₁₋₆-alkyl radical, is converted to a compound of general formula XXXIV, wherein R¹, R⁴ and W have the meaning given above, by means of epoxidation with a reagent selected from the group consisting of perbenzoic acid, preferably m-chloroperbenzoic acid, sodium peroxocarbonate, hydrogen peroxide, dioxirane and hydroperoxide. The compound of general formula XXIV is reacted with a compound of general formula III, wherein R² has the meaning given above, in a reaction medium, preferably in ethanol, to yield a compound of general formula XXXV, wherein R¹, R², W and R⁴ have the meaning given above. Subsequently, the compound of general formula XXXV is converted into the corresponding xanthate of general formula XXXVI, wherein R¹, R², W and R⁴ have the meaning given above, by reaction with an unsubstituted or at least mono-substituted phenylthionochloroformate of general formula ZO-C(=S)-Cl, wherein Z represents an unsubstituted or at least mono-substituted phenyl radical. The compound of general formula XXXVI is reacted with tributyltinhydride optionally followed by saponification and/or hydrolysis to yield a compound of general formula IV, wherein R¹, R², X and R⁴ have the meaning given above. The process is described in Synlett 1990, 11, 705-706. The respective description is hereby incorporated by reference and forms part of the disclosure.

Yet another method for the preparation of a compound of general formula IV is described in scheme XII.

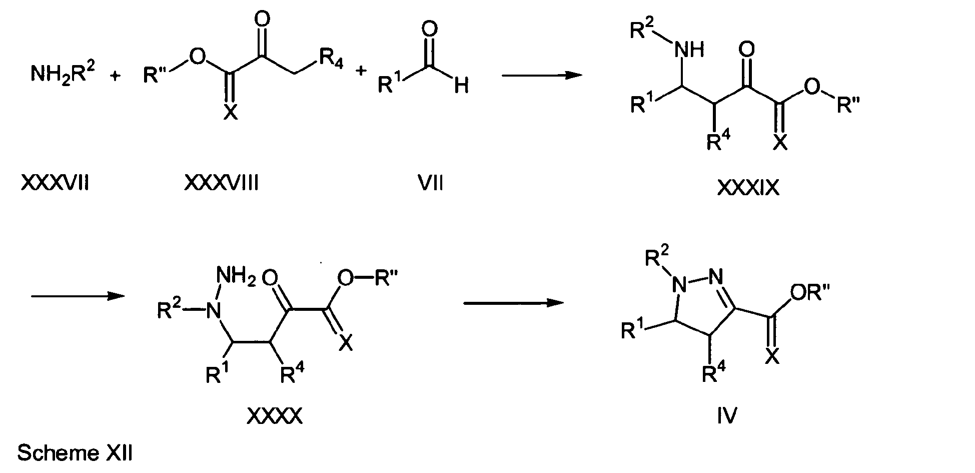

A compound of general formula XXXVII, wherein R² has the meaning given above, is reacted with a compound of general formula XXXVIII, wherein R⁴ and X have the meaning given above and R" represents a hydrogen atom or a C₁₋₆-alkyl radical, and a compound of general formula VII, wherein R¹ has the meaning given above, to yield a compound of general formula XXXIX, wherein R¹, R², R⁴ and X have the meaning given above and R" represents a hydrogen atom or a C₁₋₆-alkyl radical. The compound of general formula XXXIX is converted into the compound of general formula XXXX by using O-substituted hydroxylamines according to the method described in J. Org. Chem. 2002, 67, 6237 - 6239. The respective description is hereby incorporated by reference and forms part of the disclosure. Alternatively, this transformation can be achieved by using nitrites and subsequent reduction according to methods known to those skilled in the art. Upon cyclization of a compound of general formula XXXX according to the methods described above a compound of general formula IV is obtained. If R" represents a C₁₋₆-alkyl radical, the compound of general formula IV, wherein R" represents hydrogen, is obtained after saponification of the cyclized compound according to methods known to those skilled in the art.

In case R⁵ is unlike hydrogen the reaction sequence given in Scheme I is adapted as follows.

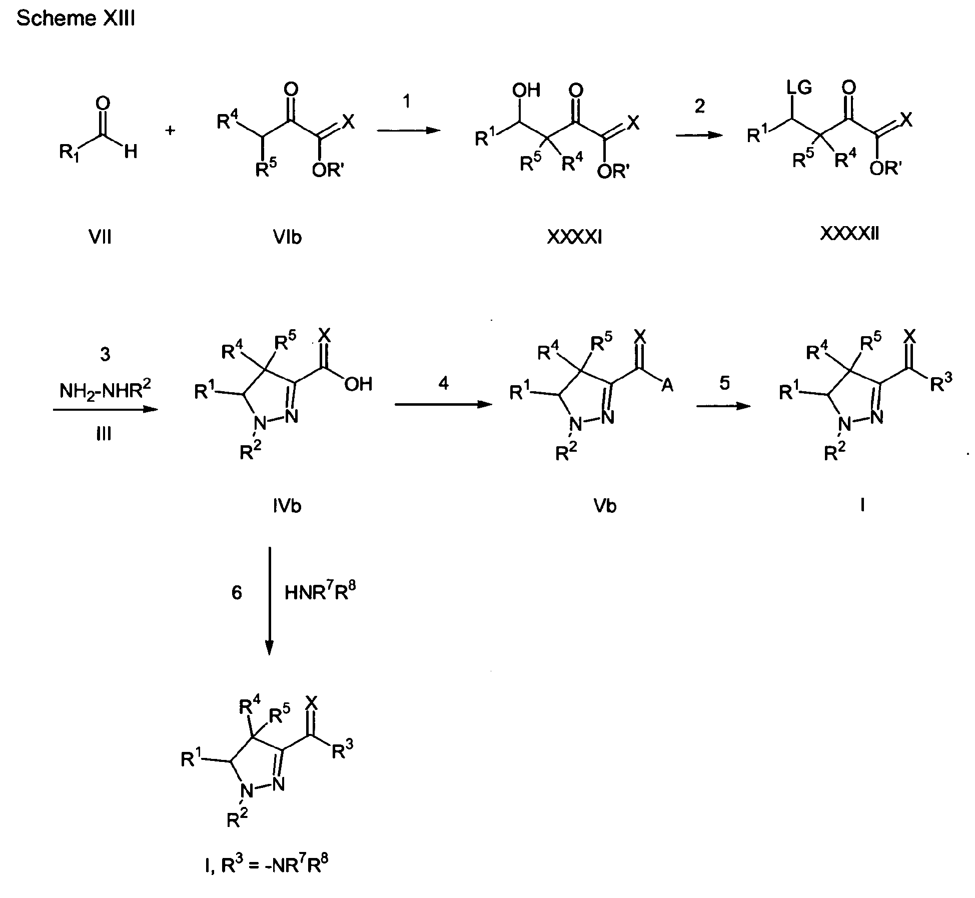

In step 1 a compound of general formula VIb or a corresponding enolate of said compound is reacted with a compound of general formula VII in a reaction medium , preferably in a protic reaction medium, more preferably in a reaction medium selected from the group consisting of methanol, ethanol, isopropanol, n-butanol, water and mixtures thereof, in the presence of at least one base, preferably in the presence of an alkali metal hydroxide such as sodium hydroxide or potassium hydroxide or an alkali metal methoxide such as sodium methoxide or in the presence of lithium diisopropylamide in an aprotic solvent, preferably in tetrahydrofuran.

In step 2 a compound of general formula XXXXI is transformed into a compound of general formula XXXXII which contains a good living group LG, preferably a leaving group selected from the group consisting of mesyl and tosyl, using conventional methods known to those skilled in the art.

In step 3 a compound of general formula XXXXII is reacted with a compound of general formula III in a reaction medium, preferably in a reaction medium selected from the group consisting of methanol, ethanol, isopropanol, n-butanol, dieethylether, tert-butyl-methylether, dioxane, tetrahydrofuran or mixtures of at least two of these afore mentioned reaction media. Also preferably, said reaction may be carried out in the presence of an acid, whereby the acid may be organic such as acetic acid and/or inorganic such as hydrochloric acid. Alternatively the reaction may also be carried out in the presence of a base such as piperidine, piperazine, sodium hydroxide, potassium hydroxide, sodium methoxide or sodium ethoxide or mixtures of at least two of these bases. Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

Step 4 can be carried out as described for step 3, scheme I; step 5 can be carried out as described for step 4, scheme I and step 6 can be carried out as described for step 5, scheme 1.

The compounds of general formula I, wherein R⁵ is unlike hydrogen, can also be obtained by the reaction sequence described in scheme XIV.

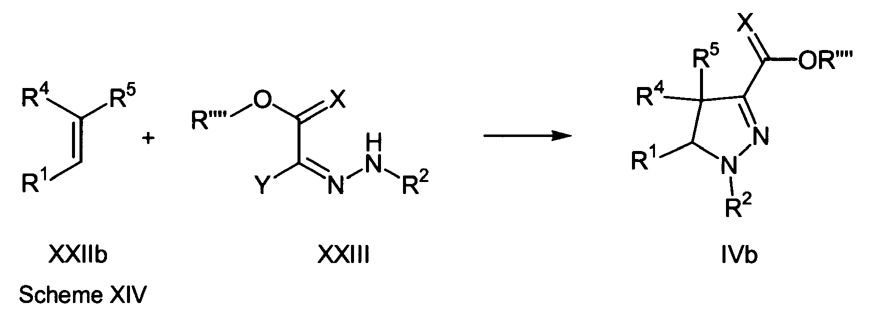

The reaction is carried out as described for the analogues reaction depicted in scheme VII. If a mixture of regioisomers is obtained, said regioisomers can be separated by standard methods known to those skilled in the art, e. g. chromatographic methods or crystallization. The process is disclosed in Bull. Chem. Soc. Japan 1984, 57 (3), 787 - 790. The respective description is hereby incorporated by reference and forms part of the disclosure.
The compounds of general formula I, wherein R³ represents -NR⁷R⁸, can also be obtained by the reaction sequence described in scheme XV.

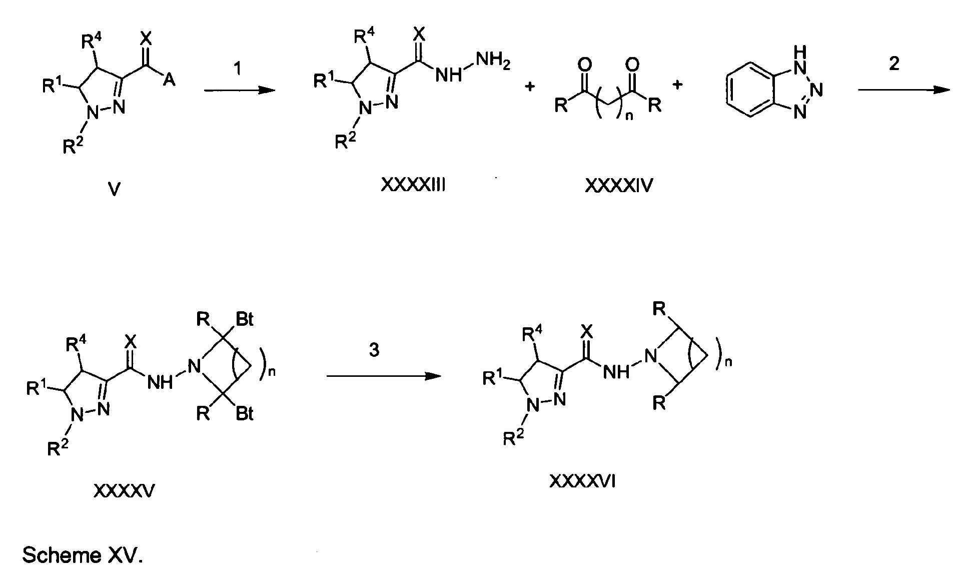

In step 1 a compound of general formula V is reacted with hydrazine hydrate in the presence of an aprotic or protic solvent, preferably in the presence of ethanol, at reflux temperature to yield a compound of general formula XXXXIII, wherein R¹, R², R⁴ and X have the meaning as defined above.

In step 2 a compound of general formula XXXXIII is reacted with a compound of general formula XXXXIV, wherein R represents a hydrogen atom or a linear or branched C₁₋₁₂ alkyl radical and n is 0, 1, 2, 3, 4, 5, 6, 7, 8, 9 or 10, in the presence of benzotriazole to yield a compound of general formula XXXXV, wherein R, n, R¹, R², R⁴ and X have the meaning as defined above and Bt represents a benzotriazolyl radical. A compound of general formula XXXXV can be transformed into a compound of general formula XXXXVI, wherein R, n, R¹, R², R⁴ and X have the meaning as defined above, in the presence of a reducing agent, preferably in the presence of sodium borohydride, in the presence of an aprotic solvent, preferably in the presence of tetrahydrofuran. Alternatively, the benzotriazole moiety in compounds of general formula XXXXV can be replaced by a linear or branched C₁₋₁₀alkyl group via reaction with the respective alkyl Grignard reagents The process is disclosed J. Org. Chem. 1990, 55, 3205-3209. The respective description is hereby incorporated by reference and forms part of the disclosure.

The compounds of general formula I, wherein R³ represents -NR⁷R⁸, can also be obtained by the reaction sequence described in scheme XVI.

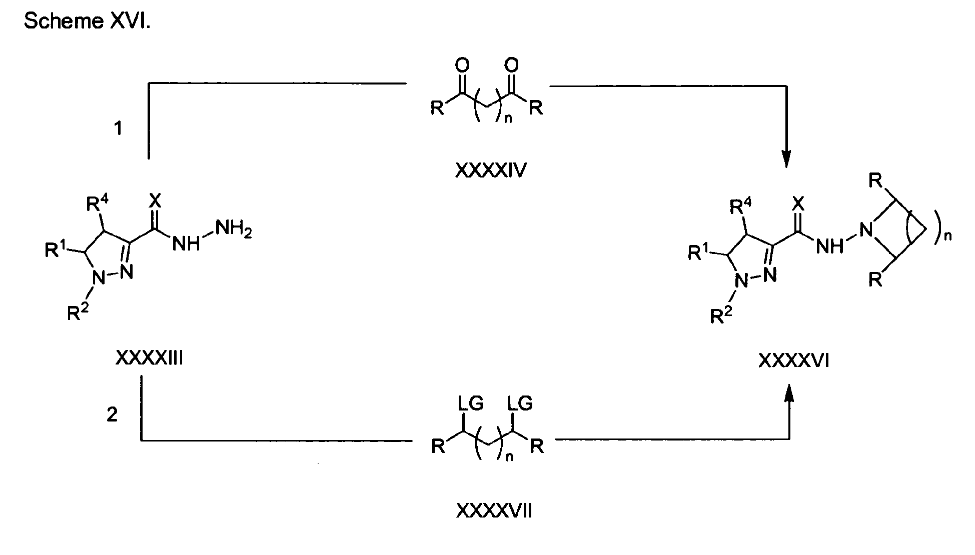

In step 1 a compound of general formula XXXXIII is reacted with a compound of general formula XXXXIV, wherein R represents a hydrogen atom or a linear or branched C₁₋₂alkyl radical and n is 0, 1, 2, 3, 4, 5, 6, 7, 8, 9 or 10, in the presence of a reducing agent, preferably in the presence of a reducing agent selected from the group consisting of sodium borohydride, sodium cyanoborohydride or triacetoxyborohydride, to yield a compound of general formula XXXXVI, wherein R, n, R¹, R², R⁴ and X have the meaning as defined above.

In step 2 a compound of general formula XXXXIII is reacted with a compound of general formula XXXXVII, wherein R represents a hydrogen atom or a linear or branched C₁₋₁₂alkyl radical, n is 0, 1, 2, 3, 4, 5, 6, 7, 8, 9 or 10 and LG represents a leaving group, preferably a leaving group selected from the group consisting of chlorine and bromine, more preferably LG represents bromine, in the presence of a base, preferably in the presence of potassium carbonate, to yield a compound of general formula XXXXVI, wherein R, n, R¹, R², R⁴ and X have the meaning as defined above.

The compounds of general formula I can also be obtained by the reaction sequence described in scheme XVII.

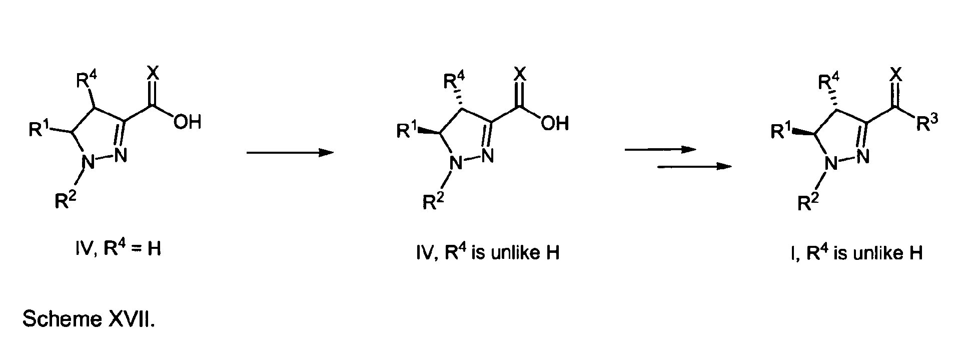

A compound of general formula IV, wherein R⁴ represents hydrogen and R¹, R² and X have the above defined meaning, is transformed into a compound of general formula IV, wherein R⁴ is unlike hydrogen and R¹, R², R⁴ and X have the above defined meaning, in the presence of a base, preferably in the presence of a base selected from the group consisting of sodium hydride, lithium diisopropylamide and lithium hexamethyldisilazide, in the presence of an aprotic solvent, preferably in the presence of an aprotic solvent selected from the group consisting of tetrahydrofuran, dichloromethane and chloroform, and in the presence of a compound of general formula R⁴-X, wherein R⁴ has the above defined meaning, preferably R⁴ represents a linear or branched C₁₋₁₀alkyl radical or CN, and X represents a leaving group, preferably a leaving group selected from the group consisting of bromine and iodine. The compounds of general formula IV, wherein R⁴ is unlike hydrogen, are obtained as racemates, and can be separated by conventional methods including HPLC and separation via formation of diastereomeric salts. The compounds of general formula IV can be transformed into compounds of general formula I while retaining the absolute configuration of the compounds of general formula I.

A compound of general formula IV, wherein both R⁴ and R⁵ are different from hydrogen and R¹, R⁴, R² and X have the above defined meaning, can be prepared from compounds of general formula IV, wherein R⁵ is hydrogen, R⁴ is unlike hydrogen and R¹, R⁴, R² and X have the above defined meaning, and a compound of general formula R⁵-X, wherein R⁵ has the above defined meaning, preferably R⁵ represents a linear or branched C₁₋₁₀alkyl radical or CN, and X represents a leaving group, preferably a leaving group selected from the group consisting of bromine and iodine, by applying the same method as described in scheme XVII.

Compounds of general formula I, wherein X represents O, can be transformed in the corresponding compound, wherein X represents S, by reacting the first compound with a dithiaphosphetane, preferably with 2,4-bis(4-methoxyphenyl)-1,3,2,4-dithiadiphosphetan-2,4-disulfide (Lawesson reagent) or phosphorous pentasulfide, in a reaction medium, preferably in a reaction medium selected from the group consisting of toluene, xylene, acetonitrile, dichloromethane and dimethylformamide, at a temperature between 50 °C to 150 °C.

The afore mentioned reactions involving the synthesis of the 4,5-dihydro-pyrazole ring or the reaction of a compound comprising said ring are preferably carried out under an inert atmosphere, preferably under a nitrogen or argon atmosphere, to avoid oxidation of the ringsystem.

During some synthetic reactions described above the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted pyrazoline compounds of general formula I are obtained in form of a mixture of stereoisomers, particularly enantiomers or diastereomers, said mixtures may be separated by standard procedures known to those skilled in the art, e.g. chromatographic methods or crystallization with chiral reagents. It is also possible to obtain pure stereoisomers via stereoselective synthesis.

It is also described a process for the preparation of salts of substituted pyrazoline compounds of general formula I and stereoisomers thereof, wherein at least one compound of general formula I having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

It is also described a process for the preparation of salts of substituted pyrazoline compounds of general formula I or stereoisomers thereof, wherein at least one compound of general formula I having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄-alkyl-radical. Suitable reaction media are, for example, any of the ones given above.

In the presence of several acidic or basic groups, mono- or poly-salts may be formed. Compounds of the formula I having an acidic group, for example a free carboxyl group, and a basic group, for example an amino group, may also be present in the form of inner salts, i.e., in zwitterionic form, or a part of the molecule may be present in the form of an inner salt and another part in the form of a normal salt.

Solvates, preferably hydrates, of the substituted pyrazoline compounds of general formula I, of corresponding stereoisomers, of corresponding N-oxides or of corresponding salts thereof may also be obtained by standard procedures known to those skilled in the art.

substituted pyrazoline compounds of general formula I, which comprise nitrogen-atom containing saturated, unsaturated or aromatic rings may also be obtained in the form of their N-oxides by methods well known to those skilled in the art.

The purification and isolation of the substituted pyrazoline compounds of general formula I, of a corresponding stereoisomer, or salt, or N-oxide, or solvate or any intermediate thereof may, if required, be carried out by conventional methods known to those skilled in the art, e.g. chromatographic methods or recrystallization.

The compounds of general formula I given above may also act as prodrugs, i.e. they represent a drug precusor, which following administration to a patient releases a drug in vivo via some kind of chemical and/or physiological process (e.g., a prodrug on being brought to a physiological pH and/or through an enzyme action is converted to a desired drug form; see, e.g., R. B. Silverman, 1992, "The Organic Chemistry of Drug Design and Drug Action", Academic Press, Chp. 8). In particular, the compounds of general formula I give rise to a compound of general formula I, wherein R³ represents a -OH moiety, upon administration to a patient.

Prodrugs can be used to alter the biodistribution (e.g., to allow compounds which would not typically enter the reactive site of the protease) or the pharmacokinetics for a particular compound. For example, a hydroxyl group, can be esterified, e.g., with a carboxylic acid group to yield an ester. When the ester is administered to a subject, the ester is cleaved, enzymatically or non-enzymatically, reductively or hydrolytically, to reveal the hydroxyl group.

The substituted pyrazoline compounds of general formula I given above, their stereoisomers, corresponding N-oxides, corresponding salts thereof and corresponding solvates are toxicologically acceptable and are therefore suitable as pharmaceutical active substances for the preparation of medicaments.

It has been found that the substituted pyrazoline compounds of general formula I given above, stereoisomers thereof, N-oxides thereof, corresponding salts and corresponding solvates have a high affinity to cannabinoid receptors, particularly cannabinoid 1 (CB₁)-receptors, i.e. they are selective ligands for the CB₁-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the pyrazoline compounds will again show the desired effect. After ending the treatment with the pyrazoline compounds, the positive influence on the body weight is found to continue.

Furthermore, these substituted pyrazoline compounds show relatively weak Herg channel affinity, thus a low risk of prolongation of the QT-interval is to be expected for these compounds.

In summary, the 4-subsituted pyrazoline compounds are distinguished by a broad spectrum of beneficial effects, while at the same time showing relatively little undesired effects, i.e. effects which do not positively contribute to or even interfere with the well being of the patient.

The purification and isolation of the substituted pyrazoline compounds of general formula (I), of a corresponding stereoisomer, or salt, or N-oxide, or solvate or any intermediate thereof may, if required, be carried out by conventional methods known to those skilled in the art, e.g. chromatographic methods or recrystallization.

It is highly preferrd if the active substance combination according to the invention is comprising
(A) one substituted pyrazoline compounds of general formula I selected from the group consisting of
   (rac)-N-piperidinyl-5-(4-chloro-phenyl)-1-(2,4-dichlorophenyl)-4,5-dihydro-1H-pyrazol-3-carboxamide,
   (R)-N-piperidinyl-5-(4-chloro-phenyl)-1-(2,4-dichlorophenyl)-4,5-dihydro-1H-pyrazol-3-carboxamide, or
   (S)-N-piperidinyl-5-(4-chloro-phenyl)-1-(2,4-dichlorophenyl)-4,5-dihydro-1H-pyrazol-3-carboxamide,
   in any mixing ratio, or a corresponding N-oxide thereof, or a corresponding salt thereof, or a corresponding solvate thereof,
   and
(B) at least one drug of component (B) selected from the following therapeutic classes: antipsychotics, comprising the atypical antipsychotics and typical neuroleptics, antidepressants including MAOls, TCAs, SSRls, "third generation" and atypical antidepressants, anti-manic agents, corticosteroids, medicaments for treating insulin resistance, impaired glucose tolerance and/or Type 2 diabetes, antihypertensives, anti-ulceratives, medicaments for treating neuropathic pain, antihistamines, medicaments for treating migraine, benzodiazepines, contraceptives and for hormone replacement therapy.

It is also highly preferrd if the active substance combination according to the invention is comprising
(A) one substituted pyrazoline compounds of general formula I selected from the group consisting of
   (rac)-N-piperidinyl-5-(4-chloro-phenyl)-1-(2,4-dichlorophenyl)-4,5-dihydro-1H-pyrazol-3-carboxamide,
   (R)-N-piperidinyl-5-(4-chloro-phenyl)-1-(2,4-dichlorophenyl)-4,5-dihydro-1H-pyrazol-3-carboxamide, or
   (S)-N-piperidinyl-5-(4-chloro-phenyl)-1-(2,4-dichlorophenyl)-4,5-dihydro-1H-pyrazol-3-carboxamide,
   in any mixing ratio, or a corresponding N-oxide thereof, or a corresponding salt thereof, or a corresponding solvate thereof,
   and
(B) at least one drug of component (B) selected from
   Antipsychotics, comprising the atypical antipsychotics including, olanzapine clozapine, quetiepine, risperidone, amisulpiride, zotepine, sertindole, aripiprazole and ziprasidone, and the older typical neuroleptics including, chlorpromazine, haloperidol, fluphenazine, mesoridazine and thioridazine;
   Antidepressants, comprising the non-selective, irreversible monoamine oxidase A and B inhibitors (MAOls) including tranylcypromine, phenelzine and isocarboxacid, the tricyclic antidepressants (TCAs) including, amitriptyline, amoxapine, clomipramine, desipramine, doxepin, imiprimine, nortriptyline, protriptyline and trimiprimine, the serotonin-selective reuptake inhibitors including fluoxetine, sertraline, paroxetine and citalopram, the "third generation" and atypical antidepressants" including mirtazepine, venlafaxine, mianserin and trazodone;
   Drugs to treat mania in bipolar disorder including lithium, olanzapine, quetiepine and sodium valproate;
   Corticosteroids to treat inflammation including, prednisone, prednisolone, budesonide, dexamethasone, methylprednisolone and betamethasone;
   Drugs for the treatment of Type 1 diabetes, insulin resistance, impaired glucose tolerance and Type 2 diabetes including the sulphonylureas (chlorpropamide, tolbutamide, acetoheximide, tolazamide, glibenclamide, glipizide, gliclazide, glimepiride, gliquidone, glyburide), the meglitinides (netaglinide, repaglinide, mitiglitinide), peroxisome proliferator activated receptors type gamma (PPARγ) agonists (troglitazone, rosiglitazone, pioglitazone, ciglitazone, darglitazone), inhibitors of hepatic glucose production (glucagon antagonists, glucose-6-phosphatase inhibitors, glycogen phosphorylase inhibitors), insulin (human insulin lispro, human insulin aspart, neutral insulin, human neutral insulin [pyr], human neutral insulin [prb], bovine neutral insulin, porcine neutral insulin, human isophane insulin, isophase insulin [prb], human isophane insulin [pyr], porcine isophane insulin, bovine isophane insulin, human insulin zinc suspension [pyr[, humulin [prb], porcine insulin zinc suspension, human insulin zinc suspension [prb], human insulin glargine, protamine zinc insulin injection [prb], bovine insulin zinc suspension, bovine protamine zinc insulin, biphasic human insulin [pyr], biphasic human insulin [prb], biphasic neutral and isophane human insulin [prb], human insulin [emp], biphasic porcine neutral and isophane insulin, biphasic insulin aspart, insulin determir), dual acting insulin secretagogues/insulin sensitisers (pituitary adenylate cyclase-activating peptide/ vasoactive intestinal peptide receptor [VPAC] 2 receptor agonists), modulators of glucose uptake (GLUT 4 translocators, retinoid X receptor agonists, I_{K} *β* kinase [IKK] β inhibitors, p38 MAP kinase inhibitors, protein tyrosine phosphatase 1B [PTP1B] inhibitors, insulin receptor tyrosine kinase activators, α-glucosidase inhibitors, sodium/glucose co-transport inhibitors, glycogen synthase kinase 3 [GSK3] inhibitors) and modulators of mitochondrial metabolism. Medications may also comprise combinations of the above listed compounds, including, but not limited to rosiglitazone + metformin, rosiglitazone + simvastatin, rosiglitazone + sulphonurea, metformin + glipizide, sitagliptin + metformin, PPARγ/α combination medications (sipoglitazar, tesaglitazar, muraglitazar, reglitazar, peliglitazar, cevoglitazar, ragaglitazar, naveglitazar, imiglitazar, aloglitazar, sodelglitazar, peliglitazar, pemoglitazar, netoglitazone), PPARγ/α/δ combination medications;
   Antihypertensives including β-adrenoceptor blockers, eg propranolol, metoprolol and atenolol, and α₁-adrenoceptor blockers, eg doxasin and prazosin;
   Drugs for the treatment of gastro-oesophogeal reflux, dyspepsia and ulcers including esomeprazole, omeprazole and lansoprazole;
   Drugs for the treatment and prevention of epilepsy and seizure disorders including vigabatrin, piracetam, carbamazepine, pregabalin, gabapentin, lamotrigine, and sodium valproate;
   Drugs for the treatment or prevention of neuropathic pain including carbamazepine, pregabalin, gabapentin, sodium valproate, lamotrigine and the tricyclic antidepressants, eg amitriptyline, amoxapine, clomipramine, desipramine, doxepin, imiprimine, nortriptyline, protriptyline and trimiprimine, or atypical and "third generation" antidepressant drugs, eg mirtazepine, venlafaxine, mianserin and trazodone;
   Drugs for the treatment and prevention of migraine including methysergide, pizotifen, memantine, antiepileptic drugs, eg vigabatrin, piracetam, carbamazepine, pregabalin, gabapentin, lamotrigine, and sodium valproate, tricyclic antidepressants, eg amitriptyline, amoxapine, clomipramine, desipramine, doxepin, imiprimine, nortriptyline, protriptyline and trimiprimine, the serotonin-selective reuptake inhibitors including, but not limited to, fluoxetine, sertraline, paroxetine and citalopram, and atypical and "third generation" antidepressant drugs, eg mirtazepine, venlafaxine, mianserin and trazodone;
   Antihistamines including diphenhydramine and chlorpheniramine;
   Benzodiazepines including clobazam;
   Contraceptive medication, particularly injectable depot contraceptives including depot medroxyprogesterone acetate (DMPA); or
   Hormone replacement therapy including, but not limited to, synthetic or natural oestrogen and progesterone and their analogues.

The inventive active substance combination may comprise the components (A) and (B) in a molar ratio of component (A) to component (B) in the range of 1:10 to 10:1, preferably 1:5 to 5:1.

The inventive active substance combination is suitable for the administration to humans, including infants, children and grown-ups, as well as animals.

Preferably the total amount of the active substance(s) according to component (A), calculated as the free compound(s), to be administered to the patient in a 24 hours period does not exceed 800 mg, preferably does not exceed 500 mg.

The total amount of the active substance(s) according to component (B), calculated as the free compound(s), to be administered to the patient in a 24 hours period does preferably not exceed 160 mg, more preferably does not exceed 80 mg.

Preferably the inventive active substance combination comprises components (A) and (B) in the above defined molar ratios and within the afore given limits for the maximum dosis to be administered per day. Medicaments on the basis of the inventive active substance combination may preferably be administered once daily, twice daily or three times daily, more preferably once daily or twice daily, most preferably once daily.

In another aspect the present invention relates to a medicament comprising an inventive active substance combination and optionally at least one further active substance and/or optionally at least one auxiliary substance.

A further aspect of the invention provides the use of an active substance combination according to the inventuion for the production of a medicament for the treatment or prevention of weight-gain, obesity, dylipidaemia, Type 2 diabetes; Metabolic Syndrome, or cardio- and cerebrovascular accidents.

Preferably the inventive pharmaceutical formulation is suitable for oral or parenteral administration, more preferably for oral, intravenous, intraperitoneal, intramuscular, subcutaneous, intrathekal, rectal, transdermal, transmucosal or nasal administration.

inventive pharmaceutical formulation for oral administration are preferably selected from the group consisting of tablets, drageés, capsules, powders, drops, gels, juices, sirups, solutions and suspensions.

The pharmaceutical formulation of the present invention for oral administration may also be in the form of multiparticulates, preferably microparticles, microtablets, pellets or granules, optionally compressed into a tablet, filled into a capsule or suspended in a suitable liquid. Suitable liquids are known to those skilled in the art.

The respective pharmaceutical formulations may - depending on their route of administration - also contain one or more auxiliary substances known to those skilled in the art. The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the present disclosure.

In one embodiment of the present invention the pharmaceutical formulation comprises one or both of the components (A) and (B) at least partially in a sustained-release form. Preferably the inventive pharmaceutical formulation comprises component (B) at least partially in a sustained-release form.

By incorporating one or both of these components at least partially or completely in a sustained-release form it is possible to extend the duration of their effect, allowing for the beneficial effects of such a sustained release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.

The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly(C₁₋₄)alkyl (meth)acrylates, poly(C₁₋₄)dialkylamino(C₁₋₄)alkyl (meth)acrylates and/or copolymers or mixtures thereof, and very particularly preferably copolymers of ethyl acrylate and methyl methacrylate with a monomer molar ratio of 2:1 (Eudragit NE30D^{®}), copolymers of ethyl acrylate, methyl methacrylate and trimethylammonium ethyl methacrylate-chloride with a monomer molar ratio of 1:2:0.1 (Eudragit RS^{®}), copolymers of ethyl acrylate, methyl methacrylate and trimethylammonium ethyl methacrylate-chloride with a monomer molar ratio of 1:2:0.2 (Eudragit RL^{®}), or a mixture of at least two of the above-mentioned copolymers. These coating materials are commercially available as 30 wt.% aqueous latex dispersions, i.e. as Eudragit RS30D^{®}, Eudragit NE30D^{®} or Eudragit RL30D^{®}, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat^{®} or Surelease^{®}.

As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a C₆-C₄₀ aliphatic or aromatic dicarboxylic acid and a C₁-C₈ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet^{®} (acetylated mono- and diglycerides, C₂₃H₄₄O₅ to C₂₅H₄₇O₇), medium-chain triglycerides (Miglyol^{®}), oleic acid or mixtures of at least two of said plasticizers.
Aqueous dispersions of Eudragit RS^{®} and optionally Eudragit RL^{®} preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) covered by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L^{®}), methacrylic acid/methyl methacrylate copolymers with a monomer molar ratio of 1:2 (Eudragit S^{®}), methacrylic acid/ethyl acrylate copolymers with a monomer molar ratio of 1:1 (Eudragit L30D-55^{®}), methacrylic acid/methyl acrylate/methyl methacrylate copolymers with a monomer molar ratio of 7:3:1 (Eudragit FS^{®}), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D^{®} and/or Eudragit RL^{®} and/or Eudragit RS^{®}.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1, 299-311. The respective descriptions are incorporated by reference and are part of the present disclosure.

In another embodiment, the pharmaceutical formulation of the present invention contains one or both of components (A) and (B) not only in sustained-release form, but also in non-retarded form. By combination with the immediately released form, a high initial dose can be achieved for the rapid onset of the beneficial effect. The slow release from the sustained release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

### Pharmacological Methods

### I. In-vitro determination of affinity to CB1/CB2-Receptors

The in-vitro determination of the affinity of the substituted pyrazoline compounds to CB₁/CB₂-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human CB₁ and CB₂ receptors of Receptor Biology, Inc. are used. The radioligand used for both receptors is [³H]-CP55940. The respective parts of the description is hereby incorporated by reference and forms part of the present disclosure.

The present invention is illustrated below with the aid of examples. These illustrations are given solely by way of example and do not limit the general spirit of the present invention.

### EXAMPLES

### CHEMICAL EXAMPLES

### SUBGROUP A1

### Soubgroup A1: Chemical Example 1:

### N-piperidinyl-5-(4-chlorophenyl)-1-(2,4-dichlorophenyl)-4,5-dihydro-1 H-pyrazole-3-carboxamide

### a) 4-(4-chlorophenyl)-2-oxo-3-butenoic acid

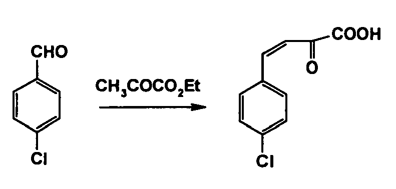

In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, cm⁻¹): 3500-2500, 1719,3, 1686,5, 1603,4, 1587,8, 1081,9.
¹H NMR(CDCl₃, δ) : 7,4 (d, J=8,4Hz, 2H), 7,5 (d, J=16,1Hz, 1H), 7,6 (d, J=8,4Hz, 2H), 8,1(d, J=16,1 Hz, 1H).

### b) 5-(4-chlorophenyl)-1-(2,4-dichlorophenyl)-4,5-dihydro-pyrazole-3-carboxylic acid

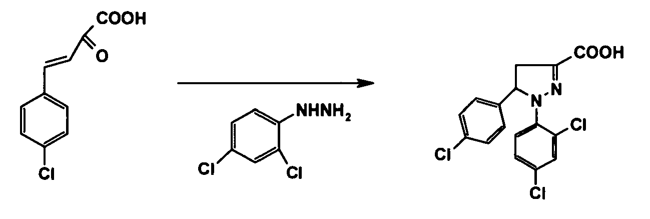

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, cm⁻¹) : 3200-2200, 1668,4, 1458, 1251,4, 1104,8.
¹H NMR (CDCl₃, δ) : 3,3 (dd, 1H), 3,7 (dd, 1 H), 5,9 (dd, 1 H), 7,09-7,25 (m, 7H).

### (c) 5-(4-chlorophenyl)-1-(2,4-dichlorophenyl)-4,5-dihydro-pyrazole-3-carboxylic acid chloride

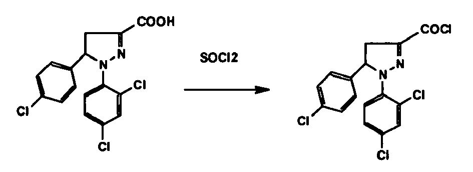

Under nitrogen atmosphere -5-(4-chlorophenyl)-1-(2,4-dichlorophenyl)-4,5-dihydro-pyrazole 3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, cm⁻¹) : 1732,3, 1700, 1533,3, 1478,1, 1212,9, 826,6.

### d) N-piperidinyl-5-(4-chlorophenyl)-1-(2,4-dichlorophenyl)-4,5-dihydropyrazole-3-carboxamide

[this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

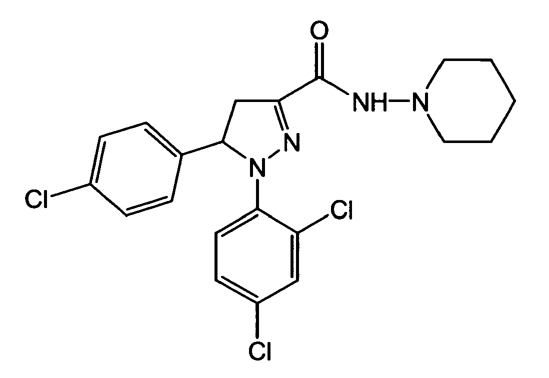

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.

IR (KBr, cm⁻¹): 3222,9, 2934,9, 1647,4, 1474,7, 1268,3, 815,6.
¹H NMR ( CDCl₃, δ): 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1H), 5,7 (dd, J=6,1 and 12,5 Hz, 1H), 7,0-7,2 (m, 6H), 7,4 (s, 1H).

The compounds according to the following examples 2-6 have been prepared analogously to the process described in Example 1.

### Subgroup A1: Chemical Example 2:

### 5-(4-Chloro-phenyl)-1-(2,4-dichloro-phenyl)-4,5-dihydro-1H-pyrazole-3-carboxylic acid-[1,2,4]triazol-4-yl amide

Melting point: 134-138 °C.
IR (KBr, cm⁻¹): 3448, 1686, 1477, 1243, 1091, 821.
¹H NMR(CDCl₃, δ): 3,1 (dd, J=6,2 and 17,9Hz, 1H), 3,7 (dd, J=12,3 and 17,9Hz, 1H), 5,9 (dd, J=6,2 and 12,3 Hz, 1H), 7,2-7,5 (m, 7H), 8,7 (s, 2H), 12,0 (bs, 1H).

### Subgroup A1: Chemical Example 3:

### 5-(4-Chloro-phenyl)-1-(2,4-dichloro-phenyl)-4,5-dihydro-1H-pyrazole-3-carboxylic acid-(4-methyl-piperazin-1-yl)-amide hydrochloride

Melting point: 150-155°C.
IR (KBr, cm⁻¹) : 3433, 1685, 1477, 1296, 1246, 1088, 1014, 825.
¹H NMR (CDCl₃, δ): 2,7 (d, J=4,2Hz, 3H), 3,0-3,4 (m, 9H), 3,6 (dd, J=11,9 and 17,9 Hz, 1H), 5,8 (dd, J=5,5 and 11,9 Hz, 1H), 7,1 (d, J=8,4Hz, 2H), 7,25 (2d, J= 8,4 and 8,7 Hz, 3H), 7,4 (d, J=2,2Hz, 1H), 7,5 (d, J=8,7Hz, 1H), 9,8 (s, 1H), 11,2 (bs).

### Subgroup A1: Chemical Example 4:

### 5-(4-Chloro-phenyl)-1-(2,4-dichloro-phenyl)-4,5-dihydro-1 H-pyrazole-3-carboxylic acid diethylamide

This compound was obtained in form of an oil.

IR (film, cm⁻¹): 2974, 1621, 1471, 1274, 1092, 820.
¹H NMR (CDCl₃, δ): 1,2 (m, 6H), 3,3-3,9 (m, 6H), 5,6 (dd, J=5,8 and 11,7 Hz, 1 H), 7-7,25 (m, 7H).

### Subgroup A1: Chemical Example 5:

### [5-(4-Chloro-phenyl)-1-(2,4-dichloro-phenyl)-4,5-dihydro-1H-pyrazol-3-yl]-piperidin-1-yl-methanone

Melting point: 105-110°C.
IR (KBr, cm⁻¹) : 2934, 1622, 1470, 1446, 1266, 1010, 817.
¹H NMR (CDCl₃, δ): 1,7 (m, 6H), 3,4 (dd, J=5,7 and 17,9Hz, 1H), 3,7 (m, 3H), 3,9 (m, 2H), 5,6 (dd, J=6,1 y 11,9 Hz, 1H), 7-7,25 (m, 7H).

### Subgroup A1: Chemical Example 6:

### N-[5-(4-Chloro-phenyl)-1-(2,4-dichloro-phenyl)-4,5-dihydro-1H-pyrazole-3-carbonyl]-4-methyl-phenylsulfonamide

This compound was obtained in form of an amorph solid.
IR (KBr, cm⁻¹) : 1697, 1481, 1436, 1340, 1169, 1074, 853.
¹H NMR (CDCl₃, δ): 2,4 (s, 3H), 3,2 (dd, J=6,6 and 18,3Hz, 1H), 3,6 (dd, J=12,8 and 18,3Hz, 1 H), 5,8 (dd, J=6,6 and 12,8Hz, 1 H), 7 (d, J=8,2Hz, 2H), 7,2 (s, 1H), 7,3-7,4 (m, 6H), 8 (d, J=8,1 Hz, 2H), 9 (s, 1 H).

### Subgroup A1: Chemical Example 7:

N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 °C and m-chloroperbenzoic acid (0,204 g, 0,83 mmoles) added in several portions. After stirring for 15 minutes a control via thin layer chromatography showed that no starting material was remaining. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120 °C.

IR (KBr, cm⁻¹): 3202, 1678, 1654, 1474, 1309, 1107.
¹H-NMR (CDCl₃, δ): 1.6 (m, 2H), 1.8-2.0 (m, 4H), 2.55 (m, 2H), 3.3 (dd, J = 6.3 Hz and 18.2 Hz, 1H), 3.7 (m, 3H), 5.8 (dd, J = 6.3 Hz and 12.5 Hz, 1H), 7.0-7.3 (m, 7H), 8.5 (s, 1H.)

### SUBGROUP A2

### Soubgroup A2: Chemical Example 1:

### (R)-N-piperidinyl-5-(4-chlorophenyl)-1-(2,4-dichlorophenyl)-4,5-dihydro-1H-pyrazole-3-carboxamide

### Resolution of 5-(4-chlorophenyl)-1-(2,4-dichlorophenyl)-4,5-dihydro-pyrazole-3-carboxylic acid with (+)-Cinchonine

35 g (294.68 mmol) of racemic 5-(4-chlorophenyl)-1-(2,4-dichlorophenyl)-4,5-dihydro-pyrazole-3-carboxylic acid in 630 m acetonitril were added to a suspension of 16.39 g (47.34 mmol) of (+)-Cinchonine in 920 ml acetonitrile under vigorous stirring. The resulting suspension was heated to reflux and acetonitrile (1300 ml) was added until dissolution was complete. The resulting solution was left to crystallise at room temperature over the weekend, whereupon a crystalline solid was obtained. The crystalline solid was filtered off, washed with 50 ml of cold acetonitrile and dried to give 25.26 g of a white solid (ee ≈ 91-92%). No second fraction of crystals could be obtained from the mother liquors, neither upon cooling in the refrigerator nor upon concentration. Thus, recrystallization of the diastereomeric salt was carried out in different solvents in order to improve the enantiomeric excess:

| Solvent | Yield | Ratio of enantiomers % (R) / % (S) |
|---|---|---|
| Acetonitrile | 90 % | 99,6 / 0,4 |
| EtOH-H₂O | 68,5 % | 99,3 / 0,7 |
| Isopropanol | 55 % | 98,9 / 1,1 |

Consequently, the product was recrystallized from acetonitrile to give 22.6 g of crystals (yield 72 mol-% related to chiral base). The ratio of enantiomers determined by capillary electrophoresis was:
99.7 % of (R)-5-(4-chlorophenyl)-1-(2,4-dichlorophenyl)-4,5-dihydro-pyrazole-3-carboxylic acid (+)-Cinchonine
0.3 % of (S)-5-(4-chlorophenyl)-1-(2,4-dichlorophenyl)-4,5-dihydro-pyrazole-3-carboxylic acid (+)-Cinchonine ee= 99.4 %.

### Preparation of (R)-5-(4-chlorophenyl)-1-(2,4-dichlorophenyl)-4,5-dihydro-pyrazole-3-carboxylic acid from addition compound (R)-5-(4-chlorophenyl)-1-(2,4-dichlorophenyl)-4,5-dihydro-pyrazole-3-carboxylic acid-(+)-Cinchonine

22.59 g (34 mmol) of the diastereomeric salt, which was recrystallized from acetonitrile, were suspended in 200 ml of 6N HCl and stirred at room temperature for 15 minutes. Afterwards, 200 ml of toluene were added until dissolution was complete. The mixture was stirred for 30 minutes and the phases were separated. The aqueous phase was extracted with toluene, the combined organic phases were washed with water, dried over sodium sulfate, filtered and evaporated to dryness, whereupon 11.9 g (95 %) of a white microcrystalline solid (melting point 129-133) were obtained.

Enantiomeric excess determined bY capillary electrophoresis. ee = 99.2 %
Chemical purity determined by HPLC: 99,3 %
[α]_{D} (c=1,23°C, MeOH) = -429.

### Isolation of the enantiomer (R)-5-(4-chlorophenyl)-1-(2,4-dichlorophenyl)-4,5-dihydro-pyrazole-3-carboxylic acid from the mother liquors obtained from the crystallisation of the enantiomer (S)-5-(4-chlorophenyl)-1-(2,4-dichlorophenyl)-4,5-dihydro-pyrazole-3-carboxylic acid with Brucine

The residue obtained from the evaporation of the mother liquors obtained from the crystallisation of (S)-5-(4-chlorophenyl)-1-(2,4-dichlorophenyl)-4,5-dihydro-pyrazole-3-carboxylic acid with Brucine (see below) was treated with 6N HCl as described above for obtaining the free acid. After evaporating the organic phase 21.86 g of a mixture with a theoretical composition of 20 mmoles (S)-5-(4-chlorophenyl)-1-(2,4-dichlorophenyl)-4,5-dihydro-pyrazole-3-carboxylic acid and 39.9 mmoles of (R)-5-(4-chlorophenyl)-1-(2,4-dichlorophenyl)-4,5-dihydro-pyrazole-3-carboxylic acid were obtained. A suspension of 13.82 g (39.9 mmoles) of (+)-cinchonine in 1000 ml of acetonitrile were added and the mixture was heated to reflux. More acetonitrile was added until dissolution was complete (total volume of acetonitrile 4000 ml). The mixture was then slowly cooled to room temperature to obtain crystals. After a few hours a white solid was obtained, which was filtered off and dried to give 22.42 (yield 84,5% related to the salt of (R)-5-(4-chlorophenyl)-1-(2,4-dichlorophenyl)-4,5-dihydro-pyrazole-3-carboxylic acid).

Enantiomeric excess as determined by capillary electrophoresis was ee = 92 %.

The purity of the isolated salt was comparable to the one of the salt obtained directly from the resolution of the racemate. Consequently, the same purification process as described above could be applied.

### Resolution of racemic 5-(4-chlorophenyl)-1-(2,4-dichlorophenyl)-4,5-dihydro-pyrazole-3-carboxylic acid with Brucine

### Preparation of the diastereomeric salt with Brucine

At room temperature 15.74 g (39.9 mmol) of Brucine were dissolved in 475 ml of acetone under vigorous stirring. A solution of 29.5 g (79.8 mmol) of the racemic 5-(4-chlorophenyl)-1-(2,4-dichlorophenyl)-4,5-dihydro-pyrazole-3-carboxylic acid in 85 ml acetone was added. The resulting mixture was allowed to crystallise for a couple of days, whereupon a crystalline solid was obtained. The crystalline solid was filtered off, washed with cold acetone and dried to yield 13.66 g (45 mol % related to the base) of a beige-coloured solid were obtained.

The ratio of the enantiomers as determined by capilar electrophoresis was:
99 % of (S)-5-(4-chlorophenyl)-1-(2,4-dichlorophenyl)-4,5-dihydro-pyrazole-3-carboxylic acid Brucine
1 % of (R)-5-(4-chlorophenyl)-1-(2,4-dichlorophenyl)-4,5-dihydro-pyrazole-3-carboxylic acid Brucine
**Enantiomeric excess ee = 98 %**

The filtered mother liquors were left in the refrigerator and yielded a second fraction of crystals, which were filtered off and dried to give another 1.56 g (5.1 molar % related to the base).

The analysis of the 2^{nd} fraction via capillar electrophoresis gave
99.3 % of (S)-5-(4-chiorophenyl)-1-(2,4-dichlorophenyl)-4,5-dihydro-pyrazole-3-carboxylic acid Brucine
0.7 % of (R)-5-(4-chlorophenyl)-1-(2,4-dichlorophenyl)-4,5-dihydro-pyrazole-3-carboxylic acid Brucine
Enantiomeric excess ee = 98.6 %
Total yield:
15.22 g (50.1 mol% related to base) of the salt with ee = 98 %.

### Determination of the chirality of the Brucine salt via X-ray crystallography

The chirality of the enantiomer that crystallized with Brucine (ee 98 %) was determined via x-ray crystallography and corresponds to the S-enantiomer. Accordingly, the other enantiomer that crystallized with (+)-cinchonine is the corresponding R-enantiomer.

### Preparation of (S)-5-(4-chlorophenyl)-1-(2,4-dichlorophenyl)-4,5-dihydro-pyrazole-3-carboxylic acid from addition compound (S)-5-(4-chlorophenyl)-1-(2,4-dichlorophenyl)-4,5-dihydro-pyrazole-3-carboxylic acid brucine

15.17 g (19.85 mmol) of (S)-5-(4-chlorophenyl)-1-(2,4-dichlorophenyl)-4,5-dihydro-pyrazole-3-carboxylic acid Brucine were suspended in 150 ml of 6N HCl and the suspension was stirred for 10 minutes, followed by the addition of 150 ml of toluene upon which complete dissolution was observed. The resulting mixture was stirred for an additional 30 minutes under control with column chromatography (silical gel, Cl₂CH₂:MeOH 95:5). Afterwards, the phases were separated, the aqueous phase was extracted with toluene and the combined toluene phases were washed with water three times, dried over sodium sulfate, filtered and evaporated to dryness. 7 g (95,5 %) of an oil were obtained, which solidified upon addition of a small amount of diethylether to yield a white amorphous solid that showed a crystalline transformation under melting at 130-133 °C.

CCF(Cl₂CH₂:MeOH 95:5) : R_{f} = 0.3
The analysis of the enantiomers via capillar electrophoresis gave

99.1 % (S)-5-(4-chlorophenyl)-1-(2,4-dichlorophenyl)-4,5-dihydro-pyrazole-3-carboxylic acid 0.9 % (R)-5-(4-chlorophenyl)-1-(2,4-dichlorophenyl)-4,5-dihydro-pyrazole-3-carboxylic acid Enantiomeric excess ee = 98.2 %
[α]_{D} (c=1,23°C, MeOH) = -480.5.
Chemical purity determined by HPLC: 99.1 %

¹H-NMR (CDCl₃) δ ppm: 3,2 (dd, J=6,4 y 18,2 Hz, 1 H), 3,7 (dd, J=12,7 y 18,2 Hz, 1 H), 5,85 (dd, J=6,4 y 12,6 Hz, 1H), 7,0-7,2 (m, 7H).

### Isolation of the enantiomer (S)-5-(4-chlorophenyl)-1-(2,4-dichlorophenyl)-4,5-dihydro-pyrazole-3-carboxylic acid from the mother liquors obtained from the crystallisation of the other enantiomer (R)-5-(4-chlorophenyl)-1-(2,4-dichlorophenyl)-4,5-dihydro-pyrazole-3-carboxylic acid with (+)-cinchonine

The residue obtained from the evaporation of the mother liquors obtained from the crystallisation of (R)-5-(4-chlorophenyl)-1-(2,4-dichlorophenyl)-4,5-dihydro-pyrazole-3-carboxylic acid with (+)-Cinchonine was treated with 6N HCl as described above for obtaining the free acid. After evaporating the organic phase 23.63 g of a mixture with a theoretical composition of 13.34 mmoles of (R)-5-(4-chlorophenyl)-1-(2,4-dichlorophenyl)-4,5-dihydro-pyrazole-3-carboxylic acid and 47.34 mmoles of (S)-5-(4-chlorophenyl)-1-(2,4-dichlorophenyl)-4,5-dihydro-pyrazole-3-carboxylic acid were obtained. Said mixture, dissolved in 118 ml of acetone, was added on top of a solution of 18.7 g (47.4 mmoles) of brucine in 650 ml of acetone, stirred for a couple of minutes and left to crystallise at room temperature overnight. A creme-coloured solid was obtained that was filtered off and dried to yield 28 g (77.5 % relative to the (S)-salt).

### Enantiomeric excess as determined by capilar electrophoresis was ee = 98.2 %

The purity of the isolated salt was comparable to the one of the salt obtained directly from the resolution of the racemate. Consequently, the same purification process as described above could be applied.

### Preparation of (R)-N-piperidinyl-5-(4-chlorophenyl)-1-(2,4-dichlorophenyl)-4,5-dihydro-1 H-pyrazole-3-carboxamide

9.94 g (26.89 mmol) of (R)-5-(4-chlorophenyl)-1-(2,4-dichlorophenyl)-4,5-dihydro-pyrazole-3-carboxylic acid were dissolved in 95 ml of toluene, followed by the addition of 2.35 ml (32.3 mmoles) of thionyl chloride and 4 drops of DMF. Under anhydrous nitrogen atmosphere the mixture was heated to a temperature of 75-80° C under vigorous stirring for two hours and then allowed to cool to room temperature. Said acid chloride solution was then added dropwise to a solution of 3.47 ml (31.2 mmol) of N-Aminopiperidine, 15 ml (107.6 mmol) of triethylamine and 38 ml of anhydrous toluene, which was cooled to 0-5 °C, thereby keeping the temperature below 10 °C during the addition. The reaction mixture was stirred at room temperature overnight, whereupon a suspension was obtained. Water and Toluene were added to said suspension until dissolution was complete and the phases were separated.

The aqueous phase was extracted with toluene, the combined organic phases washed with an NaOH solution (10 %) and water, dried over sodium sulfate, filtered off and evaporated to dryness to yield 13.41 g of light yellow oil, which failed to crystallise. The oil was purified via column chromatography over silica gel (eluent: petrol ether ethylacetate 90: 10 to 60: 40) to give 10.5 g (83.5 %) of -(R)-N-piperidinyl-5-(4-chlorophenyl)-1-(2,4-dichlorophenyl)-4,5 dihydro-1 H-pyrazole-3-carboxamide in form of an amorphous solid of light yellow colour (melting point 75-78 °C).

¹H-NMR (DMSO-d₆) δ ppm : 9,25 (s, 1H), 7,5 (d, J=8,8Hz, 1H), 7,4 (d, J=2,3Hz, 1H), 7,3-7,25 (2d, J=8,8 and 8,5Hz, 3H), 7,1 (d, J=8,5Hz, 2H), 5,8 (dd, J=5,7 and 11,8 Hz, 1H), 3,65 (dd, J=11,8 and 18,1Hz, 1H), 3,0 (dd, J=5,7 and 18,1Hz, 1H), 2,75 (m, 4H), 1,5 (m, 4H), 1,2 (m, 2H).

Analysis of the enantiomer via chiral HPLC: ee = 100 %
Chemical purity determined by HPLC: 98,5%
[α]_{D} (c=1, 23°C, MeOH) = - 293,5

### Preparation of Hydrochloride salt of (R)-N-piperidinyl-5-(4-chlorophenyl)-1-(2,4-dichlorophenyl-4,5-dihydro-1H-pyrazole-3-carboxamide

0.5 g (1.1 mmol) of (R)-N-piperidinyl-5-(4-chlorophenyl)-1-(2,4-dichlorophenyl)-4,5-dihydro-1 H-pyrazole-3-carboxamide were dissolved in 10 ml isopropanol, cooled with ice andistsaturated HCl-ethanol solution was added. The solution changed its colour and a white solid formed, which was filtered off, washed with isopropanol:diethylether (1:1) and dried to yield 0.38 g (71 %) of a white solid with a melting point of 160-165 °C. The purity determined by HPLC was 99.3 %. Determination of chlorine: 7.15 % (98.5 % of the theoretical value).

¹H-NMR (DMSO-d₆) δ ppm : 10,3 (bs, 1 H), 7,5 (m, 2H), 7,3 (2d, J= 2,5 y 8,5Hz, 3H), 7,15 (d, J=8,5Hz, 2H), 5,8 (dd, J=6,3 y 12,0Hz, 1 H), 3,7 (dd+H₂O), 3,05 (m, 5H), 1,7 (m, 4H), 1,4 (m, 2H).

### Subgroup A2: Chemical Example 2:

### Preparation of (S)-N-piperidinyl-5-(4-chlorophenyl)-1-(2,4-dichlorophenyl)-4,5-dihydro-1 H-pyrazole-3-carboxamide

5.27 g (14.26 mmol) of (S)-5-(4-chlorophenyl)-1-(2,4-dichlorophenyl)-4,5-dihydro-pyrazole-3-carboxylic acid were dissolved in 30 ml of toluene, followed by the rapid dropwise addition of 1.25 ml (17.11 mmoles) of thionyl chloride and 7 drops of DMF.Under anhydrous nitrogen atmosphere the mixture was heated to a temperature of 75-80° C under vigorous stirring for two hours and then allowed to cool to room temperature. Said acid chloride solution was then added dropwise to a solution of 1.84 ml (16.54 mmol) of N-Aminopiperidine, 8 ml (57 mmol) of triethylamine and 25 ml of anhydrous toluene, which was cooled to 0-5 °C, thereby keeping the temperature below 10 °C during the addition. The reaction mixture was stirred at room temperature overnight, whereupon a suspension was obtained. Water and Toluene were added to said suspension until dissolution was complete and the phases were separated. The aqueous phase was extracted with toluene, the combined organic phases washed with an NaOH solution (10 %) and water, dried over sodium sulfate, filtered off and evaporated to dryness to yield 6.85 g of an oil, which failed to crystallize using the solvents Ethanol, Ethylacetate and Acetone. The oil was purified via column chromatography over silica gel (eluent: petrol ether ethylacetate 90: 10 to 50: 50) to give 4.7 g (73 %) of (S)-N-piperidinyl-5-(4-chlorophenyl)-1-(2,4-dichlorophenyl)-4,5-dihydro-1H-pyrazole-3-carboxamide in form of an amorphous solid of light yellow color (melting point 73-76 °C).

¹H-NMR (DMSO-d₆) δ ppm : 9,25 (s, 1H), 7,5 (d, J=8,8Hz, 1H), 7,4 (d, J=2,3Hz, 1H), 7,3-7,25 (2d, J=8,8 and 8,5Hz, 3H), 7,1 (d, J=8,5Hz, 2H), 5,8 (dd, J=5,7 and 11,8 Hz, 1H), 3,65 (dd, J=11,8 and 18,1 Hz, 1 H), 3,0 (dd, J=5,7 and 18,1 Hz, 1H), 2,75 (m, 4H), 1,5 (m, 4H), 1,2 (m, 2H).

Analysis of the enantiomer via chiral HPLC: ee = 98 %
Chemical purity determined by HPLC: 98,7%

### Preparation of Hydrochloride salt of (S)-N-piperidinyl-5-(4-chlorophenyl)-1-(2,4-dichlorophenyl)-4,5-dihydro-1H-pyrazole-3-carboxamide

0.2 g (0.4 mmol) of (S)-N-piperidinyl-5-(4-chlorophenyl)-1-(2,4-dichlorophenyl)-4,5-dihydro-1 H-pyrazole-3-carboxamide were dissolved in 3 ml isopropanol, cooled with ice and a saturated HCl-ethanol solution was added. The solution changed its color and a white solid formed, which was filtered off, washed with isopropanol:diethylether (1:1) and dried to yield 0.14 g (72 %) of a white solid with a melting point of 165-175 °C. The purity determined by HPLC was 99.3 %. Determination of chlorine: 7.15 % (98.5 % of the theoretical value).

¹H-NMR (DMSO-d₆) δ ppm : 10,4 (bs, 1H), 7,45 (m, 2H), 7,3 (2d, J= 2,6 y 8,5Hz, 3H), 7,15 (d, J=8,5Hz, 2H), 5,8 (dd, J=6,3 y 12,0Hz, 1 H), 3,7 (dd, J=12,0 y 18,0Hz, 1 H), 3,05 (m, 5H), 1,7 (m, 4H), 1,4 (m, 2H).

### SUBGROUP A3:

### Subgroup A3: Chemical Example

### Preparation of (E)-4-(5-Chloro-thiophen-2-yl)-2-oxo-but-3-enoic acid

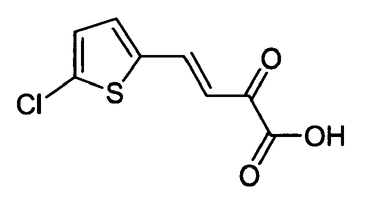

A two neck 50 mL round bottom flask equipped with a thermometer and an addition funnel was charged with 5-chlorothiophene (1.14 g, 7.6 mmol, 1.05 equiv.) and ethyl pyruvate (0.8 mL, 7.22 mmol, 1 equiv.) under nitrogen atmosphere in absolute ethanol (17 mL). The mixture was cooled to 0°C and an aqueous 2 M solution of NaOH (0.95g, 3.3 equiv. of NaOH in 10.5 mL water) was added drop wise at a rate to maintain the temperature below 10°C. The mixture was left stirring at 0°C for 1 h, the ice bath was removed and the mixture stirred at room temperature for 1 h and 30 min. The solution was cooled down in an ice bath for 30 min and a brown precipitate appeared. The solid was filtered and the mother liquors were kept at 4 °C overnight. A second crop was collected and the combined crops were washed with diethylether to eliminate traces of unreacted starting material. The remaining solid was suspended in HCl (40 mL of a 2 M aqueous solution) and stirred for 30 min, after filtration the desired product (*E*)-4-(5-chlorothiophen-2-yl)-2-oxo-3-butenoic acid was obtained as a yellow solid (0.74 g, 47%).

¹H NMR (400 MHz, CDCl₃) δ 3.5 (1 H, bs, OH), 6.98 (1 H, d, J 4.0 Hz, thiophene H), 7.19 (1H, d, J 16 Hz, ArH,), 7.29 (1 H, d, J 4.0 Hz, thiophene H), 8.09 (2H, d, J 16.0 Hz); ¹³C NMR (100 MHz, CDCl₃): δ 117.6 (CH), 128.5 (CH), 134.4 (CH), 137.5 (C), 138.6 (C), 142.1 (CH), 161.9 (CO), 182.3 (CO).

### Subgroup A3: Chemical Example 8:

### Preparation of 5-(5-Chloro-thiophen-2-yl)-1-(2,4-dichloro-phenyl)-4,5-dihydro-1H-pyrazole-3-carboxylic acid

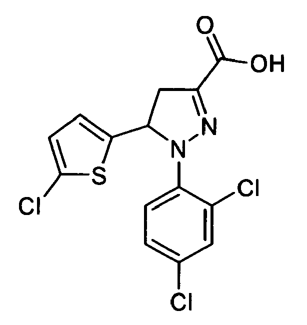

A mixture of (*E*)-4-(5-chlorothiophen-2-yl) -2-oxo-3-butenoic acid (0.72 g, 3.3 mmol) and 2,4-dichlorophenylhydrazine hydrochloride (0.71 g, 3.3 mmol) in 10.5 mL glacial acetic acid under nitrogen atmosphere was heated at reflux for 4 h. The reaction mixture was allowed to cool to room temperature and poured into ice. The dark mixture was extracted with dichloromethane, washed with H₂O, dried over Na₂SO₄ and evaporated to dryness to yield 1.35 g (≥100% of the crude acid), which was purified by semi-preparative HPLC.

¹H NMR (400 MHz, CHLOROFORM-*d*) δ ppm 3.39 (dd, *J*=17.88, 4.59 Hz, 1 H) 3.64 (dd, *J*=17.88, 11.92 Hz, 1 H) 6.11 (dd, *J*=11.92, 4.59 Hz, 1 H) 6.60 (d, *J*=3.78 Hz, 1 H) 6.61 (d, *J*=3.78 Hz, 1 H) 7.12 (dd, *J*=8.60, 2.15 Hz, 1 H) 7.25 (d, *J*=8.60 Hz, 1 H) 7.32 (d, J= 2.15 Hz, 1 H)
MS (M+H)⁺: 375

### Subgroup A3: Chemical Example 1:

### Preparation of 5-(5-Chloro-thiophen-2-yl)-1-(2,4-dichloro-phenyl)-4,5-dihydro-1 H-pyrazole-3-carboxylic acid piperidin-1-ylamide

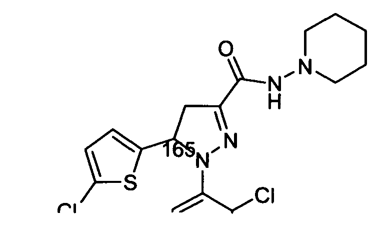

Crude 5-(5-chlorothiophen-2-yl)-1-(2,4-dichlorophenyl)-4,5-dihydropyrazol-3-carboxylic acid (0.25 g, 0.66 mmol) was dissolved several times in 5 mL of anhydrous toluene and the solvate was removed in vacuo. The acid was dissolved in 3 mL of anhydrous toluene under nitrogen atmosphere, and thionylchloride (0.8 mL, 4.86 mmol, 16 equiv) and anhydrous dimethylformamide (55 µL) were added. The reaction mixture was heated at reflux and monitored by IR (NaCl, film). After 2 h reaction mixture was left to cool to room temperature and the solvents were evaporated to dryness under high vacuum. The desired product 5-(5-chlorothiophen-2-yl)-1-(2,4-dichlorophenyl)-4,5-dihydropyrazol-3-carbonyl chloride (0.19 g, 74%) was obtained as a dark oil and used for the next step without further purification.
IR (NaCl, film) u: 802, 1106, 1213, 1477, 1735 cm⁻¹

Triethylamine (0.4 mL, 6 equiv.) was added drop wise to a solution of N-aminopiperidine (71 *µ*L, 0.66 mmol, 1.3 equiv.) in anhydrous dichloromethane (3.5mL). The mixture was cooled in an ice bath and a solution of 5-(5-chlorothiophen-2-yl)-1-(2,4-dichlorophenyl)-4,5-dihydropyrazol-3-carbonyl chloride (0.19 g, 0.48 mmol, 1 equiv.) in anhydrous DCM (3.5 mL) was added. After 15 min the ice bath was removed and the reaction was left stirring at room temperature overnight. The solution was poured in a separating funnel and washed once with water, with a saturated aqueous solution of NaHCO₃ and again with water, dried over Na₂SO₄ and evaporated to dryness to give 0.22 g of the crude product.
Purification by column chromatography (SiO₂ with 2.5% v/v of Et₃N, 30:1 ratio SiO₂: product, packed with 5% ethylacetate/hexane and eluted with a gradient of 5% ethylacetate/hexane to 20% ethylacetate/hexane) provided racemic *N*-(piperidin-1-yl)-5-(5-chlorothiophen-2-yl)-1-(2,4-dichlorophenyl)-4,5-dihydropyrazole-3-carboxamide (35 mg, 16% yield, 81% pure by HPLC) as a dark oil.

¹H NMR (400 MHz, CHLOROFORM-*d*) δ ppm 1.44 (m, 2 H) 1.76 (dt, *J*=11.23, 5.71 Hz, 4 H) 2.84 (m, 4 H) 3.45 (dd, *J*=18.17, 4.69 Hz, 1 H) 3.61 (dd, *J*=18.17, 11.14 Hz, 1 H) 5.93 (dd, *J*=11.14, 4.69 Hz, 1 H) 6.57 (m, 2 H) 7.06 - 7.19 (m, 2 H) 7.32 (d, J=2.15 Hz, 1 H) 7.40 (m, 1 H)
¹³C NMR (100 MHz, CDCl₃): δ 23.4 (CH₂), 25.3 (CH₂), 40.5 (CH₂), 57.3 (CH₂), 63.7 (CH), 125.7 (CH), 125.8 (CH), 126.1 (CH), 127.1 (C), 127.8 (CH), 130.1 (CH), 131.1 (C), 139.6 (C), 139.9 (C), 146.1 (C), 158.9 (CO).
IR (NaCl, film) u 1476, 1663, 2833, 2938 cm⁻¹
MS (M+H)⁺: 457

### Subgroup A3: Chemical Example

### Preparation of (E)-4-(4-Bromothiophen-2-yl)-2-oxo-3-butenoic acid

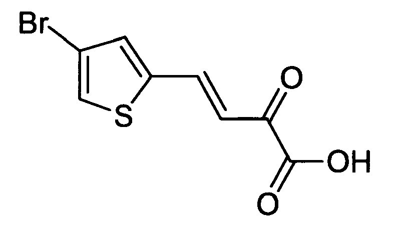

A two neck 100 mL round bottom flask equipped with a thermometer and an addition funnel was charged with 4-bromothiophene (5 g, 26.17 mmol, 1.05 equiv.) and ethyl pyruvate (2.76 mL, 24.9 mmol, 1 equiv.) under nitrogen atmosphere in absolute ethanol (61 mL). The reaction mixture was cooled to 0°C and a 2 M solution of NaOH (3.3 g, 3.2 equiv. of NaOH in 37 mL of water) was added drop wise at a rate to maintain the temperature below 10°C. The mixture was left stirring at 0°C for 1 h, the ice bath was removed and the mixture stirred at room temperature for 1 h and 30 min. The solution was cooled in an ice bath for 1 h and a yellow precipitate appeared. The solid was filtered and the mother liquors were kept at 4 °C overnight. A second crop was collected and the combined crops were washed with diethylether to eliminate traces of unreacted starting material. The solid was suspended in HCl (138 mL of a 2 M aqueous solution) and stirred for 20 min. After filtration the desired product (*E*)-4-(4-bromothiophen-2-yl) -2-oxo-3-butenoic acid was obtained as a yellow solid (3.07 g, 45%).

¹H NMR (400 MHz, CDCl₃): 7.33 (2H, d, J 14.8 Hz), 7.41 (1 H, s, thiophene H)), 7.47 (1 H, s, thiophene H), 8.13 (2H, d, J 14.8 Hz).

### Subgroup A3: Chemical Example 27:

### Preparation of 5-(5-Bromo-thiophen-2-yl)-1-(2,4-dichloro-phenyl)-4,5-dihydro-1 H-pyrazole-3-carboxylic acid

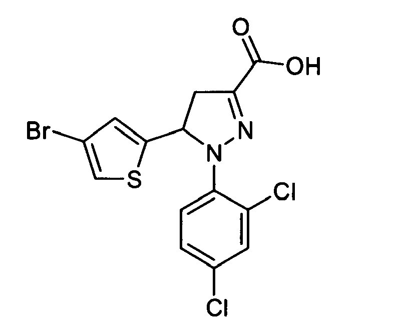

A mixture of (*E*)-4-(4-bromothiophen-2-yl)-2-oxo-3-butenoic acid (3.07 g, 11.5 mmol, 1 equiv.) and 2,4-dichlorophenylhydrazine hydrochloride (2.46 g, 11.5 mmol, 1 equiv.) in 37 mL of glacial acetic acid under nitrogen atmosphere was heated at reflux for 3 h. The reaction mixture was allowed to cool to room temperature and poured into ice. The dark mixture was extracted with dichloromethane, washed with H₂O, dried over Na₂SO₄ and evaporated to dryness to yield 5.5 g (≥100% of the crude acid).
The product was purified by preparative HPLC using samples of 1 g each of the crude product dissolved in 9 mL of MeOH which were eluted (90% heptane, 10% EtOH, 0.2% TFA, 55mL/min, 254 nm, in a Lichrospher Si60 12 *µ*l) to provide 0.41 g of a 99% pure compound by analytical HPLC (41 % yield).

¹H NMR (400 MHz, CHLOROFORM-*d*) δ ppm 3.42 (dd, *J*=18.07, 4.98 Hz, 1 H) 3.69 (dd, *J*=18.07, 12.02 Hz, 1 H) 6.14 (dd, *J*=12.02, 4.98 Hz, 1 H) 6.77 (d, *J*=1.37 Hz, 1 H) 7.04 (d, *J*=1.37 Hz, 1 H) 7.13 (dd, *J*=8.60, 2.15 Hz, 1 H) 7.20 (d, *J*=8.60 Hz, 1 H) 7.35 (d, *J*=2.15 Hz, 1 H)
¹³C NMR (100 MHz, CDCl₃): δ 40.4 (CH₂), 63.5 (CH), 110.2 (CH), 109.95 (C), 123.3 (CH), 126.8 (CH), 127.3 (C), 128.0 (CH), 129.1 (CH), 130.2 (CH), 132.0 (C), 138.3(C), 140.9 (C), 142.5 (C), 165.8 (CO).
MS (M+H)⁺: 419

### Subgroup A3: Chemical Example 23:

### Preparation of 5-(-4-Bromo-thiophen-2-yl)-1-(2,4-dichloro-phenyl)-4,5-dihydro 1H-pyrazole-3-carboxylic acid piperidin-1-ylamide hydrochloride

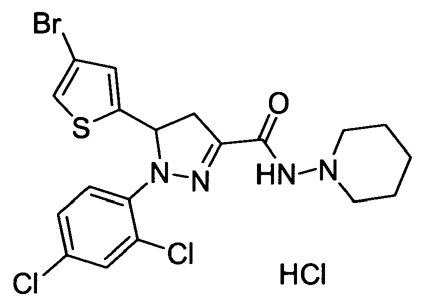

5-(5-Bromo-thiophen-2-yl)-1-(2,4-dichloro-phenyl)-4,5-dihydro-1H-pyrazole-3-carboxylic acid (0.290 g, 0.69 mmol) was dissolved in toluene (8 mL) and partially distilled at atmospheric pressure to eliminate water traces. The resulting solution was cooled to 75°C - 85 °C. Dimethylformamide was added (2 drops) and thionylchloride (0.060 mL, 0.83 mmol) was added drop by drop. The solution was stirred until the complete formation of the acid chloride (monitoring by IR). The mixture was cooled to 20 °C - 25 °C which was added drop wise under nitrogen atmosphere to a solution of 1-aminopiperidine (0.090 mL, 0.82 mmol), dichloromethane (5 mL) and N,N-diisopropylethylamine (0.281 mL, 1.64 mmol) while keeping the temperature of the mixture between 5-10 °C. The mixture was warmed to 20°C - 25 °C and stirred overnight. The organic layer was washed with water (3 x 5 mL), with a saturated aqueous solution of NaHCO₃ (3 x 25 mL) and again with water (3 x 5 mL). The final organic extract was dried with Na₂SO₄, filtered and concentrated in vacuo to afford a beige solid (263 mg, 77 % yield).

The solid was dissolved in 3 mL of ethyl acetate and 2.8 N HCl in ethanol was added (3 mL, 1.2 eq). The mixture was stirred at room temperature for 30 min and a beige solid appeared. The solid was filtered and washed several times with ethyl acetate to obtain a white solid (170 mg, 47 % yield).
¹H NMR (300 MHz, DMSO-*d*₆) δ ppm 1.42 (m, 2 H) 1.72 (m, 4 H) 3.15 (m, 4 H) 3.25 (dd, *J*=18.02, 5.13 Hz, 1 H) 3.68 (dd, *J*=18.02, 11.50 Hz, 1 H) 6.08 (dd, *J*=11.50, 5.13 Hz, 1 H) 6.95 (d, *J*=1.17 Hz, 1 H) 7.34 (m, 1 H) 7.46 (m, 2 H) 7.58 (d, *J*=2.20 Hz, 1 H) 10.68 (s, 1 H)
MS (M+H)⁺: 501

The following compounds are prepared according to the processes described above. Those skilled in the art are familiar with the starting materials that are needed to obtain said compounds.

### Subgroup A3-Chemical Examples

| **N°** | **STRUCTURE** | **Name** | **¹H-NMR** | **MS (M+H)⁺** |
|---|---|---|---|---|
| 1 | 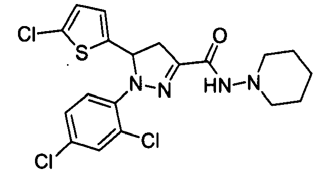 | 5-(5-Chloro-thiophen-2-yl)-1-(2,4-dichloro-phenyl)-4,5-dihydro-1H-pyrazole-3-carboxylic acid piperidin-1-ylamide | 1 H NMR (400 MHz, CHLOROFORM-*d*) δ ppm 1.44 (m, 2 H) 1.76 (dt, *J*=11.23, 5.71 Hz, 4 H) 2.84 (m, 4 H) 3.45 (dd, *J*=18.17, 4.69 Hz, 1 H) 3.61 (dd, *J*=18.17, 11.14 Hz, 1 H) 5.93 (dd, *J*=11.14, 4.69 Hz, 1 H) 6.57 (m, 2 H) 7.06 - 7.19 (m, 2 H) 7.32 (d, *J*=2.15 Hz, 1 H) 7.40 (m, 1 H) | 457 |
| 2 | 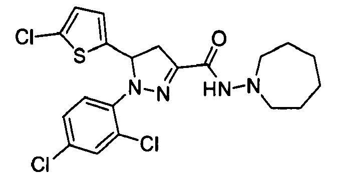 | 5-(5-Chloro-thiophen-2-yl)-1-(2,4-dichloro-phenyl)-4,5-dihydro-1H-pyrazole-3-carboxylic acid azepan-1-ylamide | | |
| 3 | 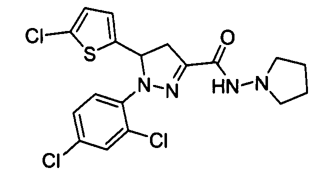 | 5-(5-Chloro-thiophen-2-yl)-1-(2,4-dichloro-phenyl)-4,5-dihydro-1H-pyrazole-3-carboxylic acid pyrrolidin-1-ylamide | | |
| 4 | 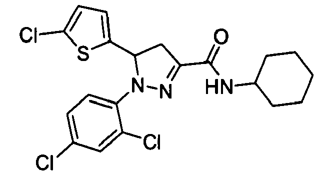 | 5-(5-Chloro-thiophen-2-yl)-1-(2,4-dichloro-phenyl)-4,5-dihydro-1H-pyrazole-3-carboxylic acid cyclohexyl-amide | | |
| 5 | 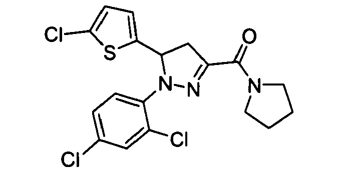 | [5-(5-Chloro-thiophen-2-yl)-1-(2,4-dichloro-phenyl)-4,5-dihydro-1H-pyrazol-3-yl]-pyrrolidin-1-yl-methanone | | |
| 6 | 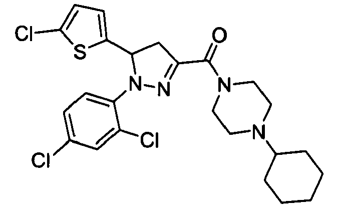 | [5-(5-Chloro-thiophen-2-yl)-1-(2,4-dichloro-phenyl)-4,5-dihydro-1H-pyrazol-3-yl]-(4-cyclohexyl-piperazin-1-yl)-methanone | | |
| 7 | 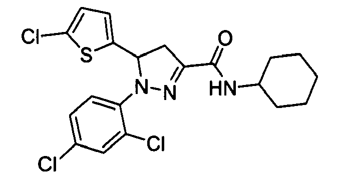 | 5-(5-Chloro-thiophen-2-yl)-1-(2,4-dichloro-phenyl)-4,5-dihydro-1H-pyrazole-3-carboxylic acid cyclohexylamide | | |
| 8 | 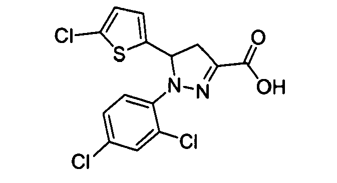 | 5-(5-Chloro-thiophen-2-yl)-1-(2,4-dichloro-phenyl)-4,5-dihydro-1H-pyrazole-3-carboxylic acid | 1 H NMR (400 MHz, CHLOROFORM-*d*) δ ppm 3.39 (dd, *J*=17.88, 4.59 Hz, 1 H) 3.64 (dd, *J*=17.88, 11.92 Hz, 1 H) 6.11 (dd, *J*=11.92, 4.59 Hz, 1 H) 6.60 (d, *J*=3.78 Hz, 1 H) 6.61 (d, *J*=3.78 Hz, 1 H) 7.12 (dd, *J*=8.60, 2.15 Hz, 1 H) 7.25 (d, *J*=8.60 Hz, 1 H) 7.32 (d, *J*= 2.15 Hz, 1H) | 375 |
| 9 | 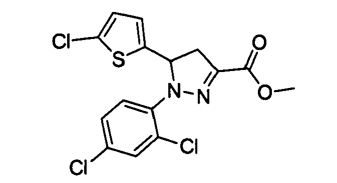 | 5-(5-Chloro-thiophen-2-yl)-1-(2,4-dichloro-phenyl)-4,5-dihydro-1H-pyrazole-3-carboxylic acid methyl ester | | |
| 10 | 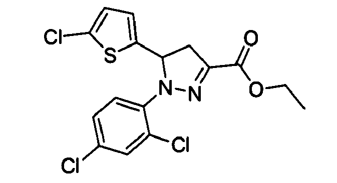 | 5-(5-Chloro-thiophen-2-yl)-1-(2,4-dichloro-phenyl)-4,5-dihydro-1H-pyrazole-3-carboxylic acid ethyl ester | | |
| 11 | 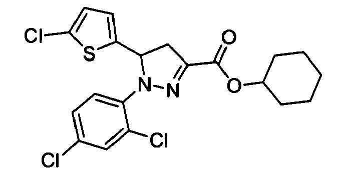 | 5-(5-Chloro-thiophen-2-yl)-1-(2,4-dichloro-phenyl)-4,5-dihydro-1H-pyrazole-3-carboxylic acid cyclohexyl ester | | |
| 12 | 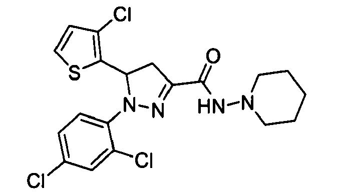 | 5-(3-Chloro-thiophen-2-yl)-1-(2,4-dichloro-phenyl)-4,5-dihydro-1H-pyrazole-3-carboxylic acid piperidin-1-ylamide | | |
| 13 | 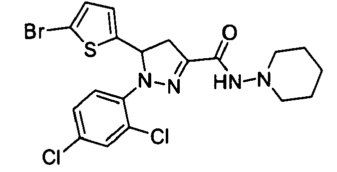 | 5-(5-Bromo-thiophen-2-yl)-1-(2,4-dichloro-phenyl)-4,5-dihydro-1H-pyrazole-3-carboxylic acid piperidin-1-ylamide | | |
| 14 | 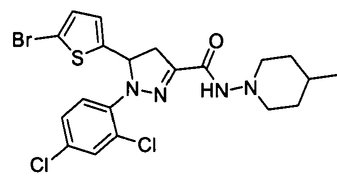 | 5-(5-Bromo-thiophen-2-yl)-1-(2,4-dichloro-phenyl)-4,5-dihydro-1H-pyrazole-3-carboxylic acid (4-methyl-piperidin-1-yl)-amide | | |
| 15 | 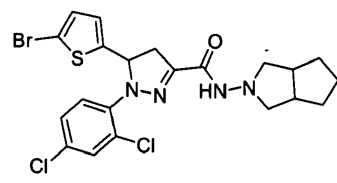 | 5-(5-Bromo-thiophen-2-yl)-1-(2,4-dichloro-phenyl)-4,5-dihydro-1H-pyrazole-3-carboxylic acid (hexahydro-cyclopenta[c]pyrrol-2-yl)-amide | | |
| 16 | 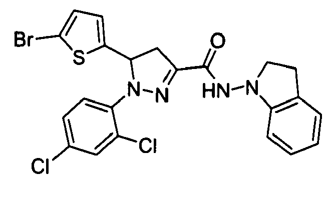 | 5-(5-Bromo-thiophen-2-yl)-1-(2,4-dichloro-phenyl)-4,5-dihydro-1H-pyrazole-3-carboxylic acid (2,3-dihydroindol-1-yl)-amide | | |
| 17 | 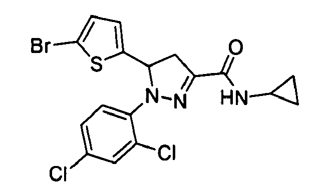 | 5-(5-Bromo-thiophen-2-yl)-1-(2,4-dichloro-phenyl)-4,5-dihydro-1H-pyrazole-3-carboxylic acid cyclopropylamide | | |
| 18 | 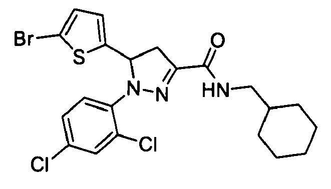 | 5-(5-Bromo-thiophen-2-yl)-1-(2,4-dichloro-phenyl)-4,5-dihydro-1H-pyrazole-3-carboxylic acid cyclohexylmethyl-amide | | |
| 19 | 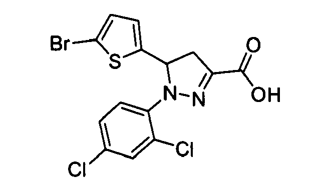 | 5-(5-Bromo-thiophen-2-yl)-1-(2,4-dichloro-phenyl)-4,5-dihydro-1H-pyrazole-3-carboxylic acid | | |
| 20 | 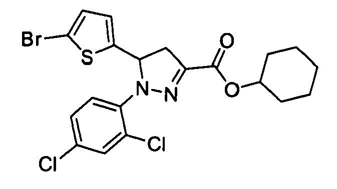 | 5-(5-Bromo-thiophen-2-yl)-1-(2,4-dichloro-phenyl)-4,5-dihydro-1H-pyrazole-3-carboxylic acid cyclohexyl ester | | |
| 21 | 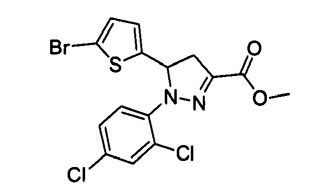 | 5-(5-Bromo-thiophen-2-yl)-1-(2,4-dichloro-phenyl)-4,5-dihydro-1H-pyrazole-3-carboxylic acid methyl ester | | |
| 22 | 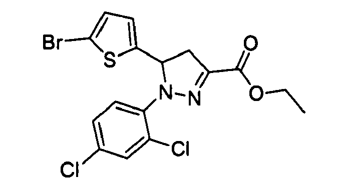 | 5-(5-Bromo-thiophen-2-yl)-1-(2,4-dichloro-phenyl)-4,5-dihydro-1H-pyrazole-3-carboxylic acid ethyl ester | | |
| 23 | 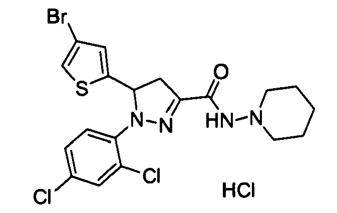 | 5-(4-Bromo-thiophen-2-yl)-1-(2,4-dichloro-phenyl)-4,5-dihydro-1H-pyrazole-3-carboxylic acid piperidin-1-ylamide hydrochloride | ¹H NMR (300 MHz, DMSO-*d*₆) δ ppm1.42(m,2H)1.72(m,4H) 3.15 (m, 4 H) 3.25 (dd, *J*=18.02, 5.13 Hz, 1 H) 3.68 (dd, *J*=18.02, 11.50 Hz, 1 H) 6.08 (dd, J=11.50, 5.13 Hz, 1 H) 6.95 (d, J=1.17 Hz, 1 H) 7.34 (m, 1 H) 7.46 (m. 2 H) 7.58 (d, *J*=2.20 Hz. | 501 |
| 24 | 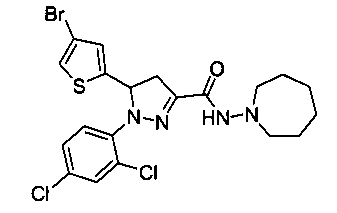 | 5-(4-Bromo-thiophen-2-yl)-1-(2,4-dichloro-phenyl)-4,5-dihydro-1H-pyrazole-3-carboxylic acid azepan-1-ylamide | | |
| 25 | 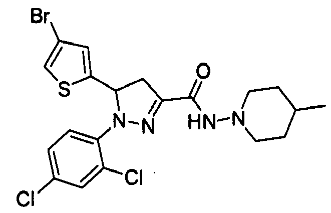 | 5-(4-Bromo-thiophen-2-yl)-1-(2,4-dichloro-phenyl)-4,5-dihydro-1H-pyrazole-3-carboxylic acid (4-methyl-piperidin-1-yl)-amide | | |
| 26 | 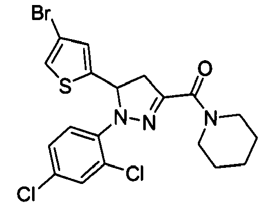 | [5-(4-Bromo-thiophen-2-yl)-1-(2,4-dichloro-phenyl)-4,5-dihydro-1H-pyrazol-3-yl]-piperidin-1-yl-methanone | | |
| 27 | 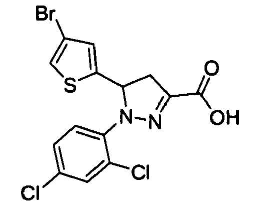 | 5-(4-Bromo-thiophen-2-yl)-1-(2,4-dichloro-phenyl)-4,5-dihydro-1H-pyrazole-3-carboxylic acid | 1 H NMR (400 MHz, CHLOROFORM-*d*) δ ppm 3.42 (dd, *J*=18.07, 4.98 Hz, 1 H) 3.69 (dd, *J*=18.07, 12.02 Hz, 1 H) 6.14 (dd, *J*=12.02, 4.98 Hz, 1 H) 6.77 (d, *J*=1.37 Hz, 1 H) 7.04 (d, *J*=1.37 Hz, 1 H) 7.13 (dd, *J*=8.60, 2.15 Hz, 1 H) 7.20 (d, *J*=8.60 Hz, 1 H) 7.35 (d, *J*=2.15 Hz, 1 H) | 419 |
| 28 | 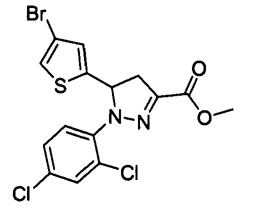 | 5-(4-Bromo-thiophen-2-yl)-1-(2,4-dichloro-phenyl)-4,5-dihydro-1H-pyrazole-3-carboxylic acid methyl ester | | |
| 29 | 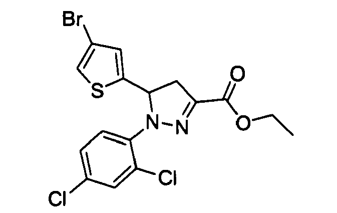 | 5-(4-Bromo-thiophen-2-yl)-1-(2,4-dichloro-phenyl)-4,5-dihydro-1H-pyrazole-3-carboxylic acid ethyl ester | | |
| 30 | 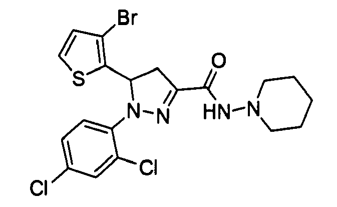 | 5-(3-Bromo-thiophen-2-yl)-1-(2,4-dichloro-phenyl)-4,5-dihydro-1H-pyrazole-3-carboxylic acid piperidin-1-ylamide | | |
| 31 | 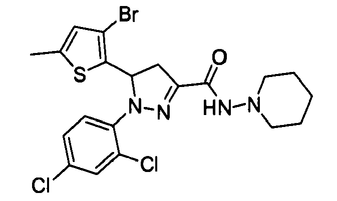 | 5-(3-Bromo-5-methyl-thiophen-2-yl)-1-(2,4-dichloro-phenyl)-4,5-dihydro-1H-pyrazole-3-carboxylic acid piperidin-1-ylamide | | |
| 32 | 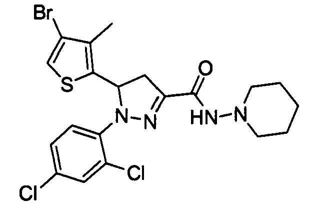 | 5-(4-Bromo-3-methyl-thiophen-2-yl)-1-(2,4-dichloro-phenyl)-4,5-dihydro-1H-pyrazole-3-carboxylic acid piperidin-1-ylamide | | |
| 33 | 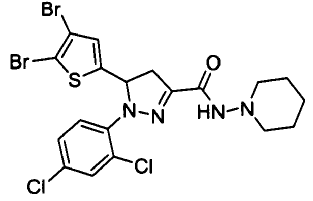 | 5-(4,5-Dibromo-thiophen-2-yl)-1-(2,4-dichloro-phenyl)-4,5-dihydro-1H-pyrazole-3-carboxylic acid piperidin-1-ylamide | | |
| 34 | 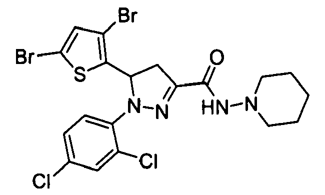 | 5-(3,5-Dibromo-thiophen-2-yl)-1-(2,4-dichloro-phenyl)-4,5-dihydro-1H-pyrazole-3-carboxylic acid piperidin-1-ylamide | | |
| 35 | 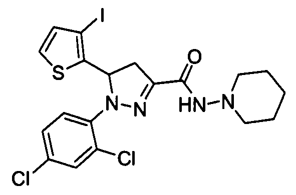 | 1-(2,4-Dichloro-phenyl)-5-(3-iodo-thiophen-2-yl)-4,5-dihydro-1H-pyrazole-3-carboxylic acid piperidin-1-ylamide | | |
| 36 | 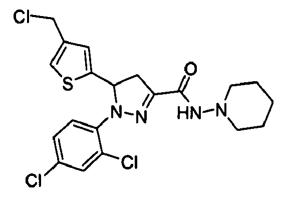 | 5-(4-Chloromethyl-thiophen-2-yl)-1-(2,4-dichloro-phenyl)-4,5-dihydro-1H-pyrazole-3-carboxylic acid piperi din-1-ylamide | | |
| 37 | 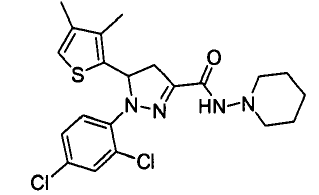 | 1-(2,4-Dichloro-phenyl)-5-(3,4-dimethyl-thiophen-2-yl)-4,5-dihydro-1H-pyrazole-3-carboxylic acid piperidin-1-ylamide | | |
| 38 | 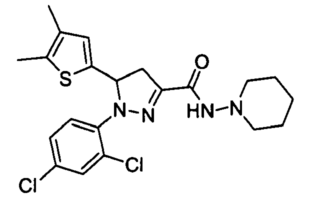 | 1-(2,4-Dichloro-phenyl)-5-(4,5-dimethyl-thiophen-2-yl)-4,5-dihydro-1H-pyrazole-3-carboxylic acid piperidin-1-ylamide | | |
| 39 | 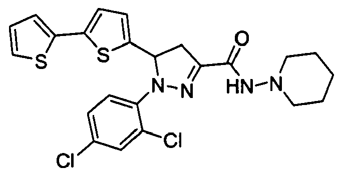 | 5-[2,2']Bithiophenyl-5-yl-1-(2,4-dichloro-phenyl)-4,5-dihydro-1H-pyrazole-3-carboxylic acid piperidin-1-ylamide | | |
| 40 | 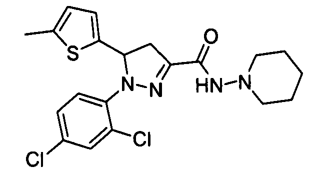 | 1-(2,4-Dichloro-phenyl)-5-(5-methyl -thiophen-2-yl)-4,5-dihydro-1H-pyrazole-3-carboxylic acid piperidin-1-ylamide | | |
| 41 | 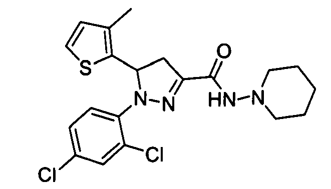 | 1-(2,4-Dichloro-phenyl)-5-(3-methyl -thiophen-2-yl)-4,5-dihydro-1H-pyrazole-3-carboxylic acid piperidin-1-ylamide | | |
| 42 | 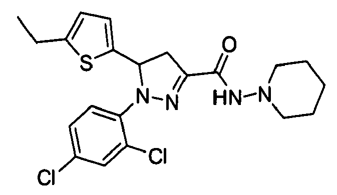 | 1-(2,4-Dichloro-phenyl)-5-(5-ethyl-thiophen-2-yl)-4,5-dihydro-1H-pyrazole-3-carboxylic acid piperidin-1-ylamide | | |
| 43 | 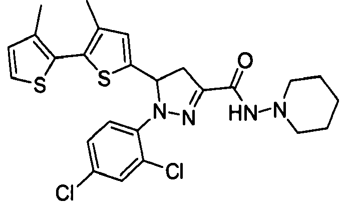 | 1-(2,4-Dichloro-phenyl)-5-(3,3'-dim ethyl-[2 ,2']bithiophenyl-5-yl)-4,5-dihydro-1H-pyrazole-3-carboxylic acid piperidin-1-ylamide | | |
| 44 | 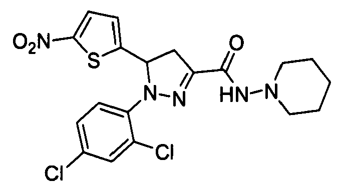 | 1-(2,4-Dichloro-phenyl)-5-(5-nitro-thiophen-2-yl)-4, 5-dihydro-1H-pyrazole-3-carboxylic acid piperidin-1-ylamide | | |
| 45 | 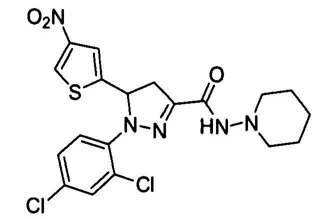 | 1-(2,4-Dichloro-phenyl)-5-(4-nitro-thiophen-2-yl)-4,5-dihydro-1H-pyrazole-3-carboxylic acid piperidin-1-ylamide | | |
| 46 | 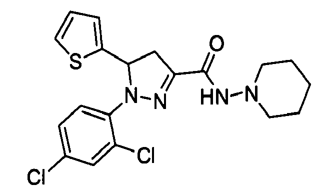 | 1-(2,4-Dichloro-phenyl)-5-thiophen-2-y)-4,5-dihydro-1H-pyrazole-3-carboxylic acid piperidin-1-ylamide | | |
| 47 | 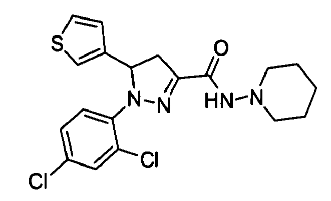 | 1-(2,4-Dichloro-phenyl)-5-thiophen-3-yl-4,5-dihydro-1H-pyrazole-3-carboxylic acid piperidin-1-ylamide | | |
| 48 | 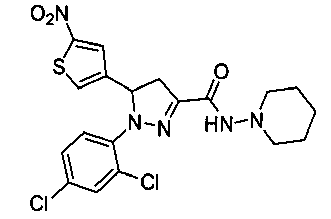 | 1-(2,4-Dichloro-phenyl)-5-(5-nitro-thiophen-3-yl)-4,5-dihydro-1H-pyrazole-3-carboxylic acid piperidin-1-ylamide | | |

### SUBGROUP A4

### Subgroup A4-Chemical Examples

The following compounds are prepared according to the processes described above. Those skilled in the art are familiar with the starting materials that are needed to obtain said compounds

| **N°** | **STRUCTURE** | **Name** | **¹H-NMR** | **MS (M+H)⁺** |
|---|---|---|---|---|
| 1 | 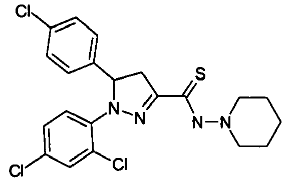 | 5-(4-Chloro-phenyl)-1-(2,4-dichloro-phenyl)-4,5-dihydro-1H-pyrazole-3-carbothioic acid piperidin-1-ylamide | | |
| 2 | 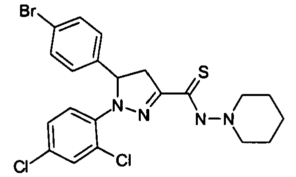 | 5-(4-Bromo-phenyl)-1-(2,4-dichloro-phenyl)-4,5-dihydro-1H-pyrazole-3-carbothioic acid piperidin-1-ylamide | | |
| 3 | 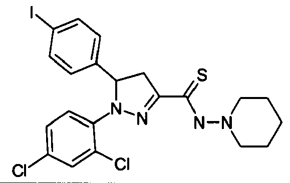 | 1-(2,4-Dichloro-phenyl)-5-(4-iodo-phenyl)-4,5-dihydro-1H-pyrazole-3-carbothioic acid piperidin-1-ylamide | | |
| 4 | 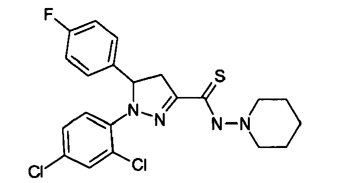 | 1-(2,4-Dichloro-phenyl)-5-(4-fluoro-phenyl)-4,5-dihydro-1H-pyrazole-3-carbothioic acid piperidin-1-ylamid e | | |
| 5 | 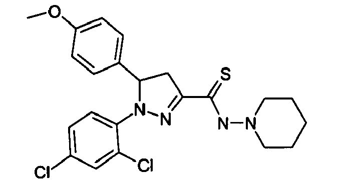 | 1-(2,4-Dichloro-phenyl)-5-(4-methoxy-phenyl)-4,5-dihydro-1H-pyrazole-3 -carbothioic acid piperidin-1-ylami de | | |
| 6 | 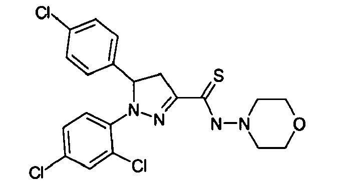 | 5-(4-Chloro-phenyl)-1-(2,4-dichloro-phenyl)-4,5-dihydro-1H-pyrazole-3-carbothioic acid morpholin-4-ylamide | | |
| 7 | 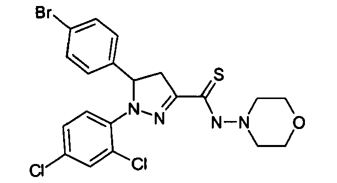 | 5-(4-Bromo-phenyl)-1-(2,4-dichloro-phenyl)-4,5-dihydro-1H-pyrazole-3-c arbothioic acid morpholin-4-ylamide | | |
| 8 | 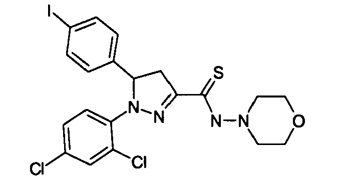 | 1-(2,4-Dichloro-phenyl)-5-(4-iodo-phenyl)-4,5-dihydro-1H-pyrazole-3-ca rbothioic acid morpholin-4-ylamide | | |
| 9 | 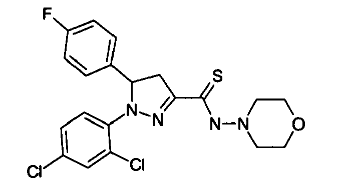 | 1-(2,4-Dichloro-phenyl)-5-(4-fluoro-phenyl)-4,5-dihydro-1H-pyrazole-3-carbothioic acid morpholin-4-ylamide | | |
| 10 | 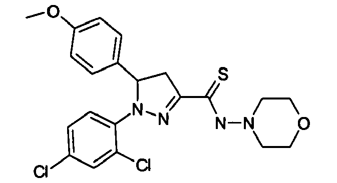 | 1-(2,4-Dichloro-phenyl)-5-(4-methox y-phenyl)-4,5-dihydro-1H-pyrazole-3 -carbothioic acid morpholin-4-ylami de | | |
| 11 | 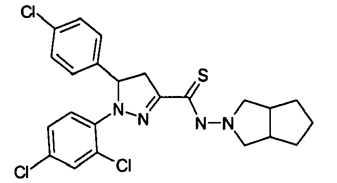 | 5-(4-Chloro-phenyl)-1-(2,4-dichloro-phenyl)-4,5-dihydro-1H-pyrazole-3-carbothioic acid (hexahydro-cyclope nta[c]pyrrol-2-yl)-amide | | |
| 12 | 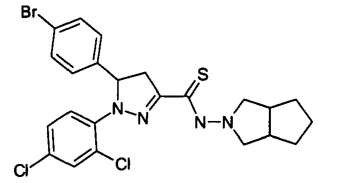 | 5-(4-Bromo-phenyl)-1-(2,4-dichloro-phenyl)-4,5-dihydro-1H-pyrazole-3-carbothioic acid (hexahydro-cyclopenta[c]pyrrol-2-yl)-amide | | |
| 13 | 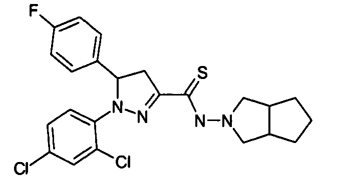 | 1-(2,4-Dichloro-phenyl)-5-(4-fluoro-phenyl)-4,5-dihydro-1H-pyrazole-3-carbothioic acid (hexahydro-cyclope nta[c]pyrrol-2-yl)-amide | | |
| 14 | 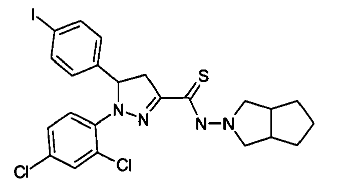 | 1-(2,4-Dichloro-phenyl)-5-(4-iodo-phenyl)-4,5-dihydro-1H-pyrazole-3-ca rbothioic acid (hexahydro-cyclopent a[c]pyrrol-2-yl)-amide | | |
| 15 | 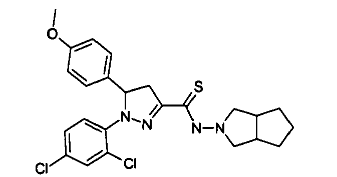 | 1-(2,4-Dichloro-phenyl)-5-(4-methoxy-phenyl)-4,5-dihydro-1H-pyrazole-3-carbothioic acid (hexahydro-cyclopenta[c]pyrrol-2-yl)-amide | | |
| 16 | 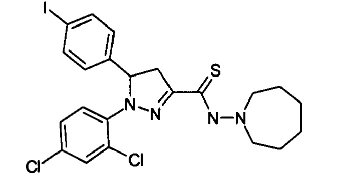 | 1-(2,4-Dichloro-phenyl)-5-(4-iodo-p henyl)-4,5-dihydro-1H-pyrazole-3-ca rbothioic acid azepan-1-ylamide | | |
| 17 | 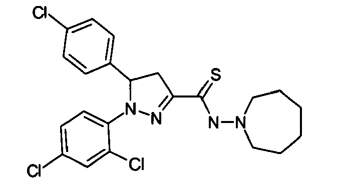 | 5-(4-Chloro-phenyl)-1-(2,4-dichloro-phenyl)-4,5-dihydro-1H-pyrazole-3-carbothioic acid azepan-1-ylamide | | |
| 18 | 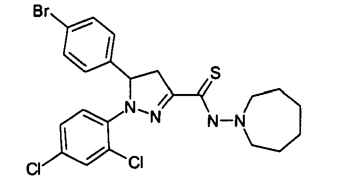 | 5-(4-Bromo-phenyl)-1-(2,4-dichloro-phenyl)-4,5-dihydro-1H-pyrazole-3-c arbothioic acid azepan-1-ylamide | | |
| 19 | 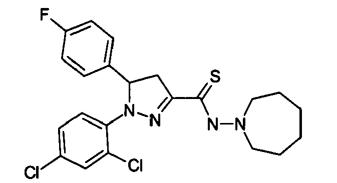 | 1-(2,4-Dichloro-phenyl)-5-(4-fluoro-phenyl)-4,5-dihydro-1H-pyrazole-3-carbothioic acid azepan-1-ylamide | | |
| 20 | 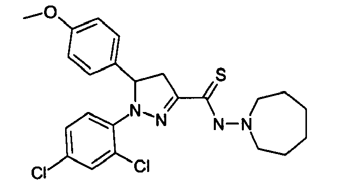 | 1-(2,4-Dichloro-phenyl)-5-(4-methoxy-phenyl)-4,5-dihydro-1H-pyrazole-3-carbothioic acid azepan-1-ylamide | | |
| 21 | 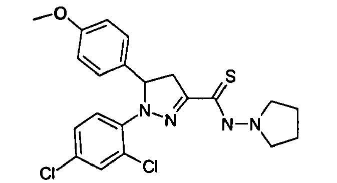 | 1-(2,4-Dichloro-phenyl)-5-(4-methoxy-phenyl)-4,5-dihydro-1H-pyrazole-3-carbothioic acid pyrrolidin-1-ylamide | | |
| 22 | 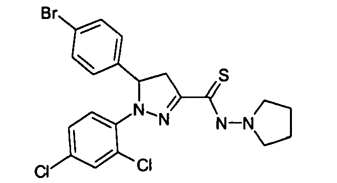 | 5-(4-Bromo-phenyl)-1-(2,4-dichloro-phenyl)-4,5-dihydro-1H-pyrazole-3-c arbothioic acid pyrrolidin-1-ylamide | | |
| 23 | 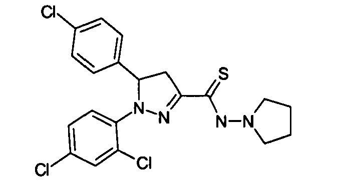 | 5-(4-Chloro-phenyl)-1-(2,4-dichloro-phenyl)-4,5-dihydro-1H-pyrazole-3-carbothioic acid pyrrolidin-1-ylamide | | |
| 24 | 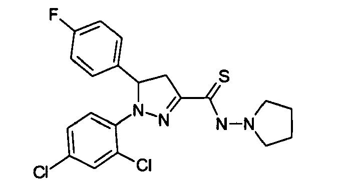 | 1-(2,4-Dichloro-phenyl)-5-(4-fluoro-phenyl)-4,5-dihydro-1H-pyrazole-3-carbothioic acid pyrrolidin-1-ylamide | | |
| 25 | 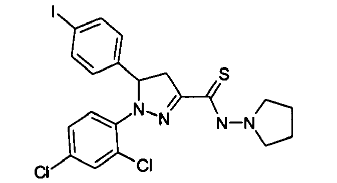 | 1-(2,4-Dichloro-phenyl)-5-(4-iodo-phenyl)-4,5-dihydro-1H-pyrazole-3-ca rbothioic acid pyrrolidin-1-ylamide | | |
| 26 | 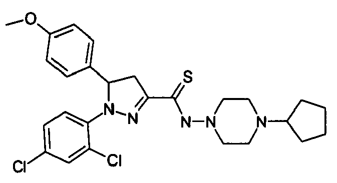 | 1-(2,4-Dichloro-phenyl)-5-(4-methoxy-phenyl)-4,5-dihydro-1H-pyrazole-3-carbothioic acid (4-cyclopentyl-piperazin-1-yl)-amide | | |
| 27 | 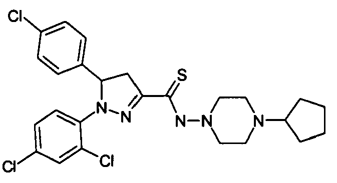 | 5-(4-Chloro-phenyl)-1-(2,4-dichloro-phenyl)-4,5-dihydro-1H-pyrazole-3-carbothioic acid (4-cyclopentyl-piperazin-1-yl)-amide | | |
| 28 | 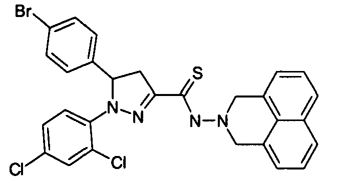 | 5-(4-Bromo-phenyl)-1-(2,4-dichloro-phenyl)-4,5-dihydro-1H-pyrazole-3-carbothioic acid (1H,3H-benzo[de]iso quinolin-2-yl)-amide | | |
| 29 | 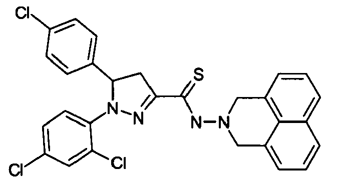 | 5-(4-Chloro-phenyl)-1-(2,4-dichloro-phenyl)-4,5-dihydro-1H-pyrazole-3-carbothioic acid (1H,3H-benzo[de]isoquinolin-2-yl)-amide | | |
| 30 | 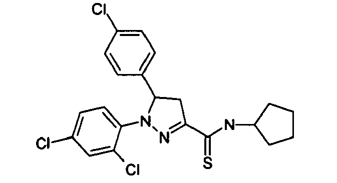 | 5-(4-Chloro-phenyl)-1-(2,4-dichloro-phenyl)-4,5-dihydro-1H-pyrazole-3-carbothioic acid cyclopentylamide | | |
| 31 | 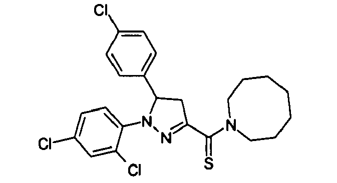 | Azocan-1-yl-[5-(4-chloro-phenyl)-1-(2,4-dichloro-phenyl)-4,5-dihydro-1H-pyrazol-3-yl]-methanethione | | |
| 32 | 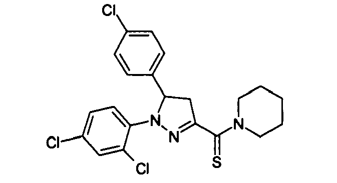 | [5-(4-Chloro-phenyl)-1-(2,4-dichloro-phenyl)-4,5-dihydro-1H-pyrazol-3-yl]-piperidin-1-yl-methanethione | | |

### SUBGROUP A5.1

### Subgroup A5.1: Chemical

### Preparation of 4-(4-chlorophenyl)-2-oxo-3-butenoic acid

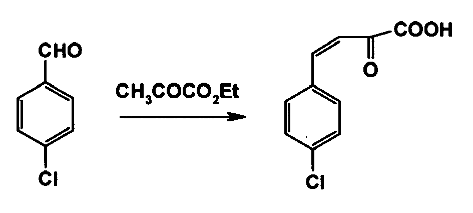

p-Chlorobenzaldehyde (13,3 g, 95 mmol) and ethyl pyruvate (10 g, 86 mmol) were dissolved in 150 mL of absolute ethanol. The solution was cooled to 0°C and an aqueous solution of NaOH (3.8 g in 45 mL water) was added drop wise keeping the temperature below or equal to 10°C, whereby a yellow-orange coloured precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate was formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, cm⁻¹): 3500-2500, 1719,3, 1686,5, 1603,4, 1587,8, 1081,9.
¹H NMR(CDCl₃, 300 MHz) δ 7,4 (d, J=8,4Hz, 2H), 7,5 (d, J=16,1 Hz, 1 H), 7,6 (d, J=8,4Hz, 2H), 8,1 (d, J=16,1 Hz, 1 H).

### Preparation of 5-(4-chlorophenyl)-1-(2,4-dichlorophenyl)-4,5-dihydro-pyrazole-3-carboxylic acid

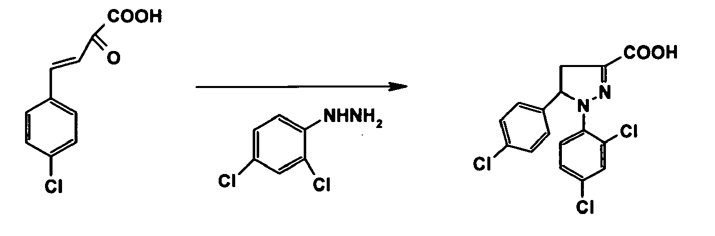

4-(4-Chlorophenyl)-2-oxo-3-butenoic acid (12.6 g, 60 mmol), 2,4-dichlorophenylhydrazine hydrochloride (12.8 g, 60 mmol) and glacial acetic acid (200 mL) were mixed under a nitrogen atmosphere and heated to reflux for 4 hours, cooled down to room temperature (approximately 25 °C) and poured into ice, whereby a sticky mass was obtained, which was extracted with methylene chloride. The combined methylene chloride fractions were washed with water, dried over sodium sulfate, filtered and evaporated to dryness to give a pale yellow solid (12.7 g, 57% of theoretical yield).

IR (KBr, cm⁻¹) : 3200-2200, 1668,4, 1458, 1251,4, 1104,8.
¹H NMR (CDCl₃, 300 MHz) δ 3,3 (dd, 1 H), 3,7 (dd, 1 H), 5,9 (dd, 1 H), 7,09-7,25 (m, 7H).

### Preparation of 5-(4-chlorophenyl)-1-(2,4-dichlorophenyl)-4,5-dihydro-pyrazole-3-carboxylic acid chloride

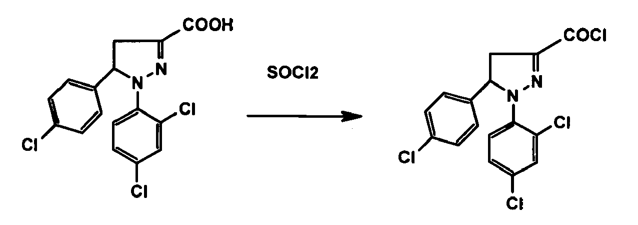

5-(4-Chlorophenyl)-1-(2,4-dichlorophenyl)-4,5-dihydro-pyrazole-3-carboxylic acid (5,54 g, 15 mmols) was dissolved in 120 mL of dry toluene and thionyl chloride (2.14g, 18 mmol) was added. The mixture was heated to 80 °C for 2.5 hours. The solvent was removed under reduced pressure and the resulting crude residue (6.06 g, 100 %) was used without any further purification.
IR (KBr, cm⁻¹) : 1732, 1700, 1533, 1478, 1212, 826

### Subgroup A5.1: Chemical Example 1: Preparation of 5-(4-Chloro-phenyl)-1-(2,4-dichloro-phenyl)-4,5-dihydro-1H-pyrazole-3-carboxylic acid piperidin-1-ylamide

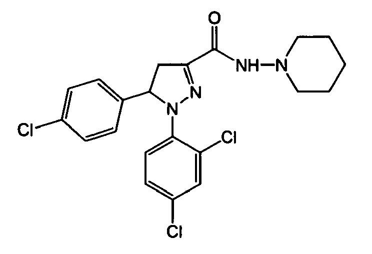

N-Aminopiperidine (0.6 mL, 5.6 mmol) and triethylamine (4 mL) were dissolved in methylene chloride (25 mL) under nitrogen atmosphere. The resulting mixture was - cooled to 0°C and a solution of 5-(4-chlorophenyl)-1-(2,4-dichlorophenyl)-4,5-dihydro-pyrazole-3-carboxylic acid chloride in methylene chloride (15 mL) was added drop wise. The resulting reaction mixture was stirred at room temperature (approximately 25 °C) overnight. The reaction mixture was washed with water, followed by a saturated aqueous solution of sodium bicarbonate, again with water, dried over sodium sulfate, filtered and evaporated to dryness in a rotavapor. The resulting crude solid was crystallised from ethanol. The crystallised solid was removed via filtration and the mother liquors were concentrated to yield a second fraction of crystallised product. The two fractions were combined to give a total amount of 1.7 g (57% of theoretical yield) of the title compound having a melting point of 183-186°C.

IR (KBr, cm⁻¹): 3222,9, 2934,9, 1647,4, 1474,7, 1268,3, 815,6.
¹H NMR ( CDCl₃, 8) : 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1 H), 5,7 (dd, J=6,1 and 12,5 Hz, 1 H), 7,0-7,2 (m, 6H), 7,4 (s, 1 H).

The following compounds were prepared according to the processes described for the preparation of compound 1 above. Those skilled in the art are familiar with the starting materials that are needed to obtain said compounds.

### Subgroup A5.1: Chemical Example 7: N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0.15 g, 0.33 mmol) was dissolved in 7 mL of dichloromethane. The resulting solution was ice-cooled to 0 °C and m-chloroperbenzoic acid (0.204 g, 0.83 mmol) was added in several portions. After stirring for 15 minutes a control via thin layer chromatography showed that no starting material was present. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120 °C.
IR (KBr, cm⁻¹): 3202, 1678, 1654, 1474, 1309, 1107.
¹H-NMR (CDCl₃, δ): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1 H), 7.0-7.3 (m, 7H), 8.5 (s, 1 H.)

### Subgroup A5.1: Chemical Example 120:[5-(4-Chloro-phenyl)-1-(2,4-dichloro-phenyl)-4,5-dihydro-1H-pyrazol-3-carboxylic acid ethyl ester

5-(4-chlorophenyl)-1-(2,4-dichlorophenyl)-4,5-dihydro-pyrazole-3-carboxylic acid (0.55 g, 1.50 mmol) was dissolved in 20 mL of toluene and (2 mL) ethanol and (0.05 g) of p-toluenesulfonic acid monohydrate were added. The mixture was heated at 80 °C for 72 hours. After cooling to room temperature, the reaction mixture washed with sodium hydrogen carbonate solution and water, dried over sodium sulphate and evaporated to dryness to give the title compound in form of oil.

IR (film, cm⁻¹): 2981, 1728, 1704, 1478, 1246.

The compounds according to the following examples 68 to 172 have been prepared by the methods described for the preparation of Example 1. The other examples were prepared or can be prepared by the methods described above.

### Subgroup A5.1: Chemical Example 68: 5-(4-Bromo-phenyl)-1-(2,4-dichloro-phenyl)-4,5-dihydro-1H-pyrazole-3-carboxylic acid azepan-1-ylamide

### Preparation of 4-(4-bromophenyl)-2-oxo-3-butenoic acid

This compound was obtained following the same method described in Example 1 starting from p-bromobenzaldehyde.
¹H NMR (400 MHz, CDCl₃,) : δ7.55 (2H, m, ArH,), 7.61 (3H, m, Ar H), 8.09 (1 H, d, J 16.4 Hz)
¹³C NMR (100 MHz, CDCl₃): δ 118.1 (CH), 127.2 (C), 130.7 (CH), 132.7 (CH), 149.7 (C + CH), 160.0 (CO), 182.2 (CO).

### Preparation of 5-(4-bromophenyl)-1-(2,4-dichlorophenyl)-4,5-dihydro-pyrazole-3-carboxylic acid

IR (KBr, cm⁻¹): 3200-2200, 1685, 1571,1549, 1480, 1112
¹H NMR (400 MHz, CDCl₃)) : δ 3.27 (1 H, dd, J 18.0, 6.4Hz, ArH,), 3.71 (1 H, dd, J 18, 12.8 Hz, Ar H), 5.88 (1 H, dd, J 12.8, 6.4Hz, CH), 7.02 (2H, d, J 7.6Hz, ArH), 7.08 (1 H, m, ArH), 7.19 (1 H, d J 8.4Hz, ArH), 7.26 (1 H, m, ArH), 7.37 (2H, d, 7.6 Hz, ArH) ¹³C NMR (100 MHz, CDCl₃): δ 40.4 (CH₂), 67.4 (CH), 122.7 (C), 126.0 (CH), 126.5 (C), 127.6 (CH), 128.3 (CH), 130.2 (CH), 131.2 (C), 132.2 (CH), 138.4 (C), 138.5 (C), 140.2(C), 165.7(C).

### Preparation of 5-(4-bromophenyl)-1-(2,4-dichlorophenyl)-4,5-dihydro-pyrazole-3-carboxylic acid chloride

IR (KBr, cm⁻¹) : 1737, 1534, 1477, 1212, 1127.

### Preparation of 5-(4-Bromo-phenyl)-1-(2,4-dichloro-phenyl)-4,5-dihydro-1H-pyrazole-3-carboxylic acid azepan-1-ylamide.

This compound was obtained following the same method described in Example 1 starting from 5-(4-bromophenyl)-1-(2,4-dichlorophenyl)-4,5-dihydro-pyrazole-3-carboxylic acid chloride and azepan-1-amine.

¹H NMR (300 MHz, CHLOROFORM-*d*) δ ppm 1.75 (br. s., 4 H) 2.00 (br. s., 4 H) 3.26 (dd, *J*=18.16, 6.15 Hz, 1 H) 3.71 (dd, *J*=18.16, 12.45 Hz, 1 H) 3.65 (br. s., 4 H) 5.82 (dd, J=12.45, 6.15 Hz, 1 H) 6.97 (d, *J*=8.35 Hz, 2 H) 7.10 (d, *J*=2.20 Hz, 1 H) 7.16 - 7.22 (m, 1 H) 7.24 (d, *J*=2.20 Hz, 1 H) 7.35 (d, *J*=8.50 Hz, 2 H)
MS (M+H)⁺: 510

### Subgroup A5.1: Chemical Example 96: 1-(2,4-Dichloro-phenyl)-5-(4-fluoro-phenyl)-4,5-dihydro-1H-pyrazole-3-carboxylic acid azepan-1-ylamide

### Preparation of 4-(4-fluorophenyl)-2-oxo-3-butenoic acid

¹H NMR (400 MHz, CDCl₃,): δ 7.15 (2H, apt, J 8.4 Hz, ArH,), 7.53 (1 H, d, J 16.0Hz, CH), 7.70 (2H, m, ArH), 8.11(1H, d, J 16.0 Hz)
¹³C NMR (100 MHz, CDCl₃): δ 116.5 (CH, d, J_{F} 24 Hz), 117.4 (CH, d, J_{F} 8.7 Hz), 130.1 (C, d, J_{F} 2.4 Hz), 131.7 (CH, d, J_{F} 9.1 Hz ), 149.9 (CH), 162.0 (C, d, J_{F} 320 Hz), 166.7 (CO), 182.4 (CO).

### Preparation of 5-(4-fluorophenyl)-1-(2,4-dichlorophenyl)-4,5-dihydro-pyrazole-3-carboxylic acid

IR (KBr, cm⁻¹): 3200-2200, 1687.5, 1478, 1230, 1107
¹H NMR (400 MHz, CDCl₃) : δ 3.30 (1 H, dd, J 18.0, 6.4Hz, ArH,), 3.72 (1 H, dd, J 18, 12.8 Hz, Ar H), 5.93 (1 H, dd, J 12.8, 6.4 Hz, CH), 6.92 (2H, t, J 8.4Hz, ArH), 7.09-7.12 (3H, m, ArH), 7.22 (1 H, bs, ArH), 7.26 (1 H, m, ArH)
¹³C NMR (100 MHz, CDCl₃): δ 40.7 (CH₂), 67.8 (CH), 115.9 (CH, d, J_{F} 21 Hz), 126.1 (CH), 126.5 (C), 127.6 (CH), 128.3 (CH, d, J_{F} 8.2 Hz), 130.1 (CH), 131.2 (C), 135.1 (C, d, J_{F} 3Hz ), 138.6 (C), 140.1 (C), 160.6 (C, d, J_{F} 240 Hz), 166.3 (C).

### Preparation of 5-(4-fluorophenyl)-1-(2,4-dichlorophenyl)-4,5-dihydro-pyrazole-3-carboxylic acid chloride

IR (KBr, cm⁻¹): 1733, 1548, 1511, 1478, 1212, 832.

### Preparation of 1-(2,4-Dichloro-phenyl)-5-(4-fluoro-phenyl)-4,5-dihydro-1H-pyrazole-3-carboxylic acid azepan-1-ylamide

1 H NMR (300 MHz, CHLOROFORM-*d*) δ ppm 1.75 (br. s., 4 H) 2.00 (br. s., 4 H) 3.28 (dd, *J*=18.16, 6.08 Hz, 1 H) 3.68 (dd, *J*=18.16, 12.38 Hz, 1 H) 3.68 (br. s., 4 H) 5.84 (dd, *J*=12.38, 6.08 Hz, 1 H) 6.90 (t, *J*=8.57 Hz, 2 H) 7.08 (m, 3 H) 7.14 - 7.19 (m, 1 H) 7.24 (d, *J*=2.20 Hz, 1 H)
MS (M+H)⁺: 449

### Subgroup A5.1: Chemical Example 106: 1-(2,4-Dichloro-phenyl)-5-(4-methoxyphenyl)-4,5-dihydro-1H-pyrazole-3-carboxylic acid piperidin-1-ylamide hydrochloride

### Preparation of 4-(4-methoxyphenyl)-2-oxo-3-butenoic acid

¹H NMR (400 MHz, CDCl₃): δ 3.88 (3H, s, OCH₃), 6.95 (2H, d, J 6.8 Hz), 7.45 (2H, d, J 15.6 H), 7.65 (2H, d, J 6.8 Hz), 8.09 (2H, d, J 15.6 Hz).

### Preparation of 5-(4-methoxyphenyl)-1-(2,4-dichlorophenyl)-4,5-dihydro-pyrazole-3-carboxylic acid

IR (KBr, cm⁻¹) 3200-2200, 1685, 1513,1478,1248,1105
¹H NMR (400 MHz, CDCl₃): δ 1.45 (2H, m, CH₂), 1.75 (4H, m), 2.84 (4H, m), 3.45 (1H, dd J 4.8, 18Hz), 3.61 (1 H, dd J 11.2, 19.6 Hz), 5.93 (1 H, dd J 4.8, 11.2 Hz), 6.57 (2H, ap s), 7.13 (2H, m), 7.32 (1 H, m), 7.40 (1 H, m)
¹³C NMR (100 MHz, CDCl₃): δ 23.4 (CH₂), 25.3 (CH₂), 40.5 (CH₂), 57.3 (CH₂), 63.7 (CH), 125.7 (CH), 125.8 (CH), 126.1 (CH), 127.1 (C), 127.8 (CH), 130.1 (CH), 131.1 (C), 139.6 (C), 139.9 (C), 146.1 (C), 158.9 (CO).

### Preparation of 5-(4-methoxyphenyl)-1-(2,4-dichlorophenyl)-4,5-dihydro-pyrazole-3-carboxylic acid chloride

IR (KBr, cm⁻¹): 1735, 1513, 1477, 1249,1129

### Preparation of 1-(2,4-Dichloro-phenyl)-5-(4-methoxy-phenyl)-4,5-dihydro-1H-pyrazole-3-carboxylic acid piperidin-1-ylamide hydrochloride

¹H NMR (300 MHz, CHLOROFORM-*d*) δ ppm 1.67 (br. s., 2 H) 2.10 (br. s., 4 H) 3.25 (dd, *J*=18.02, 6.01 Hz, 1 H) 3.64 (dd, *J*=18.02, 12.60 Hz, 1 H) 3.72 (s, 3 H) 3.89 (br. s., 4 H) 5.85 (dd, *J*=12.60, 6.01 Hz, 1 H) 6.72 (d, *J*=8.79 Hz, 2 H) 7.00 (d, *J*=8.64 Hz, 2 H) 7.07 (dd, *J*=8.64, 2.20 Hz, 1 H) 7.22 (d, *J*=8.64 Hz, 1 H) 7.22 (d, *J*=2.20 Hz, 1 H) 9.63 (br. s., 1 H)
MS (M+H)⁺: 447

### Subgroup A5.1: Chemical Example 109: 1-(2,4-Dichloro-phenyl)-5-(4-methoxyphenyl)-4,5-dihydro-1H-pyrazole-3-carboxylic acid (hexahydro-cyclopenta[c]pyrrol-2-yl)-amide hydrochloride

¹H NMR (300 MHz, CHLOROFORM-*d*) δ ppm 1.61 - 1.91 (m, 6 H) 2.96 (br. s., 2 H) 3.27 (dd, *J*=18.09, 5.86 Hz, 1 H) 3.49 (m, 2 H) 3.64 (dd, *J*=18.09, 12.60 Hz, 1 H) 3.72 (s, 3 H) 3.90 (br. s., 2 H) 5.82 (dd, *J*=12.60, 5.86 Hz, 1 H) 6.71 (d, *J*=8.64 Hz, 2 H) 7.00 (d, *J*=8.64 Hz, 2 H) 7.06 (dd, *J*=8.72, 2.27 Hz, 1 H) 7.21 (m, 2 H) 9.25 (br. s., 1 H)
MS (M+H)⁺: 473

### Subgroup A5.1: Chemical Example 172: 1-(2,4-Dichloro-phenyl)-5-(4-hydroxyphenyl)-4,5-dihydro-1H-pyrazole-3-carboxylic acid piperidin-1-ylamide hydrochloride

The free base of compound 106 (0.82 mmol) was dissolved in dichloromethane (10 ml) and a solution of 1 M BBr₃ (5 eq) in dichloromethane was slowly added at 0 °C and the reation mixture was stirred at room temperature for 8 h. The solid formed was filtered off and the solvent was evaporated. A solution of 2 N HCl in diethyl ether was added to form the hydrochloride.

¹H NMR (300 MHz, METHANOL-*d*₄) δ ppm 1.69 (br. s., 2 H) 2.00 (br. s., 4 H) 3.24 (dd, *J*=17.94, 6.23 Hz, 1 H) 3.55 (br. s., 4 H) 3.69 (dd, *J*=17.94, 12.38 Hz, 1 H) 5.86 (dd, *J*=12.38, 6.23 Hz, 1 H) 6.61 (d, *J*=8.50 Hz, 2 H) 7.00 (d, *J*=8.50 Hz, 2 H) 7.17 (dd, *J*=8.64, 2.20 Hz, 1 H) 7.26 - 7.38 (m, 2 H)
MS (M+H)⁺: 433

### Subgroup A5.1: Chemical Example 174: 1-(2,4-Dichloro-phenyl)-5-(4-hydroxyphenyl)-4,5-dihydro-1 H-pyrazole-3-carboxylic acid (hexahydro-cyclopenta[c]pyrrol-2-yl)-amide hydrochloride

The free base of compound 109 (0.82 mmol) was dissolved in dichloromethane (10 ml) and a solution of 1 M BBr₃ (5 eq) in dichloromethane was slowly added at 0 °C and the reation mixture was stirred at room temperature for 8 h. The solid formed was filtered off and the solvent was evaporated. A solution of 2 N HCl in diethyl ether was added to form the hydrochloride.

1H NMR (300 MHz, METHANOL-d₄) δ ppm 1.68 (br. s., 2 H) 1.81 (br. s., 4 H) 2.98 (br. s., 2 H) 3.06 - 3.21 (m, 2 H) 3.26 (dd, *J*=18.02, 6.30 Hz, 1 H) 3.71 (dd, *J*=18.02, 12.45 Hz, 1 H) 4.06 (m, 2 H) 5.88 (dd, *J*=12.45, 6.30 Hz, 1 H) 6.63 (d, *J*=8.64 Hz, 2 H) 7.02 (d, *J*=8.64 Hz, 2 H) 7.18 (dd, *J*=8.64, 2.34 Hz, 1 H) 7.37 (d, *J*=2.20 Hz, 1 H) 7.34 (d, *J*=8.79 Hz, 1 H)
MS (M+H)⁺: 459

| **N°** | **STRUCTURE** | **Name** | **¹H-NMR** | **MS (M+H)⁺** |
|---|---|---|---|---|
| 7 | 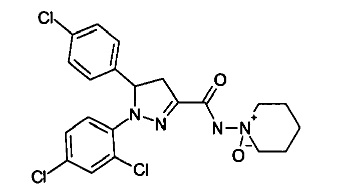 | N-oxide of 5-(4-Chloro-phenyl)-1-(2,4-dichloro-phenyl)-4,5-dihydro-1H-pyrazole-3-carboxylic acid piperidin-1-ylamide | 1 H NMR (300 MHz, CHLOROFORM-*d*) δ ppm 1.6 (m, 2 H) 1.8-2.0 (m, 4 H) 2.55 (m, 2 H) 3.3 (dd, *J=* 18.2, 6.3 Hz, 1 H) 3.7 (m, 3H) 5.8 (dd, *J* = 12.5, 6.3 Hz, 1 H) 7.0-7.3 (m, 7 H) 8.5 (s, 1 H). | |
| 8 | 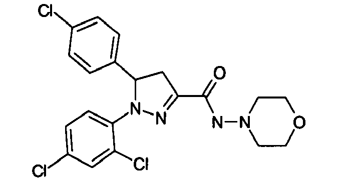 | 5-(4-Chloro-phenyl)-1-(2,4-dichloro-phenyl)-4,5-dihydro-1H-pyrazole-3-carboxylic acid morpholin-4-ylamide | 1 H NMR (300 MHz, CHLOROFORM-*d*) δ ppm 3.00 (m, 4 H) 3.32 (dd, *J*=18.31, 6.30 Hz, 1 H) 3.69 (dd, *J*=18.31, 12.30 Hz, 1H) 3.90 (m, 4 H) 5.75 (dd, *J*=12.30, 6.30 Hz, 1 H) 7.07 (m, 4 H) 7.17 (m, 3 H) 7.68 (s, 1 H) | 453 |
| 9 | 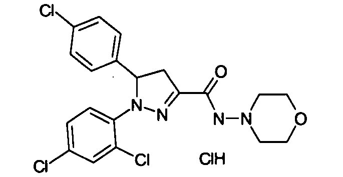 | 5-(4-Chloro-phenyl)-1-(2,4-dichloro-phenyl)-4,5-dihydro-1H-pyrazole-3-carboxylic acid morpholin-4-ylamide hydrochloride | | 453 |
| 10 | 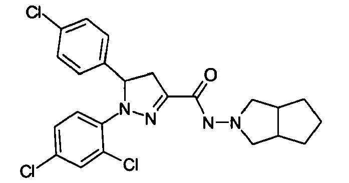 | 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide | 1 H NMR (400 MHz, CHLOROFORM-*d*) d ppm 1.61 (m, 6 H) 2.77 (m, 4 H) 3.31 (dd, *J*=18.17, 6.06 Hz, 1 H) 3.47 (s, 2 H) 3.68 (dd, *J*=18.17, 12.11 Hz, 1 H) 5.75 (dd, *J=*12.11, 6.06 Hz, 1 H) 7.05 (m, 3 H) 7.16 (t, *J*=8.60 Hz, 2 H) 7.25 (m, 2 H) 7.75 (br, 1 H) | 477 |
| 11 | 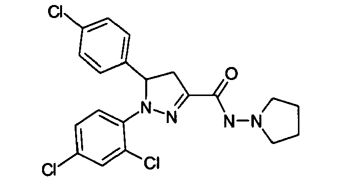 | 5-(4-Chloro-phenyl)-1-(2,4-dichloro-phenyl)-4,5-dihydro-1H-pyrazole-3-carboxylic acid pyrrolidin-1-ylamide | 1 H NMR (400 MHz, CHLOROFORM-*d*) δ ppm 2.06 (s, 4 H) 3.28 (m, 5H) 3.69 (dd, *J*=17.98, 12.50 Hz, 1 H) 5.79 (dd, *J*=12.50, 5.86 Hz, 1 H) 7.06 (m, 3 H) 7.18 (m, 3 H) 7.25(m, 1 H) 8.22 (br, 1 H) | 437 |
| 12 | 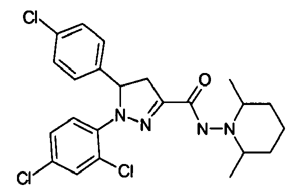 | 5-(4-Chloro-phenyl)-1-(2,4-dichloro-phenyl)-4,5-dihydro-1H-pyrazole-3-carboxylic acid (2,6-dimethyl-piperidin-1-yl)-amide | 1 H NMR (400 MHz, CHLOROFORM-*d*) δ ppm 1.16 (m, 6 H) 1.59 (m, 2H) 1.71 (m, 4 H) 2.39 (m, 2 H) 3.35 (dd, *J=*18.56, 4.88 Hz, 1 H) 3.70 (dd, *J*=18.56, 12.50 Hz, 1 H) 5.71 (dd, *J=*12.50*,* 6.06 Hz, 1 H) 6.84 (br, 1 H) 7.06 (m, 4 H) 7.18 (d, *J*=8.21 Hz, 2 H) 7.26 (m, 1 H) | 479 |
| 13 | 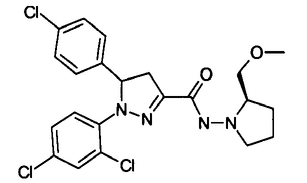 | 5-(4-Chloro-phenyl)-1-(2,4-dichloro-phenyl)-4,5-dihydro-1H-pyrazole-3-carboxylic acid (2-methoxymethyl-pyrrolidin-1-yl)-amide | 1 H NMR (400 MHz, CHLOROFORM-*d*) δ ppm 1.88 (m, 1 H) 2.06 (m, 2 H) 2.20 (m, 1 H) 3.30 (dd, *J=*18.37*,* 5.86 Hz, 1 H) 3.37 (m, 1 H) 3.40 (s, 3 H) 3.59 (m, 2 H) 3.71 (m, 3 H) 5.82 (dd, *J=*12.50*,* 5.86 Hz, 1 H) 7.05 (m, 3 H) 7.19 (d, *J*=8.60 Hz, 2 H) 7.24 (m, 2 H) | 481 |
| 14 | 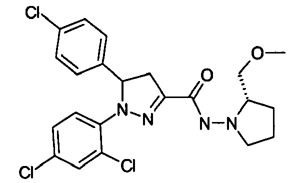 | 5-(4-Chloro-phenyl)-1-(2,4-dichloro-phenyl)-4,5-dihydro-1H-pyrazole-3-carboxylic acid (2-methoxymethyl-pyrrolidin-1-yl)-amide | 1 H NMR (400 MHz, CHLOROFORM-*d*) δ ppm 1.79 (m, 1H) 1.98 (m, 2H) 2.13 (m, 1 H) 3.32 (dd, *J*=18.76, 6.25 Hz, 1 H) 3.36 (m, 1 H) 3.38 (s, 3 H) 3.51 (m, 2 H) 3.64 (m, 2 H) 3.70 (dd, *J*=18.76, 12.31 Hz, 1 H) 5.77 (dd, *J*=12.31, 6.25 Hz, 1 H) 7.07 (td, *J*=8.99, 2.34 Hz, 3 H) 7.18 (d, *J*=8.21 Hz, 2 H) 7.24 (m, 2 H) | 481 |
| 15 | 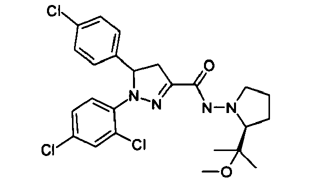 | 5-(4-Chloro-phenyl)-1-(2,4-dichloro-phenyl)-4,5-dihydro-1H-pyrazole-3-carboxylic acid [2-(1-methoxy-1-methyl-ethyl)-pyrrolidin-1-yl]-amide | 1 H NMR (300 MHz, DMSO-*d*₆) δ ppm 1.07 (m, 6 H) 1.71 (m, 3 H) 1.82 (d, *J*=7.03 Hz, 2 H) 2.89 (m, 1 H) 3.01 (m, 1 H) 3.07 (m, 3 H) 3.18 (m, 1 H) 3.64 (dd, *J*=18.02, 11.86 Hz, 1 H) 5.79(m, 1H) 7.12 (m, 2H) 7.28 (m, 3 H) 7.46 (m, 2 H) 9.33 (s, 1 H) | 509 |
| 16 | 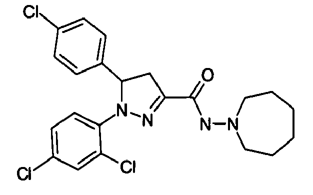 | 5-(4-Chloro-phenyl)-1-(2,4-dichloro-phenyl)-4,5-dihydro-1H-pyrazole-3-carboxylic acid azepan-1-ylamide | 1H NMR (300 MHz, CHLOROFORM-*d*) δ ppm 1.63 (m, 4 H) 1.80 (m, 4 H) 3.22 (dd, *J*=18.31, 6.15 Hz, 1 H) 3.30 (m, 4 H) 3.61 (dd, *J*=18.31, 12.30 Hz, 1 H) 5.70 (dd, *J*=12.30, 6.15 Hz, 1 H) 6.99(m, 3H) 7.10 (m, 3H) 7.19 (m, 1H) | 465 |
| 17 | 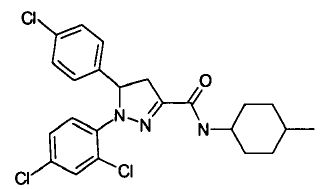 | 5-(4-Chloro-phenyl)-1-(2,4-dichloro-phenyl)-4,5-dihydro-1H-pyrazole-3-carboxylic acid (4-methyl-cyclohexyl)-amide | | 464 |
| 18 | 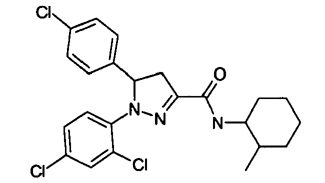 | 5-(4-Chloro-phenyl)-1-(2,4-dichloro-phenyl)-4,5-dihydro-1H-pyrazole-3-carboxylic acid (2-methyl-cyclohexyl)-amide | | 464 |
| 19 | 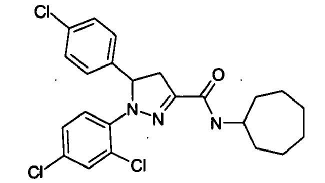 | 5-(4-Chloro-phenyl)-1-(2,4-dichloro-phenyl)-4,5-dihydro-1H-pyrazole-3-carboxylic acid cycloheptylamide | 1 H NMR (300 MHz, DMSO-*d*₆) δ ppm 1.53 (m, 10 H) 1.79 (m, 2 H) 3.05 (dd, *J*=18.16, 5.71 Hz, 1H) 3.64 (dd, *J*=18.16, 11.94 Hz, 1 H) 3.85 (m, 1 H) 5.80 (dd, *J*=11.94, 5.71 Hz, 1H) 7.13 (m, 2 H) 7.27 (m, 2 H) 7.43 (d, *J*=2.34 Hz,1 H) 7.51 (d, *J*=8.79 Hz, 1 H) 8.10 (d, *J*=8.35 Hz, 1 H) | 464 |
| 20 | 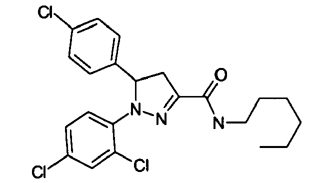 | 5-(4-Chloro-phenyl)-1-(2,4-dichloro-phenyl)-4,5-dihydro-1H-pyrazole-3-carboxylic acid hexylamide | 1 H NMR (300 MHz, DMSO-*d*₆) *δ* ppm 0.85 (m, 3 H) 1.25 (m, 6 H) 1.47 (m, 2 H) 3.05 (dd, *J*=18.16, 5.79 Hz, 1 H) 3.17 (m, 2 H) 3.65 (dd, *J*=18.16, 11.86 Hz, 1 H) 5.81 (dd, *J*=11.86, 5.79 Hz, 1 H) 7.14 (d, *J*=8.50 Hz, 2 H) 7.27 (m, 3 H) 7.45 (m, 2 H) 8.32 (t, *J*=5.86 Hz, 1 H) | 452 |
| 21 | 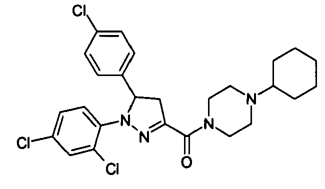 | [5-(4-Chloro-phenyl)-1-(2,4-dichloro-phenyl)-4,5-dihydro-1H-pyrazol-3-yl]-(4-cyclohexyl-piperazin-1-yl)-methanone | | 519 |
| 22 | 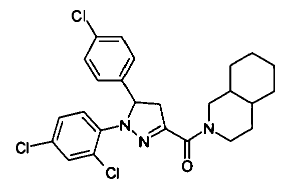 | [5-(4-Chloro-phenyl)-1-(2,4-dichloro-phenyl)-4,5-dihydro-1H-pyrazol-3-yl]-(octahydro-isoquinolin-2-yl)-methanone | | 490 |
| 23 | 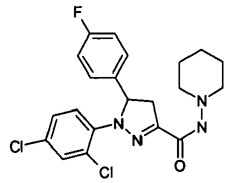 | 1-(2,4-Dichloro-phenyl)-5-(4-fluoro-phenyl)-4,5-dihydro-1H-pyrazole-3-carboxylic acid piperidin-1-ylamide | 1H NMR (300 MHz, CHLOROFORM-*d*) δ ppm 1.48 (s, 2H) 1.84 (s, 4 H) 3.25 (s, 4 H ) 3.28 (dd, *J*=18.16, 6.15 Hz, 1 H) 3.61 (dd, *J*=18.16, 12.38 Hz, 1 H) 5.73 (dd, *J*=12.38, 6.15 Hz, 1 H) 6.83 (t, *J*=8.57 Hz, 2 H) 7.02 (m, 4 H) 7.18 (m, 1 H) 8.21 (br, 1H) | 435 |
| 24 | 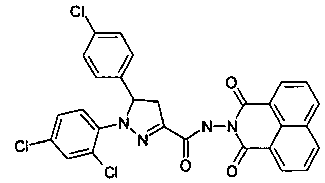 | 5-(4-Chloro-phenyl)-1-(2,4-dichloro-phenyl)-4,5-dihydro-1H-pyrazole-3-carboxylicacid (1,3-dioxo-1H,3H-benzo[de]isoquinolin-2-yl)-amide | 1 H NMR (300 MHz, DMSO-*d*₆) δ ppm 3.16 (dd, *J*=18.02, 6.45 Hz, 1 H) 3.79 (dd, *J*=18.02, 12.01 Hz, 1 H) 5.98 (dd, *J*=12.01, 6.45 Hz, 1 H) 7.24 (d, *J*=8.06 Hz, 2 H) 7.36 (m, 3 H) 7.55 (m, 2 H) 7.92 (m, 2 H) 8.55 (m, 4 H) 11.08 (s, 1 H) | 563 |
| 25 | 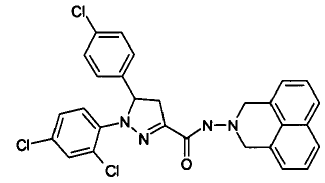 | 5-(4-Chloro-phenyl)-1-(2,4-dichloro-phenyl)-4,5-dihydro-1H-pyrazole-3-carboxylic acid (1H,3H-benzo[de]isoquinolin-2-yl)-amide | 1 H NMR (300 MHz, DMSO-*d*₆) *δ* ppm 3.13 (dd, *J*=18.09, 5.86 Hz, 1 H) 3.71 (dd, *J*=18.09, 11.86 Hz, 1 H) 4.35 (s, 4 H) 5.84 (dd, *J*=11.86, 5.86 Hz, 1 H) 7.19 (d, *J*=8.50 Hz, 2 H) 7.31 (m, 5 H) 7.47 (m, 4 H) 7.79 (d, *J*=8.20 Hz, 2 H) 9.77 (s, 1H) | 535 |
| 26 | 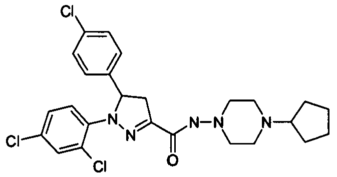 | 5-(4-Chloro-phenyl)-1-(2,4-dichloro-phenyl)-4,5-dihydro-1H-pyrazole-3-carboxylic acid (4-cyclopentyl-piperazin-1-yl)-amide | | 520 |
| 27 | 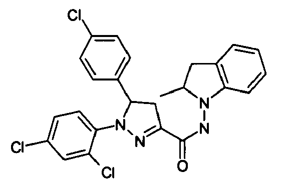 | 5-(4-Chloro-phenyl)-1-(2,4-dichloro-phenyl)-4,5-dihydro-1H-pyrazole-3-carboxylic acid (2-methyl-2,3-dihydro-indol-1-yl)-amide | | 499 |
| 28 | 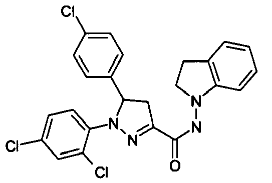 | 5-(4-Chloro-phenyl)-1-(2,4-dichloro-phenyl)-4,5-dihydro-1H-pyrazole-3-carboxylic acid (2,3-dihydro-indol-1-yl)-amide | 1 H NMR (300 MHz, DMSO-*d*₆) δ ppm 2.96 (t, *J*=8.20 Hz, 2 H) 3.11 (dd, *J*=18.02, 5.71 Hz, 1 H) 3.59 (t, *J*=8.20 Hz, 2 H) 3.73 (dd, *J*=18.02, 12.01 Hz, 1 H) 5.87 (dd, *J*=12.01, 5.71 Hz, 1 H) 6.58 (d, *J*=7.76 Hz, 1 H) 6.75 (t, *J*=7.32 Hz, 1 H) 7.10 (m, 4 H) 7.29 (m, 3 H) 7.45 (d, *J*=2.34 Hz, 1 H) 7.54 (d, *J*=8.79 Hz, 1 H) 10.20 (s, 1 H) | 485 |
| 29 | 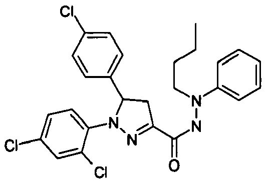 | 5-(4-Chloro-phenyl)-1-(2,4-dichloro-phenyl)-4,5-dihydro-1H-pyrazole-3-carboxylic acid N'-butyl-N'-phenyl-hydrazide | 1 H NMR (300 MHz, CHLOROFORM-*d*) δ ppm 0.98 (t, *J*=7.32 Hz, 3 H) 1.45 (m, 2 H) 1.69 (m, 2 H) 3.36 (dd, *J*=18.31, 6.84 Hz, 1 H) 3.57 (m, 2 H) 3.75 (dd, *J*=18.31, 12.21 Hz, 1 H) 5.77 (dd, *J*=12.21, 6.84 Hz, 1H) 6.90 (m, 3 H) 7.10 (m, 4 H) 7.26 (m, 5 H) 8.22 (s, 1 H) | 515 |
| 30 | 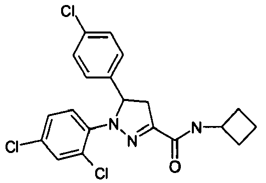 | 5-(4-Chloro-phenyl)-1-(2,4-dichloro-phenyl)-4,5-dihydro-1H-pyrazole-3-carboxylic acid cyclobutylamide | 1 H NMR (300 MHz, DMSO-*d*₆) *δ* ppm 1.61 (m, 2 H) 2.14 (m, 4 H) 3.05 (dd, *J*=18.16, 5.57 Hz, 1 H) 3.63 (dd, *J*=18.16, 11.94 Hz, 1 H) 4.33 (m, 1 H) 5.81 (dd, *J*=11.94, 5.57 Hz, 1 H) 7.13 (m, 2 H) 7.27 (m, 3 H) 7.43 (d, *J*=2.34 Hz, 1 H) 7.52 (d, *J*=8.79 Hz, 1 H) 8.53 (d, *J*=8.06 Hz, 1 H) | 422 |
| 31 | 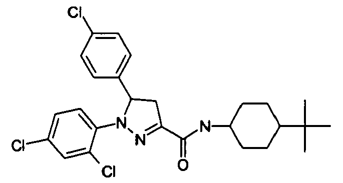 | 5-(4-Chloro-phenyl)-1-(2,4-dichloro-phenyl)-4,5-dihydro-1H-pyrazole-3-carboxylic acid (4-tert-butyl-cyclohexyl)-amide | | 506 |
| 32 | 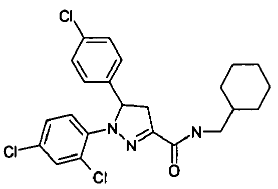 | 5-(4-Chloro-phenyl)-1-(2,4-dichloro-phenyl)-4,5-dihydro-1H-pyrazole-3-carboxylic acid cyclohexylmethyl-amide | 1 H NMR (400 MHz, CHLOROFORM-*d*) δ ppm 0.99 (m, 2 H) 1.21 (m, 4 H) 1.69 (m, 1 H) 1.76 (m, 4 H) 3.15 (dd, *J*=17.98, 6.06 Hz, 1 H) 3.32 (dt, *J*=12.60, 6.40 Hz, 2 H) 3.67 (dd, *J*=17.98, 12.11 Hz, 1 H) 5.73 (dd, *J*=12.11, 6.06 Hz, 1 H) 6.75 (t, *J*=6.06 Hz, 1 H) 7.06 (m, 3 H) 7.12 (m, 1 H) 7.17 (d, *J*=8.60 Hz, 2 H) 7.26 (m, 1 H) | 464 |
| 33 | 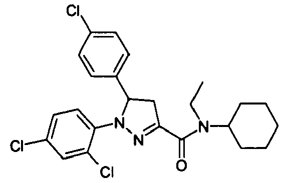 | 5-(4-Chloro-phenyl)-1-(2,4-dichloro-phenyl)-4,5-dihydro-1H-pyrazole-3-carboxylic acid cyclohexyl-ethyl-amide | | 478 |
| 34 | 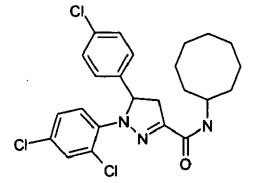 | 5-(4-Chloro-phenyl)-1-(2,4-dichloro-phenyl)-4,5-dihydro-1H-pyrazole-3-carboxylic acid cyclooctylamide | 1 H NMR (400 MHz, CHLOROFORM-d) δ ppm 1.59 (m, 12 H) 1.91 (m, 2 H) 3.32 (dd, *J*=18.37, 5.86 Hz, 1 H) 3.66 (dd, *J*=18.37; 12.11 Hz, 1 H) 4.10 (ddd, *J*=8.60, 4.01, 3.71 Hz, 1 H) 5.72 (dd, *J*=12.11, 5.86 Hz, 1 H) 6.65 (d, *J*=8.60 Hz, 1 H) 7.07 (m, 3 H) 7.15 (m, 3 H) 7.25 (m, 1 H) | 478 |
| 35 | 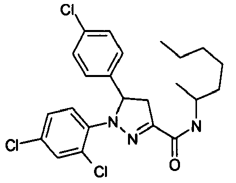 | 5-(4-Chloro-phenyl)-1-(2,4-dichloro-phenyl)-4,5-dihydro-1H-pyrazole-3-carboxylic acid (1-methyl-hexyl)-am ide | 1 H NMR (400 MHz, CHLOROFORM-*d*) δ ppm 0.89 (m, *J*=6.94 Hz, 3 H) 1.21 (m, *J*=6.64 Hz, 3 H) 1.34 (m, 6 H) 1.50 (m, 2 H) 3.32 (ddd, *J*=18.37, 6.06, 1.76 Hz, 1 H) 3.67 (ddd, *J*=18.37, 12.02, 3.32 Hz, 1 H) 4.08 (m, 1 H) 5.73 (dt, *J*=12.02, 6.06 Hz, 1 H) 6.48 (dd, *J*=8.01, 5.67 Hz, 1 H) 7.08 (m, 3 H) 7.11 (m, 1H) 7.16 (m, 2 H) 7.25 | 466 |
| 36 | 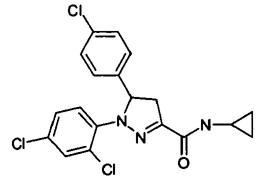 | 5-(4-Chloro-phenyl)-1-(2,4-dichloro-phenyl)-4,5-dihydro-1H-pyrazole-3-carboxylic acid cyclopropylamide | 1 H NMR (300 MHz, DMSO-*d*₆) *δ* ppm 0.63 (m, 4 H) 2.77 (m, 1 H) 3.06 (dd, *J*=18.02, 5.57 Hz, 1 H) 3.64 (dd, *J*=18.02, 11.86 Hz, 1 H) 5.81 (dd, J*=*11.86, 5.57 Hz, 1 H) 7.13 (d, *J*=8.50 Hz, 2 H) 7.26 (m, 3 H) 7.42 (d, *J*=2.34 Hz, 1 H) 7.49 (d, *J*=8.64 Hz, 1 H) 8.36 (d, *J*=4.39 Hz, 1 H) | 408 |
| 37 | 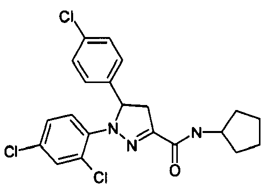 | 5-(4-Chloro-phenyl)-1-(2,4-dichloro-phenyl)-4,5-dihydro-1H-pyrazole-3-carboxylic acid cyclopentylamide | 1 H NMR (400 MHz, CHLOROFORM-*d*) δ ppm 1.44 - 1.75 (m, 6 H) 2.06 (td, *J*=13.09, 5.86 Hz, 2 H) 3.32 (dd, *J*=18.37, 6.25 Hz, 1 H) 3.66 (dd, *J*=18.37, 12.11 Hz, 1H) 4.31 (m, 1 H) 5.73 (dd, *J*=12.11, 6.25 Hz, 1 H) 6.62 (d, *J*=7.82 Hz, 1 H) 7.06 (m, 3 H) 7.12 (s, 1 H) 7.17 (m, 2 H) 7.25 (m, 1H) | 436 |
| 38 | 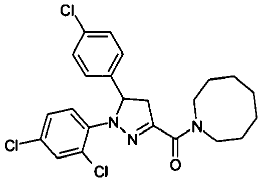 | Azocan-1-yl-[5-(4-chloro-phenyl)-1-(2,4-dichloro-phenyl)-4,5-dihydro-1H-pyrazol-3-yl]-methanone | 1 H NMR (400 MHz, CHLOROFORM-*d*) δ ppm 1.61 (m, 6 H) 1.82 (m, 4 H) 3.37 (dd, *J*=17.98, 5.86 Hz, 1 H) 3.62 (m, 2 H) 3.72 (dd, *J*=17.98, 11.72 Hz, 1 H) 3.87 (m, 2 H) 5.65 (dd, *J*=11.72, 5.86 Hz, 1 H) 7.06 (m, 4 H) 7.17 (m, 2 H) 7.25 (m, 1H) | 464 |
| 39 | 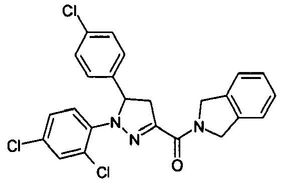 | [5-(4-Chloro-phenyl)-1-(2,4-dichloro-phenyl)-4,5-dihydro-1H-pyrazol-3-yl]-(1,3-dihydro-isoindol-2-yl)-methanone | 1 H NMR (400 MHz, CHLOROFORM-*d*) δ ppm 3.44 (dd, *J*=17.98, 6.25 Hz, 1 H) 3.78 (dd, *J*=17.98, 12.11 Hz, 1 H) 4.98 (d, *J*=3.13 Hz, 2 H) 5.24 (d, *J*=16.02 Hz, 1 H) 5.32 (d, *J*=16.02 Hz, 1 H) 5.70 (dd, *J*=12.11, 6.25Hz, 1H) 7.10(m, 3 H) 7.17 (m, 3 H) 7.32 (m, 5 H) | 470 |
| 40 | 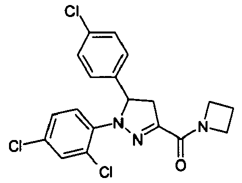 | Azetidin-1-yl-[5-(4-chloro-phenyl)-1-(2,4-dichloro-phenyl)-4,5-dihydro-1H-pyrazol-3-yl]-methanone | 1 H NMR (300 MHz, DMSO-*d₆*) δ ppm 2.26 (m, *J*=7.58, 2H) 3.01 (dd, *J*=18.02, 6.30 Hz, 1 H) 3.64 (dd, *J*=18.02, 12.01 Hz, 1 H) 3.99 (ddd, *J*=7.43, 4.10, 3.85 Hz, 2 H) 4.38 (q, *J*=7.67 Hz, 1 H) 4.53 (m, 1 H) 5.77 (dd, *J*=12.01, 6.30 Hz, 1 H) 7.17 (m, 2H) 7.27 (m, 4 H) 7.46 (d, *J*=2.20 Hz, 1H) | 408 |
| 41 | 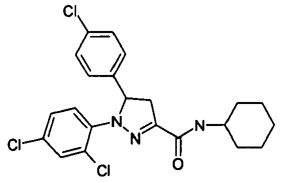 | 5-(4-Chloro-phenyl)-1-(2,4-dichloro-phenyl)-4,5-dihydro-1H-pyrazole-3-carboxylic acid cyclohexylamide | | 450 |
| 42 | 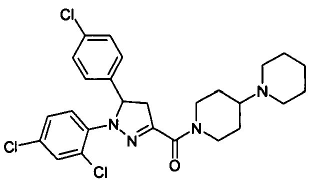 | [1,4']Bipiperidinyl-1'-yl-[5-(4-chl oro-phenyl)-1-(2,4-dichloro-phenyl) -4,5-dihydro-1H-pyrazol-3-yl]-metha none | | 519 |
| 43 | 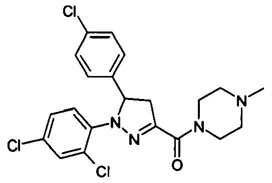 | [5-(4-Chloro-phenyl)-1-(2,4-dichloro-phenyl)-4,5-dihydro-1H-pyrazol-3-yl]-(4-methyl-piperazin-1-yl)-methanone | 1 H NMR (300 MHz, DMSO-*d*₆) δ ppm 2.18 (s, 3 H) 2.35 (m, 4 H) 3.11 (dd, *J*=17.80, 6.96 Hz, 1 H) 3.52 (m, 1 H) 3.61 (m, 1H) 3.70 (dd, *J*=17.80, 11.64 Hz, 1 H) 3.87 (m, 2 H) 5.71 (dd, *J*=11.64, 6.96 Hz, 1 H) 7.22 (m, 4 H) 7.31 (m, 2 H) 7.48 (d, *J*=1.61 Hz, 1H) | 451 |
| 44 | 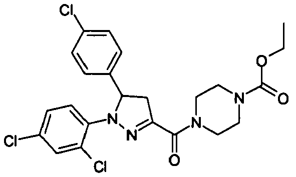 | 4-[5-(4-Chloro-phenyl)-1-(2,4-dichloro-phenyl)-4,5-dihydro-1H-pyrazole -3-carbonyl]-piperazine-1-carboxylic acid ethyl ester | | 509 |
| 45 | 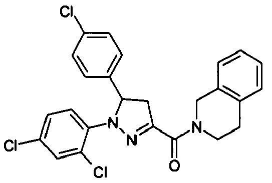 | [5-(4-Chloro-phenyl)-1-(2,4-dichloro-phenyl)-4,5-dihydro-1H-pyrazol-3-yl]-(3,4-dihydro-1H-isoquinolin-2-yl)-methanone | | 484 |
| 46 | 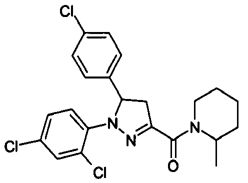 | [5-(4-Chloro-phenyl)-1-(2,4-dichloro-phenyl)-4,5-dihydro-1H-pyrazol-3-yl]-(2-methyl-piperidin-1-yl)-methanone | | 450 |
| 47 | 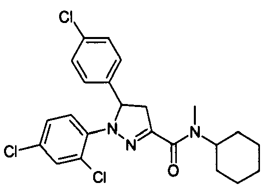 | 5-(4-Chloro-phenyl)-1-(2,4-dichloro-phenyl)-4,5-dihydro-1H-pyrazole-3-carboxylic acid cyclohexyl-methyl-amide | 1 H NMR (300 MHz, DMSO-*d*₆) δ ppm 1.22 (m, 4 H) 1.57 (m, 3 H) 1.73 (m, 3 H) 2.97 (s, 3 H) 3.10 (dd, *J*=17.80, 7.03 Hz, 1 H) 3.71 (dd, *J*=17.80, 11.64 Hz, 1 H) 4.28 (m, 1H) 5.71 (m, 1 H) 7.22 (m, 4 H) 7.30 (m, 2H) 7.48 (m, 1 H) | 464 |
| 48 | 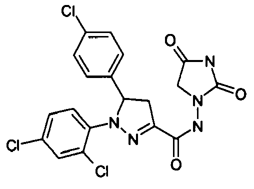 | 5-(4-Chloro-phenyl)-1-(2,4-dichloro-phenyl)-4,5-dihydro-1H-pyrazole-3-carboxylic acid (2,4-dioxo-imidazolidin-1-yl)-amide | | 466 |
| 49 | 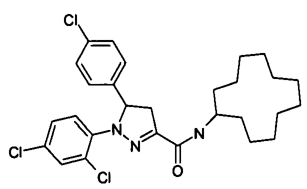 | 5-(4-Chloro-phenyl)-1-(2,4-dichloro-phenyl)-4,5-dihydro-1H-pyrazole-3-carboxylic acid cyclododecylamide | 1 H NMR (300 MHz, DMSO-*d*₆) δ ppm 1.29 (m, 20 H) 1.63 (m, 2 H) 3.06 (dd, *J*=18.09, 5.93 Hz, 1 H) 3.65 (dd, *J*=18.09, 11.86 Hz, 1 H) 4.04 (m, 1 H) 5.81 (dd, *J=*11.86*,* 5.93 Hz, 1 H) 7.14 (m, 2 H) 7.28 (m, 3 H) 7.43 (d, *J*=2.34 Hz, 1 H) 7.50 (d, *J*=8.79 Hz, 1 H) 8.00 (d, *J*=8.79 Hz, 1 H) | 534 |
| 50 | 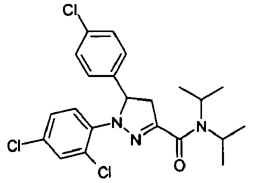 | 5-(4-Chloro-phenyl)-1-(2,4-dichloro-phenyl)-4,5-dihydro-1H-pyrazole-3-carboxylic acid diisopropylamide | 1 H NMR (300 MHz, DMSO-*d*₆) δ ppm 1.20 (m, 6 H) 1.36 (m, 6 H) 3.09 (dd, *J*=17.65, 6.66 Hz, 1 H) 3.60 (br, 1 H) 3.67 (dd, *J*=17.65, 11.43 Hz, 1 H) 4.52 (br, 1 H) 5.69 (dd, *J*=11.43, 6.66 Hz, 1 H) 7.20 (m, 4 H) 7.32 (m, 2 H) 7.46 (d, *J*=2.05 Hz, 1 H) | 452 |
| 51 | 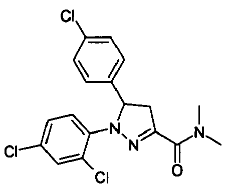 | 5-(4-Chloro-phenyl)-1-(2,4-dichloro-phenyl)-4,5-dihydro-1H-pyrazole-3-carboxylic acid dimethylamide | | 396 |
| 52 | 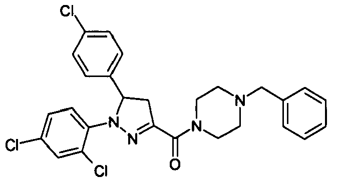 | (4-Benzyl-piperazin-1-yl)-[5-(4-chloro-phenyl)-1-(2,4-dichloro-phenyl)-4,5-dihydro-1H-pyrazol-3-yl]-methanone | | 527 |
| 53 | 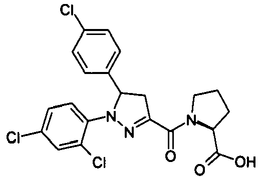 | 1-[5-(4-Chloro-phenyl)-1-(2,4-dichloro-phenyl)-4,5-dihydro-1H-pyrazole-3-carbonyl]-pyrrolidine-2-carboxylic acid | | 466 |
| 54 | 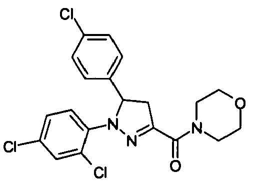 | [5-(4-Chloro-phenyl)-1-(2,4-dichloro-phenyl)-4,5-dihydro-1H-pyrazol-3-yl]-morpholin-4-yl-methanone | 1 H NMR (300 MHz, DMSO-*d*₆) δ ppm 3.12 (dd, *J*=17.87, 6.37 Hz, 1 H) 3.62 (m, 6 H) 3.72 (dd, *J*=17.87, 11.06 Hz, 1 H) 3.93 (m, 2 H) 5.71 (dd, *J*=11.06, 6.37 Hz, 1 H) 7.22 (m, 4 H) 7.31 (m, 2 H) 7.48 (s, 1 H) | 438 |
| 55 | 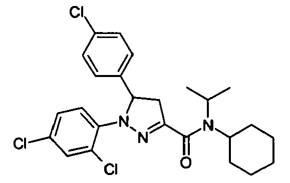 | 5-(4-Chloro-phenyl)-1-(2,4-dichloro-phenyl)-4,5-dihydro-1H-pyrazole-3-carboxylic acid cyclohexyl-isopropyl-amide | | 492 |
| 56 | 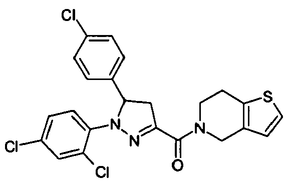 | [5-(4-Chloro-phenyl)-1-(2,4-dichloro-phenyl)-4,5-dihydro-1H-pyrazol-3-yl]-(6,7-dihydro-4H-thieno[3,2-c]pyridin-5-yl)-methanone | | 490 |
| 57 | 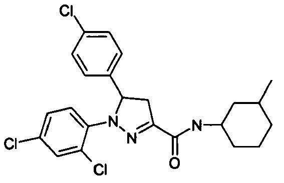 | 5-(4-Chloro-phenyl)-1-(2,4-dichloro-phenyl)-4,5-dihydro-1H-pyrazole-3-carboxylic acid (3-methyl-cyclohexyl)-amide | | 464 |
| 58 | 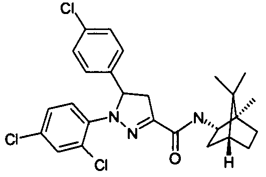 | 5-(4-Chloro-phenyl)-1-(2,4-dichloro-phenyl)-4,5-dihydro-1H-pyrazole-3-carboxylic acid (1,7,7-trimethyl-bicyclo[2.2.1]hept-2-yl)-amide | | 504 |
| 59 | 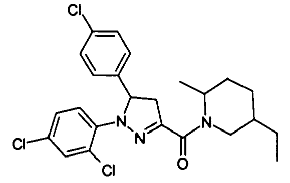 | [5-(4-Chloro-phenyl)-1-(2,4-dichloro-phenyl)-4,5-dihydro-1H-pyrazol-3-yl]-(5-ethyl-2-methyl-piperidin-1-yl)-methanone | | 478 |
| 60 | 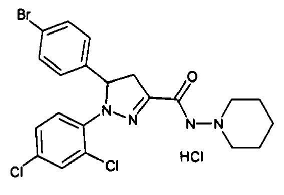 | 5-(4-Bromo-phenyl)-1-(2,4-dichloro-phenyl)-4,5-dihydro-1H-pyrazole-3-carboxylic acid piperidin-1-ylamide hydrochloride | 1 H NMR (300 MHz, METHANOL-*d*₄) δ ppm 1.68 (m, 2 H) 1.92 - 2.03 (m, 4 H) 3.24 (dd, *J*=18.02, 6.52 Hz, 1 H) 3.49 (br. s., 4 H) 3.76 (dd, *J*=18.02, 12.52 Hz, 1 H) 5.95 (dd, *J*=12.52, 6.52 Hz, 1 H) 7.14 (d, J=8.50 Hz, 2 H) 7.21 (dd, *J*=8.72, 2.42 Hz, 1 H) 7.40 (m, 3 H) 7.37 (d, *J*=2.42 Hz, 1 H) | 495 |
| 61 | 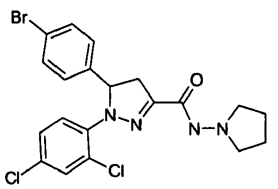 | 5-(4-Bromo-phenyl)-1-(2,4-dichloro-phenyl)-4,5-dihydro-1H-pyrazole-3-carboxylic acid pyrrolidin-1-ylamide | | 482 |
| 62 | 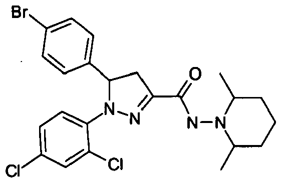 | 5-(4-Bromo-phenyl)-1-(2,4-dichloro-phenyl)-4,5-dihydro-1H-pyrazole-3-carboxylic acid (2,6-dimethyl-piperidin-1-yl)-amide | | 524 |
| 63 | 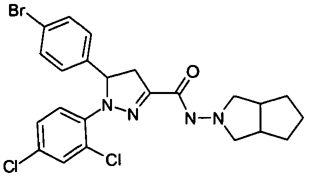 | 5-(4-Bromo-phenyl)-1-(2,4-dichloro-phenyl)-4,5-dihydro-1H-pyrazole-3-carboxylic acid (hexahydro-cyclopenta[c]pyrrol-2-yl)-amide | 1 H NMR (300 MHz, CHLOROFORM-*d*) δ ppm 1.54- 1.85 (m, 6 H) 2.91 (br. s., 2 H) 3.25 (dd, *J*=18.16, 6.08 Hz, 1 H) 3.29 (br. s., 2H) 3.68 (dd, *J*=18.16, 12.52 Hz, 1 H) 3.79 (br. s., 2 H) 5.81 (dd, *J*=12.52, 6.08 Hz, 1 H) 6.98 (d, *J*=8.50 Hz, 2 H) 7.09 (dd, *J=8.64,* 2.34 Hz, 1 H) 7.20 - 7.25 (m, 2 H) 7.34 (d, *J=8.35* Hz, 2 H) 9.04 (br. s., | 522 |
| 64 | 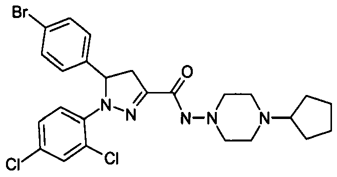 | 5-(4-Bromo-phenyl)-1-(2,4-dichloro-phenyl)-4,5-dihydro-1H-pyrazole-3-carboxylic acid (4-cyclopentyl-piperazin-1-yl)-amide | | 565 |
| 65 | 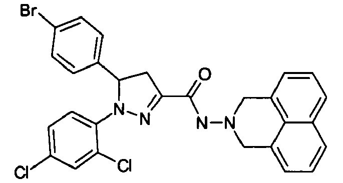 | 5-(4-Bromo-phenyl)-1-(2,4-dichloro-phenyl)-4,5-dihydro-1H-pyrazole-3-carboxylic acid (1H,3H-benzo[de]isoquinolin-2-yl)-amide | | 580 |
| 66 | 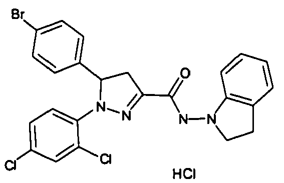 | 5-(4-Bromo-phenyl)-1-(2,4-dichloro-phenyl)-4,5-dihydro-1H-pyrazole-3-carboxylic acid (2,3-dihydro-indol-1 | 1H NMR (300 MHz, METHANOL-*d*₄) δ ppm 3.05 (t, *J* =7.98 Hz, 2 H) 3.28 (dd, *J=*18.09*,* 5.86 Hz, 1 H) 3.64 (t, *J=*8.50 Hz, 2 H) 3.77 (dd, *J=*18.09*,* 12.16 Hz, 1 H) 5.93 (dd, *J*=12.16, 5.86 Hz, 1 H) 6.71 (d, *J*=7.76 Hz, 1 H) 6.84 (t, *J*=7.40 Hz, 1 H) 7.07 - 7.21 (m, 5 H) 7.31 (d, *J*=2.34 Hz, 1 H) 7.44 (d, *J*=8.64 Hz, 1 H) 7.40 (d, | 529 |
| 67 | 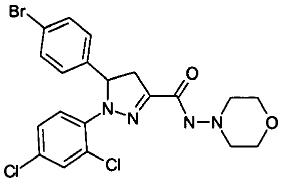 | 5-(4-Bromo-phenyl)-1-(2,4-dichloro-phenyl)-4,5-dihydro-1H-pyrazole-3-carboxylic acid morpholin-4-ylamide | | 498 |
| 68 | 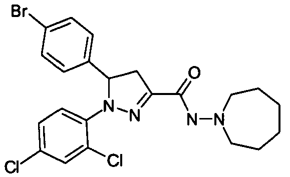 | 5-(4-Bromo-phenyl)-1-(2,4-dichloro-phenyl)-4,5-dihydro-1H-pyrazole-3-carboxylic acid azepan-1-ylamide | 1 H NMR (300 MHz, CHLOROFORM-*d*) δ ppm 1.75 (br. s., 4 H) 2.00 (br. s., 4 H) 3.26 (dd, *J*=18.16, 6.15 Hz, 1 H) 3.71 (dd, *J*=18.16, 12.45 Hz, 1 H) 3.65 (br. s., 4 H) 5.82 (dd, *J*=12.45, 6.15 Hz, 1 H) 6.97 (d, *J*=8.35 Hz, 2 H) 7.10 (d, *J*=2.20 Hz, 1 H) 7.16 - 7.22 (m, 1 H) 7.24 (d, *J*=2.20 Hz, 1 H) 7.35 (d, *J*=8.50 Hz, 2 H) | 510 |
| 69 | 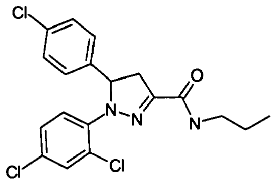 | 5-(4-Chloro-phenyl)-1-(2,4-dichloro-phenyl)-4,5-dihydro-1H-pyrazole-3-carboxylic acid propylamide | | 410 |
| 70 | 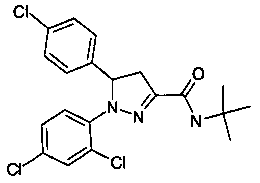 | 5-(4-Chloro-phenyl)-1-(2,4-dichloro-phenyl)-4,5-dihydro-1H-pyrazole-3-carboxylic acid tert-butylamide | | 424 |
| 71 | 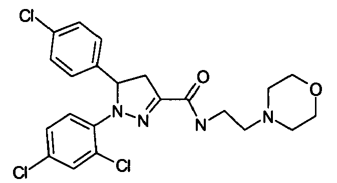 | 5-(4-Chloro-phenyl)-1-(2,4-dichloro-phenyl)-4,5-dihydro-1H-pyrazole-3-carboxylic acid (2-morpholin-4-yl-ethyl)-amide | | 481 |
| 72 | 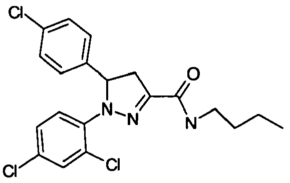 | 5-(4-Chloro-phenyl)-1-(2,4-dichloro-phenyl)-4,5-dihydro-1H-pyrazole-3-carboxylic acid butylamide | | 424 |
| 73 | 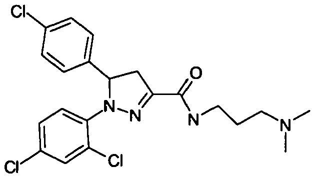 | 5-(4-Chloro-phenyl)-1-(2,4-dichloro-phenyl)-4,5-dihydro-1H-pyrazole-3-carboxylic acid (3-dimethylamino-pr opyl)-amide | | 453 |
| 74 | 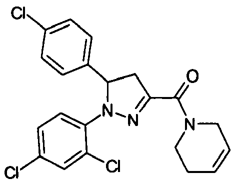 | [5-(4-Chloro-phenyl)-1-(2,4-dichloro-phenyl)-4,5-dihydro-1 H-pyrazol-3-y)]-(3,6-dihydro-2H-pyridin-1-yl)-methanone | | 434 |
| 75 | 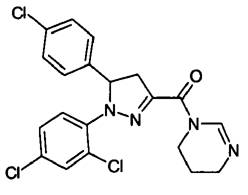 | [5-(4-Chloro-phenyl)-1-(2,4-dichloro-phenyl)-4,5-dihydro-1H-pyrazol-3-yl]-(5,6-dihydro-4H-pyrimidin-1-yl)-methanone | | 435 |
| 76 | 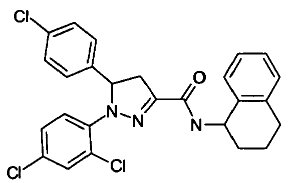 | 5-(4-Chloro-phenyl)-1-(2,4-dichloro-phenyl)-4,5-dihydro-1H-pyrazole-3-carboxylic acid (1,2,3,4-tetrahydro-naphthalen-1-yl)-amide | | 498 |
| 77 | 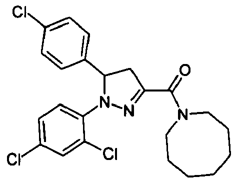 | Azocan-1-yl-[5-(4-chloro-phenyl)-1-(2,4-dichloro-phenyl)-4,5-dihydro-1H-pyrazol-3-yl]-methanone | | 464 |
| 78 | 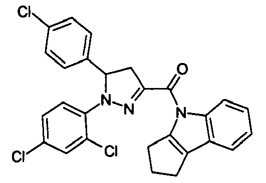 | [5-(4-Chloro-phenyl)-1-(2 ,4-dichloro-phenyl)-4,5-dihydro-1H-pyrazol-3-yl]-(2,3-dihydro-1H-cyclopenta[b]indol-4-yl)-methanone | | 508 |
| 79 | 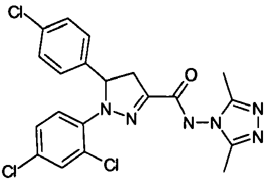 | 5-(4-Chloro-phenyl)-1-(2,4-dichloro-phenyl)-4,5-dihydro-1H-pyrazole-3-carboxylic acid (3,5-dimethyl-[1,2, 4]triazol-4-yl)-amide | | 463 |
| 80 | 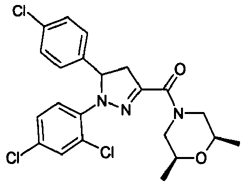 | [5-(4-Chloro-phenyl)-1-(2,4-dichloro-phenyl)-4,5-dihydro-1H-pyrazol-3-yl]-(2,6-dimethyl-morpholin-4-yl)-methanone | | 466 |
| 81 | 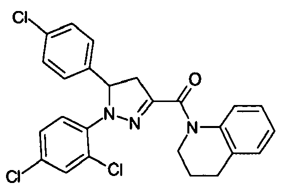 | [5-(4-Chloro-phenyl)-1-(2,4-dichloro-phenyl)-4,5-dihydro-1H-pyrazol-3-yl]-(3,4-dihydro-2H-quinolin-1-yl)-methanone | | 484 |
| 82 | 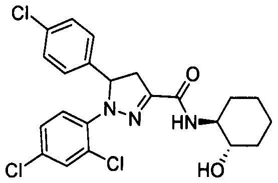 | 5-(4-chlorophenyl)-1-(2,4-dichlorophenyl)-N-((1R,2R)-2-hydroxycyclohexyl)-4,5-dihydro-1H-pyrazole-3-carboxamide | 1 H NMR (300 MHz, METHANOL-*d*₄) δ ppm 1.36 (m, 4 H) 1.75 (m, 2 H) 2.02 (m, 2 H) 3.24 (dd, *J*=5.71, 18.08 Hz, 1 H) 3.50 (m, 1H) 3.70 (dd, *J*=18.10, 12.01 Hz, 1 H) 3.74 (m, 1 H) 5.91 (dd, *J*=12.01, 5.71 Hz, 1 H) 7.12 - 7.32 (m, 6 H) 7.45 (t, *J*=8.28 Hz, 1 H) | 466 |
| 83 | 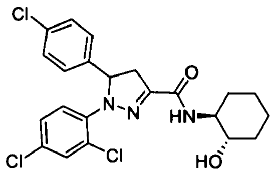 | 5-(4-chlorophenyl)-1-(2,4-dichlorophenyl)-N-((1S,2S)-2-hydroxycyclohexyl)-4,5-dihydro-1H-pyrazole-3-carboxamide | 1 H NMR (300 MHz, METHANOL-*d*₄) δ ppm 1.36 (m, 4 H) 1.75 (m, 2 H) 2.02 (m, 2 H) 3.24 (dd, *J*=5.71, 18.08 Hz, 1 H) 3.50 (m, 1 H) 3.70 (dd, *J*=18.10, 12.01 Hz, 1 H) 3.74 (m, 1 H) 5.91 (dd, *J*=12.01, 5.71 Hz, 1 H) 7.12 - 7.32 (m, 6 H) 7.45 (t, *J*=8.28 Hz, 1 H) | 466 |
| 84 | 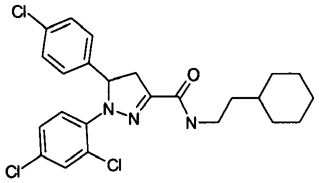 | 5-(4-Chloro-phenyl)-1-(2,4-dichloro-phenyl)-4,5-dihydro-1H-pyrazole-3-carboxylic acid (2-cyclohexyl-ethyl)-amide | | 478 |
| 85 | 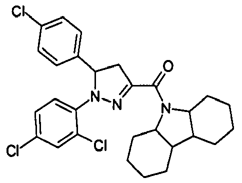 | [5-(4-Chloro-phenyl)-1-(2,4-dichloro-phenyl)-4,5-dihydro-1H-pyrazol-3-yl]-(dodecahydro-carbazol-9-yl)-methanone | | 530 |
| 86 | 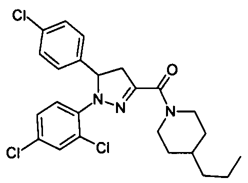 | [5-(4-Chloro-phenyl)-1-(2,4-dichloro-phenyl)-4,5-dihydro-1H-pyrazol-3-yl]-(4-propyl-piperidin-1-yl)-methanone | | 478 |
| 87 | 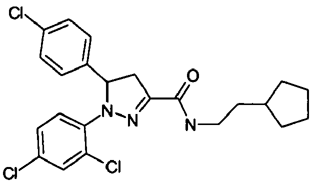 | 5-(4-Chloro-phenyl)-1-(2,4-dichloro-phenyl)-4,5-dihydro-1H-pyrazole-3-carboxylic acid (2-cyclopentyl-ethyl)-amide | | 464 |
| 88 | 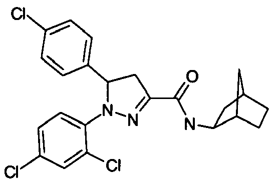 | 5-(4-Chloro-phenyl)-1-(2,4-dichloro-phenyl)-4,5-dihydro-1H-pyrazole-3-carboxylic acid bicyclo[2.2.1]hept-2-ylamide | | 462 |
| 89 | 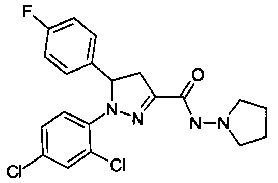 | 1-(2,4-Dichloro-phenyl)-5-(4-fluoro-phenyl)-4,5-dihydro-1H-pyrazole-3-carboxylic acid pyrrolidin-1-ylamide | | 421 |
| 90 | 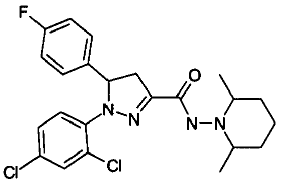 | 1-(2,4-Dichloro-phenyl)-5-(4-fluoro-phenyl)-4,5-dihydro-1H-pyrazole-3-carboxylic acid (2,6-dimethyl-piperidin-1-yl)-amide | | 463 |
| 91 | 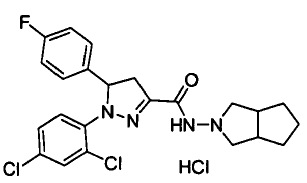 | 1-(2,4-Dichloro-phenyl)-5-(4-fluoro-phenyl)-4,5-dihydro-1H-pyrazole-3-carboxylic acid (hexahydro-cyclopen | 1 H NMR (300 MHz, DMSO-*d*₆) δ ppm 1.48 (d, *J=*8.20 Hz, 2 H) 1.52 - 1.68 (m, 4 H) 2.66 (br. s., 2 H) 2.87 (m, 2 H) 3.11 (dd, *J*=18.02, 6.30 Hz, 1 H) 3.54 (m, 2 H) 3.66 (dd, *J*=18.02, 12.01 Hz, 1 H) 5.85 (dd, *J*=12.01, 6.30 Hz, 1 H) 7.06 (t, *J*=8.79 Hz, 2 H) 7.16 - 7.24 (m, 2 H) 7.30 (dd, *J*=8.72, 2.27 Hz, 1 H) 7.47 (m, 2 H) 10.58 | 461 |
| 91A | 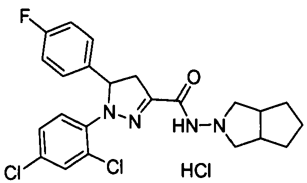 | 1-(2,4-Dichloro-phenyl)-5-(4-fluoro-phenyl)-4,5-dihydro-1H-pyrazole-3-carboxylic acid (hexahydro-cyclopen | | |
| 92 | 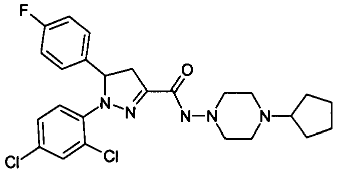 | 1-(2,4-Dichloro-phenyl)-5-(4-fluoro-phenyl)-4,5-dihydro-1H-pyrazole-3-carboxylic acid (4-cyclopentyl-pipe razin-1-yl)-amide | | 504 |
| 93 | 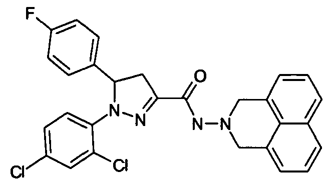 | 1-(2,4-Dichloro-phenyl)-5-(4-fluoro-phenyl)-4,5-dihydro-1H-pyrazole-3-carboxylic acid (1H,3H-benzo[de]iso quinolin-2-yl)-amide | | 519 |
| 94 | 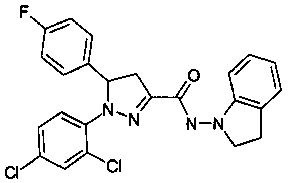 | 1-(2,4-Dichloro-phenyl)-5-(4-fluoro-phenyl)-4,5-dihydro-1H-pyrazole-3-carboxylic acid (2,3-dihydro-indol-1-yl)-amide | | 469 |
| 95 | 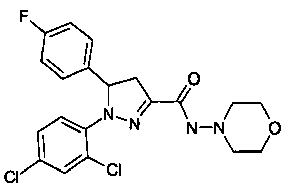 | 1-(2,4-Dichloro-phenyl)-5-(4-fluoro -phenyl)-4,5-dihydro-1 H-pyrazole-3-carboxylic acid morpholin-4-ylamide | | 437 |
| 96 | 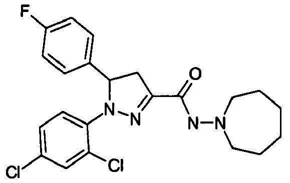 | 1-(2,4-Dichloro-phenyl)-5-(4-fluoro-phenyl)-4,5-dihydro-1H-pyrazole-3-carboxylic acid azepan-1-ylamide | 1 H NMR (300 MHz, CHLOROFORM-*d*) δ ppm 1.75 (br. s., 4 H) 2.00 (br. s., 4 H) 3.28 (dd, *J*=18.16, 6.08 Hz, 1 H) 3.68 (dd, *J*=18.16, 12.38 Hz, 1 H) 3.68 (br. s., 4 H) 5.84 (dd, | 449 |
| 97 | 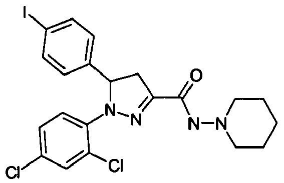 | 1-(2,4-Dichloro-phenyl)-5-(4-iodo-p henyl)-4,5-dihydro-1 H-pyrazole-3-ca rboxylic acid piperidin-1-ylamide | | 543 |
| 98 | 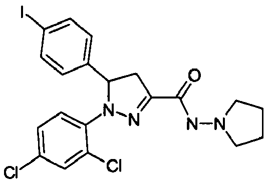 | 1-(2,4-Dichloro-phenyl)-5-(4-iodo-p henyl)-4,5-dihydro-1 H-pyrazole-3-ca rboxylic acid pyrrolidin-1-ylamide | | 529 |
| 99 | 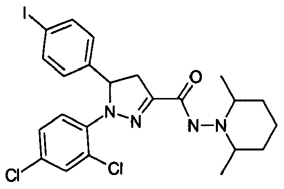 | 1-(2,4-Dichloro-phenyl)-5-(4-iodo-p henyl)-4,5-dihydro-1 H-pyrazole-3-ca rboxylic acid (2,6-dimethyl-piperid in-1-yl)-amide | | 571 |
| 100 | 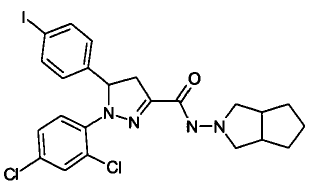 | 1-(2,4-Dichloro-phenyl)-5-(4-iodo-p henyl)-4,5-dihydro-1H-pyrazole-3-ca rboxylic acid (hexahydro-cyclopenta [c]pyrrol-2-yl)-amide | | 569 |
| 101 | 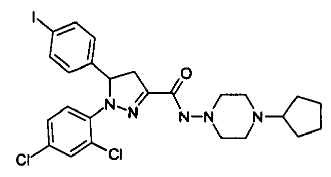 | 1-(2,4-Dichloro-phenyl)-5-(4-iodo-p henyl)-4,5-dihydro-1 H-pyrazole-3-ca rboxylic acid (4-cyclopentyl-pipera zin-1-yl)-amide | | 612 |
| 102 | 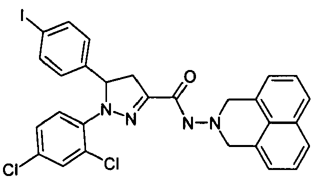 | 1-(2,4-Dichloro-phenyl)-5-(4-iodo-p henyl)-4,5-dihydro-1H-pyrazole-3-ca rboxylic acid (1 H,3H-benzo[de]isoqu inolin-2-yl)-amide | | 627 |
| 103 | 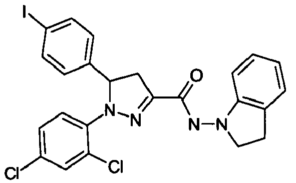 | 1-(2,4-Dichloro-phenyl)-5-(4-iodo-phenyl)-4,5-dihydro-1H-pyrazole-3-ca rboxylic acid (2,3-dihydro-indol-1-yl)-amide | | 577 |
| 104 | 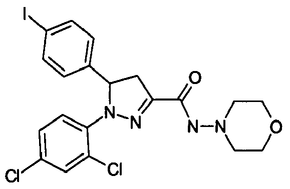 | 1-(2,4-Dichloro-phenyl)-5-(4-iodo-p henyl)-4,5-dihydro-1H-pyrazole-3-ca rboxylic acid morpholin-4-ylamide | | 545 |
| 105 | 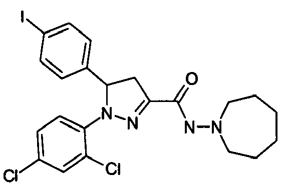 | 1-(2,4-Dichloro-phenyl)-5-(4-iodo-phenyl)-4,5-dihydro-1H-pyrazole-3-ca rboxylic acid azepan-1-ylamide | | 557 |
| 106 | 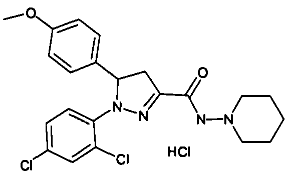 | 1-(2,4-Dichloro-phenyl)-5-(4-methoxy-phenyl)-4,5-dihydro-1H-pyrazole-3-carboxylic acid piperidin-1-ylamid | 1 H NMR (300 MHz, CHLOROFORM-*d*) δ ppm 1.67 (br. s., 2 H) 2.10 (br. s., 4 H) 3.25 (dd, *J*=18.02, 6.01 Hz, 1 H) 3.64 (dd, *J*=18.02, 12.60 Hz, 1 H) 3.72 (s, 3 H) 3.89 (br. s., 4 H) 5.85 (dd, *J*=12.60, 6.01 Hz, 1 H) 6.72 (d, *J*=8.79 Hz, 2 H) 7.00 (d, J=8.64 Hz, 2 H) 7.07 (dd, *J*=8.64, 2.20 Hz, 1 H) 7.22 (d, *J*=8.64 Hz, 1 H) 7.22 (d, | 447 |
| 107 | 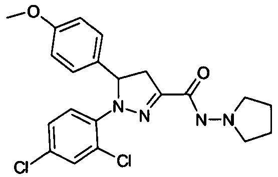 | 1-(2,4-Dichloro-phenyl)-5-(4-methox y-phenyl)-4,5-dihydro-1H-pyrazole-3 -carboxylic acid pyrrolidin-1-ylami de | | 433 |
| 108 | 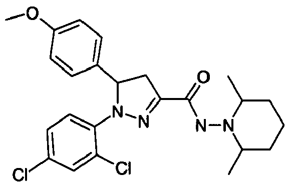 | 1-(2,4-Dichloro-phenyl)-5-(4-methox y-phenyl)-4,5-dihydro-1 H-pyrazole-3 -carboxylic acid (2,6-dimethyl-pipe ridin-1-yl)-amide | | 475 |
| 109 | 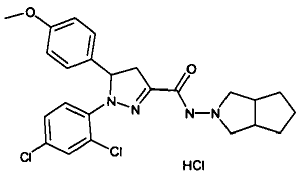 | 1-(2,4-Dichloro-phenyl)-5-(4-methoxy-phenyl)-4,5-dihydro-1H-pyrazole-3-carboxylic acid (hexahydro-cyclope | 1 H NMR (300 MHz, CHLOROFORM-*d*) δ ppm 1.61 - 1.91 (m, 6 H) 2.96 (br. s., 2 H) 3.27 (dd, *J*=18.09, 5.86 Hz, 1 H) 3.49 (m, 2 H) 3.64 (dd, *J=*18.09, 12.60 Hz, 1 H) 3.72 (s, 3 H) 3.90 (br. s., 2 H) 5.82 (dd, *J*=12.60, 5.86 Hz, 1 H) 6.71 (d, *J*=8.64 Hz, 2 H) 7.00 (d, *J*=8.64 Hz, 2 H) 7.06 (dd, *J*=8.72, 2.27 Hz, 1 H) 7.21 (m, 2 H) 9.25 | 473 |
| 109 A | 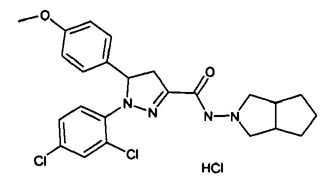 | 1-(2,4-Dichloro-phenyl)-5-(4-methoxy-phenyl)-4,5-dihydro-1H-pyrazole-3-carboxylic acid (hexahydro-cyclope | | |
| 110 | 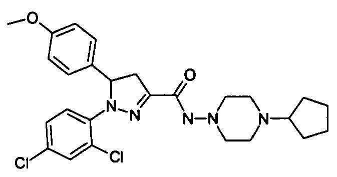 | 1-(2,4-Dichloro-phenyl)-5-(4-methox y-phenyl)-4,5-dihydro-1H-pyrazole-3 -carboxylic acid (4-cyclopentyl-pip erazin-1-yl)-amide | | 516 |
| 111 | 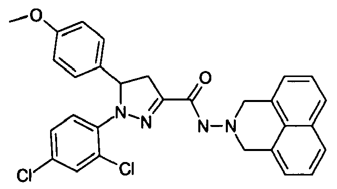 | 1-(2,4-Dichloro-phenyl)-5-(4-methox y-phenyl)-4,5-dihydro-1 H-pyrazole-3 -carboxylic acid (1H,3H-benzo[de]is oquinolin-2-yl)-amide | | 531 |
| 112 | 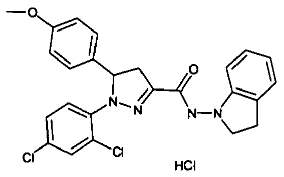 | 1-(2,4-Dichloro-phenyl)-5-(4-methox y-phenyl)-4,5-dihydro-1H-pyrazole-3 -carboxylic acid (2,3-dihydro-indol -1-yl)-amide hydrochloride | | 481 |
| 113 | 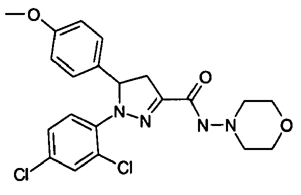 | 1-(2,4-Dichloro-phenyl)-5-(4-methox y-phenyl)-4,5-dihydro-1H-pyrazole-3 -carboxylic acid morpholin-4-ylamid e | | 449 |
| 114 | 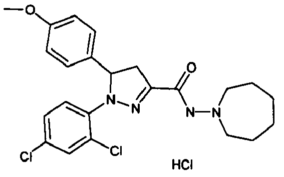 | 1-(2,4-Dichloro-phenyl)-5-(4-methoxy-phenyl)-4,5-dihydro-1H-pyrazole-3-carboxylic acid azepan-1-ylamide hydrochloride | 1 H NMR (300 MHz, METHANOL-*d₄*) δ ppm 1.77 (br. s., 4 H) 1.98 (br. s., 4 H) 3.27 (dd, *J*=6.15 Hz, 1 H) 3.63 (m, 5 H) 3.70 (s, 3H) 5.90 (dd, *J*=12.45, 6.15 Hz, 1 H) 6.76 (d, *J*=8.64 Hz, 2 H) 7.16 (dd, *J*=8.64, 2.20 Hz, 1 H) 7.10 (d, *J*=8.64 Hz, 2H)7.33(m,2H) | 461 |
| 115 | 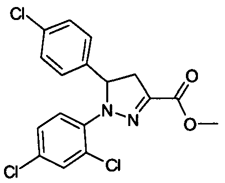 | 5-(4-Chloro-phenyl)-1-(2,4-dichloro-phenyl)-4,5-dihydro-1H-pyrazole-3-carboxylic acid methyl ester | | 383 |
| 116 | 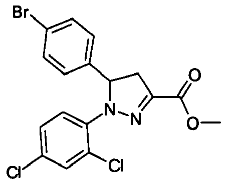 | 5-(4-Bromo-phenyl)-1-(2,4-dichloro-phenyl)-4,5-dihydro-1H-pyrazole-3-carboxylic acid methyl ester | | 427 |
| 117 | 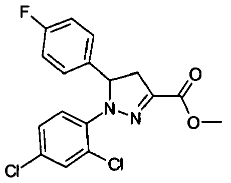 | 1-(2,4-Dichloro-phenyl)-5-(4-fluoro-phenyl)-4,5-dihydro-1H-pyrazole-3-carboxylic acid methyl ester | | 367 |
| 118 | 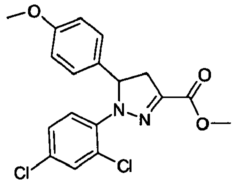 | 1-(2,4-Dichloro-phenyl)-5-(4-methox y-phenyl)-4,5-dihydro-1H-pyrazole-3 -carboxylic acid methyl ester | | 379 |
| 119 | 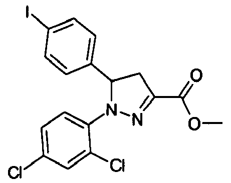 | 1-(2,4-Dichloro-phenyl)-5-(4-iodo-phenyl)-4,5-dihydro-1H-pyrazole-3-carboxylic acid methyl ester | | 475 |
| 120 | 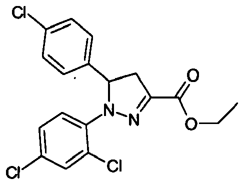 | 5-(4-Chloro-phenyl)-1-(2,4-dichloro -phenyl)-4,5-dihydro-1H-pyrazole-3-carboxylic acid ethyl ester | | 397 |
| 121 | 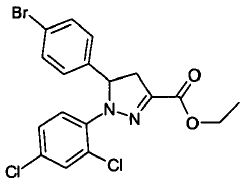 | 5-(4-Bromo-phenyl)-1-(2,4-dichloro-phenyl)-4,5-dihydro-1H-pyrazole-3-carboxylic acid ethyl ester | | 441 |
| 122 | 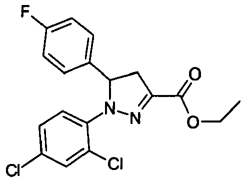 | 1-(2,4-Dichloro-phenyl)-5-(4-fluoro-phenyl)-4,5-dihydro-1H-pyrazole-3-carboxylic acid ethyl ester | | 381 |
| 123 | 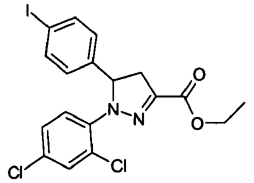 | 1-(2,4-Dichloro-phenyl)-5-(4-iodo-p henyl)-4,5-dihydro-1H-pyrazole-3-ca rboxylic acid ethyl ester | | 489 |
| 124 | 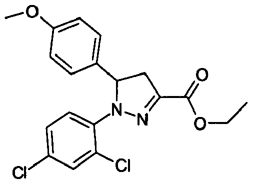 | 1-(2,4-Dichloro-phenyl)-5-(4-methox y-phenyl)-4,5-dihydro-1H-pyrazole-3 -carboxylic acid ethyl ester | | 393 |
| 125 | 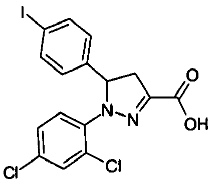 | 1-(2,4-Dichloro-phenyl)-5-(4-iodo-phenyl)-4,5-dihydro-1H-pyrazole-3-ca rboxylic acid | | 461 |
| 126 | 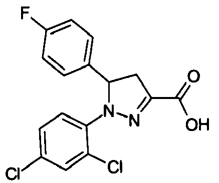 | 1-(2,4-Dichloro-phenyl)-5-(4-fluoro-phenyl)-4,5-dihydro-1H-pyrazole-3-carboxylic acid | | 353 |
| 127 | 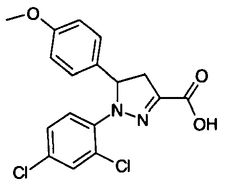 | 1-(2,4-Dichloro-phenyl)-5-(4-methoxy-phenyl)-4,5-dihydro-1H-pyrazole-3-carboxylic acid | | 365 |
| 128 | 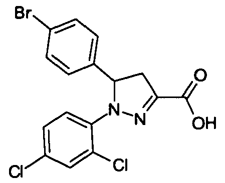 | 5-(4-Bromo-phenyl)-1-(2,4-dichloro-phenyl)-4,5-dihydro-1H-pyrazole-3-carboxylic acid | | 413 |
| 129 | 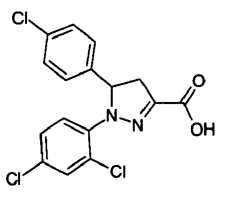 | 5-(4-Chloro-phenyl)-1-(2,4-dichloro-phenyl)-4,5-dihydro-1H-pyrazole-3-carboxylic acid | | 369 |
| 130 | 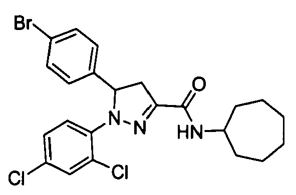 | 5-(4-Bromo-phenyl)-1-(2,4-dichloro-phenyl)-4,5-dihydro-1H-pyrazole-3-carboxylic acid cycloheptylamide | 1 H NMR (300 MHz, METHANOL-*d*₄) δ ppm 1.49 - 1.78 (m, 10 H) 1.95 (br. s., 2 H) 3.21 (dd, *J*=18.09, 5.79 Hz, 1 H) 3.67 (dd, *J*=18.09, 12.08 Hz, 1 H) 3.99 (m, 1 H) 5.86 (dd, *J*=12.08, 5.79 Hz, 1 H) 7.09 (d, | 508 |
| 131 | 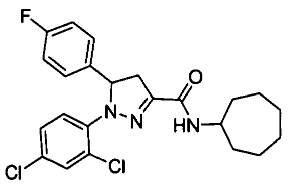 | 5-(4-Fluorophenyl)-1-(2,4-dichloro-phenyl)-4,5-dihydro-1H-pyrazole-3-carboxylic acid cycloheptylamide | 1H NMR (300 MHz, DMSO-*d*₆) δ ppm 1.36 - 1.67 (m, 10 H) 1.72 - 1.87 (m, 2 H) 3.05 (dd, *J*=18.02, 5.64 Hz, 1 H) 3.63 (dd, *J*=18.02, 11.79 Hz, 1 H) 3.79 - 3.92 (m, *J*=8.95, 1 H) 5.80 (dd, *J*=11.79, 5.64 Hz, 1 H) 7.04 (t *J*=8.79 Hz 2H) 7.12 | 448 |
| 132 | 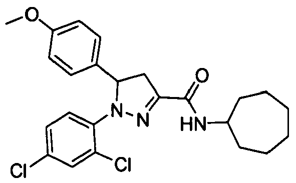 | 5-(4-methoxyphenyl)-1-(2,4-dichloro-phenyl)-4,5-dihydro-1H-pyrazole-3-carboxylic acid cycloheptylamide | | 460 |
| 133 | 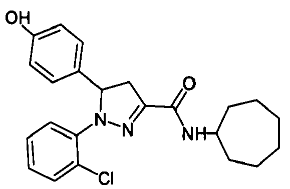 | 5-(4-hydroxyphenyl)-1-(2,4-dichloro-phenyl)-4,5-dihydro-1H-pyrazole-3-carboxylic acid cycloheptylamide | | 412 |
| 134 | 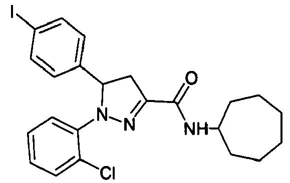 | 5-(4-iodophenyl)-1-(2,4-dichloro-phenyl)-4,5-dihydro-1H-pyrazole-3-carboxylic acid cycloheptylamide | | 522 |
| 135 | 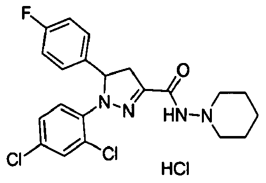 | 1-(2,4-Dichloro-phenyl)-5-(4-fluoro-phenyl)-4,5-dihydro-1H-pyrazole-3-carboxylic acid piperidin-1-ylamide hydrochloride | 1H NMR (300 MHz, DMSO-*d*₆) δ ppm 1.42 (br. s., 2 H) 1.72 (br. s., 4 H) 3.08 (dd, *J*=18.02, 6.23 Hz, 1 H) 3.14 (br. s., 4 H) 3.69 (dd, *J*=18.02, 12.08 Hz, 1 H) 5.84 (dd, *J*=12.08, 6.23 Hz, 1 H) 7.06 (t, *J*=8.79 Hz, 2 H) 7.20 (dd *J*=8 72.5 49 Hz 2 H) | 435 |
| 136 | 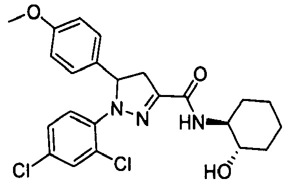 | 1-(2,4-dichlorophenyl)-N-(( 1S,2S)-2-hydroxycyclohexyl)-5-(4-methoxyphenyl)-4,5-dihydro-1H-pyrazole-3-carboxamide | | 462 |
| 137 | 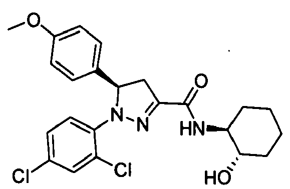 | (R)-1-(2,4-dichlorophenyl)-N-((1S,2S)-2-hydroxycyclohexyl)-5-(4-methoxyphenyl)-4,5-dihydro-1H-pyrazole-3-carboxamide | | 462 |
| 138 | 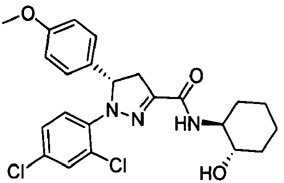 | (S)-1-(2,4-dichlorophenyl)-N-((1S,2S)-2-hydroxycyclohexyl)-5-(4-methoxyphenyl)-4,5-dihydro-1H-pyrazole-3-carboxamide | | 462 |
| 139 | 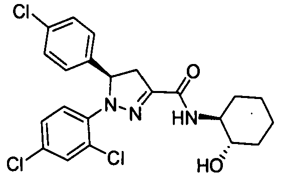 | (R)-5-(4-chlorophenyl)-1-(2,4-dichlorophenyl)-N-((1 S,2S)-2-hydroxycyclohexyl)-4,5-dihydro-1H-pyrazole-3-carboxamide | | 466 |
| 140 | 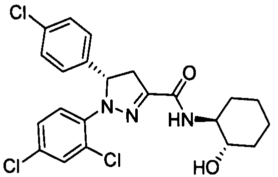 | (S)-5-(4-chlorophenyl)-1-(2,4-dichlorophenyl)-N-((1S,2S)-2-hydroxycyclohexyl)-4,5-dihydro-1H-pyrazole-3-carboxamide | | 466 |
| 141 | 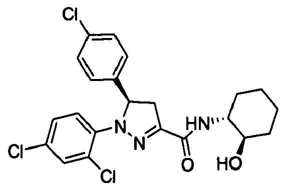 | (R)-5-(4-chlorophenyl)-1-(2,4-dichlorophenyl)-N-((1 R,2R)-2-hydroxycyclohexyl)-4,5-dihydro-1H-pyrazole-3-carboxamide | | 466 |
| 142 | 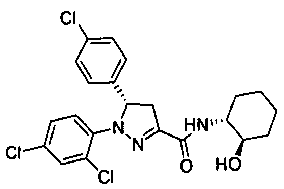 | (S)-5-(4-chlorophenyl)-1-(2,4-dichlorophenyl)-N-((1 R,2R)-2-hydroxycyclohexyl)-4,5-dihydro-1H-pyrazole-3-carboxamide | | 466 |
| 143 | 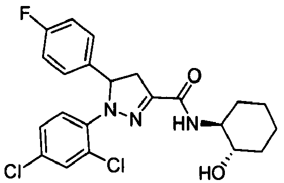 | 1-(2,4-dichlorophenyl)-5-(4-fluorophenyl)-N-((1S,2S)-2-hydroxycyclohexyl)-4,5-dihydro-1H-pyrazole-3-carboxamide | | 450 |
| 144 | 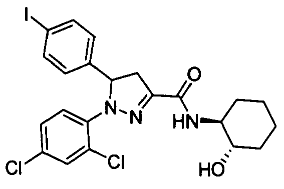 | 1-(2,4-dichlorophenyl)-5-(4-iodophenyl)-N-((1S,2S)-2-hydroxycyclohexyl)-4,5-dihydro-1H-pyrazole-3-carboxamide | | 558 |
| 145 | 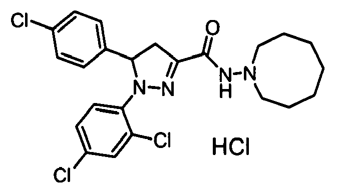 | 5-(4-Chloro-phenyl)-1-(2,4-dichloro -phenyl)-4,5-dihydro-1H-pyrazole-3-carboxylic acid azocan-1-ylamide hydrochloride | 1 H NMR (300 MHz, METHANOL-*d*₄) δ ppm 1.76 (br. s., 6 H) 2.01 (br. s., 4 H) 3.26 (dd, *J*=18.02, 6.52 Hz, 1 H) 3.65 (m, 4 H) 3.76 (dd, *J*=18.02, 12.52 Hz, 1 H) 5.96 (dd, *J*=12.52, 6.52 Hz, 1 H) | 479 |
| 146 | 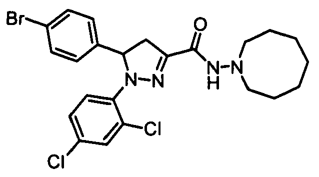 | 5-(4-bromo-phenyl)-1-(2,4-dichloro-phenyl)-4,5-dihydro-1H-pyrazole-3-carboxylic acid azocan-1-ylamide | | 523 |
| 147 | 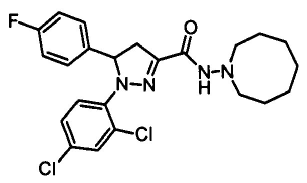 | 5-(4-fluoro-phenyl)-1-(2,4-dichloro-phenyl)-4,5-dihydro-1H-pyrazole-3-carboxylic acid azocan-1-ylamide | | 463 |
| 148 | 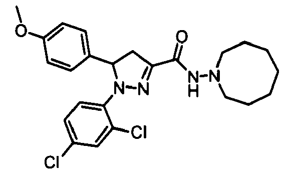 | 5-(4-methoxy-phenyl)-1-(2,4-dichloro-phenyl)-4,5-dihydro-1H-pyrazole-3-carboxylic acid azocan-1-ylamide | 1 H NMR (300 MHz, CHLOROFORM-*d*) δ ppm 1.53 -1.62(m,2H)1.68(br.s.,8H) 3.10 (m, 4 H) 3.34 (dd, J=18.31, 6.01 Hz, 1 H) 3.64 (dd, *J*=18.31, 12.01 Hz, 1 H) 3.72 (s, 3 H) 5.68 (dd, *J*=12.01, 6.01 Hz 1 H) 6.70 (d, *J*=8, 64 | 475 |
| 149 | 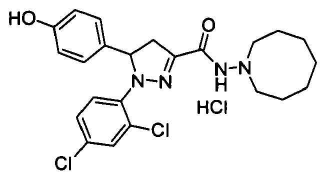 | 5-(4-hydroxy-phenyl)-1-(2,4-dichloro-phenyl)-4,5-dihydro-1H-pyrazole-3-carboxylic acid azocan-1-ylamide hydrochloride | | 461 |
| 150 | 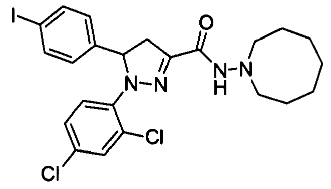 | 5-(4-iodo-phenyl)-1-(2,4-dichloro-phenyl)-4,5-dihydro-1H-pyrazole-3-carboxylic acid azocan-1-ylamide | | 571 |
| 151 | 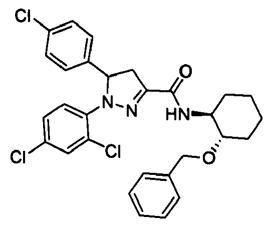 | N-((1S,2S)-2-(benzyloxy)cyclohexyl)-5-(4-chlorophenyl)-1-(2,4-dichlorophenyl)-4,5-dihydro-1H-pyrazole-3-carboxamide | | 556 |
| 152 | 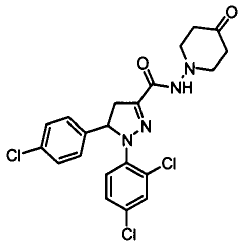 | 5-(4-Chloro-phenyl)-1-(2,4-dichloro-phenyl)-4,5-dihydro-1H-pyrazole-3-carboxylic acid (4-oxo-piperidin-1-yl)-amide | | 465 |
| 153 | 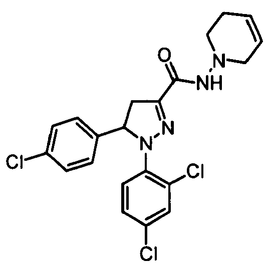 | 5-(4-Chloro-phenyl)-1-(2,4-dichloro-phenyl)-4,5-dihydro-1H-pyrazole-3-carboxylic acid (3,6-dihydro-2H-pyr idin-1-yl)-amide | 1 H NMR (250 MHz, DMSO-*d*₆) δ ppm 2.20 (br. s., 2 H) 2.96 (t, J=5.63 Hz, 2 H) 3.08 (dd, *J*=17.98, 5.76 Hz, 1 H) 3.37 (br. s., 2 H) 3.67 (dd, *J*=17.98, 11.80 Hz, 1 H) 5.58 - 5.74 (m, 2 H) 5.81 (dd, *J*=11.80, 5.76 Hz, 1 H) 7.13 - 7.24 (m, 2 H) 7.30 (d *J*=8.23 HZ 3 H) 7.51 (d | 449 |
| 154 | 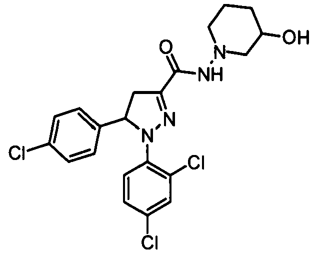 | 5-(4-Chloro-phenyl)-1-(2,4-dichloro-phenyl)-4,5-dihydro-1H-pyrazole-3-carboxylic acid (3-hydroxy-piperidi n-1-yl)-amide | 1 H NMR (300 MHz, DMSO-d₆) δ ppm 1.05 (d, *J*=10.26 Hz, 1 H) 1.43 (d, *J*=10.55 Hz, 1 H) 1.52 - 1.82 (m, 2 H) 2.57 (m, 1 H) 2.83 (m, 1 H) 3.04 (m, 2 H) 3.41 - 3.70 (m, 2 H) 4.75 (t, J=3.66 Hz, 1 H) 5.78 (dd, J=11.57, 5.42 Hz, 1 H) 7.14 (d, *J*=8, 20 Hz, 2H), 7.28 (d, *J*=7, 91 | 467 |
| 155 | 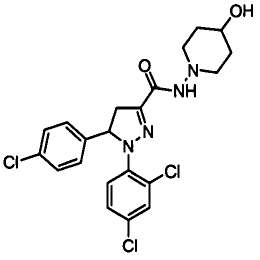 | 5-(4-Chloro-phenyl)-1-(2,4-dichloro-phenyl)-4,5-dihydro-1H-pyrazole-3-carboxylic acid (4-hydroxy-piperidi n-1-yl)-amide | 1 H NMR (250 MHz, CHLOROFORM-*d*) δ ppm 1.91 (m, 2 H) 2.08 (m, 2 H) 2.89 (m, 2 H) 3.15 (m, 2 H) 3.40 (dd, J=18.39, 6.31 Hz, 1 H) 3.76 (dd, *J*=18.39, 12.08 Hz, 1 H) 3.91 (d, *J*=3.57 Hz, 1 H) 5.80 (dd, *J*=12.08, 6.31 Hz, 1 H) 7.09-7.20(m, 4H) 7.24(m, 2 | 467 |
| 156 | 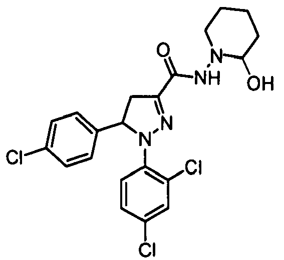 | 5-(4-Chloro-phenyl)-1-(2,4-dichloro-phenyl)-4,5-dihydro-1H-pyrazole-3-carboxylic acid (2-hydroxy-piperidin-1-yl)-amide | | 467 |
| 157 | 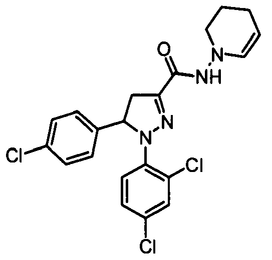 | 5-(4-Chloro-phenyl)-1-(2,4-dichloro-phenyl)-4,5-dihydro-1H-pyrazole-3-carboxylic acid (3,4-dihydro-2H-pyridin-1-yl)-amide | | 449 |
| 158 | 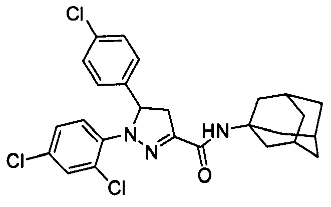 | 5-(4-Chloro-phenyl)-1-(2,4-dichloro-phenyl)-4,5-dihydro-1H-pyrazole-3-carboxylic acid adamantan-1-ylamide | | 502 |
| 159 | 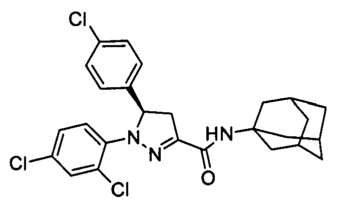 | (R)-5-(4-Chloro-phenyl)-1-(2,4-dichloro-phenyl)-4,5-dihydro-1H-pyrazole-3-carboxylic acid adamantan-1-ylamide | | 502 |
| 160 | 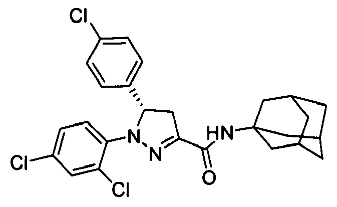 | (S)-5-(4-Chloro-phenyl)-1-(2,4-dichloro-phenyl)-4,5-dihydro-1H-pyrazole-3-carboxylic acid adamantan-1-ylamide | | 502 |
| 161 | 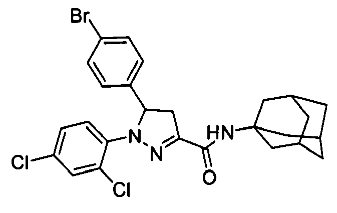 | 5-(4-bromo-phenyl)-1-(2,4-dichloro-phenyl)-4,5-dihydro-1H-pyrazole-3-carboxylic acid adamantan-1-ylamide | | 546 |
| 162 | 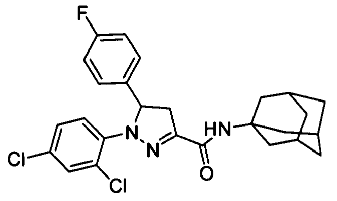 | 5-(4-fluoro-phenyl)-1-(2,4-dichloro-phenyl)-4,5-dihydro-1H-pyrazole-3-carboxylic acid adamantan-1-ylamide | | 486 |
| 163 | 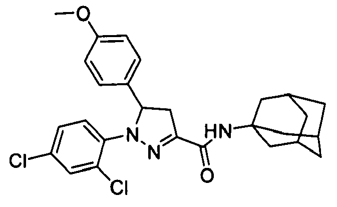 | 5-(4-methoxy-phenyl)-1-(2,4-dichloro-phenyl)-4,5-dihydro-1H-pyrazole-3-carboxylic acid adamantan-1-ylamide | | 498 |
| 164 | 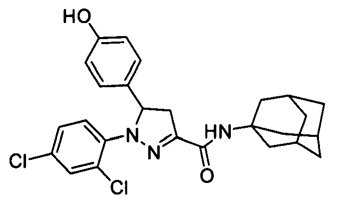 | 5-(4-hydroxy-phenyl)-1-(2,4-dichloro-phenyl)-4,5-dihydro-1H-pyrazole-3-carboxylic acid adamantan-1-ylamide | | 484 |
| 165 | 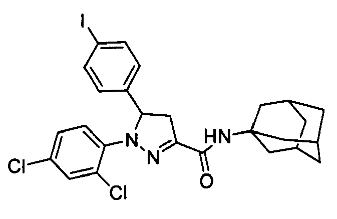 | 5-(4-iodo-phenyl)-1-(2,4-dichloro-phenyl)-4,5-dihydro-1H-pyrazole-3-carboxylic acid adamantan-1-ylamide | | 594 |
| 166 | 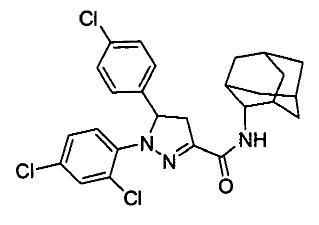 | 5-(4-Chloro-phenyl)-1-(2,4-dichloro-phenyl)-4,5-dihydro-1H-pyrazole-3-carboxylic acid adamantan-2-ylamide | | 502 |
| 167 | 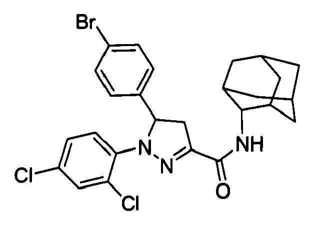 | 5-(4-bromo-phenyl)-1-(2,4-dichloro-phenyl)-4,5-dihydro-1H-pyrazole-3-carboxylic acid adamantan-2-ylamide | | 546 |
| 168 | 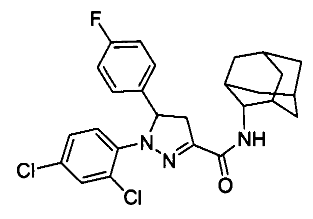 | 5-(4-fluoro-phenyl)-1-(2,4-dichloro-phenyl)-4,5-dihydro-1H-pyrazole-3-carboxylic acid adamantan-2-ylamide | | 486 |
| 169 | 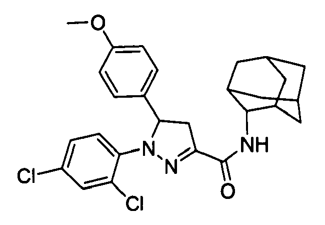 | 5-(4-methoxy-phenyl)-1-(2,4-dichloro-phenyl)-4,5-dihydro-1H-pyrazole-3-carboxylic acid adamantan-2-ylamide | | 498 |
| 170 | 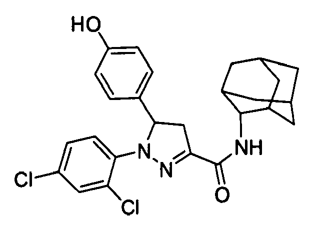 | 5-(4-hydroxy-phenyl)-1-(2,4-dichloro-phenyl)-4,5-dihydro-1H-pyrazole-3-carboxylic acid adamantan-2-ylamide | | 484 |
| 171 | 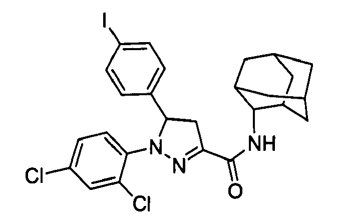 | 5-(4-iodo-phenyl)-1-(2,4-dichloro-phenyl)-4,5-dihydro-1H-pyrazole-3-carboxylic acid adamantan-2-ylamide | | 594 |
| 172 | 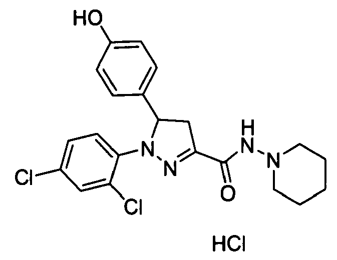 | 1-(2,4-Dichloro-phenyl)-5-(4-hydroxy-phenyl)-4,5-dihydro-1H-pyrazole-3 -carboxylic acid piperidin-1-ylamide hydrochloride | 1H NMR (300 MHz, METHANOL-*d*₄) δ ppm 1.69 (br. s., 2 H) 2.00 (br. s., 4 H) 3.24 (dd, *J*=17.94, 6.23 Hz, 1 H) 3.55 (br. s., 4 H) 3.69 (dd, *J*=17.94, 12.38 Hz, 1 H) 5.86 (dd, *J*=12.38, 6.23 Hz, 1 H) 6.61 (d, *J*=8.50 Hz, 2 H) 7.00 *(d J*=8.50 Hz, 2H) 7.17 (dd | 433 |
| 173 | 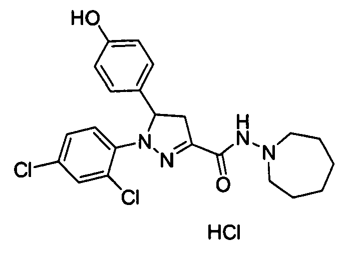 | 1-(2,4-Dichloro-phenyl)-5-(4-hydroxy-phenyl)-4,5-dihydro-1H-pyrazole-3 -carboxylic acid azepan-1-ylamide hydrochloride | 1H NMR (300 MHz, DMSO-*d*₆) δ ppm 0.81 (d, *J*=7.18 Hz, 3 H) 1.59 (br. s., 4 H) 1.78 (br. s., 4 H) 3.36 (br. s., 4 H) 3.76 (dq, *J*=11.28, 7.18 Hz, 1 H) 5.81 (d, J=11.28 Hz, 1 H) 6.59 (d, J=8.50 Hz, 2 H) 6.89 (d, *J*=8.50 Hz, 2 H) 7.31 (dd, *J*=8.79, 2.34 Hz, 1 H) 7.49 (d, *J*=2.34 Hz, 1 | 447 |
| 174 | 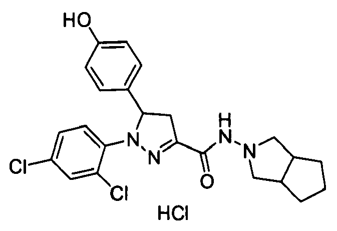 | 1-(2,4-Dichloro-phenyl)-5-(4-hydroxy-phenyl)-4,5-dihydro-1H-pyrazole-3 -carboxylic acid (hexahydro-cyclopenta[c]pyrrol-2-yl)-amide hydrochloride | 1 H NMR - (300 MHz, METHANOL-*d*₄) □ ppm 1.68 (br. s., 2 H) 1.81 (br. s., 4 H) 2.98 (br. s., 2 H) 3.06 - 3.21 (m, 2 H) 3.26 (dd, *J*=18.02, 6.30 Hz, 1 H) 3.71 (dd, *J*=18.02, 12.45 Hz, 1 H) 4.06 (m, 2 H) 5.88 (dd, *J*=12.45, 6.30 Hz, 1 H) 6.63 (d, *J*=8.64 Hz, 2 H) | 459 |
| 175 | 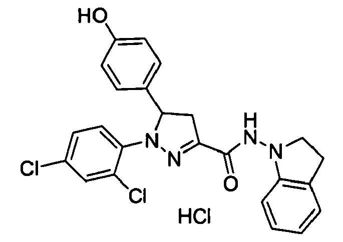 | 1-(2,4-Dichloro-phenyl)-5-(4-hydroxy-phenyl)-4,5-dihydro-1H-pyrazole-3 -carboxylic acid (2,3-dihydro-indol-1-yl)-amide hydrochloride | | 467 |
| 176 | 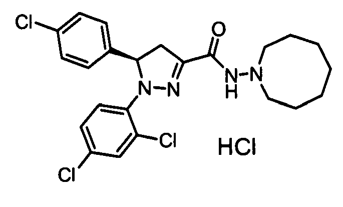 | (R)-5-(4-Chloro-phenyl)-1-(2,4-dichloro-phenyl)-4,5-dihydro-1H-pyrazole-3-carboxylic acid azocan-1-ylamide hydrochloride | 1 H NMR (300 MHz, METHANOL-*d*₄) δ ppm 1.76 (br. s., 6 H) 2.01 (br. s., 4 H) 3.26 (dd, *J*=18.02, 6.52 Hz, 1 H) 3.65 (m, 4 H) 3.76 (dd, J=18.02, 12.52 Hz, 1 H) 5.96 (dd, *J*=12.52, 6.52 Hz, 1 H) 7.16 - 7.28 (m, 5 H) 7.40 (d, *J*=8.64 Hz 1H)7.37 (d, *J*=2, 34 | 479 |
| 177 | 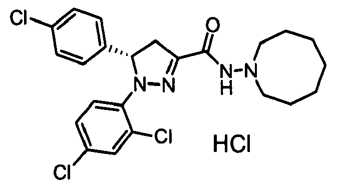 | (S)-5-(4-Chloro-phenyl)-1-(2,4-dichloro-phenyl)-4,5-dihydro-1H-pyrazole-3-carboxylic acid azocan-1-ylamide hydrochloride | 1 H NMR (300 MHz, METHANOL-*d*₄) δ ppm 1.76 (br. s., 6 H) 2.01 (br. s., 4 H) 3.26 (dd, *J*=18.02, 6.52 Hz, 1 H) 3.65 (m, 4 H) 3.76 (dd, J=18.02, 12.52 Hz, 1 H) 5.96 (dd, *J*=12.52, 6.52 Hz, 1 H) 7.16 - 7.28 (m, 5 H) 7.40 (d, *J*=8.64 Hz, 1 H) 7.37 (d, *J*=2.34 | 479 |
| 184 | 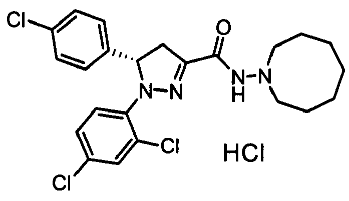 | (S)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride | | 477 |
| 188 | 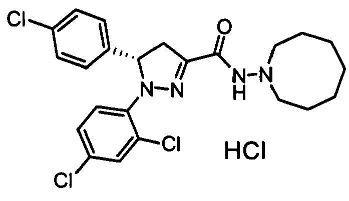 | (R)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide | | 477 |
| 193 | 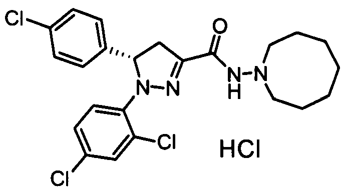 | 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride | | 477 |
| 196 | 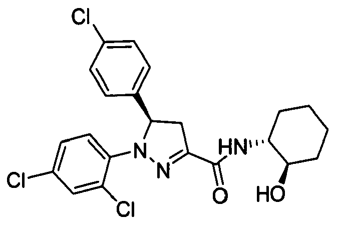 | (R)-5-(4-chlorophenyl)-1-(2,4-dichlorophenyl)-N-((1R,2R)-2-hydroxycyclohexyl)-4,5-dihydro-1H-pyrazole-3-carboxamide | | 466 |
| 197 | 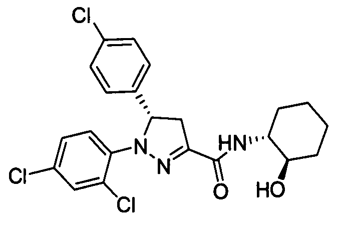 | (S)-5-(4-chlorophenyl)-1-(2,4-dichlorophenyl)-N-((1R,2R)-2-hydroxycyclohexyl)-4,5-dihydro-1H-pyrazole-3-carboxamide | | 466 |
| 198 | 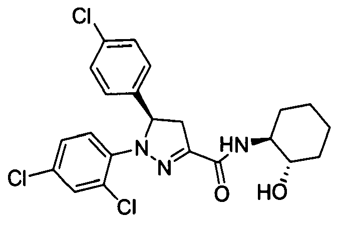 | (R)-5-(4-chlorophenyl)-1-(2,4-dichlorophenyl)-N-((1S,2S)-2-hydroxycyclohexyl)-4,5-dihydro-1H-pyrazole-3-carboxamide | | 466 |
| 199 | 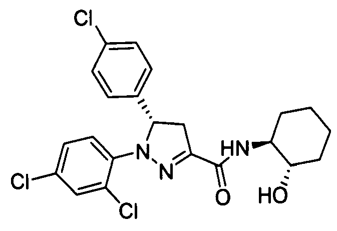 | (S)-5-(4-chlorophenyl)-1-(2,4-dichlorophenyl)-N-((1S,2S)-2-hydroxycyclohexyl)-4,5-dihydro-1H-pyrazole-3-carboxamide | | 466 |
| 200 | 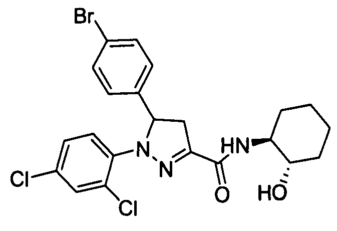 | 5-(4-bromophenyl)-1-(2,4-dichlorophenyl)-N-((1S,2S)-2-hydroxycyclohexyl)-4,5-dihydro-1H-pyrazole-3-carboxamide | | 510 |
| 201 | 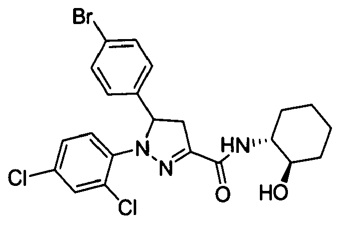 | 5-(4-bromophenyl)-1-(2,4-dichlorophenyl)-N-((1R,2R)-2-hydroxycyclohexyl)-4,5-dihydro-1H-pyrazole-3-carboxamide | | 510 |
| 202 | 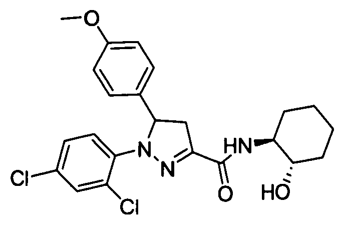 | 1-(2,4-dichlorophenyl)-N-((1S,2S)-2-hydroxycyclohexyl)-5-(4-methoxyphenyl)-4,5-dihydro-1H-pyrazole-3-carboxamide | | 462 |
| 203 | 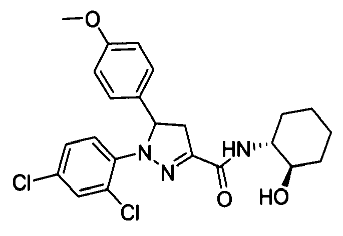 | 1-(2,4-dichlorophenyl)-N-((1 R,2R)-2-hydroxycyclohexyl)-5-(4-methoxyphenyl)-4,5-dihydro-1H-pyrazole-3-carboxamide | | 462 |

[178] N-((1R,2R)-2-(benzyloxy)cyclohexyl)-5-(4-chlorophenyl)-1-(2,4-dichlorophenyl)-4,5-dihydro-1H-pyrazole-3-carboxamide [M+H]⁺ = 556
[179] 1-(2,4-Dichloro-phenyl)-5-(4-fluoro-phenyl)-4,5-dihydro-1 H-pyrazole-3-carboxylic acid (2,3-dihydro-indol-1-yl)-amide hydrochloride [M+H]⁺ = 469
[180] 1-(2,4-Dichloro-phenyl)-5-(4-hydroxy-phenyl)-4,5-dihydro-1H-pyrazole-3-carboxylic acid [M+H]⁺ = 351
[181] (R)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride [M+H]⁺ = 485
[182] (5)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride [M+H]⁺ = 485
[183] (S)-5-(4-Chloro-phenyl)-1-(2,4-dichloro-phenyl)-4,5-dihydro-1H-pyrazole-3-carboxylic acid azepan-1-ylamide hydrochloride [M+H]⁺ = 465
[184] (S)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride [M+H]⁺ = 477
[185] (S)-5-(4-Chloro-phenyl)-1-(2,4-dichloro-phenyl)-4,5-dihydro-1H-pyrazole-3-carboxylic acid azepan-1-ylamide [M+H]⁺ = 465
[186] (S)- 5-(4-Chloro-phenyl)-1-(2,4-dichloro-phenyl)-4,5-dihydro-1H-pyrazole-3-carboxylic acid cycloheptylamide [M+H]⁺ = 464
[187] (R)-5-(4-Chloro-phenyl)-1-(2,4-dichloro-phenyl)-4,5-dihydro-1H-pyrazole-3-carboxylic acid azepan-1-ylamide hydrochloride [M+H]⁺ = 465
[188] (R)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide [M+H]⁺ = 477
[189] (R)- 5-(4-Chloro-phenyl)-1-(2,4-dichloro-phenyl)-4,5-dihydro-1H-pyrazole-3-carboxylic acid cycloheptylamide [M+H]⁺ = 464
[190] (R)- 5-(4-Chloro-phenyl)-1-(2,4-dichloro-phenyl)-4,5-dihydro-1H-pyrazole-3-carboxylic acid (2,3-dihydro-indo)-1-yl)-amide [M+H]⁺ = 485
[191] 5-(4-Chloro-phenyl)-1-(2,4-dichloro-phenyl)-4,5-dihydro-1H-pyrazole-3-carboxylic acid cycloheptylmethyl-amide [M+H]⁺ = 478
[192] 5-(4-Chloro-phenyl)-1-(2,4-dichloro-phenyl)-4,5-dihydro-1H-pyrazole-3-carboxylic acid (2,6-dimethyl-piperidin-1-yl)-amide hydrochloride [M+H]⁺ = 479
[193] 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride [M+H]⁺ = 477
[194] 5-(4-Chloro-phenyl)-1-(2,4-dichloro-phenyl)-4,5-dihydro-1H-pyrazole-3-carboxylic acid pyrrolidin-1-ylamide hydrochloride [M+H]⁺ = 437
[195] [5-(4-Chloro-phenyl)-1-(2,4-dichloro-phenyl)-4,5-dihydro-1 H-pyrazole-3-yl]-(4-methyl-piperazin-1-yl)-methanone hydrochloride [M+H]⁺ = 451
[60A] 5-(4-Bromo-phenyl)-1-(2,4-dichloro-phenyl)-4,5-dihydro-1H-pyrazole-3-carboxylic acid piperidin-1-ylamide
[66A] 5-(4-Bromo-phenyl)-1-(2,4-dichloro-phenyl)-4,5-dihydro-1H-pyrazole-3-carboxylic acid (2,3-dihydro-indol-1-yl)-amide
[82A] 5-(4-Chloro-phenyl)-1-(2,4-dichloro-phenyl)-4,5-dihydro-1H-pyrazole-3-carboxylic acid (2-(R)-hydroxy-cyclohexyl)-amide
[83A] 5-(4-Chloro-phenyl)-1-(2,4-dichloro-phenyl)-4,5-dihydro-1 H-pyrazole-3-carboxylic acid (2-(S)-hydroxy-cyclohexyl)-amide
[91A] 1-(2,4-Dichloro-phenyl)-5-(4-fluoro-phenyl)-4,5-dihydro-1H-pyrazole-3-carboxylic acid (hexahydro-cyclopenta[c]pyrrol-2-yl)-amide
[106A]1-(2,4-Dichloro-phenyl)-5-(4-methoxy-phenyl)-4,5-dihydro-1H-pyrazole-3-carboxylic acid piperidin-1-ylamide
[109A]1-(2,4-Dichloro-phenyl)-5-(4-methoxy-phenyl)-4,5-dihydro-1H-pyrazole-3-carboxylic acid (hexahydro-cyclopenta[c]pyn-ol-2-yl)-amide
[112A] 1-(2,4-Dichloro-phenyl)-5-(4-methoxy-phenyl)-4,5-dihydro-1H-pyrazole-3-carboxylic acid (2,3-dihydro-indol-1-yl)-amide
[114A] 1-(2,4-Dichloro-phenyl)-5-(4-methoxy-phenyl)-4,5-dihydro-1H-pyrazole-3-carboxylic acid azepan-1-ylamide

### SUBGROUP A6.1

### Subgroup A6.1: Chemical Example

### Preparation of 4-(4-Chlorophenyl)-3-methyl-2-oxo-but-3-enoic acid

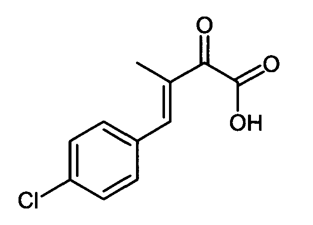

4-Chlorobenzaldehyde (1.4 g, 10 mmol) was dissolved in MeOH (1 mL) and 2-oxobutyric acid (1 g, 9.8 mmol) was subsequently added. A third of a 2 M KOH solution (0.84 g) in MeOH (2.5 mL) was added drop wise at a rate that the temperature of the reaction medium did not rise above 27 °C. The remaining alkaline solution was added quickly causing an increase of the temperature to 42 °C and the yellow colour of the solution turns into orange-red. After approximately five minutes a voluminous and yellow precipitate appeared. After 30 min, water (5 mL) was added and the organic layer was separated. After extraction with *tert*-butyl methyl ether (8 mL), the aqueous phase was acidified with conc. HCl to pH 1 and thoroughly extracted with *tert*-butyl methyl ether (3 x 8 mL). The combined organic layers were washed with aq. NaCl solution, dried with Na₂SO₄, and evaporated to dryness to afford a cream-white solid (0.88 g, 40% yield). recrystallization of 0.6 g of the crude solid in hexane (40 mL) and ethyl acetate (5 mL) afforded 0.44 g of pure product (80 % recovery).
¹H NMR (400 MHz, CDCl₃): δ 2.17 (3H, s, CH₃), 7.44 (4H, d, J 3.28 Hz, ArH), 8.41 (1H, s, CH); ¹³C NMR (100 MHz, CDCl₃): δ 13.2 (CH₃), 129.2 (CH), 129.6 (C), 131.9 (CH), 133.4 (C), 136.4 (C), 147.6 (CH), 164.4 (CO), 187.1 (CO)

### Preparation of 5-(4-Chlorophenyl)-1-(2,4-dichlorophenyl)-4-methyl-4,5-dihydro-1H-pyrazole-3-carboxylic acid

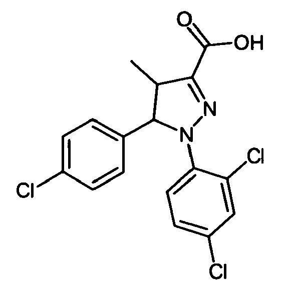

A mixture of 4-(4-chlorophenyl)-3-methyl-2-oxo-but-3-enoic acid (0.72 g, 3.2 mmol) and 2,4-dichlorophenyl hydrazine hydrochloride (0.68 g, 3.2 mmol) in glacial acetic acid (10.6 mL) was heated under nitrogen atmosphere to reflux for 4 h. The crude mixture was allowed to cool down to room temperature and poured into ice. The dark mixture was extracted with dichloromethane, washed with aq. NaCl, dried over Na₂SO₄, and evaporated to dryness to yield 1.18 g of crude product (100 % yield) as a mixture of two diastereomers. The ratio of the two diastereomers was determined by NMR spectroscopy to be 80:20.

### Preparation of 5-(4-Chlorophenyl)-1-(2,4-dichlorophenyl)-4-methyl-4,5-dihydro-1H-pyrazole-3-carboxylic acid methyl ester

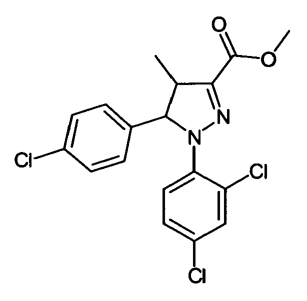

Methyl iodide (0.16 mL, 2.25 mmol) was added drop wise to a mixture of 5-(4-chlorophenyl)-1-(2,4-dichlorophenyl)-4-methyl-4,5-dihydro-1H-pyrazole-3-carboxylic acid (0.47 g, 1.27 mmol) and KHCO₃ (0.19 g, 1.9 mmol) in anhydrous dimethylformamide (10 mL) under nitrogen atmosphere. The mixture was stirred at room temperature under nitrogen overnight (ca 16 h). Water was added, the mixture was extracted with ethyl acetate and the combined organic layers were thoroughly washed with aq. NaCl solution. After drying over Na₂SO₄ and evaporation under reduced pressure, 0.5 g of the crude product were obtained. Purification by column chromatography (SiO₂, 40:1 SiO₂, packed with 100% hexane and eluted with a gradient of 1% to 5% ethyl acetate) affords 0.045 g (9% yield) of the minor isomer (RS and SR as a racemic mixture, trans isomer) and 0.23 g (48% yield) of the major isomer (RR and SS as a racemic mixture, cis isomer).

### Subgroup A6.1: Chemical Example 24: Minor isomer: trans-5-(4-Chlorophenyl)-1-(2,4-dichlorophenyl)-4-methyl-4,5-dihydro-1H-pyrazole-3-carboxylic acid methyl ester

| | |
|---|---|
| 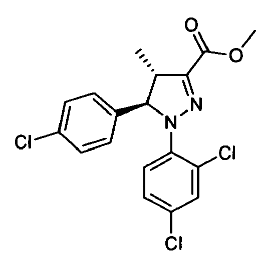 | ¹H NMR (400 MHz, CDCl₃): δ 1.49 (3H, d, J 7.6 Hz, CH₃), 3.51 (1H. m, CH), 3.90 (3H, s, OCH₃), 5.32 (1H, d, J 5.6 Hz, CH), 7.03-7.10 (3H, m, ArH), 7.20 (2H, ap d J 8.4 Hz, ArH), 7.24-7.28 (2H, m, ArH) ¹³C NMR (100 MHz, CDCl₃): δ 18.4 (CH₃), 49.6 (CH), 52.5 (OCH₃), 75.5 (CH), 126.2 (CH), 126.7 (C), 127.8 (CH), 128.1 (CH), 129.3 (CH), 130.4 (CH), 134.5 (C), 137.6 (C), 139.1 (C), 145.2 (C), 162.8 (CO) |

MS (M+H)⁺: 398

### Subgroup A6.1: Chemical Example 23: Major isomer: cis-5-(4-Chlorophenyl)-1-(2,4-dichlorophenyl)-4-methyl-4,5-dihydro-1H-pyrazole-3-carboxylic acid methyl ester

| | |
|---|---|
| 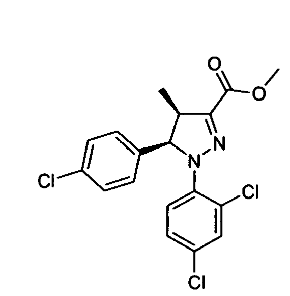 | ¹H NMR (400 MHz, CDCl₃): δ 0.94 (3H, d, J 7.2 Hz, CH₃), 3.80 (1H, m, CH), 3.88 (3H, s, OCH₃), 5.82 (1H, d, J 11.2 Hz, CH), 7.04-7.10 (3H, m, ArH), 7.22 (2H, m, ArH), 7.24-7.29 (2H, m, ArH) ¹³C NMR (100 MHz, CDCl₃): δ 17.8 (CH₃), 49.5 (CH), 52.7 (OCH₃), 75.7 (CH), 126.2 (CH), 127.7 (C), 127.9 (CH), 129.4 (CH), 130.4 (CH), 130.8 (C), 134.4 (C), 137.9 (C), 139.0 (C), 144.8 (C), 162.8 (CO) |

MS (M+H)⁺: 398

### Hydrolysis of the racemic mixture of (RR)- and (SS)-5-(4-Chlorophenyl)-1-(2,4-dichlorophenyl)-4-methyl-4,5-dihydro-1H-pyrazole-3-carboxylic acid methyl ester

Aq. 2 M NaOH (6.4 mL) was added to a solution of the racemic mixture of (*RR*)- and (*SS*)-5-(4-chlorophenyl)-1-(2,4-dichlorophenyl)-4-methyl-4,5-dihydro-1H-pyrazole-3-carboxylic acid methyl ester (0.36 g, 0.9 mmol) in THF (6.4 mL). The mixture was left to stir at room temperature for 3 h (until TLC showed disappearance of the starting material). Water was added and the reaction mixture was concentrated under reduced pressure. The residue was washed once with Et₂O and the aqueous phase acidified to pH 1 with 1 M HCl. Extraction with ethyl acetate, drying with Na₂SO₄ and concentration to dryness yielded pure acid (0.23 g, 68% yield).

### Subgroup A6.1: Chemical Example 19: Major isomer: cis-5-(4-Chloro-phenyl)-1-(2,4-dichloro-phenyl)-4-methyl-4,5-dihydro-1H-pyrazole-3-carboxylic acid

| | |
|---|---|
| 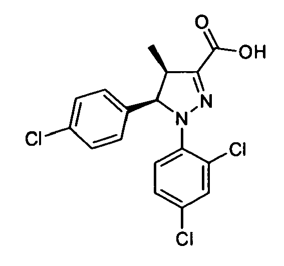 | ¹H NMR (400 MHz, CHLOROFORM-d) δ ppm 0.96 (d, *J*=7.42 Hz, 3 H) 3.82 (td, *J*=11.72, 7.42 Hz, 1 H) 5.91 (d, *J*=11.72 Hz, 1 H) 7.04 (d, J=8.60 Hz, 2 H) 7.11 (dd, *J*=8.60, 2.34 Hz, 1 H) 7.21 - 7.30 (m, 4 H) |
| | |
| | ¹³C NMR (100 MHz, CDCl₃): δ 13.6 (CH₃), 43.5 (CH), 72.0 (CH), 124.9 (CH), 127.6 (CH), 129.1 (CH), 129.6 (CH), 130.9 (CH), 131.3 (C), 132.7 (C), 134.5 (C), 138.7 (C), 144.6 (C), 165.9 (CO). |

MS (M+H)⁺: 384

### Hydrolysis of the racemic mixture of (RS)- and (SR)-5-(4-Chlorophenyl)-1-(2,4-dichlorophenyl)-4-methyl-4,6-dihydro-1H-pyrazole-3-carboxylic acid methyl ester

The hydrolysis was carried out as described above for the racemic mixture of (*RR*)- and (*SS*)-5-(4-Chlorophenyl)-1-(2,4-dichlorophenyl)-4-methyl-4,5-dihydro-1H-pyrazole-3-carboxylic acid methyl ester

### Subgroup A6.1: Chemical Example 20: Minor isomer: trans-5-(4-Chlorophenyl)-1-(2,4-dichlorophenyl)-4-methyl-4,5-dihydro-1H-pyrazole-3-carboxylic acid

| | |
|---|---|
| 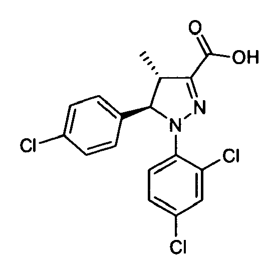 | ¹H NMR (400 MHz, CHLOROFORM-d) δ ppm 1.52 (d, *J*=7.03 Hz, 3 H) 3.52 (m, 1 H) 5.39 (d, *J*=5.47 Hz, 1 H) 7.08 (ddd, *J*=13.92, 5.62, 5.28 Hz, 3 H) 7.14-7.34 (m, 4 H) |
| | |
| | ¹³C NMR (100 MHz, CDCl₃): δ 18.4 (CH₃), 48.9 (CH), 76.2 (CH), 126.4 (CH), 126.7 (C), 127.8 (CH), 128.0 (CH), 129.5 (CH), 130.5 (CH), 131.5 (C), 134.7 (C), 137.6 (C), 138.6 (C), 144.2 (C), 166.5 (CO). |

MS (M+H)⁺: 384

### Preparation of 4-(4-Chlorophenyl)-3-methyl-2-oxo-but-3-enoic acid methyl ester

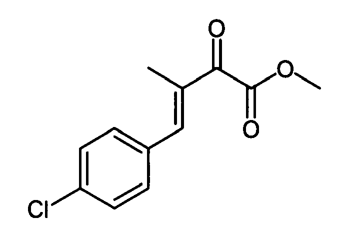

4-(4-Chlorophenyl)-3-methyl-2-oxo-but-3-enoic acid (0.13 g, 0.58 mmol) was dissolved in Et₂O (0.8 mL) and EtOH (1.2 mL) and the solution was cooled down to 0 °C. TMSCH₂N₂ (0.43 mL of a 2 M solution in Et₂O, 0.87 mmol) was added drop wise and the mixture was stirred at 0 °C for 1 h. A second portion of TMSCH₂N₂ (0.2 mL of a 2 M solution in Et₂O, 0.40 mmol) was added and the reaction was allowed to reach room temperature. After 30 min, the solvents were evaporated to dryness and the residue was taken up in a mixture of Et₂O/hexane 1:1 (10 mL). The organic layer was washed with 2% aq. HCl (10 mL), aq. NaHCO₃ (10 mL) and aq. NaCl (10 mL), dried over Na₂SO₄ and concentrated to dryness to give pure product (68 mg, 49% yield).

### Preparation of 5-(4-Chlorophenyl)-1-(2,4-dichlorophenyl)-4-methyl-4,5-dihydro-1H-pyrazole-3-carboxylic acid methyl ester

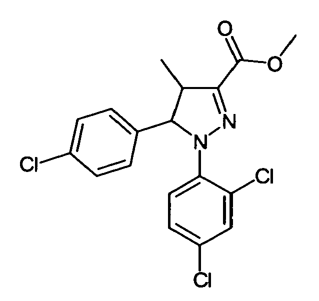

A mixture of 4-(4-chlorophenyl)-3-methyl-2-oxo-but-3-enoic acid methyl ester (0.4 g, 1.68 mmol) and 2,4-dichlorohydrazine hydrochloride (0.356 g, 1.68 mmol) in glacial acetic acid (4 mL) was heated at reflux under nitrogen atmosphere for 4 h. The reaction mixture was then allowed to cool down to room temperature and poured into ice. The dark mixture was extracted with dichloromethane, washed with aq. NaCl, dried over Na₂SO₄ and evaporated to dryness to yield the crude product (0.64 g, 95 % yield, as a mixture of two diastereomers). Purification by column chromatography (SiO₂, 40:1 SiO₂, packed with 100% hexane and eluted with a gradient of 1% to 5% ethyl acetate) of 0.5 g of crude product afforded 0.051 g of the minor isomer (RS and SR as a racemic mixture) and 0.20 g of the major isomer (*RR* and SS as racemic mixture).

### Preparation of Example 1: cis-5-(4-Chloro-phenyl)-1-(2,4-dichlorophenyl)-4-methyl-4,5-dihydro-1H-pyrazole-3-carboxylic acid piperidin-1-ylamide Hydrochloride

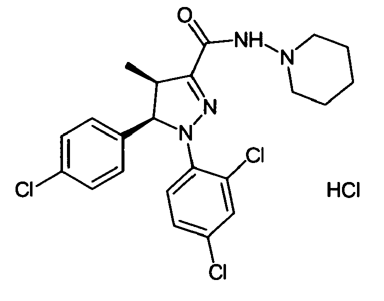

5-(4-Chlorophenyl)-1-(2,4-dichlorophenyl)-4-methyl-4,5-dihydro-1H-pyrazole-3-carboxylic acid (0.160 g, 0.417 mmol) was dissolved in toluene (4 mL) and was partially distilled at atmospheric pressure to eliminate water traces. The resulting solution was cooled to 75-85 °C. DMF was added (2 drops) as a catalyst and thionyl chloride (0.060 mL, 0.5 mmol) was added drop by drop. The solution was stirred until formation of the acid chloride was complete (monitoring by IR). The mixture was cooled to 20 - 25 °C and was added drop wise to a solution of 1-aminopiperidine (0.053 mL, 0.500 mmol), dichloromethane (5 mL) and N,N-diisopropylethylamine (0.170 mL, 1 mmol) under nitrogen at a temperature between 5-10 °C. After complete addition the mixture was warmed to 20 - 25 °C and stirred overnight. The organic layer was washed with water (3 x 5 mL), a sat. NaHCO₃ solution (3 x 25 mL) and again with water (3 x 5 mL). The combined organic layers were dried with Na₂SO₄, filtered and concentrated under vacuum to give a solid (130 mg, 68 % yield).

The crude solid was dissolved in ethyl acetate and a 2.8 M HCl solution in ethanol was added drop wise to form the hydrochloride which was obtained as a beige solid (82 mg, 49 % yield).
¹H NMR (400 MHz, METHANOL-*d*₄) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H)
MS (M+H)⁺: 466

### Subgroup A6.1: Chemical Example 2: trans-5-(4-Chloro-phenyl)-1-(2,4-dichlorophenyl)-4-methyl-4,5-dihydro-1H-pyrazole-3-carboxylic acid piperidin-1-ylamide Hydrochloride

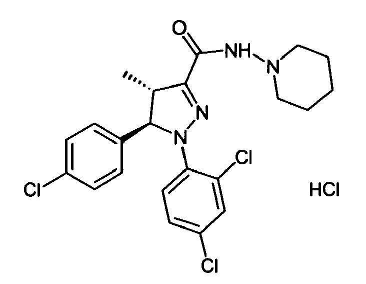

5-(4-Chlorophenyl)-1-(2,4-dichlorophenyl)-4-methyl-4,5-dihydro-1H-pyrazole-3-carboxylic acid (0.190 g, 0.50 mmol) was dissolved in toluene (4 mL) and was partially distilled at atmospheric pressure to eliminate water traces. The resulting solution was cooled to 75-85 °C. DMF was added (2 drops) as a catalyst and thionyl chloride (0.060 mL, 0.5 mmol) was added drop by drop. The solution was stirred until formation of the acid chloride was complete (monitoring by IR). The mixture was cooled to 20 - 25 °C and was added drop wise to a solution of 1-aminopiperidine (0.0623 mL, 0.57 mmol), dichloromethane (5 mL) and N,N-diisopropylethylamine (0.197 mL, 1.15 mmol) under nitrogen at a temperature between 5 - 10 °C. After complete addition the mixture was warmed to 20 - 25 °C and stirred overnight. The organic layer was washed with water (3 x 5 mL), a sat. NaHCO₃ solution (3 x 25 mL) and again with water (3 x 5 mL). The combined organic layers were dried with Na₂SO₄, filtered and concentrated under vacuum to give a solid.
The crude solid was dissolved in ethyl acetate and a 2.8 M HCl solution in ethanol was added drop wise to form the hydrochloride which was obtained as a beige solid (120 mg, 50 % yield).

¹H NMR (400 MHz, DMSO-*d*₆) δ ppm 1.38 (d, *J*=7.03 Hz, 3 H) 1.42 (m, 2 H) 1.71 (m, 4 H) 3.12 (m, 4 H) 3.42 (dt, *J*=13.38, 5.86 Hz, 1 H) 5.36 (d, *J*=5.86 Hz, 1 H) 7.19 (d, *J*=8.21 Hz, 2 H) 7.31 (d, *J*=8.60 Hz, 3 H) 7.49 (d, *J*=2.34 Hz, 1 H) 7.53 (d, *J*=8.60 Hz, 1 H) 10.49 (s, 1 H)
MS (M+H)⁺: 466

The following compounds were prepared according to the processes described above. Those skilled in the art are familiar with the starting materials that are needed to obtain said compounds.

### Subgroup A6.1: Chemical Example: Preparation of compounds of general formula 11

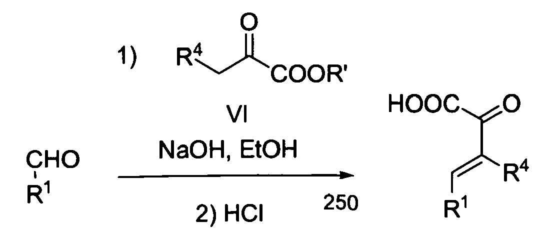

Acid of general formula VI (1.1 equivalents) was added in portions to a solution of 0.5 M aqueous NaOH (1.5 equivalents) under N₂ at room temperature. The reaction was then left stirring for 5 min and a solution of a compound of general formula VII (1 equivalent) in abs. EtOH (0.9 M) was then slowly added (10 mL/h). The reaction was left stirring at 25 °C overnight. Water was added and the solvent was evaporated under reduced pressure to eliminate the excess of EtOH. The solution was then washed with toluene and the solvent was again evaporated. The aqueous solution was then cooled in an ice bath and conc. aqueous HCl (0.2 mL of conc. HCl per mL of base) were slowly added while stirring. A white solid precipitated from the solution which was kept at 0 °C for another hour. The solid was filtered under vacuum through a sintered funnel (porosity 3) and dried at 40 °C under vacuum.
As a example, if the starting material was 2-oxobutyric acid, the yield range for this reaction condensation was around 60-86%. However, if the starting material was 2-oxopentanoic acid (oxovaleric acid) instead of 2-oxobutyric acid, the yield of the reaction was slightly lower (around 40-60%)

### Compound A1. (E)-4-(4-chlorophenyl)-3-methyl-2-oxobut-3-enoic acid

| | |
|---|---|
| 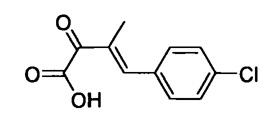 | ¹H NMR (400 MHz, CDCl₃): δ 2.17 (3H, s, CH₃), 7.44 (4H, ap d, *J*= 3.28 Hz, ArH), 8.41 (1H, s, CH). |

### Compound A2: (E)-4-(4-bromophenyl)-3-methyl-2-oxobut-3-enoic acid

| | |
|---|---|
| 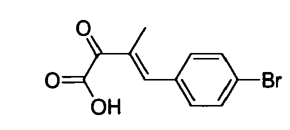 | ¹H NMR (400 MHz, CD₃OD): δ 1.99 (3H, s, CH₃), 7.36 (2H, d, J= 8.5 Hz, ArH), 7.40 (1 H, s, CH), 7.53 (2H, d, J= 8.5 Hz, ArH). |

### Compound A3: (E)-4-(4-fluorophenyl)-3-methyl-2-oxobut-3-enoic acid

| | |
|---|---|
| 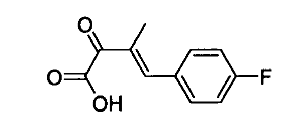 | ¹H NMR (300 MHz, DMSO-d6): δ 2.18 (3H, s, CH₃), 7.20 (2H, m), 7.53 (2H, m), 8.26 (1 H, s, CH). |

### Compound A4: (E)-4-(4-methoxyphenyl)-3-methyl-2-oxobut-3-enoic acid.

This compound was prepared following the method described above but using instead 3 eq. of NaOH.

| | |
|---|---|
| 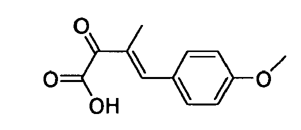 | ¹H NMR (200 MHz, CDCl₃): δ 2.09 (3H, s, CH₃), 3.84 (3H, s, OCH₃), 7.01 (2H, d, *J=* 8.8 Hz, ArH), 7.50 (1 H, s, CH), 7.52 (2H, d, J= 8.8 Hz, ArH). |

### Compound A5: (E)-3-methyl-2-oxo-4-phenylbut-3-enoic acid.

| | |
|---|---|
| 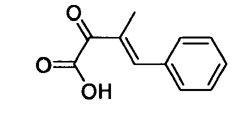 | ¹H NMR (200 MHz, CDCl₃): δ 2.19 (3H, s, CH₃), 7.33-7.51 (5H, m, ArH), 8.36 (1 H, s, CH) |

### Compound A6: (E)-4-(4-bromophenyl)-3-ethyl-2-oxobut-3-enoic acid

| | |
|---|---|
| 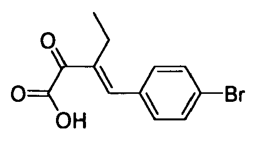 | ¹H NMR (400 MHz, CDCl₃): δ 1.16 (3H, t, J 7.5 Hz, CH₃), 2.62 (2H, q, J 7.5 Hz, CH₂), 7.35 (2H, d, J 8.4 Hz, ArH), 7.59 (2H, d, J 8.4 Hz, ArH), 8.23 (1H, s, CH). |

### Compound A7: (E)-4-(4-chlorophenyl)-3-ethyl-2-oxobut-3-enoic acid

| | |
|---|---|
| 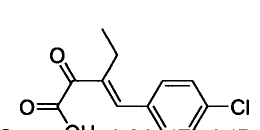 | ¹H NMR (400 MHz, CDCl₃): δ 1.16 (3H, t, J 7.2 Hz, CH₃), 2.61 (2H, q, J 15.6 Hz, J 7.2 Hz ArH), 7.44 (4H, m, ArH), 7.85 (1 H, bs, CH). |

### Compound A8: (E)-4-(5-chlorothiophen-2-yl)-3-methyl-2-oxobut-3-enoic acid

| | |
|---|---|
| 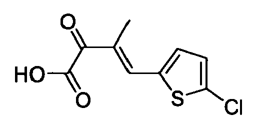 | ¹H NMR (400 MHz, CDCl₃): δ 2.09 (3H, s, CH₃), 7.10 (1H, d, J 4.8, ArH), 7.32 (1 H, d, J 4.8 Hz, ArH), 7.64 (1 H, s, ArH). |

### Compound A9: (E)-4-(5-bromothiophen-2-yl)-3-methyl-2-oxobut-3-enoic acid

| | |
|---|---|
| 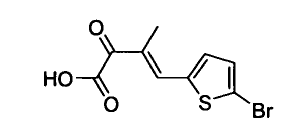 | ¹H NMR (400 MHz, CDCl₃): δ 2.19 (3H, d, J 0.9 Hz, CH₃), 7.19 (1 H, d, J 3.7Hz thiophene H), 7.27 (1 H, d, J 3.7 Hz, thiophene H), 8.74 (1 H, bs, CH). |

### Compound A10: (E)-3-methyl-2-oxo-4-(thiophen-2-yl)but-3-enoic acid

| |
|---|
| 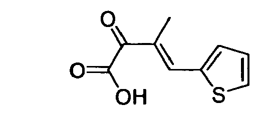 |
| ¹H NMR (400 MHz, CDCl₃): δ 2.25 (3H, s, CH₃), 7.22 (1 H, dd, J 5.1, 3.6 Hz, ArH), 7.52 (1 H, d, J 3.6 Hz, ArH), 7.72 (1H, d, J 5.1, Hz, ArH), 8.68 (1 H, s, CH). |

### Subgroup A6.1: Chemical Example: Preparation of compounds of general formula V

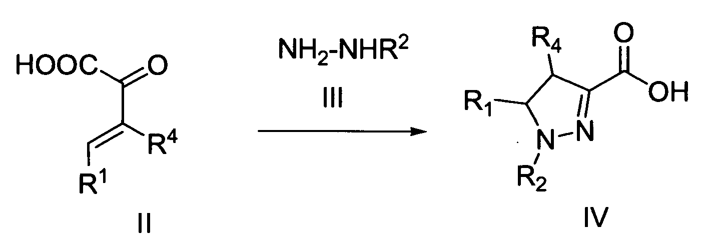

A suspension of a compound of general formula III (1 equivalent) in glacial acetic acid (40 equivalents) was heated at 80 °C under nitrogen atmosphere. When the suspension became a solution, a solution of acid of general formula II (1 equivalent) in acetic acid (20 equivalents) was added and the solution was left stirring at 80 °C for 2 h. The reaction mixture was allowed to crystallise at 0 - 4 °C overnight after partial evaporation of acetic acid. The beige solid formed was filtered off under vacuum through a sintered funnel (porosity 4) and washed several times with water. This crystallization process led to the major diastereomeric acid with *cis* configuration. The yield range for the cyclization with hydrazine to obtain the main diastereomer was around 46 - 80%.

The reaction mixture can also be cooled down to room temperature and poured through an addition funnel into water which is cooled in an ice bath with magnetically stirring. The addition must be slow and at least double volume of water per volume of acetic acid is required. A precipitate should form, but in the case a gum starts to form, it should be filtered and the rest of the material should be poured into another large volume of water. The solid obtained is suspended in water several times and filtered off until the pH of the water was above 3. This solid also corresponds to the *cis* form.

Alternatively, the dark mixture can be extracted with dichloromethane washed thoroughly with H₂O, dried over Na₂SO₄ and evaporated to dryness. Recrystallisation of the crude material from toluene (3 to 4 mL of toluene per gram of material) allows the recovery of the major diastereomer with *cis* configuration.

Formation of the methyl esters as described in the method above, followed by purification by column chromatography, allows for the separation of the two diastereoisomers.

### Subgroup A6.1: Chemical Example

### Preparation of compounds of general formula IV with trans-configuration

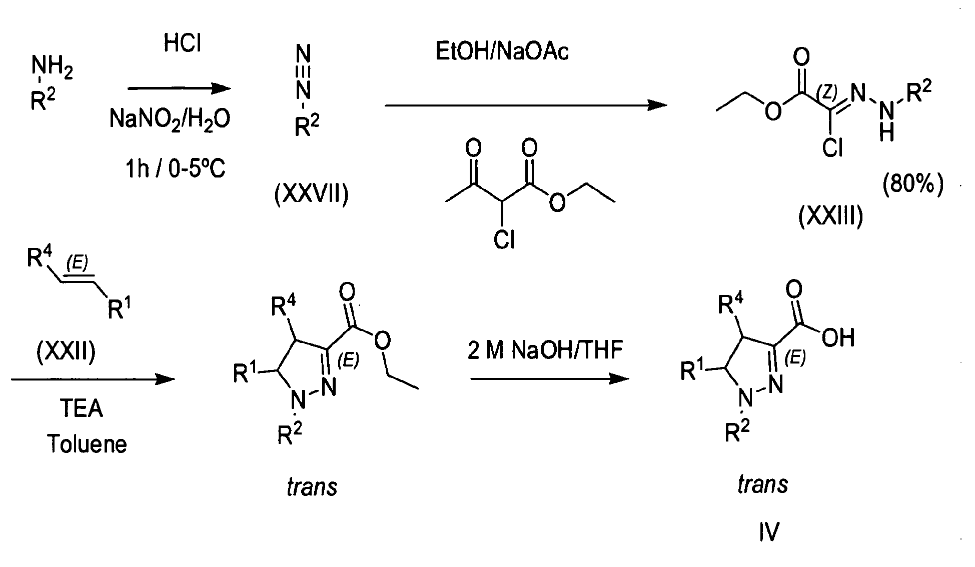

A solution of NaNO₂ (0.460 mg, 6.68 mmol) in water (0.8 ml) was added to a stirred solution of a an amine, wherein R² denotes an unsubstituted or at least mono-substituted phenyl radical, (6.17 mmol) and concentrated hydrochloric acid (1.5 ml) in ice (1.5 ml). The reaction mixture was stirred for 1 h at 0 - 5 °C and added to a cold mixture of NaOAc (1.64 g, 19.6 mmol), ethanol (26 ml) and ethyl-2-chloro-3-oxobutanoate (1.0 g, 6.06 mmol). The reaction mixture was left stirring for 1 hour until a precipitate formed which was collected by filtration, washed with ethanol and dichloromethane and dried in vacuo to give the yellow solid ethyl of general formula XXIII (70-80% yield), which was used in the next step without any further purification.

Triethylamine (2.8 eq) was added to a solution of a compound of general formula XXIII (1 eq) and a disubstituted (E)-alkene of general formula XXII (3 eq) in toluene, and the reaction mixture was stirred at reflux temperature for 1 hour. A precipitate formed which was removed by filtration after cooling to room temperature. The filtrate was concentrated and purified using a Combiflash system from Isco, eluted with cyclohexane and ethyl acetate (in a gradient program until 30% AcOEt), to obtain ethyl ester derivatives of compounds of general formula IV, (- 40 % yield).

Ethyl esters derivatives of compounds of general formula IV were hydrolysed in the presence of aqueous 2 M NaOH (2 eq) and tetrahydrofuran for 4 hours. Tetrahydofuran was partially removed by evaporation, 1 M aqueous HCl was added until pH was below 3 and the aqueous mixture was extracted with ethyl acetate, dried over Na₂SO₄, filtered and concentrated in vacuo to yield a white solid identified as carboxylic acid of general formula IV (85 % yield).

### The enantiomers of each acid (cis or trans) can be separated by chiral HPLC or by crystallization of the diastereomeric salts formed with chiral amines.

### Subgroup A6.1: Chemical Example 199: cis-5-(4-bromophenyl)-1-(2,4-dichlorophenyl)-4-methyl-4,5-dihydro-1H-pyrazole-3-carboxylic acid

| | |
|---|---|
| 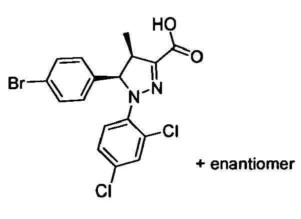 | IR (NaCl film, cm⁻¹): 2976, 1683, 1541, 1486, 1385, 1270, 1242,1116. |
| | |
| | ¹H NMR (400 MHz, CDCl₃): δ 0.96 (3H, d, J= 7.3 Hz, CH₃), 3.82 (1H. qd, J= 11.9, 7.3, 7.3, 7.3 Hz, 1H), 5.88 (1H. d, J= 11.9 Hz, CH), 6.99 (2H, ap d, J= 8.4 Hz, ArH), 7.12 (1 H, dd, *J=* 8.7, 2.3 Hz, 1 H), 7.17 (1 H, m, ArH), 7.27 (1H, m, ArH), 7.39 (2H, d, J= 8.4 Hz, ArH). |

### Subgroup A6.1: Chemical Example 221: cis-5-(4-fluorophenyl)-1-(2,4-dichlorophenyl)- 4-methyl-4,5-dihydro-1H-pyrazole-3-carboxylic acid

| | |
|---|---|
| 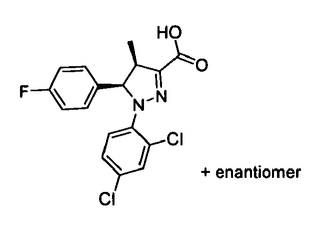 | IR (NaCl film, cm⁻¹) : 2978, 1682, 1486, 1471, 1264, 1117. |
| | |
| | ¹H NMR (400 MHz, CHLOROFORM-*d*) δ ppm 0.97 (d, *J*= 7.42 Hz, 3 H) 3.82 (td, *J*= 11.72, 7.42 Hz, 1 H) 5.91 (d, *J*=12.0 Hz, 1 H), 6.95 (m, 2 H), 7.10 (m, 3 H), 7.20 (d, *J*=9.0 Hz, 1 H), 7.30 (d, J= 3.0 Hz, 1 H). |

### Subgroup A6.1: Chemical Example 261: cis-5-(4-methoxyphenyl)-1-(2,4-dichlorophenyl)-4-methyl-4,5-dihydro-1 H-pyrazole-3-carboxylic acid

| | |
|---|---|
| 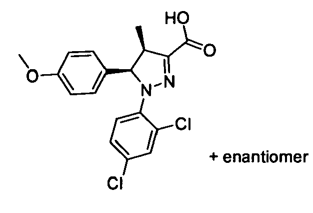 | IR (NaCl film, cm⁻¹) : 2936, 2836, 1681, 1612, 1512, 1480, 1460, 1248, 1113. |
| | |
| | ¹H NMR (400 MHz, CDCl₃): δ 0.97 (3H, d, *J=* 7.3 Hz, CH₃), 3.74 (3H, s, OCH₃), 3.80 (1 H, m, 1 H), 5.88 (1 H, d, *J=* 11.8 Hz, CH), 6.76 (2H, ap d, J= 8.6 Hz, ArH), 7.00 (2H, d, J= 8.6, ArH), 7. 09 (1 H, dd, J= 8.6, 2.3 Hz, ArH), 7.16-7.28 (2H, m, ArH). |

### Subgroup A6.1: Chemical Example 185: cis-5-(4-bromophenyl)-1-(2,4-dichlorophenyl)-4-ethyl-4,5-dihydro-1H-pyrazole-3-carboxylic acid

| | |
|---|---|
| 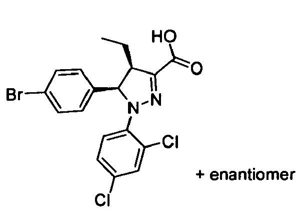 | IR (NaCl film, cm⁻¹) : 2969, 1682, 1480, 1452, 1270, 1236, 1151,1106. |
| | |
| | ¹H NMR (400 MHz, CDCl₃): δ 0.69 (3H, t, J 7.4 Hz, CH₃), 1.28-1.34 (1 H, m, CHH), 1.95-2.00 (1 H, m, CHH), 3.68 (1 H, ddd, J = 11.5, 9.7, 4.0 Hz, CH), 5.92 (1H, d, J 11.5 Hz, CH), 7.02 (2H, ap d, J 8.4 Hz, ArH), 7.09 (1 H, dd, J = 8.7, 2.4 Hz, ArH), 7.20 (1 H, d, J 8.7 Hz, ArH), 7.28 (1 H, d, J 2.4 Hz, ArH), 7.36 (2H, ap d, J 8.4 Hz, ArH); |
| | |
| | ¹³C NMR (100 MHz, CDCl₃): δ 12.4 (CH₃), 20.0 (CH₂), 50.5 (CH₃), 52.3 (OCH₃), 72.0 (CH), 122.5 ©, 124.8 (CH), 127.4 ©, 127.5 (CH), 129.9 (CH), 130.4 (CH), 131.1 ©, 131.7 (CH), 132.7 ©, 138.5 ©, 143.8 ©, 165.4 (CO). |

### Subgroup A6.1: Chemical Example 21: cis-5-(4-chlorophenyl)-1-(2,4-dichlorophenyl)-4-ethyl-4,5-dihydro-1H-pyrazole-3-carboxylic acid

| | |
|---|---|
| 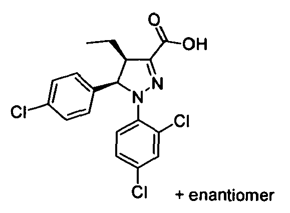 | ¹H NMR (400 MHz, CDCl₃): δ 0.70 (3H, t, *J* 7.2 Hz, CH₃), 1.31 (1 H, m, C*H*H), 2.01 (1 H, m, CH*H*), 3.68 (1 H, m, CH), 5.92 (1 H, d, *J* 11.6 Hz, CH), 7.07-7.31 (7H, m, ArH). |

### Subgroup A6.1: Chemical Example 22: trans-5-(4-chlorophenyl)-1-(2,4-dichlorophenyl)-4-ethyl-4,5-dihydro-1 H-pyrazole-3-carboxylic acid

| | |
|---|---|
| 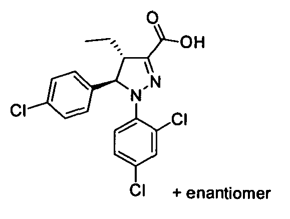 | ¹H NMR (400 MHz, CDCl₃): δ 1.08 (3H, t, J 7.2 Hz, CH₃), 1.90 (1 H, m, CHH), 1.96 (1 H, m, CHH), 3.50 (1 H, m, CH), 5.59 (1 H, d, J 4.80 Hz, CH), 7.01-7.31 (7H, m, ArH). |

### Subgroup A6.1: Chemical Example 200: trans-5-(4-bromophenyl)-1-(2,4-dichlorophenyl)-4-methyl-4,5-dihydro-1H-pyrazole-3-carboxylic acid

| | |
|---|---|
| 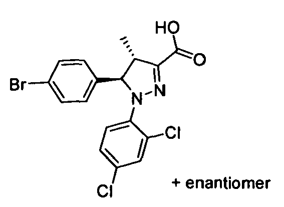 | ¹H NMR (400 MHz, CDCl₃): δ 1.52 (3H, d, J= 7.1 Hz, CH₃), 3.52 (1 H, m, 1 H), 5.35(1 H, d, J= 5.8 Hz, CH), 7.00 (2H, ap d, J= 8.4 Hz, ArH), 7.10 (1 H, dd, *J*= 8.7, 2.3 Hz, 1H), 7.22 (1H, d, *J*= 8.7, ArH), 7.27 (1 H, d, J= 2.3 Hz, ArH), 7.37 (2H, d, J= 8.4 Hz, ArH). |

### Subgroup A6.1: Chemical Example 373: cis-1-(2,4-dichlorophenyl)-4-methyl-5-phenyl-4,5-dihydro-1H-pyrazole-3-carboxylic acid

| | |
|---|---|
| 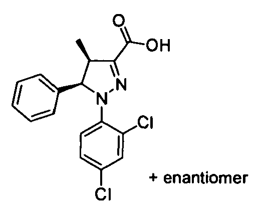 | ¹H NMR (400 MHz, CDCl₃): δ 0.96 (3 H, d, 7.6 Hz, CH₃), 3.82 (1 H, m, CH), 3.88 (3 H, s, OCH₃), 5.91 (1 H, d, J 11.6 Hz, CH), 7.09-7.31 (8 H, m , ArH) |

### Subgroup A6.1: Chemical Example 374: trans-1-(2,4-dichlorophenyl)-4-methyl-5-phenyl-4,5-dihydro-1H-pyrazole-3-carboxylic acid

| | |
|---|---|
| 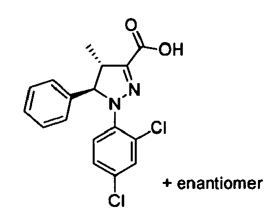 | IR (NaCl film, cm⁻¹) : 3400-2800, 1680, 1479, 1456, 1108 |
| | |
| | ¹H NMR (400 MHz, CDCl₃): δ 1.53 (3 H, d, 7.2 Hz, CH₃), 3.57 (1 H, m, CH), 3.90 (3 H, s, OCH₃), 5.37 (1 H, d, J 5.6 Hz, CH), 7.06-7.28 (8 H, m , ArH) |

### Subgroup A6.1: Chemical Example 361: cis-5-(5-chlorothiophen-2-yl)-1-(2,4-dichlorophenyl)-4-methyl-4,5-dihydro-1H-pyrazole-3-carboxylic acid

| | |
|---|---|
| 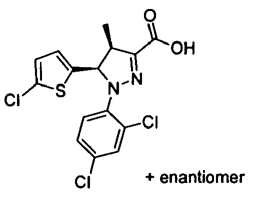 | IR (NaCl film, cm⁻¹) : 3400-2800, 1684, 1479, 1447, 1107. |
| | |
| | ¹H NMR (400 MHz, CDCl₃): δ 1.24 (3H, d, J 7.2, CH₃), 3.81 (1H, m, CH), 6.09 (1 H, d, J 12.0, CH), 6.64 (2H, ap dd, J₁ 12.0, J₂ 3.8 Hz, ArH), 7.16 (1 H, dd,, J₁ 8.8, J₂ 2.2 Hz, ArH), 7.32 (1 H, d J 2.2 Hz, ArH), 7.34 (1 H, aps, ArH), 7.36 (1 H, aps, ArH). |

### Subgroup A6.1: Chemical Example 362: trans-5-(5-chlorothiophen-2-yl)-1-(2,4-dichlorophenyl)-4-methyl-4,5-dihydro-1H-pyrazole-3-carboxylic acid

| | |
|---|---|
| 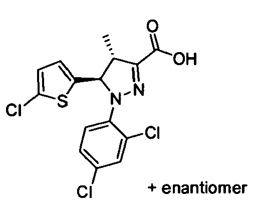 | IR (NaCl film, cm⁻¹) : 3400-2800, 1682, 1569, 1544, 1479, 1448, 1246, 1105. |
| | |
| | ¹H NMR (400 MHz, CDCl₃): δ 1.50 (3H, d, J 7.1 Hz, CH₃), 3.64 (1H, m, CH), 5.6 (1H, d, J 3.9 Hz, CH), 6.60 (2H, aps, ArH), 7.12 (1H, dd, J₁ 8.8, J₂ 2.3 Hz, ArH), 7.27 (1 H, m, ArH), 7.33 (1 H, d, J 2.3 Hz, ArH). |

### Subgroup A6.1: Chemical Example 88: cis-5-(5-bromothiophen-2-yl)-1-(2,4-dichlorophenyl)-4-methyl-4,5-dihydro-1H-pyrazole-3-carboxylic acid

| | |
|---|---|
| 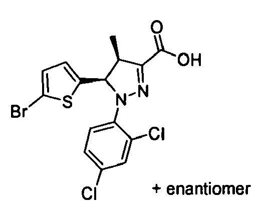 | IR (NaCl film, cm⁻¹): 3400-2800, 1682, 1569, 1545, 1479, 1459, 1245,1105. |
| | |
| | ¹H NMR (400 MHz, CDCl₃): δ 1.23 (3H, d, *J* 7.2 Hz, CH₃), 3.81 (1H,m,CH),6.11 (1H, d, *J* 11.6Hz, CH), 6.61 (1H, d, *J* 3.9 Hz, ArH), 6.80 (1 H, d, *J* 4.4 Hz, ArH), 7.16 (1 H, dd, *J*₁ 8.4 Hz, *J*₂ 3.0 Hz, ArH), 7.30-7.34 (2H, m, ArH). |
| | |
| | ¹³C NMR (100 MHz, CDCl₃): δ 12.3 (CH₃), 43.7 (CH), 69.5 (CH), 112.9 ©, 125.9 (CH), 126.8 ©, 127.8 (CH), 128.7 (CH), 129.7 (CH), 130.5 (CH), 131.8 ©, 137.8 ©, 138.6 ©, 144.9 ©, 164.5 (CO). |

### Subgroup A6.1: Chemical Example 367: cis-1-(2,4-dichlorophenyl)-4-methyl-5-(thiophen-2-yl)-4,5-dihydro-1H-pyrazole-3-carboxylic acid

| | |
|---|---|
| 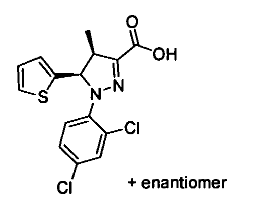 | IR (NaCl film, cm⁻¹) : 3400-2800, 1682, 1567, 1543, 1479, 1449, 1245, 1108. |
| | |
| | ¹H NMR (400 MHz, CDCl₃): δ 1.19 (3H, d, J 7.2 Hz, CH₃), 3.83 (1 H, m, CH), 6.22 (1 H, d, J 11.2 Hz, CH), 6.84 (2H, m, ArH), 7.12 (2H, m, ArH), 7.29 (1 H, d, J 2.3, ArH), 7.34 (1 H, d, J 8.8 Hz, ArH). |

### Subgroup A6.1: Chemical Example: Preparation of compounds of general formula V

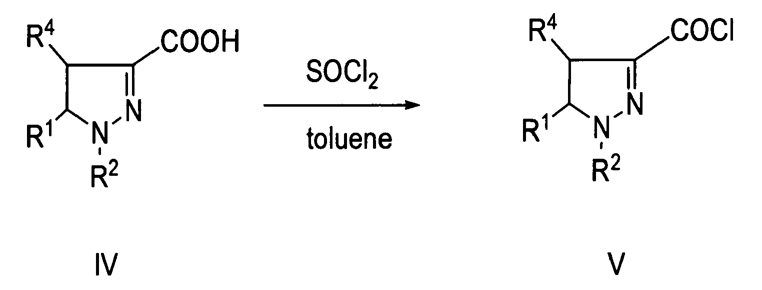

A compound of general formula IV (15 mmol) was dissolved in 120 mL of dry toluene and thionyl chloride (18 mmol) was added. The mixture was heated to 80 °C for 2.5 hours. The solvent was removed under reduced pressure and the resulting crude residue was used without any further purification.

### Cis-5-(4-chlorophenyl)-1-(2,4-dichlorophenyl)-4-methyl-4,5-dihydro-1H-pyrazole-3-carbonyl chloride

IR (NaCl film, cm⁻¹): 1732, 1700, 1533, 1478, 1212, 826.

### Cis-5-(4-bromophenyl)-1-(2,4-dichlorophenyl)-4-methyl-4,5-dihydro-1H-pyrazole-3-carbonyl chloride

IR (NaCl film, cm⁻¹): 1731, 1527, 1477, 1204, 1153, 1132, 825, 802.

### Cis-5-(4-fluorophenyl)-1-(2,4-dichlorophenyl)-4-methyl-4,5-dihydro-1H-pyrazole-3-carbonyl chloride

IR (NaCl film, cm⁻¹) : 1731, 1509, 1478, 1227, 1153, 1132, 853, 803.

### Cis-5-(4-methoxyphenyl)-1-(2,4-dichlorophenyl)-4-methyl-4,5-dihydro-1 H-pyrazole-3-carbonyl chloride

IR (NaCl film, cm⁻¹) : 1730, 1611, 1512, 1477, 1271, 1250, 1034, 831, 800.

### Cis-5-(4-bromophenyl)-1-(2,4-dichlorophenyl)-4-ethyl-4,5-dihydro-1H-pyrazole-3-carbonyl chloride

IR (NaCl film, cm⁻¹): 1728, 1526, 1478, 1227, 1200, 1153, 1129, 834, 801.

### Cis-5-(5-chlorothiophen-2-yl)-1-(2,4-dichlorophenyl)-4-methyl-4,5-dihydro-1H-pyrazole-3-carbonyl chloride

IR (NaCl film, cm⁻¹): 1732, 1528, 1477, 1446, 1226, 1112, 808.

### Trans-5-(5-chlorothiophen-2-yl)-1-(2,4-dichlorophenyl)-4-methyl-4,5-dihydro-1H-pyrazole-3-carbonyl chloride

IR (NaCl film, cm⁻¹): 1730, 1531, 1479, 1204, 1122, 806.

### Cis-5-(5-bromothiophen-2-yl)-1-(2,4-dichlorophenyl)-4-methyl-4,5-dihydro-1H-pyrazole-3-carbonyl chloride

IR (NaCl film, cm⁻¹): 1732, 1528, 1477, 1109, 806

### cis-1-(2,4-dichlorophenyl)-4-methyl-5-(thiophen-2-yl)-4,5-dihydro-1H-pyrazole-3-carbonyl chloride

IR (NaCl film, cm⁻¹): 1731, 1527, 1477, 1224, 1111, 803.

### Subgroup A6.1: Chemical Example: Preparation of compounds of general formula I

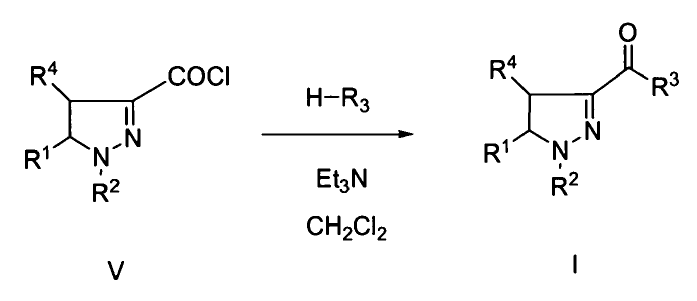

A compound of general formula R³-H (5.6 mmol) and triethylamine (4 mL) were dissolved in dichloromethane (25 mL) under nitrogen atmosphere. The resulting mixture was cooled to 0°C and a solution of a compound of general formula V (4.6 mmol) in dichloromethane (15 mL) was added dropwise. The resulting reaction mixture was stirred at room temperature (approximately 25 °C) overnight, washed with water, saturated aqueous solution of sodium bicarbonate and again with water, dried over sodium sulfate, filtered and evaporated to dryness in a rotavapor. The residue was crystallized from ethanol, ethyl acetate or acetone. The crystallized solid was removed via filtration and the mother liquors were concentrated to yield a second fraction of crystallized product. The two fractions were combined to give the desired product (yield range: 60-80 %). Alternatively, a solution of 2 N HCl in diethyl ether or 2.8 N in ethanol was added to form the respective hydrochloride, which was collected by filtration.

### Subgroup A6.1: Chemical Example 1: cis-5-(4-Chlorophenyl)-1-(2,4-dichlorophenyl)-4-methyl-4,5-dihydro-1 H-pyrazole-3-carboxylic acid piperidin-1-ylamide hydrochloride

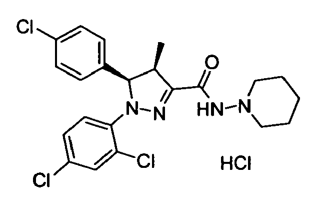

¹H NMR (400 MHz, METHANOL-*d*₄) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H)
MS (M+H)⁺: 466

### Subgroup A6.1: Chemical Example 2: trans-5-(4-Chloro-phenyl)-1-(2,4-dichlorophenyl)-4-methyl-4,5-dihydro-1H-pyrazole-3-carboxylic acid piperidin-1-ylamide hydrochloride

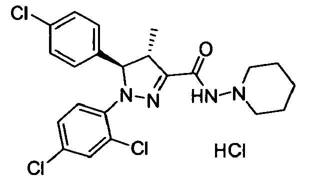

¹H NMR (400 MHz, DMSO-*d*₆) δ ppm 1.38 (d, *J*=7.03 Hz, 3 H) 1.42 (m, 2 H) 1.71 (m, 4 H) 3.12 (m, 4 H) 3.42 (dt, *J*=13.38, 5.86 Hz, 1 H) 5.36 (d, *J*=5.86 Hz, 1 H) 7.19 (d, *J*=8.21 Hz, 2 H) 7.31 (d, *J*=8.60 Hz, 3 H) 7.49 (d, *J*=2.34 Hz, 1 H) 7.53 (d, *J*=8.60 Hz, 1 H) 10.49 (s, 1 H)
MS (M+H)⁺: 466

### Subgroup A6.1: Chemical Example 15: cis-5-(4-chlorophenyl)-1-(2,4-dichlorophenyl)-N-(indolin-1-yl)-4-methyl-4,5-dihydro-1H-pyrazole-3-carboxamide

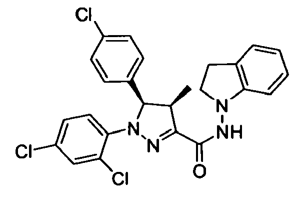

¹H NMR (300 MHz, CHLOROFORM-*d*): δ ppm 0.97 (d, *J*=7.18 Hz, 3 H) 3.08 (t, *J*=7.98 Hz, 2 H) 3.67 (t, *J*=7.98 Hz, 2 H) 3.86 (dd, *J*=11.06, 7.40 Hz, 1 H) 5.69 (d, *J*=11.28 Hz, 1 H) 6.73 (d, J=7.76 Hz, 1 H) 6.88 (t, *J*=7.40 Hz, 1 H) 7.02 - 7.12 (m, 5 H) 7.15 (d, *J*=7.76 Hz, 2 H) 7.24 (s, 1 H) 7.33 (d, *J*=1.61 Hz, 1 H) 7.99 (s, 1 H)
MS (M+H)⁺: 499

### Subgroup A6.1: Chemical Example 113: cis-5-(4-chlorophenyl)-1(2,4-dichlorophenyl)-4-methyl-N-(2-methylindolin-1-yl)-4,5-dihydro-1H-pyrazole-3-carboxamide

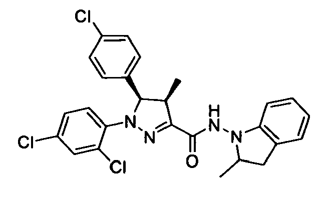

¹H NMR (300 MHz, DMSO-*d*₆): δ ppm 0.81 (t, *J*=7.40 Hz, 3 H) 1.29 (d, *J*=5.86 Hz, 3 H) 2.54 - 2.65 (m, 1H) 3.14 (m, 1 H) 3.92 (m, 2 H) 5.99 (d, *J*=11.28 Hz, 1 H) 6.45 (dd, *J*=7.54, 2.86 Hz, 1 H) 6.68 - 6.82 (m, 1 H) 6.96 - 7.23 (m, 4 H) 7.30 - 7.36 (m, 3H) 7.48 (d, *J*=2.05 Hz, 1 H) 7.71 (d, *J*=8.79 Hz, 1 H) 10.09 (s, 1 H)
MS (M+H)⁺: 513

### Subgroup A6.1: Chemical Example 215: cis-5-(4-bromophenyl)-1-(2,4-dichlorophenyl)-N-(indolin-1-yl)-4-methyl-4,5-dihydro-1H-pyrazole-3-carboxamide

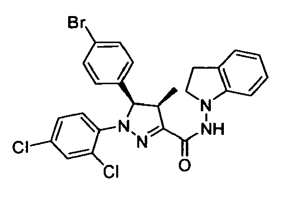

¹H NMR (300 MHz, DMSO-*d*₆): δ ppm 0.80 (d, *J*=7.18 Hz, 3 H) 2.96 (t, *J*=8.50 Hz, 2 H) 3.59 (t, *J*=8.50 Hz, 2 H) 3.89 (dd, *J*=11.13, 7.18 Hz, 1 H) 5.95 (d, *J*=11.13 Hz, 1 H) 6.49 (d, *J*=7.62 Hz, 1 H) 6.75 (t, *J*=7.03 Hz, 1 H) 6.99 - 7.14 (m, 4 H) 7.31 (dd, *J*=8.79, 2.49 Hz, 1 H) 7.42 - 7.50 (m, 3 H) 7.66 (d, *J*=8.79 Hz, 1 H) 10.19 (s, 1 H)
MS (M+H)⁺: 543

### Subgroup A6.1: Chemical Example 235: cis-1-(2,4-dichlorophenyl)-5-(4-fluorophenyl)-N-(indolin-1-yl)-4-methyl-4,5-dihydro-1H-pyrazole-3-carboxamide

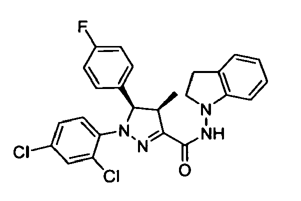

¹H NMR (300 MHz, DMSO-*d*₆)δ ppm 0.80 (d, *J*=7.32 Hz, 3 H) 2.96 (t, *J*=8.50 Hz, 2 H) 3.59 (t, J=8.50 Hz, 2 H) 3.87 (dd, *J*=11.13, 7.32 Hz, 1 H) 5.96 (d, *J*=11.13 Hz, 1 H) 6.49 (d, *J*=7.62 Hz, 1 H) 6.75 (t, *J*=7.40 Hz, 1 H) 7.00 - 7.19 (m, 6 H) 7.30 (dd, *J*=8.72, 2.42 Hz, 1 H) 7.47 (d, J=2.34 Hz, 1 H) 7.67 (d, *J*=8.79 Hz, 1 H) 10.18 (s, 1 H)
MS (M+H)⁺: 483

### Subgroup A6.1: Chemical Example 279: cis-1-(2,4-dichlorophenyl)-N-(indolin-1-yl)-5-(4-methoxyphenyl)-4-methyl-4,5-dihydro-1H-pyrazole-3-carboxamide

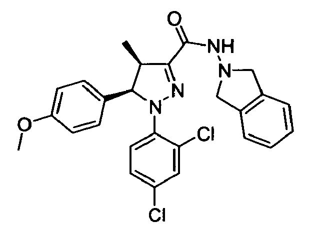

¹H NMR (300 MHz, CHLOROFORM-*d*) δ ppm 0.99 (d, *J*=7.18 Hz, 3 H) 3.08 (t, *J*=7.98 Hz, 2 H) 3.68 (t, *J*=7.91 Hz, 2 H) 3.76 (s, 3 H) 3.83 (dd, *J*=11.13, 7.18 Hz, 1 H) 5.68 (d, *J*=11.13 Hz, 1 H) 6.79 (d, *J*=8.50 Hz, 2 H) 6.74 (d, *J*=7.76 Hz, 1 H) 6.88 (t, *J*=7.40 Hz, 1 H) 7.02 - 7.09 (m, 4 H) 7.16 (d, *J*=7.47 Hz, 2 H) 7.32 (s, 1 H) 8.01 (s, 1 H)
MS (M+H)⁺: 495

### Subgroup A6.1: Chemical Example 123: cis-5-(4-chlorophenyl)-N-cycloheptyl-1-(2,4-dichlorophenyl)-4-methyl-4,5-dihydro-1H-pyrazole-3-carboxamide

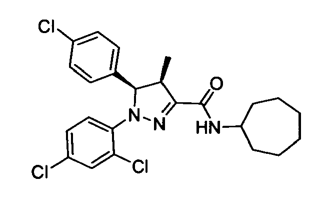

¹H NMR (300 MHz, DMSO-*d*₆) δ ppm 0.76 (d, *J*=7.18 Hz, 3 H) 1.39-1.70 (m, 10 H) 1.78 (m, 2 H) 3.83 (m, 2 H) 5.89 (d, *J*=10.99 Hz, 1 H) 7.14 (d, *J*=8.35 Hz, 2 H) 7.32 (m, 3 H) 7.49 (d, *J*=2.34 Hz, 1 H) 7.62 (d, *J*=8.79 Hz, 1 H) 8.04 (d, *J*=8.06 Hz, 1 H)
MS (M+H)⁺: 478

### Subgroup A6.1: Chemical Example 193: cis-5-(4-bromophenyl)-N-cycloheptyl-1-(2,4-dichlorophenyl)-4-methyl-4,5-dihydro-1H-pyrazole-3-carboxamide

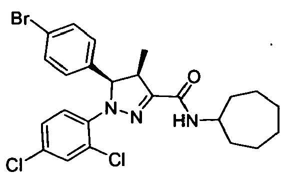

¹H NMR (300 MHz, DMSO-*d*₆) δ ppm 0.74 (d, *J*=7.18 Hz, 3 H) 1.35 - 1.66 (m, 10 H) 1.78 (m, 2 H) 3.72 - 3.90 (m, 2 H) 5.85 (d, *J*=10.99 Hz, 1 H) 7.06 (d, *J*=8.35 Hz, 2 H) 7.30 (dd, J=8.72, 2.27 Hz, 1 H) 7.44 (d, *J*=8.35 Hz, 2 H) 7.47 (d, *J*=2.20 Hz, 1 H) 7.60 (d, *J*=8.79 Hz, 1 H) 8.02 (d, *J*=8.20 Hz, 1 H)
MS (M+H)⁺: 522

### Subgroup A6.1: Chemical Example 231: cis-N-cycloheptyl-1-(2,4-dichlorophenyl)-5-(4-fluorophenyl)-4-methyl-4,5-dihydro-1H-pyrazole-3-carboxamide

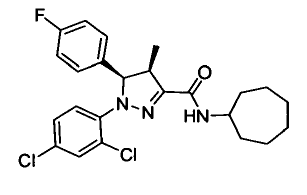

¹H NMR (300 MHz, DMSO-*d*₆) δ ppm 0.75 (d, *J*=7.32 Hz, 3 H) 1.36-1.67 (m, 10 H) 1.71 - 1.85 (m, 2 H) 3.70 - 3.90 (m, 2 H) 5.86 (d, *J*=10.99 Hz, 1 H) 7.02-7.18 (m, 4 H) 7.29 (dd, *J*=8.79, 2.34 Hz, 1 H) 7.46 (d, *J*=2.34 Hz, 1 H) 7.61 (d, *J*=8.79 Hz, 1 H) 8.02 (d, J=8.20 Hz, 1 H)
MS (M+H)⁺: 462

### Subgroup A6.1: Chemical Example 275: cis-N-cycloheptyl-1-(2,4-dichlorophenyl)-5-(4-methoxyphenyl)-4-methyl-4,5-dihydro-1H-pyrazole-3-carboxamide

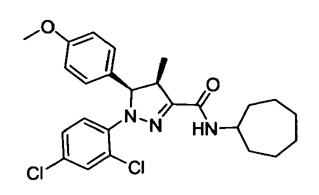

¹H NMR (300 MHz, DMSO-*d*₆) δ ppm 0.75 (d, *J*=7.32 Hz, 3 H) 1.36 - 1.67 (m, 10 H) 1.75-1.86 (m, 2 H) 3.65 (s, 3 H) 3.72 (dd, *J*=10.99, 7.32 Hz, 1 H) 3.82 (dd, *J*=8.72, 4.47 Hz, 1 H) 5.79 (d, J=10.99 Hz, 1 H) 6.77 (d, *J*=8.64 Hz, 2 H) 7.01 (d, *J*=8.64 Hz, 2 H) 7.27 (dd, *J*=8.79, 2.34 Hz, 1 H) 7.44 (d, *J*=2.34 Hz, 1 H) 7.61 (d, *J*=8.79 Hz, 1 H) 7.99 (d, *J*=8.20 Hz, 1 H)
MS (M+H)⁺: 474

### Subgroup A6.1: Chemical Example 128: (+)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide

¹H NMR (300 MHz, CHLOROFORM-*d*) δ ppm 0.94 (d, *J*=7.32 Hz, 3H) 1.19-1.45 (m, 4H) 1.76 (m, 2 H) 2.00 (m, 1 H) 2.09 (m, 1 H) 3.43 (m, 1 H) 3.78 (m, 2 H) 5.66 (d, *J*=11.28 Hz, 1 H) 6.65 (d, *J*=7.32 Hz, 1 H) 7.00 - 7.15 (m, 4 H) 7.24 (m, 2 H) 7.33 (s, 1 H)
MS (M+H)⁺: 480

### Subgroup A6.1: Chemical Example 129: (-)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide

¹H NMR (300 MHz, CHLOROFORM-*d*) δ ppm 0.93 (d, *J*=7.32 Hz, 3 H) 1.24 - 1.40 (m, 4 H) 1.75 (m, 2 H) 2.06 (m, 2 H) 3.39 (m, 1 H) 3.75 (m, 2 H) 5.64 (d, *J*=11.13 Hz, 1 H) 6.68 (d, *J*=7.32 Hz, 1 H) 7.00-7.13 (m, 4 H) 7.23 (d, *J*=8.50 Hz, 2 H) 7.35 (d, *J*=2.20 Hz, 1 H)
MS (M+H)⁺: 480

### Subgroup A6.1: Chemical Example 108: cis-5-(4-chlorophenyl)-1-(2,4-dichlorophenyl)-N-(hexahydrocyclopenta-[c]pyrrol-2(1H)-yl)-4-methyl-4,5-dihydro-1H-pyrazole-3-carboxamide hydrochloride

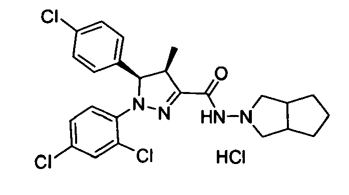

¹H NMR (300 MHz, DMSO-*d*₆) δ ppm 0.79 (d, *J*=7.18 Hz, 3 H) 1.49 (d, *J*=7.76 Hz, 2 H) 1.61 (m, 4 H) 2.66 (br. S., 2 H) 2.86 (m, 2 H) 3.54 (br. S., 2 H) 3.85 (m, 1 H) 5.96 (d, J=11.28 Hz, 1 H) 7.17 (d, *J*=8.35 Hz, 2 H) 7.35 (m, 3 H) 7.55 (d, *J*=2.20 Hz, 1 H) 7.62 (d, J=8.64 Hz, 1 H) 10.52 (br. S., 1 H)
MS (M+H)⁺: 491

### Subgroup A6.1: Chemical Example 213: cis-5-(4-bromophenyl)-1-(2,4-dichlorophenyl)-N-(hexahydrocyclopenta-[c]pyrrol-2(1H)-yl)-4-methyl-4,5-dihydro-1H-pyrazole-3-carboxamide hydrochloride

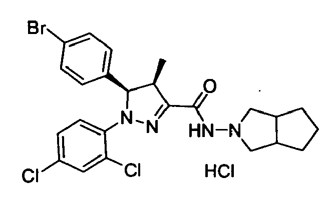

¹H NMR (300 MHz, DMSO-*d*₆) δ ppm 0.77 (d, *J*=7.32 Hz, 3 H) 1.47 (d, *J*=7.47 Hz, 2 H) 1.58-1.64 (m, 4 H) 2.64 (br. S., 2 H) 2.83 (dd, *J*=16.70, 7.47 Hz, 2 H) 3.52 (br. S., 2 H) 3.83 (dd, *J*=11.21, 7.40 Hz, 1 H) 5.92 (d, *J*=11.13 Hz, 1 H) 7.08 (d, *J*=8.20 Hz, 2 H) 7.33 (dd, *J*=8.72, 2.27 Hz, 1 H) 7.46 (d, *J*=8.35 Hz, 2 H) 7.52 (d, *J*=2.20 Hz, 1 H) 7.59 (d, J=8.79 Hz, 1 H) 10.47 (br. S., 1 H)
MS (M+H)⁺: 535

### Subgroup A6.1: Chemical Example 233: cis-1-(2,4-dichlorophenyl)-5-(4-fluorophenyl)-N-(hexahydrocyclopenta-[c]pyrrol-2(1H)-yl)-4-methyl-4,5-dihydro-1 H-pyrazole-3-carboxamide hydrochloride

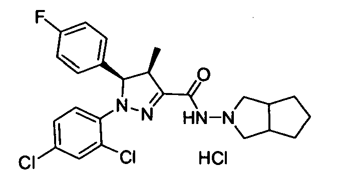

¹H NMR (300 MHz, DMSO-*d*₆) δ ppm 0.77 (d, *J*=7.32 Hz, 3 H) 1.47 (d, *J*=8.64 Hz, 2 H) 1.55-1.67 (m, 4 H) 2.63 (br. S., 2 H) 2.81 (dd, *J*=15.16, 8.86 Hz, 2 H) 3.49 (br. S., 3 H) 5.93 (d, *J*=11.28 Hz, 1 H) 7.01 - 7.25 (m, 4 H) 7.32 (dd, *J*=8.72, 2.42 Hz, 1 H) 7.51 (d, J=2.34 Hz, 1 H) 7.60 (d, *J*=8.64 Hz, 1 H) 10.35 (br. S., 1 H)
MS (M+H)⁺: 475

### Subgroup A6.1: Chemical Example 96: cis-N-(azepan-1-yl)-5-(4-chlorophenyl)-1-(2,4-dichlorophenyl)-4-methyl-4,5-dihydro-1 H-pyrazole-3-carboxamide hydrochloride

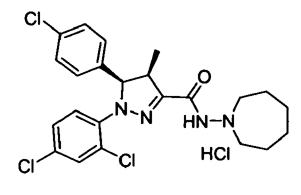

¹H NMR (300 MHz, DMSO-*d*₆) δ ppm 0.78 (d, *J*=7.18 Hz, 3 H) 1.58 (br. S., 4 H) 1.76 (br. S., 4 H) 3.32 (br. S, 4 H) 3.85 (m, 1 H) 5.95 (d, *J*=11.28 Hz, 1 H) 7.15 (d, *J*=8.50 Hz, 2 H) 7.23 - 7.36 (m, 3 H) 7.51 (d, *J*=2.34 Hz, 1 H) 7.61 (d, *J*=8.79 Hz, 1 H) 10.89 (br. S., 1 H)
MS (M+H)⁺: 479

### Subgroup A6.1: Chemical Example 207: cis-N-(azepan-1-yl)-5-(4-bromophenyl)-1-(2,4-dichlorophenyl)-4-methyl-4,5-dihydro-1 H-pyrazole-3-carboxamide hydrochloride

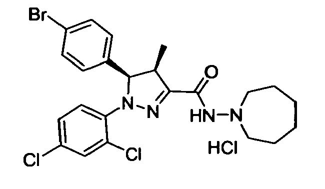

¹H NMR (300 MHz, DMSO-*d*₆) δ ppm 0.78 (d, *J*=7.18 Hz, 3 H) 1.58 (br. S., 4 H) 1.77 (br. S., 4 H) 3.33 (br. S., 4 H) 3.85 (dd, *J*=11.28, 7.18 Hz, 1 H) 5.94 (d, *J*=11.28 Hz, 1 H) 7.08 (d, *J*=8.35 Hz, 2 H) 7.34 (dd, *J*=8.72, 2.27 Hz, 1 H) 7.46 (d, *J*=8.35 Hz, 2 H) 7.52 (d, J=2.34 Hz, 1 H) 7.61 (d, *J*=8.64 Hz, 1 H) 10.98 (br. S., 1 H)
MS (M+H)⁺: 523

### Subgroup A6.1: Chemical Example 227: cis-N-(azepan-1-yl)-5-(4-fluorophenyl)-1-(2,4-dichlorophenyl)-4-methyl-4,5-dihydro-1H-pyrazole-3-carboxamide hydrochloride

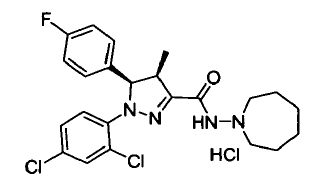

¹H NMR (300 MHz, DMSO-*d*₆) δ ppm 0.78 (d, *J*=7.32 Hz, 3 H) 1.59 (br. S., 4 H) 1.78 (br. S., 4 H) 3.36 (br. S., 4 H) 3.84 (dd, *J*=11.43, 7.32 Hz, 1 H) 5.96 (d, *J*=11.43 Hz, 1 H) 6.99 - 7.21 (m, 4 H) 7.33 (dd, *J*=8.79, 2.20 Hz, 1 H) 7.51 (d, J= 2.34 Hz, 1 H) 7.63 (d, *J*=8.79 Hz, 1 H), 11.06 (br. S., 1 H)
MS (M+H)⁺: 463

### Subgroup A6.1: Chemical Example 271:

### cis-N-(azepan-1-yl)-1-(2,4-dichlorophenyl)-5-(4-methoxyphenyl)-4-methyl-4,5-dihydro-1H-pyrazole-3-carboxamide hydrochloride

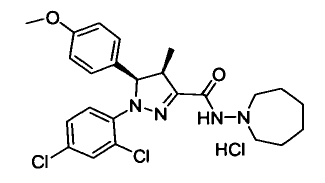

¹H NMR (300 MHz, DMSO-*d*₆) δ ppm 0.80 (d, *J*=7.18 Hz, 3 H) 1.59 (br. S., 4 H) 1.79 (br. S., 4 H) 3.38 (d, *J*=4.54 Hz, 4 H) 3.66 (s, 3 H) 3.80 (dd, *J*=11.28, 7.32 Hz, 1 H) 5.89 (d, *J*=11.28 Hz, 1 H) 6.78 (d, *J*=8.50 Hz, 2 H) 7.03 (d, *J*=8.50 Hz, 2 H) 7.32 (dd, *J*=8.79, 2.34 Hz, 1 H) 7.49 (d, J= 2.34 Hz, 1 H) 7.63 (d, *J*=8.79 Hz, 1 H) 11.12 (br. S., 1 H)
MS (M+H)⁺: 475

### Subgroup A6.1: Chemical Example 277: cis-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride

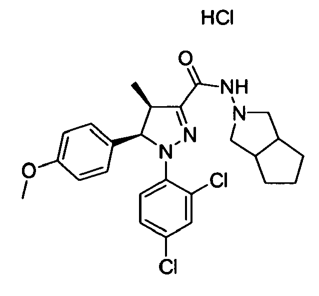

¹H NMR (300 MHz, CHLOROFORM-*d*) δ ppm 0.94 (d, *J*=7.32 Hz, 3 H) 1.78 (m, 6 H) 3.03 (br. S., 2 H) 3.74 (s, 3 H) 3.66 - 3.88 (m, 3 H) 3.99 (br. S., 2 H) 5.86 (d, *J*=11.86 Hz, 1 H) 6.75 (d, J=8.50 Hz, 2 H) 6.96 (d, *J*=8.50 Hz, 2 H) 7.12 (dd, *J*=8.64, 2.20 Hz, 1 H) 7.25 (d, *J*=2.20 Hz, 1 H) 7.33 (d, *J*=8.64 Hz, 1 H) 9.58 (br. S., 1 H)
MS (M+H)⁺: 487

### Subgroup A6.1: Chemical Example 343: cis-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride

Example compound 277 (0.82 mmol) was dissolved in 10 mL dichloromethane. A solution of 1 M BBr₃ (5 eq.) in dichloromethane was added at 0 °C and the reaction was stirred overnight at ambient temperature. The solid formed was filtered off and the solvent was evaporated. A solution of 2 N HCl/diethyl ether was added to form the hydrochloride.

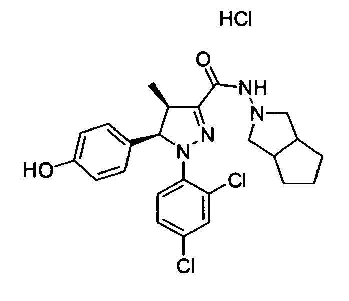

¹H NMR (300 MHz, METHANOL-*d*₄) δ ppm 0.95 (d, *J*=7.18 Hz, 3 H) 1.65 (br. S., 2 H) 1.79 (br. S., 4 H) 2.95 (br. S., 2 H) 3.11 (dd, *J*=16.48, 10.03 Hz, 2 H) 3.81 (dd, *J*=11.43, 7.18 Hz, 1 H) 4.03 (m, 2 H) 5.90 (d, *J*=11.43 Hz, 1 H) 6.64 (d, *J*=8.06 Hz, 2 H) 6.95 (d, *J*=8.06 Hz, 2 H) 7.20 (m, 1 H) 7.39 (d, *J*=2.20 Hz, 1 H) 7.42 (d, *J*=8.79 Hz, 1 H)
MS (M+H)⁺: 473

### Subgroup A6.1: Chemical Example 92: (+)-cis-5-(4-Chlorophenyl)-1-(2,4-dichlorophenyl)-4-methyl-4,5-dihydro-1H-pyrazole-3-carboxylic acid piperidin-1-ylamide hydrochloride

¹H NMR (400 MHz, METHANOL-*d*₄) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H)
[α]₅₈₉^{RT}= + 131.73

### Subgroup A6.1: Chemical Example 93: (-)-cis-5-(4-Chlorophenyl)-1-(2,4-dichlorophenyl)-4-methyl-4,5-dihydro-1H-pyrazole-3-carboxylic acid piperidin-1-ylamide hydrochloride

¹H NMR (400 MHz, METHANOL-*d*₄) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H)
[α]₅₈₉^{RT}= - 126.71

### Subgroup A6.1: Chemical Example 379: cis-5-(4-chlorophenyl)-1-(2,4-dichlorophenyl)-4-metyl-N-(piperidin-1-yl)-4,5-dihydro-1H-pyrazole-3-carbothioamide hydrochloride

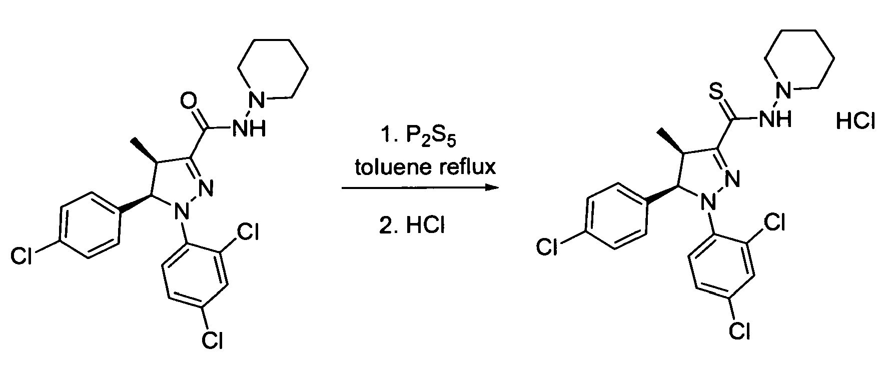

Phosphorous pentasulfide was added in portions (5 x 0.570 g, 12.9 mmol) to a solution of example compound 1 (6.46 mmol) in dry toluene (30 ml). The mixture was stirred and heated to reflux for 15 hours. The solid was filtered off and washed with dichloromethane. The solvent was evaporated and a bright yellow solid was isolated, which was then treated with aqueous ammonium solution and extracted with ethyl acetate, dried over Na₂SO₄ and filtered off. The solid obtained after evaporation of the solvent was dissolved in acetone and a solution of 2 N HCI in diethyl ether to form the corresponding hydrochloride. The pale yellow solid obtained by filtration was identified as *cis*-5-(4-chlorophenyl)-1-(2,4-dichlorophenyl)-4-methyl-N-(piperidin-1-yl)-4,5-dihydro-1H-pyrazole-3-carbothioamide hydrochloride (85% yield).

¹H NMR (300 MHz, METHANOL-*d*₄) δ ppm 0.95 (d, *J*=7.32 Hz, 3 H) 1.62 - 1.78 (m, 2 H) 2.00 (m, 4 H) 3.60 (m, 4 H) 3.99 (dd, *J*=10.84, 7.32 Hz, 1 H) 5.91 (d, *J*=10.84 Hz, 1 H) 7.22 (d, *J*=8.79 Hz, 1 H) 7.18 - 7.25 (m, 2 H) 7.25 - 7.31 (m, 2 H) 7.40 (d, *J*=8.79 Hz, 1 H) 7.45 (d, J=2.20 Hz, 1 H)
MS (M+H)⁺: 481

### Subgroup A6.1: Chemical Example 1: cis-5-(4-Chlorophenyl)-1-(2,4-dichlorophenyl)-4-methyl-4,5-dihydro-1H-pyrazole-3-carboxylic acid piperidin-1-ylamide hydrochloride

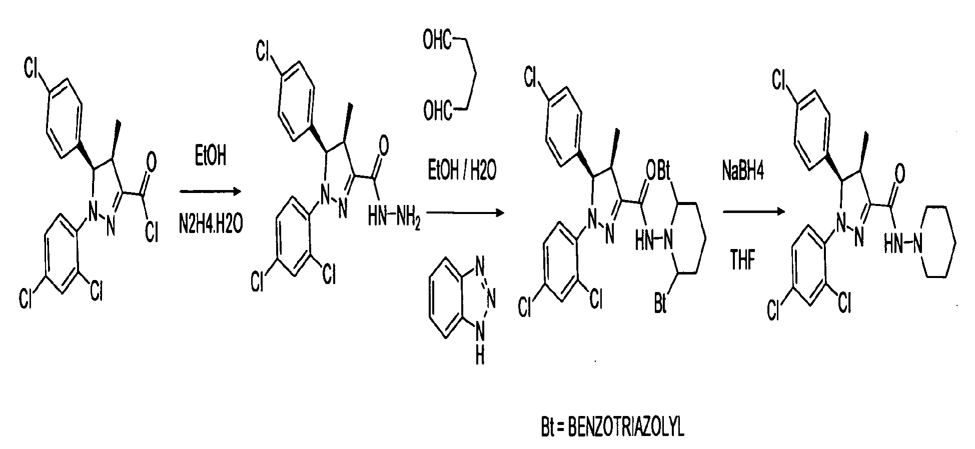

### Step 1: Preparation of cis-5-(4-Chloro-phenyl)-1-(2,4-dichlorophenyl)-4-methyl-4,5-dihydro-1H-pyrazole-3-carboxylic acid hydrazide

*cis-*5-(4-Chloro-phenyl)-1-(2,4-dichloro-phenyl)-4-methyl-4,5-dihydro-1H-pyrazole-3-carbonyl chloride (5.40 mmol) was refluxed for 2 hours in ethanol (25 mL). The reaction mixture was cooled to room temperature and 5 g of the hydrazine hydrate were added. The reaction mixture was heated to reflux for 4 hours, cooled to room temperature and the solvent was removed in vacuum. The resulting residue was taken up in dichloromethane (30 mL) and washed with water, dried and concentrated in vacuum. Chromatography with silica gel, eluting with Chloroform/ Methanol system of appropriate polarity, yielded the title compound (65% yield) as a white solid.

### Step 2: cis-5-(4-Chloro-phenyl)-1-(2,4-dichloro-phenyl)-4-methyl-4,5-dihydro-1H-pyrazole-3-carboxylic acid(2,6-benzotriazlyl-piperidin-1-yl)amide

Benzotriazole (119 mg, 1 mmol), *cis*-5-(4-Chloro-phenyl)-1-(2,4-dichloro-phenyl)-4-methyl-4,5-dihydro-1H-pyrazole-3-carboxylic acid hydrazide (0.5 mmol), water (5 mL) and ethanol (5 mL) were stirred for 10 min at room temperature. Glutaraldehyde (200 mg, 0.5 mmol, 25% aqueous solution) was slowly added to the reaction mixture, and stirring was continued overnight at room temperature. The solvent was removed and the insoluble products were filtered off, washed with water, and dried under vacuum, yielding the title compound (66% yield) as a white solid.

### Step 3: cis-5-(4-Chloro-phenyl)-1-(2,4-dichloro-phenyl)-4-methyl-4,5-dihydro-1H-pyrazole-3-carboxylic acid piperidin-1-ylamide

*cis*-5-(4-Chloro-phenyl)-(2,4-dichloro-phenyl)-4-methyl-4,5-dihydro-1H-pyrazole-3-carboxylic acid(2,6-benzotriazlyl-piperidin-1-yl)amide (0.33mmol) in tetrahydrofuran (10 mL) and sodium borohydride ( 38 mg, 1 mmol) were stirred at room temperature overnight. The reaction was quenched with water, and the product was extracted with dichloromethane, washed with water and dried. Solvent evaporation under reduced pressure led to the title compound. The product was purified by recrystallization from ethanol, yielding the title compound (41% yield) as a white solid.

### Subgroup A6.1: Chemical Examples

The following examples were prepared using the methods described above.

| **N°** | **STRUCTURE** | **Name** | **¹H-NMR** | **MS (M+H)⁺** |
|---|---|---|---|---|
| 1 | 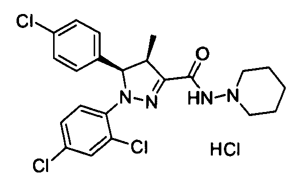 | *cis*-5-(4-Chloro-phenyl)-1-(2,4-dichloro-phenyl)-4-methyl-4,5-dihydro-1H-pyrazole-3-carboxylic acid piperidin-1-ylamide hydro-chloride | 1 H NMR (400 MHz, METHANOL-*d*₄*)* δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d,*J*=11.53Hz,1H)7.16(d, | 466 |
| 2 | 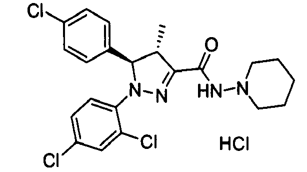 | trans-5-(4-Chloro-phenyl)-1-(2,4-dichloro-phenyl)-4-methyl-4,5-dihydro-1H-pyrazole-3-carboxylic acid piperidin-1-ylamide hydro-chloride | ¹H NMR (400 MHz, DMSO-d₆) δ ppm 1.38 (d, *J*=7.03 Hz, 3 H) 1.42 (m,2H)1.71 (m, 4 H) 3.12 (m, 4 H) 3.42 (dt, *J*=13.38, 5.86 Hz, 1 H) 5.36 (d, *J*=5.86 Hz, 1 H) 7.19 (d, *J*=8.21 Hz, 2 H) 7.31 (d, | 466 |
| 3 | 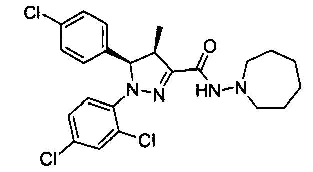 | *cis*-5-(4-Chloro-phenyl)-1-(2,4-dichloro-phenyl)-4-methyl-4,5-dihydro-1H-pyrazole-3-carboxylic acid azepan-1-ylamide | | |
| 4 | 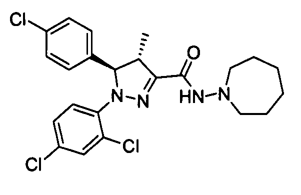 | *trans*-5-(4-Chloro-phenyl)-1-(2,4-dichloro-phenyl)-4-methyl-4,5-dihydro-1H-pyrazole-3-carboxylic acid azepan-1-ylamide | | |
| 5 | 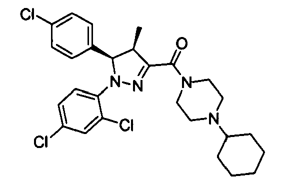 | cis-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone | | 533 |
| 6 | 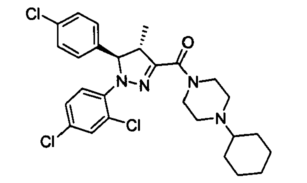 | *trans-*[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone | | 533 |
| 7 | 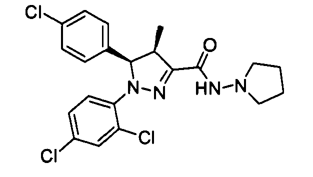 | *cis*-5-(4-Chloro-phenyl)-1-(2,4-dichloro-phenyl)-4-methyl-4,5-dihydro-1H-pyrazole-3-carboxylic acid pyrrolidin-1-ylamide | | 451 |
| 8 | 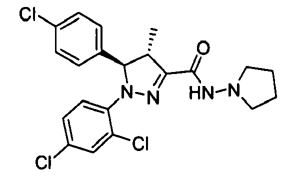 | trans-5-(4-Chloro-phenyl)-1-(2,4-dichloro-phenyl)-4-methyl-4,5-dihydro-1H-pyrazole-3-carboxylic acid pyrrolidin-1-ylamide | | 451 |
| 9 | 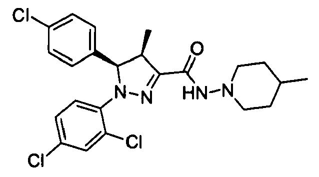 | *cis-*5-(4-Chloro-phenyl)-1-(2,4-dichloro-phenyl)-4-methyl-4,5-dihydro-1H-pyrazole-3-carboxylic acid (4-methyl-piperidin-1-yl)-amide | | 479 |
| 10 | 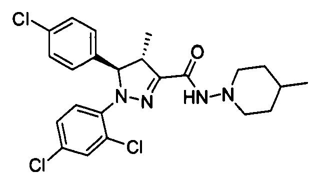 | *trans*-5-(4-Chloro-phenyl)-1-(2,4-dichloro-phenyl)-4-methyl-4,5-dihydro-1H-pyrazole-3-carboxylic acid (4-methyl-piperidin-1-yl)-amide | | 479 |
| 11 | 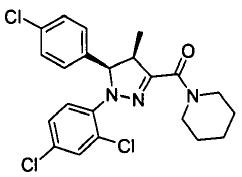 | *cis*-[5-(4-Chloro-phenyl)-1-(2,4-dichloro-phenyl)-4-methyl-4,5-dihydro-1H-pyrazol-3-yl]-piperidin-1-yl-methanone | | 450 |
| 12 | 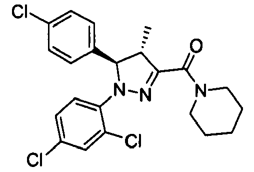 | trans-[5-(4-Chloro-phenyl)-1-(2,4-dichloro-phenyl)-4-methyl-4,5-dihydro-1H-pyrazol-3-yl]-piperidin-1-yl-methanone | | 450 |
| 13 | 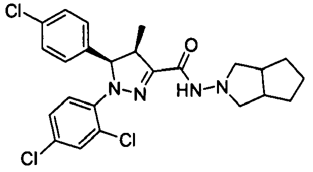 | cis-5-(4-Chloro-phenyl)-1-(2,4-dichloro-phenyl)-4-methyl-4,5-dihydro-1H-pyrazole-3-carboxylic acid (hexahydro-cyclopenta[c]pyrrol-2-yl)- | | 491 |
| 14 | 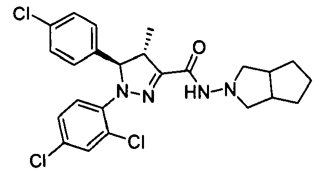 | *trans*-5-(4-Chloro-phenyl)-1-(2,4-dichloro-phenyl)-4-methyl-4,5-dihydro-1H-pyrazole-3-carboxylic acid (hexahydro-cyclopenta-[c]py-rrol-2-yl)-amide | | 491 |
| 15 | 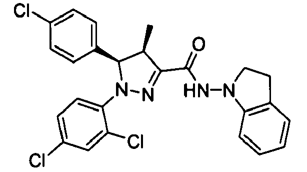 | *cis*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide | | 499 |
| 16 | 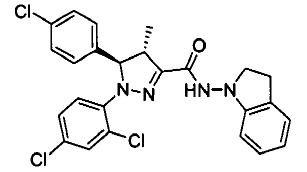 | *trans*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide | | 499 |
| 17 | 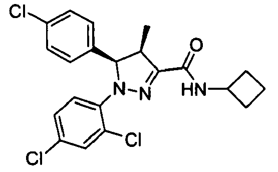 | *cis*-5-(4-Chloro-phenyl)-1-(2,4-dichloro-phenyl)-4-methyl-4,5-dihydro-1H-pyrazole-3-carboxylic acid cyclobutylamide | | 436 |
| 18 | 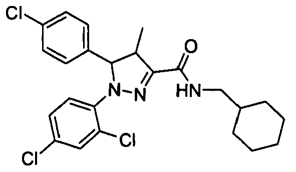 | 5-(4-Chloro-phenyl)-1-(2,4-dichloro-phenyl)-4-methyl-4,5-dihydro-1H-pyrazole-3-carboxylic acid cyclo-hexyl methyl-amide | | 478 |
| 19 | 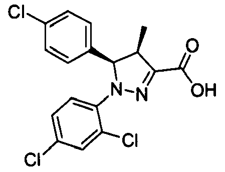 | *cis*-5-(4-Chloro-phenyl)-1-(2,4-dichloro-phenyl)-4-methyl-4,5-dihydro-1H-py-razole-3-carboxylic acid | 1 H NMR (400 MHz, CHLOROFORM-*d*) δ ppm 0.96 (d, *J*=7.42 Hz, 3 H) 3.82 (td, *J*=11.72, 7.42 Hz, 1 H) 5.91 (d, *J*=11.72 Hz, 1 H) 7.04 (d, *J*=8.60 Hz, 2 H) 7.11 (dd, *J*=8.60, 2.34 Hz, 1 H) 7.21 - | 384 |
| 20 | 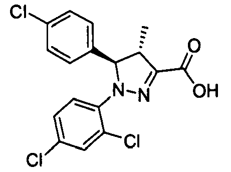 | trans-5-(4-Chloro-phenyl)-1-(2,4-dichloro-phenyl)-4-methyl-4,5-dihydro-1H-py-razole-3-carboxylic acid | 1 H NMR (400 MHz, CHLOROFORM-*d*) δ ppm 1.52 (d, J=7.03 Hz, 3 H) 3.52 (m, 1 H) 5.39 (d, *J*=5.47 Hz, 1 H) 7.08 (ddd, *J*=13.92, 5.62, 5.28 Hz, 3 H) 7.14 - 7.34 (m, 4 H) | 384 |
| 21 | 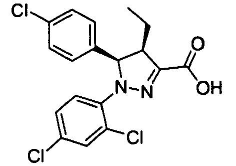 | cis-5-(4-Chloro-phenyl)-1-(2,4-dichloro-phenyl)-4-ethyl-4,5-dihydro-1H-pyrazole-3-carboxylic acid | | 398 |
| 22 | 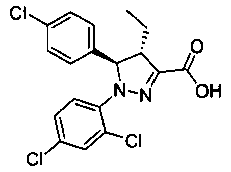 | trans-5-(4-Chloro-phenyl)-1-(2,4-dichloro-phenyl)-4-ethyl-4,5-dihydro-1H-pyrazole-3-carboxylic acid piperidin-1-ylamide | | 398 |
| 23 | 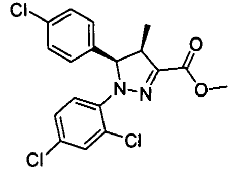 | cis-5-(4-Chloro-phenyl)-1-(2,4-dichloro-phenyl)-4-methyl-4,5-dihydro-1H-pyrazole-3-carboxylic acid methyl ester | 1 H NMR (400 MHz, CHLOROFORM-*d*) δ ppm 0.94 (d, J=7.32 Hz, 3 H) 3.80 (td, *J*=11.48, 7.32 Hz, 1 H) 3.88 (s, 3 H) 5.82 (d, *J*=11.48 Hz, 1 H) 7.04 (m, 2 H) 7.10 (dd, *J*=8.67, 2.32 Hz, 1 H) | 398 |
| 24 | 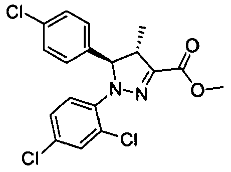 | *trans*-5-(4-Chloro-phenyl)-1-(2,4-dichloro-phenyl)-4-methyl-4,5-dihydro-1H-pyrazole-3-carboxylic acid methyl ester | 1 H NMR (400 MHz, CHLOROFORM-*d*) δ ppm 1.49 (d, J=7.32 Hz, 3 H) 3.50 (td, *J*=7.32 Hz, 5.60 1 H) 3.90 (s, 3 H) 5.35 (d, *J*=5.60 Hz, 1 H) 7.04 (m, 2 H) 7.10 (m, 1 H) 7.20-7.27 (m, 4 H) | 398 |
| 25 | 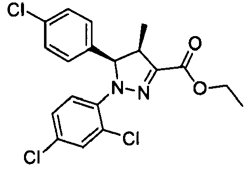 | cis-5-(4-Chloro-phenyl)-1-(2,4-dichloro-phenyl)-4-methyl-4,5-dihydro-1H-pyrazole-3-carboxylic acid ethyl ester | | 411 |
| 26 | 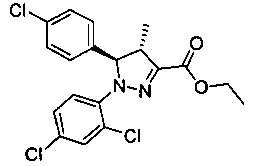 | trans-5-(4-Chloro-phenyl)-1-(2,4-dichloro-phenyl)-4-methyl-4,5-dihydro-1H-pyrazole-3-carboxylic acid ethyl ester | | 411 |
| 27 | 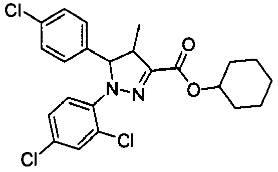 | 5-(4-Chloro-phenyl)-1-(2,4-dichloro-phenyl)-4-methyl-4,5-dihydro-1H-pyrazole-3-carboxylic acid cyclohe-xyl ester | | 465 |
| 28 | 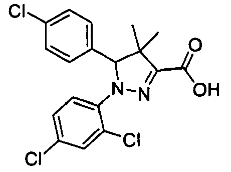 | 5-(4-Chloro-phenyl)-1-(2,4-dichloro-phenyl)-4,4-dimethyl-4,5-dihydro-1-H-pyrazole-3-carboxylic acid | | 397 |
| 29 | 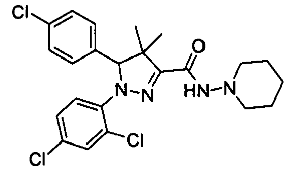 | 5-(4-Chloro-phenyl)-1-(2,4-dichloro-phenyl)-4,4-dimethyl-4,5-dihydro-1-H-pyrazole-3-carboxylic acid piperi- | | 479 |
| 30 | 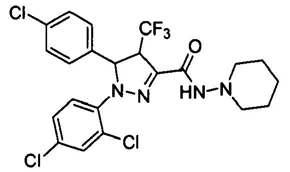 | 5-(4-Chloro-phenyl)-1-(2,4-dichloro-phenyl)-4-trifluoromethyl-4,5-dihydro-1H-pyrazole-3-carboxylic acid piperidin-1-ylamide | | 519 |
| 31 | 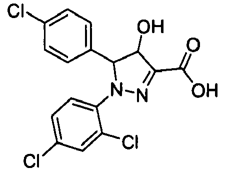 | 5-(4-Chloro-phenyl)-1-(2,4-dichloro-phenyl )-4-hyd roxy-4,5-dihydro-1H-pyrazole-3-carboxylic acid | | 385 |
| 32 | 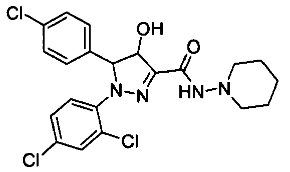 | 5-(4-Chloro-phenyl)-1-(2,4-dichloro-phenyl)-4-hydroxy-4,5-dihydro-1H-pyrazole-3-carboxylic acid piperidin-1-ylamide | | 467 |
| 33 | 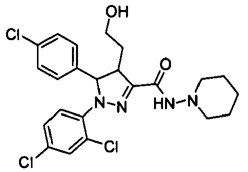 | 5-(4-Chloro-phenyl)-1-(2,4-dichloro-phenyl)-4-(2-hydroxy-ethyl)-4,5-di-hydro-1H-pyrazole-3-carboxylic acid piperidin-1- | | 495 |
| 34 | 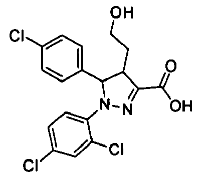 | 5-(4-Chloro-phenyl)-1-(2,4-dichloro-phenyl)-4-(2-hydroxy-ethyl)-4,5-di-hydro-1H-pyrazole-3-carboxylic acid | | 413 |
| 35 | 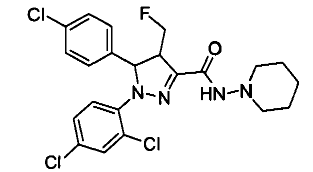 | 5-(4-Chloro-phenyl)-1-(2,4-dichloro-phenyl)-4-fluoromethyl-4,5-dihydro-1H-pyrazole-3-carboxylic acid piperidin-1-ylamide | | 483 |
| 36 | 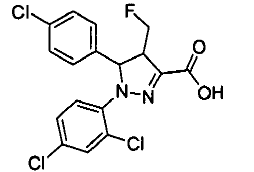 | 5-(4-Chloro-phenyl)-1-(2,4-dichloro-phenyl)-4-fluoromethyl-4,5-dihydro-1H-pyrazole-3-carboxylic acid | | 401 |
| 37 | 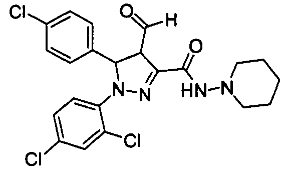 | 5-(4-Chloro-phenyl)-1-(2,4-dichloro-phenyl)-4-formyl-4,5-dihydro-1H-pyrazole-3-carboxylic acid piperidin-1-ylamide | | 479 |
| 38 | 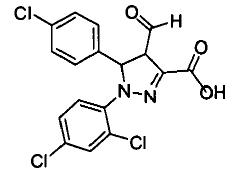 | 5-(4-Chloro-phenyl)-1-(2,4-dichloro-phenyl)-4-formyl-4,5-dihydro-1H-py-razole-3-carboxylic acid | | 397 |
| 39 | 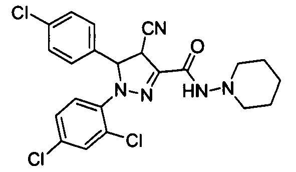 | 5-(4-chlorophenyl)-4-cyano-1-(2,4-dichlorophenyl)-N-(piperidin-1-yl)-4,5-dihydro-1H-pyrazole-3-carboxamide | | 476 |
| 40 | 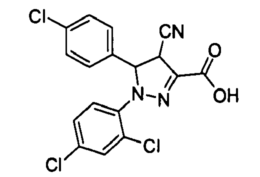 | 5-(4-Chloro-phenyl)-4-cyano-1-(2,4-dichloro-phenyl)-4,5-dihydro-1H-pyrazole-3-carboxylic acid | | 394 |
| 41 | 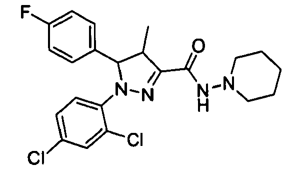 | 1-(2,4-Dichloro-phenyl)-5-(4-fluoro-phenyl)-4-methyl-4,5-dihydro-1H-pyrazole-3-carboxylic acid piperidin-1-ylamide | | 449 |
| 42 | 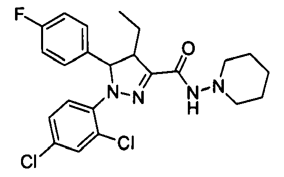 | 1-(2,4-Dichloro-phenyl)-4-ethyl-5-(4-fluoro-phenyl)-4,5-dihydro-1H-pyrazole-3-carboxylic acid piperidin-1-ylamide | | 463 |
| 43 | 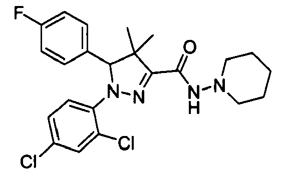 | 1-(2,4-Dichloro-phenyl)-5-(4-fluoro-phenyl)-4,4-dimethyl-4,5-dihydro-1-H-pyrazole-3-carboxylic acid piperidin-1-ylamide | | 463 |
| 44 | 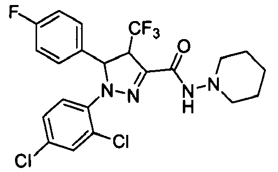 | 1-(2,4-Dichloro-phenyl)-5-(4-fluoro-phenyl)-4-trifluoromethyl-4,5-dihydro-1H-pyrazole-3-carboxylic acid piperidin-1-ylamide | | 503 |
| 45 | 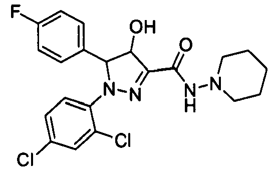 | 1-(2,4-Dichloro-phenyl)-5-(4-fluoro-phenyl)-4-hydroxy-4,5-dihydro-1H-pyrazole-3-carboxylic acid piperidin-1-ylamide | | 451 |
| 46 | 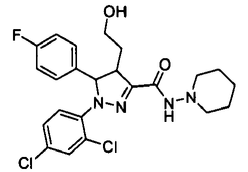 | 1-(2,4-Dichloro-phenyl)-5-(4-fluoro-phenyl)-4-(2-hydroxy-ethyl)-4,5-di-hydro-1H-pyrazole-3-carboxylic acid | | 479 |
| 47 | 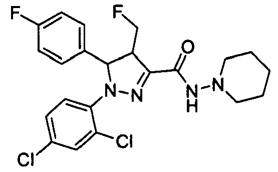 | 1-(2,4-Dichloro-phenyl)-4-fluoromethyl-5-(4-fluoro-phenyl)-4,5-dihydro-1H-pyrazole-3-carboxylic acid piperidin-1-ylamide | | 467 |
| 48 | 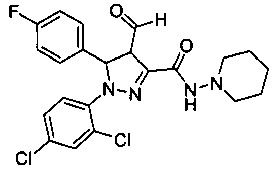 | 1-(2,4-Dichloro-phenyl)-5-(4-fluoro-phenyl)-4-formyl-4,5-dihydro-1H-pyrazole-3-carboxylic acid piperidin-1-ylamide | | 463 |
| 49 | 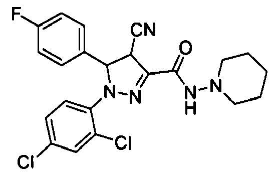 | 4-Cyano-1-(2,4-dichloro-phenyl)-5-(4-fluoro-phenyl)-4,5-dihydro-1H-pyrazole-3-carboxylic acid piperidin-1-ylamide | | 460 |
| 50 | 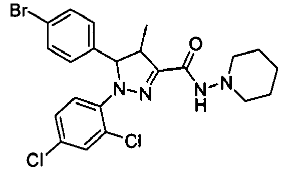 | 5-(4-Bromo-phenyl)-1-(2,4-dichloro-phenyl)-4-methyl-4,5-dihydro-1H-pyrazole-3-carboxylic acid piperidin-1-ylamide | | 509 |
| 51 | 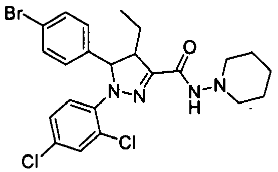 | 5-(4-Bromo-phenyl)-1-(2,4-dichloro-phenyl)-4-ethyl-4,5-dihydro-1H-pyrazole-3-carboxylic acid piperidin-1-ylamide | | 523 |
| 52 | 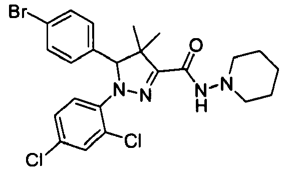 | 5-(4-Bromo-phenyl)-1-(2,4-dichloro-phenyl)-4,4-dimethyl-4,5-dihydro-1H-pyrazole-3-carboxylic acid piperidin-1-ylamide | | 523 |
| 53 | 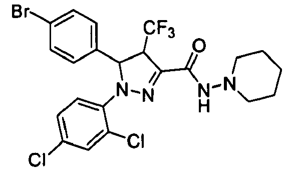 | 5-(4-Bromo-phenyl)-1-(2,4-dichloro-phenyl)-4-trifluoromethyl-4,5-dihydro-1H-pyrazole-3-carboxylic acid piperidin-1-ylamide | | 563 |
| 54 | 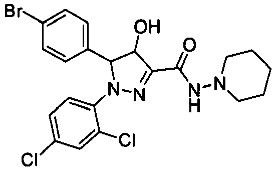 | 5-(4-Bromo-phenyl)-1-(2,4-dichloro-phenyl)-4-hydroxy-4,5-dihydro-1H-pyrazole-3-carboxylic acid piperidin-1-ylamide | | 511 |
| 55 | 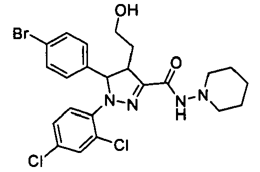 | 5-(4-Bromo-phenyl)-1-(2,4-dichloro-phenyl)-4-(2-hydroxy-ethyl)-4,5-dihydro-1H-pyrazole-3-carboxylic acid piperidin-1-ylamide | | 539 |
| 56 | 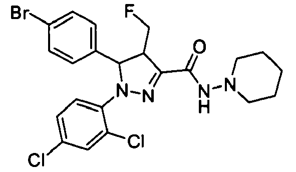 | 5-(4-Bromo-phenyl)-1-(2,4-dichloro-phenyl)-4-fluoromethyl-4,5-dihydro-1H-pyrazole-3-carboxylic acid piperidin-1-ylamide | | 527 |
| 57 | 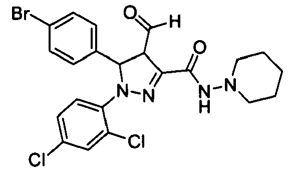 | 5-(4-Bromo-phenyl)-1-(2,4-dichloro-phenyl)-4-formyl-4,5-dihydro-1H-pyrazole-3-carboxylic acid piperidin-1-ylamide | | 523 |
| 58 | 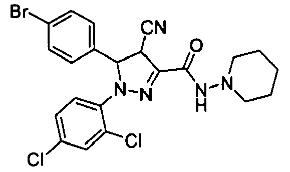 | 5-(4-Bromo-phenyl)-4-cyano-1-(2,4-dichloro-phenyl)-4,5-dihydro-1H-pyrazole-3-carboxylic acid piperidin-1-ylamide | | 520 |
| 59 | 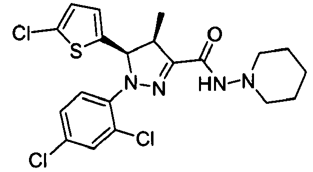 | cis-5-(5-Chloro-thiophen-2-yl)-1-(2,4-dichloro-phenyl)-4-methyl-4,5-dihydro-1H-pyrazole-3-carboxylic acid piperidin-1-ylamide | | 471 |
| 60 | 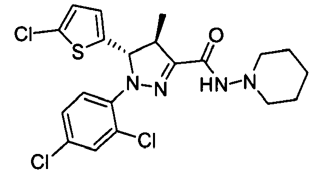 | *trans*-5-(5-Chloro-thiophen-2-yl)-1-(2,4-dichloro-phenyl)-4-methyl-4,5-dihydro-1H-pyrazole-3-carboxylic acid piperidin-1-ylamide | | 471 |
| 61 | 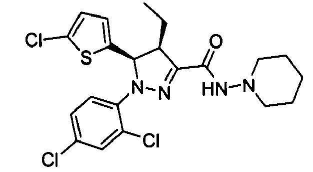 | *cis*-5-(5-Chloro-thiophen-2-yl)-1-(2,4-dichloro-phenyl)-4-ethyl-4,5-dihydro-1H-pyrazole-3-carboxylic acid piperidin-1-ylamide | | 485 |
| 62 | 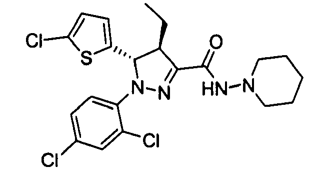 | *trans*-5-(5-Chloro-thiophen-2-yl)-1-(2,4-dichloro-phenyl)-4-ethyl-4,5-dihydro-1H-pyrazole-3-carboxylic acid piperidin-1-ylamide | | 485 |
| 63 | 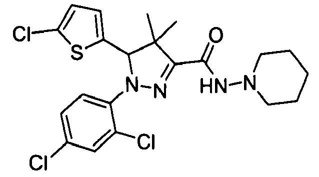 | 5-(5-Chloro-thiophen-2-yl)-1-(2,4-d ichloro-phenyl)-4,4-dimethyl-4,5-di hydro-1H-pyrazole-3-carboxylic acid | | 485 |
| 64 | 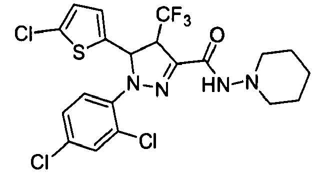 | 5-(5-Chloro-thiophen-2-yl)-1-(2,4-dichloro-phenyl)-4-trifluoromethyl-4,5-dihydro-1H-pyrazole-3-carboxylic | | 525 |
| 65 | 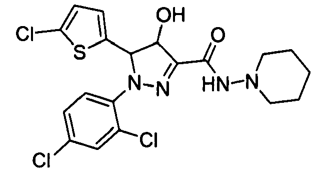 | 5-(5-Chloro-thiophen-2-yl)-1-(2,4-dichloro-phenyl)-4-hydroxy-4,5-dihydro-1H-pyrazole-3-carboxylic acid piperidin-1- | | 473 |
| 66 | 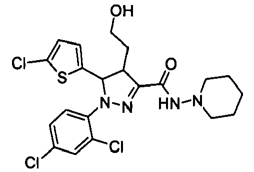 | 5-(5-Chloro-thiophen-2-yl)-1-(2,4-dichloro-phenyl)-4-(2-hydroxy-ethyl)-4,5-dihydro-1H-pyrazole-3-carboxylic acid piperidin-1- | | 501 |
| 67 | 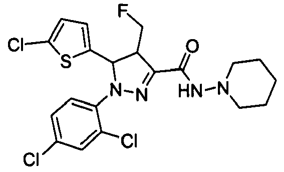 | 5-(5-Chloro-thiophen-2-yl)-1-(2,4-dichloro-phenyl)-4-fluoromethyl-4,5-dihydro-1H-pyrazole-3-carboxylic | | 489 |
| 68 | 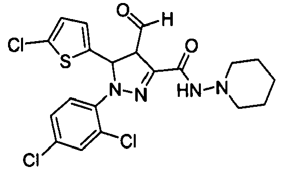 | 5-(5-Chloro-thiophen-2-yl)-1-(2,4-dichloro-phenyl)-4-formyl-4,5-dihydro-1H-pyrazole-3-carboxylic acid piperidin-1-ylamide | | 485 |
| 69 | 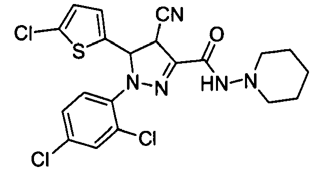 | 5-(5-Chloro-thiophen-2-yl)-4-cyano-1-(2,4-dichloro-phenyl)-4,5-dihydro-1H-pyrazole-3-carboxylic acid piperidin-1-ylamide | | 482 |
| 70 | 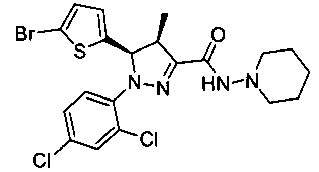 | *cis*-5-(5-Bromo-thiophen-2-yl)-1-(2,4-dichloro-phenyl)-4-methyl-4,5-dihydro-1H-pyrazole-3-carboxylic acid piperidin-1-ylamide | | 515 |
| 71 | 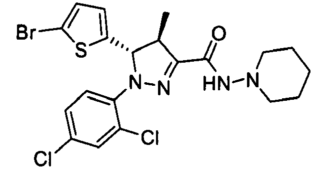 | *trans*-5-(5-Bromo-thiophen-2-yl)-1-(2,4-dichloro-phenyl)-4-methyl-4,5-dihydro-1H-pyrazole-3-carboxylic acid piperidin-1-ylamide | | 515 |
| 72 | 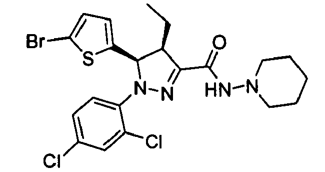 | *cis*-5-(5-Bromo-thiophen-2-yl)-1-(2,4-dichloro-phenyl)-4-ethyl-4,5-dihydro-1H-pyrazole-3-carboxylic acid piperidin-1-ylamide | | 529 |
| 73 | 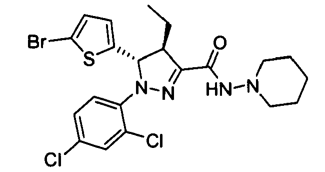 | *trans*-5-(5-Bromo-thiophen-2-yl)-1-(2,4-dichloro-phenyl)-4-ethyl-4,5-dihydro-1H-pyrazole-3-carboxylic acid piperidin-1-ylamide | | 529 |
| 74 | 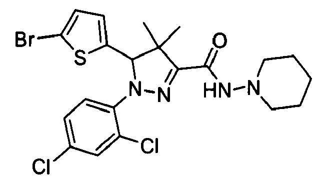 | 5-(5-Bromo-thiophen-2-yl)-1-(2,4-di-chloro-phenyl)-4,4-dimethyl-4,5-di-hydro-1H-pyrazole-3-carboxylic acid | | 529 |
| 75 | 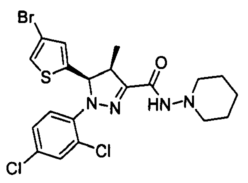 | *cis*-5-(4-Bromo-thiophen-2-yl)-1-(2,4-dichloro-phenyl)-4-methyl-4,5-dihydro-1H-pyrazole-3-carboxylic acid piperidin-1-ylamide | | 515 |
| 76 | 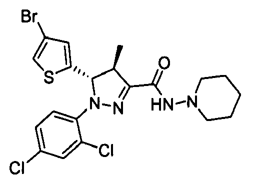 | *trans*-5-(4-Bromo-thiophen-2-yl)-1-(2,4-dichloro-phenyl)-4-methyl-4,5-dihydro-1H-pyrazole-3-carboxylic acid piperidin-1-ylamide | | 515 |
| 77 | 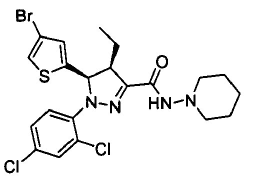 | *cis-*5-(4-Bromo-thiophen-2-yl)-1-(2,4-dichloro-phenyl)-4-ethyl-4,5-dihydro-1H-pyrazole-3-carboxylic acid piperidin-1-ylamide | | 529 |
| 78 | 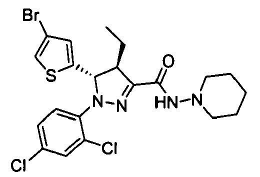 | t*rans*-5-(4-Bromo-thiophen-2-yl)-1-(2,4-dichloro-phenyl)-4-ethyl-4,5-dihydro-1H-pyrazole-3-carboxylic acid piperidin-1-ylamide | | 529 |
| 79 | 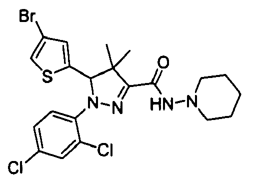 | 5-(4-Bromo-thiophen-2-yl)-1-(2,4-dichloro-phenyl)-4,4-dimethyl-4,5-dihydro-1H-pyrazole-3-carboxylic acid | | 529 |
| 80 | 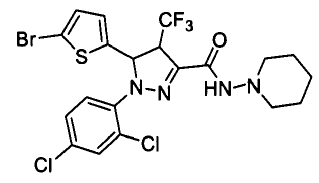 | 5-(5-Bromo-thiophen-2-yl)-1-(2,4-dichloro-phenyl)-4-trifluoromethyl-4,5-dihydro-1H-pyrazole-3-carboxylic acid piperidin-1-ylamide | | 569 |
| 81 | 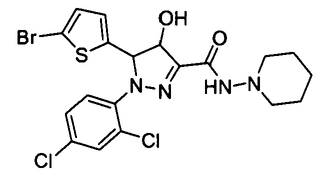 | 5-(5-Bromo-thiophen-2-yl)-1-(2,4-di-chloro-phenyl)-4-hydroxy-4,5-dihydro-1H-pyrazole-3-carboxylic acid piperidin-1-ylamide | | 517 |
| 82 | 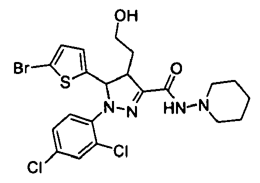 | 5-(5-Bromo-thiophen-2-yl)-1-(2,4-di-chloro-phenyl)-4-(2-hydroxy-ethyl)-4,5-dihydro-1H-pyrazole-3-carboxylic acid piperidin-1- | | 545 |
| 83 | 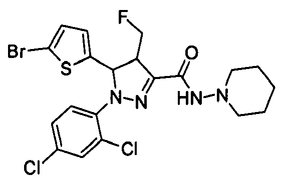 | 5-(5-Bromo-thiophen-2-yl)-1-(2,4-di-chloro-phenyl)-4-fluoromethyl-4,5-dihydro-1H-pyrazole-3-carboxylic acid piperidin-1- | | 533 |
| 84 | 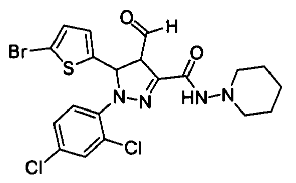 | 5-(5-Bromo-thiophen-2-yl)-1-(2,4-di-chloro-phenyl)-4-formyl-4,5-dihydro-1H-pyrazole-3-carboxylic acid piperidin-1-ylamide | | 529 |
| 85 | 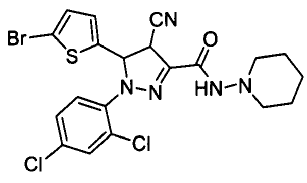 | 5-(5-Bromo-thiophen-2-yl)-4-cyano-1-(2,4-dichloro-phenyl)-4,5-dihydro-1H-pyrazole-3-carboxylic acid piperidin-1-ylamide | | 526 |
| 86 | 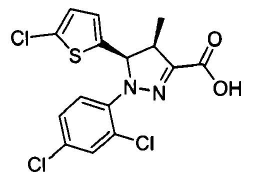 | cis-5-(5-Chloro-thiophen-2-yl)-1-(2,4-dichloro-phenyl)-4-methyl-4,5-dihydro-1H-pyrazole-3-carboxylic acid | | 389 |
| 87 | 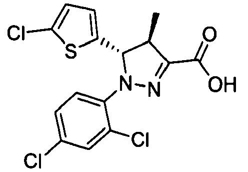 | *trans*-[5-(5-Chloro-thiophen-2-yl)-1-(2,4-dichloro-phenyl)-4-methyl-4,5-dihydro-1H-pyrazol-3-yl]-piperidin-1-yl-methanone | | 389 |
| 88 | 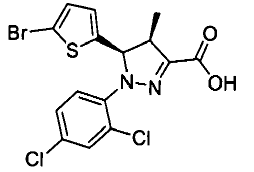 | *cis*-5-(5-Bromo-thiophen-2-yl)-1-(2,4-dichloro-phenyl)-4-methyl-4,5-dihydro-1H-pyrazole-3-carboxylic acid | | 433 |
| 89 | 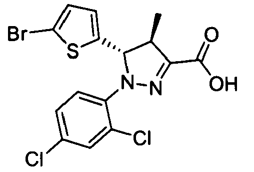 | trans-5-(5-Bromo-thiophen-2-yl)-1-(2,4-dichloro-phenyl)-4-methyl-4,5-dihydro-1H-pyrazole-3-carboxylic acid | | 433 |
| 90 | 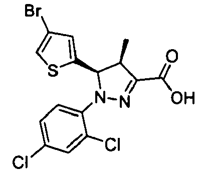 | *cis*-5-(4-Bromo-thiophen-2-yl)-1-(2,4-dichloro-phenyl)-4-methyl-4,5-dihydro-1H-pyrazole-3-carboxylic acid | | 433 |
| 91 | 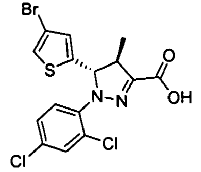 | *trans-*5-(4-Bromo-thiophen-2-yl)-1-(2,4-dichloro-phenyl)-4-methyl-4,5-dihydro-1H-pyrazole-3-carboxylic acid | | 433 |
| 446 | 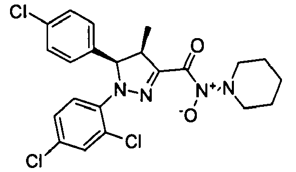 | *cis*-5-(4-chlorophenyl)-1-(2,4-dichlorophenyl)-4-methyl-N-(piperidin-1-yl)-4,5-dihydro-1H-pyrazole-3-carboxamide N-oxide | | 480 |
| 447 | 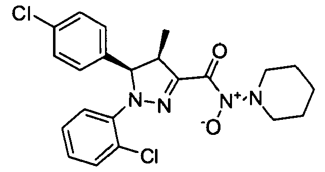 | *cis*-5-(4-chlorophenyl)-1-(2-chlorophenyl)-4-methyl-N-(piperidin-1-yl)-4,5-dihydro-1H-pyrazole-3-carboxamide N-oxide | | 446 |
| 448 | 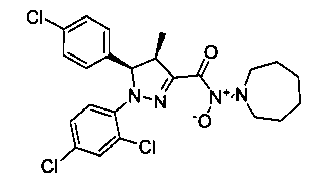 | *cis*-N-(azepan-1-yl)-5-(4-chlorophenyl)-1-(2,4-dichlorophenyl)-4-methyl-4,5-dihydro-1H-pyrazole-3-carboxamide N-oxide | | 494 |
| 449 | 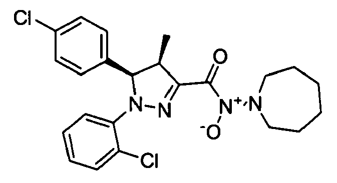 | *cis*-N-(azepan-1-yl)-5-(4-chlorophenyl)-1-(2-chlorophenyl)-4-methyl-4,5-dihydro-1H-pyrazole-3-carboxamide N-oxide | | 460 |
| 450 | 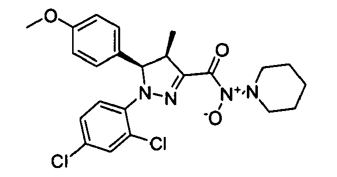 | *cis*-1-(2,4-dichlorophenyl)-5-(4-methoxyphenyl)-4-methyl-N-(piperidin-1-yl)-4,5-dihydro-1H-pyrazole-3-carboxamide N-oxide | | 476 |
| 451 | 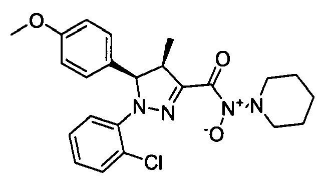 | *cis*-1-(2-chlorophenyl)-5-(4-methoxyphenyl)-4-methyl-N-(piperidin-1-yl)-4,5-dihydro-1H-pyrazole-3-carboxamide N-oxide | | 442 |

| | |
|---|---|
| 92 | (+)-cis-5-(4-chlorophenyl)-1-(2,4-dichlorophenyl)-4-methyl-N-(piperidin-1-yl)-4,5-dihydro-1H-pyrazole-3-carboxamide hydrochloride |
| 93 | (-)-cis-5-(4-chlorophenyl)-1-(2,4-dichlorophenyl)-4-methyl-N-(piperidin-1-yl)-4,5-dihydro-1H-pyrazole-3-carboxamide hydrochloride |
| 94 | *cis*-1-(2-chlorophenyl)-5-(4-chlorophenyl)-4-methyl-N-(piperidin-1-yl)-4,5-dihydro-1H-pyrazole-3-carboxamide |
| 95 | *trans*-1-(2-chlorophenyl)-5-(4-chlorophenyl)-4-methyl-N-(piperidin-1-yl)-4,5-dihydro-1H-pyrazole-3-carboxamide |
| 96 | *cis*-5-(4-Chlorophenyl)-1-(2,4-dichlorophenyl)-4-methyl-4,5-dihydro-1H-pyrazole-3-carboxylic acid azepan-1-ylamide hydrochloride |
| 97 | *trans*-5-(4-Chlorophenyl)-1-(2,4-dichloro-phenyl)-4-methyl-4,5-dihydro-1H-pyrazole-3-carboxylic acid azepan-1-ylamide hydrochloride |
| 98 | *cis*-N-(azepan-1-yl)-1-(2-chlorophenyl)-5-(4-chlorophenyl)-4-methyl-4,5-dihydro-1H-pyrazole-3-carboxamide |
| 99 | *trans*-N-(azepan-1-yl)-1-(2-chlorophenyl)-5-(4-chlorophenyl)-4-methyl-4,5-dihydro-1H-pyrazole-3-carboxamide |
| 100 | *cis*-5-(4-chlorophenyl)-N-(4-cyclopentylpiperazin-1-yl)-1-(2,4-dichlorophenyl)-4-methyl-4,5-dihydro-1H-pyrazole-3-carboxamide hydrochloride |
| 101 | *cis*-5-(4-chlorophenyl)-1-(2,4-dichlorophenyl)-4-methyl-N-morpholino-4,5-dihydro-1H-pyrazole-3-carboxamide |
| 102 | *cis*-1-(2-chlorophenyl)-5-(4-chlorophenyl)-4-methyl-N-(4-methylpiperidin-1-yl)-4,5-dihydro-1H-pyrazole-3-carboxamide |
| 103 | *cis*-5-(4-chlorophenyl)-1-(2,4-dichlorophenyl)-N-(2,6-dimethylpiperidin-1-yl)-4-methyl-4,5-dihydro-1H-pyrazole-3-carboxamide |
| 104 | *cis*-5-(4-Chlorophenyl)-1-(2,4-dichlorophenyl)-4-methyl-4,5-dihydro-1H-pyrazole-3-carboxylic acid (1,3-dioxo-1H,3H-benzo[de]isoquinolin-2-yl)-amide |
| 105 | *cis-*5-(4-Chlorophenyl)-1-(2,4-dichlorophenyl)-4-methyl-4,5-dihydro-1H-pyrazole-3-carboxylic acid (1 H,3H-benzo[de]isoquinolin-2-yl)-amide |
| 106 | *cis-*5-(4-chlorophenyl)-1-(2,4-dichlorophenyl)-N-(®-2-(methoxymethyl)pyrrolidin-1-yl)-4-methyl-4,5-dihydro-1H-pyrazole-3-carboxamide hydrochloride |
| 107 | *cis-*5-(4-chlorophenyl)-1-(2,4-dichlorophenyl)-N-((S)-2-(methoxymethyl)pyrrolidin-1-yl)-4-methyl-4,5-dihydro-1H-pyrazole-3-carboxamide hydrochloride |
| 108 | *cis-*5*-*(4-Chlorophenyl)-1-(2,4-dichlorophenyl)-4-methyl-4,5-dihydro-1H-pyrazole-3-carboxylic acid (hexahydro-cyclopenta[c]pyrrol-2-yl)-amide hydrochloride |
| 109 | *cis*-1-(2-chlorophenyl)-5-(4-chlorophenyl)-N-(hexahydrocyclopenta[c]pyrrol-2(1H)-yl)-4-methyl-4,5-dihydro-1H-pyrazole-3-carboxamide |
| 110 | *trans*-1-(2-chlorophenyl)-5-(4-chlorophenyl)-N-(hexahydrocyclopenta[c]pyrrol-2(1H)-yl)-4-methyl-4,5-dihydro-1H-pyrazole-3-carboxamide |
| 111 | *cis*-1-(2-chlorophenyl)-5-(4-chlorophenyl)-N-(indolin-1-yl)-4-methyl-4,5-dihydro-1H-pyrazole-3-carboxamide |
| 112 | *trans*-1-(2-chlorophenyl)-5-(4-chlorophenyl)-N-(indolin-1-yl)-4-methyl-4,5-dihydro-1H-pyrazole-3-carboxamide |
| 113 | *cis*-5-(4-chlorophenyl)-1-(2,4-dichlorophenyl)-4-methyl-N-(2-methylindolin-1-yl)-4,5-dihydro-1H-pyrazole-3-carboxamide |
| 114 | *cis*-5-(4-chlorophenyl)-N-(cyclohexylmethyl)-1-(2,4-dichlorophenyl)-4-methyl-4,5-dihydro-1H-pyrazole-3-carboxamide |
| 115 | *cis*-5-(4-chlorophenyl)-N-(cycloheptylmethyl)-1-(2,4-dichlorophenyl)-4-methyl-4,5-dihydro-1H-pyrazole-3-carboxamide |
| 116 | *cis*-5-(4-chlorophenyl)-N-cyclododecyl-1-(2,4-dichlorophenyl)-4-methyl-4,5-dihydro-1H-pyrazole-3-carboxamide |
| 117 | *cis*-5-(4-chlorophenyl)-1-(2,4-dichlorophenyl)-4-methyl-N-(1,2,3,4-tetrahydronaphthalen-1-yl)-4,5-dihydro-1H-pyrazole-3-carboxamide |
| 118 | *cis*-N-(4-tert-butylcyclohexyl)-5-(4-chlorophenyl)-1-(2,4-dichlorophenyl)-4-methyl-4,5-dihydro-1H-pyrazole-3-carboxamide |
| 119 | *cis*-5-(4-chlorophenyl)-N-cyclooctyl-1-(2,4-dichlorophenyl)-4-methyl-4,5-dihydro-1H-pyrazole-3-carboxamide |
| 120 | *cis-*N-(azocan-1-yl)-5-(4-chlorophenyl)-1-(2,4-dichlorophenyl)-4-methyl-4,5-dihydro-1H-pyrazole-3-carboxamide |
| 121 | *cis*-N-(azocan-1-yl)-1-(2-chlorophenyl)-5-(4-chlorophenyl)-4-methyl-4,5-dihydro-1H-pyrazole-3-carboxamide |
| 122 | *cis-*azocan-1-yl(cis-5-(4-chlorophenyl)-1-(2,4-dichlorophenyl)-4-methyl-4,5-dihydro-1H-pyrazol-3-yl)methanone |
| 123 | *cis*-5-(4-chlorophenyl)-N-cycloheptyl-1-(2,4-dichlorophenyl)-4-methyl-4,5-dihydro-1H-pyrazole-3-carboxamide |
| 124 | *cis*-1-(2-chlorophenyl)-5-(4-chlorophenyl)-N-cycloheptyl-4-methyl-4,5-dihydro-1H-pyrazole-3-carboxamide |
| 125 | *trans*-5-(4-chlorophenyl)-N-cycloheptyl-1-(2,4-dichlorophenyl)-4-methyl-4,5-dihydro-1H-pyrazole-3-carboxamide |
| 126 | *trans*-1-(2-chlorophenyl)-5-(4-chlorophenyl)-N-cycloheptyl-4-methyl-4,5-dihydro-1H-pyrazole-3-carboxamide |
| 127 | *cis*-5-(4-chlorophenyl)-N-(cyclohexylmethyl)-1-(2,4-dichlorophenyl)-4-methyl-4,5-dihydro-1H-pyrazole-3-carboxamide |
| 128 | (+)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide |
| 129 | (-)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide |
| 130 | (-)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide |
| 131 | (+)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide |
| 132 | (+)-cis-1-(2-chlorophenyl)-5-(4-chlorophenyl)-N-((1S,2S)-2-hydroxycyclohexyl)-4-methyl-4,5-dihydro-1H-pyrazole-3-carboxamide |
| 133 | (-)-cis-1-(2-chlorophenyl)-5-(4-chlorophenyl)-N-((1S,2S)-2-hydroxycyclohexyl)-4-methyl-4,5-dihydro-1H-pyrazole-3-carboxamide |
| 134 | *cis*-5-(4-chlorophenyl)-1-(2,4-dichlorophenyl)-N-(4-hydroxypiperidin-1-yl)-4-methyl-4,5-dihydro-1H-pyrazole-3-carboxamide |
| 135 | *cis*-1-(2-chlorophenyl)-5-(4-chlorophenyl)-N-(4-hydroxypiperidin-1-yl)-4-methyl-4,5-dihydro-1H-pyrazole-3-carboxamide |
| 136 | *cis*-5-(4-chlorophenyl)-1-(2,4-dichlorophenyl)-N-(3-hydroxypiperidin-1-yl)-4-methyl-4,5-dihydro-1H-pyrazole-3-carboxamide |
| 137 | *cis*-1-(2-chlorophenyl)-5-(4-chlorophenyl)-N-(3-hydroxypiperidin-1-yl)-4-methyl-4,5-dihydro-1H-pyrazole-3-carboxamide |
| 138 | *cis*-5-(4-chlorophenyl)-1-(2,4-dichlorophenyl)-N-(2-hydroxypiperidin-1-yl)-4-methyl-4,5-dihydro-1H-pyrazole-3-carboxamide |
| 139 | *cis*-1-(2-chlorophenyl)-5-(4-chlorophenyl)-N-(2-hydroxypiperidin-1-yl)-4-methyl-4,5-dihydro-1H-pyrazole-3-carboxamide |
| 140 | *cis*-5-(4-chlorophenyl)-1-(2,4-dichlorophenyl)-4-methyl-N-(4-oxopiperidin-1-yl)-4,5-dihydro-1H-pyrazole-3-carboxamide |
| 141 | *cis-*1-(2-chlorophenyl)-5-(4-chlorophenyl)-4-methyl-N-(4-oxopiperidin-1-yl)-4,5-dihydro-1H-pyrazole-3-carboxamide |
| 142 | *cis*-5-(4-chlorophenyl)-1-(2,4-dichlorophenyl)-4-methyl-N-(3-oxopiperidin-1-yl)-4,5-dihydro-1H-pyrazole-3-carboxamide |
| 143 | *cis-*1-(2-chlorophenyl)-5-(4-chlorophenyl)-4-methyl-N-(3-oxopiperidin-1-yl)-4,5-dihydro-1H-pyrazole-3-carboxamide |
| 144 | *cis*-5-(4-chlorophenyl)-1-(2,4-dichlorophenyl)-N-(3,4-dihydropyridin-1(2H)-yl)-4-methyl-4,5-dihydro-1H-pyrazole-3-carboxamide |
| 145 | *cis*-1-(2-chlorophenyl)-5-(4-chlorophenyl)-N-(3,4-dihydropyridin-1(2H)-yl)-4-methyl-4,5-dihydro-1H-pyrazole-3-carboxamide |
| 146 | *cis*-5-(4-chlorophenyl)-1-(2,4-dichlorophenyl)-N-(5,6-dihydropyridin-1(2H)-yl)-4-methyl-4,5-dihydro-1H-pyrazole-3-carboxamide |
| 147 | *cis*-1-(2-chlorophenyl)-5-(4-chlorophenyl)-N-(5,6-dihydropyridin-1(2H)-yl)-4-methyl-4,5-dihydro-1H-pyrazole-3-carboxamide |
| 148 | *cis*-1-(2-chlorophenyl)-5-(4-chlorophenyl)-4-methyl-4,5-dihydro-1H-pyrazole-3-carboxylic acid |
| 149 | *trans*-1-(2-chlorophenyl)-5-(4-chlorophenyl)-4-methyl-4,5-dihydro-1H-pyrazole-3-carboxylic acid |
| 150 | *cis*-5-(4-Chlorophenyl)-1-(2,4-dichlorophenyl)-4-methyl-4,5-dihydro-1H-py-razole-3-carboxylic acid cyclohexyl ester |
| 151 | N-(azepan-1-yl)-5-(4-chlorophenyl)-1-(2,4-dichlorophenyl)-4,4-dimethyl-4,5-dihydro-1H-pyrazole-3-carboxamide |
| 152 | 1-(2-chlorophenyl)-5-(4-chlorophenyl)-4,4-dimethyl-N-(piperidin-1-yl)-4,5-dihydro-1 H-pyrazole-3-carboxamide |
| 153 | N-(azepan-1-yl)-1-(2-chlorophenyl)-5-(4-chlorophenyl)-4,4-dimethyl-4,5-dihydro-1 H-pyrazole-3-carboxamide |
| 154 | *cis*-5-(4-Chlorophenyl)-1-(2,4-dichlorophenyl)-4-trifluoromethyl-4,5-dihydro-1H-pyrazole-3-carboxylic acid piperidin-1-ylamide |
| 155 | *trans*-5-(4-Chloro-phenyl)-1-(2,4-dichlorophenyl)-4-trifluoromethyl-4,5-dihydro-1H-pyrazole-3-carboxylicacid piperidin-1-ylamide |
| 156 | *cis*-N-(azepan-1-yl)-5-(4-chlorophenyl)-1-(2,4-dichlorophenyl)-4-(trifluoromethyl)-4,5-dihydro-1H-pyrazole-3-carboxamide |
| 157 | *trans*-N-(azepan-1-yl)-5-(4-chlorophenyl)-1-(2,4-dichlorophenyl)-4-(trifluoromethyl)-4,5-dihydro-1H-pyrazole-3-carboxamide |
| 158 | *cis*-1-(2-chlorophenyl)-5-(4-chlorophenyl)-N-(piperidin-1-yl)-4-(trifluoromethyl)-4,5-dihydro-1 H-pyrazole-3-carboxamide |
| 159 | *trans* 1-(2-chlorophenyl)-5-(4-chlorophenyl)-N-(piperidin-1-yl)-4-(trifluoromethyl)-4,5-dihydro-1H-pyrazole-3-carboxamide |
| 160 | 5-(4-chlorophenyl)-1-(2,4-dichlorophenyl)-3-(piperidin-1-ylcarbamoyl)-4,5-dihydro-1H-pyrazole-4-carboxylic acid |
| 161 | 5-(4-chlorophenyl)-1-(2,4-dichlorophenyl)-4,4-difluoro-N-(piperidin-1-yl)-4,5-dihydro-1H-pyrazole-3-carboxamide |
| 162 | cis-5-(4-chlorophenyl)-4-cyano-1-(2,4-dichlorophenyl)-N-(piperidin-1-yl)-4,5-dihydro-1 H-pyrazole-3-carboxamide |
| 163 | *cis*-N-(azepan-1-yl)-5-(4-chlorophenyl)-4-cyano-1-(2,4-dichlorophenyl)-4,5-dihydro-1H-pyrazole-3-carboxamide |
| 164 | cis-1-(2-chlorophenyl)-5-(4-chlorophenyl)-4-cyano-N-(piperidin-1-yl)-4,5-dihydro-1 H-pyrazole-3-carboxamide |
| 165 | *cis*-N-(azepan-1-yl)-1-(2-chlorophenyl)-5-(4-chlorophenyl)-4-cyano-4,5-dihydro-1H-pyrazole-3-carboxamide |
| 166 | trans-5-(4-chlorophenyl)-4-cyano-1-(2,4-dichlorophenyl)-N-(piperidin-1-yl)-4,5-dihydro-1 H-pyrazole-3-carboxamide |
| 167 | trans-N-(azepan-1-yl)-5-(4-chlorophenyl)-4-cyano-1-(2,4-dichlorophenyl)-4,5-dihydro-1 H-pyrazole-3-carboxamide |
| 168 | trans-1-(2-chlorophenyl)-5-(4-chlorophenyl)-4-cyano-N-(piperidin-1-yl)-4,5-dihydro-1 H-pyrazole-3-carboxamide |
| 169 | *cis*-1-(2-chlorophenyl)-5-(4-chlorophenyl)-4-ethyl-4,5-dihydro-1H-pyrazole-3-carboxylic acid |
| 170 | *trans*-1-(2-chlorophenyl)-5-(4-chlorophenyl)-4-ethyl-4,5-dihydro-1H-pyrazole-3-carboxylic acid |
| 171 | cis-5-(4-chlorophenyl)-1-(2,4-dichlorophenyl)-4-ethyl-N-(piperidin-1-yl)-4,5-dihydro-1 H-pyrazole-3-carboxamide |
| 172 | *trans*-5-(4-chlorophenyl)-1-(2,4-dichlorophenyl)-4-ethyl-N-(piperidin-1-yl)-4,5-dihydro-1H-pyrazole-3-carboxamide |
| 173 | *cis*-1-(2-chlorophenyl)-5-(4-chlorophenyl)-4-ethyl-N-(piperidin-1-yl)-4,5-dihydro-1H-pyrazole-3-carboxamide |
| 174 | *trans*-1-(2-chlorophenyl)-5-(4-chlorophenyl)-4-ethyl-N-(piperidin-1-yl)-4,5-dihydro-1H-pyrazole-3-carboxamide |
| 175 | *cis* -N-(azepan-1-yl)-5-(4-chlorophenyl)-1-(2,4-dichlorophenyl)-4-ethyl-4,5-dihydro-1H-pyrazole-3-carboxamide |
| 176 | *trans*-N-(azepan-1-yl)-5-(4-chlorophenyl)-1-(2,4-dichlorophenyl)-4-ethyl-4,5-dihydro-1H-pyrazole-3-carboxamide |
| 177 | *cis*-N-(azepan-1-yl)-1-(2-chlorophenyl)-5-(4-chlorophenyl)-4-ethyl-4,5-dihydro-1H-pyrazole-3-carboxamide |
| 178 | *trans*-N-(azepan-1-yl)-1-(2-chlorophenyl)-5-(4-chlorophenyl)-4-ethyl-4,5-dihydro-1H-pyrazole-3-carboxamide |
| 179 | *cis*-5-(4-chlorophenyl)-N-cycloheptyl-1-(2,4-dichlorophenyl)-4-ethyl-4,5-dihydro-1H-pyrazole-3-carboxamide |
| 180 | *cis*-1-(2-chlorophenyl)-5-(4-chlorophenyl)-N-cycloheptyl-4-ethyl-4,5-dihydro-1H-pyrazole-3-carboxamide |
| 181 | *cis*-5-(4-chlorophenyl)-1-(2,4-dichlorophenyl)-4-ethyl-N-(hexahydrocyclopenta[c]pyrrol-2(1H)-yl)-4,5-dihydro-1H-pyrazole-3-carboxamide |
| 182 | *cis*-1-(2-chlorophenyl)-5-(4-chlorophenyl)-4-ethyl-N-(hexahydrocyclopenta[c]pyrrol-2(1H)-yl)-4,5-dihydro-1H-pyrazole-3-carboxamide |
| 183 | *cis*-5-(4-chlorophenyl)-1-(2,4-dichlorophenyl)-4-ethyl-N-(indolin-1-yl)-4,5-dihydro-1H-pyrazole-3-carboxamide |
| 184 | *cis*-1-(2-chlorophenyl)-5-(4-chlorophenyl)-4-ethyl-N-(indolin-1-yl)-4,5-dihydro-1H-pyrazole-3-carboxamide |
| 185 | *cis*-5-(4-bromophenyl)-1-(2,4-dichlorophenyl)-4-ethyl-4,5-dihydro-1H-pyrazole-3-carboxylic acid |
| 186 | *trans*-5-(4-bromophenyl)-1-(2,4-dichlorophenyl)-4-ethyl-4,5-dihydro-1H-pyrazole-3-carboxylic acid |
| 187 | *cis*-5-(4-bromophenyl)-1-(2,4-dichlorophenyl)-4-ethyl-N-(piperidin-1-yl)-4,5-dihydro-1H-pyrazole-3-carboxamide |
| 188 | *trans*-5-(4-bromophenyl)-1-(2,4-dichlorophenyl)-4-ethyl-N-(piperidin-1-yl)-4,5-dihydro-1H-pyrazole-3-carboxamide |
| 189 | *cis*-5-(4-bromophenyl)-1-(2-chlorophenyl)-4-ethyl-N-(piperidin-1-yl)-4,5-dihydro-1H-pyrazole-3-carboxamide |
| 190 | *trans*-5-(4-bromophenyl)-1-(2-chlorophenyl)-4-ethyl-N-(piperidin-1-yl)-4,5-dihydro-1H-pyrazole-3-carboxamide |
| 191 | *cis*-N-(azepan-1-yl)-5-(4-bromophenyl)-1-(2,4-dichlorophenyl)-4-ethyl-4,5-dihydro-1H-pyrazole-3-carboxamide |
| 192 | *cis*-N-(azepan-1-yl)-5-(4-bromophenyl)-1-(2-chlorophenyl)-4-ethyl-4,5-dihydro-1H-pyrazole-3-carboxamide |
| 193 | *cis*-5-(4-bromophenyl)-N-cycloheptyl-1-(2,4-dichlorophenyl)-4-ethyl-4,5-dihydro-1H-pyrazole-3-carboxamide |
| 194 | *cis*-5-(4-bromophenyl)-1-(2-chlorophenyl)-N-cycloheptyl-4-ethyl-4,5-dihydro-1H-pyrazole-3-carboxamide |
| 195 | *cis*-5-(4-bromophenyl)-1-(2,4-dichlorophenyl)-4-ethyl-N-(hexahydrocyclopenta[c]pyrrol-2(1H)-yl)-4,5-dihydro-1H-pyrazole-3-carboxamide |
| 196 | *cis*-5-(4-bromophenyl)-1-(2-chlorophenyl)-4-ethyl-N-(hexahydrocyclopenta[c]pyrrol-2(1H)-yl)-4,5-dihydro-1H-pyrazole-3-carboxamide |
| 197 | *cis*-5-(4-bromophenyl)-1-(2,4-dichlorophenyl)-4-ethyl-N-(indolin-1-yl)-4,5-dihydro-1H-pyrazole-3-carboxamide |
| 198 | *cis-*5-(4-bromophenyl)-1-(2-chlorophenyl)-4-ethyl-N-(indolin-1-yl)-4,5-dihydro-1H-pyrazole-3-carboxamide |
| 199 | *cis*-5-(4-bromophenyl)-1-(2,4-dichlorophenyl)-4-methyl-4,5-dihydro-1H-pyrazole-3-carboxylic acid |
| 200 | *trans*-5-(4-bromophenyl)-1-(2,4-dichlorophenyl)-4-methyl-4,5-dihydro-1H-pyrazole-3-carboxylic acid |
| 201 | *cis-*5-(4-bromophenyl)-1-(2-chlorophenyl)-4-methyl-4,5-dihydro-1H-pyrazole-3-carboxylic acid |
| 202 | *trans* -5-(4-bromophenyl)-1-(2-chlorophenyl)-4-methyl-4,5-dihydro-1H-pyrazole-3-carboxylic acid |
| 203 | *cis*-5-(4-bromophenyl)-1-(2,4-dichlorophenyl)-4-methyl-N-(piperidin-1-yl)-4,5-dihydro-1H-pyrazole-3-carboxamide |
| 204 | *trans*-5-(4-bromophenyl)-1-(2,4-dichlorophenyl)-4-methyl-N-(piperidin-1-yl)-4,5-dihydro-1 H-pyrazole-3-carboxamide |
| 205 | *cis* 5-(4-bromophenyl)-1-(2-chlorophenyl)-4-methyl-N-(piperidin-1-yl)-4,5-dihydro-1H-pyrazole-3-carboxamide |
| 206 | *trans*-5-(4-bromophenyl)-1-(2-chlorophenyl)-4-methyl-N-(piperidin-1-yl)-4,5-dihydro-1 H-pyrazole-3-carboxamide |
| 207 | *cis*-N-(azepan-1-yl)-5-(4-bromophenyl)-1-(2,4-dichlorophenyl)-4-methyl-4,5-dihydro-1H-pyrazole-3-carboxamide hydrochloride |
| 208 | *trans-*N-(azepan-1-yl)-5-(4-bromophenyl)-1-(2,4-dichlorophenyl)-4-methyl-4,5-dihydro-1H-pyrazole-3-carboxamide hydrochloride |
| 209 | *cis*-N-(azepan-1-yl)-5-(4-bromophenyl)-1-(2-chlorophenyl)-4-methyl-4,5-dihydro-1H-pyrazole-3-carboxamide |
| 210 | *cis*-N-(azepan-1-yl)-5-(4-bromophenyl)-1-(2-chlorophenyl)-4-methyl-4,5-dihydro-1H-pyrazole-3-carboxamide |
| 211 | *cis*-5-(4-bromophenyl)-N-cycloheptyl-1-(2,4-dichlorophenyl)-4-methyl-4,5-dihydro-1H-pyrazole-3-carboxamide |
| 212 | *cis*-5-(4-bromophenyl)-1-(2-chlorophenyl)-N-cycloheptyl-4-methyl-4,5-dihydro-1H-pyrazole-3-carboxamide |
| 213 | *cis*-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride |
| 214 | *cis*-5-(4-bromophenyl)-1-(2-chlorophenyl)-N-(hexahydrocyclopenta[c]pyrrol-2(1H)-yl)-4-methyl-4,5-dihydro-1H-pyrazole-3-carboxamide |
| 215 | *cis*-5-(4-bromophenyl)-1-(2,4-dichlorophenyl)-N-(indolin-1-yl)-4-methyl-4,5-dihydro-1H-pyrazole-3-carboxamide |
| 216 | *cis*-5-(4-bromophenyl)-1-(2-chlorophenyl)-N-(indolin-1-yl)-4-methyl-4,5-dihydro-1H-pyrazole-3-carboxamide |
| 217 | (+)-cis-5-(4-bromophenyl)-1-(2,4-dichlorophenyl)-N-((1S,2S)-2-hydroxycyclohexyl)-4-methyl-4,5-dihydro-1H-pyrazole-3-carboxamide |
| 218 | (-)-cis-5-(4-bromophenyl)-1-(2,4-dichlorophenyl)-N-((1S,2S)-2-hydroxycyclohexyl)-4-methyl-4,5-dihydro-1H-pyrazole-3-carboxamide |
| 219 | (+)-cis-5-(4-bromophenyl)-1-(2-chlorophenyl)-N-((1S,2S)-2-hydroxycyclohexyl)-4-methyl-4,5-dihydro-1H-pyrazole-3-carboxamide |
| 220 | (-)-cis-5-(4-bromophenyl)-1-(2-chlorophenyl)-N-((1S,2S)-2-hydroxycyclohexyl)-4-methyl-4,5-dihydro-1H-pyrazole-3-carboxamide |
| 221 | *cis*-1-(2,4-dichlorophenyl)-5-(4-fluorophenyl)-4-methyl-4,5-dihydro-1H-pyrazole-3-carboxylic acid |
| 222 | *trans*-1-(2,4-dichlorophenyl)-5-(4-fluorophenyl)-4-methyl-4,5-dihydro-1H-pyrazole-3-carboxylic acid |
| 223 | *cis*-1-(2,4-dichlorophenyl)-5-(4-fluorophenyl)-4-methyl-N-(piperidin-1-yl)-4,5-dihydro-1H-pyrazole-3-carboxamide |
| 224 | *trans*-1-(2,4-dichlorophenyl)-5-(4-fluorophenyl)-4-methyl-N-(piperidin-1-yl)-4,5-dihydro-1H-pyrazole-3-carboxamide |
| 225 | *cis-*1-(2-chlorophenyl)-5-(4-fluorophenyl)-4-methyl-N-(piperidin-1-yl)-4,5-dihydro-1H-pyrazole-3-carboxamide |
| 226 | *trans*-1-(2-chlorophenyl)-5-(4-fluorophenyl)-4-methyl-N-(piperidin-1-yl)-4,5-dihydro-1H-pyrazole-3-carboxamide |
| 227 | *cis*-N-(azepan-1-yl)-1-(2,4-dichlorophenyl)-5-(4-fluorophenyl)-4-methyl-4,5-dihydro-1H-pyrazole-3-carboxamide hydrochloride |
| 228 | *trans*-N-(azepan-1-yl)-1-(2,4-dichlorophenyl)-5-(4-fluorophenyl)-4-methyl-4,5-dihydro-1H-pyrazole-3-carboxamide hydrochloride |
| 229 | *cis*-N-(azepan-1-yl)-1-(2-chlorophenyl)-5-(4-fluorophenyl)-4-methyl-4,5-dihydro-1H-pyrazole-3-carboxamide |
| 230 | *trans*-N-(azepan-1-yl)-1-(2-chlorophenyl)-5-(4-fluorophenyl)-4-methyl-4,5-dihydro-1H-pyrazole-3-carboxamide |
| 231 | *cis*-N-cycloheptyl-1-(2,4-dichlorophenyl)-5-(4-fluorophenyl)-4-methyl-4,5-dihydro-1H-pyrazole-3-carboxamide |
| 232 | *cis*-1-(2-chlorophenyl)-N-cycloheptyl-5-(4-fluorophenyl)-4-methyl-4,5-dihydro-1H-pyrazole-3-carboxamide |
| 233 | *cis*-1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride |
| 234 | *cis*-1-(2-chlorophenyl)-5-(4-fluorophenyl)-N-(hexahydrocyclopenta[c]pyrrol-2(1H)-yl)-4-methyl-4,5-dihydro-1H-pyrazole-3-carboxamide |
| 235 | *cis*-1-(2,4-dichlorophenyl)-5-(4-fluorophenyl)-N-(indolin-1-yl)-4-methyl-4,5-dihydro-1H-pyrazole-3-carboxamide |
| 236 | *cis*-1-(2-chlorophenyl)-5-(4-fluorophenyl)-N-(indolin-1-yl)-4-methyl-4,5-dihydro-1H-pyrazole-3-carboxamide |
| 237 | (+)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide |
| 238 | (-)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide |
| 239 | (+)-cis-1-(2-chlorophenyl)-5-(4-fluorophenyl)-N-((1S,2S)-2-hydroxycyclohexyl)-4-methyl-4,5-dihydro-1H-pyrazole-3-carboxamide |
| 240 | (-)-cis-1-(2-chlorophenyl)-5-(4-fluorophenyl)-N-((1S,2S)-2-hydroxycyclohexyl)-4-methyl-4,5-dihydro-1H-pyrazole-3-carboxamide |
| 241 | *cis*-1-(2,4-dichlorophenyl)-5-(4-iodophenyl)-4-methyl-4,5-dihydro-1H-pyrazole-3-carboxylic acid |
| 242 | *trans*-1-(2,4-dichlorophenyl)-5-(4-iodophenyl)-4-methyl-4,5-dihydro-1H-pyrazole-3-carboxylic acid |
| 243 | *cis-*1-(2,4-dichlorophenyl)-5-(4-iodophenyl)-4-methyl-N-(piperidin-1-yl)-4,5-dihydro-1H-pyrazole-3-carboxamide |
| 244 | *trans*-1-(2,4-dichlorophenyl)-5-(4-iodophenyl)-4-methyl-N-(piperidin-1-yl)-4,5-dihydro-1H-pyrazole-3-carboxamide |
| 245 | *cis*-1-(2-chlorophenyl)-5-(4-iodophenyl)-4-methyl-N-(piperidin-1-yl)-4,5-dihydro-1H-pyrazole-3-carboxamide |
| 246 | *trans*-1-(2-chlorophenyl)-5-(4-iodophenyl)-4-methyl-N-(piperidin-1-yl)-4,5-dihydro-1H-pyrazole-3-carboxamide |
| 247 | *cis-*N*-*(azepan-1-yl)-1-(2,4-dichlorophenyl)-5-(4-iodophenyl)-4-methyl-4,5-dihydro-1H-pyrazole-3-carboxamide |
| 248 | *trans*-N-(azepan-1-yl)-1-(2,4-dichlorophenyl)-5-(4-iodophenyl)-4-methyl-4,5-dihydro-1H-pyrazole-3-carboxamide |
| 249 | *cis*-N-(azepan-1-yl)-1-(2-chlorophenyl)-5-(4-iodophenyl)-4-methyl-4,5-dihydro-1H-pyrazole-3-carboxamide |
| 250 | *trans*-N-(azepan-1-yl)-1-(2-chlorophenyl)-5-(4-iodophenyl)-4-methyl-4,5-dihydro-1H-pyrazole-3-carboxamide |
| 251 | *cis-*N-cycloheptyl-1-(2,4-dichlorophenyl)-5-(4-iodophenyl)-4-methyl-4,5-dihydro-1H-pyrazole-3-carboxamide |
| 252 | *cis*-1-(2-chlorophenyl)-N-cycloheptyl-5-(4-iodophenyl)-4-methyl-4,5-dihydro-1H-pyrazole-3-carboxamide |
| 253 | *cis*-1-(2,4-dichlorophenyl)-N-(hexahydrocyclopenta[c]pyrrol-2(1H)-yl)-5-(4-iodophenyl)-4-methyl-4,5-dihydro-1H-pyrazole-3-carboxamide |
| 254 | *cis*-1-(2-chlorophenyl)-N-(hexahydrocyclopenta[c]pyrrol-2(1H)-yl)-5-(4-iodophenyl)-4-methyl-4,5-dihydro-1H-pyrazole-3-carboxamide |
| 255 | *cis*-1-(2,4-dichlorophenyl)-N-(indolin-1-yl)-5-(4-iodophenyl)-4-methyl-4,5-dihydro-1H-pyrazole-3-carboxamide |
| 256 | *cis*-1-(2-chlorophenyl)-N-(indolin-1-yl)-5-(4-iodophenyl)-4-methyl-4,5-dihydro-1H-pyrazole-3-carboxamide |
| 257 | (+)-cis-1-(2,4-dichlorophenyl)-N-((1S,2S)-2-hydroxycyclohexyl)-5-(4-iodophenyl)-4-methyl-4,5-dihydro-1H-pyrazole-3-carboxamide |
| 258 | (-)-cis-1-(2,4-dichlorophenyl)-N-((1S,2S)-2-hydroxycyclohexyl)-5-(4-iodophenyl)-4-methyl-4,5-dihydro-1H-pyrazole-3-carboxamide |
| 259 | (+)-cis-1-(2-chlorophenyl)-N-((1S,2S)-2-hydroxycyclohexyl)-5-(4-iodophenyl)-4-methyl-4,5-dihydro-1H-pyrazole-3-carboxamide |
| 260 | (-)-cis-1-(2-chlorophenyl)-N-((1S,2S)-2-hydroxycyclohexyl)-5-(4-iodophenyl)-4-methyl-4,5-dihydro-1H-pyrazole-3-carboxamide |
| 261 | *cis*-1-(2,4-dichlorophenyl)-5-(4-methoxyphenyl)-4-methyl-4,5-dihydro-1H-pyrazole-3-carboxylic acid |
| 262 | *trans*-1-(2,4-dichlorophenyl)-5-(4-methoxyphenyl)-4-methyl-4,5-dihydro-1H-pyrazole-3-carboxylic acid |
| 263 | *cis-1-(2-chlorophenyl)-5-(4-methoxyphenyl)-4-methyl-4,5-dihydro-1H-pyrazole-3-carboxylic acid* |
| 264 | trans-1-(2-chlorophenyl)-5-(4-methoxyphenyl)-4-methyl-4,5-dihydro-1H-pyrazole-3-carboxylic acid |
| 265 | cis-1-(2,4-dichlorophenyl)-5-(4-methoxyphenyl)-4-methyl-N-(piperidin-1-yl)-4,5-dihydro-1H-pyrazole-3-carboxamide hydrochloride |
| 266 | (+)-cis-1-(2,4-dichlorophenyl)-5-(4-methoxyphenyl)-4-methyl-N-(piperidin-1-yl)-4,5-dihydro-1 H-pyrazole-3-carboxamide hydrochloride |
| 267 | (-)-cis-1-(2,4-dichlorophenyl)-5-(4-methoxyphenyl)-4-methyl-N-(piperidin-1-yl)-4,5-dihydro-1H-pyrazole-3-carboxamide hydrochloride |
| 268 | *trans*-1-(2,4-dichlorophenyl)-5-(4-methoxyphenyl)-4-methyl-N-(piperidin-1-yl)-4,5-dihydro-1H-pyrazole-3-carboxamide |
| 269 | *cis*-1-(2-chlorophenyl)-5-(4-methoxyphenyl)-4-methyl-N-(piperidin-1-yl)-4,5-dihydro-1H-pyrazole-3-carboxamide |
| 270 | *trans*-1-(2-chlorophenyl)-5-(4-methoxyphenyl)-4-methyl-N-(piperidin-1-yl)-4,5-dihydro-1H-pyrazole-3-carboxamide |
| 271 | *cis*-N-(azepan-1-yl)-1-(2,4-dichlorophenyl)-5-(4-methoxyphenyl)-4-methyl-4,5-dihydro-1H-pyrazole-3-carboxamide hydrochloride |
| 272 | *trans*-N-(azepan-1-yl)-1-(2,4-dichlorophenyl)-5-(4-methoxyphenyl)-4-methyl-4,5-dihydro-1H-pyrazole-3-carboxamide hydrochloride |
| 273 | *cis*-N-(azepan-1-yl)-1-(2-chlorophenyl)-5-(4-methoxyphenyl)-4-methyl-4,5-dihydro-1H-pyrazole-3-carboxamide |
| 274 | *trans*-N-(azepan-1-yl)-1-(2-chlorophenyl)-5-(4-methoxyphenyl)-4-methyl-4,5-dihydro-1H-pyrazole-3-carboxamide |
| 275 | *cis*-N-cycloheptyl-1-(2,4-dichlorophenyl)-5-(4-methoxyphenyl)-4-methyl-4,5-dihydro-1H-pyrazole-3-carboxamide |
| 276 | *cis*-1-(2-chlorophenyl)-N-cycloheptyl-5-(4-methoxyphenyl)-4-methyl-4,5-dihydro-1H-pyrazole-3-carboxamide |
| 277 | *cis*-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride |
| 278 | *cis*-1-(2-chlorophenyl)-N-(hexahydrocyclopenta[c]pyrrol-2(H)-yl)-5-(4-methoxyphenyl)-4-methyl-4,5-dihydro-1H-pyrazole-3-carboxamide |
| 279 | *cis*-1-(2,4-dichlorophenyl)-N-(indolin-1-yl)-5-(4-methoxyphenyl)-4-methyl-4,5-dihydro-1H-pyrazole-3-carboxamide |
| 280 | *cis*-1-(2-chlorophenyl)-N-(indolin-1-yl)-5-(4-methoxyphenyl)-4-methyl-4,5-dihydro-1H-pyrazole-3-carboxamide |
| 281 | (+)-cis-1-(2,4-dichlorophenyl)-N-((1S,2S)-2-hydroxycyclohexyl)-5-(4-methoxyphenyl)-4-methyl-4,5-dihydro-1 H-pyrazole-3-carboxamide |
| 282 | (-)-cis-1-(2,4-dichlorophenyl)-N-((1S,2S)-2-hydroxycyclohexyl)-5-(4-methoxyphenyl)-4-methyl-4,5-dihydro-1 H-pyrazole-3-carboxamide |
| 283 | (+)-cis-1-(2-chlorophenyl)-N-((1S,2S)-2-hydroxycyclohexyl)-5-(4-methoxyphenyl)-4-methyl-4,5-dihydro-1 H-pyrazole-3-carboxamide |
| 284 | (-)-cis-1-(2-chlorophenyl)-N-((1S,2S)-2-hydroxycyclohexyl)-5-(4-methoxyphenyl)-4-methyl-4,5-dihydro-1H-pyrazole-3-carboxamide |
| 285 | *cis*-1-(2,4-dichlorophenyl)-N-(4-hydroxypiperidin-1-yl)-5-(4-methoxyphenyl)-4-methyl-4,5-dihydro-1H-pyrazole-3-carboxamide |
| 286 | *cis-*1-(2-chlorophenyl)-N-(4-hydroxypiperidin-1-yl)-5-(4-methoxyphenyl)-4-methyl-4,5-dihydro-1H-pyrazole-3-carboxamide |
| 287 | *cis-*1-(2,4-dichlorophenyl)-N-(3-hydroxypiperidin-1-yl)-5-(4-methoxyphenyl)-4-methyl-4,5-dihydro-1H-pyrazole-3- |
| | carboxamide |
| 288 | *cis*-1-(2-chlorophenyl)-N-(3-hydroxypiperidin-1-yl)-5-(4-methoxyphenyl)-4-methyl-4,5-dihydro-1 H-pyrazole-3-carboxamide |
| 289 | *cis*-1-(2,4-dichlorophenyl)-N-(2-hydroxypiperidin-1-yl)-5-(4-methoxyphenyl)-4-methyl-4,5-dihydro-1H-pyrazole-3-carboxamide |
| 290 | *cis*-1-(2-chlorophenyl)-N-(2-hydroxypiperidin-1-yl)-5-(4-methoxyphenyl)-4-methyl-4,5-dihydro-1 H-pyrazole-3-carboxamide |
| 291 | *cis*-1-(2,4-dichlorophenyl)-5-(4-methoxyphenyl)-4-methyl-N-(4-oxopiperidin-1-yl)-4,5-dihydro-1 H-pyrazole-3-carboxamide |
| 292 | *cis*-1-(2-chlorophenyl)-5-(4-methoxyphenyl)-4-methyl-N-(4-oxopiperidin-1-yl)-4,5-dihydro-1H-pyrazole-3-carboxamide |
| 293 | *cis*-1-(2,4-dichlorophenyl)-5-(4-methoxyphenyl)-4-methyl-N-(3-oxopiperidin-1-yl)-4,5-dihydro-1 H-pyrazole-3-carboxamide |
| 294 | *cis*-1-(2-chlorophenyl)-5-(4-methoxyphenyl)-4-methyl-N-(3-oxopiperidin-1-yl)-4,5-dihydro-1H-pyrazole-3-carboxamide |
| 295 | *cis*-1-(2,4-dichlorophenyl)-N-(3,4-dihydropyridin-1(2H)-yl)-5-(4-methoxyphenyl)-4-methyl-4,5-dihydro-1H-pyrazole-3-carboxamide |
| 296 | *cis*-1-(2-chlorophenyl)-N-(3,4-dihydropyridin-1(2H)-yl)-5-(4-methoxyphenyl)-4-methyl-4,5-dihydro-1H-pyrazole-3-carboxamide |
| 297 | *cis*-1-(2,4-dichlorophenyl)-N-(5,6-dihydropyridin-1(2H)-yl)-5-(4-methoxyphenyl)-4-methyl-4,5-dihydro-1H-pyrazole-3-carboxamide |
| 298 | *cis*-1-(2-chlorophenyl)-N-(5,6-dihydropyridin-1(2H)-yl)-5-(4-methoxyphenyl)-4-methyl-4,5-dihydro-1H-pyrazole-3-carboxamide |
| 299 | *cis*-1-(2,4-dichlorophenyl)-5-(4-methoxyphenyl)-N-(piperidin-1-yl)-4-(trifluoromethyl)-4,5-dihydro-1H-pyrazole-3-carboxamide |
| 300 | *trans*-1-(2,4-dichlorophenyl)-5-(4-methoxyphenyl)-N-(piperidin-1-yl)-4-(trifluoromethyl)-4,5-dihydro-1H-pyrazole-3-carboxamide |
| 301 | *cis*-N-(azepan-1-yl)-1-(2,4-dichlorophenyl)-5-(4-methoxyphenyl)-4-(trifluoromethyl)-4,5-dihydro-1H-pyrazole-3-carboxamide |
| 302 | *trans*-N-(azepan-1-yl)-1-(2,4-dichlorophenyl)-5-(4-methoxyphenyl)-4-(trifluoromethyl)-4,5-dihydro-1H-pyrazole-3-carboxamide |
| 303 | *cis*-1-(2-chlorophenyl)-5-(4-methoxyphenyl)-N-(piperidin-1-yl)-4-(trifluoromethyl)-4,5-dihydro-1H-pyrazole-3-carboxamide |
| 304 | *trans*-1-(2-chlorophenyl)-5-(4-methoxyphenyl)-N-(piperidin-1-yl)-4-(trifluoromethyl)-4,5-dihydro-1H-pyrazole-3-carboxamide |
| 305 | 1-(2,4-dichlorophenyl)-5-(4-methoxyphenyl)-4,4-dimethyl-N-(piperidin-1-yl)-4,5-dihydro-1H-pyrazole-3-carboxamide |
| 306 | N-(azepan-1-yl)-1-(2,4-dichlorophenyl)-5-(4-methoxyphenyl)-4,4-dimethyl-4,5-dihydro-1H-pyrazole-3-carboxamide |
| 307 | 1-(2-chlorophenyl)-5-(4-methoxyphenyl)-4,4-dimethyl-N-(piperidin-1-yl)-4,5-dihydro-1H-pyrazole-3-carboxamide |
| 308 | 1-(2,4-dichlorophenyl)-4-formyl-5-(4-methoxyphenyl)-N-(piperidin-1-yl)-4,5-dihydro-1H-pyrazole-3-carboxamide |
| 309 | 1-(2,4-dichlorophenyl)-5-(4-methoxyphenyl)-3-(piperidin-1-ylcarbamoyl)-4,5-dihydro-1H-pyrazole-4-carboxylic acid |
| 310 | 1-(2,4-dichlorophenyl)-4-(2-hydroxyethyl)-5-(4-methoxyphenyl)-N-(piperidin-1-yl)-4,5-dihydro-1H-pyrazole-3-carboxamide |
| 311 | 1-(2,4-dichlorophenyl)-4-(fluoromethyl)-5-(4-methoxyphenyl)-N-(piperidin-1-yl)-4,5-dihydro-1H-pyrazole-3-carboxamide |
| 312 | *cis*-4-cyano-1-(2,4-dichlorophenyl)-5-(4-methoxyphenyl)-N-(piperidin-1-yl)-4,5-dihydro-1H-pyrazole-3-carboxamide |
| 313 | *trans-*4-cyano-1-(2,4-dichlorophenyl)-5-(4-methoxyphenyl)-N-(piperidin-1-yl)-4,5-dihydro-1H-pyrazole-3-carboxamide |
| 314 | *cis*-N-(azepan-1-yl)-4-cyano-1-(2,4-dichlorophenyl)-5-(4-methoxyphenyl)-4,5-dihydro-1H-pyrazole-3-carboxamide |
| 315 | *trans*-N-(azepan-1-yl)-4-cyano-1-(2,4-dichlorophenyl)-5-(4-methoxyphenyl)-4,5-dihydro-1H-pyrazole-3-carboxamide |
| 316 | *cis*-1-(2-chlorophenyl)-4-cyano-5-(4-methoxyphenyl)-N-(piperidin-1-yl)-4,5-dihydro-1H-pyrazole-3-carboxamide |
| 317 | *trans*-1-(2-chlorophenyl)-4-cyano-5-(4-methoxyphenyl)-N-(piperidin-1-yl)-4,5-dihydro-1H-pyrazole-3-carboxamide |
| 318 | 1-(2,4-dichlorophenyl)-4-hydroxy-5-(4-methoxyphenyl)-N-(piperidin-1-yl)-4,5-dihydro-1H-pyrazole-3-carboxamide |
| 319 | 1-(2-chlorophenyl)-4,4-difluoro-5-(4-methoxyphenyl)-N-(piperidin-1-yl)-4,5-dihydro-1H-pyrazole-3-carboxamide |
| 320 | 1-(2-chlorophenyl)-4-formyl-5-(4-methoxyphenyl)-N-(piperidin-1-yl)-4,5-dihydro-1H-pyrazole-3-carboxamide |
| 321 | 1-(2-chlorophenyl)-5-(4-methoxyphenyl)-3-(piperidin-1-ylcarbamoyl)-4,5-dihydro-1H-pyrazole-4-carboxylic acid |
| 322 | 1-(2-chlorophenyl)-4-(2-hydroxyethyl)-5-(4-methoxyphenyl)-N-(piperidin-1-yl)-4,5-dihydro-1 H-pyrazole-3-carboxamide |
| 323 | 1-(2-chlorophenyl)-4-(fluoromethyl)-5-(4-methoxyphenyl)-N-(piperidin-1-yl)-4,5-dihydro-1H-pyrazole-3-carboxamide |
| 324 | 1-(2-chlorophenyl)-4-hydroxy-5-(4-methoxyphenyl)-N-(piperidin-1-yl)-4,5-dihydro-1H-pyrazole-3-carboxamide |
| 325 | *cis*-1-(2,4-dichlorophenyl)-5-(4-ethoxyphenyl)-4-methyl-N-(piperidin-1-yl)-4,5-dihydro-1H-pyrazole-3-carboxamide |
| 326 | *trans*-1-(2,4-dichlorophenyl)-5-(4-ethoxyphenyl)-4-methyl-N-(piperidin-1-yl)-4,5-dihydro-1H-pyrazole-3-carboxamide |
| 327 | *cis*-1-(2-chlorophenyl)-5-(4-ethoxyphenyl)-4-methyl-N-(piperidin-1-yl)-4,5-dihydro-1H-pyrazole-3-carboxamide |
| 328 | *trans*-1-(2-chlorophenyl)-5-(4-ethoxyphenyl)-4-methyl-N-(piperidin-1-yl)-4,5-dihydro-1H-pyrazole-3-carboxamide |
| 329 | *cis*-N-(azepan-1-yl)-1-(2,4-dichlorophenyl)-5-(4-ethoxyphenyl)-4-methyl-4,5-dihydro-1H-pyrazole-3-carboxamide |
| 330 | *trans*-N-(azepan-1-yl)-1-(2,4-dichlorophenyl)-5-(4-ethoxyphenyl)-4-methyl-4,5-dihydro-1H-pyrazole-3-carboxamide |
| 331 | *cis*-N-(azepan-1-yl)-1-(2-chlorophenyl)-5-(4-ethoxyphenyl)-4-methyl-4,5-dihydro-1H-pyrazole-3-carboxamide |
| 332 | *trans*-N-(azepan-1-yl)-1-(2-chlorophenyl)-5-(4-ethoxyphenyl)-4-methyl-4,5-dihydro-1H-pyrazole-3-carboxamide |
| 333 | *cis*-N-cycloheptyl-1-(2,4-dichlorophenyl)-5-(4-ethoxyphenyl)-4-methyl-4,5-dihydro-1H-pyrazole-3-carboxamide |
| 334 | *cis-*1-(2-chlorophenyl)-N-cycloheptyl-5-(4-ethoxyphenyl)-4-methyl-4,5-dihydro-1H-pyrazole-3-carboxamide |
| 335 | *cis*-1-(2,4-dichlorophenyl)-5-(4-ethoxyphenyl)-N-(hexahydrocyclopenta[c]pyrrol-2(1H)-yl)-4-methyl-4,5-dihydro-1H-pyrazole-3-carboxamide |
| 336 | *cis*-1-(2-chlorophenyl)-5-(4-ethoxyphenyl)-N-(hexahydrocyclopenta[c]pyrrol-2(H)-yl)-4-methyl-4,5-dihydro-1H-pyrazole-3-carboxamide |
| 337 | *cis*-1-(2,4-dichlorophenyl)-5-(4-ethoxyphenyl)-N-(indolin-1-yl)-4-methyl-4,5-dihydro-1H-pyrazole-3-carboxamide |
| 338 | *cis*-1-(2-chlorophenyl)-5-(4-ethoxyphenyl)-N-(indolin-1-yl)-4-methyl-4,5-dihydro-1H-pyrazole-3-carboxamide |
| 339 | (+)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide |
| 340 | (-)-cis-1-(2,4-dichlorophenyl)-5-(4-ethoxyphenyl)-N-((1S,2S)-2-hydroxycyclohexyl)-4-methyl-4,5-dihydro-1 H-pyrazole-3-carboxamide |
| 341 | (+)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide |
| 342 | (-)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide |
| 343 | *cis*-1-(2,4-dichlorophenyl)-5-(4-hydroxyphenyl)-4-methyl-N-(piperidin-1-yl)-4,5-dihydro-1H-pyrazole-3-carboxamide |
| 344 | *trans*-1-(2,4-dichlorophenyl)-5-(4-hydroxyphenyl)-4-methyl-N-(piperidin-1-yl)-4,5-dihydro-1H-pyrazole-3-carboxamide |
| 345 | *cis-*1-(2-chlorophenyl)-5-(4-hydroxyphenyl)-4-methyl-N-(piperidin-1-yl)-4,5-dihydro-1H-pyrazole-3-carboxamide |
| 346 | *trans*-1-(2-chlorophenyl)-5-(4-hydroxyphenyl)-4-methyl-N-(piperidin-1-yl)-4,5-dihydro-1H-pyrazole-3-carboxamide |
| 347 | *cis*-N-(azepan-1-yl)-1-(2,4-dichlorophenyl)-5-(4-hydroxyphenyl)-4-methyl-4,5-dihydro-1H-pyrazole-3-carboxamide |
| 348 | *trans*-N-(azepan-1-yl)-1-(2,4-dichlorophenyl)-5-(4-hydroxyphenyl)-4-methyl-4,5-dihydro-1H-pyrazole-3-carboxamide |
| 349 | *cis*-N-(azepan-1-yl)-1-(2-chlorophenyl)-5-(4-hydroxyphenyl)-4-methyl-4,5-dihydro-1H-pyrazole-3-carboxamide |
| 350 | *trans*-N-(azepan-1-yl)-1-(2-chlorophenyl)-5-(4-hydroxyphenyl)-4-methyl-4,5-dihydro-1H-pyrazole-3-carboxamide |
| 351 | *cis*-N-cycloheptyl-1-(2 ,4-dichlorophenyl)-5-(4-hydroxyphenyl)-4-methyl-4,5-dihydro-1 H-pyrazole-3-carboxamide |
| 352 | *cis*-1-(2-chlorophenyl)-N-cycloheptyl-5-(4-hydroxyphenyl)-4-methyl-4,5-dihydro-1H-pyrazole-3-carboxamide |
| 353 | *cis*-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride |
| 354 | *cis*-1-(2-chlorophenyl)-N-(hexahydrocyclopenta[c]pyrrol-2(1H)-yl)-5-(4-hydroxyphenyl)-4-methyl-4,5-dihydro-1H-pyrazole-3-carboxamide |
| 355 | *cis*-1-(2,4-dichlorophenyl)-5-(4-hydroxyphenyl)-N-(indolin-1-yl)-4-methyl-4,5-dihydro-1H-pyrazole-3-carboxamide |
| 356 | *cis*-1-(2-chlorophenyl)-5-(4-hydroxyphenyl)-N-(indolin-1-yl)-4-methyl-4,5-dihydro-1 H-pyrazole-3-carboxamide |
| 357 | (+)-cis-1-(2,4-dichlorophenyl)-N-((1S,2S)-2-hydroxycyclohexyl)-5-(4-hydroxyphenyl)-4-methyl-4,5-dihydro-1 H-pyrazole-3-carboxamide |
| 358 | (-)-cis-1-(2 ,4-dichlorophenyl)-N-((1S,2S)-2-hydroxycyclohexyl)-5-(4-hydroxyphenyl)-4-methyl-4,5-dihydro-1H-pyrazole-3-carboxamide |
| 359 | (+)-cis-1-(2-chlorophenyl)-N-((1S,2S)-2-hydroxycyclohexyl)-5-(4-hydroxyphenyl)-4-methyl-4,5-dihydro-1 H-pyrazole-3-carboxamide |
| 360 | (-)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide |
| 361 | *cis*-5-(5-chlorothiophen-2-yl)-1-(2,4-dichlorophenyl)-4-methyl-4,5-dihydro-1H-pyrazole-3-carboxylic acid |
| 362 | *trans*-5-(5-chlorothiophen-2-yl)-1-(2,4-dichlorophenyl)-4-methyl-4,5-dihydro-1H-pyrazole-3-carboxylic acid |
| 363 | *cis*-1-(2-chlorophenyl)-5-(5-chlorothiophen-2-yl)-4-methyl-4,5-dihydro-1H-pyrazole-3-carboxylic acid |
| 364 | *trans-*1-(2-chlorophenyl)-5-(5-chlorothiophen-2-yl)-4-methyl-4,5-dihydro-1H-pyrazole-3-carboxylic acid |
| 365 | *cis*-5-(5-bromothiophen-2-yl)-1-(2,4-dichlorophenyl)-4-ethyl-4,5-dihydro-1H-pyrazole-3-carboxylic acid |
| 366 | *trans*-5-(5-bromothiophen-2-yl)-1-(2,4-dichlorophenyl)-4-ethyl-4,5-dihydro-1H-pyrazole-3-carboxylic acid |
| 367 | *cis*-1-(2,4-dichlorophenyl)-4-methyl-5-(thiophen-2-yl)-4,5-dihydro-1H-pyrazole-3-carboxylic acid |
| 368 | *trans*-1-(2,4-dichlorophenyl)-4-methyl-5-(thiophen-2-yl)-4,5-dihydro-1H-pyrazole-3-carboxylic acid |
| 369 | *cis*-1-(2,4-dichlorophenyl)-4-methyl-N-(piperidin-1-yl)-5-(thiophen-2-yl)-4,5-dihydro-1H-pyrazole-3-carboxamide |
| 370 | *trans*-1-(2,4-dichlorophenyl)-4-methyl-N-(piperidin-1-yl)-5-(thiophen-2-yl)-4,5-dihydro-1H-pyrazole-3-carboxamide |
| 371 | *cis*-1-(2-chlorophenyl)-4-methyl-N-(piperidin-1-yl)-5-(thiophen-2-yl)-4,5-dihydro-1H-pyrazole-3-carboxamide |
| 372 | *trans*-1-(2-chlorophenyl)-4-methyl-N-(piperidin-1-yl)-5-(thiophen-2-yl)-4,5-dihydro-1H-pyrazole-3-carboxamide |
| 373 | *cis*-1-(2 ,4-dichlorophenyl)-4-methyl-5-phenyl-4,5-dihydro-1H-pyrazole-3-carboxylic acid |
| 374 | *trans*-1-(2,4-dichlorophenyl)-4-methyl-5-phenyl-4,5-dihydro-1H-pyrazole-3-carboxylic acid |
| 375 | *cis-*1-(2,4-dichlorophenyl)-4-methyl-5-phenyl-N-(piperidin-1-yl)-4,5-dihydro-1H-pyrazole-3-carboxamide |
| 376 | *trans* -1-(2,4-dichlorophenyl)-4-methyl-5-phenyl-N-(piperidin-1-yl)-4,5-dihydro-1H-pyrazole-3-carboxamide |
| 377 | *cis*-1-(2-chlorophenyl)-4-methyl-5-phenyl-N-(piperidin-1-yl)-4,5-dihydro-1H-pyrazole-3-carboxamide |
| 378 | *trans*-1-(2-chlorophenyl)-4-methyl-5-phenyl-N-(piperidin-1-yl)-4,5-dihydro-1H-pyrazole-3-carboxamide |
| 379 | *cis-*5-(4-chlorophenyl)-1-(2,4-dichlorophenyl)-4-methyl-N-(piperidin-1-yl)-4,5-dihydro-1H-pyrazole-3-carbothioamide hydrochloride |
| 380 | *trans*-5-(4-chlorophenyl)-1-(2,4-dichlorophenyl)-4-methyl-N-(piperidin-1-yl)-4,5-dihydro-1H-pyrazole-3-carbothioamide |
| 381 | *cis* -1-(2-chlorophenyl)-5-(4-chlorophenyl)-4-methyl-N-(piperidin-1-yl)-4,5-dihydro-1H-pyrazole-3-carbothioamide |
| 382 | *trans*-1-(2-chlorophenyl)-5-(4-chlorophenyl)-4-methyl-N-(piperidin-1-yl)-4,5-dihydro-1H-pyrazole-3-carbothioamide |
| 383 | *cis-*N-(azepan-1-yl)-5-(4-chlorophenyl)-1-(2,4-dichlorophenyl)-4-methyl-4,5-dihydro-1H-pyrazole-3-carbothioamide |
| 384 | *cis*-N-(azepan-1-yl)-1-(2-chlorophenyl)-5-(4-chlorophenyl)-4-methyl-4,5-dihydro-1H-pyrazole-3-carbothioamide |
| 385 | c*is-*5-(4-bromophenyl)-1-(2,4-dichlorophenyl)-4-methyl-N-(piperidin-1-yl)-4,5-dihydro-1H-pyrazole-3-carbothioamide |
| 386 | *trans*-5-(4-bromophenyl)-1-(2,4-dichlorophenyl)-4-methyl-N-(piperidin-1-yl)-4,5-dihydro-1H-pyrazole-3-carbothioamide |
| 387 | *cis*-5-(4-bromophenyl)-1-(2-chlorophenyl)-4-methyl-N-(piperidin-1-yl)-4,5-dihydro-1H-pyrazole-3-carbothioamide |
| 388 | *trans*-5-(4-bromophenyl)-1-(2-chlorophenyl)-4-methyl-N-(piperidin-1-yl)-4,5-dihydro-1H-pyrazole-3-carbothioamide |
| 389 | *cis*-1-(2,4-dichlorophenyl)-5-(4-fluorophenyl)-4-methyl-N-(piperidin-1-yl)-4,5-dihydro-1H-pyrazole-3-carbothioamide |
| 390 | *trans*-1-(2,4-dichlorophenyl)-5-(4-fluorophenyl)-4-methyl-N-(piperidin-1-yl)-4,5-dihydro-1 H-pyrazole-3-carbothioamide |
| 391 | *cis*-1-(2-chlorophenyl)-5-(4-fluorophenyl)-4-methyl-N-(piperidin-1-yl)-4,5-dihydro-1H-pyrazole-3-carbothioamide |
| 392 | *trans*-1-(2-chlorophenyl)-5-(4-fluorophenyl)-4-methyl-N-(piperidin-1-yl)-4,5-dihydro-1H-pyrazole-3-carbothioamide |
| 393 | *cis*-1-(2,4-dichlorophenyl)-5-(4-methoxyphenyl)-4-methyl-N-(piperidin-1-yl)-4,5-dihydro-1 H-pyrazole-3-carbothioamide |
| 394 | *cis*-N-(azepan-1-yl)-1-(2,4-dichlorophenyl)-5-(4-methoxyphenyl)-4-methyl-4,5-dihydro-1H-pyrazole-3-carbothioamide |
| 395 | *trans*-1-(2,4-dichlorophenyl)-5-(4-methoxyphenyl)-4-methyl-N-(piperidin-1-yl)-4,5-dihydro-1H-pyrazole-3-carbothioamide |
| 396 | *cis*-1-(2-chlorophenyl)-5-(4-methoxyphenyl)-4-methyl-N-(piperidin-1-yl)-4,5-dihydro-1H-pyrazole-3-carbothioamide |
| 397 | *cis*-N-(azepan-1-yl)-1-(2-chlorophenyl)-5-(4-methoxyphenyl)-4-methyl-4,5-dihydro-1 H-pyrazole-3-carbothioamide |
| 398 | *trans-*1-(2-chlorophenyl)-5-(4-methoxyphenyl)-4-methyl-N-(piperidin-1-yl)-4,5-dihydro-1H-pyrazole-3-carbothioamide |
| 399 | *cis*-1-(2,4-dichlorophenyl)-5-(4-ethoxyphenyl)-4-methyl-N-(piperidin-1-yl)-4,5-dihydro-1H-pyrazole-3-carbothioamide |
| 400 | *trans*-1-(2,4-dichlorophenyl)-5-(4-ethoxyphenyl)-4-methyl-N-(piperidin-1-yl)-4,5-dihydro-1H-pyrazole-3-carbothioamide |
| 401 | *cis*-1-(2-chlorophenyl)-5-(4-ethoxyphenyl)-4-methyl-N-(piperidin-1-yl)-4,5-dihydro-1H-pyrazole-3-carbothioamide |
| 402 | *trans-*1-(2*-*chlorophenyl)*-*5-(4-ethoxyphenyl)-4-methyl-N-(piperidin-1-yl)-4,5-dihydro-1H-pyrazole-3-carbothioamide |
| 403 | *cis*-1-(2,4-dichlorophenyl)-5-(4-hydroxyphenyl)-4-methyl-N-(piperidin-1-yl)-4,5-dihydro-1H-pyrazole-3-carbothioamide |
| 404 | *trans*-1-(2,4-dichlorophenyl)-5-(4-hydroxyphenyl)-4-methyl-N-(piperidin-1-yl)-4,5-dihydro-1 H-pyrazole-3-carbothioamide |
| 405 | *cis*-1-(2-chlorophenyl)-5-(4-hydroxyphenyl)-4-methyl-N-(piperidin-1-yl)-4,5-dihydro-1H-pyrazole-3-carbothioamide |
| 406 | *trans-*1-(2-chlorophenyl)-5-(4-hydroxyphenyl)-4-methyl-N-(piperidin-1-yl)-4,5-dihydro-1H-pyrazole-3-carbothioamide |
| 407 | 7-(4-Chloro-phenyl)-6-(2,4-dichlorophenyl)-5,6-diaza-spiro[2.4]hept-4-ene-4-carboxylic acid piperidin-1-ylamide |
| 408 | 6-(2,4-Dichloro-phenyl)-7-(4-methoxy-phenyl)-5,6-diaza-spiro[2.4]hept-4-ene-4-carboxylic acid piperidin-1-ylamide; |
| 409 | (+)-*cis*-N-((1S,2S)-2-(benzyloxy)cyclohexyl)-5-(4-chlorophenyl)-1-(2,4-dichlorophenyl)-4-methyl-4,5-dihydro-1 H-pyrazole-3-carboxamide |
| [410] | *cis*-1-(2,4-dichlorophenyl)-5-(4-methoxyphenyl)-4-methyl-N-(piperidin-1-yl)-4,5-dihydro-1H-pyrazole-3-carboxamide |
| [411] | *cis-*1-(2,4-dichlorophenyl)-5-(4-methoxyphenyl)-4-methyl-N-(piperidin-1-yl)-4,5-dihydro-1H-pyrazole-3-carboxamide |
| [412] | (4R,5S)-1-(2,4-dichlorophenyl)-5-(4-methoxyphenyl)-4-methyl-4,5-dihydro-1H-pyrazole-3-carboxylic acid |
| [413] | cis-5-(4-bromophenyl)-1-(2,4-dichlorophenyl)-4-methyl-4,5-dihydro-1H-pyrazole-3-carboxylic acid |
| [414] | cis-N-(azepan-1-yl)-1-(2,4-dichlorophenyl)-5-(4-fluorophenyl)-4-methyl-4,5-dihydro-1H-pyrazole-3-carboxamide |
| 415 | *cis*-5-(4-bromophenyl)-1-(2,4-dichlorophenyl)-N-(indolin-1-yl)-4-methyl-4,5-dihydro-1H-pyrazole-3-carboxamide |
| 416 | *trans*-ethyl 5-(4-chlorophenyl)-1-(2,4-dichlorophenyl)-4-(trifluoromethyl)-4,5-dihydro-1H-pyrazole-3-carboxylate |
| 417 | *cis*-1-(2,4-dichlorophenyl)-5-(4-fluorophenyl)-4-methyl-N-(piperidin-1-yl)-4,5-dihydro-1H-pyrazole-3-carboxamide hydrochloride |
| 418 | *cis-*5-(4-bromophenyl)-1-(2,4-dichlorophenyl)-4-methyl-N-(piperidin-1-yl)-4,5-dihydro-1H-pyrazole-3-carboxamide hydrochloride |
| 419 | (-)-*cis*-5-(4-chlorophenyl)-1-(2,4-dichlorophenyl)-4-methyl-N-(piperidin-1-yl)-4,5-dihydro-1H-pyrazole-3-carboxamide |
| 420 | (+)-*cis*-5-(4-chlorophenyl)-1-(2,4-dichlorophenyl)-4-methyl-N-(piperidin-1-yl)-4,5-dihydro-1H-pyrazole-3-carboxamide |
| 421 | *trans*-ethyl 1-(2,4-dichlorophenyl)-4-methyl-5-phenyl-4,5-dihydro-1H-pyrazole-3-carboxylate |
| 422 | *cis*-5-(4-chlorophenyl)-1-(2,4-dichlorophenyl)-4-methyl-N-((1R,2S,4R)-1,7,7-trimethylbicyclo[2.2.1]heptan-2-yl)-4,5-dihydro-1H-pyrazole-3-carboxamide |
| 423 | *cis*-5-(4-chlorophenyl)-1-(2,4-dichlorophenyl)-4-methyl-N-(3-methylcyclohexyl)-4,5-dihydro-1H-pyrazole-3-carboxamide |
| 424 | *cis*-5-(4-chlorophenyl)-1-(2,4-dichlorophenyl)-4-methyl-N-(2-methylcyclohexyl)-4,5-dihydro-1H-pyrazole-3-carboxamide |
| 426 | *trans*-5-(5-chlorothiophen-2-yl)-1-(2,4-dichlorophenyl)-4-methyl-N-(piperidin-1-yl)-4,5-dihydro-1H-pyrazole-3-carboxamide hydrochloride |
| 427 | *cis*-5-(5-chlorothiophen-2-yl)-1-(2,4-dichlorophenyl)-4-methyl-N-(piperidin-1-yl)-4,5-dihydro-1H-pyrazole-3-carboxamide hydrochloride |
| 428 | *cis-*5-(4-bromophenyl)-1-(2,4-dichlorophenyl)-4-ethyl-N-(piperidin-1-yl)-4,5-dihydro-1H-pyrazole-3-carboxamide hydrochloride |
| [429] | 1-(2,4-dichlorophenyl)-4-methyl-5-phenyl-4,5-dihydro-1H-pyrazole-3-carboxylic acid |
| [430] | *cis*-1-(2,4-dichlorophenyl)-4-methyl-N-(piperidin-1-yl)-5-(thiophen-2-yl)-4,5-dihydro-1H-pyrazole-3-carboxamide hydrochloride |
| [431] | trans-5-(4-chlorophenyl)-1-(2,4-dichlorophenyl)-4-methyl-N-(piperidin-1-yl)-4,5-dihydro-1H-pyrazole-3-carboxamide |
| 432 | *cis-*5-(4-chlorophenyl)-1-(2,4-dichlorophenyl)-4-ethyl-N-(piperidin-1-yl)-4,5-dihydro-1H-pyrazole-3-carboxamide |
| 434 | *cis*-5-(4-chlorophenyl)-1-(2,4-dichlorophenyl)-4-methyl-N-(piperidin-1-yl)-4,5-dihydro-1H-pyrazole-3-carboxamide |
| 436 | (+)-*cis*-N-((1S,2S)-2-(benzyloxy)cyclohexyl)-5-(4-chlorophenyl)-1-(2,4-dichlorophenyl)-4-methyl-4,5-dihydro-1H-pyrazole-3-carboxamide |
| 437 | (-)-*cis*-N-((1R,2R)-2-(benzyloxy)cyclohexyl)-5-(4-chlorophenyl)-1-(2,4-dichlorophenyl)-4-methyl-4,5-dihydro-1H-pyrazole-3-carboxamide |
| 438 | *cis*-N-(azocan-1-yl)-1-(2,4-dichlorophenyl)-5-(4-methoxyphenyl)-4-methyl-4,5-dihydro-1H-pyrazole-3-carboxamide |
| 439 | *cis*-N-(azocan-1-yl)-1-(2-chlorophenyl)-5-(4-methoxyphenyl)-4-methyl-4,5-dihydro-1H-pyrazole-3-carboxamide |
| 440 | *cis*-N-(azocan-1-yl)-5-(4-bromophenyl)-1-(2,4-dichlorophenyl)-4-methyl-4,5-dihydro-1H-pyrazole-3-carboxamide |
| 441 | *cis-*N-(azocan-1-yl)-5-(4-bromophenyl)-1-(2-chlorophenyl)-4-methyl-4,5-dihydro-1H-pyrazole-3-carboxamide |
| 442 | *cis-*N*-*(azocan-1-yl)-1-(2,4-dichlorophenyl)-5-(4-fluorophenyl)-4-methyl-4,5-dihydro-1H-pyrazole-3-carboxamide |
| 443 | *cis-*N-(azocan-1-yl)-1-(2-chlorophenyl)-5-(4-fluorophenyl)-4-methyl-4,5-dihydro-1H-pyrazole-3-carboxamide |
| 444 | (+)-*cis*-5-(4-Chlorophenyl)-1-(2,4-dichlorophenyl)-4-methyl-4,5-dihydro-1H-pyrazole-3-carboxylic acid azepan-1-ylamide hydrochloride |
| 445 | (-)-*cis*- (4-Chlorophenyl)-1-(2,4-dichlorophenyl)-4-methyl-4,5-dihydro-1H-pyrazole-3-carboxylic acid azepan-1-ylamide hydrochloride |

### PHARMACOLOGICAL PART:

### Example 1 Determination of the affinity of N-piperidinyl-5-(4-chlorophenyl)-1-(2,4-dichlorophenyl)-4,5-dihydro-1H-pyrazole-3-carboxamide (see Subgroup A1, Chemical example 1) for rat CB1 receptors.

### Methods

Binding affinity to CB1 receptor was evaluated according to the method of Govaerts et al (2004) with the following modifications Briefly, cerebella from male Wistar rats (250-300g) were carefully dissected on ice and homogenates were prepared in cold 50 mM Tris-HCl, 5 mM MgCl₂, 1 mM EDTA, 0.25 M sucrose, pH 7.4 with a Potter-Heljheim homogenizer and the suspension was centrifuged at 1,000 x g for 5 minutes. The supernatants were collected and centrifuged 50,000 x g for 15 min. The resulting pellets were resuspended in Tris-HCl buffer without sucrose, homogenized and incubated for 15 min at 37°C in an orbital shaker bath and centrifuged again at 50,000 x g for 15 min. Pellets were weighed, resuspended in Tris-HCl buffer without sucrose, homogenized with an Ultraturrax at 13,500 rpm for 3 x 5 seconds and aliquoted in 0.9 ml volumes in Eppendorf tubes. Aliquots were centrifuged at 20,800 x g for 5 minutes, supernatants discarded and pellets were frozen at -80°C until use. Total protein concentration was determined using a Bio-Rad commercial assay base on the Lowry method. Competitive binding experiments were performed in presence of 1 nM [³H]-CP 55,940 in siliconized glass tubes containing 100 µg protein/tube resuspended in 1ml final volume of 50 mM Tris-HCl, 5 mM MgCl₂, 1 mM EDTA, 0.5% (w/v) bovine serum albumin, pH 7.4. Competitors were present at various concentrations and the non-specific binding was determined in the presence of 10 µM HU-210. After 1 hour incubation at 30°C, the suspension was rapidly filtered through 0.5% PEI pre-treated GF/B fiber filters on a 96-well harvester and washed 3 times with 3ml ice-cold binding buffer without bovine serum albumin. Radioactivity on filters was measured with Wallac Winspectral 1414 counter by liquid scintillation in 6 ml Ecoscint H (National Diagnostics, U.K.). Assays were made in triplicate.

Binding data were analyzed by non-linear regression with the software GraphPad Prism Version 3.03.

### Results

When the affinity of N-piperidinyl-5-(4-chlorophenyl)-1-(2,4-dichlorophenyl)-4,5-dihydro-1H-pyrazole-3-carboxamide for the rat CB1 receptor subtype was determined in vitro, it was observed to have good affinity for these receptors with an IC₅₀ of 26 nM (Table 1).

**Table 1 The affinity of N-piperidinyl-5-(4-chlorophenyl)-1-(2,4-dichlorophenyl)-4,5 dihydro-1H-pyrazole-3-carboxamide for rat CB1 receptors**

| Example | pIC₅₀ | IC₅₀ |
|---|---|---|
| **Subgroup A1, Chemical example 1** | 7.65 ± 0.29 | 26 |

### Example 2 Effect of repeated administration of N-piperidinyl-5-(4-chlorophenyl)-1-(2,4-dichlorophenyl)-4,5-dihydro-1H-pyrazole-3-carboxamide on body weight-gain and hyperphagia evoked in rats by the atypical antipsychotic drug, olanzapine.

### METHODS

### Animals

The experiment was performed on 40 female Sprague-Dawley rats (originally in the weight range 250-300 g). Animals were individually housed in polypropylene cages with metal grid floors. The animal room was maintained at a temperature of 21±4°C and 55±20% humidity and on a reverse phase light-dark cycle (lights off for 8 h from 09.30-17.30 h) during which time the room was illuminated by red light. Animals had free access to powdered high fat diet (VRF1 plus 20% lard). The diet was contained in a glass feeding jar with an aluminium lid with a 3-4 cm hole cut in it to allow access to the food. Animals were housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

### Experimental procedures for the feeding study

Approximately a week before the start of the study, animals were weighed (to the nearest 0.1 g using an electronic top-pan balance) and allocated into 4 weight-matched treatment groups, each containing 10 animals, ie vehicle (0.5% HPMC (hydroxyl-propyl-methyl-cellulose) 3ml/kg po), olanzapine (3 mg/kg po in 0.5% HPMC), N-piperidinyl-5-(4-chlorophenyl)-1-(2,4-dichlorophenyl)-4,5-dihydro-1 H-pyrazole-3-carboxamide (30 mg/kg po in 0.5% HPMC), and olanzapine (3 mg/kg po in 0.5% HPMC), + N-piperidinyl-5-(4-chlorophenyl)-1-(2,4-dichlorophenyl)-4,5-dihydro-1 H-pyrazole-3-carboxamide (30 mg/kg po in 0.5% HPMC).
Following a 7 day baseline run-in period, during which the animals were dosed with orally with vehicle to acclimatise them to dosing/handling, the treatments above were then given once daily for 14 days.

Food intake and body weight were measured once daily. Variations in body weight were accounted for by expressing the food intake results in terms of g/kg rat weight.

Statistical comparisons between the food intakes of the different treatment groups were made by analysis of covariance followed by multiple t tests (two-tailed) to compare each treatment group with the vehicle-treated controls. Food intake of the animals at baseline (ie average of Day -6 to 0) was used as the covariate. Standard errors of the mean (SEM) were calculated from the residuals of the statistical model.

Statistical comparisons between the body weights of the different treatment groups were made by analysis of covariance followed by multiple t tests (two-tailed) to compare each treatment group with the vehicle-treated controls. For the body weight data (body weights each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

### RESULTS

### Effects of N-piperidinyl-5-(4-chlorophenyl)-1-(2,4-dichlorophenyl)-4,5-dihydro-1H-pyrazole-3-carboxamide (Cpd. A) on bodyweight and on olanzapine-induced weight-gain in female Sprague-Dawley rats

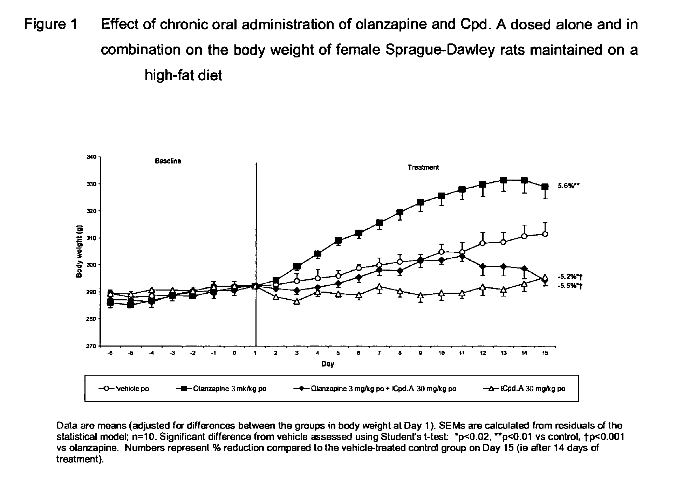

As shown in Figure 1, when olanzapine (3 mg/kg po) was administered once daily for a period of 14 days to female, Sprague-Dawley rats that were given access to a high-fat diet, it evoked a marked and sustained increase in the body weights of these rats when compared to the vehicle treated control (Figure 1). On Day 15, the mean weight of the olanzapine (3 mg/kg po)-treated group of rats was statistically (p<0.01) greater than that of the vehicle-treated controls (Figure 1). In contrast, the group of rats given N-piperidinyl-5-(4-chlorophenyl)1-(2,4-dichlorophenyl)-4,5-dihydro-1 H-pyrazole-3-carboxamide (Cpd. A) (30 mg/kg po) alone showed a gradual decrease in body weight compared to the vehicle-treated controls that was prolonged and sustained (Figure 1). On Day 15, the mean weight of the N-piperidinyl-5-(4-chlorophenyl)-1-(2,4-dichiorophenyl)-4,5-dihydro-1H-pyrazole-3-carboxamide (30 mg/kg po)-treated group of rats was statistically (p < 0.02) lower than that of the vehicle-treated controls (Figure 1). When rats were orally administered the combination medicament comprising a fixed dose 30 mg/kg of N-piperidinyl-5-(4-chlorophenyl)-1-(2,4-dichlorophenyl)-4,5-dihydro-1H-pyrazole-3-carboxamide + 3 mg/kg of olanzapine, N-piperidinyl-5-(4-chlorophenyl)-1-(2,4-dichlorophenyl)-4,5-dihydro-1H-pyrazole-3-carboxamide not only prevented the weight-gain evoked by the atypical antipsychotic, but it also surprisingly reduced the body weights of the rats to less than those treated with vehicle alone, and in fact, to the same degree as those treated with N-piperidinyl-5-(4-chlorophenyl)-1-(2,4-dichlorophenyl)-4,5-dihydro-1H-pyrazole-3-carboxamide alone (Figure 1). On Day 15, the mean decrease in the body weights of the rats treated with N-piperidinyl-5-(4-chlorophenyl)-1-(2,4-dichlorophenyl)-4,5-dihydro-1H-pyrazole-3-carboxamide + olanzapine was statistically lower than those treated with olanzapine (3 mg/kg po; p < 0.001) or vehicle (p < 0.02) and were not statistically different from those of the group of rats administered N-piperidinyl-5-(4-chlorophenyl)-1-(2,4-dichlorophenyl)-4,5-dihydro-1H-pyrazole-3-carboxamide (30 mg/kg po) alone (Figure 1).

### Effects of N-piperidinyl-5-(4-chlorophenyl)-1-(2,4-dichlorophenyl)-4,5-dihydro-1H-pyrazole-3-carboxamide (Cpd. A) on food intake and on olanzapine-induced hyperphagia in female Sprague-Dawley rats

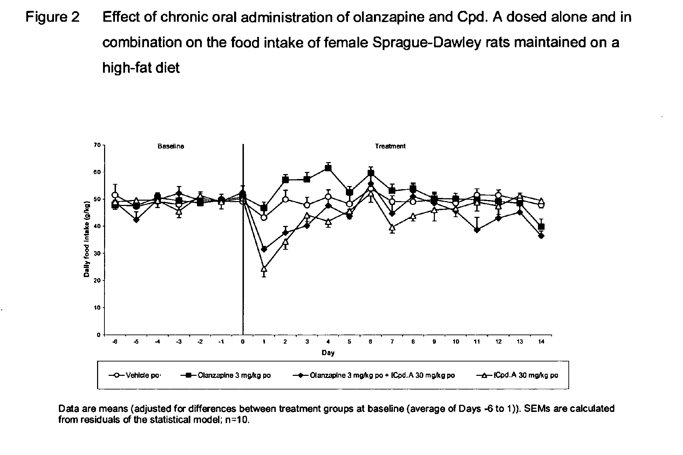

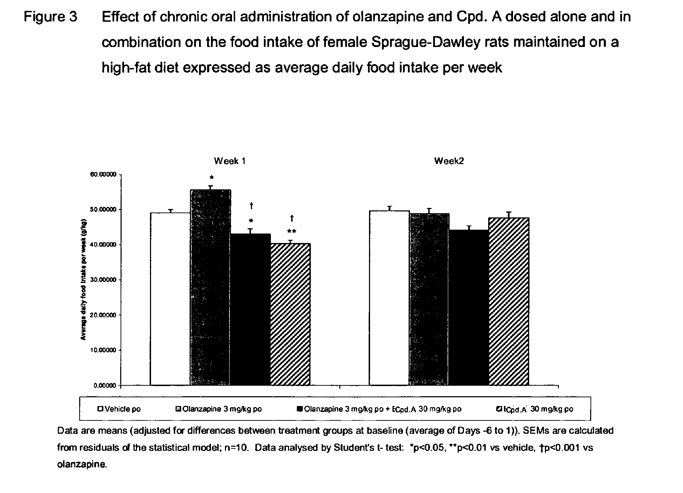

As shown in Figure 2, when olanzapine (3 mg/kg po) was administered once daily for a period of 14 days to female, Sprague-Dawley rats that were given access to a high-fat diet, it evoked an increase in food intake when compared to the vehicle-treated controls. The increase in daily food intake was particularly pronounced from Day 2 to Day 7 of olanzapine administration (Figure 2). The cumulative intake of food by the olanzapine (3 mg/kg po)-treated group of rats was statistically greater (p < 0.05) than that of the vehicle controls over the first week of the experiment (Figure 3). In contrast the group of rats given N-piperidinyl-5-(4-chlorophenyl)-1-(2,4-dichlorophenyl)-4,5-dihydro-1 H-pyrazole-3-carboxamide (30 mg/kg po) alone markedly reduced their food intakes compared to the vehicle-treated controls (Figure 3). The cumulative intake of food by the N-piperidinyl-5-(4-chlorophenyl)-1-(2,4-dichlorophenyl)-4,5-dihydro-1H-pyrazole-3-carboxamide (30 mg/kg po)-treated group of rats was statistically lower (p < 0.01) than that of the vehicle controls over the first week of the experiment (Figure 3). When rats were orally administered the combination medicament comprising a fixed dose 30 mg/kg of N-piperidinyl-5-(4-chlorophenyl)-1-(2,4-dichlorophenyl)-4,5-dihydro-1H-pyrazole-3-carboxamide +3 mg/kg of olanzapine, N-piperidinyl-5-(4-chlorophenyl)-1-(2,4-dichlorophenyl)-4,5-dihydro-1H-pyrazole-3-carboxamide not only prevented the hyperphagia evoked by the atypical antipsychotic, but it also surprisingly reduced the food intake of the rats to less than those treated with vehicle alone, and in fact, to the same degree as those treated with N-piperidinyl-5-(4-chlorophenyl)-1-(2,4-dichlorophenyl)-4,5-dihydro-1H-pyrazole-3-carboxamide alone (Figure 3). The decrease in cumulative food consumption over the first week of administration with N-piperidinyl-5-(4-chlorophenyl)-1-(2,4-dichlorophenyl)-4,5-dihydro-1H-pyrazole-3-carboxamide + olanzapine was statistically lower than that of the groups of rats treated with olanzapine (3 mg/kg po; p < 0.001) or vehicle (p < 0.05) and was not statistically different from that of the group of rats administered N-piperidinyl-5-(4-chlorophenyl)-1-(2,4-dichlorophenyl)-4,5-dihydro-1 H-pyrazole-3-carboxamide (30 mg/kg po) alone (Figure 3).

### DISCUSSION

Weight-gain as a side-effect is observed with drugs from many different pharmacological and therapeutic classes. However in most instances, it is both an unwanted effect and one that has adverse health consequences by predisposing the patient to an increased risk of dyslipidaemia, insulin resistance, impaired glucose tolerance, Type 2 diabetes, Metabolic Syndrome and/or a cardio- or cerebrovascular accident. Moreover, unwanted weight-gain can be a major factor in patients' non-compliance to drug therapy.

Although the major focus of this claim is drug-induced obesity, any increase of adiposity is unacceptable in patients who have metabolic disturbances associated with increased cardiac risk. Thus, weight gain induced by anti-diabetic drugs, eg the sulphonylureas, thiazolidinediones and/or insulin therapy, in treating insulin-resistance, impaired glucose tolerance and/or Type 2 diabetes is contra-indicated because of the deleterious effect that adiposity has on glycaemic control. In such cases, not only is increased weight not an acceptable outcome, but weight-loss will be in many instances of considerable therapeutic benefit to the patients concerned.

When dealing with the atypical antipsychotic drugs, it has been reported that these drugs cause deleterious increases in the plasma concentrations of triglycerides and glucose (Wirshing et al, 2002; Casey 2004; see also the reviews of Newcomer, 2005 and Melkersson & Dahl, 2004), and this is in a vulnerable patient population who already have increased visceral adiposity compared with normal subjects (Ryan et al, 2004).

The findings reported here show that an active substance combination comprising at least one substituted pyrazoline compound as described herein as exemplified by N-piperidinyl-5-(4-chlorophenyl)-1-(2,4-dichlorophenyl)-4,5-dihydro-1H-pyrazole-3-carboxamide, together with a fixed dose of a drug known to produce weight-gain, exemplified by olanzapine, prevented the weight-gain that was evoked by this atypical antipsychotic drug. Furthermore, olanzapine is a drug that is known not only to be one of the worst offenders for producing weight-gain as a side-effect in this class of drugs (Newcomer, 2005), but also amongst all drugs that cause weight increase (Pijl; & Meinders, 1996). Unexpectedly, the combination of a fixed dose of N-piperidinyl-5-(4-chlorophenyl)-1-(2,4-dichlorophenyl)-4,5-dihydro-1H-pyrazole-3-carboxamide together with a fixed dose of olanzapine was found not only to abolish olanzapine-induced weight-gain, but to actually produce weight-loss in this group of rats. The degree of weight-loss observed in the rats administered the N-piperidinyl-5-(4-chlorophenyl)-1-(2,4-dichlorophenyl)-4,5-dihydro-1H-pyrazole-3-carboxamide + olanzapine combination was not significantly different from that observed when N-piperidinyl-5-(4-chlorophenyl)-1-(2,4-dichlorophenyl)-4,5-dihydro-1 H-pyrazole-3-carboxamide was given alone. In defining the pharmacological mechanisms responsible for these effects, it is evident that the weight-gain observed with olanzapine treatment was caused by an increase in food consumption and the action of N-piperidinyl-5-(4-chlorophenylyl-(2,4-dichlorophenyl)-4,5-dihydro-1H-pyrazole-3-carboxamide to prevent this weight-gain and to cause weight-loss was due to a reduction in food intake to a level that was significantly lower than that of the vehicle-treated control group. In short, these observations demonstrate a clinically acceptable mechanism for the prevention of antipsychotic-induced weight-gain and weight-loss.

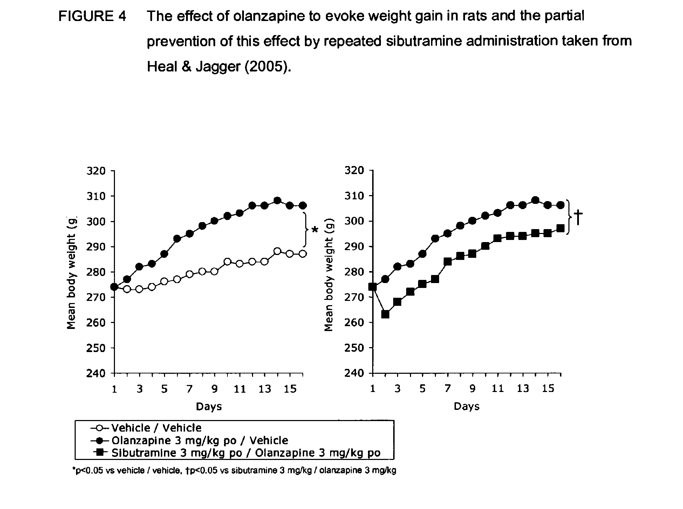

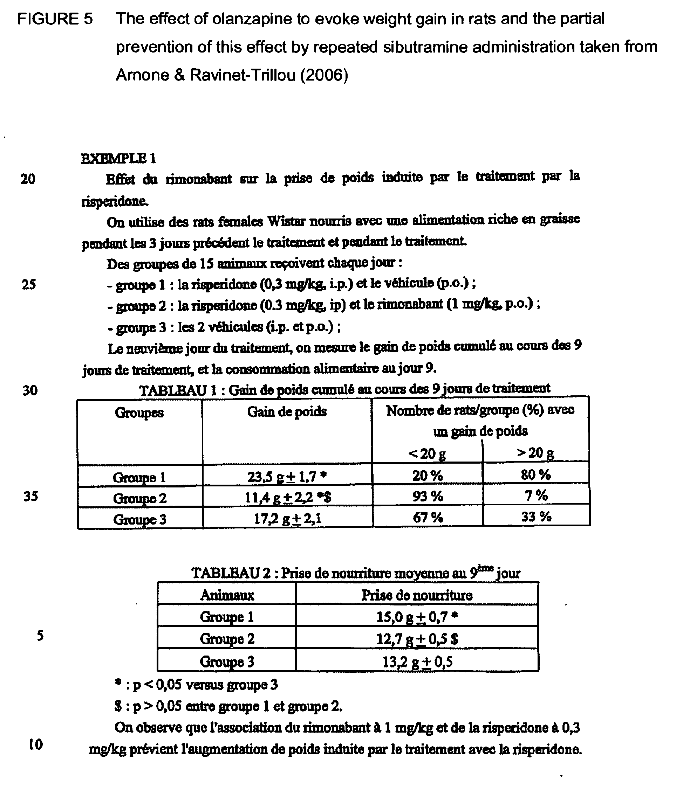

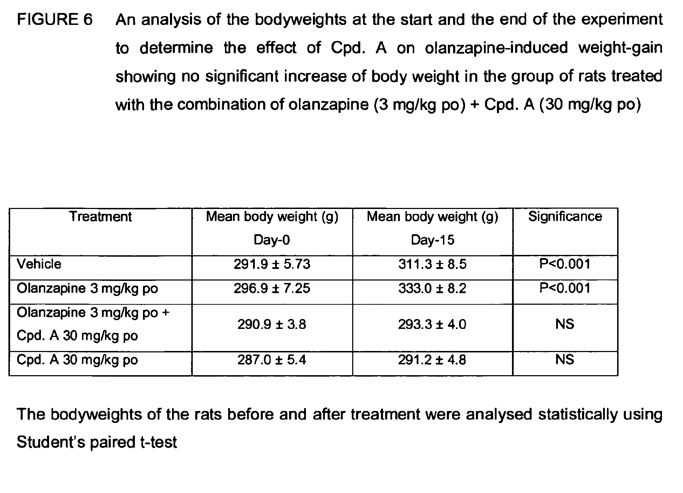

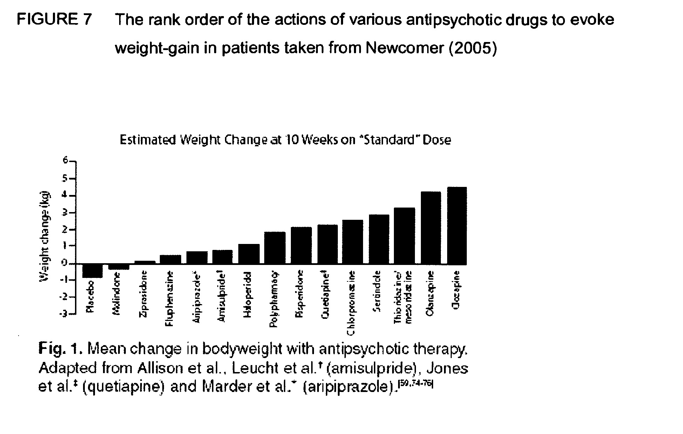

**Partial prevention** of antipsychotic-induced weight-gain has been observed previously with other anti-obesity drugs, eg the noradrenaline and serotonin reuptake inhibitor, sibutramine (Meridia™, Reductil®) (Heal & Jagger, 2005), but as shown in Figure 4, this effect is not complete and the animals do not show weight-loss when treated with the combination of sibutramine + olanzapine. What makes this finding with the N-piperidinyl-5-(4-chlorophenyl)-1-(2,4-dichlorophenyl)-4,5-dihydro-1H-pyrazole-3-carboxamide + olanzapine combination even more unexpected is that rimonabant, which is another CB1 receptor antagonist, has also been evaluated for its ability to prevent antipsychotic-induced weight-gain, and as shown in Figure 5, it was also found to be only partially effective in this regard (Arnone & Ravinet-Trillou, 2006). Although the data reported here and those taken from the filing by Arnone & Ravinet-Trillou (2006) have not been presented in identical fashion, the models employed are very similar, ie female rats given access to a high fat diet being made obese by administering atypical antipsychotic drugs to these animals, ie olanzapine (this filing) and risperidone (Arnone & Ravinet-Trillou, 2006), this difference is illustrated in 2 ways. First, Arnone & Ravinet-Trillou (2006) reported a weight-gain of 11.4 ± 2.2g in the group of rats given the combination of risperidone (0.3 mg/kg ip) + rimonabant (1.0 mg/kg po) over the 9 days of treatment (Figure 5), whereas as reported in this filing, the rats administered the combination of olanzapine (3 mg/kg po) + N-piperidinyl-5-(4-chlorophenyl)-1-(2,4-dichlorophenyl)-4,5-dihydro-1H-pyrazole-3-carboxamide (30 mg/kg po) did not gain any weight (Figure 6). Second, according to the data reported by (Arnone & Ravinet-Trillou, 2006), there was no significant difference in the weight-gain between the rats given risperidone (0.3 mg/kg ip) + rimonabant (1.0 mg/kg po) versus those given risperidone (0.3 mg/kg ip) + vehicle (Figure 5) indicating that rimonabant was ineffective in preventing risperidone-induced weight-gain; this may be a typographical error. There are other factors which should disbar this claim. Thus, Arnone & Ravinet-Trillou (2006) claim their results support the use of the combination of rimonabant or another CB1 receptor antagonist with an antipsychotic, but their exemplification is from an experiment where rimonabant was administered orally by gavage whilst risperidone was injected intraperitoneally; hence, these two drugs were evidently not administered as a combination medicament and the data do not, therefore, support the use of the combination of rimonabant [or another CB1 receptor antagonist] together with risperidone [or another atypical antipsychotic] either as an oral or an injectable medicament. In contrast, in this invention, olanzapine (3 mg/kg) + N-piperidinyl-5-(4-chlorophenyl)-1-(2,4-dichlorophenyl)-4,5-dihydro-1H-pyrazole-3-carboxamide (30 mg/kg) was administered as a combination medicament and via the clinically-relevant oral route of administration for both drugs. Furthermore, as shown in Figure 7, in the rankings of atypical antipsychotic drugs that cause weight-gain and obesity, risperidone is not ranked highly (see also Newcomer, 2005 for a discussion of the issue) and risperidone has a low propensity to cause Type 2 diabetes (Melkersson & Dahl, 2004) and, therefore, the data do not support the broader claim for the combination of rimonabant [or another CB1 receptor antagonist] together with any atypical antipsychotic. In contrast, olanzapine, which was used to exemplify this patent filing, is ranked amongst the top 2 worst offenders on both counts (Figure 7; see Newcomer, 2005 and Melkersson & Dahl, 2004). Thus, the earlier claim by Arnone & Ravinet-Trillou (2006) does not demonstrate the unexpected therapeutic advantages of weight-loss that have been demonstrated in the current invention with the orally administered active substance combination comprising at least one substituted pyrazoline compound as described herein as exemplified by N-piperidinyl-5-(4-chlorophenyl)-1-(2,4-dichlorophenyl)-4,5-dihydro-1H-pyrazole-3-carboxamide, + an atypical antipsychotic drug, as exemplified by olanzapine. Moreover, the results provided in support of the claim by (Arnone & Ravinet-Trillou, 2006) neither demonstrate the clinical benefit of a medicament comprising the combination of risperidone + rimonabant because the routes of administration are different, nor do they support the broader claim that there is unexpected weight-gain prevention benefit to be derived from the combination medicament comprising rimonabant, or another CB1 antagonist, with an atypical antipsychotic because the drug used to exemplify the claim, ie risperidone, is not one that causes marked weight-gain.

The prevention of drug-induced weight-gain by the use of an active substance combination comprising at least one substituted pyrazoline compound as described herein together with a fixed dose of a drug known to produce weight-gain will provide clinical benefit to the patient by avoiding deleterious changes in obesity-related cardio-metabolic risk factors, ie increased visceral adiposity and/or increases in plasma triglycerides, cholesterol and/or LDL-cholesterol, decreases in HDL-cholesterol, insulin resistance and/or impaired glucose tolerance and/or hypertension that predisposes him/her to a greatly increased risk of developing dyslipidaemia and/or Type 2 diabetes and/or Metabolic Syndrome, or of suffering a cardio- or cerebrovascular accident.

In addition, the weight-loss provided by a medicament comprising the active substance combination comprising a fixed dose of one substituted pyrazoline compound as described herein together with a fixed dose of a drug known to produce weight-gain or obesity will be of especial therapeutic benefit to a patient, who is in need of therapy with a medication that causes weight-gain, and who is overweight or obese and/or metabolically compromised, and as a consequence, is at increased cardio-metabolic risk. The weight-loss will improve various obesity-related cardio-metabolic risk factors, ie increased visceral adiposity and/or increased plasma triglycerides, cholesterol and/or LDL-cholesterol, decreases in HDL-cholesterol, insulin resistance and/or impaired glucose tolerance and/or hypertension that predispose him/her to a greatly increased risk of developing dyslipidaemia and/or Type 2 diabetes and/or Metabolic Syndrome, or of suffering a cardio- or cerebrovascular accident.

The finding that the weight-loss produced by the combination medicament comprising N-piperidinyl-5-(4-chlorophenyl)-1-(2,4-dichlorophenyl)-4,5-dihydro-1H-pyrazole-3-carboxamide + olanzapine was not different from that evoked N-piperidinyl-5-(4-chlorophenyl)-1-(2,4-dichlorophenyl)-4,5-dihydro-1H-pyrazole-3-carboxamide alone clearly demonstrates that the pharmacological actions of olanzapine do not reduce the cardio-metabolic benefits evoked by N-piperidinyl-5-(4-chlorophenyl)-1-(2,4-dichlorophenyl)-4,5-dihydro-1H-pyrazole-3-carboxamide treatment. The implication of this finding is that in the said combination medicament, any drug causing weight-gain, which produces its therapeutic benefits by a pharmacological mechanism (or mechanisms) that does not include CB1 agonism, will not attenuate the cardio-metabolic benefits of e.g. N-piperidinyl-5-(4-chlorophenyl)-1-(2,4-dichlorophenyl)-4,5-dihydro-1 H-pyrazole-3-carboxamide.

Conversely, none of the drugs, which have been selected from the following therapeutic classes that are known to produce weight-gain as a side-effect including, but not limited to, antipsychotics, comprising the atypical antipsychotics and typical neuroleptics, antidepressants including MAOls, TCAs, SSRls, "third generation" and atypical antidepressants, anti-manic agents, corticosteroids, agents for insulin resistance, impaired glucose tolerance and Type 2 diabetes, antihypertensives, anti-ulceratives, agents for neuropathic pain, antihistamines, agents for migraine, benzodiazepines, female hormonal contraceptives and female hormone replacement therapy, produce their therapeutic actions via the stimulation of CB1 receptors that are located either in the brain or in peripheral tissues. Since substituted pyrazolines according to the invention as described herein as exemplified by N-piperidinyl-5-(4-chlorophenyl)-1-(2,4-dichlorophenyl)-4,5-dihydro-1H-pyrazole-3-carboxamide, are selective CB1 antagonists, it can be concluded that the selected substituted pyrazolines according to the invention as described herein as exemplified by N-piperidinyl-5-(4-chlorophenyl)-1-(2,4-dichlorophenyl)-4,5-dihydro-1 H-pyrazole-3-carboxamide, will not detract from the clinical benefits provided by the other drug in the said combination medicament.

These results, therefore, demonstrate the surprising and unexpected therapeutic advantages of the use of a substituted pyrazolines according to the invention as described herein and as exemplified by N-piperidinyl-5-(4-chlorophenyl)-1-(2,4-dichlorophenyl)-4,5-dihydro-1H-pyrazole-3-carboxamide, as a fixed combination medicament with a drug selected from the following therapeutic classes that are known to produce weight-gain as a side-effect including, but not limited to, antipsychotics, comprising the atypical antipsychotics and typical neuroleptics, antidepressants including MAOIs, TCAs, SSRIs, "third generation" and atypical antidepressants, anti-manic agents, corticosteroids, agents for insulin resistance, impaired glucose tolerance and Type 2 diabetes, antihypertensives, anti-ulceratives, agents for neuropathic pain, antihistamines, agents for migraine, benzodiazepines, female hormonal contraceptives and female hormone replacement therapy.

### Effect of N-piperidinyl-5-(4-chlorophenyl)-1-(2,4-dichlorophenyl)-4,5-dihydro-1H-pyrazole-3-carboxamide in the novel object recognition test

In addition N-piperidinyl-5-(4-chlorophenyl)-1-(2,4-dichlorophenyl)-4,5-dihydro-1H-pyrazole-3-carboxamide was also tested in the novel object recognition test as known in the art showing very positive results, stressing also that the active substance combination according to the invention especially when combined with an antidepressant or an antipsychotic has a high benefit for the patient.

### REFERENCES

American Heart Association. Third Report of the National Cholesterol Education Program (NCEP) Expert Panel on Detection Evaluation, and Treatment of High Blood Cholesterol in Adults (Adult Treatment Panel III) Final Report. Circulation 2002;106:3143-3420.
Arnone M, Ravinet-Trillou C. Pharmaceutical compositions containing a combination of a cannabinoid receptor antagonist agent and an antipsychotic agent. WO 2006/097605 A1.
Casey DE. Dyslipidemia and atypical antipsychotic drugs. J Clin Psychiatry. 2004;65 Suppl 18:27-35.
Govaerts SJ, Hermans E, Lambert DM. Comparison of cannabinoid ligand affinities and efficacies in murine tissues and in transfected cells expressing human recombinant cannabinoid receptors. Eur J Pharm Sci. 2004; 23: 233 - 43.
Haapasalo-Pesu KM, Saarijarvi S. Olanzapine induces remarkable weight gain in adolescent patients. Eur Child Adolesc Psychiatry. 2001;10:205-8.
Heal DJ & Jagger E. Development, regulatory and marketing challenges for novel anti-obesity therapies. In: Obesity and Metabolic Disorders. Eds Antel J, Finer N, Heal DJ, Krause G.IOS Press, Amsterdam. 2005, pp73-91.
IDF consensus worldwide definition of the Metabolic Syndrome. IDF 2005. www.idf.org.
Melkersson K, Dahl M-L. Adverse metabolic effects associated with atypical antipsychotics. Literature review and clinical implications. Drugs. 2004;64:701-23.
Newcomer JW. Second-generation (atypical) antipsychotics and metabolic effects: a comprehensive literature review. CNS Drugs. 2005;19 Suppl 1:1-93.
Pijl H, Meinders AE. Bodyweight change as an adverse effect of drug treatment. Mechanisms and management. Drug Saf. 1996;14:329-42.
Ratzoni G, Gothelf D, Brand-Gothelf A, Reidman J, Kikinzon L, Gal G, Phillip M, Apter A, Weizman R. Weight gain associated with olanzapine and risperidone in adolescent patients: a comparative prospective study. J Am Acad Child Adolesc Psychiatry. 2002;41:337-43.
Ryan MC, Flanagan S, Kinsella U, Keeling F, Thakore JH. The effects of atypical antipsychotics on visceral fat distribution in first episode, drug-naive patients with schizophrenia. Life Sci. 2004;74:1999-2008.
WHO: World Health Organization. Definition, diagnosis and classification of diabetes mellitus and its complications. Report of a WHO consultation 1999.
Wirshing DA, Boyd JA, Meng LR, Ballon JS, Marder SR, Wirshing WC. The effects of novel antipsychotics on glucose and lipid levels. J Clin Psychiatry. 2002;63:856-65.

## Claims

1. Active substance combination comprising
(A) at least one substituted pyrazoline compound of general formula I 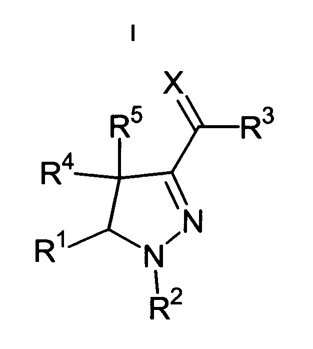 wherein
X is O or S;
R¹ and R², independently of one another, in each case represent an unsubstituted or at least mono-substituted aryl or heteroaryl radical, which may be condensed with an unsubstituted or at least mono-substituted mono- or polycyclic ring system;
or a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bridged by at least one unsubstituted or at least mono-substituted alkylene group;
R³ an unsubstituted or at least mono-substituted aryl or heteroaryl radical, which may be condensed with an unsubstituted or at least mono-substituted mono- or polycyclic ring system;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bridged by at least one unsubstituted or at least mono-substituted alkylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
a -O-R⁶ moiety; a -NR⁷R⁸ moiety or a -NR⁹-O-R¹⁰ moiety;
R⁴ represents H; F; Cl; Br; I; -CN; -NO₂; -NC; -OH; -NH₂; -SH; -C(=O)-H; -C(=O)-OH; -C(=O)-OR¹⁷;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an optionally at least mono-substituted alkylene group, alkenylene group or alkinylene group;
a -O-R¹¹ moiety; a -S-R¹² moiety; a -NH-R¹³ moiety or a -NR¹⁴R¹⁵ moiety;
R⁵ represents H; F; Cl; Br; I; -CN; -NO₂; -NC; -OH; -NH₂; -SH; -C(=O)-H; -C(=O)-OH; -C(=O)-OR¹⁷;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
a -O-R¹¹ moiety; a -S-R¹² moiety; a -NH-R¹³ moiety or a -NR¹⁴R¹⁵ moiety;
or R⁴ and R⁵ together with the bridging carbon atom form a saturated or unsaturated, unsubstituted or at least mono-substituted, optionally at least one heteroatom as a ring member containing cycloaliphatic radical;
R⁶ represents a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
a -P(=O)(OR¹⁶)₂ moiety; a -C(=O)-OR¹⁷ moiety; a -C(=O)-NH-R¹⁸ moiety or a-C(=O)-R¹⁹ moiety;
R⁷ and R⁸, independently of one another, in each case represent a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
a -P(=O)(OR¹⁶)₂ moiety; a -C(=O)-OR¹⁷ moiety; a -C(=O)-NH-R¹⁸ moiety; a -C(=O)-R¹⁹ moiety; a -S(=O)₂-R²⁰ moiety; or a -NR²¹R²² moiety;
R⁹ represents hydrogen or a saturated or unsaturated, unsubstituted or at least mono-substituted aliphatic radical;
R¹⁰, R¹¹, R¹², R¹³ , R¹⁴, R¹⁵ and R²⁰, independently of one another, in each case represent a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
R¹⁶, R¹⁷, R¹⁸ and R¹⁹, independently of one another, in each case represent a saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
and
R²¹ and R²², independently of one another, in each case represent a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.
and
(B) a drug producing weight-gain or obesity.

2. Active substance combination according to claim 1, **characterized in that** for the compound (A) according to formula I
X is O or S;
R¹ and R², independently of one another, in each case represent an unsubstituted or at least mono-substituted 6- or 10-membered aryl radical, which may be condensed with an unsubstituted or at least mono-substituted mono- or polycyclic ring system;
an unsubstituted or at least mono-substituted 5- to 14-membered heteroaryl radical, which may be condensed with an unsubstituted or at least mono-substituted mono- or polycyclic ring system;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅ alkylene group;
or an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅ alkylene group;
R³ represents an unsubstituted or at least mono-substituted 6- or 10-membered aryl radical, which may be condensed with an unsubstituted or at least mono-substituted mono- or polycyclic ring system;
an unsubstituted or at least mono-substituted 5- to 14-membered heteroaryl radical, which may be condensed with an unsubstituted or at least mono-substituted mono- or polycyclic ring system;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅alkylene group;
an unsubstituted or at least mono-substituted C₄₋₁₆ heterocycloalkyl radical or C₅-₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅ alkylene group;
an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical which together with an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical forms an unsubstituted or at least mono-substituted spirocyclic residue via a common ring atom;
a -O-R⁶ moiety; a -NR⁷R⁸ moiety or a -NR⁹-O-R¹⁰ moiety;
R⁴ represents H; F; Cl; Br; I; -CN; -NO₂; -NC; -OH; -NH₂; -SH; -C(=O)-H; -C(=O)-OH; -C(=O)-OR¹⁷;
an unsubstituted or at least mono-substituted C₁₋₁₆ alkyl radical, C₂₋₁₆ alkenyl radical or C₂₋₁₆alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bonded via an unsubstituted or at least mono-substituted C₁₋₅ alkylene group, C₂₋₅ alkenylene group or C₂₋₅ alkinylene group and/or may be bridged by at least one unsubstituted or at least mono-substituted C₁₋₅alkylene group;
an unsubstituted or at least mono-substituted C₄₋₁₆ heterocycloalkyl radical or **C₅-**₁₆ heterocycloalkenyl radical, which in each case may be condensed with an unsubstituted or at least mono-substituted mono- or polycyclic ring system and/or may be bonded via an unsubstituted or at least mono-substituted C₁₋₅ alkylene group, C₂₋₅ alkenylene group or C₂₋₅ alkinylene group and/or may be bridged by at least one unsubstituted or at least mono-substituted C₁₋₅ alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅ alkylene group, C₂₋₅ alkenylene group or C₂₋₅ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅alkinylene group;
a -O-R¹¹ moiety; a -S-R¹² moiety; a -NH-R¹³ moiety or a -NR¹⁴R¹⁵ moiety;
R⁵ represents H; F; Cl; Br; I; -CN; -NO₂; -NC; -OH; -NH₂; -SH; -C(=O)-H; -C(=O)-OH; -C(=O)-OR¹⁷;
an unsubstituted or at least mono-substituted C₁₋₁₆alkyl radical, C₂₋₁₆ alkenyl radical or C₂₋₁₆alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bonded via an unsubstituted or at least mono-substituted C₁₋₅ alkylene group, C₂₋₅ alkenylene group or C₂₋₅alkinylene group and/or may be bridged by at least one unsubstituted or at least mono-substituted C₁₋₅ alkylene group;
an unsubstituted or at least mono-substituted C₄₋₁₆ heterocycloalkyl radical or C₅-₁₆ heterocycloalkenyl radical, which in each case may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅ alkinylene group and/or may be bridged by at least one unsubstituted or at least mono-substituted C₁₋₅ alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅alkinylene group;
-O-R¹¹ moiety; a -S-R¹² moiety; a -NH-R¹³ moiety or a -NR¹⁴R¹⁵ moiety;
or R⁴ and R⁵ together with the bridging carbon atom form an unsubstituted or at least mono-substituted C₃₋₁₆ cycloalkyl radical or C₄₋₁₆ cycloalkenyl radical;
R⁶ represents a hydrogen atom;
an unsubstituted or at least mono-substituted C₁₋₁₆ alkyl radical, C₂₋₁₆alkenyl radical or C₂₋₁₆alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅ alkylene group and/or may be bonded via an unsubstituted or at least mono-substituted C₁₋₅ alkylene group, C₂₋₅ alkenylene group or C₂₋₅ alkinylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅ alkylene group and/or may be bonded via an unsubstituted or at least mono-substituted C₁₋₅alkylene group, C₂₋₅alkenylene group or C₂₋₅alkinylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅ alkylene group, C₂₋₅alkenylene group or C₂₋₅ alkinylene group;
an unsubstituted or at least mono-substituted 5- to 14-membered heteroaryl radical, which may be condensed with an unsubstituted or at least mono-substituted mono- or polycyclic ring system and/or may be bonded via an
unsubstituted or at least mono-substituted C₁₋₅alkylene group, C₂₋₅alkenylene group or C₂₋₅ alkinylene group;
a -P(=O)(OR¹⁶)₂ moiety; a -C(=O)-OR¹⁷ moiety; a -C(=O)-NH-R¹⁸ moiety or a-C(=O)-R¹⁹ moiety;
R⁷ and R⁸, independently of one another, in each case represent a hydrogen atom;
an unsubstituted or at least mono-substituted C₁₋₁₆ alkyl radical, C₂₋₁₆alkenyl radical or C₂₋₁₆alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅ alkylene group and/or may be bonded via an unsubstituted or at least mono-substituted C₁₋₅alkylene group, C₂₋₅alkenylene group or C₂₋₅alkinylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅ alkylene group and/or may be bonded via an unsubstituted or at least mono-substituted C₁₋₅ alkylene group, C₂₋₅alkenylene group or C₂₋₅ alkinylene group;
an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical which together with an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical forms an unsubstituted or at least mono-substituted spirocyclic residue via a common ring atom;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅ alkylene group, C₂₋₅alkenylene group or C₂₋₅ alkinylene group;
an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅alkinylene group;
a -P(=O)(OR¹⁶)₂ moiety; a -C(=O)-OR¹⁷ moiety; a -C(=O)-NH-R¹⁸ moiety; a-C(=O)-R¹⁹ moiety; a -S(=O)₂-R²⁰ moiety; or a -NR²¹R²² moiety;
R⁹ represents a hydrogen atom or an unsubstituted or at least mono-substituted C₁₋₁₆ alkyl radical, C₂₋₁₆ alkenyl radical or C₂₋₁₆ alkinyl radical;
R¹⁰, R¹¹, R¹², R¹³, R¹⁴, R¹⁵ and R²⁰, independently of one another, in each case represent an unsubstituted or at least mono-substituted C₁₋₁₆ alkyl radical, C₂₋₁₆alkenyl radical or C₂₋₁₆ alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bridged by at least one unsubstituted or at least mono-substituted C₁₋₅alkylene group and/or may be bonded via an unsubstituted or at least mono-substituted C₁₋₅alkylene group, C₂₋₅alkenylene group or C₂₋₅alkinylene group;
an unsubstituted or at least mono-substituted C₄₋₁₆ heterocycloalkyl radical or C₅₋₁₆ heterocycloalkenyl radical, which in each case may be condensed with an unsubstituted or at least mono-substituted mono- or polycyclic ring system and/or may be bridged by at least one unsubstituted or at least mono-substituted C₁₋₅ alkylene group and/or may be bonded via an unsubstituted or at least mono-substituted C₁₋₅ alkylene group, C₂₋₅alkenylene group or C₂₋₅alkinylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅ alkinylene group;
R¹⁶, R¹⁷, R¹⁸ and R¹⁹, independently of one another, in each case represent an unsubstituted or at least mono-substituted C₁₋₁₆ alkyl radical, C₂₋₁₆ alkenyl radical or C₂₋₁₆ alkinyl radical;
or an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅ alkylene group, C₂₋₅ alkenylene group or C₂₋₅ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅ alkylene group, C₂₋₅ alkenylene group or C₂₋₅alkinylene group;
and
R²¹ and R²², independently of one another, in each case represent a hydrogen atom;
an unsubstituted or at least mono-substituted C₁₋₁₆ alkyl radical, C₂₋₁₆ alkenyl radical or C₂₋₁₆ alkinyl radical;
an unsubstituted or at least mono-substituted C₃₋₁₆ cycloalkyl radical or C₄₋₁₆ cycloalkenyl radical, which in each case may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅ alkinylene group;
an unsubstituted or at least mono-substituted C₄₋₁₆ heterocycloalkyl radical or C₅-₁₆ heterocycloalkenyl radical, which in each case may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅alkinylene group;
an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical which together with an unsubstituted or at least mono-substituted C₃₋₁₆ cycloalkyl radical, C₄₋₁₆ cycloalkenyl radical, C₄₋₁₆ heterocycloalkyl radical or C₅₋₁₆ heterocycloalkenyl radical forms an unsubstituted or at least mono-substituted spirocyclic residue via a common ring atom;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted C₁₋₅alkylene group, C₂₋₅ alkenylene group or C₂₋₅ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted C₁₋₅alkylene group, C₂₋₅alkenylene group or C₂₋₅ alkinylene group;
whereby
the rings of the aforementioned ring system are in each case independently of one another 5- 6- or 7-membered and may in each case independently of one another optionally contain 1, 2 or 3 heteroatom(s) independently selected from the group consisting of nitrogen, oxygen and sulfur;
the aforementioned heteroaryl radicals in each case optionally contain 1, 2, 3 or 4 heteroatom(s) independently selected from the group consisting of nitrogen, oxygen and sulfur as ring member(s);
the aforementioned heterocycloalkyl radicals and heterocycloalkenyl radicals in each case optionally contain 1, 2, 3 or 4 heteroatom(s) independently selected from the group consisting of nitrogen, oxygen and sulfur as ring member(s);
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

3. Active substance combination according to claim 1, **characterized in that** for the compound (A) according to formula I
X is O or S;
R¹ represents a radical selected from the group consisting of phenyl and thienyl (thiophenyl), which may optionally be substituted with 1, 2, 3, 4 or 5 substituent(s) independently selected from the group consisting of -OH, -O-CH₃, -O-C₂H₅, F, Cl, Brand I;
R² represents a phenyl radical, which may be substituted with 1, 2, 3, 4 or 5 substituent(s) independently selected from the group consisting of -OH, -O-CH₃,-O-C₂H₅, F, Cl, Br and I;
R³ represents a radical selected from the group consisting of 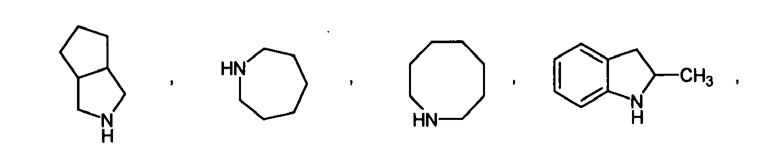 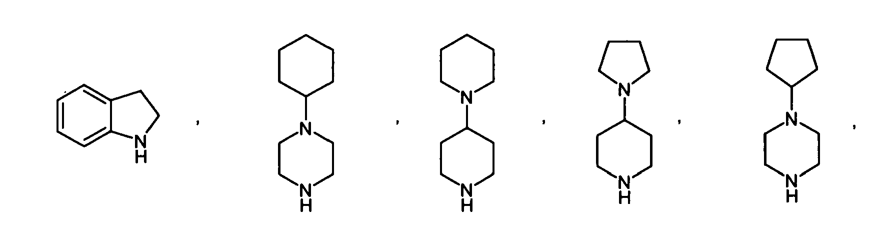 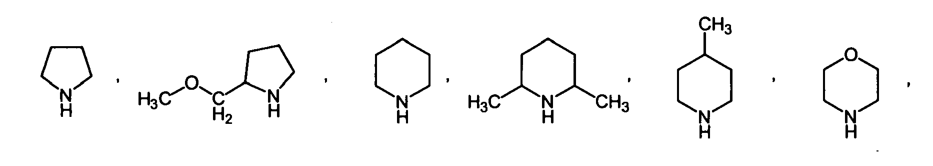 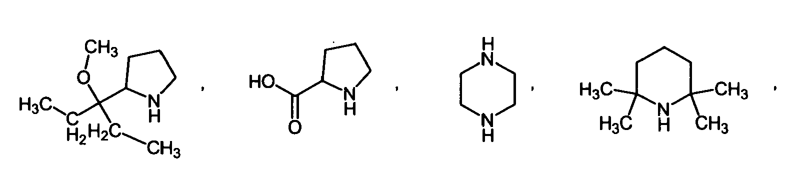 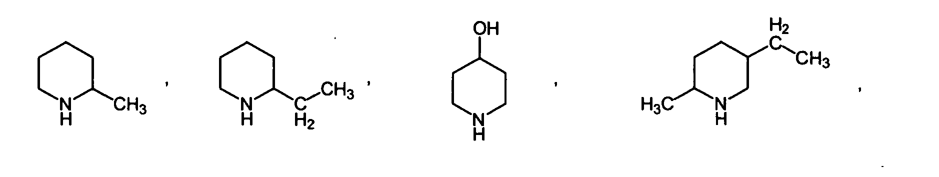 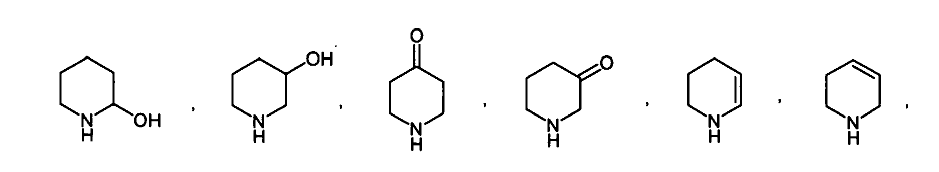 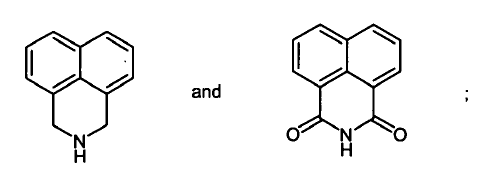 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a -OR⁶-moiety or a -NR⁷R⁸ moiety;
R⁴ represents H; F; Cl; Br; I; -OH, -CN; -C(=O)-H; -C(=O)-OH; -C(=O)-OR¹⁷;
a radical selected from the group consisting of methyl, -CF₃, -CH₂F, -CF₂H, -C₂F₅, ethyl, -CH₂-CN, -CH₂-OH, n-propyl, isopropyl, -CH₂-CH₂-CN, -CH₂-CH₂-OH, n-butyl, -CH₂-CH₂-CH₂-CN, -CH₂-CH₂-CH₂-OH, isobutyl, sec-butyl, tert-butyl, n-pentyl, 2-pentyl, 3-pentyl, neo-pentyl, n-hexyl, 2-hexyl and 3-hexyl;
a benzyl radical or a -O-R¹¹ moiety;
R⁵ represents H; F; Cl; Br;-C(=O)-OH: -C(=O)-OR¹⁷; or a radical selected from the group consisting of methyl, ethyl, n-propyl, iso-propyl and n-butyl;
or R⁴ and R⁵ together with the bridging carbon atom form a radical selected from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl;
R⁶ represents a hydrogen atom; a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;
R⁷ represents a hydrogen atom or a radical selected from the group consisting of methyl, ethyl, n-propyl, isopropyl, n-butyl, sec-butyl and tert-butyl;
R⁸ represents a radical selected from the group consisting of [1,2,3,4]-tetrahydronaphthyl, bicyclo[2.2.1]heptyl, cyclopropyl, cyclobutyl, cyclopentyl, cyclohexyl, cycloheptyl, cyclooctyl, cyclononyl, cyclodecyl, cycloundecyl and cyclododecyl, which may be bonded via a -(CH₂)-, -(CH₂)-(CH₂)- or -(CH₂)-(CH₂)-(CH₂)-group and/or subsituted with 1, 2, 3 or 4 substituents selected from the group consisting of methyl, ethyl, n-propyl, isopropyl, n-butyl, sec-butyl, tert-butyl, -OH, -O-CH₃ and -O-C₂H₅;
or a radical selected from the group consisting of 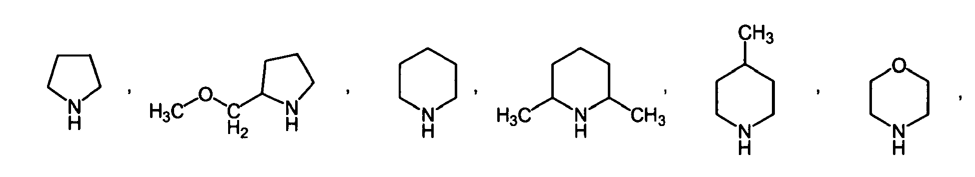 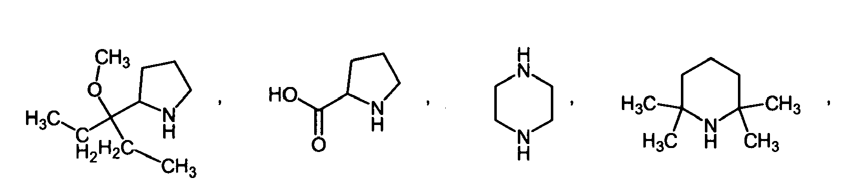 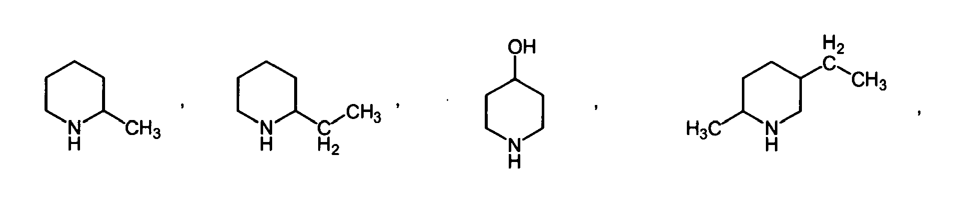 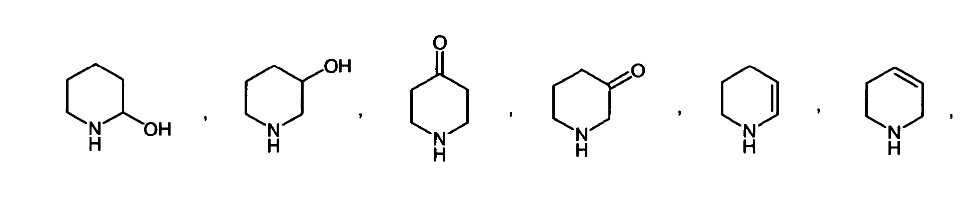 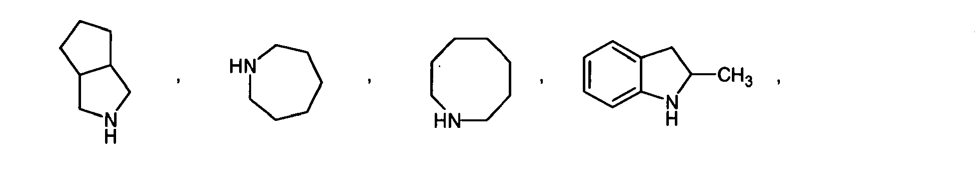 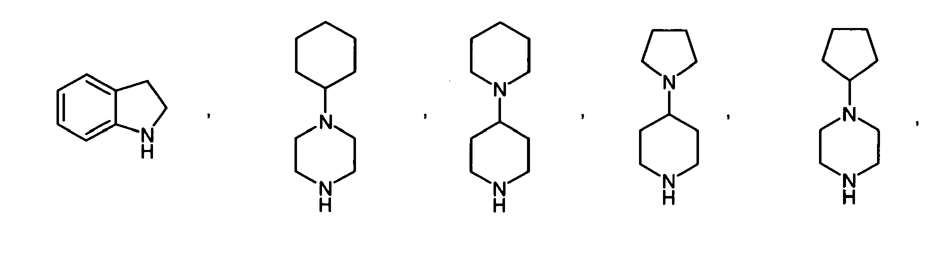 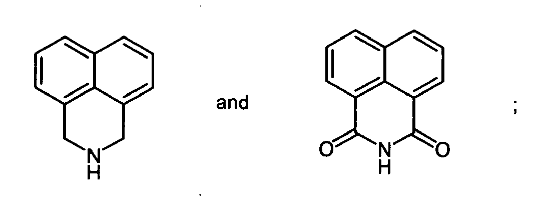 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
and
R¹¹ and R¹⁷, independently of one another, each represent a radical selected from the group consisting of methyl, ethyl, n-propyl, isopropyl, n-butyl, isobutyl, sec-butyl, tert-butyl, n-pentyl, 2-pentyl, 3-pentyl and neo-pentyl;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

4. Active substance combination according to claim 1, 2 or 3, **characterized in that** for the compound (A) of subgroup A1 according to formula I
X represents O
R¹ represents a phenyl ring, which is mono-substituted with a halogen atom, preferably a chlorine atom, in its 4-position,
R² represents a phenyl ring, which is di-substituted with two halogen atoms, preferably chlorine atoms, in its 2- and 4-position,
R³ represents a pyrrolidinyl group; a piperidinyl group; a piperazinyl group; a homo-piperazinyl group; a morpholinyl group; or an -NR⁷R⁸-moiety,
R⁴ represents a hydrogen atom,
R⁵ represents a hydrogen atom
R⁷ represents a hydrogen atom or a linear or branched C₁₋₆-alkyl group,
R⁸ represents a linear or branched C₁₋₆ alkyl group; an SO₂-R²⁰-moiety; a pyrrolidinyl group; a piperidinyl group; a piperazinyl group; a homo-piperazinyl group; a morpholinyl group; or a triazolyl group, whereby each of the heterocyclic rings may be substituted with one or more, identical or different, C₁₋₆-alkyl groups, and
R²⁰ represents a phenyl group, which is optionally substituted with one or more C₁₋₆ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

5. Active substance combination according to claim 4, **characterized in that** the compound (A) of subgroup A1 according to formula I is selected from the group consisting of:
N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenylyl -(2,4-dichlorophenyl)-4,5-dihydro-1H-pyrazole-3-carbonyl]-4-methylphenylsulfonamide,
optionally in the form of a corresponding N-oxide, a corresponding salt or a corresponding solvate.

6. Active substance combination according to claim 1, 2 or 3, **characterized in that** for the compound (A) of subgroup A2 according to formula Ia or Ib 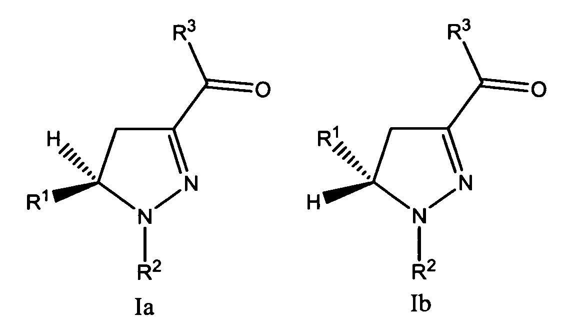 wherein
X represents O
R¹ represents a phenyl ring, which is mono-substituted with a halogen atom, preferably a chlorine atom, in its 4-position,
R² represents a phenyl ring, which is di-substituted with two halogen atoms, preferably chlorine atoms, in its 2- and 4-position,
R³ represents a pyrrolidinyl group, a piperidinyl group, a piperazinyl group, a homo-piperazinyl group, a morpholinyl group, or an -NR⁷R⁸-moiety,
R⁴ represents a hydrogen atom,
R⁵ represents a hydrogen atom,
R⁷ represents a hydrogen atom or a linear or branched C₁₋₆-alkyl group,
R⁸ represents a linear or branched C₁₋₆ alkyl group; an -SO₂-R²⁰-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, C₁₋₆-alkyl groups, and
R²⁰ represents a phenyl group, which is optionally substituted with one or more C₁₋₆ alkyl groups, which may be identical or different,
optionally in form of a corresponding N-oxide thereof, or a corresponding salt thereof, or a corresponding solvate thereof.

7. Active substance combination according to claim 6, **characterized in that** the compound (A) of subgroup A2 is a compound selected from:
• (R)-N-piperidinyl-5-(4-chlorophenyl)-1-(2,4-dichlorophenyl)-4,5-dihydro-1H-pyrazole-3-carboxamide and
• (S)-N-piperidinyl-5-(4-chlorophenyl)-1-(2,4-dichlorophenyl)-4,5-dihydro-1H-pyrazole-3-carboxamide;
optionally in form of a corresponding N-oxide thereof, or a corresponding salt thereof, or a corresponding solvate thereof.

8. Active substance combination according to claim 1, 2 or 3, **characterized in that** for the compound (A) of subgroup A3 according to formula I
X is O;
R¹ represents a radical selected from the group consisting of 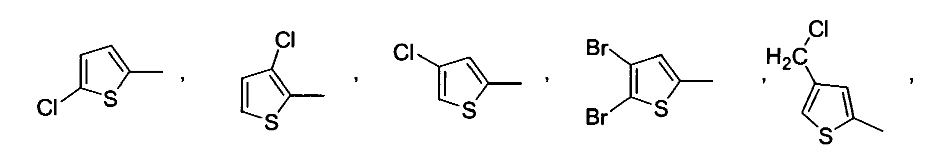 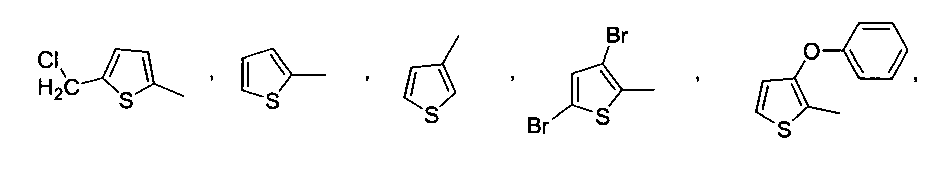 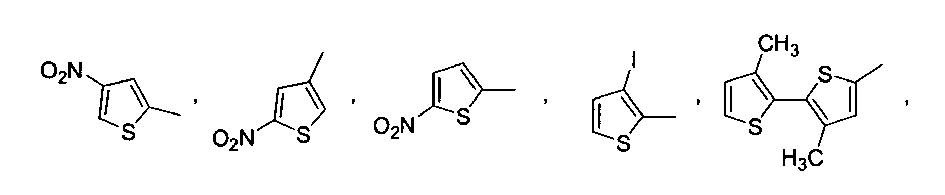 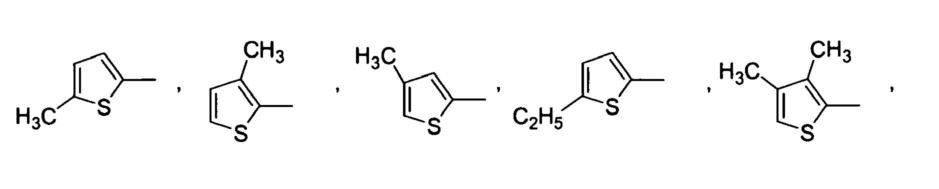 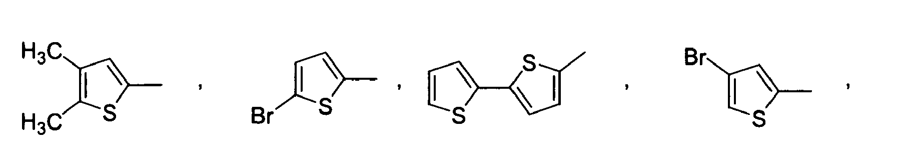 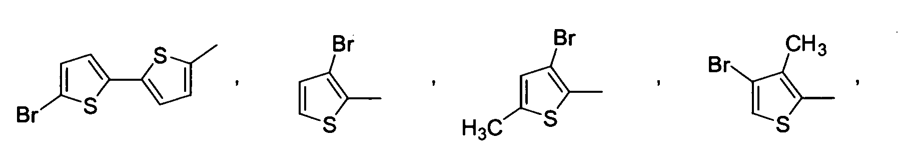 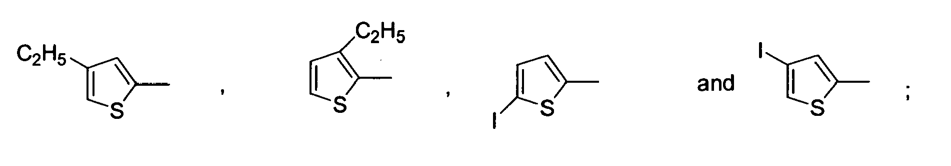 wherein the single line in each case represents the bond to the pyrazoline compound of general formula I;
R² represents a phenyl radical, which may be substituted with 1, 2, 3, 4 or 5 substituent(s) independently selected from the group consisting of F, Cl, Br and I;
R³ represents a radical selected from the group consisting 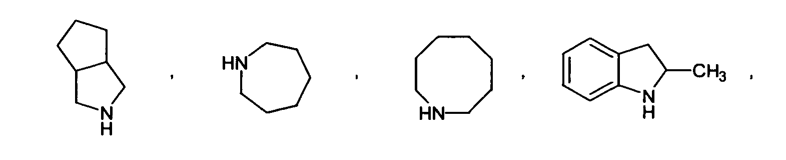 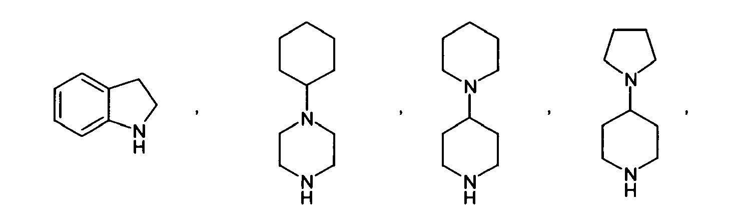 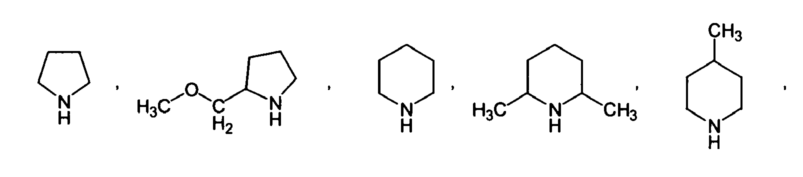 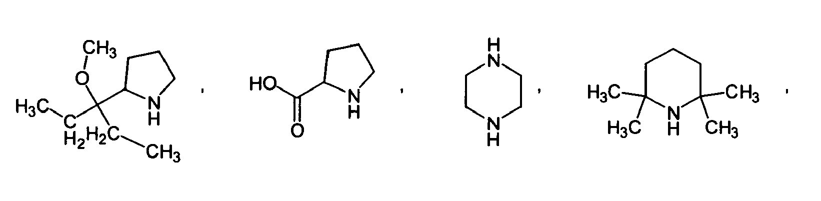 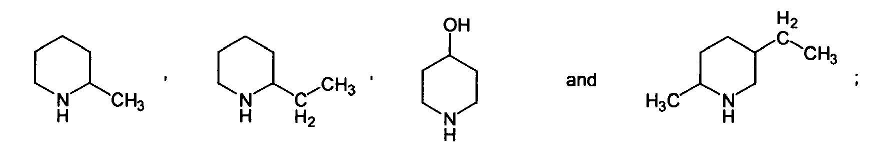 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a -O-R⁶ moiety or a -NR⁷R⁸-moiety;
R⁴ represents a hydrogen atom;
R⁵ represents a hydrogen atom;
R⁶ represents a hydrogen atom, a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;
R⁷ represents a hydrogen atom;
R⁸ a radical selected from the group consisting of cyclopropyl, cyclobutyl, cyclopentyl, cyclohexyl and cycloheptyl, which may be bonded via a -(CH₂)-, -(CH₂)-(CH₂)- or -(CH₂)-(CH₂)-(CH₂)-group;
or a radical selected from the group consisting of 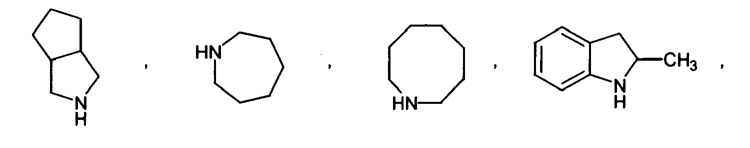 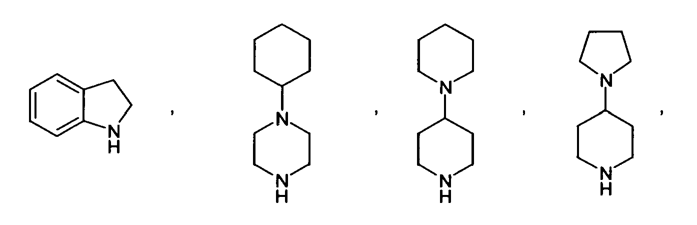 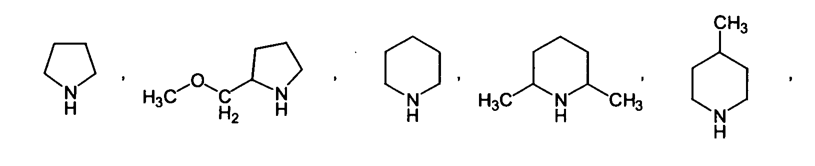 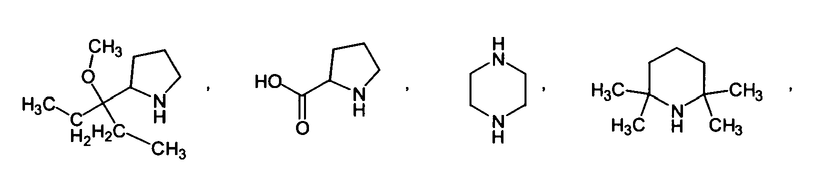 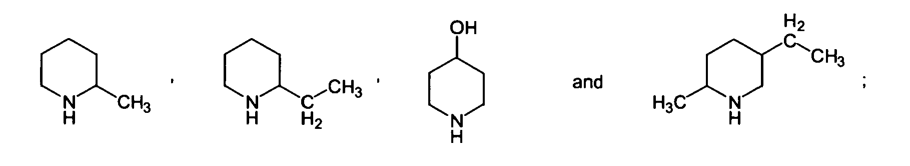 which is in each case bonded to the pyrazoline compound of general formula I in any position of the cyclic part of the aforementioned radicals including the NH-
groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

9. Active substance combination according to claim 8, **characterized in that** the compound (A) of subgroup A3 is a compound selected from:
[1] 5-(5-Chloro-thiophen-2-yl)-1-(2 ,4-dichloro-phenyl)-4,5-dihydro-1H-pyrazole-3-carboxylic acid piperidin-1-ylamide
[2] 5-(5-Chloro-thiophen-2-yl)-1-(2,4-dichloro-phenyl)-4,5-dihydro-1H-pyrazole-3-carboxylic acid azepan-1-ylamide
[3] 5-(5-Chloro-thiophen-2-yl)-1-(2,4-dichloro-phenyl)-4,5-dihydro-1H-pyrazole-3-carboxylic acid pyrrolidin-1-ylamide
[4] 5-(5-Chloro-thiophen-2-yl)-1-(2 ,4-dichloro-phenyl)-4,5-dihydro-1 H-pyrazole-3-carboxylic acid cyclohexyl-amide
[5] [5-(5-Chloro-thiophen-2-yl)-1-(2,4-dichloro-phenyl)-4,5-dihydro-1 H-pyrazol-3-yl]-pyrrolidin-1-yl-methanone
[6] [5-(5-Chloro-thiophen-2-yl)-1-(2,4-dichloro-phenyl)-4,5-dihydro-1H-pyrazol-3-yl]-(4-cyclohexyl-piperazin-1-yl)-methanone
[7] 5-(5-Chloro-thiophen-2-yl)-1-(2,4-dichloro-phenyl)-4,5-dihydro-1H-pyrazole-3-carboxylic acid cyclohexylamide
[8] 5-(5-Chloro-thiophen-2-yl)-1-(2,4-dichloro-phenyl)-4,5-dihydro-1H-pyrazole-3-carboxylic acid
[9] 5-(5-Chloro-thiophen-2-yl)-1-(2,4-dichloro-phenyl)-4,5-dihydro-1H-pyrazole-3-carboxylic acid methyl ester
[10] 5-(5-Chloro-thiophen-2-yl)-1-(2,4-dichloro-phenyl)-4,5-dihydro-1H-pyrazole-3-carboxylic acid ethyl ester
[11] 5-(5-Chloro-thiophen-2-yl)-1-(2,4-dichloro-phenyl)-4,5-dihydro-1H-pyrazole-3-carboxylic acid cyclohexyl ester
[12] 5-(3-Chloro-thiophen-2-yl)-1-(2,4-dichloro-phenyl)-4,5-dihydro-1H-pyrazole-3-carboxylic acid piperidin-1-ylamide
[13] 5-(5-Bromo-thiophen-2-yl)-1-(2,4-dichloro-phenyl)-4,5-dihydro-1H-pyrazole-3-carboxylic acid piperidin-1-ylamide
[14] 5-(5-Bromo-thiophen-2-yl)-1-(2,4-dichloro-phenyl)-4,5-dihydro-1H-pyrazole-3-carboxylic acid (4-methyl-piperidin-1-yl)-amide
[15] 5-(5-Bromo-thiophen-2-yl)-1-(2,4-dichloro-phenyl)-4,5-dihydro-1 H-pyrazole-3-carboxylic acid (hexahydro-cyclopenta[c]pyrrol-2-yl)-amide
[16] 5-(5-Bromo-thiophen-2-yl)-1-(2,4-dichloro-phenyl)-4,5-dihydro-1H-pyrazole-3-carboxylic acid (2,3-dihydroindol-1-yl)-amide
[17] 5-(5-Bromo-thiophen-2-yl)-1-(2,4-dichloro-phenyl)-4,5-dihydro-1H-pyrazole-3-carboxylic acid cyclopropylamide
[18] 5-(5-Bromo-thiophen-2-yl)-1-(2,4-dichloro-phenyl)-4,5-dihydro-1H-pyrazole-3-carboxylic acid cyclohexylmethyl-amide
[19] 5-(5-Bromo-thiophen-2-yl)-1-(2,4-dich)oro-phenyl)-4,5-dihydro-1 H-pyrazole-3-carboxylic acid
[20] 5-(5-Bromo-thiophen-2-yl)-1-(2,4-dichloro-phenyl)-4,5-dihydro-1H-pyrazole-3-carboxylic acid cyclohexyl ester
[21] 5-(5-Bromo-thiophen-2-yl)-1-(2,4-dichloro-phenyl)-4,5-dihydro-1H-pyrazole-3-carboxylic acid methyl ester
[22] 5-(5-Bromo-thiophen-2-yl)-1-(2,4-dichloro-phenyl)-4,5-dihydro-1H-pyrazole-3-carboxylic acid ethyl ester
[23] 5-(4-Bromo-thiophen-2-yl)-1-(2,4-dichloro-phenyl)-4,5-dihydro-1H-pyrazole-3-carboxylic acid piperidin-1-ylamide hydrochloride
[24] 5-(4-Bromo-thiophen-2-yl)-1-(2,4-dichloro-phenyl)-4,5-dihydro-1H-pyrazole-3-carboxylic acid azepan-1-ylamide
[25] 5-(4-Bromo-thiophen-2-yl)-1-(2,4-dichloro-phenyl)-4,5-dihydro-1H-pyrazole-3-carboxylic acid (4-methyl-piperidin-1-yl)-amide
[26] [5-(4-Bromo-thiophen-2-yl)-1-(2,4-dichloro-phenyl)-4,5-dihydro-1 H-pyra zol-3-yl]-piperidin-1-yl-methanone
[27] 5-(4-Bromo-thiophen-2-yl)-1-(2,4-dichloro-phenyl)-4,5-dihydro-1H-pyrazole-3-carboxylic acid
[28] 5-(4-Bromo-thiophen-2-yl)-1-(2,4-dichloro-phenyl)-4,5-dihydro-1H-pyrazole-3-carboxylic acid methyl ester
[29] 5-(4-Bromo-thiophen-2-yl)-1-(2,4-dichloro-phenyl)-4,5-dihydro-1H-pyrazole-3-carboxylic acid ethyl ester
[30] 5-(3-Bromo-thiophen-2-yl)-1-(2,4-dichloro-phenyl)-4,5-dihydro-1H-pyrazole-3-carboxylic acid piperidin-1-ylamide
[31] 5-(3-Bromo-5-methyl-thiophen-2-yl)-1-(2.4-dichloro-phenyl)-4.5-dihydro-1H-pyrazole-3-carboxylic acid piperidin-1-ylamide
[32] 5-(4-Bromo-3-methyl-thiophen-2-yl)-1-(2,4-dichloro-phenyl)-4.5-dihydro-1H-pyrazole-3-carboxylic acid piperidin-1-ylamide
[33] 5-(4,5-Dibromo-thiophen-2-yl)-1-(2 ,4-dichloro-phenyl)-4,5-dihydro-1H-pyrazole-3-carboxylic acid piperidin-1-ylamide
[34] 5-(3,5-Dibromo-thiophen-2-yl)-1-(2,4-dich)oro-phenyl)-4,5-dihydro-1H-pyrazole-3-carboxylic acid piperidin-1-ylamide
[35] 1-(2,4-Dichloro-phenyl)-5-(3-iodo-thiophen-2-yl)-4,5-dihydro-1H-pyrazole-3-carboxylic acid piperidin-1-ylamide
[36] 5-(4-Chloromethyl-thiophen-2-yl)-1-(2,4-dichloro-phenyl)-4,5-dihydro-1H-pyrazole-3-carboxylic acid piperidin-1-ylamide
[37] 1-(2,4-Dichloro-phenyl)-5-(3,4-dimethyl-thiophen-2-yl)-4,5-dihydro-1H-pyrazole-3-carboxylic acid piperidin-1-ylamide
[38] 1-(2,4-Dichloro-phenyl)-5-(4,5-dimethyl-thiophen-2-yl)-4,5-dihydro-1H-pyrazole-3-carboxylic acid piperidin-1-ylamide
[39] 5-[2,2']Bithiophenyl-5-yl-1-(2,4-dichloro-phenyl)-4,5-dihydro-1H-pyrazole-3-carboxylic acid piperidin-1-ylamide
[40] 1-(2,4-Dichloro-phenyl)-5-(5-methyl-thiophen-2-yl)-4,5-dihydro-1H-pyrazole-3-carboxylic acid piperidin-1-ylamide
[41] 1-(2,4-Dichloro-phenyl)-5-(3-methyl-thiophen-2-yl)-4,5-dihydro-1H-pyrazole-3-carboxylic acid piperidin-1-ylamide
[42] 1-(2,4-Dichloro-phenyl)-5-(5-ethyl-thiophen-2-yl)-4,5-dihydro-1H-pyrazole-3-carboxylic acid piperidin-1-ylamide
[43] 1-(2,4-Dichloro-phenyl)-5-(3,3'-dimethyl-[2,2']bithiophenyl-5-yl)-4,5-dihydro-1H-pyrazole-3-carboxylic acid piperidin-1-ylamide
[44] 1-(2,4-Dichloro-phenyl)-5-(5-nitro-thiophen-2-yl)-4,5-dihydro-1H-pyrazole-3-carboxylic acid piperidin-1-ylamide
[45] 1-(2,4-Dichloro-phenyl)-5-(4-nitro-thiophen-2-yl)-4,5-dihydro-1H-pyrazole-3-carboxylic acid piperidin-1-ylamide
[46] 1-(2,4-Dichloro-phenyl)-5-thiophen-2-yl-4,5-dihydro-1H-pyrazole-3-carboxylic acid piperidin-1-ylamide
[47] 1-(2,4-Dichloro-phenyl)-5-thiophen-3-yl-4,5-dihydro-1H-pyrazole-3-carboxylic acid piperidin-1-ylamide
and
[48] 1-(2,4-Dichloro-phenyl)-5-(5-nitro-thiophen-3-yl)-4,5-dihydro-1H-pyrazole-3-carboxylic acid piperidin-1-ylamide;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

10. Active substance combination according to claim 1, 2 or 3, **characterized in that** for the compound (A) of subgroup A4 according to formula I
X represents S;
R¹ represents a phenyl radical which may be substituted with 1, 2, 3, 4 or 5 substituent(s) independently selected from the group consisting of F, Cl, Br, I, -O-CH₃ and -O-C₂H₅;
R² represents a phenyl radical which may be substituted with 1, 2, 3, 4 or 5 substituent(s) independently selected from the group consisting of F, Cl, Br and I;
R³ represents a radical selected from the group consisting of 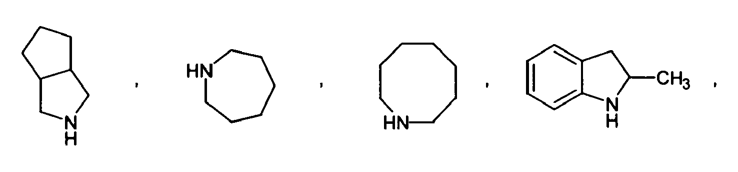 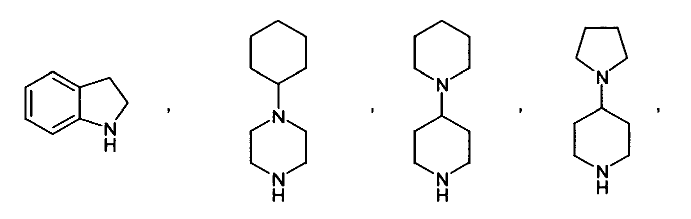 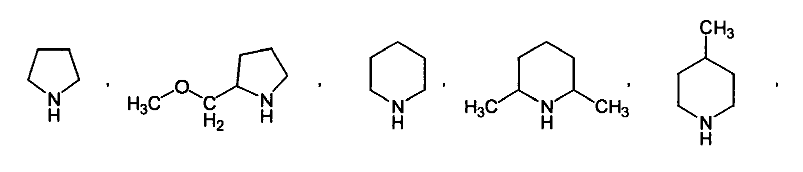 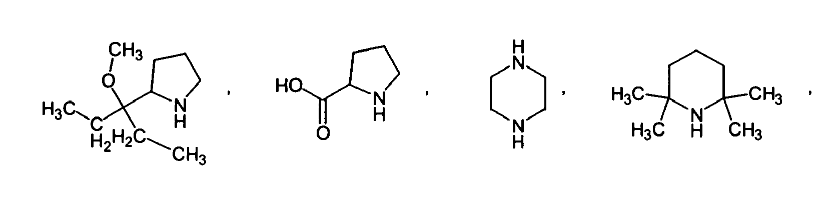 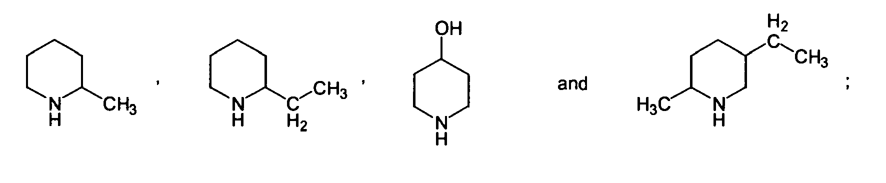 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a -O-R⁶ moiety or a -NR⁷R⁸-moiety;
R⁴ represents a hydrogen atom;
R⁵ represents a hydrogen atom;
R⁶ represents a hydrogen atom or a radical selected from the group consisting of cyclopropyl, cyclobutyl, cyclopentyl, cyclohexyl and cycloheptyl;
R⁷ represents a hydrogen atom;
R⁸ a radical selected from the group consisting of cyclopropyl, cyclobutyl, cyclopentyl, cyclohexyl and cycloheptyl, which may be bonded via a-(CH₂)-, -(CH₂)-(CH₂)- or -(CH₂)-(CH₂)-(CH₂)-group;
or a radical selected from the group consisting of 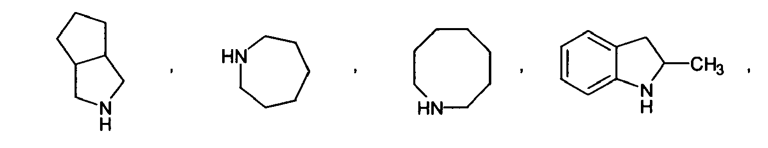 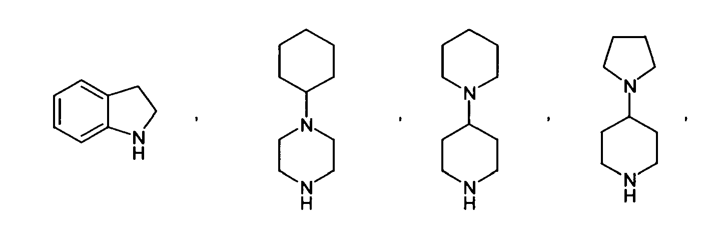 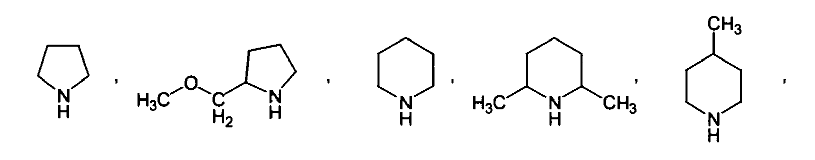 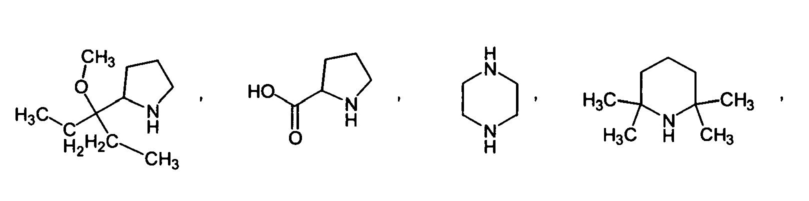 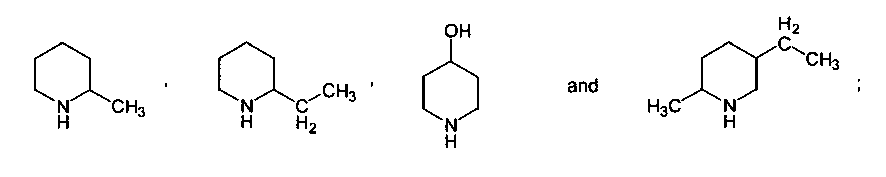 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

11. Active substance combination according to claim 10, **characterized in that** the compound (A) of subgroup A4 is a compound selected from:
[1] 5-(4-Chloro-phenyl)-1-(2,4-dichloro-phenyl)-4,5-dihydro-1H-pyrazole-3-carbothioic acid piperidin-1-ylamide
[2] 5-(4-Bromo-phenyl)-1-(2,4-dichloro-phenyl)-4,5-dihydro-1H-pyrazole-3-carbothioic acid piperidin-1-ylamide
[3] 1-(2,4-Dichloro-phenyl)-5-(4-iodo-phenyl)-4,5-dihydro-1H-pyrazole-3-carbothioic acid piperidin-1-ylamide
[4] 1-(2,4-Dichloro-phenyl)-5-(4-fluoro-phenyl)-4,5-dihydro-1H-pyrazole-3-carbothioic acid piperidin-1-ylamide
[5] 1-(2,4-Dichloro-phenyl)-5-(4-methoxy)-4,5-dihydro-1H-pyrazole-3-carbothioic acid piperidin-1-ylamide
[6] 5-(4-Chloro-phenyl)-1-(2,4-dichloro-phenyl)-4,5-dihydro-1H-pyrazole-3-carbothioic acid morpholin-4-ylamide
[7] 5-(4-Bromo-phenyl)-1-(2,4-dichloro-phenyl)-4,5-dihydro-1H-pyrazole-3-carbothioic acid morpholin-4-ylamide
[8] 1-(2,4-Dichloro-phenyl)-5-(4-iodo-phenyl)-4,5-dihydro-1H-pyrazole-3-carbothioic acid morpholin-4-ylamide
[9] 1-(2,4-Dichloro-phenyl)-5-(4-fluoro-phenyl)-4,5-dihydro-1H-pyrazole-3-carbothioic acid morpholin-4-ylamide
[10] 1-(2,4-Dichloro-phenyl)-5-(4-methoxy-phenyl)-4,5-dihydro-1H-pyrazole-3-carbothioic acid morpholin-4-ylamide
[11] 5-(4-Chloro-phenyl)-1-(2,4-dichloro-phenyl)-4,5-dihydro-1H-pyrazole-3-carbothioic acid (hexahydro-cyclopenta[c]pyrrol-2-yl)-amide
[12] 5-(4-Bromo-phenyl)-1-(2,4-dichloro-phenyl)-4,5-dihydro-1H-pyrazole-3-carbothioic acid (hexahydro-cyclopenta[c]pyrrol-2-yl)-amide
[13] 1-(2,4-Dichloro-phenyl)-5-(4-fluoro-phenyl)-4,5-dihydro-1H-pyrazole-3-carbothioic acid (hexahydro-cyclopenta[c]pyrrol-2-yl)-amide
[14] 1-(2,4-Dichloro-phenyl)-5-(4-iodo-phenyl)-4,5-dihydro-1H-pyrazole-3-carbothioic acid (hexahydro-cyclopenta[c]pyrrol-2-yl)-amide
[15] 1-(2,4-Dichloro-phenyl)-5-(4-methoxy-phenyl)-4,5-dihydro-1H-pyrazole-3-carbothioic acid (hexahydro-cyclopenta[c]pyrrol-2-yl)-amide
[16] 1-(2,4-Dichloro-phenyl)-5-(4-iodo-phenyl)-4,5-dihydro-1H-pyrazole-3-carbothioic acid azepan-1-ylamide
[17] 5-(4-Chloro-phenyl)-1-(2,4-dichloro-phenyl)-4,5-dihydro-1H-pyrazole-3-carbothioic acid azepan-1-ylamide
[18] 5-(4-Bromo-phenyl)-1-(2,4-dichloro-phenyl)-4,5-dihydro-1H-pyrazole-3-carbothioic acid azepan-1-ylamide
[19] 1-(2,4-Dichloro-phenyl)-5-(4-fluoro-phenyl)-4,5-dihydro-1H-pyrazole-3-carbothioic acid azepan-1-ylamide
[20] 1-(2,4-Dichloro-phenyl)-5-(4-methoxy-phenyl)-4,5-dihydro-1H-pyrazole-3-carbothioic acid azepan-1-ylamide
[21] 1-(2,4-Dichloro-phenyl)-5-(4-methoxy-phenyl)-4,5-dihydro-1H-pyrazole-3-carbothioic acid pyrrolidin-1-ylamide
[22] 5-(4-Bromo-phenyl)-1-(2,4-dichloro-phenyl)-4,5-dihydro-1H-pyrazole-3-carbothioic acid pyrrolidin-1-ylamide
[23] 5-(4-Chloro-phenyl)-1-(2,4-dichloro-phenyl)-4,5-dihydro-1H-pyrazole-3-carbothioic acid pyrrolidin-1-ylamide
[24] 1-(2,4-Dichloro-phenyl)-5-(4-fluoro-phenyl)-4,5-dihydro-1H-pyrazole-3-carbothioic acid pyrrolidin-1-ylamide
[25] 1-(2,4-Dichloro-phenyl)-5-(4-iodo-phenyl)-4,5-dihydro-1H-pyrazole-3-carbothioic acid pyrrolidin-1-ylamide
[26] 1-(2,4-Dichloro-phenyl)-5-(4-methoxy-phenyl)-4,5-dihydro-1H-pyrazole-3-carbothioic acid (4-cyclopentyl-piperazin-1-yl)-amide
[27] 5-(4-Chloro-phenyl)-1-(2,4-dichloro-phenyl)-4,5-dihydro-1H-pyrazole-3-carbothioic acid (4-cyclopentyl-piperazin-1-yl)-amide
[28] 5-(4-Bromo-phenyl)-1-(2,4-dichloro-phenyl)-4,5-dihydro-1H-pyrazole-3-carbothioic acid (1H,3H-benzo[de]isoquinolin-2-yl)-amide
[29] 5-(4-Chloro-phenyl)-1-(2,4-dichloro-phenyl)-4,5-dihydro-1H-pyrazole-3-carbothioic acid (1 H,3H-benzo[de]isoquinolin-2-yl)-amide
[30] 5-(4-Chloro-phenyl)-1-(2,4-dichloro-phenyl)-4,5-dihydro-1H-pyrazole-3-carbothioic acid cyclopentylamide
[31] Azocan-1-yl-[5-(4-chloro-phenyl)-1-(2,4-dichloro-phenyl)-4,5-dihydro-1H-pyrazole-3-yl]-methanethione
and
[32] [5-(4-Chloro-phenyl)-1-(2,4-dichloro-phenyl)-4,5-dihydro-1H-pyrazole-3-yl]-piperidin-1-yl-methanethione;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

12. Active substance combination according to claim 1, 2 or 3, **characterized in that** for the compound (A) of subgroup A5.1 according to formula I
X represents O
R¹ represents a phenyl radical that is substituted with a hydroxy, fluorine, chlorine, bromine or iodine atom or a -O-CH₃-group in the para-(4-)-position of the phenyl radical;
R² represents a (2,4)-dichloro-phenyl radical;
R³ represents a radical selected from the group consisting of 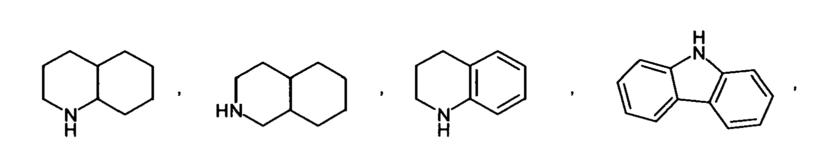 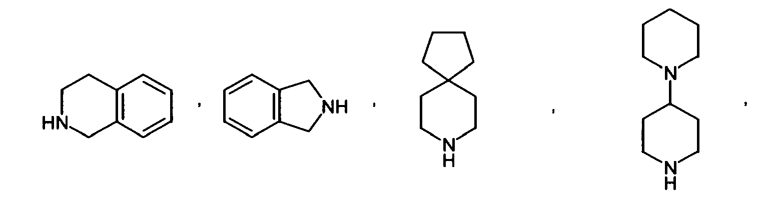 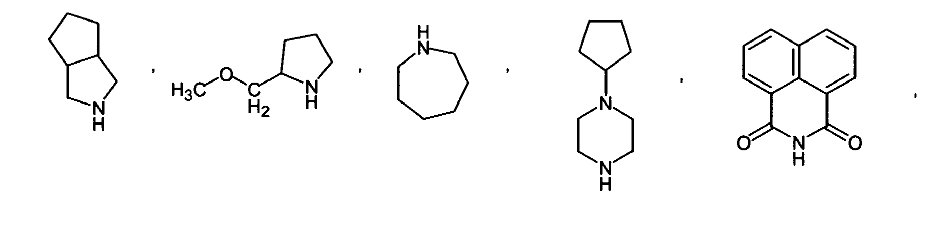 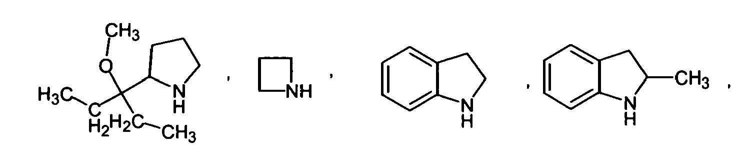 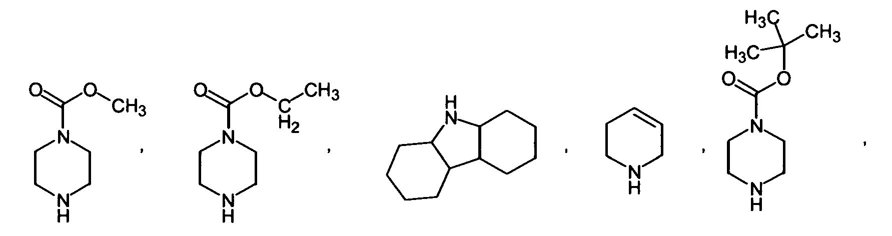 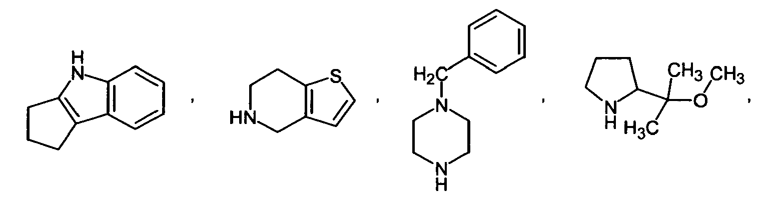 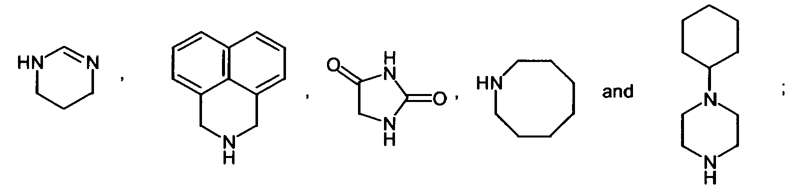 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
or a -NR⁷R⁸ moiety;
R⁴ represents a hydrogen atom;
R⁵ represents a hydrogen atom;
R⁷ represents a hydrogen atom;
R⁸ represents a radical selected from the group consisting of 2-heptyl, 3-heptyl, 4-heptyl, 2-octyl, 3-octyl, 4-octyl, -CH₂-N(CH₃)₂, -CH₂-N(C₂H₅)₂, -CH₂-CH₂-N(CH₃)₂, -CH₂-CH₂-N(C₂H₅)₂, -CH₂-CH₂-CH₂-N(CH₃)₂ and -CH₂-CH₂-CH₂-N(C₂H₅)₂;
a radical selected from the group consisting of adamantyl, bicyclo[2.2.1]heptyl, bicyclo[3.1.1]heptyl, (1,7,7)-trimethyl-bicyclo[2.2.1]heptyl, [1,2,3,4]-tetrahydronaphthyl, cyclononyl, cyclodecyl, cycloundecyl and cyclododecyl;
a radical selected from the group consisting of cyclopropyl, cyclobutyl, cyclopentyl, cyclohexyl, cycloheptyl, cyclooctyl, pyrrolidinyl, piperidinyl, piperazinyl, homopiperazinyl, morpholinyl, azepanyl, diazepanyl and azocanyl, which is bonded via a -(CH₂)-, -(CH₂)-(CH₂)- or -(CH₂)-(CH₂)-(CH₂)-group;
a substituted radical selected from the group consisting of cyclopentyl, cyclohexyl and cyloheptyl which is substituted with a -O-Benzyl radical;
or a radical selected from the group consisting of 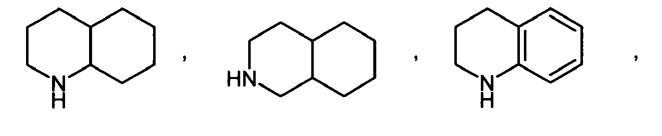 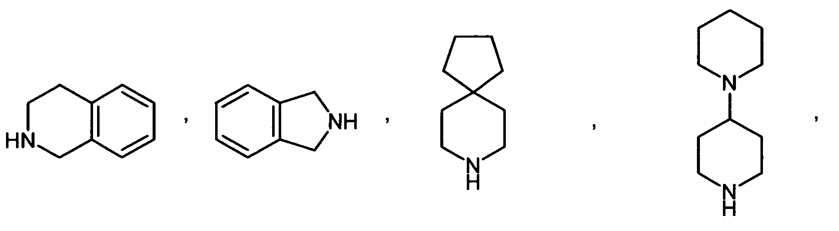 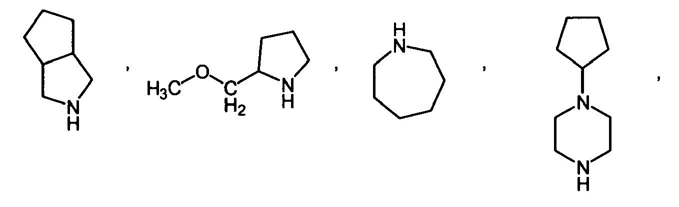 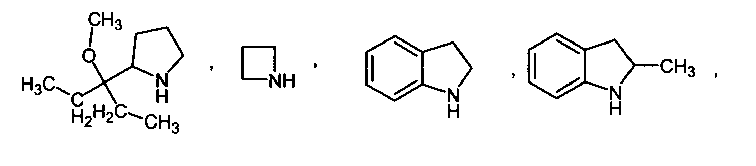 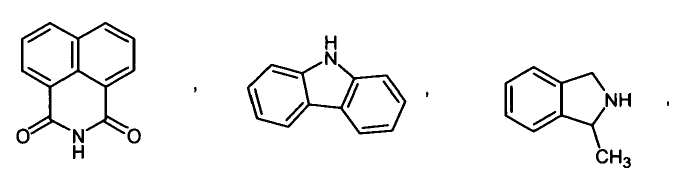 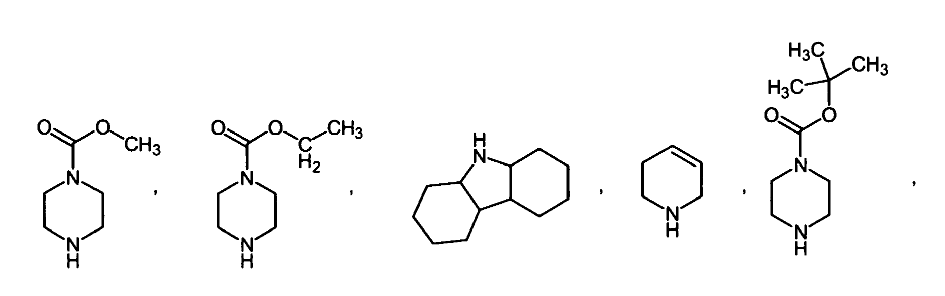 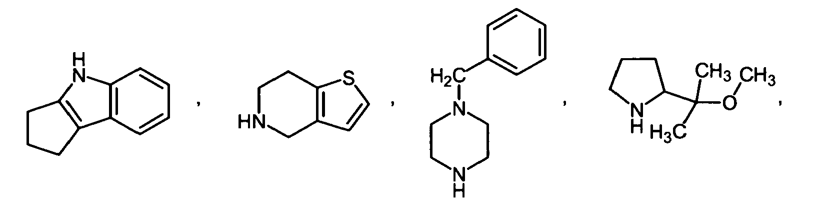 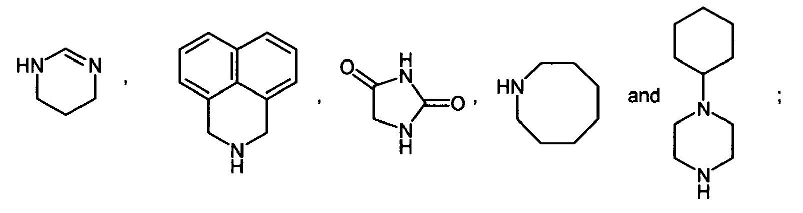 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof. acceptable salt thereof, or a corresponding solvate thereof.

13. Active substance combination according to claim 12, **characterized in that** the compound (A) of subgroup A5.1 is a compound selected from:
[10] 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide
[13] 5-(4-Chloro-phenyl)-1-(2,4-dichloro-phenyl)-4,5-dihydro-1H-pyrazole-3-carboxylic acid [2-(S)-(1-methoxy-1-methyl-ethyl)-pyrrolidin-1-yl]-amide
[14] 5-(4-Chloro-phenyl)-1-(2,4-dichloro-phenyl)-4,5-dihydro-1H-pyrazole-3-carboxylic acid [2-(R)-(1-methoxy-1-methyl-ethyl)-pyrrolidin-1-yl]-amide
[15] 5-(4-Chloro-phenyl)-1-(2,4-dichloro-phenyl)-4,5-dihydro-1H-pyrazole-3-carboxylic acid [2-(1-methoxy-1-methyl-ethyl)-pyrrolidin-1 -yl]-amide
[16] 5-(4-Chloro-phenyl)-1-(2,4-dichloro-phenyl)-4,5-dihydro-1H-pyrazole-3-carboxylic acid azepan-1-ylamide
[21] [5-(4-Chloro-phenyl)-1-(2,4-dichloro-phenyl)-4,5-dihydro-1H-pyrazole-3-yl]-(4-cyclohexyl-piperazin-1-yl)-methanone
[22] [5-(4-Chloro-phenyl)-1-(2,4-dichloro-phenyl)-4,5-dihydro-1H-pyrazole-3-yl]-(octahydro-isoquinolin-2-yl)-methanone
[24] 5-(4-Chloro-phenyl)-1-(2,4-dichloro-phenyl)-4,5-dihydro-1H-pyrazole-3-carboxylic acid (1,3-dioxo-1H,3H-benzo[de]isoquinolin-2-yl)-amide
[25] 5-(4-Chloro-phenyl)-1-(2,4-dichloro-phenyl)-4,5-dihydro-1H-pyrazole-3-carboxylicacid (1 H,3H-benzo[de]isoquinolin-2-yl)-amide
[26] 5-(4-Chloro-phenyl)-1-(2,4-dichloro-phenyl)-4,5-dihydro-1H-pyrazole-3-carboxylic acid (4-cyclopentyl-piperazin-1-yl)-amide
[27] 5-(4-Chloro-phenyl)-1-(2 ,4-dichloro-phenyl)-4,5-dihydro-1H-pyrazole-3-carboxylic acid (2-methyl-2,3-dihydro-indol-1-yl)-amide
[28] 5-(4-Chloro-phenyl)-1-(2,4-dichloro-phenyl)-4,5-dihydro-1H-pyrazole-3-carboxylic acid (2,3-dihydro-indol-1-yl)-amide
[32] 5-(4-Chloro-phenyl)-1-(2,4-dichloro-phenyl)-4,5-dihydro-1H-pyrazole-3-carboxylic acid cyclohexylmethyl-amide
[35] 5-(4-Chloro-phenyl)-1-(2,4-dichloro-phenyl)-4,5-dihydro-1H-pyrazole-3-carboxylic acid (1-methyl-hexyl)-amide
[38] Azocan-1-yl-[5-(4-chloro-phenyl)-1-(2,4-dichloro-phenyl)-4,5-dihydro-1H-pyrazol-3-yl]-methanone
[39] [5-(4-Chloro-phenyl)-1-(2,4-dichloro-phenyl)-4,5-dihydro-1H-pyrazole-3-yl]-(1,3-dihydro-isoindol-2-yl)-methanone
[40] Azetidin-1-yl-[5-(4-chloro-phenyl)-1-(2,4-dichloro-phenyl)-4,5-dihydro-1H-pyrazol-3-yl]-methanone
[42] [1,4']Bipiperidin-1'-yl-[5-(4-chloro-phenyl)-1-(2,4-dichloro-phenyl)-4,5-dihydro-1H-pyrazol-3-yl]-metha none
[44] 4-[5-(4-Chloro-phenyl)-1-(2,4-dichloro-phenyl)-4,5-dihydro-1H-pyrazole-3-carbonyl]-piperazine-1-carboxylic acid ethyl ester
[45] [5-(4-Chloro-phenyl)-1-(2,4-dichloro-phenyl)-4,5-dihydro-1H-pyrazole-3-yl]-(3,4-dihydro-1H-isoquinolin-2-yl)-methanone
[48] 5-(4-Chloro-phenyl)-1-(2,4-dichloro-phenyl)-4,5-dihydro-1H-pyrazole-3-carboxylic acid (2,4-dioxo-imidazolidin-1-yl)-amide
[49] 5-(4-Chloro-phenyl)-1-(2,4-dichloro-phenyl)-4,5-dihydro-1H-pyrazole-3-carboxylic acid cyclododecylamide
[52] (4-Benzyl-piperazin-1-yl)-[5-(4-chloro-phenyl)-1-(2,4-dichloro-phenyl)-4,5-dihydro-1H-pyrazol-3-yl]-methanone
[56] [5-(4-Chloro-phenyl)-1-(2,4-dichloro-phenyl)-4,5-dihydro-1H-pyrazole-3-yl]-(6,7-dihydro-4H-thieno[3,2-c]pyridin-5-yl)-methanone
[58] 5-(4-Chloro-phenyl)-1-(2,4-dichloro-phenyl)-4,5-dihydro-1H-pyrazole-3-carboxylic acid (1,7,7-trimethyl-bicyclo[2.2.1]hept-2-yl)-amide
[63] 5-(4-Bromo-phenyl)-1-(2,4-dichloro-phenyl)-4,5-dihydro-1H-pyrazole-3-carboxylic acid (hexahydro-cyclopenta[c]pyrrol-2-yl)-amide
[64] 5-(4-Bromo-phenyl)-1-(2,4-dichloro-phenyl)-4,5-dihydro-1H-pyrazole-3-carboxylic acid (4-cyclopentyl-piperazin-1-yl)-amide
[65] 5-(4-Bromo-phenyl)-1-(2,4-dichloro-phenyl)-4,5-dihydro-1H-pyrazole-3-carboxylic acid (1 H,3H-benzo[de]isoquinolin-2-yl)-amide
[66] 5-(4-Bromo-phenyl)-1-(2,4-dichloro-phenyl)-4,5-dihydro-1H-pyrazole-3-carboxylic acid (2,3-dihydro-indol-1-yl)-amide hydrochloride
[68] 5-(4-Bromo-phenyl)-1-(2,4-dichloro-phenyl)-4,5-dihydro-1H-pyrazole-3-carboxylic acid azepan-1-yl-amide
[71] 5-(4-Chloro-phenyl)-1-(2,4-dichloro-phenyl)-4,5-dihydro-1H-pyrazole-3-carboxylic acid (2-morpholin-4-yl-ethyl)-amide
[73] 5-(4-Chloro-phenyl)-1-(2,4-dichloro-phenyl)-4,5-dihydro-1H-pyrazole-3-carboxylic acid (3-dimethylamino-propyl)-amide
[74] [5-(4-Chloro-phenyl)-1-(2,4-dichloro-phenyl)-4,5-dihydro-1H-pyrazole-3-yl]-(3,6-dihydro-2H-pyridin-1-yl)-methanone
[75] [5-(4-Chloro-phenyl)-1-(2,4-dichloro-phenyl)-4,5-dihydro-1H-pyrazole-3-yl]-(5,6-dihydro-4H-pyrimidin-1-yl)-methanone
[76] 5-(4-Chloro-phenyl)-1-(2,4-dichloro-phenyl)-4,5-dihydro-1H-pyrazole-3-carboxylic acid (1,2,3,4-tetrahydro-naphthalen-1-yl)-amide
[78] [5-(4-Chloro-phenyl)-1-(2,4-dichloro-phenyl)-4,5-dihydro-1H-pyrazole-3-yl]-(2,3-dihydro-1H-cyclopenta[b]indol-4-yl)-methanone
[81] [5-(4-Chloro-phenyl)-1-(2,4-dichloro-phenyl)-4,5-dihydro-1H-pyrazole-3-yl]-(3,4-dihydro-2H-quino)in-1-yl)-methanone
[84] 5-(4-Chloro-phenyl)-1-(2,4-dichloro-phenyl)-4,5-dihydro-1H-pyrazole-3-carboxylic acid (2-cyclohexyl-ethyl)-amide
[85] [5-(4-Chloro-phenyl)-1-(2,4-dichloro-phenyl)-4,5-dihydro-1H-pyrazole-3-yl]-(dodecahydro-carbazol-9-yl)-methanone
[87] 5-(4-Chloro-phenyl)-1-(2,4-dichloro-phenyl)-4,5-dihydro-1H-pyrazole-3-carboxylic acid (2-cyclopentyl-ethyl)-amide
[88] 5-(4-Chloro-phenyl)-1-(2,4-dichloro-phenyl)-4,5-dihydro-1H-pyrazole-3-carboxylic acid bicyclo[2.2.1]hept-2-ylamide
[91] 1-(2,4-Dichloro-phenyl)-5-(4-fluoro-phenyl)-4,5-dihydro-1H-pyrazole-3-carboxylic acid (hexahydro-cyclopenta[c]pyrrol-2-yl)-amide hydrochloride
[92] 1-(2,4-Dichloro-phenyl)-5-(4-fluoro-phenyl)-4,5-dihydro-1H-pyrazole-3-carboxylic acid (4-cyclopentyl-piperazin-1-yl)-amide
[93] 1-(2,4-Dichloro-phenyl)-5-(4-fluoro-phenyl)-4,5-dihydro-1H-pyrazole-3-carboxylic acid (1 H,3H-benzo[de]isoquinolin-2-yl)-amide
[94] 1-(2,4-Dichloro-phenyl)-5-(4-fluoro-phenyl)-4,5-dihydro-1H-pyrazole-3-carboxylic acid (2,3-dihydro-indol-1-yl)-amide
[96] 1-(2,4-Dichloro-phenyl)-5-(4-fluoro-phenyl)-4,5-dihydro-1H-pyrazole-3-carboxylic acid azepan-1-yl-amide
[100] 1-(2,4-Dichloro-phenyl)-5-(4-iodo-phenyl)-4,5-dihydro-1H-pyrazole-3-carboxylic acid (hexahydro-cyclopenta[c]pyrrol-2-yl)-amide
[101] 1-(2,4-Dichloro-phenyl)-5-(4-iodo-phenyl)-4,5-dihydro-1H-pyrazole-3-carboxylic acid (4-cyclopentyl-piperazin-1-yl)-amide
[102] 1-(2,4-Dichloro-phenyl)-5-(4-iodo-phenyl)-4,5-dihydro-1H-pyrazole-3-carboxylic acid (1H,3H-benzo[de]isoquinolin-2-yl)-amide
[103] 1-(2,4-Dichloro-phenyl)-5-(4-iodo-phenyl)-4,5-dihydro-1H-pyrazole-3-carboxylic acid (2,3-dihydro-indol-1-yl)-amide
[105] 1-(2,4-Dichloro-phenyl)-5-(4-iodo-phenyl)-4,5-dihydro-1H-pyrazole-3-carboxylic acid azepan-1-ylamide;
[109] 1-(2,4-Dichloro-phenyl)-5-(4-methoxy-phenyl)-4,5-dihydro-1H-pyrazole-3-carboxylic acid (hexahydro-cyclopenta[c]pyrrol-2-yl)-amide hydrochloride
[110] 1-(2,4-Dichloro-phenyl)-5-(4-methoxy-phenyl)-4,5-dihydro-1H-pyrazole-3-carboxylic acid (4-cyclopentyl-piperazin-1-yl)-amide
[111] 1-(2,4-Dichloro-phenyl)-5-(4-methoxy-phenyl)-4,5-dihydro-1H-pyrazole-3-carboxylic acid (1H,3H-benzo[de]isoquinolin-2-yl)-amide
[112] 1-(2,4-Dichloro-phenyl)-5-(4-methoxy-phenyl)-4,5-dihydro-1H-pyrazole-3-carboxylic acid (2,3-dihydro-indol-1-yl)-amide hydrochloride
[114] 1-(2,4-Dichloro-phenyl)-5-(4-methoxy-phenyl)-4,5-dihydro-1H-pyrazole-3-carboxylic acid azepan-1-ylamide hydrochloride
[145] 5-(4-Chloro-phenyl)-1-(2,4-dichloro-phenyl)-4,5-dihydro-1H-pyrazole-3-carboxylic acid azocan-1-ylamide hydrochloride
[146] 5-(4-bromo-phenyl)-1-(2,4-dichbro-phenyl)-4,5-dihydro-1H-pyrazole-3-carboxylic acid azocan-1-ylamide
[147] 5-(4-fluoro-phenyl)-1-(2,4-dichloro-phenyl)-4,5-dihydro-1H-pyrazole-3-carboxylic acid azocan-1-ylamide
[148] 5-(4-methoxy-phenyl)-1-(2,4-dichloro-phenyl)-4,5-dihydro-1 H-pyrazole-3-carboxylic acid azocan-1-ylamide
[149] 5-(4-hydroxy-phenyl)-1-(2,4-dichloro-phenyl)-4,5-dihydro-1H-pyrazole-3-carboxylic acid azocan-1-ylamide hydrochloride
[150] 5-(4-iodo-phenyl)-1-(2,4-dichloro-phenyl)-4,5-dihydro-1H-pyrazole-3-carboxylic acid azocan-1-ylamide
[151] N-((1S,2S)-2-(benzyloxy)cyclohexyl)-5-(4-chlorophenyl)-1-(2,4-dichlorophenyl)-4,5-dihydro-1 H-pyrazole-3-carboxamide
[158] 5-(4-Chloro-phenyl)-1-(2,4-dichloro-phenyl)-4,5-dihydro-1H-pyrazole-3-carboxylic acid adamantan-1-ylamide
[159] (R)-5-(4-Ch)oro-phenyl)-1-(2.4-dich)oro-phenyl)-4,5-dihydro-1H-pyrazole-3-carboxylic acid adamantan-1-ylamide
[160] (S)-5-(4-Chloro-phenyl)-1-(2,4-dichloro-phenyl)-4,5-dihydro-1H-pyrazole-3-carboxylic acid adamantan-1-ylamide
[161] 5-(4-bromo-phenyl)-1-(2,4-dichloro-phenyl)-4,5-dihydro-1H-pyrazole-3-carboxylic acid adamantan-1-ylamide
[162] 5-(4-fluoro-phenyl)-1-(2,4-dichloro-phenyl)-4,5-dihydro-1 H-pyrazole-3-carboxylic acid adamantan-1-ylamide
[163] 5-(4-methoxy-phenyl)-1-(2,4-dichloro-phenyl)-4,5-dihydro-1H-pyrazole-3-carboxylic acid adamantan-1-ylamide
[164] 5-(4-hydroxy-phenyl)-1-(2,4-dichloro-phenyl)-4,5-dihydro-1H-pyrazole-3-carboxylic acid adamantan-1-ylamide
[165] 5-(4-iodo-phenyl)-1-(2,4-dichloro-phenyl)-4,5-dihydro-1H-pyrazole-3-carboxylic acid adamantan-1-ylamide
[166] 5-(4-Chloro-phenyl)-1-(2,4-dichloro-phenyl)-4,5-dihydro-1H-pyrazole-3-carboxylic acid adamantan-2-ylamide
[167] 5-(4-bromo-phenyl)-1-(2,4-dichloro-phenyl)-4,5-dihydro-1H-pyrazole-3-carboxylic acid adamantan-2-ylamide
[168] 5-(4-fluoro-phenyl)-1-(2,4-dichloro-phenyl)-4,5-dihydro-1H-pyrazole-3-carboxylic acid adamantan-2-ylamide
[169] 5-(4-methoxy-phenyl)-1-(2,4-dichloro-phenyl)-4,5-dihydro-1H-pyrazole-3-carboxylic acid adamantan-2-ylamide
[170] 5-(4-hydroxy-phenyl)-1-(2,4-dichloro-phenyl)-4,5-dihydro-1H-pyrazole-3-carboxylic acid adamantan-2-ylamide
[171] 5-(4-iodo-phenyl)-1-(2,4-dichloro-phenyl)-4,5-dihydro-1H-pyrazole-3-carboxylic acid adamantan-2-ylamide
[173] 1-(2,4-Dichloro-phenyl)-5-(4-hydroxy-phenyl)-4,5-dihydro-1 H-pyrazole-3-carboxylic acid azepan-1-ylamide hydrochloride
[174] 1-(2,4-Dichloro-phenyl)-5-(4-hydroxy-phenyl)-4,5-dihydro-1H-pyrazole-3 -carboxylic acid (hexahydro-cyclopenta[c]pyrrol-2-yl)-amide hydrochloride
[175] 1-(2,4-Dichloro-phenyl)-5-(4-hydroxy-phenyl)-4,5-dihydro-1H-pyrazole-3 -carboxylic acid (2,3-dihydro-indol-1-yl)-amide hydrochloride
[176] (R)-5-(4-Chloro-phenyl)-1-(2,4-dichloro-phenyl)-4,5-dihydro-1H-pyrazole-3-carboxylic acid azocan-1-ylamide hydrochloride
[177] (S)-5-(4-Chloro-phenyl)-1-(2,4-dichloro-phenyl)-4,5-dihydro-1H-pyrazole-3-carboxylic acid azocan-1-ylamide hydrochloride,
[178] N-((1R,2R)-2-(benzyloxy)cyclohexyl)-5-(4-chlorophenyl)-1-(2,4-dichlorophenyl)-4,5-dihydro-1H-pyrazole-3-carboxamide
[179] 1-(2,4-Dichloro-phenyl)-5-(4-fluoro-phenyl)-4,5-dihydro-1H-pyrazole-3-carboxylic acid (2,3-dihydro-indol-1-yl)-amide hydrochloride
[181] (R)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride
[182] (S)-5-(4-Chloro-phenyl)-1-(2,4-dichloro-phenyl)-4,5-dihydro-1H-pyrazole-3-carboxylic acid (2,3-dihydro-indol-1-yl)-amide hydrochloride
[183] (S)-5-(4-Chloro-phenyl)-1-(2,4-dichloro-phenyl)-4,5-dihydro-1H-pyrazole-3-carboxylic acid azepan-1-ylamide hydrochloride
[184] (S)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride
[185] (S)-5-(4-Chloro-phenyl)-1-(2,4-dichloro-phenyl)-4,5-dihydro-1H-pyrazole-3-carboxylic acid azepan-1-ylamide
[187] (R)-5-(4-Chloro-phenyl)-1-(2,4-dichloro-phenyl)-4,5-dihydro-1H-pyrazole-3-carboxylic acid azepan-1-ylamide hydrochloride
[188] (R)-5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide
[190] (R)- 5-(4-Chloro-phenyl)-1-(2,4-dichloro-phenyl)-4,5-dihydro-1H-pyrazole-3-carboxylic acid (2,3-dihydro-indol-1-yl)-amide
[191] 5-(4-Chloro-phenyl)-1-(2,4-dichloro-phenyl)-4,5-dihydro-1H-pyrazole-3-carboxylic acid cycloheptylmethyl-amide
[193] 5-(4-Chloro-phenyl)-1-(2,4-dichloro-phenyl)-4,5-dihydro-1H-pyrazole-3-carboxylic acid (hexahydro-cyclopenta[c]pyrrol-2-yl)-amide hydrochloride
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

14. Active substance combination according to claim 1, 2 or 3, **characterized in that** for the compound (A) of subgroup A6.1 according to formula I
X is O or S;
R¹ represents a radical selected from the group consisting of phenyl and thienyl (thiophenyl), which may optionally be substituted with 1, 2, 3, 4 or 5 substituent(s) independently selected from the group consisting of -OH, -O-CH₃, -O-C₂H₅, F, Cl, Brand I;
R² represents a phenyl radical, which may be substituted with 1, 2, 3, 4 or 5 substituent(s) independently selected from the group consisting of -OH, -O-CH₃,-O-C₂H₅, F, Cl, Br and I;
R³ represents a radical selected from the group consisting of 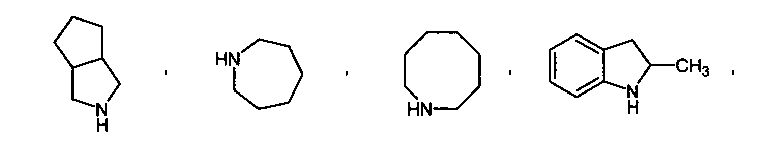 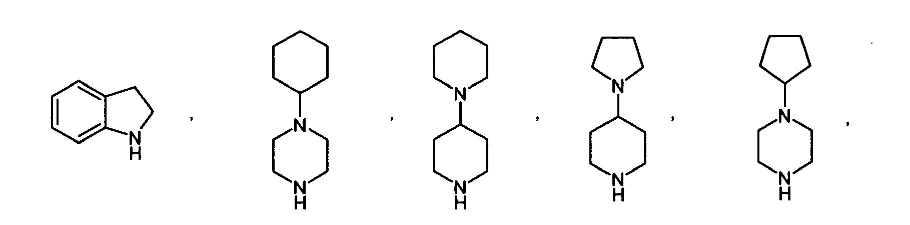 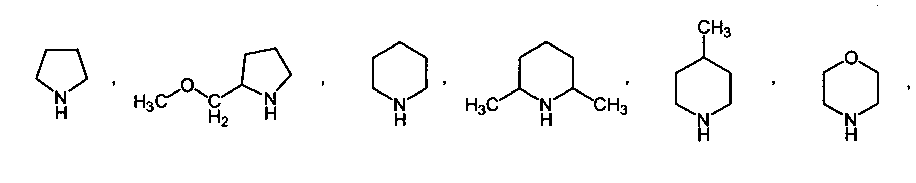 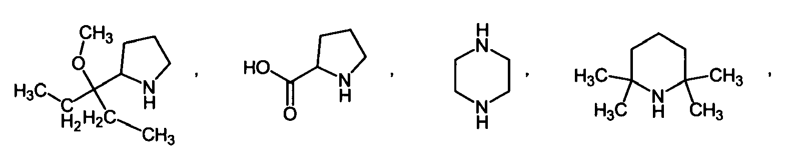 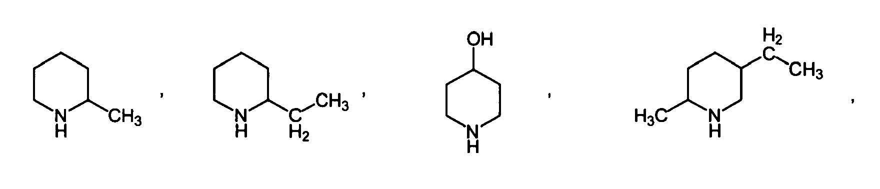 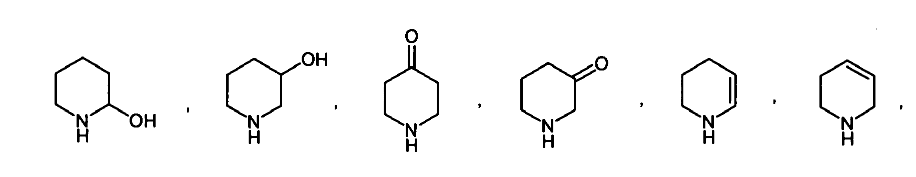 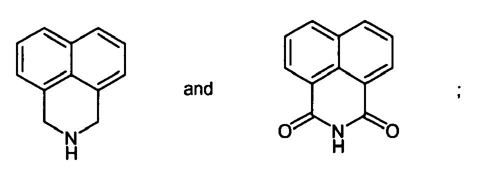 which is in each case bonded to the pyrazoline compound of general formula I in any position of the cyclic part of the aforementioned radicals including the NH-groups,
preferably said radicals are bonded to the pyrazoline compound of general formula I at the nitrogen atom of the cyclic part of the aforementioned radicals;
a -OR⁶-moiety or a -NR⁷R⁸ moiety;
R⁴ represents F; Cl; Br; I; -OH, -CN; -C(=O)-H; -C(=O)-OH; -C(=O)-OR¹⁷;
a radical selected from the group consisting of methyl, -CF₃, -CH₂F, -CF₂H, -C₂F₅, ethyl, -CH₂-CN, -CH₂-OH, n-propyl, isopropyl, -CH₂-CH₂-CN, -CH₂-CH₂-OH, n-butyl, -CH₂-CH₂-CH₂-CN, -CH₂-CH₂-CH₂-OH, isobutyl, sec-butyl, tert-butyl, n-pentyl, 2-pentyl, 3-pentyl, neo-pentyl, n-hexyl, 2-hexyl and 3-hexyl;
a benzyl radical or a -O-R¹¹ moiety;
R⁵ represents H; F; Cl; Br;-C(=O)-OH; -C(=O)-OR¹⁷; or a radical selected from the group consisting of methyl, ethyl, n-propyl, iso-propyl and n-butyl;
or R⁴ and R⁵ together with the bridging carbon atom form a radical selected from the group consisting of cyclopropyl, cyclobutyl, cyclopentyl, cyclohexyl, cycloheptyl, cyclooctyl, cyclopentenyl, cyclohexenyl, cycloheptenyl and cyclooctenyl;
R⁶ represents a hydrogen atom; a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;
R⁷ represents a hydrogen atom or a radical selected from the group consisting of methyl, ethyl, n-propyl, isopropyl, n-butyl, sec-butyl and tert-butyl;
R⁸ represents a radical selected from the group consisting of [1,2,3,4]-tetrahydronaphthyl, bicyclo[2.2.1]heptyl, cyclopropyl, cyclobutyl, cyclopentyl, cyclohexyl, cycloheptyl, cyclooctyl, cyclononyl, cyclodecyl, cycloundecyl and cyclododecyl, which may be bonded via a -(CH₂)-, -(CH₂)-(CH₂)- or -(CH₂)-(CH₂)-(CH₂)-group and/or subsituted with 1, 2, 3 or 4 substituents selected from the group consisting of methyl, ethyl, n-propyl, isopropyl, n-butyl, sec-butyl, tert-butyl, -OH, -O-CH₃ and -O-C₂H₅;
or a radical selected from the group consisting of 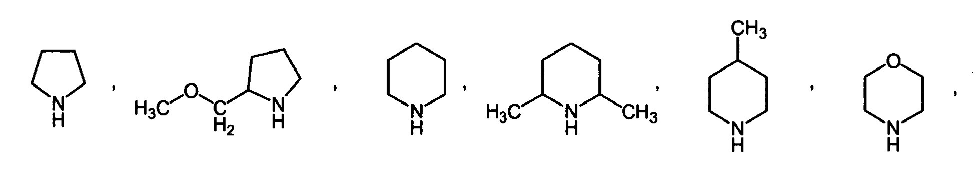 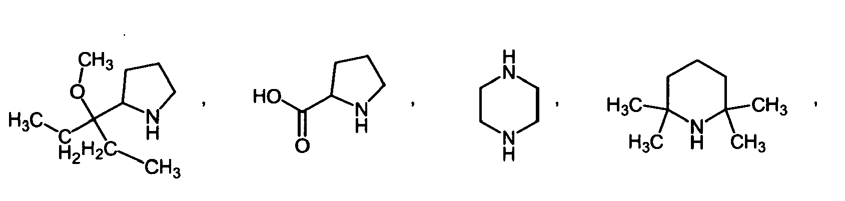 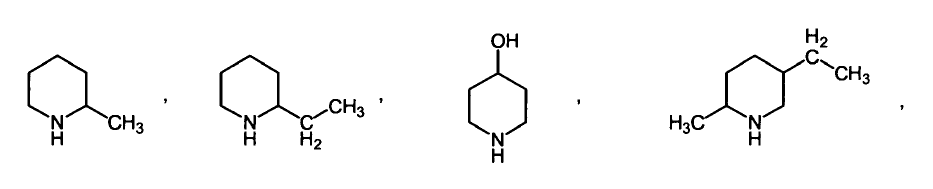 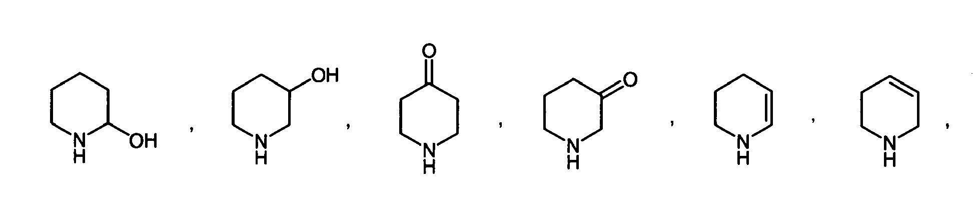 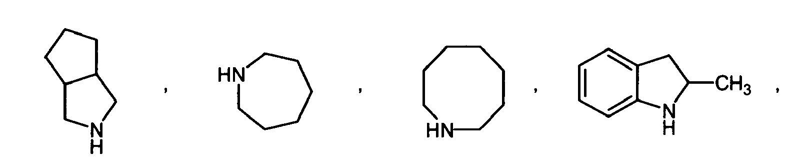 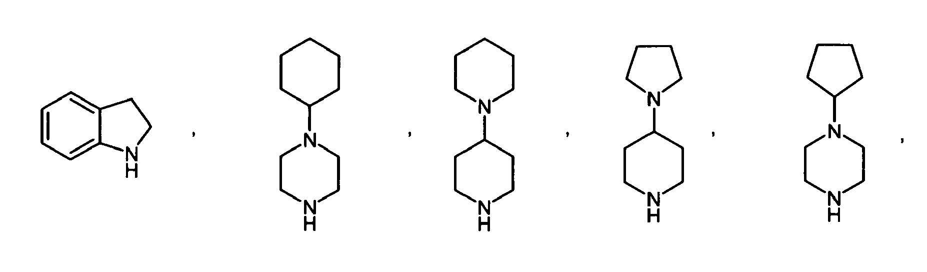 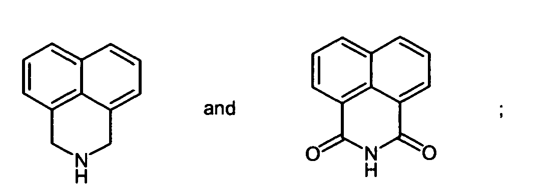 which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
and
R¹¹ and R¹⁷, independently of one another, each represent a radical selected from the group consisting of methyl, ethyl, n-propyl, isopropyl, n-butyl, isobutyl, sec-butyl, tert-butyl, n-pentyl, 2-pentyl, 3-pentyl and neo-pentyl;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

15. Active substance combination according to claim 14, **characterized in that** the compound (A) of subgroup A6.1 is a compound selected from:
[1] *cis-5-(*4-Chloro-phenyl)-1-(2,4-dichloro-phenyl)-4-methyl-4,5-dihydro-1H-pyrazole-3-carboxylic acid piperidin-1-ylamide hydro-chloride
[2] *trans-5-*(4-Chloro-phenyl)-1-(2,4-dichloro-phenyl)-4-methyl-4,5-dihydro-1H-pyrazole-3-carboxylic acid piperidin-1-ylamide hydro-chloride
[3] *cis-5-*(4-Chloro-phenyl)-1-(2,4-dichloro-phenyl)-4-methyl-4,5-dihydro-1H-pyrazole-3-carboxylic acid azepan-1-ylamide
[4] *trans-5-*(4-Chloro-phenyl)-1-(2,4-dichloro-phenyl)-4-methyl-4,5-dihydro-1H-pyrazole-3-carboxylic acid azepan-1-ylamide
[5] *cis*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone
[6] *trans*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone
[7] *cis-*5-(4-Chloro-phenyl)-1-(2,4-dichloro-phenyl)-4-methyl-4,5-dihydro-1H-pyrazole-3-carboxylic acid pyrrolidin-1-ylamide
[8] *trans-*5-(4-Chloro-phenyl)-1-(2,4-dichloro-phenyl)-4-methyl-4,5-dihydro-1H-pyrazole-3-carboxylic acid pyrrolidin-1-ylamide
[9] *cis-*5-(4-Chloro-phenyl)-1-(2,4-dichloro-phenyl)-4-methyl-4,5-dihydro-1H-pyrazole-3-carboxylic acid (4-methyl-piperidin-1-yl)-amide
[10] *trans*-5-(4-Chloro-phenyl)-1-(2,4-dichloro-phenyl)-4-methyl-4,5-dihydro-1H-pyrazole-3-carboxylic acid (4-methyl-piperidin-1-yl)-amide
[11] *cis-*[5*-*(4-Chloro-phenyl)-1-(2 ,4-dichloro-phenyl)-4-methyl-4,5-dihydro-1H-pyrazol-3-yl]-piperidin-1-yl-methanone
[12] *trans-*[5*-*(4-Chloro-phenyl)-1-(2,4-dichloro-phenyl)-4-methyl-4,5-dihydro-1H-pyrazol-3-yl]-piperidin-1-yl-methanone
[13] *cis-*5*-*(4-Chloro-phenyl)-1-(2,4-dichloro-phenyl)-4-methyl-4,5-dihydro-1H-pyrazole-3-carboxylic acid (hexahydro-cyclopenta[c]pyrrol-2-ylamide
[14] *trans*-5-(4-Chloro-phenyl)-1-(2,4-dichloro-phenyl)-4-methyl-4,5-dihydro-1H-pyrazole-3-carboxylic acid (hexahydro-cyclopenta-[c]py-rrol-2-yl)-amide
[15] *cis*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide
[16] *trans*-5-(4-Chloro-phenyl)-1-(2,4-dichloro-phenyl)-4-methyl-4,5-dihydro-1H-pyrazole-3-carboxylic acid (2,3-dihydro-indo)-1-yl)-amide
[17] *cis*-5-(4-Chloro-phenyl)-1-(2,4-dichloro-phenyl)-4-methyl-4,5-dihydro-1H-pyrazole-3-carboxylic acid cyclobutylamide
[18] 5-(4-Chloro-phenyl)-1-(2,4-dichloro-phenyl)-4-methyl-4,5-dihydro-1H-pyrazole-3-carboxylic acid cyclohexyl methyl-amide
[19] *cis*-5-(4-Chloro-phenyl)-1-(2,4-dichloro-phenyl)-4-methyl-4,5-dihydro-1H-pyrazole-3-carboxylic acid
[20] *trans*-5-(4-Chloro-phenyl)-1-(2,4-dichloro-phenyl)-4-methyl-4,5-dihydro-1H-pyrazole-3-carboxylic acid
[21] *cis*-5-(4-Chloro-phenyl)-1-(2,4-dichloro-phenyl)-4-ethyl-4,5-dihydro-1H-pyrazole-3-carboxylic acid
[22] *trans*-5-(4-Chloro-phenyl)-1-(2,4-dichloro-phenyl)-4-ethyl-4,5-dihydro-1H-pyrazole-3-carboxylic acid piperidin-1-ylamide
[23] *cis*-5-(4-Chloro-phenyl)-1-(2,4-dichloro-phenyl)-4-methyl-4,5-dihydro-1H-pyrazole-3-carboxylic acid methyl ester
[24] *trans*-5-(4-Chloro-phenyl)-1-(2,4-dichloro-phenyl)-4-methyl-4,5-dihydro-1H-pyrazole-3-carboxylic acid methyl ester
[25] *cis*-5-(4-Chloro-phenyl)-1-(2,4-dichloro-phenyl)-4-methyl-4,5-dihydro-1H-pyrazole-3-carboxylic acid ethyl ester
[26] *trans*-5-(4-Chloro-phenyl)-1-(2,4-dichloro-phenyl)-4-methyl-4,5-dihydro-1H-pyrazole-3-carboxylic acid ethyl ester
[27] 5-(4-Chloro-phenyl)-1-(2,4-dichloro-phenyl)-4-methyl-4,5-dihydro-1H-pyrazole-3-carboxylic acid cyclohe-xyl ester
[28] 5-(4-Chloro-phenyl)-1-(2,4-dichloro-phenyl)-4,4-dimethyl-4,5-dihydro-1-H-pyrazole-3-carboxylic acid
[29] 5-(4-Chloro-phenyl)-1-(2,4-dichloro-phenyl)-4,4-dimethyl-4,5-dihydro-1-H-pyrazole-3-carboxylic acid piperidin-1-ylamide
[30] 5-(4-Chloro-phenyl)-1-(2,4-dichloro-phenyl)-4-trifluoromethyl-4,5-dihydro-1H-pyrazole-3-carboxylic acid piperidin-1-ylamide
[31] 5-(4-Chloro-phenyl)-1-(2,4-dichloro-phenyl)-4-hydroxy-4,5-dihydro-1H-pyrazole-3-carboxylic acid
[32] 5-(4-Chloro-phenyl)-1-(2,4-dichloro-phenyl)-4-hydroxy-4,5-dihydro-1H-pyrazole-3-carboxylic acid piperidin-1-ylamide
[33] 5-(4-Chloro-phenyl)-1-(2,4-dichloro-phenyl)-4-(2-hydroxy-ethyl)-4,5-di-hydro-1H-pyrazole-3-carboxylic acid piperidin-1-ylamide
[34] 5-(4-Chloro-phenyl)-1-(2,4-dichloro-phenyl)-4-(2-hydroxy-ethyl)-4,5-di-hydro-1H-pyrazole-3-carboxylic acid
[35] 5-(4-Chloro-phenyl)-1-(2,4-dichloro-phenyl)-4-fluoromethyl-4,5-dihydro-1H-pyrazole-3-carboxylic acid piperidin-1-ylamide
[36] 5-(4-Chloro-phenyl)-1-(2,4-dichloro-phenyl)-4-fluoromethyl-4,5-dihydro-1H-pyrazole-3-carboxylic acid
[37] 5-(4-Chloro-phenyl)-1-(2,4-dichloro-phenyl)-4-formyl-4,5-dihydro-1H-pyrazole-3-carboxylic acid piperidin-1-ylamide
[38] 5-(4-Chloro-phenyl)-1-(2,4-dichloro-phenyl)-4-formyl-4,5-dihydro-1H-pyrazole-3-carboxylic acid
[39] 5-(4-chlorophenyl)-4-cyano-1-(2,4-dichlorophenyl)-N-(piperidin-1-yl)-4,5-dihydro-1 H-pyrazole-3-carboxamide
[40] 5-(4-Chloro-phenyl)-4-cyano-1-(2,4-dichloro-phenyl)-4,5-dihydro-1H-pyrazole-3-carboxylic acid
[41] 1-(2,4-Dichloro-phenyl)-5-(4-fluoro-phenyl)-4-methyl-4,5-dihydro-1H-pyrazole-3-carboxylic acid piperidin-1-ylamide
[42] 1-(2,4-Dichloro-phenyl)-4-ethyl-5-(4-fluoro-phenyl)-4,5-dihydro-1H-pyrazole-3-carboxylic acid piperidin-1-ylamide
[43] 1-(2,4-Dichloro-phenyl)-5-(4-fluoro-phenyl)-4,4-dimethyl-4,5-dihydro-1-H-pyrazole-3-carboxylic acid piperidin-1-ylamide
[44] 1-(2,4-Dichloro-phenyl)-5-(4-fluoro-phenyl)-4-trifluoromethyl-4,5-dihydro-1H-pyrazole-3-carboxylic acid piperidin-1-ylamide
[45] 1-(2,4-Dichloro-phenyl)-5-(4-fluoro-phenyl)-4-hydroxy-4,5-dihydro-1H-pyrazole-3-carboxylic acid piperidin-1-ylamide
[46] 1-(2,4-Dichloro-phenyl)-5-(4-fluoro-phenyl)-4-(2-hydroxy-ethyl)-4,5-dihydro-1H-pyrazole-3-carboxylic acid piperidin-1-ylamide
[47] 1-(2,4-Dichloro-phenyl)-4-fluoromethyl-5-(4-fluoro-phenyl)-4,5-dihydro-1H-pyrazole-3-carboxylic acid piperidin-1-ylamide
[48] 1-(2,4-Dichloro-phenyl)-5-(4-fluoro-phenyl)-4-formyl-4,5-dihydro-1H-pyrazole-3-carboxylic acid piperidin-1-ylamide
[49] 4-Cyano-1-(2,4-dichloro-phenyl)-5-(4-fluoro-phenyl)-4,5-dihydro-1H-pyrazole-3-carboxylic acid piperidin-1-ylamide
[50] 5-(4-Bromo-phenyl)-1-(2,4-dichloro-phenyl)-4-methyl-4,5-dihydro-1H-pyrazole-3-carboxylic acid piperidin-1-ylamide
[51] 5-(4-Bromo-phenyl)-1-(2,4-dichloro-phenyl)-4-ethyl-4,5-dihydro-1H-pyrazole-3-carboxylic acid piperidin-1-ylamide
[52] 5-(4-Bromo-phenyl)-1-(2,4-dichloro-phenyl)-4,4-dimethyl-4,5-dihydro-1H-pyrazole-3-carboxylic acid piperidin-1-ylamide
[53] 5-(4-Bromo-phenyl)-1-(2,4-dichloro-phenyl)-4-trifluoromethyl-4,5-dihydro-1H-pyrazole-3-carboxylic acid piperidin-1-ylamide
[54] 5-(4-Bromo-phenyl)-1-(2,4-dichloro-phenyl)-4-hydroxy-4,5-dihydro-1H-pyrazole-3-carboxylic acid piperidin-1-ylamide
[55] 5-(4-Bromo-phenyl)-1-(2,4-dichloro-phenyl)-4-(2-hydroxy-ethyl)-4,5-dihydro-1H-pyrazole-3-carboxylic acid piperidin-1-ylamide
[56] 5-(4-Bromo-phenyl)-1-(2,4-dichloro-phenyl)-4-fluoromethyl-4,5-dihydro-1H-pyrazole-3-carboxylic acid piperidin-1-ylamide
[57] 5-(4-Bromo-phenyl)-1-(2,4-dichloro-phenyl)-4-formyl-4,5-dihydro-1H-pyrazole-3-carboxylic acid piperidin-1-ylamide
[58] 5-(4-Bromo-phenyl)-4-cyano-1-(2,4-dichloro-phenyl)-4,5-dihydro-1H-pyrazole-3-carboxylic acid piperidin-1-ylamide
[59] *cis*-5-(5-Chloro-thiophen-2-yl)-1-(2,4-dichloro-phenyl)-4-methyl-4,5-dihydro-1H-pyrazole-3-carboxylic acid piperidin-1-ylamide
[60] *trans*-5-(5-Chloro-thiophen-2-yl)-1-(2,4-dichloro-phenyl)-4-methyl-4,5-dihydro-1H-pyrazole-3-carboxylic acid piperidin-1-ylamide
[61] *cis*-5-(5-Chloro-thiophen-2-yl)-1-(2,4-dichloro-phenyl)-4-ethyl-4,5-dihydro-1H-pyrazole-3-carboxylic acid piperidin-1-ylamide
[62] *trans*-5-(5-Chloro-thiophen-2-yl)-1-(2,4-dichloro-phenyl)-4-ethyl-4,5-dihydro-1H-pyrazole-3-carboxylic acid piperidin-1-ylamide
[63] 5-(5-Chloro-thiophen-2-yl)-1-(2,4-dichloro-phenyl)-4,4-dimethyl-4,5-dihydro-1 H-pyrazole-3-carboxylic acid piperidin-1-ylamide
[64] 5-(5-Chloro-thiophen-2-yl)-1-(2,4-dichloro-phenyl)-4-trifluoromethyl-4,5-dihydro-1H-pyrazole-3-carboxylic acid piperidin-1-ylamide
[65] 5-(5-Chloro-thiophen-2-yl)-1-(2,4-dichloro-phenyl)-4-hydroxy-4,5-dihydro-1H-pyrazole-3-carboxylic acid piperidin-1-ylamide
[66] 5-(5-Chloro-thiophen-2-yl)-1-(2,4-dichloro-phenyl)-4-(2-hydroxy-ethyl)-4,5-dihydro-1H-pyrazole-3-carboxylic acid piperidin-1-ylamide
[67] 5-(5-Chloro-thiophen-2-yl)-1-(2,4-dichloro-phenyl)-4-fluoromethyl-4,5-dihydro-1H-pyrazole-3-carboxylic acid piperidin-1-ylamide
[68] 5-(5-Chloro-thiophen-2-yl)-1-(2,4-dichloro-phenyl)-4-formyl-4,5-dihydro-1H-pyrazole-3-carboxylic acid piperidin-1-ylamide
[69] 5-(5-Chloro-thiophen-2-yl)-4-cyano-1-(2 ,4-dichloro-phenyl)-4,5-dihydro-1H-pyrazole-3-carboxylic acid piperidin-1-ylamide
[70] *cis*-5-(5-Bromo-thiophen-2-yl)-1-(2,4-dichloro-phenyl)-4-methyl-4,5-dihydro-1H-pyrazole-3-carboxylic acid piperidin-1-ylamide
[71] *trans*-5-(5-Bromo-thiophen-2-yl)-1-(2 ,4-dichloro-phenyl)-4-methyl-4,5-dihydro-1H-pyrazole-3-carboxylic acid piperidin-1-ylamide
[72] *cis*-5-(5-Bromo-thiophen-2-yl)1-(2,4-dichloro-phenyl)-4-ethyl-4,5-dihydro-1H-pyrazole-3-carboxylic acid piperidin-1-ylamide
[73] *trans-*5-(5-Bromo-thiophen-2-yl)-1-(2,4-dichloro-phenyl)-4-ethyl-4,5-dihydro-1H-pyrazole-3-carboxylic acid piperidin-1-ylamide
[74] 5-(5-Bromo-thiophen-2-yl)-1-(2,4-di-chloro-phenyl)-4,4-dimethyl-4,5-dihydro-1H-pyrazole-3-carboxylic acid piperidin-1-ylamide
[75] *cis*-5-(4-Bromo-thiophen-2-yl)-1-(2,4-dichloro-phenyl)-4-methyl-4,5-dihydro-1H-pyrazole-3-carboxylic acid piperidin-1-ylamide
[76] *trans*-5-(4-Bromo-thiophen-2-yl)-1-(2,4-dichloro-phenyl)-4-methyl-4,5-dihydro-1H-pyrazole-3-carboxylic acid piperidin-1-ylamide
[77] *cis-*5-(4-Bromo-thiophen-2-yl)-1-(2,4-dichloro-phenyl)-4-ethyl-4,5-dihydro-1H-pyrazole-3-carboxylic acid piperidin-1-ylamide
[78] *trans*-5-(4-Bromo-thiophen-2-yl)-1-(2,4-dichloro-phenyl)-4-ethyl-4,5-dihydro-1H-pyrazole-3-carboxylic acid piperidin-1-ylamide
[79] 5-(4-Bromo-thiophen-2-yl)-1-(2,4-dichloro-phenyl)-4,4-dimethyl-4,5-dihydro-1H-pyrazole-3-carboxylic acid piperidin-1-ylamide
[80] 5-(5-Bromo-thiophen-2-yl)-1-(2,4-dichloro-phenyl)-4-trifluoromethyl-4,5-dihydro-1H-pyrazole-3-carboxylic acid piperidin-1-ylamide
[81] 5-(5-Bromo-thiophen-2-yl)-1-(2,4-di-chloro-phenyl)-4-hydroxy-4,5-dihydro-1H-pyrazole-3-carboxylic acid piperidin-1-ylamide
[82] 5-(5-Bromo-thiophen-2-yl)-1-(2,4-di-chloro-phenyl)-4-(2-hydroxy-ethyl)-4,5-dihydro-1H-pyrazole-3-carboxylic acid piperidin-1-ylamide
[83] 5-(5-Bromo-thiophen-2-yl)-1-(2,4-di-chloro-phenyl)-4-fluoromethyl-4,5-dihydro-1 H-pyrazole-3-carboxylic acid piperidin-1-ylamide
[84] 5-(5-Bromo-thiophen-2-yl)-1-(2,4-di-chloro-phenyl)-4-formyl-4,5-dihydro-1H-pyrazole-3-carboxylic acid piperidin-1-ylamide
[85] 5-(5-Bromo-thiophen-2-yl)-4-cyano-1-(2,4-dichloro-phenyl)-4,5-dihydro-1 H-pyrazole-3-carboxylic acid piperidin-1-ylamide
[86] *cis*-5-(5-Chloro-thiophen-2-y1)-1-(2,4-dichloro-phenyl)-4-methyl-4,5-dihydro-1H-pyrazole-3-carboxylic acid
[87] *trans*-[5-(5-Chloro-thiophen-2-yl)-1-(2,4-dichloro-phenyl)-4-methyl-4,5-dihydro-1H-pyrazol-3-yl]-piperidin-1-yl-methanone
[88] *cis*-5-(5-Bromo-thiophen-2-yl)-1-(2,4-dichloro-phenyl)-4-methyl-4,5-dihydro-1H-pyrazole-3-carboxylic acid
[89] *trans*-5-(5-Bromo-thiophen-2-yl)-1-(2,4-dichloro-phenyl)-4-methyl-4,5-dihydro-1H-pyrazole-3-carboxylic acid
[90] *cis*-5-(4-Bromo-thiophen-2-yl)-1-(2,4-dichloro-phenyl)-4-methyl-4,5-dihydro-1H-pyrazole-3-carboxylic acid
[91] *trans*-5-(4-Bromo-thiophen-2-yl)-1-(2,4-dichloro-phenyl)-4-methyl-4,5-dihydro-1H-pyrazole-3-carboxylic acid,
92 (+)-cis-5-(4-chlorophenyl)-1-(2,4-dichlorophenyl)-4-methyl-N-(piperidin-1-yl)-4,5-dihydro-1H-pyrazole-3-carboxamide hydrochloride
93 (-)-cis-5-(4-chlorophenyl)-1-(2,4-dichlorophenyl)-4-methyl-N-(piperidin-1-yl)-4,5-dihydro-1H-pyrazole-3-carboxamide hydrochloride
94 *cis*-1-(2-chlorophenyl)-5-(4-chlorophenyl)-4-methyl-N-(piperidin-1-yl)-4,5-dihydro-1H-pyrazole-3-carboxamide
95 *trans*-1-(2-chlorophenyl)-5-(4-chlorophenyl)-4-methyl-N-(piperidin-1-yl)-4,5-dihydro-1H-pyrazole-3-carboxamide
96 *cis*-5-(4-Chlorophenyl)-1-(2,4-dichlorophenyl)-4-methyl-4,5-dihydro-1H-pyrazole-3-carboxylic acid azepan-1-ylamide hydrochloride
97 *trans*-5-(4-Chlorophenyl)-1-(2,4-dichloro-phenyl)-4-methyl-4,5-dihydro-1H-pyrazole-3-carboxylic acid azepan-1-ylamide hydrochloride
98 *cis*-N-(azepan-1-yl)-1-(2-chlorophenyl)-5-(4-chlorophenyl)-4-methyl-4,5-dihydro-1H-pyrazole-3-carboxamide
99 *trans*-N-(azepan-1-yl)-1-(2-chlorophenyl)-5-(4-chlorophenyl)-4-methyl-4,5-dihydro-1 H-pyrazole-3-carboxamide
100 *cis*-5-(4-chlorophenyl)-N-(4-cyclopentylpiperazin-1-yl)-1-(2,4-dichlorophenyl)-4-methyl-4,5-dihydro-1 H-pyrazole-3-carboxamide hydrochloride
101 1 *cis-*5-(4-chlorophenylyl-(2,4-dichlorophenyl)-4-methyl-N-morpholino-4,5-dihydro-1 H-pyrazole-3-carboxamide
102 cis-1-(2-chlorophenyl)-5-(4-chlorophenyl)-4-methyl-N-(4-methylpiperidin-1-yl)-4,5-dihydro-1 H-pyrazole-3-carboxamide
103 *cis-5-(* 4-chlorophenyl)-1-(2,4-dichlorophenyl)-N-(2,6-dimethylpiperidin-1-yl)-4-methyl-4,5-dihydro-1 H-pyrazole-3-carboxamide
104 *cis-*5-(4-Chlorophenyl)-1-(2,4-dichlorophenyl)-4-methyl-4,5-dihydro-1H-pyrazole-3-carboxylic acid (1,3-dioxo-1H,3H-benzo[de]isoquinolin-2-yl)-amide
105 *cis*-5-(4-Chlorophenyl)-1-(2,4-dichlorophenyl)-4-methyl-4,5-dihydro-1H-pyrazole-3-carboxylic acid (1H,3H-benzo[de]isoquinolin-2-yl)-amide
106 *cis*-5-(4-chlorophenyl)-1-(2,4-dichlorophenyl)-N-((R)-2-(methoxymethyl)pyrrolidin-1-yl)-4-methyl-4,5-dihydro-1H-pyrazole-3-carboxamide hydrochloride
107 *cis*-5-(4-chlorophenyl)-1 -(2,4-dichlorophenyl)-N-((S)-2-(methoxymethyl)pyrrolidin-1-yl)-4-methyl-4,5-dihydro-1H-pyrazole-3-carboxamide hydrochloride
108 *cis*-5-(4-Chlorophenyl)-1-(2,4-dichlorophenyl)-4-methyl-4,5-dihydro-1H-pyrazole-3-carboxylic acid (hexahydro-cyclopenta[c]pyrrol-2-yl)-amide hydrochloride
109 *cis*-1-(2-chlorophenyl)-5-(4-chlorophenyl)-N-(hexahydrocyclopenta[c]pyrrol-2(1H)-yl)-4-methyl-4,5-dihydro-1H-pyrazole-3-carboxamide
110 *trans*-1-(2-chlorophenyl)-5-(4-chlorophenyl)-N-(hexahydrocyclopenta[c]pyrrol-2(1H)-yl)-4-methyl-4,5-dihydro-1H-pyrazole-3-carboxamide
111 cis-1-(2-chlorophenyl)-5-(4-chlorophenyl)-N-(indolin-1-yl)-4-methyl-4,5-dihydro-1H-pyrazole-3-carboxamide
112 trans-1-(2-chlorophenyl)-5-( 4-chlorophenyl)-N-(indolin-1-yl)-4-methyl-4,5-dihydro-1H-pyrazole-3-carboxamide
113 cis-5-(4-chlorophenyl)-1-(2,4-dichlorophenyl)-4-methyl-N-(2-methylindolin-1-yl)-4,5-dihydro-1 H-pyrazole-3-carboxamide
114 *cis-*5*-(*4-chlorophenyl)-N-(cyclohexylmethyl)-1-(2,4-dichlorophenyl)-4-methyl-4,5-dihydro-1H-pyrazole-3-carboxamide
115 *cis*-5-(4-chlorophenyl)-N-(cycloheptylmethyl)-1-(2,4-dichlorophenyl)-4-methyl-4,5-dihydro-1 H-pyrazole-3-carboxamide
116 *cis-*5*-*(4-chlorophenyl)-N-cyclododecyl-1-(2,4-dichlorophenyl)-4-methyl-4,5-dihydro-1H-pyrazole-3-carboxamide
117 *cis-5-*(4-chlorophenyl)-1-(2,4-dichlorophenyl)-4-methyl-N-(1,2,3,4-tetrahydronaphthalen-1-yl)-4,5-dihydro-1H-pyrazole-3-carboxamide
118 *cis-*N-(4-tert-butylcyclohexyl)-5-(4-chlorophenyl)-1-(2,4-dichlorophenyl)-4-methyl-4,5-dihydro-1H-pyrazole-3-carboxamide
119 *cis*-5-(4-chlorophenyl)-N-cyclooctyl-1-(2,4-dichlorophenyl)-4-methyl-4,5-dihydro-1 H-pyrazole-3-carboxamide
120 *cis*-N-(azocan-1-yl)-5-(4-chlorophenyl)-1-(2,4-dichlorophenyl)-4-methyl-4,5-dihydro-1H-pyrazole-3-carboxamide
121 cis-N-(azocan-1-yl)-1-(2-chlorophenyl)-5-(4-chlorophenyl)-4-methyl-4,5-dihydro-1 H-pyrazole-3-carboxamide
122 *cis*-azocan-1-yl(cis-5-(4-chlorophenyl)-1-(2,4-dichlorophenyl)-4-methyl-4,5-dihydro-1H-pyrazol-3-yl)methanone
123 *cis*-5-(4-chlorophenyl)-N-cycloheptyl-1-(2,4-dichlorophenyl)-4-methyl-4,5-dihydro-1H-pyrazole-3-carboxamide
124 *cis*-1-(2-chlorophenyl)-5-(4-chlorophenyl)-N-cycloheptyl-4-methyl-4,5-dihydro-1H-pyrazole-3-carboxamide
125 *trans*-5-(4-chlorophenyl)-N-cycloheptyl-1-(2,4-dichlorophenyl)-4-methyl-4,5-dihydro-1H-pyrazole-3-carboxamide
126 *trans*-1-(2-chlorophenyl)-5-(4-chlorophenyl)-N-cycloheptyl-4-methyl-4,5-dihydro-1H-pyrazole-3-carboxamide
127 *cis*-5-(4-chlorophenyl)-N-(cyclohexylmethyl)-1-(2,4-dichlorophenyl)-4-methyl-4,5-dihydro-1H-pyrazole-3-carboxamide
128 (+)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide
129 (-)-cis-5-(4-chlorophenyl)-1-(2,4-dichlorophenyl)-N-((1S,2S)-2-hydroxycyclohexyl)-4-methyl-4,5-dihydro-1H-pyrazole-3-carboxamide
130 (-)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide
131 (+)-cis-5-(4-chlorophenyl)-1-(2,4-dichlorophenyl)-N-((1R,2R)-2-hydroxycyclohexyl)-4-methyl-4,5-dihydro-1H-pyrazole-3-carboxamide
132 (+)-*cis*-1-(2-chlorophenyl)-5-(4-chlorophenyl)-N-((1S,2S)-2-hydroxycyclohexyl)-4-methyl-4,5-dihydro-1H-pyrazole-3-carboxamide
133 (-)-*cis-*1-(2-chlorophenyl)-5-(4-chlorophenyl)-N-((1 S,2S)-2-hydroxycyclohexyl)-4-methyl-4,5-dihydro-1H-pyrazole-3-carboxamide
134 *cis-*5-(4-chlorophenyl)-1-(2,4-dichlorophenyl)-N-(4-hydroxypiperidin-1-yl)-4-methyl-4,5-dihydro-1H-pyrazole-3-carboxamide
135 *cis*-1-(2-chlorophenyl)-5-(4-chlorophenyl)-N-(4-hydroxypiperidin-1-yl)-4-methyl-4,5-dihydro-1 H-pyrazole-3-carboxamide
136 *cis-*5-(4-chlorophenyl)-1-(2,4-dichlorophenyl)-N-(3-hydroxypiperidin-1-yl)-4-methyl-4,5-dihydro-1 H-pyrazole-3-carboxamide
137 *cis*-1-(2-chlorophenyl)-5-(4-chlorophenyl)-N-(3-hydroxypiperidin-1-yl)-4-methyl-4,5-dihydro-1 H-pyrazole-3-carboxamide
138 *cis-*5-(4-chlorophenyl)-1-(2,4-dichlorophenyl)-N-(2-hydroxypiperidin-1-yl)-4-methyl-4,5-dihydro-1 H-pyrazole-3-carboxamide
139 *cis*-1-(2-chlorophenyl)-5-(4-chlorophenyl)-N-(2-hydroxypiperidin-1-yl)-4-methyl-4,5-dihydro-1 H-pyrazole-3-carboxamide
140 *cis*-5-(4-chlorophenyl)-1-(2,4-dichlorophenyl)-4-methyl-N-(4-oxopiperidin-1-yl)-4,5-dihydro-1H-pyrazole-3-carboxamide
141 *cis*-1-(2-chlorophenyl)-5-(4-chlorophenyl)-4-methyl-N-(4-oxopiperidin-1-yl)-4,5-dihydro-1H-pyrazole-3-carboxamide
142 *cis*-5-(4-chlorophenyl)-1-(2,4-dichlorophenyl)-4-methyl-N-(3-oxopiperidin-1-yl)-4,5-dihydro-1H-pyrazole-3-carboxamide
143 *cis*-1-(2-chlorophenyl)-5-(4-chlorophenyl)-4-methyl-N-(3-oxopiperidin-1-yl)-4,5-dihydro-1H-pyrazole-3-carboxamide
144 *cis*-5-(4-chlorophenyl)-1-(2,4-dichlorophenyl)-N-(3,4-dihydropyridin-1(2H)-yl)-4-methyl-4,5-dihydro-1H-pyrazole-3-carboxamide
145 *cis-*1-(2-chlorophenyl)-5-(4-chlorophenyl)-N-(3,4-dihydropyridin-1(2H)-yl)-4-methyl-4,5-dihydro-1H-pyrazole-3-carboxamide
146 *cis*-5-(4-chlorophenyl)-1-(2,4-dichlorophenyl)-N-(5,6-dihydropyridin-1(2H)-yl)-4-methyl-4,5-dihydro-1H-pyrazole-3-carboxamide
147 *cis*-1-(2-chlorophenyl)-5-(4-chlorophenyl)-N-(5,6-dihydropyridin-1(2H)-yl)-4-methyl-4,5-dihydro-1H-pyrazole-3-carboxamide
148 *cis*-1-(2-chlorophenyl)-5-(4-chlorophenyl)-4-methyl-4,5-dihydro-1H-pyrazole-3-carboxylic acid
149 *trans*-1-(2-chlorophenyl)-5-(4-chlorophenyl)-4-methyl-4,5-dihydro-1H-pyrazole-3-carboxylic acid
150 *cis*-5-(4-Chlorophenyl)-1-(2,4-dichlorophenyl)-4-methyl-4,5-dihydro-1H-py-razole-3-carboxylic acid cyclohexyl ester
151 N-(azepan-1-yl)-5-(4-chlorophenyl)-1-(2,4-dichlorophenyl)-4,4-dimethyl-4,5-dihydro-1H-pyrazole-3-carboxamide
152 1-(2-chlorophenyl)-5-(4-chlorophenyl)-4,4-dimethyl-N-(piperidin-1-yl)-4,5-dihydro-1H-pyrazole-3-carboxamide
153 N-(azepan-1-yl)-1-(2-chlorophenyl)-5-(4-chlorophenyl)-4,4-dimethyl-4,5-dihydro-1H-pyrazole-3-carboxamide
154 *cis*-5-(4-Chlorophenyl)-1-(2,4-dichlorophenyl)-4-trifluoromethyl-4,5-dihydro-1H-pyrazole-3-carboxylic acid piperidin-1-ylamide
155 *trans-*5-(4-Chloro-phenyl)-1-(2,4-dichlorophenyl)-4-trifluoromethyl-4,5-dihydro-1 H-pyrazole-3-carboxylic acid piperidin-1-ylamide
156 *cis*-N-(azepan-1-yl)-5-(4-chlorophenyl)-1-(2,4-dichlorophenyl)-4-(trifluoromethyl)-4,5-dihydro-1H-pyrazole-3-carboxamide
157 *trans*-N-(azepan-1-yl)-5-(4-chlorophenyl)-1-(2,4-dichlorophenyl)-4-(trifluoromethyl)-4,5-dihydro-1H-pyrazole-3-carboxamide
158 *cis*-1-(2-chlorophenyl)-5-(4-chlorophenyl)-N-(piperidin-1-yl)-4-(trifluoromethyl)-4,5-dihydro-1H-pyrazole-3-carboxamide
159 *trans* 1-(2-chlorophenyl)-5-(4-chlorophenyl)-N-(piperidin-1-yl)-4-(trifluoromethyl)-4,5-dihydro-1H-pyrazole-3-carboxamide
160 5-(4-chlorophenyl)-1-(2,4-dichlorophenyl)-3-(piperidin-1-ylcarbamoyl)-4,5-dihydro-1H-pyrazole-4-carboxylic acid
161 5-(4-chlorophenyl)-1-(2,4-dichlorophenyl)-4,4-difluoro-N-(piperidin-1-yl)-4,5-dihydro-1H-pyrazole-3-carboxamide
162 *cis*-5-(4-chlorophenyl)-4-cyano-1-(2,4-dichlorophenyl)-N-(piperidin-1-yl)-4,5-dihydro-1H-pyrazole-3-carboxamide
163 *cis*-N-(azepan-1-yl)-5-(4-chlorophenyl)-4-cyano-1-(2,4-dichlorophenyl)-4,5-dihydro-1 H-pyrazole-3-carboxamide
164 *cis*-1-(2-chlorophenyl)-5-(4-chlorophenyl)-4-cyano-N-(piperidin-1-yl)-4,5-dihydro-1 H-pyrazole-3-carboxamide
165 *cis*-N-(azepan-1-yl)-1-(2-chlorophenyl)-5-(4-chlorophenyl)-4-cyano-4,5-dihydro-1H-pyrazole-3-carboxamide
166 *trans*-5-(4-chlorophenyl)-4-cyano-1-(2,4-dichlorophenyl)-N-(piperidin-1-yl)-4,5-dihydro-1 H-pyrazole-3-carboxamide
167 *trans*-N-(azepan-1-yl)-5-(4-chlorophenyl)-4-cyano-1-(2,4-dichlorophenyl)-4,5-dihydro-1 H-pyrazole-3-carboxamide
168 *trans*-1-(2-chlorophenyl)-5-(4-chlorophenyl)-4-cyano-N-(piperidin-1-yl)-4,5-dihydro-1 H-pyrazole-3-carboxamide
169 *cis*-1-(2-chlorophenyl)-5-(4-chlorophenyl)-4-ethyl-4,5-dihydro-1H-pyrazole-3-carboxylic acid
170 *trans-*1-(2-chlorophenyl)-5-(4-chlorophenyl)-4-ethyl-4,5-dihydro-1H-pyrazole-3-carboxylic acid
171 *cis*-5-(4-chlorophenyl)-1-(2,4-dichlorophenyl)-4-ethyl-N-(piperidin-1-yl)-4,5-dihydro-1H-pyrazole-3-carboxamide
172 trans-5-(4-chlorophenyl)-1-(2,4-dichlorophenyl)-4-ethyl-N-(piperidin-1-yl)-4,5-dihydro-1H-pyrazole-3-carboxamide
173 cis-1-(2-chlorophenyl)-5-(4-chlorophenyl)-4-ethyl-N-(piperidin-1-yl)-4,5-dihydro-1H-pyrazole-3-carboxamide
174 *trans*-1-(2-chlorophenyl)-5-(4-chlorophenyl)-4-ethyl-N-(piperidin-1-yl)-4,5-dihydro-1 H-pyrazole-3-carboxamide
175 *cis*-N-(azepan-1-yl)-5-(4-chlorophenyl)-1-(2,4-dichlorophenyl)-4-ethyl-4,5-dihydro-1H-pyrazole-3-carboxamide
176 *trans*-N-(azepan-1-yl)-5-(4-chlorophenyl)-1-(2,4-dichlorophenyl)-4-ethyl-4,5-dihydro-1H-pyrazole-3-carboxamide
177 *cis*-N-(azepan-1-yl)-1-(2-chlorophenyl)-5-(4-chlorophenyl)-4-ethyl-4,5-dihydro-1H-pyrazole-3-carboxamide
178 *trans*-N-(azepan-1-yl)-1-(2-chlorophenyl)-5-(4-chlorophenyl)-4-ethyl-4,5-dihydro-1 H-pyrazole-3-carboxamide
179 *cis-*5*-*(4-chlorophenyl)-N-cycloheptyl-1-(2,4-dichlorophenyl)-4-ethyl-4,5-dihydro-1 H-pyrazole-3-carboxamide
180 *cis*-1-(2-chlorophenyl)-5-(4-chlorophenyl)-N-cycloheptyl-4-ethyl-4,5-dihydro-1H-pyrazole-3-carboxamide
181 *cis*-5-(4-chlorophenyl)-1-(2,4-dichlorophenyl)-4-ethyl-N-(hexahydrocyclopenta[c]pyrrol-2(1H)-yl)-4,5-dihydro-1H-pyrazole-3-carboxamide
182 *cis*-1-(2-chlorophenyl)-5-(4-chlorophenyl)-4-ethyl-N-(hexahydrocyclopenta[c]pyrrol-2(1H)-yl)-4,5-dihydro-1H-pyrazole-3-carboxamide
183 *cis-*5*-*(4-chlorophenyl)-1-(2,4-dichlorophenyl)-4-ethyl-N-(indolin-1-yl)-4,5-dihydro-1 H-pyrazole-3-carboxamide
184 *cis*-1-(2-chlorophenyl)-5-(4-chlorophenyl)-4-ethyl-N-(indolin-1-yl)-4,5-dihydro-1H-pyrazole-3-carboxamide
185 *cis*-5-(4-bromophenyl)-1-(2,4-dichlorophenyl)-4-ethyl-4,5-dihydro-1H-pyrazole-3-carboxylic acid
186 *trans*-5-(4-bromophenyl)-1-(2,4-dichlorophenyl)-4-ethyl-4,5-dihydro-1H-pyrazole-3-carboxylic acid
187 *cis*-5-(4-bromophenyl)-1-(2,4-dichlorophenyl)-4-ethyl-N-(piperidin-1-yl)-4,5-dihydro-1H-pyrazole-3-carboxamide
188 *trans-*5-(4-bromophenyl)-1-(2,4-dichlorophenyl)-4-ethyl-N-(piperidin-1-yl)-4,5-dihydro-1H-pyrazole-3-carboxamide
189 *cis*-5-(4-bromophenyl)-1-(2-chlorophenyl)-4-ethyl-N-(piperidin-1-yl)-4,5-dihydro-1 H-pyrazole-3-carboxamide
190 *trans-*5-(4-bromophenyl)-1-(2-chlorophenyl)-4-ethyl-N-(piperidin-1-yl)-4,5-dihydro-1H-pyrazole-3-carboxamide
191 *cis-*N-(azepan-1-yl)-5-(4-bromophenyl)-1-(2,4-dichlorophenyl)-4-ethyl-4,5-dihydro-1 H-pyrazole-3-carboxamide
192 *cis*-N-(azepan-1-yl)-5-(4-bromophenyl)-1-(2-chlorophenyl)-4-ethyl-4,5-dihydro-1 H-pyrazole-3-carboxamide
193 *cis-*5-(4-bromophenyl)-N-cycloheptyl-1-(2,4-dichlorophenyl)-4-ethyl-4,5-dihydro-1H-pyrazole-3-carboxamide
194 *cis*-5-(4-bromophenyl)-1-(2-chlorophenyl)-N-cycloheptyl-4-ethyl-4,5-dihydro-1H-pyrazole-3-carboxamide
195 *cis*-5-(4-bromophenyl)-1-(2,4-dichlorophenyl)-4-ethyl-N-(hexahydrocyclopenta[c]pyrrol-2(1H)-yl)-4,5-dihydro-1H-pyrazole-3-carboxamide
196 *cis*-5-(4-bromophenyl)-1-(2-chlorophenyl)-4-ethyl-N-(hexahydrocyclopenta[c]pyrrol-2(1H)-yl)-4,5-dihydro-1H-pyrazole-3-carboxamide
197 *cis-*5*-*(4-bromophenyl)-1-(2,4-dichlorophenyl)-4-ethyl-N-(indolin-1-yl)-4,5-dihydro-1H-pyrazole-3-carboxamide
198 *cis*-5-(4-bromophenyl)-1-(2-chlorophenyl)-4-ethyl-N-(indolin-1-yl)-4,5-dihydro-1H-pyrazole-3-ca rboxa m ide
199 *cis*-5-(4-bromophenyl)-1-(2,4-dichlorophenyl)-4-methyl-4,5-dihydro-1H-pyrazole-3-carboxylic acid
200 *trans*-5-(4-bromophenyl)-1-(2,4-dichlorophenyl)-4-methyl-4,5-dihydro-1H-pyrazole-3-carboxylic acid
201 *cis*-5-(4-bromophenyl)-1-(2-chlorophenyl)-4-methyl-4,5-dihydro-1H-pyrazole-3-carboxylic acid
202 *trans*-5-(4-bromophenyl)-1-(2-chlorophenyl)-4-methyl-4,5-dihydro-1H-pyrazole-3-carboxylic acid
203 *cis*-5-(4-bromophenyl)-1-(2,4-dichlorophenyl)-4-methyl-N-(piperidin-1-yl)-4,5-dihydro-1H-pyrazole-3-carboxamide
204 trans-5-(4-bromophenyl)-1-(2,4-dichlorophenyl)-4-methyl-N-(piperidin-1-yl)-4,5-dihydro-1 H-pyrazole-3-carboxamide
205 cis 5-(4-bromophenyl)-1-(2-chlorophenyl)-4-methyl-N-(piperidin-1-yl)-4,5-dihydro-1 H-pyrazole-3-carboxamide
206 *trans*-5-(4-bromophenyl)-1-(2-chlorophenyl)-4-methyl-N-(piperidin-1-yl)-4,5-dihydro-1 H-pyrazole-3-carboxamide
207 *cis*-N-(azepan-1-yl)-5-(4-bromophenyl)-1-(2,4-dichlorophenyl)-4-methyl-4,5-dihydro-1H-pyrazole-3-carboxamide hydrochloride
208 *trans*-N-(azepan-1-yl)-5-(4-bromophenyl)-1-(2,4-dichlorophenyl)-4-methyl-4,5-dihydro-1H-pyrazole-3-carboxamide hydrochloride
209 *cis-*N-(azepan-1-yl)-5-(4-bromophenyl)-1-(2-chlorophenyl)-4-methyl-4,5-dihydro-1 H-pyrazole-3-carboxamide
210 *cis-*N-(azepan-1-yl)-5-(4-bromophenyl)-1-(2-chlorophenyl)-4-methyl-4,5-dihydro-1 H-pyrazole-3-carboxamide
211 *cis*-5-(4-bromophenyl)-N-cycloheptyl-1-(2,4-dichlorophenyl)-4-methyl-4,5-dihydro-1 H-pyrazole-3-carboxamide
212 *cis-5-*(4-bromophenyl)-1-(2-chlorophenyl)-N-cycloheptyl-4-methyl-4,5-dihydro-1H-pyrazole-3-carboxamide
213 *cis*-5-(4-bromophenyl)-1-(2,4-dichlorophenyl)-N-(hexahydrocyclopenta[c]pyrrol-2(1H)-yl)-4-methyl-4,5-dihydro-1H-pyrazole-3-carboxamide hydrochloride
214 *cis*-5-(4-bromophenyl)-1-(2-chlorophenyl)-N-(hexahydrocyclopenta[c]pyrrol-2(1H)-yl)-4-methyl-4,5-dihydro-1H-pyrazole-3-carboxamide
215 *cis*-5-(4-bromophenyl)-1-(2,4-dichlorophenyl)-N-(indolin-1-yl)-4-methyl-4,5-dihydro-1H-pyrazole-3-carboxamide
216 *cis*-5-(4-bromophenyl)-1-(2-chlorophenyl)-N-(indolin-1-yl)-4-methyl-4,5-dihydro-1H-pyrazole-3-carboxamide
217 (+)-cis-5-(4-bromophenyl)-1-(2,4-dichlorophenyl)-N-((1S,2S)-2-hydroxycyclohexyl)-4-methyl-4,5-dihydro-1H-pyrazole-3-carboxamide
218 (-)-cis-5-(4-bromophenyl)-1-(2,4-dichlorophenyl)-N-((1S,2S)-2-hydroxycyclohexyl)-4-methyl-4,5-dihydro-1H-pyrazole-3-carboxamide
219 (+)-cis-5-(4-bromophenyl)-1-(2-chlorophenyl)-N-((1S,2S)-2-hydroxycyclohexyl)-4-methyt-4,5-dihydro-1H-pyrazole-3-carboxamide
220 (-)-cis-5-(4-bromophenyl)-1-(2-chlorophenyl)-N-((1S,2S)-2-hydroxycyclohexyl)-4-methyl-4,5-dihydro-1H-pyrazole-3-carboxamide
221 *cis*-1-(2,4-dichlorophenyl)-5-(4-fluorophenyl)-4-methyl-4,5-dihydro-1H-pyrazole-3-carboxylic acid
222 *trans*-1-(2,4-dichlorophenyl)-5-(4-fluorophenyl)-4-methyl-4,5-dihydro-1H-pyrazole-3-carboxylic acid
223 *cis*-1-(2,4-dichlorophenyl)-5-(4-fluorophenyl)-4-methyl-N-(piperidin-1-yl)-4,5-dihydro-1H-pyrazole-3-carboxamide
224 *trans-*1-(2,4-dichlorophenyl)-5-(4-fluorophenyl)-4-methyl-N-(piperidin-1-yl)-4,5-dihydro-1H-pyrazole-3-carboxamide
225 *cis*-1-(2-chlorophenyl)-5-(4-fluorophenyl)-4-methyl-N-(pipeddin-1-yl)-4,5-dihydro-1 H-pyrazole-3-carboxamide
226 *trans*-1-(2-chlorophenyl)-5-(4-fluorophenyl)-4-methyl-N-(piperidin-1-yl)-4,5-dihydro-1H-pyrazole-3-carboxamide
227 *ci*s*-*N-(azepan-1-yl)-1-(2,4-dichlorophenyl)-5-(4-fluorophenyl)-4-methyl-4,5-dihydro-1H-pyrazole-3-carboxamide hydrochloride
228 *trans*-N-(azepan-1-yl)-1-(2,4-dichlorophenyl)-5-(4-fluorophenyl)-4-methyl-4,5-dihydro-1H-pyrazole-3-carboxamide hydrochloride
229 *cis*-N-(azepan-1-yl)-1-(2-chlorophenyl)-5-(4-fluorophenyl)-4-methyl-4,5-dihydro-1 H-pyrazole-3-carboxamide
230 *trans*-N-(azepan-1-yl)-1-(2-chlorophenyl)-5-(4-fluorophenyl)-4-methyl-4,5-dihydro-1 H-pyrazole-3-carboxamide
231 *cis*-N-cycloheptyl-1-(2,4-dichlorophenyl)-5-(4-fluorophenyl)-4-methyl-4,5-dihydro-1H-pyrazole-3-carboxamide
232 *cis*-1-(2-chlorophenyl)-N-cycloheptyl-5-(4-fluorophenyl)-4-methyl-4,5-dihydro-1H-pyrazole-3-carboxamide
233 *cis-*1-(2,4-dichlorophenyl)-5-(4-fluorophenyl)-N-(hexahydrocyclopenta[c]pyrrol-2(1H)-yl)-4-methyl-4,5-dihydro-1H-pyrazole-3-carboxamide hydrochloride
234 *cis*-1-(2-chlorophenyl)-5-(4-fluorophenyl)-N-(hexahydrocyclopenta[c]pyrrol-2(1H)-yl)-4-methyl-4,5-dihydro-1H-pyrazole-3-carboxamide
235 *cis*-1-(2,4-dichlorophenyl)-5-(4-fluorophenyl)-N-(indolin-1-yl)-4-methyl-4,5-dihydro-1H-pyrazole-3-carboxamide
236 *cis*-1-(2-chlorophenyl)-5-(4-fluorophenyl)-N-(indolin-1-yl)-4-methyl-4,5-dihydro-1 H-pyrazole-3-carboxamide
237 (+)cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide
238 (-)-cis-1-(2,4-dichlorophenyl)-5-(4-fluorophenyl)-N-((1S,2S)-2-hydroxycyclohexyl)-4-methyl-4,5-dihydro-1H-pyrazole-3-carboxamide
239 (+)-cis-1-(2-chlorophenyl)-5-(4-fluorophenyl)-N-((1S,2S)-2-hydroxycyclohexyl)-4-methyl-4,5-dihydro-1H-pyrazole-3-carboxamide
240 (-)-cis-1-(2-chlorophenyl)-5-(4-fluorophenyl)-N-((1S,2S)-2-hydroxycyclohexyl)-4-methyl-4,5-dihydro-1 H-pyrazole-3-carboxamide
241 *cis*-1-(2,4-dichlorophenyl)5-(4-iodophenyl)4-methyl-4,5-dihydro-1H-pyrazole-3-carboxylic acid
242 *trans*-1-(2,4-dichlorophenyl)-5-(4-iodophenyl)-4-methyl-4,5-dihydro-1H-pyrazole-3-carboxylic acid
243 *cis*-1-(2,4-dichlorophenyl)-5-(4-iodophenyl)-4-methyl-N-(piperidin-1-yl)-4,5-dihydro-1H-pyrazole-3-carboxamide
244 *trans*-1-(2,4-dichlorophenyl)-5-(4-iodophenyl)-4-methyl-N-(piperidin-1-yl)-4,5-dihydro-1H-pyrazole-3-carboxamide
245 *cis*-1-(2-chlorophenyl)-5-(4-iodophenyl)-4-methyl-N-(piperidin-1-yl)-4,5-dihydro-1 H-pyrazole-3-carboxamide
246 *trans*-1-(2-chlorophenyl)-5-(4-iodophenyl)-4-methyl-N-(piperidin-1-yl)-4,5-dihydro-1 H-pyrazole-3-carboxamide
247 *cis-*N-(azepan-1-yl)-1-(2,4-dichlorophenyl)-5-(4-iodophenyl)-4-methyl-4,5-dihydro-1 H-pyrazole-3-carboxamide
248 *trans*-N-(azepan-1-yl)-1-(2,4-dichlorophenyl)-5-(4-iodophenyl)-4-methyl-4,5-dihydro-1H-pyrazole-3-carboxamide
249 *cis*-N-(azepan-1-yl)-1-(2-chlorophenyl)-5-(4-iodophenyl)-4-methyl-4,5-dihydro-1H-pyrazole-3-carboxamide
250 *trans*-N-(azepan-1-yl)-1-(2-chlorophenyl)-5-(4-iodophenyl)-4-methyl-4,5-dihydro-1 H-pyrazole-3-carboxamide
251 *cis*-N-cycloheptyl-1-(2,4-dichlorophenyl)-5-(4-iodophenyl)-4-methyl-4,5-dihydro-1 H-pyrazole-3-carboxamide
252 *cis*-1-(2-chlorophenyl)-N-cycloheptyl-5-(4-iodophenyl)-4-methyl-4,5-dihydro-1H-pyrazole-3-carboxamide
253 *cis*-1-(2,4-dichlorophenyl)-N-(hexahydrocyclopenta[c]pyrrol-2(1H)-yl)-5-(4-iodophenyl)-4-methyl-4,5-dihydro-1H-pyrazole-3-carboxamide
254 *cis*-1-(2-chlorophenyl)-N-(hexahydrocyclopenta[c]pyrrol-2(1H)-yl)-5-(4-iodophenyl)-4-methyl-4,5-dihydro-1H-pyrazole-3-carboxamide
255 *cis*-1-(2,4-dichlorophenyl)-N-(indolin-1-yl)-5-(4-iodophenyl)-4-methyl-4,5-dihydro-1 H-pyrazole-3-carboxamide
256 *cis*-1-(2-chlorophenyl)-N-(indolin-1-yl)-5-(4-iodophenyl)-4-methyl-4,5-dihydro-1H-pyrazole-3-carboxamide
257 (+)-cis-1-(2,4-dichlorophenyl)-N-((1S,2S)-2-hydroxycyclohexyl)-5-(4-iodophenyl)-4-methyl-4,5-dihydro-1H-pyrazole-3-carboxamide
258 (-)-cis-1-(2,4-dichlorophenyl)-N-((1S,2S)-2-hydroxycyclohexyl)-5-(4-iodophenyl)-4-methyl-4,5-dihydro-1H-pyrazole-3-carboxamide
259 (+)-cis-1-(2-ch)orophenyl)-N-((1S,2S)-2-hydroxycyclohexyl)-5-(4-iodophenyl)-4-methyl-4,5-dihydro-1H-pyrazole-3-carboxamide
260 (-)-cis-1-(2-chlorophenyl)-N-((1S,2S)-2-hydroxycyclohexyl)-5-(4-iodophenyl)-4-methyl-4,5-dihydro-1H-pyrazole-3-carboxamide
261 *cis*-1-(2,4-dichlorophenyl)-5-(4-methoxyphenyl)4,5-methyl-4,5-dihydro-1H-pyrazole-3-carboxylic acid
262 *trans-*1-(2,4-dichlorophenyl)-5-(4-methoxyphenyl)-4-methyl-4,5-dihydro-1H-pyrazole-3-carboxylic acid
263 *cis*-1-(2-chlorophenyl)-5-(4-methoxyphenyl)-4-methyl-4,5-dihydro-1H-pyrazole-3-carboxylic acid
264 *trans-*1-(2-chlorophenyl)-5-(4-methoxyphenyl)-4-methyl-4,5-dihydro-1H-pyrazole-3-carboxylic acid
265 *cis*-1-(2,4-dichlorophenyl)-5-(4-methoxyphenyl)-4-methyl-N-(piperidin-1-yl)-4,5-dihydro-1H-pyrazole-3-carboxamide hydrochloride
266 (+)-cis-1-(2,4-dichlorophenyl)-5-(4-methoxyphenyl)-4-methyl-N-(piperidin-1-yl)-4,5-dihydro-1H-pyrazole-3-carboxamide hydrochloride
267 (-)-cis-1-(2,4-dichlorophenyl)-5-(4-methoxyphenyl)-4-methyl-N-(piperidin-1-yl)-4,5-dihydro-1H-pyrazole-3-carboxamide hydrochloride
268 *trans*-1-(2,4-dichlorophenyl)-5-(4-methoxyphenyl)-4-methyl-N-(piperidin-1-yl)-4,5-dihydro-1H-pyrazole-3-carboxamide
269 *cis*-1-(2-chlorophenyl)-5-(4-methoxyphenyl)-4-methyl-N-(piperidin-1-yl)-4,5-dihydro-1H-pyrazole-3-carboxamide
270 *trans*-1-(2-chlorophenyl)-5-(4-methoxyphenyl)-4-methyl-N-(piperidin-1-yl)-4,5-dihydro-1H-pyrazole-3-carboxamide
271 *cis*-N-(azepan-1-yl)-1-(2,4-dichlorophenyl)-5-(4-methoxyphenyl)-4-methyl-4,5-dihydro-1H-pyrazole-3-carboxamide hydrochloride
272 *trans*-N-(azepan-1-yl)-1-(2,4-dichlorophenyl)-5-(4-methoxyphenyl)-4-methyl-4,5-dihydro-1H-pyrazole-3-carboxamide hydrochloride
273 *cis*-N-(azepan-1-yl)-1-(2-chlorophenyl)-5-(4-methoxyphenyl)-4-methyl-4,5-dihydro-1H-pyrazole-3-carboxamide
274 *trans*-N-(azepan-1-yl)-1-(2-chlorophenyl)-5-(4-methoxyphenyl)-4-methyl-4,5-dihydro-1H-pyrazole-3-carboxamide
275 *cis*-N-cycloheptyl-1-(2,4-dichlorophenyl)-5-(4-methoxyphenyl)-4-methyl-4,5-dihydro-1H-pyrazole-3-carboxamide
276 *cis*-1-(2-chlorophenyl)-N-cycloheptyl-5-(4-methoxyphenyl)-4-methyl-4,5-dihydro-1 H-pyrazole-3-carboxamide
277 *cis*-1-(2,4-dichlorophenyl)-N-(hexahydrocyclopenta[c]pyrrol-2(1H)-yl)-5-(4-methoxyphenyl)-4-methyl-4,5-dihydro-1H-pyrazole-3-carboxamide hydrochloride
278 *cis*-1-(2-chlorophenyl)-N-(hexahydrocyclopenta[c]pyrrol-2(1H)-yl)-5-(4-methoxyphenyl)-4-methyl-4,5-dihydro-1H-pyrazole-3-carboxamide
279 *cis-*1-(2,4-dichlorophenyl)-N-(indolin-1-yl)-5-(4-methoxyphenyl)-4-methyl-4,5-dihydro-1H-pyrazole-3-carboxamide
280 *cis*-1-(2-chlorophenyl)-N-(indolin-1-yl)-5-(4-methoxyphenyl)-4-methyl-4,5-dihydro-1H-pyrazole-3-carboxamide
281 (+)-cis-1-(2.4-dichlorophenyl)-N-((1S,2S)-2-hydroxycyclohexyl)-5-(4-methoxyphenyl)-4-methyl-4,5-dihydro-1H-pyrazole-3-carboxamide
282 (-)-*cis-*1-(2,4-dichlorophenyl)-N-((1S,2S)-2-hydroxycyclohexyl)-5-(4-methoxyphenyl)-4-methyl-4,5-dihydro-1H-pyrazole-3-carboxamide
283 (+)-*cis-*1-(2-chlorophenyl)-N-((1S.2S)-2-hydroxycydohexyl)-5-(4-methoxyphenyl)-4-methyl-4,5-dihydro-1H-pyrazole-3-carboxamide
284 (-)-cis-1-(2-chlorophenyl)-N-((1S,2S)-2-hydroxycyclohexyl)-5-(4-methoxyphenyl)-4-methyl-4,5-dihydro-1H-pyrazole-3-carboxamide
285 *cis-*1-(2,4-dichlorophenyl)-N-(4-hydroxypiperidin-1-yl)-5-(4-methoxyphenyl)-4-methyl-4,5-dihydro-1H-pyrazole-3-carboxamide
286 *cis*-1-(2-chlorophenyl)-N-(4-hydroxypiperidin-1-yl)-5-(4-methoxyphenyl)-4-methyl-4,5-dihydro-1H-pyrazole-3-carboxamide
287 *cis*-1-(2,4-dichlorophenyl)-N-(3-hydroxypiperidin-1-yl)-5-(4-methoxyphenyl)-4-methyl-4,5-dihydro-1H-pyrazole-3-carboxamide
288 *cis*-1-(2-chlorophenyl)-N-(3-hydroxypiperidin-1-yl)-5-(4-methoxyphenyl)-4-methyl-4,5-dihydro-1H-pyrazole-3-carboxamide
289 *cis*-1-(2,4-dichlorophenyl)-N-(2-hydroxypiperidin-1-yl)-5-(4-methoxyphenyl)-4-methyl-4,5-dihydro-1H-pyrazole-3-carboxamide
290 *cis*-1-(2-chlorophenyl)-N-(2-hydroxypiperidin-1-yl)-5-(4-methoxyphenyl)-4-methyl-4,5-dihydro-1H-pyrazole-3-carboxamide
291 *cis*-1-(2,4-dichlorophenyl)-5-(4-methoxyphenyl)-4-methyl-N-(4-oxopiperidin-1-yl)-4,5-dihydro-1H-pyrazole-3-carboxamide
292 *cis*-1-(2-chlorophenyl)-5-(4-methoxyphenyl)-4-methyl-N-(4-oxopiperidin-1-yl)-4,5-dihydro-1H-pyrazole-3-carboxamide
293 *cis*-1-(2,4-dichlorophenyl)-5-(4-methoxyphenyl)-4-methyl-N-(3-oxopiperidin-1-yl)-4,5-dihydro-1H-pyrazole-3-carboxamide
294 *cis*-1-(2-chlorophenyl)-5-(4-methoxyphenyl)-4-methyl-N-(3-oxopiperidin-1-yl)-4,5-dihydro-1H-pyrazole-3-carboxamide
295 *cis*-1-(2,4-dichlorophenyl)-N-(3,4-dihydropyridin-1(2H)-yl)-5-(4-methoxyphenyl)-4-methyl-4,5-dihydro-1H-pyrazole-3-carboxamide
296 *cis*-1-(2-chlorophenyl)-N-(3,4-dihydropyridin-1(2H)-yl)-5-(4-methoxyphenyl)-4-methyl-4,5-dihydro-1H-pyrazole-3-carboxamide
297 *cis*-1-(2,4-dichlorophenyl)-N-(5,6-dihydropyridin-1(2H)-yl)-5-(4-methoxyphenyl)-4-methyl-4,5-dihydro-1H-pyrazole-3-carboxamide
298 *cis*-1-(2-chlorophenyl)-N-(5,6-dihydropyridin-1(2H)-yl)-5-(4-methoxyphenyl)-4-methyl-4,5-dihydro-1H-pyrazole-3-carboxamide
299 *cis*-1-(2,4-dichlorophenyl)-5-(4-methoxyphenyl)-N-(piperidin-1-yl)-4-(trifluoromethyl)-4,5-dihydro-1H-pyrazole-3-carboxamide
300 *trans*-1-(2,4-dichlorophenyl)-5-(4-methoxyphenyl)-N-(piperidin-1-yl)-4-(trifluoromethyl)-4,5-dihydro-1H-pyrazole-3-carboxamide
301 *cis*-N-(azepan-1-yl)-1-(2,4-dichlorophenyl)-5-(4-methoxyphenyl)-4-(trifluoromethyl)-4,5-dihydro-1H-pyrazole-3-carboxamide
302 *trans*-N-(azepan-1-yl)-1-(2,4-dichlorophenyl)-5-(4-methoxyphenyl)-4-(trifluoromethyl)-4,5-dihydro-1H-pyrazole-3-carboxamide
303 *cis*-1-(2-chlorophenyl)-5-(4-methoxyphenyl)-N-(pipendin-1-yl)-4-(trifluoromethyl)-4,5-dihydro-1 H-pyrazole-3-carboxamide
304 *trans*-1-(2-chlorophenyl)-5-(4-methoxyphenyl)-N-(piperidin-1-yl)-4-(trifluoromethyl)-4,5-dihydro-1H-pyrazole-3-carboxamide
305 1-(2,4-dichlorophenyl)-5-(4-methoxyphenyl)-4,4-dimethyl-N-(piperidin-1-yl)-4,5-dihydro-1 H-pyrazole-3-carboxamide
306 N-(azepan-1-yl)-1-(2,4-dichlorophenyl)-5-(4-methoxyphenyl)-4,4-dimethyl-4,5-dihydro-1 H-pyrazole-3-carboxamide
307 1-(2-chlorophenyl)-5-( 4-methoxyphenyl)-4,4-dimethyl-N-(piperidin-1-yl)-4,5-dihydro-1H-pyrazole-3-carboxamide
308 1-(2,4-dichlorophenyl)-4-formyl-5-(4-methoxyphenyl)-N-(piperidin-1-yl)-4,5-dihydro-1 H-pyrazole-3-carboxamide
309 1-(2,4-dichlorophenyl)-5-(4-methoxyphenyl)-3-(piperidin-1-ylcarbamoyl)-4,5-dihydro-1 H-pyrazole-4-carboxylic acid
310 1-(2,4-dichlorophenyl)-4-(2-hydroxyethyl)-5-(4-methoxyphenyl)-N-(piperidin-1-yl)-4,5-dihydro-1H-pyrazole-3-carboxamide
311 1 1-(2,4-dichlorophenyl)-4-(fluoromethyl)-5-(4-methoxyphenyl)-N-(piperidin-1-yl)-4,5-dihydro-1 H-pyrazole-3-carboxamide
312 *cis-*4-cyano-1-(2,4-dichlorophenyl)-5-(4-methoxyphenyl)-N-(piperidin-1-yl)-4,5-dihydro-1 H-pyrazole-3-carboxamide
313 *trans-*4-cyano-1-(2,4-dichlorophenyl)-5-(4-methoxyphenyl)-N-(piperidin-1-yl)-4,5-dihydro-1 H-pyrazole-3-carboxamide
314 *cis*-N-(azepan-1-yl)-4-cyano-1-(2,4-dichlorophenyl)-5-(4-methoxyphenyl)-4,5-dihydro-1 H-pyrazole-3-carboxamide
315 *trans*-N-(azepan-1-yl)-4-cyano-1-(2,4-dichlorophenyl)-5-(4-methoxyphenyl)-4,5-dihydro-1H-pyrazole-3-carboxamide
316 *cis* -1-(2-chlorophenyl)-4-cyano-5-(4-methoxyphenyl)-N-(piperidin-1-yl)-4,5-dihydro-1H-pyrazole-3-carboxamide
317 *trans* -1-(2-chlorophenyl)-4-cyano-5-(4-methoxyphenyl)-N-(piperidin-1-yl)-4,5-dihydro-1H-pyrazole-3-carboxamide
318 1-(2,4-dichlorophenyl)-4-hydroxy-5-(4-methoxyphenyl)-N-(piperidin-1-yl)-4,5-dihydro-1H-pyrazole-3-carboxamide
319 1-(2-chlorophenyl)-4,4-difluoro-5-(4-methoxyphenyl)-N-(piperidin-1-yl)-4,5-dihydro-1H-pyrazole-3-carboxamide
320 1-(2-chlorophenyl)-4-formyl-5-(4-methoxyphenyl)-N-(piperidin-1-yl)-4,5-dihydro-1 H-pyrazole-3-carboxamide
321 1-(2-chlorophenyl)-5-(4-methoxyphenyl)-3-(piperidin-1-ylcarbamoyl)-4,5-dihydro-1 H-pyrazole-4-carboxylic acid
322 1-(2-ch)orophenyl-4-(2-hydroxyethyl)-5-(4-methoxyphenyl)-N-(piperidin-1-yl)-4,5-dihydro-1H-pyrazole-3-carboxamide
323 1-(2-chlorophenyl)-4-(fluoromethyl)-5-(4-methoxyphenyl)-N-(piperidin-1-yl)-4,5-dihydro-1H-pyrazole-3-carboxamide
324 1-(2-chlorophenyl)-4-hydroxy-5-(4-methoxyphenyl)-N-(pipendin-1-yl)-4,5-dihydro-1 H-pyrazole-3-carboxamide
325 *cis*-1-(2,4-dichlorophenyl)-5-(4-ethoxyphenyl)-4-methyl-N-(piperidin-1-yl)-4,5-dihydro-1 H-pyrazole-3-carboxamide
326 *trans*-1-(2,4-dichlorophenyl)-5-(4-ethoxyphenyl)-4-methyl-N-(piperidin-1-yl)-4,5-dihydro-1 H-pyrazole-3-carboxamide
327 *cis*-1-(2-chlorophenyl)-5-(4-ethoxyphenyl)-4-methyl-N-(piperidin-1-yl)-4,5-dihydro-1 H-pyrazole-3-carboxamide
328 *trans-*1-(2-chlorophenyl)-5-(4-ethoxyphenyl)-4-methyl-N-(piperidin-1-yl)-4,5-dihydro-1 H-pyrazole-3-carboxamide
329 *cis*-N-(azepan-1-yl)-1-(2,4-dichlorophenyl)-5-(4-ethoxyphenyl)-4-methyl-4,5-dihydro-1 H-pyrazole-3-carboxamide
330 *trans*-N-(azepan-1-yl)-1-(2,4-dichlorophenyl)-5-(4-ethoxyphenyl)-4-methyl-4,5-dihydro-1H-pyrazole-3-carboxamide
331 *cis*-N-(azepan-1-yl)-1-(2-chlorophenyl)-5-(4-ethoxyphenyl)-4-methyl-4,5-dihydro-1 H-pyrazole-3-carboxamide
332 *trans*-N-(azepan-1-yl)-1-(2-chlorophenyl)-5-(4-ethoxyphenyl)-4-methyl-4,5-dihydro-1H-pyrazole-3-carboxamide
333 *cis*-N-cycloheptyl-1-(2,4-dichlorophenyl)-5-(4-ethoxyphenyl)-4-methyl-4,5-dihydro-1 H-pyrazole-3-carboxamide
334 *cis*-1-(2-chlorophenyl)-N-cycloheptyl-5-(4-ethoxyphenyl)-4-methyl-4,5-dihydro-1 H-pyrazole-3-carboxamide
335 c*is*-1-(2,4-dichlorophenyl)-5-(4-ethoxyphenyl)-N-(hexahydrocyclopenta[c]pyrrol-2(1H)-yl)-4-methy)-4,5-dihydro-1H-pyrazole-3-carboxamide
336 *cis*-1-(2-chlorophenyl)-5-(4-ethoxyphenyl)-N-(hexahydrocyclopenta[c]pyrrol-2(1 H)-yl)-4-methyl-4,5-dihydro-1 H-pyrazole-3-carboxamide
337 *cis*-1-(2,4-dichlorophenyl)-5-(4-ethoxyphenyl)-N-(indolin-1-yl)-4-methyl-4,5-dihydro-1 H-pyrazole-3-carboxamide
338 *cis*-1-(2-chlorophenyl)-5-(4-ethoxyphenyl)-N-(indolin-1-yl)-4-methyl-4,5-dihydro-1H-pyrazole-3-carboxamide
339 (+)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide
340 (-)-cis-1-(2,4-dichlorophenyl)-5-(4-ethoxyphenyl)-N-((1S,2S)-2-hydroxycyclohexyl)-4-methyl-4,5-dihydro-1H-pyrazole-3-carboxamide
341 (+)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide
342 (-)-cis-1-(2-chlorophenyl)-5-(4-ethoxyphenyl)-N-((1S,2S)-2-hydroxycyclohexyl)-4-methyl-4,5-dihydro-1H-pyrazole-3-carboxamide
343 *cis-*1-(2,4-dichlorophenyl)-5-(4-hydroxyphenyl)-4-methyl-N-(piperidin-1-yl)-4,5-dihydro-1H-pyrazole-3-carboxamide
344 *trans-*1-(2,4-dichlorophenyl)-5-(4-hydroxyphenyl)-4-methyl-N-(piperidin-1-yl)-4,5-dihydro-1H-pyrazole-3-carboxamide
345 *cis*-1-(2-chlorophenyl)-5-(4-hyd roxyphenyl)-4-methyl-N-(piperidin-1-yl)-4,5-dihydro-1H-pyrazole-3-carboxamide
346 *trans*-1-(2-chlorophenyl)-5-(4-hydroxyphenyl)-4-methyl-N-(piperidin-1-yl)-4,5-dihydro-1H-pyrazole-3-carboxamide
347 *cis*-N-(azepan-1-yl)-1-(2,4-dichlorophenyl)-5-(4-hydroxyphenyl)-4-methyl-4,5-dihydro-1H-pyrazole-3-carboxamide
348 *trans*-N-(azepan-1-yl)-1-(2,4-dichlorophenyl)-5-(4-hydroxyphenyl)-4-methyl-4,5-dihydro-1H-pyrazole-3-carboxamide
349 *cis*-N-(azepan-1-yl)-1-(2-chlorophenyl)-5-(4-hydroxyphenyl)-4-methyl-4,5-dihydro-1H-pyrazole-3-carboxamide
350 *trans*-N-(azepan-1-yl)-1-(2-chlorophenyl)-5-(4-hydroxyphenyl)-4-methyl-4,5-dihydro-1H-pyrazole-3-carboxamide
351 *cis-*N-cycloheptyl-1-(2,4-dichlorophenyl)-5-(4-hydroxyphenyl)-4-methyl-4,5-dihydro-1H-pyrazole-3-carboxamide
352 *cis*-1-(2-chlorophenyl)-N-cycloheptyl-5-(4-hydroxyphenyl)-4-methyl-4,5-dihydro-1H-pyrazole-3-carboxamide
353 *cis*-1-(2,4-dichlorophenyl)-N-(hexahydrocyclopenta[c]pyrrol-2(1H)-yl)-5-(4-hydroxyphenyl)-4-methyl-4,5-dihydro-1H-pyrazole-3-carboxamide hydrochloride
354 *cis*-1-(2-chlorophenyl)-N-(hexahydrocyclopenta[c]pyrrol-2(1H)-yl)-5-(4-hydroxyphenyl)-4-methyl-4,5-dihydro-1H-pyrazole-3-carboxamide
355 *cis*-1-(2,4-dichlorophenyl)-5-(4-hydroxyphenyl)-N-(indolin-1-yl)-4-methyl-4,5-dihydro-1H-pyrazole-3-carboxamide
356 *cis*-1-(2-chlorophenyl)-5-(4-hydroxyphenyl)-N-(indolin-1-yl)-4-methyl-4,5-dihydro-1 H-pyrazole-3-carboxamide
357 (+)-cis-1-(2,4-dichlorophenyl)-N-((1S,2S)-2-hydroxycyclohexyl)-5-(4-hydroxyphenyl)-4-methyl-4,5-dihydro-1H-pyrazole-3-carboxamide
358 (-)-cis-1-(2,4-dichlorophenyl)-N-((1S,2S)-2-hydroxycyclohexyl)-5-(4-hydroxyphenyl)-4-methyl-4,5-dihydro-1H-pyrazole-3-carboxamide
359 (+)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide
360 (-)-cis-1-(2-chlorophenyl)-N-((1S,2S)-2-hydroxycyclohexyl)-5-(4-hydroxyphenyl)-4-methyl-4,5-dihydro-1H-pyrazole-3-carboxamide
361 *cis*-5-(5-chlorothiophen-2-yl)-1-(2,4-dichlorophenyl)-4-methyl-4,5-dihydro-1H-pyrazole-3-carboxylic acid
362 *trans*-5-(5-chlorothiophen-2-yl)-1-(2,4-dichlorophenyl)-4-methyl-4,5-dihydro-1H-pyrazole-3-carboxylic acid
363 *cis*-1-(2-chlorophenyl)-5-(5-chlorothiophen-2-yl)-4-methyl-4,5-dihydro-1H-pyrazole-3-carboxylic acid
364 *trans*-1-(2-chlorophenyl)-5-(5-chlorothiophen-2-yl)-4-methyl-4,5-dihydro-1H-pyrazole-3-carboxylic acid
365 *cis*-5-(5-bromothiophen-2-yl)-1-(2,4-dichlorophenyl)-4-ethyl-4,5-dihydro-1H-pyrazole-3-carboxylic acid
366 *trans*-5-(5-bromothiophen-2-yl)-1-(2,4-dichlorophenyl)-4-ethyl-4,5-dihydro-1H-pyrazole-3-carboxylic acid
367 *cis*-1-(2,4-dichlorophenyl)-4-methyl-5-(thiophen-2-yl)-4,5-dihydro-1H-pyrazole-3-carboxylic acid
368 *trans*-1-(2,4-dichlorophenyl)-4-methyl-5-(thiophen-2-yl)-4,5-dihydro-1H-pyrazole-3-carboxylic acid
369 *cis-*1-(2,4-dichlorophenyl)-4-methyl-N-(piperidin-1-yl)-5-(thiophen-2-yl)-4,5-dihydro-1H-pyrazole-3-carboxamide
370 *trans*-1-(2,4-dichlorophenyl)-4-methyl-N-(piperidin-1-yl)-5-(thiophen-2-yl)-4,5-dihydro-1 H-pyrazole-3-carboxamide
371 *cis*-1-(2-chlorophenyl)-4-methyl-N-(piperidin-1-yl)-5-(thiophen-2-yl)-4,5-dihydro-1 H-pyrazole-3-carboxamide
372 *trans-*1-(2-chlorophenyl)-4-methyl-N-(piperidin-1-yl)-5-(thiophen-2-yl)-4,5-dihydro-1 H-pyrazole-3-carboxamide
373 *cis*-1-(2,4-dichlorophenyl)-4-methyl-5-phenyl-4,5-dihydro-1H-pyrazole-3-carboxylic acid
374 *trans*-1-(2,4-dichlorophenyl)-4-methyl-5-phenyl-4,5-dihydro-1H-pyrazole-3-carboxylic acid
375 *cis*-1-(2,4-dichlorophenyl)-4-methyl-5-phenyl-N-(piperidin-1-yl)-4,5-dihydro-1H-pyrazole-3-carboxamide
376 *trans* -1-(2,4-dichlorophenyl)-4-methyl-5-phenyl-N-(piperidin-1-yl)-4,5-dihydro-1H-pyrazole-3-carboxamide
377 *cis*-1-(2-chlorophenyl)-4-methyl-5-phenyl-N-(piperidin-1-yl)-4,5-dihydro-1H-pyrazole-3-carboxamide
378 *trans*-1-(2-chlorophenyl)-4-methyl-5-phenyl-N-(piperidin-1-yl)-4,5-dihydro-1H-pyrazole-3-carboxamide
379 *cis*-5-(4-chlorophenyl)-1-(2,4-dichlorophenyl)-4-methyl-N-(piperidin-1-yl)-4,5-dihydro-1H-pyrazole-3-carbothioamide hydrochloride
380 *trans*-5-(4-chlorophenyl)-1-(2,4-dichlorophenyl)-4-methyl-N-(piperidin-1-yl)-4,5-dihydro-1H-pyrazole-3-carbothioamide
381 *cis* -1-(2-chlorophenyl)-5-(4-chlorophenyl)-4-methyl-N-(piperidin-1-yl)-4,5-dihydro-1 H-pyrazole-3-carbothioamide
382 *trans*-1-(2-chlorophenyl)-5-(4-chlorophenyl)-4-methyl-N-(piperidin-1-yl)-4,5-dihydro-1H-pyrazole-3-carbothioamide
383 *cis*-N-(azepan-1-yl)-5-(4-chlorophenyl)-1-(2,4-dichlorophenyl)-4-methyl-4,5-dihydro-1H-pyrazole-3-carbothioamide
384 *cis*-N-(azepan-1-yl)-1-(2-chlorophenyl)-5-(4-chlorophenyl)-4-methyl-4,5-dihydro-1 H-pyrazole-3-carbothioamide
385 *cis*-5-(4-bromophenyl)-1-(2,4-dichlorophenyl)-4-methyl-N-(piperidin-1-yl)-4,5-dihydro-1H-pyrazole-3-carbothioamide
386 *trans*-5-(4-bromophenyl)-1-(2,4-dichlorophenyl)-4-methyl-N-(piperidin-1-yl)-4,5-dihydro-1H-pyrazole-3-ca rbothioamide
387 *cis-*5-(4-bromophenyl)-1-(2-chlorophenyl)-4-methyl-N-(piperidin-1-y1)-4,5-dihydro-1H-pyrazole-3-carbothioamide
388 *trans*-5-(4-bromophenyl)-1-(2-chlorophenyl)-4-methyl-N-(piperidin-1-yl)-4,5-dihydro-1H-pyrazole-3-carbothioamide
389 *cis-*1-(2,4-dichlorophenyl)-5-(4-fluorophenyl)-4-methyl-N-(piperidin-1-yl)-4,5-dihydro-1 H-pyrazole-3-carbothioamide
390 *trans*-1-(2,4-dichlorophenyl)-5-(4-fluorophenyl)-4-methyl-N-(piperidin-1-yl)-4,5-dihydro-1 H-pyrazole-3-carbothioamide
391 *cis*-1-(2-chlorophenyl)-5-(4-fluorophenyl)-4-methyl-N-(piperidin-1-yl)-4,5-dihydro-1 H-pyrazole-3-carbothioamide
392 *trans*-1-(2-chlorophenyl)-5-(4-fluorophenyl)-4-methyl-N-(piperidin-1-yl)-4,5-dihydro-1 H-pyrazole-3-carbothioamide
393 *cis*-1-(2,4-dichlorophenyl)-5-(4-methoxyphenyl)-4-methyl-N-(piperidin-1-yl)-4,5-dihydro-1 H-pyrazole-3-carbothioamide
394 *cis*-N-(azepan-1-yl)-1-(2,4-dichlorophenyl)-5-(4-methoxyphenyl)-4-methyl-4,5-dihydro-1 H-pyrazole-3-carbothioamide
395 *trans*-1-(2,4-dichlorophenyl)-5-(4-methoxyphenyl)-4-methyl-N-(piperidin-1-yl)-4,5-dihydro-1 H-pyrazole-3-carbothioamide
396 *cis*-1-(2-chlorophenyl)-5-(4-methoxyphenyl)-4-methyl-N-(piperidin-1-yl)-4,5-dihydro-1 H-pyrazole-3-ca rboth ioa m id e
397 cis-N-(azepan-1-yl)-1-(2-chlorophenyl)-5-(4-methoxyphenyl)-4-methyl-4,5-dihydro-1H-pyrazole-3-carbothioamide
398 *trans*-1-(2-chlorophenyl)-5-(4-methoxyphenyl)-4-methyl-N-(piperidin-1-yl)-4,5-dihydro-1H-pyrazole-3-carbothioamide
399 *cis-*1-(2,4-dichlorophenyl)-5-(4-ethoxyphenyl)-4-methyl-N-(piperidin-1-yl)-4,5-dihydro-1H-pyrazole-3-carbothioamide
400 *trans*-1-(2,4-dichlorophenyl)-5-(4-ethoxyphenyl)-4-methyl-N-(piperidin-1-yl)-4,5-dihydro-1H-pyrazole-3-carbothioamide
401 *cis*-1-(2-chlorophenyl)-5-(4-ethoxyphenyl)-4-methyl-N-(piperidin-1-yl)-4,5-dihydro-1 H-pyrazole-3-carbothioamide
402 *trans*-1-(2-chlorophenyl)-5-(4-ethoxyphenyl)-4-methyl-N-(piperidin-1-yl)-4,5-dihydro-1H-pyrazole-3-carbothioamide
403 *cis*-1-(2,4-dichlorophenyl)-5-(4-hydroxyphenyl)-4-methyl-N-(piperidin-1-yl)-4,5-dihydro-1H-pyrazole-3-carbothioamide
404 *trans*-1-(2,4-dichlorophenyl)-5-(4-hydroxyphenyl)-4-methyl-N-(piperidin-1-yl)-4,5-dihydro-1H-pyrazole-3-carbothioamide
405 *cis*-1-(2-chlorophenyl)-5-(4-hydroxyphenyl)-4-methyl-N-(piperidin-1-yl)-4,5-dihydro-1H-pyrazole-3-carbothioamide
406 *trans*-1-(2-chlorophenyl)-5-(4-hydroxyphenyl)-4-methyl-N-(piperidin-1-yl)-4,5-dihydro-1H-pyrazole-3-carbothioamide
407 7-(4-Chloro-phenyl)-6-(2,4-dichlorophenyl)-5,6-diaza-spiro[2.4]hept-4-ene-4-carboxylic acid piperidin-1-ylamide
408 6-(2,4-Dichloro-phenyl)-7-(4-methoxy-phenyl)-5,6-diaza-spiro[2.4]hept-4-ene-4-carboxylic acid piperidin-1-ylamide
409 (+)-cis-N-((1S,2S)-2-(benzyloxy)cyclohexyl)-5-(4-chlorophenyl)-1-(2,4-dichlorophenyl)-4-methyl-4,5-dihydro-1H-pyrazole-3-carboxamide
[410] *cis*-1-(2,4-dichlorophenyl)-5-(4-methoxyphenyl)-4-methyl-N-(piperidin-1-yl)-4,5-dihydro-1H-pyrazole-3-carboxamide
[411] *cis*-1-(2,4-dichlorophenyl)-5-(4-methoxyphenyl)-4-methyl-N-(piperidin-1-yl)-4,5-dihydro-1H-pyrazole-3-carboxamide
[412] (4R,5S)-1-(2,4-dichlorophenyl)-5-(4-methoxyphenyl)-4-methyl-4,5-dihydro-1H-pyrazole-3-carboxylic acid
[413] cis-5-(4-bromophenyl)-1-(2,4-dichlorophenyl)-4-methyl-4,5-dihydro-1H-pyrazole-3-carboxylic acid
[414] cis-N-(azepan-1-yl)-1-(2,4-dichlorophenyl)-5-(4-fluorophenyl)-4-methyl-4,5-dihydro-1H-pyrazole-3-carboxamide
415 *cis*-5-(4-bromophenyl)-1-(2,4-dichlorophenyl)-N-(indolin-1-yl)-4-methyl-4,5-dihydro-1H-pyrazole-3-carboxamide
416 trans-ethyl 5-(4-chlorophenyl)-1-(2,4-dichlorophenyl)-4-(trifluoromethyl)-4,5-dihydro-1 H-pyrazole-3-carboxylate
417 cis-1-(2,4-dichlorophenyl)-5-(4-fluorophenyl)-4-methyl-N-(piperidin-1-yl)-4,5-dihydro-1H-pyrazole-3-carboxamide hydrochloride
418 cis-5-(4-bromophenyl)-1-(2,4-dichlorophenyl)-4-methyl-N-(piperidin-1-yl)-4,5-dihydro-1 H-pyrazole-3-carboxamide hydrochloride
419 (-)-*cis*-5-(4-chlorophenyl)-1-(2,4-dichlorophenyl)-4-methyl-N-(piperidin-1-yl)-4,5-dihydro-1 H-pyrazole-3-carboxamide
420 (+)-*cis*-5-(4-chlorophenyl)-1-(2,4-dichlorophenyl)-4-methyl-N-(piperidin-1-yl)-4,5-dihydro-1H-pyrazole-3-carboxamide
421 *trans*-ethyl 1-(2,4-dichlorophenyl)-4-methyl-5-phenyl-4,5-dihydro-1H-pyrazole-3-carboxylate
422 *cis*-5-(4-chlorophenyl)-1-(2,4-dichlorophenyl)-4-methyl-N-((1R,2S,4R)-1,7,7-trimethylbicyclo[2.2.1]heptan-2-yl)-4,5-dihydro-1H-pyrazole-3-carboxamide
423 *cis*-5-(4-chlorophenyl)-1-(2,4-dichlorophenyl)-4-methyl-N-(3-methylcyclohexyl)-4,5-dihydro-1H-pyrazole-3-carboxamide
424 *cis*-5-(4-chlorophenyl)-1-(2,4-dichlorophenyl)-4-methyl-N-(2-methylcyclohexyl)-4,5-dihydro-1 H-pyrazole-3-carboxamide
426 *trans*-5-(5-chlorothiophen-2-yl)-(2,4-dichlorophenyl)-4-methyl-N-(piperidin-1-yl)-4,5-dihydro-1H-pyrazole-3-carboxamide hydrochloride
427 *cis*-5-(5-chlorothiophen-2-yl)-1-(2,4-dichlorophenyl)-4-methyl-N-(piperidin-1-yl)-4,5-dihydro-1H-pyrazole-3-carboxamide hydrochloride
428 *cis*-5-(4-bromophenyl)-1-(2,4-dichlorophenyl)-4-ethyl-N-(piperidin-1-yl)-4,5-dihydro-1H-pyrazole-3-carboxamide hydrochloride
[429] 1-(2,4-dichlorophenyl)-4-methyl-5-phenyl-4,5-dihydro-1H-pyrazole-3-carboxylic acid
[430] cis-1-(2,4-dichlorophenyl)-4-methyl-N-(piperidin-1-yl)-5-(thiophen-2-yl)-4,5-dihydro-1H-pyrazole-3-carboxamide hydrochloride
[431] trans-5-(4-chlorophenyl)-1-(2,4-dichlorophenyl)-4-methyl-N-(piperidin-1-yl)-4,5-dihydro-1H-pyrazole-3-carboxamide
432 *cis*-5-(4-chlorophenyl)-1-(2,4-dichlorophenyl)-4-ethyl-N-(piperidin-1-yl)-4,5-dihydro-1H-pyrazole-3-carboxamide
434 *cis*-5-(4-chlorophenyl)-1-(2,4-dichlorophenyl)-4-methyl-N-(piperidin-1-yl)-4,5-dihydro-1H-pyrazole-3-carboxamide
436 (+)-*cis*-N-((1S,2S)-2-(benzyloxy)cyclohexyl)-5-(4-chlorophenyl)-1-(2,4-dichlorophenyl)-4-methyl-4,5-dihydro-1H-pyrazole-3-carboxamide
437 (-)-*cis*-N-((1R,2R)-2-(benzyloxy)cyclohexyl)-5-(4-chlorophenyl)-1-(2,4-dichlorophenyl)-4-methyl-4,5-dihydro-1H-pyrazole-3-carboxamide
438 *cis*-N-(azocan-1-yl)-1-(2,4-dichlorophenyl)-5-(4-methoxyphenyl)-4-methyl-4,5-dihydro-1H-pyrazole-3-carboxamide
439 *cis*-N-(azocan-1-yl)-1-(2-chlorophenyl)-5-(4-methoxyphenyl)-4-methyl-4,5-dihydro-1H-pyrazole-3-carboxamide
440 *cis*-N-(azocan-1-yl)-5-(4-bromophenyl)-1-(2,4-dichlorophenyl)-4-methyl-4,5-dihydro-1H-pyrazole-3-carboxamide
441 *cis*-N-(azocan-1-yl)-5-(4-bromophenyl)-1-(2-chlorophenyl)-4-methyl-4,5-dihydro-1 H-pyrazole-3-carboxamide
442 *cis*-N-(azocan-1-yl)-1-(2,4-dichlorophenyl)-5-(4-fluorophenyl)-4-methyl-4,5-dihydro-1 H-pyrazole-3-carboxamide
443 *cis*-N-(azocan-1-yl)-1-(2-chlorophenyl)-5-(4-fluorophenyl)-4-methyl-4,5-dihydro-1 H-pyrazole-3-carboxamide
444 (+)-*cis*-5-(4-Chlorophenyl)-1-(2,4-dichlorophenyl)-4-methyl-4,5-dihydro-1H-pyrazole-3-carboxylic acid azepan-1-ylamide hydrochloride
445 (-)*-cis-* (4-Chlorophenyl)-1-(2,4-dichlorophenyl)-4-methyl-4,5-dihydro-1H-pyrazole-3-carboxylic acid azepan-1-ylamide hydrochloride
[446] *cis-*5-(4-chlorophenyl)-1-(2,4-dichlorophenyl)-4-methyl-N-(piperidin-1-yl)-4,5-dihydro-1H-pyrazole-3-carboxamide N-oxide
[447] *cis*-5-(4-chlorophenyl)-1-(2-chlorophenyl)-4-methy)-N-(piperidin-1-yl)-4,5-dihydro-1 H-pyrazole-3-carboxamide N-oxide
[448] *cis*-N-(azepan-1-yl)-5-(4-chlorophenyl)-1-(2,4-dichlorophenyl)-4-methyl-4,5-dihydro-1H-pyrazole-3-carboxamide N-oxide
[449] *cis-*N*-*(azepan-1-yl)-5-(4-chlorophenyl)-1-(2-chlorophenyl)-4-methyl-4,5-dihydro-1 H-pyrazole-3-carboxamide N-oxide
[450] *cis*-1-(2,4-dichlorophenyl)-5-(4-methoxyphenyl)-4-methyl-N-(piperidin-1-yl)-4,5-dihydro-1H-pyrazole-3-carboxamide N-oxide and
[451] *cis*-1-(2-chlorophenyl)-5-(4-methoxyphenyl)-4-methyl-N-(piperidin-1-yl)-4,5-dihydro-1H-pyrazole-3-carboxamide N-oxide;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

16. Active substance combination according to one or more of claims 1-15, wherein the drug of component (B) is selected from the group consisting of a drug selected from the following therapeutic classes: antipsychotics, comprising the atypical antipsychotics and typical neuroleptics, antidepressants including MAOls, TCAs, SSRls, "third generation" and atypical antidepressants, anti-manic agents, corticosteroids, medicaments for treating insulin resistance, impaired glucose tolerance and/or Type 2 diabetes, antihypertensives, anti-ulceratives, medicaments for treating neuropathic pain, antihistamines, medicaments for treating migraine, benzodiazepines, contraceptives and for hormone replacement therapy.

17. Active substance combination according to claim 16, wherein the drug of component (B) is selected from
Antipsychotics, comprising the atypical antipsychotics including, olanzapine clozapine, quetiepine, risperidone, amisulpiride, zotepine, sertindole, aripiprazole and ziprasidone, and the older typical neuroleptics including, chlorpromazine, haloperidol, fluphenazine, mesoridazine and thioridazine;
Antidepressants, comprising the non-selective, irreversible monoamine oxidase A and B inhibitors (MAOIs) including tranylcypromine, phenelzine and isocarboxacid, the tricyclic antidepressants (TCAs) including, amitriptyline, amoxapine, clomipramine, desipramine, doxepin, imiprimine, nortriptyline, protriptyline and trimiprimine, the serotonin-selective reuptake inhibitors including fluoxetine, sertraline, paroxetine and citalopram, the "third generation" and atypical antidepressants" including mirtazepine, venlafaxine, mianserin and trazodone;
Drugs to treat mania in bipolar disorder including lithium, olanzapine, quetiepine and sodium valproate;
Corticosteroids to treat inflammation including, prednisone, prednisolone, budesonide, dexamethasone, methylprednisolone and betamethasone;
Drugs for the treatment of Type 1 diabetes, insulin resistance, impaired glucose tolerance and Type 2 diabetes including the sulphonylureas (chlorpropamide, tolbutamide, acetoheximide, tolazamide, glibenclamide, glipizide, gliclazide, glimepiride, gliquidone, glyburide), the meglitinides (netaglinide, repaglinide, mitiglitinide), peroxisome proliferator activated receptors type gamma (PPARγ) agonists (troglitazone, rosiglitazone, pioglitazone, ciglitazone, darglitazone), inhibitors of hepatic glucose production (glucagon antagonists, glucose-6-phosphatase inhibitors, glycogen phosphorylase inhibitors), insulin (human insulin lispro, human insulin aspart, neutral insulin, human neutral insulin [pyr], human neutral insulin [prb], bovine neutral insulin, porcine neutral insulin, human isophane insulin, isophase insulin [prb], human isophane insulin [pyr], porcine isophane insulin, bovine isophane insulin, human insulin zinc suspension [pyr], humulin [prb], porcine insulin zinc suspension, human insulin zinc suspension [prb], human insulin glargine, protamine zinc insulin injection [prb], bovine insulin zinc suspension, bovine protamine zinc insulin, biphasic human insulin [pyr], biphasic human insulin [prb], biphasic neutral and isophane human insulin [prb], human insulin [emp], biphasic porcine neutral and isophane insulin, biphasic insulin aspart, insulin determir), dual acting insulin secretagogues/insulin sensitisers (pituitary adenylate cyclase-activating peptide/ vasoactive intestinal peptide receptor [VPAC] 2 receptor agonists), modulators of glucose uptake (GLUT 4 translocators, retinoid X receptor agonists, |κ*β* kinase [IKK] *β* inhibitors, p38 MAP kinase inhibitors, protein tyrosine phosphatase 1 B [PTP1 B] inhibitors, insulin receptor tyrosine kinase activators, α-glucosidase inhibitors, sodium/glucose co-transport inhibitors, glycogen synthase kinase 3 [GSK3] inhibitors) and modulators of mitochondrial metabolism. Medications may also comprise combinations of the above listed compounds, including, but not limited to rosiglitazone + metformin, rosiglitazone + simvastatin, rosiglitazone + sulphonurea, metformin + glipizide, sitagliptin + metformin, PPARγ/α combination medications (sipoglitazar, tesaglitazar, muraglitazar, reglitazar, peliglitazar, cevoglitazar, ragaglitazar, naveglitazar, imiglitazar, aloglitazar, sodelglitazar, peliglitazar, pemoglitazar, netoglitazone), PPARγ/α/δ combination medications;
Antihypertensives including β-adrenoceptor blockers, eg propranolol, metoprolol and atenolol, and α₁-adrenoceptor blockers, eg doxasin and prazosin;
Drugs for the treatment of gastro-oesophogeal reflux, dyspepsia and ulcers including esomeprazole, omeprazole and lansoprazole;
Drugs for the treatment and prevention of epilepsy and seizure disorders including vigabatrin, piracetam, carbamazepine, pregabalin, gabapentin, lamotrigine, and sodium valproate;
Drugs for the treatment or prevention of neuropathic pain including carbamazepine, pregabalin, gabapentin, sodium valproate, lamotrigine and the tricyclic antidepressants, eg amitriptyline, amoxapine, clomipramine, desipramine, doxepin, imiprimine, nortriptyline, protriptyline and trimiprimine, or atypical and "third generation" antidepressant drugs, eg mirtazepine, venlafaxine, mianserin and trazodone;
Drugs for the treatment and prevention of migraine including methysergide, pizotifen, memantine, antiepileptic drugs, eg vigabatrin, piracetam, carbamazepine, pregabalin, gabapentin, lamotrigine, and sodium valproate, tricyclic antidepressants, eg amitriptyline, amoxapine, clomipramine, desipramine, doxepin, imiprimine, nortriptyline, protriptyline and trimiprimine, the serotonin-selective reuptake inhibitors including, but not limited to, fluoxetine, sertraline, paroxetine and citalopram, and atypical and "third generation" antidepressant drugs, eg mirtazepine, venlafaxine, mianserin and trazodone;
Antihistamines including diphenhydramine and chlorpheniramine;
Benzodiazepines including clobazam;
Contraceptive medication, particularly injectable depot contraceptives including depot medroxyprogesterone acetate (DMPA); or
Hormone replacement therapy including, but not limited to, synthetic or natural oestrogen and progesterone and their analogues.

18. Active substance combination according to one or more of claims 1-17 comprising
(A) one substituted pyrazoline compound (A) of general formula I selected from the group consisting of
(rac)-N-piperidinyl-5-(4-chloro-phenyl)-1-(2,4-dichlorophenyl)-4,5-dihydro-1H-pyrazol-3-carboxamide,
(R)-N-piperidinyl-5-(4-chloro-phenyl)-1-(2,4-dichlorophenyl)-4,5-dihydro-1H-pyrazol-3-carboxamide, or
(S)-N-piperidinyl-5-(4-chloro-phenyl)-1-(2,4-dichlorophenyl)-4,5-dihydro-1H-pyrazol-3-carboxamide,
in any mixing ratio, or a corresponding N-oxide thereof, or a corresponding salt thereof, or a corresponding solvate thereof,
and
(B) at least one drug of component (B) selected from the following therapeutic classes: antipsychotics, comprising the atypical antipsychotics and typical neuroleptics, antidepressants including MAOIs, TCAs, SSRIₛ, "third generation" and atypical antidepressants, anti-manic agents, corticosteroids, medicaments for treating insulin resistance, impaired glucose tolerance and/or Type 2 diabetes, antihypertensives, anti-ulceratives, medicaments for treating neuropathic pain, antihistamines, medicaments for treating migraine, benzodiazepines, contraceptives and for hormone replacement therapy.

19. Active substance combination according to one or more of claims 1-18 comprising
(A) one substituted pyrazoline compounds of general formula I selected from the group consisting of
(rac)-N-piperidinyl-5-(4-chloro-phenyl)-1-(2,4-dichlorophenyl)-4,5-dihydro-1H-pyrazol-3-carboxamide,
(R)-N-piperidinyl-5-(4-chloro-phenyl)-1-(2,4-dichlorophenyl)-4,5-dihydro-1H-pyrazol-3-carboxamide, or
(S)-N-piperidinyl-5-(4-chloro-phenyl)-1-(2,4-dichlorophenyl)-4,5-dihydro-1H-pyrazol-3-carboxamide,
in any mixing ratio, or a corresponding N-oxide thereof, or a corresponding salt thereof, or a corresponding solvate thereof,
and
(B) at least one drug of component (B) selected from
Antipsychotics, comprising the atypical antipsychotics including, olanzapine clozapine, quetiepine, risperidone, amisulpiride, zotepine, sertindole, aripiprazole and ziprasidone, and the older typical neuroleptics including, chlorpromazine, haloperidol, fluphenazine, mesoridazine and thioridazine;
Antidepressants, comprising the non-selective, irreversible monoamine oxidase A and B inhibitors (MAOls) including tranylcypromine, phenelzine and isocarboxacid, the tricyclic antidepressants (TCAs) including, amitriptyline, amoxapine, clomipramine, desipramine, doxepin, imiprimine, nortriptyline, protriptyline and trimiprimine, the serotonin-selective reuptake inhibitors including fluoxetine, sertraline, paroxetine and citalopram, the "third generation" and atypical antidepressants" including mirtazepine, venlafaxine, mianserin and trazodone;
Drugs to treat mania in bipolar disorder including lithium, olanzapine, quetiepine and sodium valproate;
Corticosteroids to treat inflammation including, prednisone, prednisolone, budesonide, dexamethasone, methylprednisolone and betamethasone;
Drugs for the treatment of Type 1 diabetes, insulin resistance, impaired glucose tolerance and Type 2 diabetes including the sulphonylureas (chlorpropamide, tolbutamide, acetoheximide, tolazamide, glibenclamide, glipizide, gliclazide, glimepiride, gliquidone, glyburide), the meglitinides (netaglinide, repaglinide, mitiglitinide), peroxisome proliferator activated receptors type gamma (PPARγ) agonists (troglitazone, rosiglitazone, pioglitazone, ciglitazone, darglitazone), inhibitors of hepatic glucose production (glucagon antagonists, glucose-6-phosphatase inhibitors, glycogen phosphorylase inhibitors), insulin (human insulin lispro, human insulin aspart, neutral insulin, human neutral insulin [pyr], human neutral insulin [prb], bovine neutral insulin, porcine neutral insulin, human isophane insulin, isophase insulin [prb], human isophane insulin [pyr], porcine isophane insulin, bovine isophane insulin, human insulin zinc suspension [pyr[, humulin [prb], porcine insulin zinc suspension, human insulin zinc suspension [prb], human insulin glargine, protamine zinc insulin injection [prb], bovine insulin zinc suspension, bovine protamine zinc insulin, biphasic human insulin [pyr], biphasic human insulin [prb], biphasic neutral and isophane human insulin [prb], human insulin [emp], biphasic porcine neutral and isophane insulin, biphasic insulin aspart, insulin determir), dual acting insulin secretagogues/insulin sensitisers (pituitary adenylate cyclase-activating peptide/ vasoactive intestinal peptide receptor [VPAC] 2 receptor agonists), modulators of glucose uptake (GLUT 4 translocators, retinoid X receptor agonists, Iκβ kinase [IKK] β inhibitors, p38 MAP kinase inhibitors, protein tyrosine phosphatase 1 B [PTP1 B] inhibitors, insulin receptor tyrosine kinase activators, α-glucosidase inhibitors, sodium/glucose co-transport inhibitors, glycogen synthase kinase 3 [GSK3] inhibitors) and modulators of mitochondrial metabolism. Medications may also comprise combinations of the above listed compounds, including, but not limited to rosiglitazone + metformin, rosiglitazone + simvastatin, rosiglitazone + sulphonurea, metformin + glipizide, sitagliptin + metformin, PPARγ/α combination medications (sipoglitazar, tesaglitazar, muraglitazar, reglitazar, peliglitazar, cevoglitazar, ragaglitazar, naveglitazar, imiglitazar, aloglitazar, sodelglitazar, peliglitazar, pemoglitazar, netoglitazone), PPARγ/α/δ combination medications;
Antihypertensives including β-adrenoceptor blockers, eg propranolol, metoprolol and atenolol, and α₁-adrenoceptor blockers, eg doxasin and prazosin;
Drugs for the treatment of gastro-oesophogeal reflux, dyspepsia and ulcers including esomeprazole, omeprazole and lansoprazole;
Drugs for the treatment and prevention of epilepsy and seizure disorders including vigabatrin, piracetam, carbamazepine, pregabalin, gabapentin, lamotrigine, and sodium valproate;
Drugs for the treatment or prevention of neuropathic pain including carbamazepine, pregabalin, gabapentin, sodium valproate, lamotrigine and the tricyclic antidepressants, eg amitriptyline, amoxapine, clomipramine, desipramine, doxepin, imiprimine, nortriptyline, protriptyline and trimiprimine, or atypical and "third generation" antidepressant drugs, eg mirtazepine, venlafaxine, mianserin and trazodone;
Drugs for the treatment and prevention of migraine including methysergide, pizotifen, memantine, antiepileptic drugs, eg vigabatrin, piracetam, carbamazepine, pregabalin, gabapentin, lamotrigine, and sodium valproate, tricyclic antidepressants, eg amitriptyline, amoxapine, clomipramine, desipramine, doxepin, imiprimine, nortriptyline, protriptyline and trimiprimine, the serotonin-selective reuptake inhibitors including, but not limited to, fluoxetine, sertraline, paroxetine and citalopram, and atypical and "third generation" antidepressant drugs, eg mirtazepine, venlafaxine, mianserin and trazodone;
Antihistamines including diphenhydramine and chlorpheniramine;
Benzodiazepines including clobazam;
Contraceptive medication, particularly injectable depot contraceptives including depot medroxyprogesterone acetate (DMPA); or
Hormone replacement therapy including, but not limited to, synthetic or natural oestrogen and progesterone and their analogues.

20. Active substance combination according to one or more of claims 1-19, **characterized in that** the molar ratio of component (A) to component (B) is in the range of 1:10 to 10:1, preferably 1:5 to 5:1.

21. Medicament comprising an active substance combination according to one or more of claims 1 to 20 and optionally at least one further active substance and/or optionally at least one auxiliary substance.

22. Use of an active substance combination according to to one or more of claims 1 to 20 for the production of a medicament for the treatment or prevention of weight-gain, obesity, dylipidaemia, Type 2 diabetes, Metabolic Syndrome, or cardio- and cerebrovascular accidents.
